(19) Europäisches Patentamt
European Patent Office
Office européen des brevets

(11) EP 4 741 392 A1

(12) **EUROPEAN PATENT APPLICATION**
published in accordance with Art. 153(4) EPC

(43) Date of publication:
**13.05.2026 Bulletin 2026/20**

(21) Application number: **24835344.3**

(22) Date of filing: **02.07.2024**

(51) International Patent Classification (IPC):
***C07D 471/04*** *(2006.01)* ***A61P 35/00*** *(2006.01)*
***A61P 37/00*** *(2006.01)*

(52) Cooperative Patent Classification (CPC):
**A61K 31/437; A61K 31/4375; A61K 31/444; A61K 31/4545; A61K 31/496; A61K 31/497; A61K 31/4985; A61K 31/501; A61K 31/5025; A61K 31/506; A61K 31/517; A61K 31/519; A61K 31/53; A61K 31/5377; A61K 31/5383;**
(Cont.)

(86) International application number:
**PCT/CN2024/103173**

(87) International publication number:
**WO 2025/007863 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.07.2023 CN 202310806589**

(71) Applicant: **Shenzhen TargetRx Co., Ltd.**
**Shenzhen, Guangdong 518057 (CN)**

(72) Inventors:
- **WANG, Yihan**
  **Shenzhen, Guangdong 518057 (CN)**
- **LI, Huanyin**
  **Shenzhen, Guangdong 518057 (CN)**
- **AI, Yixin**
  **Shenzhen, Guangdong 518057 (CN)**

(74) Representative: **Novitas Patent AB**
**P.O. Box 55557**
**102 04 Stockholm (SE)**

(54) **1H-PYRAZOLO[4,3-C]PYRIDINE COMPOUND, AND COMPOSITION THEREOF AND USE THEREOF**

(57) The present invention relates to a compound as represented by formula (A), or a tautomer, a stereoisomer, a prodrug, a crystal form, a pharmaceutically acceptable salt, a hydrate or a solvate thereof, and a pharmaceutical composition thereof and the use thereof in the treatment and/or prevention of diseases mediated by a wild-type and/or mutated BTK.

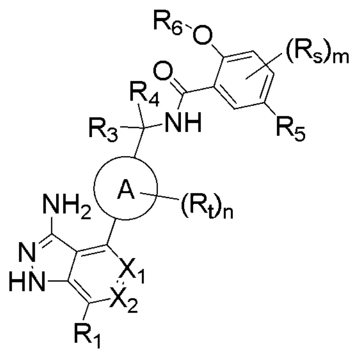

(A)

(52) Cooperative Patent Classification (CPC): (Cont.)
**A61P 29/00; A61P 35/00; A61P 37/00;
A61P 37/02; A61P 37/08; C07D 471/04;
C07D 519/00**

## Description

This application claims the priority of Chinese application No. 202310806589.3, filed on July 3, 2023, which is incorporated herein by reference in its entirety.

## TECHNICAL FIELD

The present disclosure belongs to the technical field of medicine, and particularly relates to a highly selective compound which has an inhibitory effect on Bruton's tyrosine kinase (BTK) and its drug resistance mutation, a pharmaceutical composition comprising the same, and a preparation method and use thereof.

## BACKGROUND

Bruton's tyrosine kinase belongs to the Tec family of cytoplasmic tyrosine kinases, which is the second largest family of non-receptor kinases in human. It is expressed in all cell lineages of hematopoietic system (except T cells), and it is located in bone marrow, spleen and lymph nodes tissues. Inactive mutations in the gene encoding Btk lead to X-linked agammaglobulinemia (XLA) in humans and X-linked immunodeficiency (XID) in mice. These two diseases are characterized by major defects in the development and function of B cells, indicating that Btk plays a vital role in the development and function of B cells. In addition, the constitutive activation of Btk in B cells leads to the accumulation of autoreactive plasma cells. Preclinical studies have shown that Btk-deficient mice are resistant to the development of collagen-induced arthritis. In addition, the clinical study of Rituxan (a CD20 antibody that exhausts mature B cells) have revealed the key role of B cells in many inflammatory diseases (such as rheumatoid arthritis, systemic lupus erythematosus and multiple sclerosis). Moreover, the abnormal activation of Btk plays an important role in the pathogenesis of B-cell lymphoma, indicating that the inhibition of Btk can be used to treat hematological malignancies.

Ibrutinib, a covalent Btk inhibitor, has been approved by the US Food and Drug Administration for the treatment of chronic lymphocytic leukemia (CLL), mantle cell lymphoma (MCL), Waldenstrom macroglobulinemia (WM) and chronic graft-versus-host disease (cGVHD). Despite its excellent efficacy and general tolerance, adverse events such as bleeding, rash and diarrhea have been reported.

These adverse reactions related to ibrutinib are thought to be mainly related to the off-target effect of ibrutinib, which has been proved to be associated with the inhibition of EGFR and Tec. Targeting EGFR can induce significant skin toxicity and gastrointestinal adverse reactions, because EGFR signaling cascade involves the biology of skin and gastrointestinal system. Both Btk and Tec belong to Tec family kinases. Platelets express Btk and Tec, which serve the downstream of glycoprotein VI(GPVI) signaling. Tec compensates for the loss of Btk in the downstream signaling of GPVI in rat platelets. The inhibition of Tec kinase by ibrutinib interferes with platelet aggregation and may contribute to the observed bleeding. Therefore, Btk inhibitors with high Btk inhibition and low EGFR and Tec inhibition are needed to reduce or avoid bleeding, rash and diarrhea.

Ibrutinib also irreversibly binds to interleukin-2-inducible tyrosine kinase (ITK). ITK plays a key role in the natural killer function of NK cells stimulated by FcR, primarily through antibody-dependent NK cell-mediated cytotoxicity (ADCC). ADCC is the standard of treatment for B-cell malignancies today, so it is desirable to have a Btk inhibitor that has high Btk inhibition and low ITK inhibition.

Covalent (irreversible) BTK inhibitors specifically target the cysteine residue C481 within BtK. After treatment with ibrutinib, primary and secondary drug resistance occurred. Mutations in BTK such as C481S, C481Y, C481R and C481F have been proved to clearly interfere with drug binding. It is predicted that the observed incidence of drug resistance will increase when clinical use goes on over time.

Non-covalent (reversible) BTK inhibitors do not need to bind to the C481 residue of BTK to effectively inhibit the wild-type and mutant BTK with the C481 residue replaced. However, existing literature has reported that non-covalent BTK inhibitors can also have acquired drug resistance mutations, such as V416L, A428D, M437R, T474I, and L528W mutations.

Therefore, it is necessary to develop new BTK inhibitors.

## SUMMARY

The present disclosure provides a novel 1H-pyrazolo[4,3-c]pyridine compound, a composition comprising the compound, and use thereof, wherein the compound has better inhibitory activity on BTK, C481 mutated BTK, and V416L, A428D, M437R, T474I, or L528W mutanted BTK kinases, higher selectivity compared with EGFR, TEC, and ITK kinases, and better pharmacodynamic and/or pharmacokinetic properties, and can treat a BTK kinase-mediated disease or condition.

In this regard, the present disclosure adopts the following technical solutions:

In one aspect, the present disclosure relates to a compound of formula (A), or a tautomer, a stereoisomer, a prodrug, a crystal form, a pharmaceutically acceptable salt, a hydrate or a solvate thereof:

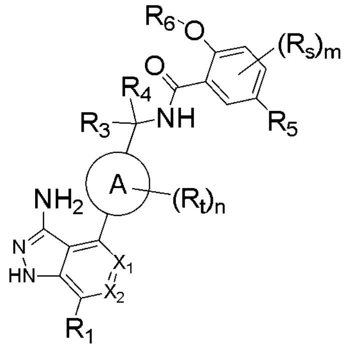

(A)

wherein,

ring A is a benzene ring or a 5- to 6-membered heteroaromatic ring;
$X_1$ is N, CD or CH;
$X_2$ is N or $CR_2$;
$R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R;
wherein, each of $R_a$, $R_b$, and $R_c$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;
$R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R';
$R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_s$ and $R_t$ are independently H, D, halogen, $-OR_a$, $-NR_bR_c$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
m is 0, 1, 2 or 3;
n is 0, 1, 2, 3 or 4;
each R is independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $-C_{1-6}$ alkylene-$C_{3-6}$ cycloalkyl, $-C_{1-6}$ alkylene-3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R";

each R' is independently H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl,

$C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

wherein, each of $R_d$, $R_e$, and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R"';

each R"' is independently H, D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein, the $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl is optionally substituted with one or more D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl;

wherein, each of $R_g$, $R_h$, and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In another aspect, the present disclosure relates to a compound of formula (I), or a tautomer, a stereoisomer, a prodrug, a crystal form, a pharmaceutically acceptable salt, a hydrate or a solvate thereof:

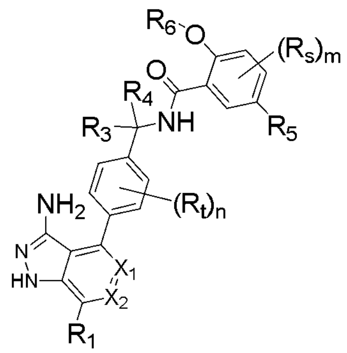

(I)

wherein,

$X_1$ is N, CD or CH;

$X_2$ is N or $CR_2$;

$R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R;

wherein each of $R_a$, $R_b$ and $R_c$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;

$R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-

membered heterocyclyl, $C_{2-6}$ alkenyl, $C_{2-6}$ alkynyl, $C_{6-10}$ aryl and 5- to 10-membered heteroaryl are optionally substituted with one or more R';

$R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_5$ is halogen;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_s$ and $R_t$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

m is 0, 1, 2 or 3;

n is 0, 1, 2, 3 or 4;

each R is independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R";

each R' is independently H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In another aspect, the present disclosure relates to a pharmaceutical composition, comprising the compound of the present disclosure, or a tautomer, a stereoisomer, a prodrug, a crystal form, a pharmaceutically acceptable salt, a hydrate or a solvate thereof, and a pharmaceutically acceptable excipient, and optionally, other therapeutic agent(s).

In another aspect, the present disclosure relates to use of the compound of the present disclosure, or a tautomer, a stereoisomer, a prodrug, a crystal form, a pharmaceutically acceptable salt, a hydrate or a solvate thereof, or the pharmaceutical composition of the present disclosure in the manufacture of a medicament for the treatment and/or prevention of a wild and/or mutated BTK kinase-mediated disease; alternatively, the mutated BTK kinase is selected from BTK C481S, BTK C481F, BTK C481Y, BTK C481R, BTK C481T, BTK C481G and BTK C481W; alternatively, the mutated BTK kinase is BTK C481S; alternatively, the mutated BTK kinase is BTK V416L, BTK A428D, BTK M437R, BTK T474I or BTK L528W; alternatively, the mutated BTK kinase is BTK T474I or BTK L528W.

In another aspect, the present disclosure relates to a method of treating and/or preventing a wild and/or mutated

BTK kinase-mediated disease in a subject, comprising administering to the subject the compound of the present disclosure or a pharmaceutically acceptable salt, a stereoisomer, a solvate, a hydrate, a polymorph, a prodrug or an isotopic variant thereof, or the pharmaceutical composition of the present disclosure; alternatively, the mutated BTK kinase is selected from BTK C481S, BTK C481F, BTK C481Y, BTK C481R, BTK C481T, BTK C481G and BTK C481W; alternatively, the mutated BTK kinase is BTK C481S; alternatively, the mutated BTK kinase is BTK V416L, BTK A428D, BTK M437R, BTK T474I or BTK L528W; alternatively, the mutated BTK kinase is BTK T474I or BTK L528W.

In another aspect, the present disclosure relates to the compound of the present disclosure, or a tautomer, a stereoisomer, a prodrug, a crystal form, a pharmaceutically acceptable salt, a hydrate or a solvate thereof, or the pharmaceutical composition of the present disclosure, for use in the treatment and/or prevention of a wild and/or mutated BTK kinase-mediated disease; alternatively, the mutated BTK kinase is selected from BTK C481S, BTK C481F, BTK C481Y, BTK C481R, BTK C481T, BTK C481G and BTK C481W; alternatively, the mutated BTK kinase is BTK C481S; alternatively, the mutated BTK kinase is BTK V416L, BTK A428D, BTK M437R, BTK T474I or BTK L528W; alternatively, the mutated BTK kinase is BTK T474I or BTK L528W.

In a more specific aspect, the BTK-mediated disease of the present disclosure is selected from allergic diseases, autoimmune diseases, inflammatory diseases and cancers.

In a more specific aspect, the BTK-mediated disease of the present disclosure is a B-cell proliferative disease selected from chronic lymphocytic lymphoma, non-Hodgkin's lymphoma, diffuse large B-cell lymphoma, mantle cell lymphoma, follicular lymphoma, chronic lymphocytic leukemia, acute lymphocytic leukemia, acute myeloid leukemia, acute promyelocytic leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplasia, mixed lineage leukemia, myelodysplastic syndrome, myeloproliferative disorder, marginal zone lymphoma and Waldenstrom's macroglobulinemia.

In a more specific aspect, the BTK-mediated disease of the present disclosure is multiple myeloma.

Other objects and advantages of the present disclosure will be apparent to those skilled in the art from the specific embodiments, examples and claims that follow.

Definitions

Chemical definitions

The definitions of specific functional groups and chemical terms are described in more detail below.

When a range of values is listed, it is intended to encompass each value and sub-range within the range. For example, "$C_{1-6}$ alkyl" is intended to encompass $C_1$, $C_2$, $C_3$, $C_4$, Cs, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyl.

"$C_{1-6}$ alkyl" refers to a radical of a straight or branched, saturated hydrocarbon group having 1 to 6 carbon atoms, and is also referred to herein as "lower alkyl". In some embodiments, $C_{1-4}$ alkyl is yet alternative; in some embodiments, $C_{1-3}$ alkyl is yet alternative. Examples of the alkyl include, but are not limited to, methyl ($C_1$), ethyl ($C_2$), n-propyl ($C_3$), iso-propyl ($C_3$), n-butyl ($C_4$), t-butyl ($C_4$), sec-butyl ($C_4$), iso-butyl ($C_4$), n-pentyl ($C_5$), 3-pentyl ($C_5$), pentyl ($C_5$), neo-pentyl ($C_5$), 3-methyl-2-butyl ($C_5$), t-pentyl ($C_5$) and n-hexyl ($C_6$). Regardless of whether or not the alkyl group is modified with "substituted", each alkyl group is independently optionally substituted, for example, with 1 to 5 substituents, 1 to 3 substituents or 1 substituent. Appropriate substituents are defined as follows.

"$C_{1-6}$ alkylene" refers to a divalent group formed by removing one hydrogen atom of the "$C_{1-6}$ alkyl".

"Halo" or "halogen" refers to fluoro (F), chloro (Cl), bromo (Br) and iodo (I). In some embodiments, the halo group is F, Cl or Br. In some embodiments, the halo group is F or Cl. In some embodiments, the halo group is F.

Therefore, "$C_{1-6}$ haloalkyl" and "$C_{1-3}$ haloalkyl" refer to the above "$C_{1-6}$ alkyl" and "$C_{1-3}$ alkyl" substituted with one or more halo groups, respectively. In some embodiments, $C_{1-4}$ haloalkyl is yet alternative, and $C_{1-2}$ haloalkyl is yet alternative. Examples of the haloalkyl include, but are not limited to, $-CF_3$, $-CH_2F$, - $CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, etc.

"$C_{2-6}$ alkenyl" refers to a radical of a straight or branched hydrocarbon group having 2 to 6 carbon atoms and one or more carbon-carbon double bonds (e.g., 1, 2 or 3 carbon-carbon double bonds). The one or more carbon-carbon double bonds can be internal (e.g., in 2-butenyl) or terminal (e.g., in 1-butenyl). In some embodiments, $C_{2-4}$ alkenyl is yet alternative. Examples of the alkenyl include, but are not limited to, vinyl ($C_2$), 1-propenyl ($C_3$), 2-propenyl ($C_3$), 1-propen-2-yl ($C_3$), 1-butenyl ($C_4$), 2-butenyl ($C_4$), butadienyl ($C_4$), pentenyl ($C_5$), pentadienyl ($C_5$), hexenyl ($C_6$), etc. Regardless of whether or not the alkenyl group is modified with "substituted", each alkenyl group is independently optionally substituted, for example, with 1 to 5 substituents, 1 to 3 substituents or 1 substituent. Appropriate substituents are defined as follows. In some embodiments, the alkenyl is unsubstituted $C_{2-6}$ alkenyl. In some embodiments, the alkenyl is substituted $C_{2-6}$ alkenyl.

"$C_{2-6}$ alkynyl" refers to a radical of a straight or branched hydrocarbon group having 2 to 6 carbon atoms, one or

more carbon-carbon triple bonds (e.g., 1, 2 or 3 carbon-carbon triple bonds), and optionally one or more carbon-carbon double bonds (e.g., 1, 2 or 3 carbon-carbon double bonds). In some embodiments, $C_{2-4}$ alkynyl is yet alternative. In some embodiments, the alkynyl group does not contain any double bonds. One or more carbon-carbon triple bonds may be internal (e.g., in 2-butynyl) or terminal (e.g., in 1-butynyl). Examples of the alkynyl include, but are not limited to, ethynyl ($C_2$), 1-propynyl ($C_3$), 2-propynyl ($C_3$), 1-butynyl ($C_4$), 2-butynyl ($C_4$), pentynyl ($C_5$), 3-methylbut-1-ynyl ($C_5$), hexynyl ($C_6$), etc. Regardless of whether or not the alkynyl group is modified with "substituted", each alkynyl group is independently optionally substituted, for example, with 1 to 5 substituents, 1 to 3 substituents or 1 substituent. Appropriate substituents are defined as follows. In some embodiments, the alkynyl is substituted $C_{2-6}$ alkynyl.

"$C_{3-10}$ cycloalkyl" refers to a radical of a non-aromatic cyclic hydrocarbon group having from 3 to 10 ring carbon atoms and zero heteroatoms. In some embodiments, $C_{3-8}$ cycloalkyl is alternative, $C_{3-6}$ cycloalkyl is still alternative, and $C_{4-6}$ cycloalkyl and $C_{5-6}$ cycloalkyl are yet alternative. The cycloalkyl also includes a ring system in which the cycloalkyl described above is fused with one or more aryl or heteroaryl groups, wherein the point of attachment is on the cycloalkyl ring, and in such case, the number of carbon atoms continues to represent the number of carbon atoms in the cycloalkyl system. Examples of the cycloalkyl include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), bicyclo[2.2.1]heptyl ($C_7$), bicyclo[2.2.2]octyl ($C_8$), cyclononyl ($C_9$), cyclononenyl ($C_9$), cyclodecyl ($C_{10}$), cyclodecenyl ($C_{10}$), octahydro-1H-indenyl ($C_9$), decahydronaphthyl ($C_{10}$), spiro[4.5]decyl ($C_{10}$), etc. Regardless of whether or not the cycloalkyl group is modified with "substituted", each cycloalkyl group is independently optionally substituted, for example, with 1 to 5 substituents, 1 to 3 substituents or 1 substituent. Appropriate substituents are defined as follows.

"3- to 12-membered heterocyclyl" refers to a radical of a 3- to 12-membered non-aromatic ring system having ring carbon atoms and 1 to 4 ring heteroatoms, wherein each heteroatom is independently selected from nitrogen, oxygen, sulfur, boron, phosphorus, and silicon. In heterocyclyl groups that contain one or more nitrogen atoms, the point of attachment can be a carbon or nitrogen atom, as valency permits. In some embodiments, 3- to 10-membered heterocyclyl is alternative, and it is a 3- to 10-membered non-aromatic ring system having ring carbon atoms and 1 to 4 ring heteroatoms; in some embodiments, 5- to 10-membered heterocyclyl is alternative, and it is a 5- to 10-membered non-aromatic ring system having ring carbon atoms and 1 to 4 ring heteroatoms; in some embodiments, 3- to 7-membered heterocyclyl is alternative, and it is a 3-to 7-membered non-aromatic ring system having ring carbon atoms and 1 to 3 ring heteroatoms, especially 3-to 7-membered monocyclic non-aromatic ring system (also known as 3- to 7-membered monocyclic heterocyclyl); in some embodiments, 4- to 7-membered heterocyclyl is yet alternative, and it is a 4- to 7-membered non-aromatic ring system having ring carbon atoms and 1 to 3 ring heteroatoms; in some embodiments, 3- to 6-membered heterocyclyl is yet alternative, and it is a 3- to 6-membered non-aromatic ring system having ring carbon atoms and 1 to 3 ring heteroatoms; in some embodiments, 4- to 6-membered heterocyclyl is yet alternative, and it is a 4- to 6-membered non-aromatic ring system having ring carbon atoms and 1 to 3 ring heteroatoms; 5- to 6-membered heterocyclyl is yet alternative (e.g., 5-membered heterocyclyl or 6-membered heterocyclyl), and it is a 5- or 6-membered non-aromatic ring system having ring carbon atoms and 1 to 3 ring heteroatoms. The heterocyclyl also includes a ring system wherein the heterocyclyl ring described above is fused or spiro-linked with one or more cycloalkyl, heterocyclyl, aryl or heteroaryl groups, wherein the point of attachment is on the heterocyclyl ring, and in such cases, the number of ring members continues to represent the number of ring members in the heterocyclyl ring system. Therefore, this application also includes "6- to 12-membered fused bicyclic heterocycles" and "8- to 10-membered fused bicyclic heterocycles" which represent a heterocyclyl in which two rings are fused together by sharing two adjacent atoms, having a total of 6 to 12 ring atoms and 8 to 10 ring atoms, respectively. This application also includes "7- to 10-membered spiro heterocycles", which represent a heterocyclyl in which two rings are spiro-linked together by sharing one atom, having a total of 7 to 10 ring atoms. Regardless of whether or not the heterocyclyl group is modified with "substituted", each heterocyclyl group is independently optionally substituted, for example, with 1 to 5 substituents, 1 to 3 substituents or 1 substituent. Appropriate substituents are defined as follows.

Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocyclyl groups containing one heteroatom include, but are not limited to, azepanyl, oxepanyl and thiepanyl. Exemplary 8-membered heterocyclyl groups containing

one heteroatom include, but are not limited to, azocanyl, oxecanyl and thiocanyl. Exemplary 5-membered heterocyclyl groups fused with a $C_6$ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a $C_6$ aryl (also referred as 6,6-bicyclic heterocyclyl herein) include, but are not limited to, tetrahydroquinolinyl, tetrahydroisoquinolinyl, etc.

"$C_{6-10}$ aryl" refers to a radical of monocyclic or polycyclic (e.g., bicyclic) 4n+2 aromatic ring system having 6 to 10 ring carbon atoms and zero heteroatoms provided in the aromatic ring system (e.g., having 6 or 10 shared $\pi$ electrons in a cyclic array). In some embodiments, the aryl group has six ring carbon atoms ("$C_6$ aryl"; for example, phenyl). In some embodiments, the aryl group has ten ring carbon atoms ("$C_{10}$ aryl"; for example, naphthyl, e.g., 1-naphthyl and 2-naphthyl). "$C_{6-10}$ aryl" also includes a ring system in which the aryl ring described above is fused with one or more carbocyclyl or heterocyclyl groups, wherein, the atomic groups or the point of attachment is on the aryl ring, in which case the number of carbon atoms continues to represent the number of carbon atoms in the aryl ring system. Regardless of whether or not the aryl group is modified with "substituted", each aryl group is independently optionally substituted, for example, with 1 to 5 substituents, 1 to 3 substituents or 1 substituent. Appropriate substituents are defined as follows.

"5- to 10-membered heteroaryl" refers to a radical of 5- to 10-membered monocyclic or bicyclic 4n+2 aromatic ring system (e.g., having 6 or 10 shared $\pi$ electrons in a cyclic array) having ring carbon atoms and 1 to 4 ring heteroatoms, wherein each heteroatom is independently selected from nitrogen, oxygen and sulfur. In some embodiments, 5- to 6-membered heteroaryl groups are alternative (e.g., 5-membered heteroaryl or 6-membered heteroaryl), which are radicals of 5- or 6-membered monocyclic 4n+2 aromatic ring systems (e.g., having 6 shared $\pi$ electrons in a cyclic array) having ring carbon atoms and 1 to 4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more carbocyclyl or heterocyclyl groups, and the point of attachment is on the heteroaryl ring. In such case, the number of the carbon atoms continues to represent the number of carbon atoms in the heteroaryl ring system. Regardless of whether or not the heteroaryl group is modified with "substituted", each heteroaryl group is independently optionally substituted, for example, with 1 to 5 substituents, 1 to 3 substituents or 1 substituent. Appropriate substituents are defined as follows.

Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl, and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl.

Exemplary substituents on carbon atoms include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, carbocyclyl , heterocyclyl, aryl and heteroaryl, wherein each of the alkyl, alkenyl, alkynyl, carbocyclyl , heterocyclyl, aryl and heteroaryl is independently substituted with 0, 1, 2, 3, 4 or 5 $R^{dd}$ groups;

or two geminal hydrogens on a carbon atom are substituted with =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$ groups;

each of the $R^{aa}$ is independently selected from alkyl, haloalkyl, alkenyl, alkynyl, carbocyclyl, heterocyclyl, aryl and heteroaryl, or two $R^{aa}$ groups are combined to form a heterocyclyl or heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{bb}$ is independently selected from hydrogen, -OH, -$OR^{aa}$, -$N(R^{cc})_2$, -CN, -$C(=O)R^{aa}$, - $C(=O)N(R^{cc})_2$, -$CO_2R^{aa}$, -$SO_2R^{aa}$, -$C(=NR^{cc})OR^{aa}$, -$C(=NR^{cc})N(R^{cc})_2$, -$SO_2N(R^{cc})_2$, -$SO_2R^{cc}$, -$SO_2OR^{cc}$, - $SOR^{aa}$, -$C(=S)N(R^{cc})_2$, -$C(=O)SR^{cc}$, -$C(=S)SR^{cc}$, -$P(=O)_2R^{aa}$, -$P(=O)(R^{aa})_2$, -$P(=O)_2N(R^{cc})_2$, -$P(=O)(NR^{cc})_2$, alkyl, haloalkyl, alkenyl, alkynyl, carbocyclyl, heterocyclyl, aryl and heteroaryl, or two $R^{bb}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{cc}$ is independently selected from hydrogen, alkyl, haloalkyl, alkenyl, alkynyl, carbocyclyl, heterocyclyl, aryl and heteroaryl, or two $R^{cc}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{dd}$ is independently selected from halogen, -CN, -$NO_2$, -$N_3$, -$SO_2H$, -$SO_3H$, -OH, -$OR^{ee}$, -$ON(R^{ff})_2$, -$N(R^{ff})_2$, -$N(R^{ff})_3{}^+X^-$, -$N(OR^{ee})R^{ff}$, -SH, -$SR^{ee}$, -$SSR^{ee}$, -$C(=O)R^{ee}$, -$CO_2H$, -$CO_2R^{ee}$, -$OC(=O)R^{ee}$, - $OCO_2R^{ee}$, -$C(=O)N(R^{ff})_2$, -$OC(=O)N(R^{ff})_2$, -$NR^{ff}C(=O)R^{ee}$, -$NR^{ff}CO_2R^{ee}$, -$NR^{ff}C(=O)N(R^{ff})_2$, -$C(=NR^{ff})OR^{ee}$, -$OC(=NR^{ff})R^{ee}$, -$OC(=NR^{ff})OR^{ee}$, -$C(=NR^{ff})N(R^{ff})_2$, -$OC(=NR^{ff})N(R^{ff})_2$, -$NR^{ff}C(=NR^{ff})N(R^{ff})_2$, -$NR^{ff}SO_2R^{ee}$, -$SO_2N(R^{ff})_2$, -$SO_2R^{ee}$, -$SO_2OR^{ee}$, -$OSO_2R^{ee}$, -$S(=O)R^{ee}$, -$Si(R^{ee})_3$, -$OSi(R^{ee})_3$, -$C(=S)N(R^{ff})_2$, -$C(=O)SR^{ee}$, - $C(=S)SR^{ee}$, -$SC(=S)SR^{ee}$, -$P(=O)_2R^{ee}$, -$P(=O)(R^{ee})_2$, -$OP(=O)(R^{ee})_2$, -$OP(=O)(OR^{ee})_2$, alkyl, haloalkyl, alkenyl, alkynyl, carbocyclyl, heterocyclyl, aryl, and heteroaryl, wherein, each of the alkyl, alkenyl, alkynyl, carbocyclyl, heterocyclyl, aryl and heteroaryl is independently substituted with 0, 1, 2, 3, 4 or 5 $R^{gg}$ groups, or two geminal $R^{dd}$ substituents can be combined to form=O or =S;

each of the $R^{ee}$ is independently selected from alkyl, haloalkyl, alkenyl, alkynyl, carbocyclyl, aryl, heterocyclyl, and heteroaryl, wherein, each of the alkyl, alkenyl, alkynyl, carbocyclyl, heterocyclyl, aryl and heteroaryl is independently substituted with 0, 1, 2, 3, 4 or 5 $R^{gg}$ groups;

each of the $R^{ff}$ is independently selected from hydrogen, alkyl, haloalkyl, alkenyl, alkynyl, carbocyclyl, heterocyclyl, aryl and heteroaryl, or two $R^{ff}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{gg}$ groups;

each of the $R^{gg}$ is independently halogen, -CN, -$NO_2$, -$N_3$, -$SO_2H$, -$SO_3H$, -OH, -$OC_{1-6}$ alkyl, - $ON(C_{1-6}alkyl)_2$, -$N(C_{1-6}$ $alkyl)_2$, -$N(C_{1-6}$ $alkyl)_3{}^+X^-$, -$NH(C_{1-6}$ $alkyl)_2{}^+X^-$, -$NH_2(C_{1-6}$ $alkyl)^+X^-$, -$NH_3{}^+X^-$, - $N(OC_{1-6}$ alkyl)($C_{1-6}$ alkyl), -N(OH)($C_{1-6}$ alkyl), -NH(OH), -SH, -$SC_{1-6}$ alkyl, -SS($C_{1-6}$ alkyl), -C(=O)($C_{1-6}$ alkyl), -$CO_2H$, -$CO_2(C_{1-6}$ alkyl), -OC(=O)($C_{1-6}$ alkyl), -$OCO_2(C_{1-6}$ alkyl), -$C(=O)NH_2$, -C(=O)N($C_{1-6}$ $alkyl)_2$, - OC(=O)NH($C_{1-6}$ alkyl), -NHC(=O)($C_{1-6}$ alkyl), -N($C_{1-6}$ alkyl) C(=O)($C_{1-6}$ alkyl), -$NHCO_2(C_{1-6}$ alkyl), - NHC(=O)N($C_{1-6}$ $alkyl)_2$, -NHC(=O)NH($C_{1-6}$ alkyl), -$NHC(=O)NH_2$, -C(=NH) O($C_{1-6}$ alkyl), -OC(=NH)($C_{1-6}$ alkyl), -OC(=NH)$OC_{1-6}$ alkyl, -C(=NH)N($C_{1-6}$ $alkyl)_2$, -C(=NH)NH($C_{1-6}$ alkyl), -C(=NH) $NH_2$, - OC(=NH)N($C_{1-6}$ $alkyl)_2$, -OC(NH)NH($C_{1-6}$ alkyl), -$OC(NH)NH_2$, -NHC(NH)N($C_{1-6}$ $alkyl)_2$, -$NHC(=NH)NH_2$, -$NHSO_2(C_{1-6}$ alkyl), -$SO_2N(C_{1-6}$ $alkyl)_2$, -$SO_2NH(C_{1-6}$ alkyl), -$SO_2NH_2$, -$SO_2C_{1-6}$ alkyl, -$SO_2OC_{1-6}$ alkyl, - $OSO_2C_{1-6}$ alkyl, -$SOC_{1-6}$ alkyl, -Si($C_{1-6}$ $alkyl)_3$, -OSi($C_{1-6}$ $alkyl)_3$, -C(=S)N($C_{1-6}$ $alkyl)_2$, C(=S)NH($C_{1-6}$ alkyl), C(=S)$NH_2$, -C(=O) S($C_{1-6}$ alkyl), -C(=S)$SC_{1-6}$ alkyl, -SC(=S)$SC_{1-6}$ alkyl, -$P(=O)_2(C_{1-6}$ alkyl), -P(=O)($C_{1-6}$ $alkyl)_2$, -OP(=O)($C_{1-6}$ $alkyl)_2$, -OP(=O)($OC_{1-6}$ $alkyl)_2$, $C_{1-6}$ alkyl, $C_{1-6}$ haloalkyl, $C_2$-$C_6$ alkenyl, $C_2$-$C_6$ alkynyl, $C_3$-$C_7$ carbocyclyl, $C_6$-$C_{10}$ aryl, $C_3$-$C_7$ heterocyclyl, or $C_5$-$C_{10}$ heteroaryl; or two geminal $R^{gg}$ substituents may combine to form =O or =S; wherein, $X^-$ is a counter-ion.

Exemplary substituents on nitrogen atoms include, but are not limited to, hydrogen, -OH, -$OR^{aa}$, - $N(R^{cc})_2$, -CN, -$C(=O)R^{aa}$, -$C(=O)N(R^{cc})_2$, -$CO_2R^{aa}$, -$SO_2R^{aa}$, -$C(=NR^{bb})R^{aa}$, -$C(=NR^{cc})OR^{aa}$, - $C(=NR^{cc})N(R^{cc})_2$, -$SO_2N(R^{cc})_2$, -$SO_2R^{cc}$, -$SO_2OR^{cc}$, -$SOR^{aa}$, -$C(=S)N(R^{cc})_2$, -$C(=O)SR^{cc}$, -$C(=S)SR^{cc}$, - $P(=O)_2R^{aa}$, -$P(=O)(R^{aa})_2$, -$P(=O)_2N(R^{cc})_2$, -$P(=O)(NR^{cc})_2$, alkyl, haloalkyl, alkenyl, alkynyl, carbocyclyl, heterocyclyl, aryl and heteroaryl, or two $R^{cc}$ groups attached to a nitrogen atom combine to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described herein.

"Deuteration", "deuterated" or "D substituted" means that one or more hydrogens in the compound or in the group are substituted with deuterium; Deuteration can be monosubstituted, disubstituted, polysubstituted or fully substituted. The terms "substituted with one or more deuteriums" and "deuterated one or more times" are used interchangeably.

"Non-deuterated compound" refers to a compound with deuterium atom content not higher than the natural deuterium isotope content (0.015%).

The deuterium isotope content of deuterium at the deuterated position is at least greater than the natural deuterium isotope content (0.015%), alternatively greater than 30%, yet alternatively greater than 50%, yet alternatively greater than 75%, yet alternatively greater than 95%, and yet alternatively greater than 99%.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are

well known in the art. For example, Berge et al., describe pharmaceutically acceptable salts in detail in J. Pharmaceutical Sciences (1977) 66:1-19. Pharmaceutically acceptable salts of the compounds disclosed herein include those derived from suitable inorganic and organic acids and bases. Examples of pharmaceutically acceptable, nontoxic acid addition salts are salts formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid. Salts formed using conventional methods in the art such as ion exchange are also included. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and $N^+(C_{1-4}\ alkyl)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

"Disease", "disorder" and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplate an action that occurs before a subject begins to suffer from the specified disease, disorder or condition ("prophylactic treatment").

"Combination", "combined", and related terms refer to the simultaneous or sequential administration of the therapeutic agents of the present disclosure. For example, the compound disclosed herein may be administered with another therapeutic agent simultaneously or sequentially in separate unit dosage forms, or together in a single unit dosage form.

## DETAILED DESCRIPTION

Compounds

As used herein, "compound of the present disclosure" refers to a compound of formula (I) below (including subsets of each formula), or a tautomer, a stereoisomer, a prodrug, a crystal form, a pharmaceutically acceptable salt, a hydrate or a solvate thereof.

In one embodiment, the present disclosure relates to a compound of formula (A), or a tautomer, a stereoisomer, a prodrug, a crystal form, a pharmaceutically acceptable salt, a hydrate or a solvate thereof:

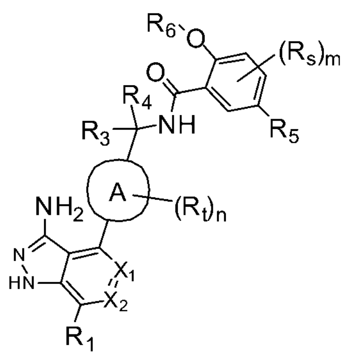

(A)

wherein,

ring A is a benzene ring or a 5- to 6-membered heteroaromatic ring;

$X_1$ is N, CD or CH;

$X_2$ is N or $CR_2$;

$R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R;

wherein each of $R_a$, $R_b$ and $R_c$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;

$R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R';

$R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_5$ is halogen;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_s$ and $R_t$ are independently H, D, halogen, $-OR_a$, $-NR_bR_c$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

m is 0, 1, 2 or 3;

n is 0, 1, 2, 3 or 4;

each R is independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $-C_{1-6}$ alkylene-$C_{3-6}$ cycloalkyl, $-C_{1-6}$ alkylene-3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R";

each R' is independently H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R'";

each R"' is independently H, D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered

heterocyclyl, $C_{6-10}$ aryl or 5- to 10-membered heteroaryl is optionally substituted with one or more D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In another embodiment, the present disclosure relates to a compound of formula (I), or a tautomer, a stereoisomer, a prodrug, a crystal form, a pharmaceutically acceptable salt, a hydrate or a solvate thereof:

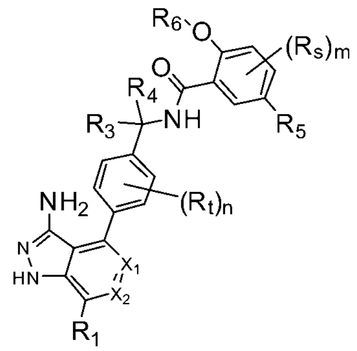

(I)

wherein,

$X_1$ is N, CD or CH;
$X_2$ is N or $CR_2$;
$R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R;
wherein each of $R_a$, $R_b$ and $R_c$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;
$R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R';
$R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_s$ and $R_t$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
m is 0, 1, 2 or 3;
n is 0, 1, 2, 3 or 4;
each R is independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R''; or
3) two R on the same atom or on two adjacent atoms are taken together with the atom(s) to which they are attached

to form $C_{3-6}$ cycloalkyl, 3- to 12-membered heterocyclyl, $C_{6-10}$ aryl or 5- to 10-membered heteroaryl, wherein the groups are optionally substituted with one or more R";

each R' is independently H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_e$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

Ring A

In one embodiment, ring A is a benzene ring; in another embodiment, ring A is a 5- to 6-membered heteroaromatic ring, for example, 6-membered heteroaromatic ring, for example, pyridine ring.

$X_1$

In one embodiment, $X_1$ is N; in another embodiment, $X_1$ is CH; in another embodiment, $X_1$ is CD.

$X_2$ and $R_2$

In one embodiment, $X_2$ is N; in another embodiment, $X_2$ is $CR_2$.

In one specific embodiment, $X_2$ is $CR_2$, and wherein $R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R'; in another specific embodiment, $X_2$ is $CR_2$, and wherein $R_2$ is H, D, halogen, $C_{1-6}$ alkyl, $C_{1-6}$ haloalkyl, $C_{3-6}$ cycloalkyl, 3- to 12-membered heterocyclyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{6-10}$ aryl or 5- to 10-membered heteroaryl is optionally substituted with one or more R'; in another specific embodiment, $X_2$ is $CR_2$, and wherein $R_2$ is H, D, halogen, $C_{1-6}$ alkyl, $C_{1-6}$ haloalkyl, $C_{3-6}$ cycloalkyl, 3- to 7-membered monocyclic heterocyclyl, 6- to 12-membered fused bicyclic heterocycle, 7- to 10-membered spiro heterocycle, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 7-membered monocyclic heterocyclyl, 6- to 12-membered fused bicyclic heterocycle, 7- to 10-membered spiro heterocycle, $C_{6-10}$ aryl and 5- to 10-membered heteroaryl are optionally substituted with one or more R'; in another specific embodiment, $X_2$ is $CR_2$, and wherein $R_2$ is H, D, $C_{1-6}$ alkyl, $C_{1-6}$ haloalkyl, $C_{3-6}$ cycloalkyl, 3- to 7-membered monocyclic heterocyclyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 7-membered monocyclic heterocyclyl, $C_{6-10}$ aryl and 5- to 10-membered heteroaryl are optionally substituted with one or more R'.

In one more specific embodiment, $X_2$ is $CR_2$, and wherein $R_2$ is H, D, halogen, methyl, $-CHF_2$, ethyl, isopropyl,

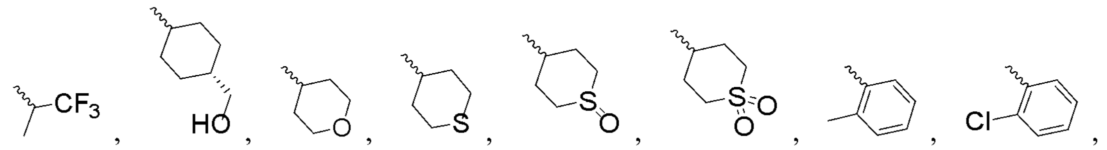

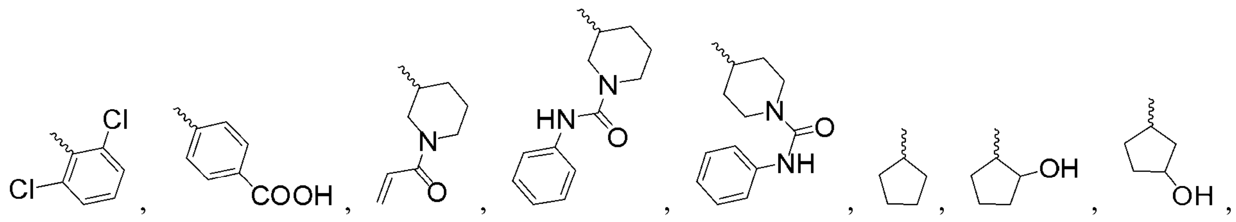

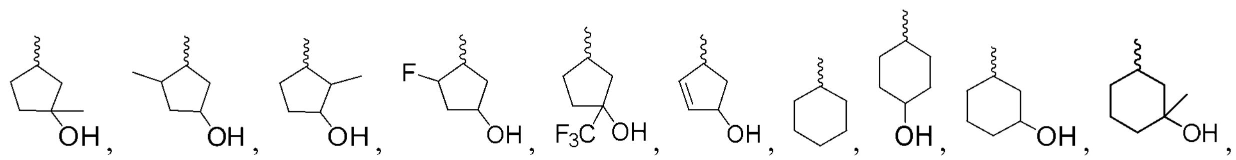

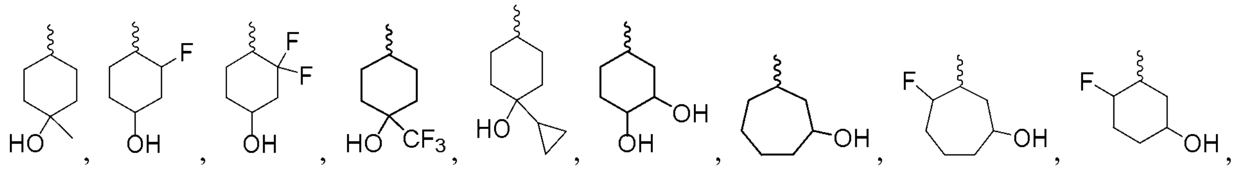

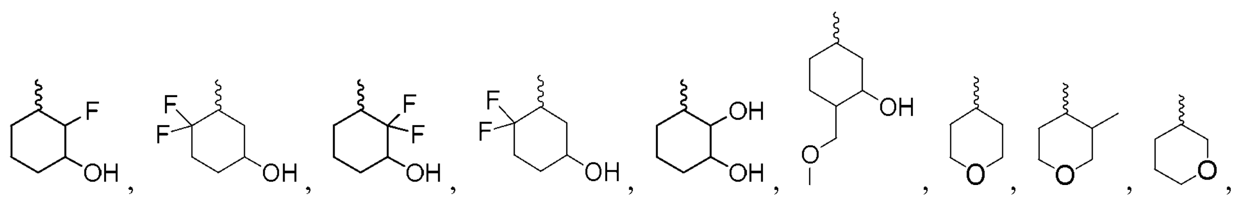

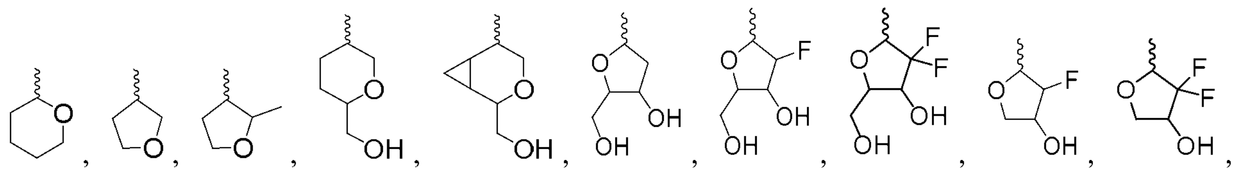

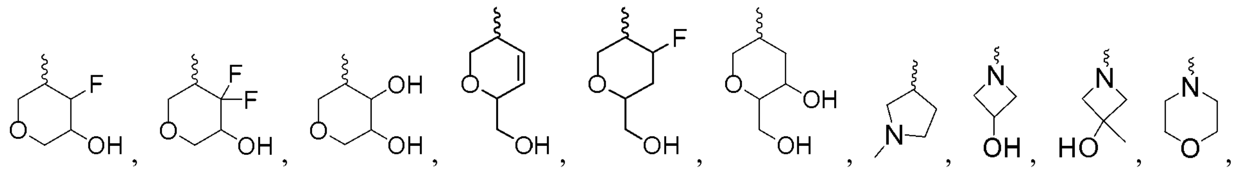

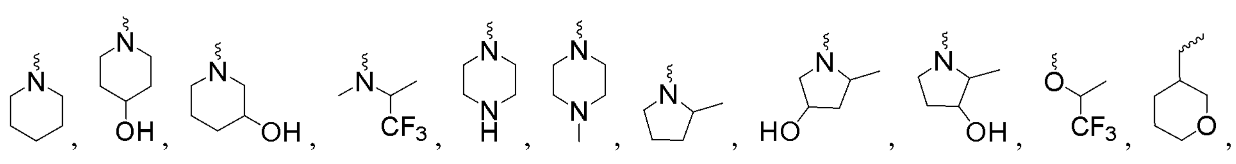

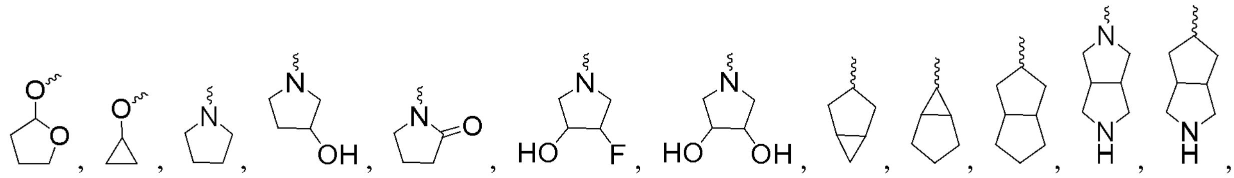

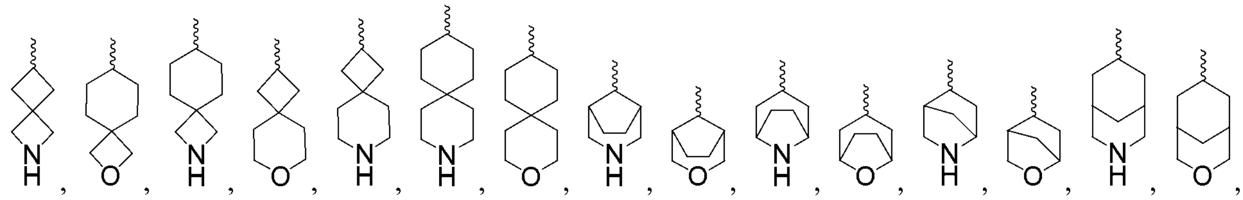

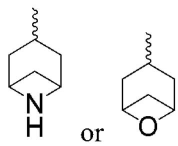
or ,

, wherein the above groups are optionally substituted with one or more D, up to fully deuterated; alternatively $R_2$ is H, D, halogen, methyl, $-CHF_2$, ethyl, isopropyl,

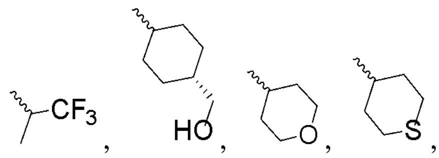

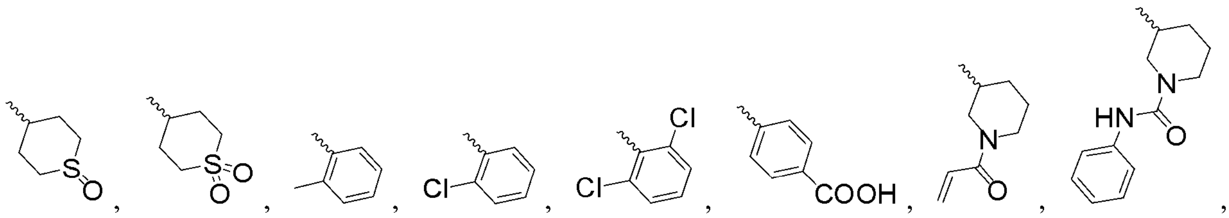

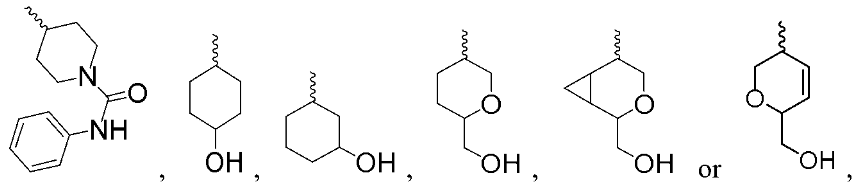
or ,

wherein the above groups are optionally substituted with one or more D, up to fully deuterated; alternatively $R_2$ is H, D, methyl, $-CHF_2$,

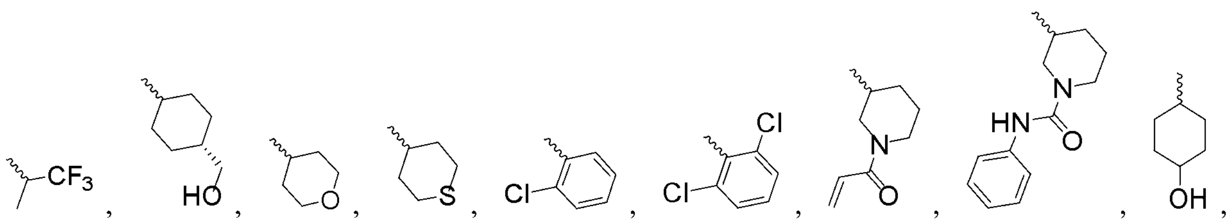

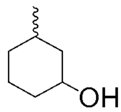, 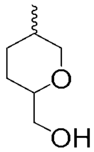, 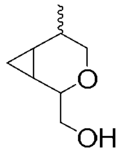 or 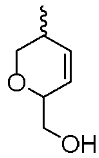,

wherein the above groups are optionally substituted with one or more D, up to fully deuterated; alternatively $R_2$ is H, D, methyl, $-CHF_2$,

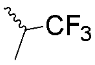, 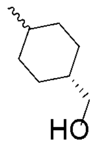, 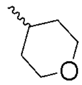

or

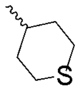,

wherein the above groups are optionally substituted with one or more D, up to fully deuterated; alternatively $R_2$ is H, D, $-CHF_2$ or

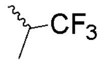,

wherein the above groups are optionally substituted with one or more D, up to fully deuterated; alternatively $R_2$ is H; alternatively $R_2$ is $-CHF_2$; alternatively $R_2$ is

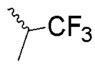,

wherein the above groups are optionally substituted with one or more D, up to fully deuterated.

$R_1$

In one embodiment, $R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R; in another embodiment, $R_1$ is H, D, halogen, $-C(O)NR_bR_c$, $C_{6-10}$ aryl or 5- to 10-membered heteroaryl, wherein the $C_{6-10}$ aryl and 5- to 10-membered heteroaryl are optionally substituted with one or more R; in another embodiment, $R_1$ is H; in another embodiment, $R_1$ is $C_{6-10}$ aryl or 5- to 10-membered heteroaryl, wherein the $C_{6-10}$ aryl and 5- to 10-membered heteroaryl are optionally substituted with one or more R; in another embodiment, $R_1$ is 5- to 10-membered heteroaryl, wherein the 5- to 10-membered heteroaryl is optionally substituted with one or more R. In another embodiment, $R_1$ is 5- or 6-membered heteroaryl, wherein the 5- or 6-membered heteroaryl is optionally substituted with one or more R.

In one specific embodiment, $R_1$ is

1) H; or

2)

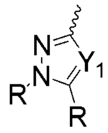

,

wherein $Y_1$ is N or CR; or

3)

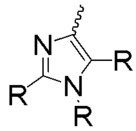

;

or

4)

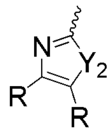

,

, wherein $Y_2$ is O or S; or

5)

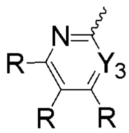

,

wherein $Y_3$ is N, CH or CD; or

6)

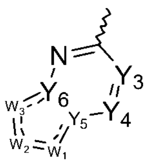 , 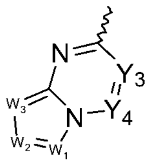 or 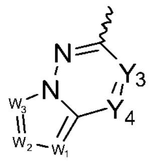 ,

wherein $Y_5$ and $Y_6$ are independently N or C, and at least one of $Y_5$ and $Y_6$ is N, $W_1$, $W_2$ or $W_3$ is independently N or CR, --- indicates single or double bonds, with the proviso that the bicyclic ring containing $Y_3$, $Y_4$, $Y_5$, $Y_6$, $W_1$, $W_2$ and $W_3$ is an aromatic ring; or

7)

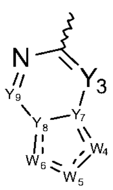 , 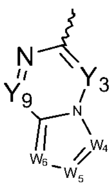 or 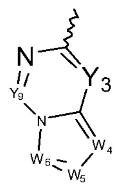 ;

, wherein $Y_7$ and $Y_8$ are independently N or C, and at least one of $Y_7$ and $Y_8$ is N, $Y_9$ is N, CH or CD, $W_4$, $W_5$ and $W_6$ are independently N or CR, $\overline{---}$ indicates single or double bonds, with the proviso that the bicyclic ring containing $Y_3$, $Y_7$, $Y_8$, $Y_9$, $W_4$, $W_5$ and $W_6$ is an aromatic ring.

In another specific embodiment, $R_1$ is

1) H; or

2)

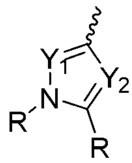,

wherein $Y_1$ and $Y_2$ are independently N or CR; or

3)

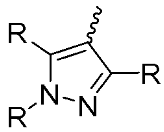;

or

4)

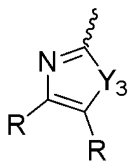,

wherein $Y_3$ is O or S; or

5)

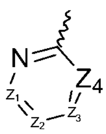, 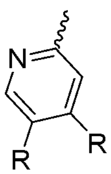 or 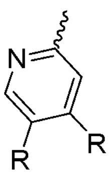,

wherein $Z_1$, $Z_2$, $Z_3$ and $Z_4$ are independently N or CR.

In one specific embodiment, $R_1$ is H, D,

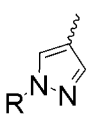, 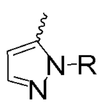, 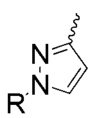, 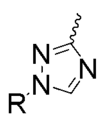, 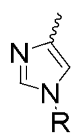, 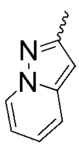,

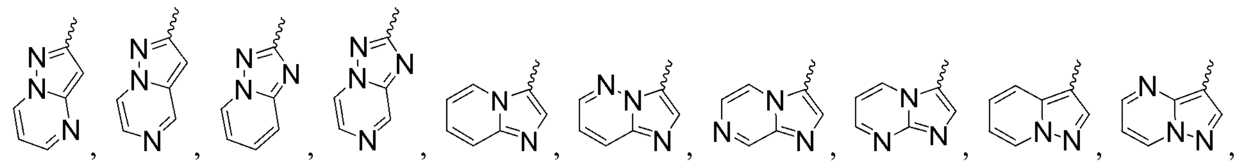
, , , , , , , , , ,

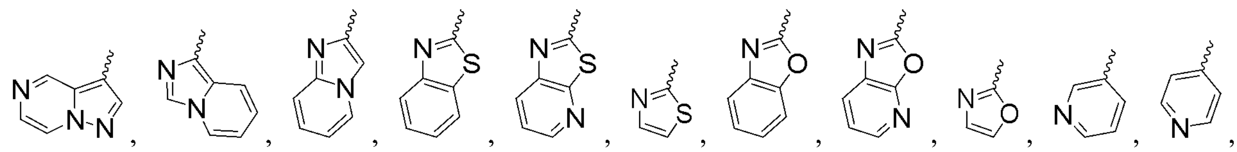
, , , , , , , , , , ,

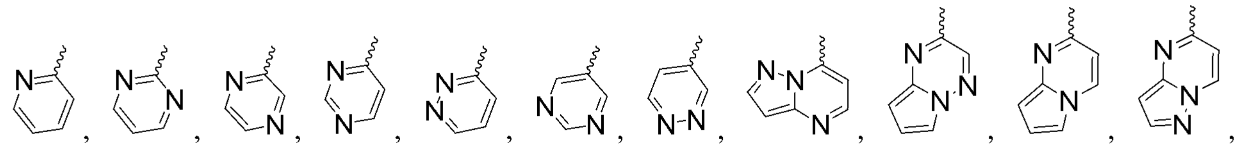
, , , , , , , , , , ,

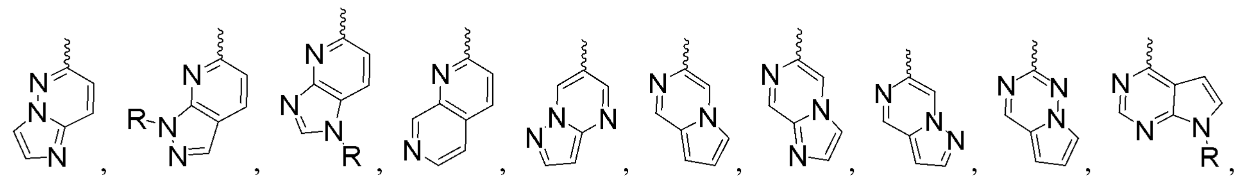
, , , , , , , , , ,

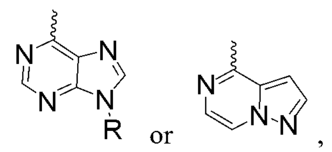
or ,

wherein the above groups are optionally substituted with one or more R; alternatively $R_1$ is

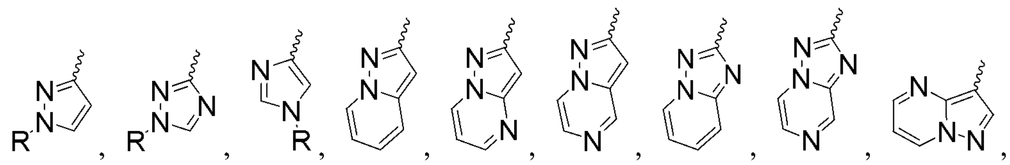
, , , , , , , , , ,

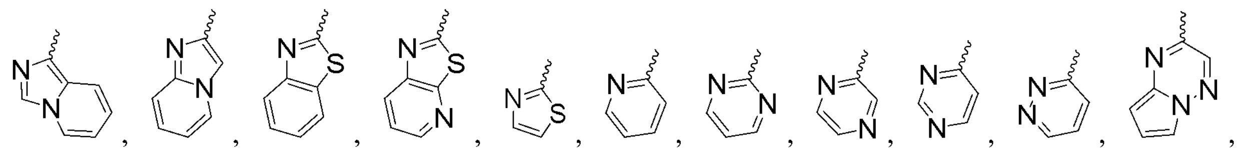
, , , , , , , , , , , ,

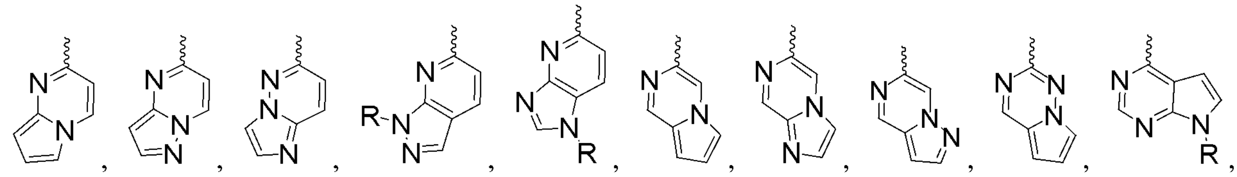
, , , , , , , , , ,

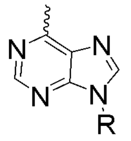 or 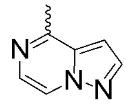 ,

wherein the above groups are optionally substituted with one or more R; alternatively $R_1$ is

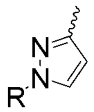 , 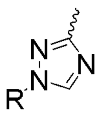 , 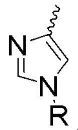 , 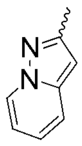 , 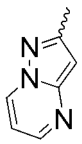 , 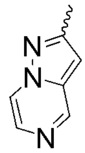 , 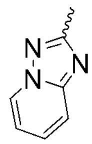 , 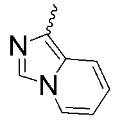 , 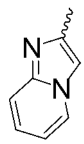 ,

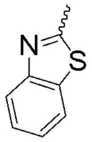 , 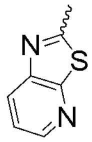 , 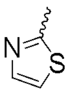 , 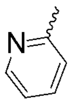 , 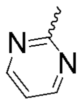 , 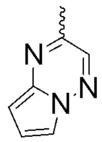 , 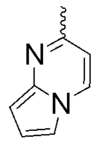 , 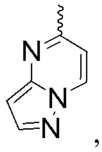 , 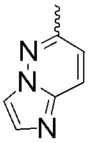 , 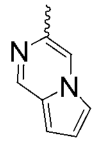 or 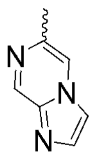 ,

wherein the above groups are optionally substituted with one or more R; alternatively $R_1$ is H.

$R_3$ and $R_4$

In one embodiment, $R_3$ and $R_4$ are independently H, D, halogen, $C_{1-6}$ alkyl or $C_{1-6}$ haloalkyl, wherein the $C_{1-6}$ alkyl and $C_6$ haloalkyl are optionally substituted with one or more D, up to fully deuterated; in another embodiment, $R_3$ and $R_4$ are independently H, D or halogen; in another embodiment, $R_3$ and $R_4$ are independently H, D or F; in another embodiment, $R_3$ and $R_4$ are independently H or D; in another embodiment, $R_3$ and $R_4$ are independently H. In another embodiment, $R_3$ and $R_4$ are independently D.

$R_5$ and $R_6$

In one embodiment, $R_5$ is halogen; in another embodiment, $R_5$ is F.

In one embodiment, $R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated; in another embodiment, $R_6$ is $C_{1-3}$ alkyl or $C_{1-3}$ haloalkyl, wherein the $C_{1-3}$ alkyl and $C_{1-3}$ haloalkyl are optionally substituted with one or more D, up to fully deuterated; in another embodiment, $R_6$ is methyl, $CD_3$, ethyl, isopropyl, $CHF_2$, $CH_2F$ or $CF_3$; in another embodiment, $R_6$ is methyl, $CF_3$ or $CD_3$. In another embodiment, $R_6$ is methyl or $CD_3$.

$R_s$ and m

In one embodiment, $R_s$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; in another embodiment, $R_s$ is H, D, halogen, $C_{1-3}$ alkyl or $C_{1-3}$ haloalkyl, wherein the $C_{1-3}$ alkyl and $C_{1-3}$ haloalkyl are optionally substituted with one or more D, up to fully deuterated; in another embodiment, $R_s$ is H, D or halogen; in another embodiment, $R_s$ is H, D or F; in another embodiment, $R_s$ is $-OR_a$ or $-NR_bR_c$; in another embodiment, $R_s$ is $-OR_a$.

In one embodiment, m is 0, 1, 2 or 3; in another embodiment, m is 0, 1 or 2; in another embodiment, m is 0 or 1; in another embodiment, m is 0; in another embodiment, m is 1; in another embodiment, m is 2; in another embodiment, m is 3.

In one specific embodiment, m is 1, and $R_s$ is H, D, F, methyl, $CD_3$ or $CF_3$; in another specific embodiment, m is 1, and $R_s$ is H, D, F, methyl or $CD_3$; in another specific embodiment, m is 1, and $R_s$ is H, D or F; in another specific embodiment, m is 1, and $R_s$ is methyl or $CD_3$.

$R_t$ and n

In one embodiment, Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; in another embodiment, $R_t$ is H, D, halogen, $C_{1-3}$ alkyl or $C_{1-3}$ haloalkyl, wherein the $C_{1-3}$ alkyl and $C_{1-3}$ haloalkyl are optionally substituted with one or more D, up to fully deuterated;

in another embodiment, $R_t$ is H, D, halogen or $C_{1-3}$ alkyl, wherein the $C_{1-3}$ alkyl is optionally substituted with one or more D, up to fully deuterated; in another embodiment, $R_t$ is H, D or halogen; in another embodiment, $R_t$ is H, D or F; in another embodiment, $R_t$ is $-OR_a$ or $-NR_bR_c$; in another embodiment, Rt is $-OR_a$.

In one embodiment, n is 0, 1, 2, 3 or 4; in another embodiment, n is 0, 1, 2 or 3; in another embodiment, n is 0, 1 or 2; in another embodiment, n is 0 or 1; in another embodiment, n is 0; in another embodiment, n is 1; in another embodiment, n is 2; in another embodiment, n is 3; in another embodiment, n is 4.

In one specific embodiment, n is 1, and $R_t$ is H, D, F, methyl, $CD_3$ or $CF_3$; in another specific embodiment, n is 1, and $R_t$ is H, D, F, methyl or $CD_3$; in another specific embodiment, n is 1, and $R_t$ is H, D or F; in another specific embodiment, n is 1, and $R_t$ is methyl or $CD_3$.

R

In one embodiment, each R is independently

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, - $NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, - $P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $-C_{1-6}$ alkylene-$C_{3-6}$ cycloalkyl, $-C_{1-6}$ alkylene-3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R".

In another embodiment, each R is independently

1) H, D, halogen, $-C(O)R_d$, $-C(O)OR_d$, $-C(O)NR_eR_f$, $-NR_dC(O)R_e$, $-OR_d$, $-S(O)_2R_d$ or $-P(=O)R_eR_f$; or
2) $C_{1-6}$ alkyl, $C_{1-6}$ haloalkyl, $C_{3-6}$ cycloalkyl, or 3- to 7-membered heterocyclyl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R";

In another embodiment, each R is independently

1) H, D, halogen, $-C(O)R_d$, $-C(O)OR_d$, $-C(O)NR_eR_f$, $-NR_dC(O)R_e$, $-OR_d$, $-S(O)_2R_d$ or $-P(=O)R_eR_f$; or
2) $C_{1-6}$ alkyl, $C_{1-6}$ haloalkyl, $C_{3-6}$ cycloalkyl or 3- to 7-membered heterocyclyl, wherein the groups are optionally substituted with one or more R"; or
3) two R on two adjacent atoms are taken together with the atoms to which they are attached to form $C_{3-6}$ cycloalkyl, 3- to 7-membered monocyclic heterocyclyl, 6- to 12-membered fused bicyclic heterocycle, 7-to 10-membered spiro heterocycle, $C_{6-10}$ aryl or 5- to 10-membered heteroaryl, wherein the groups are optionally substituted with one or more R".

In another embodiment, each R is independently

1) H, D, halogen, $-C(O)R_d$, $-C(O)OR_d$, $-C(O)NR_eR_f$, $-NR_dC(O)R_e$, $-OR_d$, $-S(O)_2R_d$; or
2) $C_{1-6}$ alkyl, $C_{1-6}$ haloalkyl, $C_{3-6}$ cycloalkyl or 3- to 7-membered heterocyclyl, wherein the groups are optionally substituted with one or more R"; or
3) two R on two adjacent atoms are taken together with the atoms to which they are attached to form $C_{3-6}$ cycloalkyl, 3- to 7-membered monocyclic heterocyclyl, 6- to 12-membered fused bicyclic heterocycle, $C_{6-10}$ aryl or 5- to 10-membered heteroaryl, wherein the groups are optionally substituted with one or more R".

In another embodiment, each R is independently

1) H, D, halogen, $-C(O)R_d$, $-C(O)OR_d$, $-C(O)NR_eR_f$, $-NR_dC(O)R_e$, $-OR_d$, $-S(O)_2R_d$; or
2) $C_{1-6}$ alkyl, $C_{1-6}$ haloalkyl, $C_{3-6}$ cycloalkyl or 3- to 7-membered heterocyclyl, wherein the groups are optionally substituted with one or more R"; or
3) two R on two adjacent atoms are taken together with the atoms to which they are attached to form 3- to 7-membered monocyclic heterocyclyl, 6- to 12-membered fused bicyclic heterocycle or 5- to 10-membered heteroaryl, wherein the

groups are optionally substituted with one or more R".

In another embodiment, each R is independently

1) H, D or halogen; or
2) $C_{1-6}$ alkyl, $C_{1-6}$ haloalkyl, $C_{4-6}$ cycloalkyl or 5- to 6-membered heterocyclyl, wherein the groups are optionally substituted with one or more R"; or
3) two R on two adjacent atoms are taken together with the atoms to which they are attached to form 5- to 10-membered heterocyclyl or 5- to 6-membered heteroaryl, wherein the groups are optionally substituted with one or more R".

In another embodiment, each R is independently

1) H, D or halogen; or
2) $C_{1-6}$ alkyl, $C_{1-6}$ haloalkyl, $C_{4-6}$ cycloalkyl or 5- to 6-membered heterocyclyl, wherein the groups are optionally substituted with one or more R"; or
3) two R on two adjacent atoms are taken together with the atoms to which they are attached to form 5- to 6-membered heterocyclyl or 5- to 6-membered heteroaryl, wherein the groups are optionally substituted with one or more R".

In another embodiment, each R is independently

1) H, D or halogen; or
2) $C_{1-6}$ alkyl, $C_{1-6}$ haloalkyl or 5- to 6-membered heterocyclyl, wherein the groups are optionally substituted with one or more R"; or
3) two R on two adjacent atoms are taken together with the atoms to which they are attached to form 5- to 6-membered heterocyclyl or 5- to 6-membered heteroaryl, wherein the groups are optionally substituted with one or more R".

In another embodiment, each R is independently

1) H, D or halogen; or
2) $C_{1-6}$ alkyl or $C_{1-6}$ haloalkyl, wherein the groups are optionally substituted with one or more R"; or
3) two R on two adjacent atoms are taken together with the atoms to which they are attached to form 5- to 6-membered heterocyclyl or 5- to 6-membered heteroaryl, wherein the groups are optionally substituted with one or more R".

In another embodiment, each R is independently

1) H or D; or
2) Me, $CHF_2$, $CH_2CF_3$ or piperidyl, wherein the groups are optionally substituted with one or more R"; or
3) two R on two adjacent atoms are taken together with the atoms to which they are attached to form 5- to 6-membered heterocyclyl or 5- to 6-membered heteroaryl, wherein the groups are optionally substituted with one or more R".

R'

In one embodiment, each R' is independently H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R".

In another embodiment, each R' is independently H, D, halogen, oxo, $-C(O)R_d$, $-C(O)OR_d$, $-C(O)NR_eR_f$, $C_{1-6}$ alkyl, $C_{1-6}$ haloalkyl, $C_{3-6}$ cycloalkyl or 3- to 7-membered heterocyclyl, or two R' on the same atom or on two adjacent atoms are taken together with the atom(s) to which they are attached to form $C_{3-6}$ cycloalkyl or 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R".

In another embodiment, each R' is independently H, D, halogen, oxo, $-C(O)R_d$, $-C(O)OR_d$ or $-C(O)NR_eR_f$.

In another embodiment, each R' is independently H, D or halogen.

In another embodiment, each R' is independently H or D.

$R_a$, $R_b$ and $R_c$

In one embodiment, each of $R_a$, $R_b$ and $R_c$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R.

In another embodiment, each of $R_a$, $R_b$ and $R_c$ is independently H, $C_{1-6}$ alkyl, $C_{1-6}$ haloalkyl or $C_{3-6}$ cycloalkyl, wherein the $C_{1-6}$ alkyl, $C_{1-6}$ haloalkyl and $C_{3-6}$ cycloalkyl are optionally substituted with one or more R.

In another embodiment, each of $R_a$, $R_b$ and $R_c$ is independently H or $C_{3-6}$ cycloalkyl, wherein the $C_{3-6}$ cycloalkyl is optionally substituted with one or more R.

$R_d$, $R_e$ and $R_f$

In one embodiment, each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R".

In another embodiment, each of $R_d$, $R_e$ and $R_f$ is independently H, $C_{1-6}$ alkyl, $C_{3-6}$ cycloalkyl, 3- to 7-membered heterocyclyl, $C_{2-6}$ alkenyl or $C_{6-10}$ aryl, or $R_e$, $R_f$ and the atom to which they are attached are taken together to form 3- to 7-membered heterocyclyl; wherein the $C_{1-6}$ alkyl, $C_{3-6}$ cycloalkyl, 3- to 7-membered heterocyclyl, $C_{2-6}$ alkenyl and $C_{6-10}$ aryl are optionally substituted with one or more R".

In another embodiment, each of $R_d$, $R_e$ and $R_f$ is independently H, $C_{1-6}$ alkyl, $C_{3-6}$ cycloalkyl or 3-to 7-membered heterocyclyl, or $R_e$, $R_f$ and the atom to which they are attached are taken together to form 3-to 7-membered heterocyclyl; wherein the $C_{1-6}$ alkyl, $C_{3-6}$ cycloalkyl and 3- to 7-membered heterocyclyl are optionally substituted with one or more R".

R"

In one embodiment, each R" is independently H, D, halogen, oxo, -C(O)$R_g$, -C(O)O$R_g$, -C(O)N$R_h$$R_j$, -N$R_h$$R_j$, -N$R_g$C(O)$R_h$, -N$R_g$C(O)O$R_h$, -N$R_g$C(O)N$R_h$$R_j$, -O$R_g$, -OC(O)$R_g$, -OC(O)N$R_h$$R_j$, -N$R_g$S(O)$_2$$R_h$, - S(O)$_2$N$R_h$$R_j$, -S(O)$R_g$, -S(O)$_2$$R_g$, -P(=O)$R_h$$R_j$, -OP(=O)$R_h$$R_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In another embodiment, each R" is independently H, D, -C(O)O$R_g$, -C(O)N$R_h$$R_j$, -N$R_h$$R_j$, - N$R_g$C(O)$R_h$, -O$R_g$, $C_{1-6}$ alkyl or 3- to 7-membered heterocyclyl, or two R" on the same atom or on two adjacent atoms are taken together with the atom(s) to which they are attached to form $C_{3-6}$ cycloalkyl or 3- to 7-membered heterocyclyl; wherein the $C_{1-6}$ alkyl and 3- to 7-membered heterocyclyl are optionally substituted with one or more D, up to fully deuterated.

In another embodiment, R" is substituted with one or more R‴.

R‴

In one embodiment, each R‴ is independently H; in one embodiment, each R"' is independently D; in one embodiment, each R"' is independently halogen; in one embodiment, each R‴ is independently $C_{1-6}$ alkyl; in one embodiment, each R‴ is independently $C_{1-6}$ haloalkyl; in one embodiment, each R‴ is independently $C_{3-6}$ cycloalkyl; in one embodiment, each R‴ is independently 3- to 7-membered heterocyclyl; in one embodiment, each R‴ is independently $C_{6-10}$ aryl; in one embodiment, each R‴ is independently substituted with 5- to 10-membered heteroaryl; in one embodiment, each R‴ is substituted with one or more D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl.

$R_g$, $R_h$ and $R_j$

In one embodiment, H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl,

$C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In another embodiment, each of $R_g$, $R_h$ and $R_j$ is independently H, D, $C_{1-6}$ alkyl or $C_{1-6}$ haloalkyl, or $R_h$, $R_j$ and the atom to which they are attached are taken together to form 3- to 7-membered heterocyclyl; wherein the $C_{1-6}$ alkyl, $C_{1-6}$ haloalkyl and 3- to 7-membered heterocyclyl are optionally substituted with one or more D, up to fully deuterated.

In another embodiment, each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

Any technical solution in any one of the above specific embodiments, or any combination thereof, may be combined with any technical solution in other specific embodiments or any combination thereof. For example, any technical solution of $X_1$, $X_2$ and $R_2$, $R_1$, $R_3$ and $R_4$, $R_5$ and $R_6$, $R_s$ and m, $R_t$ and n, R, R', R", $R_d$, $R_e$ and $R_f$, $R_g$, $R_h$ and $R_j$, or any combination thereof may be combined. The present disclosure is intended to include all combination of such technical solutions, which are not exhaustively listed here to save space.

In one more specific embodiment, the present disclosure relates to a compound of formula (A), or a tautomer, a stereoisomer, a prodrug, a crystal form, a pharmaceutically acceptable salt, a hydrate or a solvate thereof:

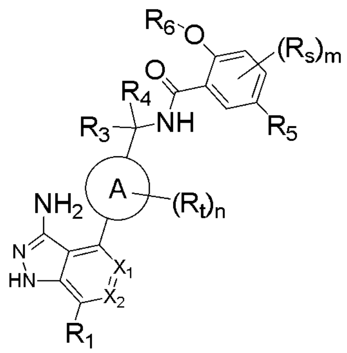

(A)

wherein,

- ring A is a benzene ring or a 5- to 6-membered heteroaromatic ring;
- $X_1$ is N, CD or CH;
- $X_2$ is N or $CR_2$;
- $R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R;
- wherein each of $R_a$, $R_b$ and $R_c$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;
- $R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R';
- $R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
- $R_5$ is halogen;
- $R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
- $R_s$ and $R_t$ are independently H, D, halogen, $-OR_a$, $-NR_bR_c$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
- m is 0, 1, 2 or 3;
- n is 0, 1, 2, 3 or 4;
- each R is independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $-C_{1-6}$ alkylene-$C_{3-6}$ cycloalkyl, $-C_{1-6}$ alkylene-3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R";

each R' is independently H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR^dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R‴;
each R‴ is independently H, D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl is optionally substituted with one or more D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl;
wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (I), or a tautomer, a stereoisomer, a prodrug, a crystal form, a pharmaceutically acceptable salt, a hydrate or a solvate thereof:

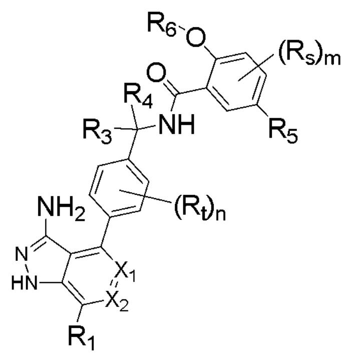

(I)

wherein,

$X_1$ is N, CD or CH;
$X_2$ is N or $CR_2$;

$R_1$ is H, D, halogen, -C(O)$R_a$, -C(O)O$R_a$, -C(O)N$R_bR_c$, -N$R_bR_c$, -N$R_a$C(O)$R_b$, -N$R_a$C(O)O$R_b$, - N$R_a$C(O)N$R_bR_c$, -O$R_a$, -OC(O)$R_a$, -OC(O)N$R_bR_c$, -N$R_a$S(O)$_2R_b$, -S(O)$_2$N$R_bR_c$, -S(O)$R_a$, -S(O)$_2R_a$, - P(=O)$R_bR_c$, -OP(=O)$R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R;
wherein each of $R_a$, $R_b$ and $R_c$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;
$R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R';
$R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_s$ and $R_t$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
m is 0, 1, 2 or 3;
n is 0, 1, 2, 3 or 4;
each R is independently:

1) H, D, halogen, oxo, -C(O)$R_d$, -C(O)O$R_d$, -C(O)N$R_eR_f$, -N$R_eR_f$, -N$R_d$C(O)$R_e$, -N$R_d$C(O)O$R_e$, - N$R_d$C(O)N$R_eR_f$, -O$R_d$, -OC(O)$R_d$, -OC(O)N$R_eR_f$, -N$R_d$S(O)$_2R_e$, -S(O)$_2$N$R_eR_f$, -S(O)$R_d$, -S(O)$_2R_d$, - P(=O)$R_eR_f$, -OP(=O)$R_eR_f$, or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R";

each R' is independently H, D, halogen, oxo, -C(O)$R_d$, -C(O)O$R_d$, -C(O)N$R_eR_f$, -N$R_eR_f$, - N$R_d$C(O)$R_e$, -N$R_4$C(O)O$R_e$, -N$R_d$C(O)N$R_eR_f$, -O$R_d$, -OC(O)$R_d$, -OC(O)N$R_eR_f$, -N$R_d$S(O)$_2R_e$, -S(O)$_2$N$R_eR_f$, - S(O)$R_d$, -S(O)$_2R_d$, -P(=O)$R_eR_f$, -OP(=O)$R_eR_f$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, -C(O)$R_g$, -C(O)O$R_g$, -C(O)N$R_hR_j$, -N$R_hR_j$, - N$R_g$C(O)$R_h$, -N$R_g$C(O)O$R_h$, -N$R_g$C(O)N$R_hR_j$, -O$R_g$, -OC(O)$R_g$, -OC(O)N$R_hR_j$, -N$R_g$S(O)$_2R_h$, -S(O)$_2$N$R_hR_j$, -S(O)$R_g$, -S(O)$_2R_g$, -P(=O)$R_hR_j$, -OP(=O)$R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (I) as mentioned above, or a tautomer, a stereoisomer, a prodrug, a crystal form, a pharmaceutically acceptable salt, a hydrate or a solvate thereof, wherein $R_1$ is H, D, $C_{6-10}$ aryl or 5- to 10-membered heteroaryl, wherein the $C_{6-10}$ aryl and 5- to 10-membered heteroaryl are optionally substituted with one or more R;

alternatively, $R_1$ is H, D,

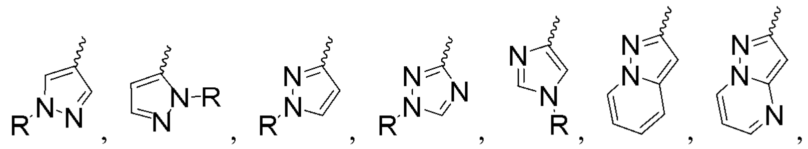

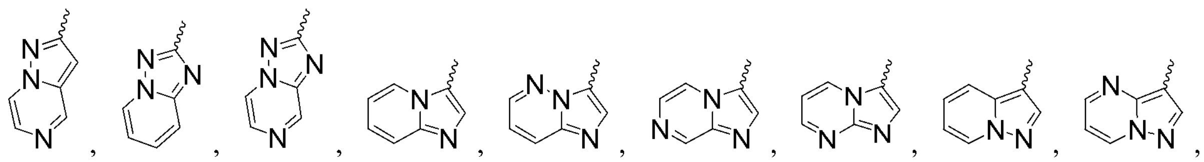

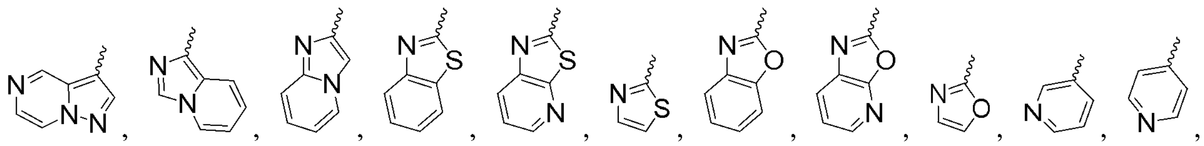

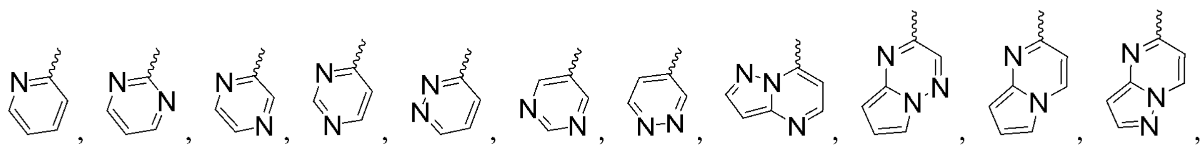

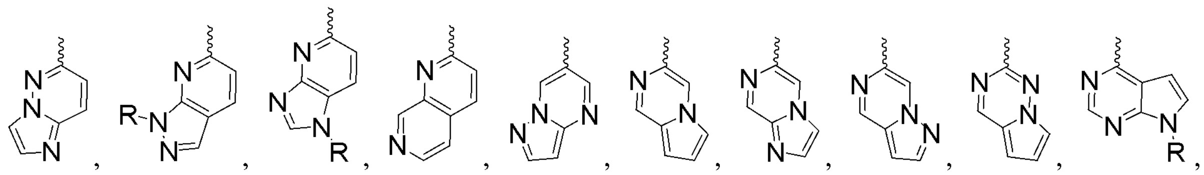

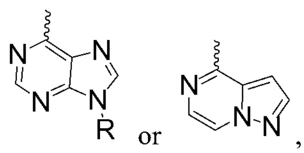

wherein the above groups are optionally substituted with one or more R;
alternatively, $R_1$ is

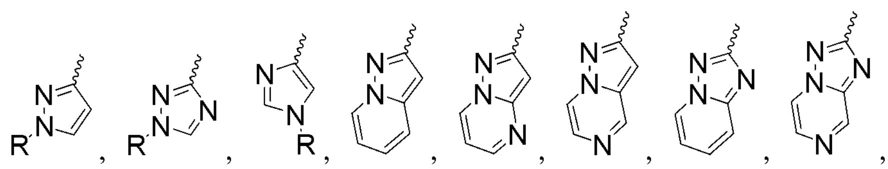

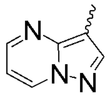, 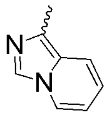, 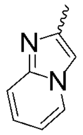, 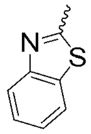, 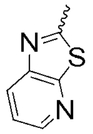, 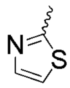, 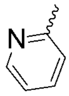, 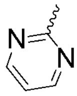, 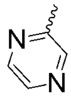, 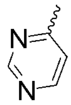, 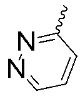,

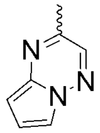, 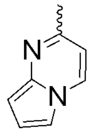, 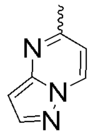, 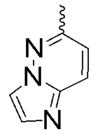, 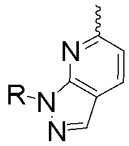, 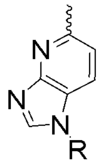, 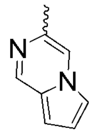, 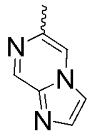, 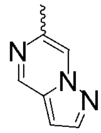, 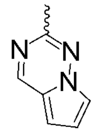,

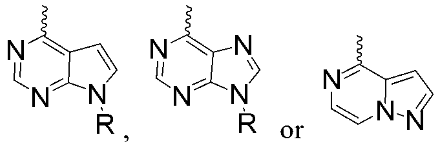
, , or ,

wherein the above groups are optionally substituted with one or more R;
alternatively, $R_1$ is

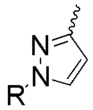, 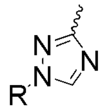, 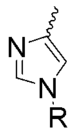, 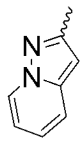, 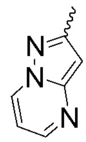, 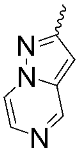, 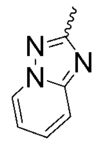, 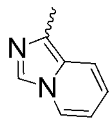,

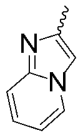, 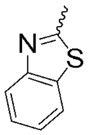, 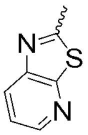, 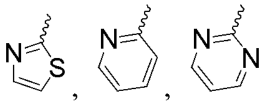
, , ,
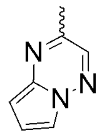, 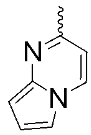, 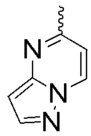, 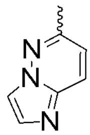, 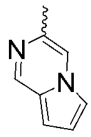 or

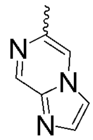,

wherein the above groups are optionally substituted with one or more R;
alternatively, $R_1$ is H.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein $R_2$ is H, D, halogen, $C_{1-6}$ alkyl, $C_{1-6}$ haloalkyl, $C_{3-6}$ cycloalkyl, 3- to 7-membered monocyclic heterocyclyl, 6- to 12-membered fused bicyclic heterocycle, 7- to 10-membered spiro heterocycle, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 7-membered monocyclic heterocyclyl, 6- to 12-membered fused bicyclic heterocycle, 7- to 10-membered spiro heterocycle, $C_{6-10}$ aryl and 5- to 10-membered heteroaryl are optionally substituted with one or more R';

alternatively, $R_2$ is H, D, halogen, methyl, $-CHF_2$, ethyl, isopropyl,

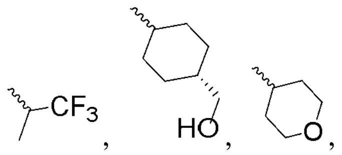
, , ,

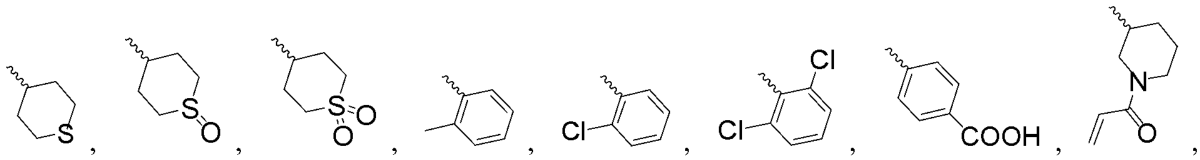
, , , , , , , ,

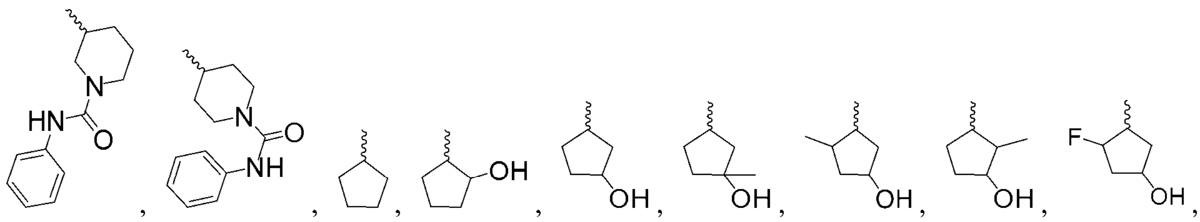
, , , , , , , , ,

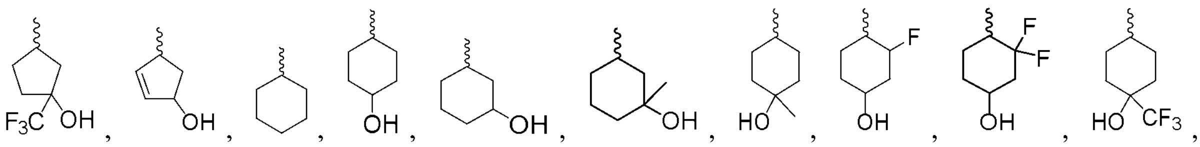
, , , , , , , , , ,

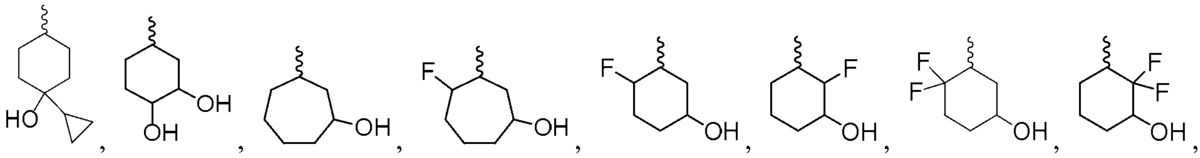
, , , , , , , ,

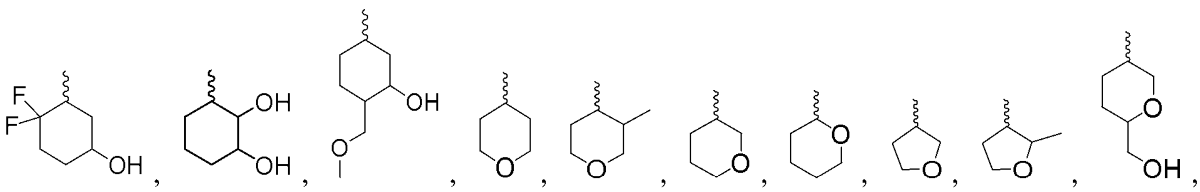
, , , , , , , , , , ,

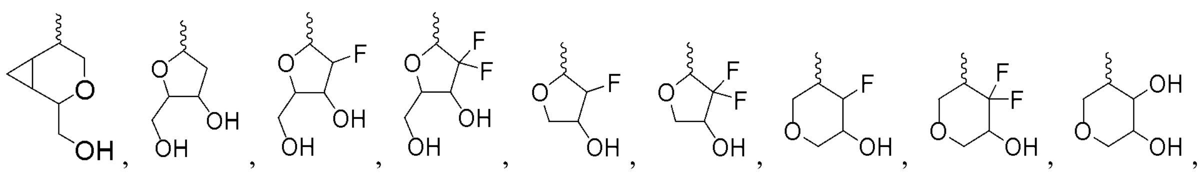
, , , , , , , , ,

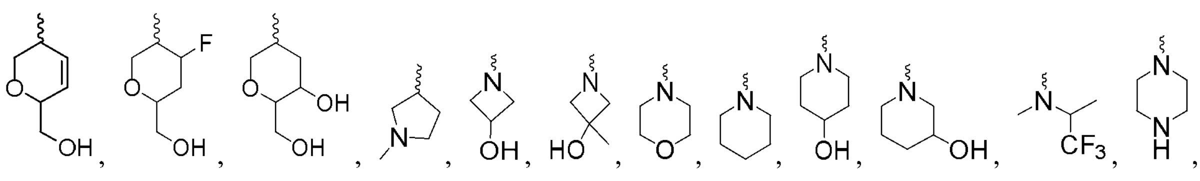
, , , , , , , , , , , , ,

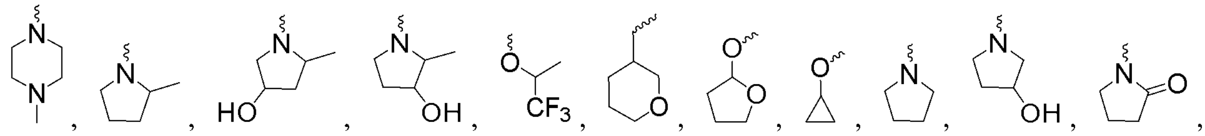

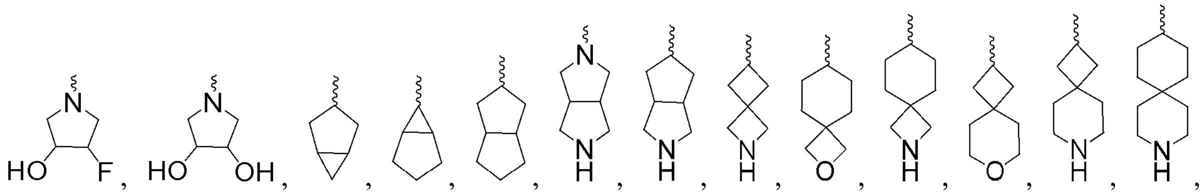

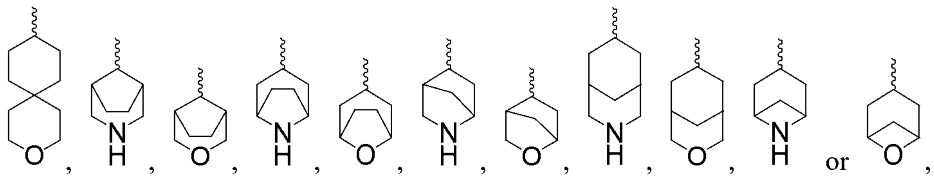

wherein the above groups are optionally substituted with one or more D, up to fully deuterated;

alternatively, $R_2$ is H, D, halogen, methyl, $-CHF_2$, ethyl, isopropyl,

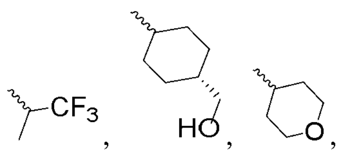

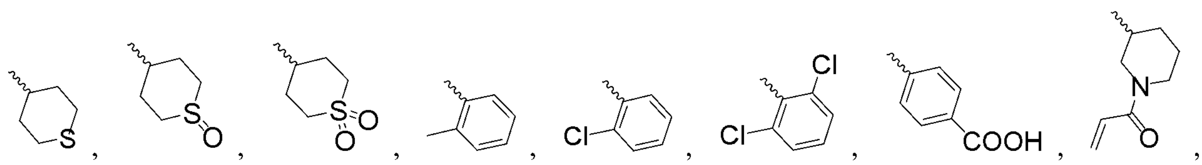

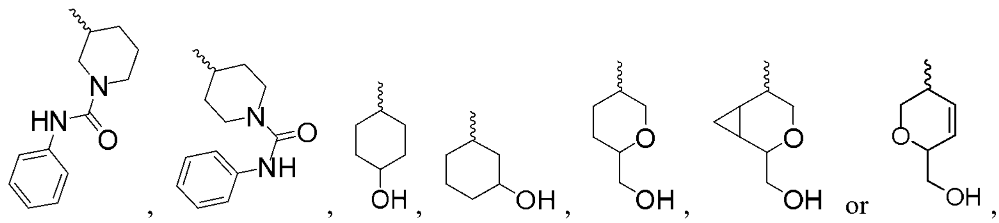

wherein the above groups are optionally substituted with one or more D, up to fully deuterated;

alternatively, $R_2$ is H, D, methyl, $-CHF_2$,

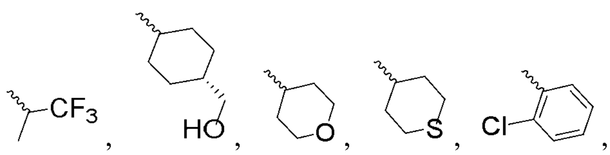

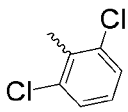, 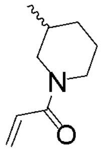, 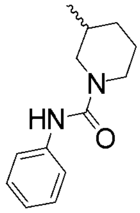, 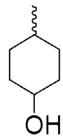, 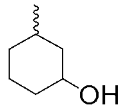, 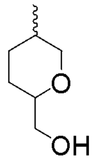, 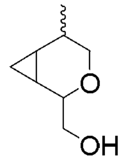 or 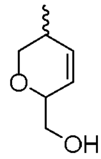,

wherein the above groups are optionally substituted with one or more D, up to fully deuterated;

alternatively, $R_2$ is H, D, $-CHF_2$ or

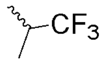,

wherein the above groups are optionally substituted with one or more D, up to fully deuterated;

alternatively, $R_2$ is H or D;

alternatively, $R_2$ is $-CHF_2$ or

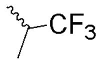,

wherein the above groups are optionally substituted with one or more D, up to fully deuterated;

alternatively, $R_2$ is

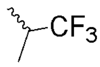

, wherein the above group is optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein $R_5$ is F.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein $R_6$ is methyl or $-CD_3$.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (II):

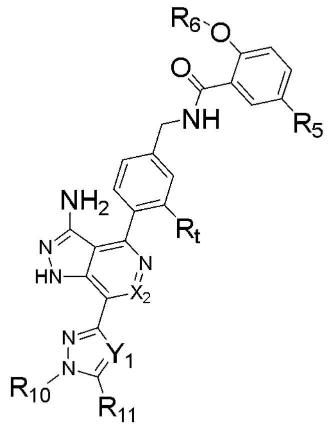

(II)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_1$ is N or $CR_{12}$;
$R_{10}$, $R_{11}$ and $R_{12}$ are independently:

1) H, D, halogen, oxo, -C(O)$R_d$, -C(O)O$R_d$, -C(O)N$R_eR_f$, -N$R_eR_f$, -N$R_d$C(O)$R_e$, -N$R_d$C(O)O$R_e$, - N$R_d$C(O)N$R_eR_f$, -O$R_d$, -OC(O)$R_d$, -OC(O)N$R_eR_f$, -N$R_d$S(O)$_2R_e$, -S(O)$_2$N$R_eR_f$, -S(O)$R_d$, -S(O)$_2R_d$, - P(=O)$R_eR_f$, -OP(=O)$R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{11}$, $R_{12}$ and the atom to which they are attached are taken together to form $C_{3-6}$ cycloalkyl, which is optionally substituted with one or more R"; or
4) $R_{10}$ and $R_{11}$ or $R_{11}$ and $R_{12}$ and the atom to which they are attached are taken together to form 3-to 12-membered heterocyclyl or 5- to 10-membered heteroaryl, wherein the groups are optionally substituted with one or more R"; or
5) $R_{11}$, $R_{12}$ and the atom to which they are attached are taken together to form $C_{6-10}$ aryl, which is optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, -C(O)$R_g$, -C(O)O$R_g$, -C(O)N$R_hR_j$, -N$R_hR_j$, - N$R_g$C(O)$R_h$, -N$R_g$C(O)O$R_h$, -N$R_g$C(O)N$R_hR_j$, -O$R_g$, -OC(O)$R_g$, -OC(O)N$R_hR_j$, -N$R_g$S(O)$_2R_h$, -S(O)$_2$N$R_hR_j$, -S(O)$R_g$, -S(O)$_2R_g$, -P(=O)$R_hR_j$, -OP(=O)$R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (II) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_1$ is N or $CR_{12}$;
$R_{10}$ is H, $C_{1-6}$ alkyl, $C_{1-6}$ haloalkyl, $C_{3-6}$ cycloalkyl, or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more R";
$R_{11}$ and $R_{12}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{11}$, $R_{12}$ and the atom to which they are attached are taken together to form $C_{3-6}$ cycloalkyl, which is optionally substituted with one or more R"; or
$R_{10}$ and $R_{11}$ or $R_{11}$ and $R_{12}$ and the atom to which they are attached are taken together to form 3- to 7-membered monocyclic heterocyclyl, 6- to 12-membered fused bicyclic heterocycle or 5- to 10-membered heteroaryl, wherein the groups are optionally substituted with one or more R"; or
$R_{11}$, $R_{12}$ and the atom to which they are attached are taken together to form $C_{6-10}$ aryl, which is optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl, or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (II) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is halogen; alternatively, $R_5$ is F;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_6$ is methyl or $-CD_3$;
$R_t$ is H, D or halogen; alternatively, $R_t$ is H, D or F;
$Y_1$ is N or $CR_{12}$;
$R_{10}$ is H, $C_{1-6}$ alkyl or $C_{1-6}$ haloalkyl, wherein the $C_{1-6}$ alkyl and $C_{1-6}$ haloalkyl are optionally substituted with one or more R"; alternatively, $R_{10}$ is H, $C_{1-3}$ alkyl or $C_{1-3}$ haloalkyl, wherein the $C_{1-3}$ alkyl and $C_{1-3}$ haloalkyl are optionally substituted with one or more D, up to fully deuterated;
$R_{11}$ and $R_{12}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{11}$ and $R_{12}$ are independently H or D;
or, $R_{11}$, $R_{12}$ and the atom to which they are attached are taken together to form $C_{3-6}$ cycloalkyl, which is optionally substituted with one or more R"; alternatively, $R_{11}$, $R_{12}$ and the atom to which they are attached are taken together to form cyclohexyl, which is optionally substituted with one or more R"; or
$R_{10}$ and $R_{11}$ or $R_{11}$ and $R_{12}$ and the atom to which they are attached are taken together to form 3- to 7-membered monocyclic heterocyclyl, which is optionally substituted with one or more R"; alternatively, $R_{10}$ and $R_{11}$ or $R_{11}$ and $R_{12}$ and the atom to which they are attached are taken together to form 6-membered heterocyclyl having 1 or 2 ring heteroatoms N, which is optionally substituted with one or more R"; or
$R_{10}$ and $R_{11}$ or $R_{11}$ and $R_{12}$ and the atom to which they are attached are taken together to form 6- to 12-membered fused bicyclic heterocycle, which is optionally substituted with one or more R"; alternatively, $R_{10}$ and $R_{11}$ or $R_{11}$ and $R_{12}$ and the atom to which they are attached are taken together to form 8- to 10-membered fused bicyclic heterocycle having 1 or 2 ring heteroatoms N, which is optionally substituted with one or more R"; or
$R_{11}$, $R_{12}$ and the atom to which they are attached are taken together to form $C_{6-10}$ aryl, which is optionally substituted

with one or more R"; alternatively, $R_{11}$, $R_{12}$ and the atom to which they are attached are taken together to form phenyl, which is optionally substituted with one or more R"; or

$R_{10}$ and $R_{11}$ or $R_{11}$ and $R_{12}$ and the atom to which they are attached are taken together to form 5- to 10-membered heteroaryl, which is optionally substituted with one or more R"; alternatively, $R_{10}$ and $R_{11}$ or $R_{11}$ and $R_{12}$ and the atom to which they are attached are taken together to form 5-membered or 6-membered heteroaryl having 1 or 2 ring heteroatoms N, wherein the group is optionally substituted with one or more R";

each R" is independently D, -C(O)OR$_g$, -C(O)NR$_h$R$_j$, -NR$_g$C(O)R$_h$, $C_{1-6}$ alkyl, $C_{1-6}$ haloalkyl, $C_{3-6}$ cycloalkyl, or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (II-1):

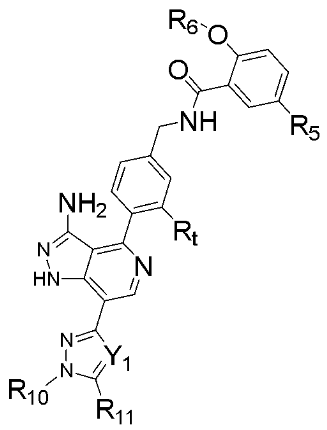

(II-1)

wherein,

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

$R_t$ is H, D or halogen; alternatively, $R_t$ is H, D or F;

$Y_1$ is N, CH or CD;

$R_{10}$, $R_{11}$ and the atom to which they are attached are taken together to form 3- to 7-membered monocyclic heterocyclyl, which is optionally substituted with one or more R"; alternatively, $R_{10}$, $R_{11}$ and the atom to which they are attached are taken together to form 6-membered heterocyclyl having 1 or 2 ring heteroatoms N, which is optionally substituted with one or more R"; or

$R_{10}$, $R_{11}$ and the atom to which they are attached are taken together to form 6- to 12-membered fused bicyclic heterocycle, which is optionally substituted with one or more R"; alternatively, $R_{10}$, $R_{11}$ and the atom to which they are attached are taken together to form 8- to 10-membered fused bicyclic heterocycle having 1 or 2 ring heteroatoms N, which is optionally substituted with one or more R"; or

$R_{10}$, $R_{11}$ and the atom to which they are attached are taken together to form 5- to 10-membered heteroaryl, which is optionally substituted with one or more R"; alternatively, $R_{10}$, $R_{11}$ and the atom to which they are attached are taken together to form 6-membered heteroaryl having 1 or 2 ring heteroatoms N, which is optionally substituted with one or more R";

each R" is independently D, -C(O)OR$_g$, -C(O)NR$_h$R$_j$, -NR$_g$C(O)R$_h$, $C_{1-6}$ alkyl, $C_{1-6}$ haloalkyl, $C_{3-6}$ cycloalkyl, or 3- to 7-membered heterocyclyl, wherein the $C_{1-6}$ alkyl, $C_{1-6}$ haloalkyl, $C_{3-6}$ cycloalkyl, 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$

haloalkyl are optionally substituted with one or more D, up to fully deuterated.

In one more specific embodiment, the present disclosure relates to a compound of formula (II-1) above, or a tautomer, a stereoisomer, a prodrug, a crystal form, a pharmaceutically acceptable salt, a hydrate or a solvate thereof, wherein,

$R_5$ is F;
$R_6$ is methyl or $-CD_3$;
Rt is H, D or F;
$Y_1$ is N, CH or CD;
$R_{10}$, $R_{11}$ and the atom to which they are attached are taken together to form the following groups:

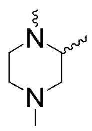 , 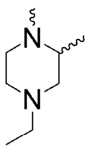 , 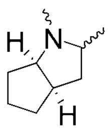 , 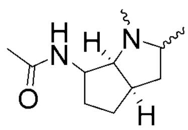 , 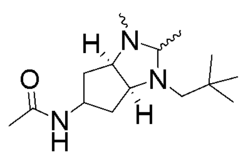 , 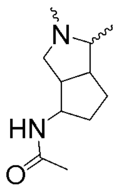 , 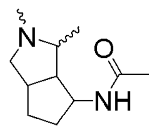 ,

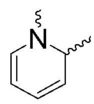 , 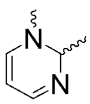 , 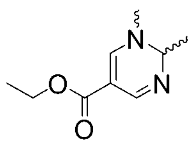 or 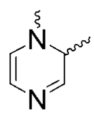 ,

wherein the groups are optionally substituted with one or more D, up to fully deuterated;
alternatively, $R_{10}$, $R_{11}$ and the atom to which they are attached are taken together to form the following groups:

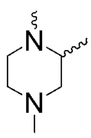 , 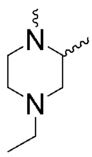 , 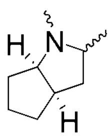 , 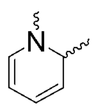 , 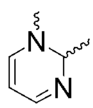 or 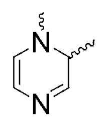

, wherein the groups are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (III):

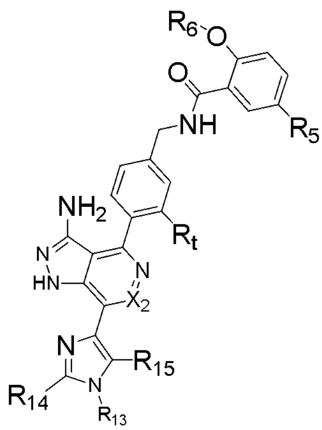

(III)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{13}$, $R_{14}$ and $R_{15}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{13}$ and $R_{14}$ or $R_{13}$ and $R_{15}$ and the atom to which they are attached are taken together to form 3-to 12-membered heterocyclyl or 5- to 10-membered heteroaryl, wherein the groups are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (III) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{13}$ is H, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more R";
$R_{14}$ and $R_{15}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{13}$ and $R_{14}$ or $R_{13}$ and $R_{15}$ and the atom to which they are attached are taken together to form 3-to 7-membered monocyclic heterocyclyl, 6- to 12-membered fused bicyclic heterocycle or 5- to 10-membered heteroaryl, wherein the groups are optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are

optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (III) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is halogen; alternatively, $R_2$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
$R_t$ is H, D or halogen; alternatively, $R_t$ is H, D or F;
$R_{13}$ is H, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{13}$ is H, $C_{1-3}$ alkyl, $C_{1-3}$ haloalkyl or 6-membered heterocyclyl having 1 or 2 ring heteroatoms N, wherein the $C_{1-3}$ alkyl, $C_{1-3}$ haloalkyl and 6-membered heterocyclyl are optionally substituted with one or more R";
$R_{14}$ and $R_{15}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{14}$ and $R_{15}$ are independently H or D; or, $R_{13}$ and $R_{14}$ or $R_{13}$ and $R_{15}$ and the atom to which they are attached are taken together to form 3-to 7-membered monocyclic heterocyclyl, which is optionally substituted with one or more R"; alternatively, $R_{13}$ and $R_{14}$ or $R_{13}$ and $R_{15}$ and the atom to which they are attached are taken together to form 6-membered heterocyclyl having 1 or 2 ring heteroatoms N, which is optionally substituted with one or more R"; or
$R_{13}$ and $R_{14}$ or $R_{13}$ and $R_{15}$ and the atom to which they are attached are taken together to form 6- to 12-membered fused bicyclic heterocycle, which is optionally substituted with one or more R"; alternatively, $R_{13}$ and $R_{14}$ or $R_{13}$ and $R_{15}$ and the atom to which they are attached are taken together to form 8- to 10-membered fused bicyclic heterocycle having 1 or 2 ring heteroatoms N, which is optionally substituted with one or more R"; or
$R_{13}$ and $R_{14}$ or $R_{13}$ and $R_{15}$ and the atom to which they are attached are taken together to form 5- to 10-membered heteroaryl, which is optionally substituted with one or more R"; alternatively, $R_{13}$ and $R_{14}$ or $R_{13}$ and $R_{15}$ and the atom to which they are attached are taken together to form 6-membered heteroaryl having 1 or 2 ring heteroatoms N, which is optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (III-1):

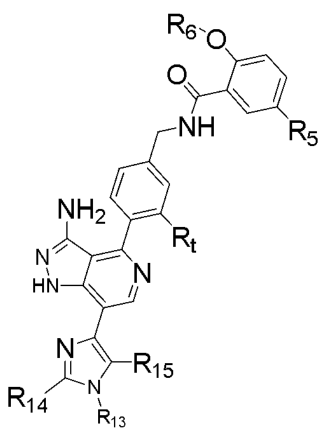

(III-1)

wherein,

$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
$R_t$ is H, D or halogen; alternatively, $R_t$ is H, D or F;
$R_{13}$ and $R_{14}$ or $R_{13}$ and $R_{15}$ and the atom to which they are attached are taken together to form 3- to 7-membered monocyclic heterocyclyl, which is optionally substituted with one or more R"; alternatively, $R_{13}$ and $R_{14}$ or $R_{13}$ and $R_{15}$ and the atom to which they are attached are taken together to form 6-membered heterocyclyl having 1 or 2 ring heteroatoms N, which is optionally substituted with one or more R"; or
$R_{13}$ and $R_{14}$ or $R_{13}$ and $R_{15}$ and the atom to which they are attached are taken together to form 6- to 12-membered fused bicyclic heterocycle, which is optionally substituted with one or more R"; alternatively, $R_{13}$ and $R_{14}$ or $R_{13}$ and $R_{15}$ and the atom to which they are attached are taken together to form 8- to 10-membered fused bicyclic heterocycle having 1 or 2 ring heteroatoms N, which is optionally substituted with one or more R"; or
$R_{13}$ and $R_{14}$ or $R_{13}$ and $R_{15}$ and the atom to which they are attached are taken together to form 5- to 10-membered heteroaryl, which is optionally substituted with one or more R"; alternatively, $R_{13}$ and $R_{14}$ or $R_{13}$ and $R_{15}$ and the atom to which they are attached are taken together to form 6-membered heteroaryl having 1 or 2 ring heteroatoms N, which is optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (III-1) above, or a tautomer, a stereoisomer, a prodrug, a crystal form, a pharmaceutically acceptable salt, a hydrate or a solvate thereof, wherein,

$R_5$ is F;
$R_6$ is methyl or $-CD_3$;
Rt is H, D or F;
$R_{13}$ and $R_{14}$ or $R_{13}$ and $R_{15}$ and the atom to which they are attached are taken together to form

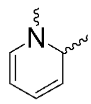

,

wherein the group is optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (IV):

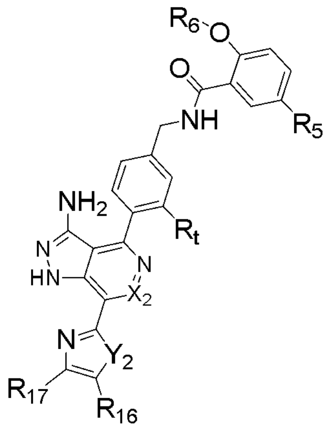

(IV)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_2$ is O or S;
$R_{16}$ and $R_{17}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{16}$, $R_{17}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (IV) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_2$ is O or S;
$R_{16}$ and $R_{17}$ are independently H, D, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more R";
or, $R_{16}$, $R_{17}$ and the atom to which they are attached are taken together to form $C_{3-6}$ cycloalkyl, which is optionally substituted with one or more R"; or
$R_{16}$, $R_{17}$ and the atom to which they are attached are taken together to form 3- to 7-membered monocyclic heterocyclyl, which is optionally substituted with one or more R"; or
$R_{16}$, $R_{17}$ and the atom to which they are attached are taken together to form 6- to 12-membered fused bicyclic heterocycle, which is optionally substituted with one or more R"; or
$R_{16}$, $R_{17}$ and the atom to which they are attached are taken together to form $C_{6-10}$ aryl, which is optionally substituted with one or more R"; or
$R_{16}$, $R_{17}$ and the atom to which they are attached are taken together to form 5- to 10-membered heteroaryl, which is optionally substituted with one or more R";
each R" is independently D, -C(O)OR$_g$, -C(O)NR$_h$R$_j$, -NR$_g$C(O)R$_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (IV) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or -$CD_3$;
$R_t$ is H, D or halogen; alternatively, $R_t$ is H, D or F;
$Y_2$ is O or S;
$R_{16}$ and $R_{17}$ are independently H, D, $C_{1-6}$ alkyl or $C_{1-6}$ haloalkyl, wherein the $C_{1-6}$ alkyl and $C_{1-6}$ haloalkyl are optionally substituted with one or more R"; alternatively, $R_{10}$ is H, D, $C_{1-3}$ alkyl or $C_{1-3}$ haloalkyl, wherein the $C_{1-3}$ alkyl and $C_{1-3}$ haloalkyl are optionally substituted with one or more D, up to fully deuterated;
or, $R_{16}$, $R_{17}$ and the atom to which they are attached are taken together to form $C_{3-6}$ cycloalkyl, which is optionally substituted with one or more R"; alternatively, $R_{16}$, $R_{17}$ and the atom to which they are attached are taken together to form cyclohexyl, which is optionally substituted with one or more R"; or
$R_{16}$, $R_{17}$ and the atom to which they are attached are taken together to form 3- to 7-membered monocyclic heterocyclyl, which is optionally substituted with one or more R"; alternatively, $R_{16}$, $R_{17}$ and the atom to which they are attached are taken together to form 6-membered heterocyclyl having 1 or 2 ring heteroatoms N, which is optionally substituted with one or more R"; or
$R_{16}$, $R_{17}$ and the atom to which they are attached are taken together to form 6- to 12-membered fused bicyclic heterocycle, which is optionally substituted with one or more R"; alternatively, $R_{16}$, $R_{17}$ and the atom to which they are attached are taken together to form 8- to 10-membered fused bicyclic heterocycle having 1 or 2 ring heteroatoms N, which is optionally substituted with one or more R"; or
$R_{16}$, $R_{17}$ and the atom to which they are attached are taken together to form $C_{6-10}$ aryl, which is optionally substituted with one or more R"; alternatively, $R_{16}$, $R_{17}$ and the atom to which they are attached are taken together to form phenyl, which is optionally substituted with one or more R"; or
$R_{16}$, $R_{17}$ and the atom to which they are attached are taken together to form 5- to 10-membered heteroaryl, which is optionally substituted with one or more R"; alternatively, $R_{16}$, $R_{17}$ and the atom to which they are attached are taken

together to form 6-membered heteroaryl having 1 or 2 ring heteroatoms N, which is optionally substituted with one or more R";

each R" is independently D, -C(O)OR$_g$, -C(O)NR$_h$R$_j$, -NR$_g$C(O)R$_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (IV-1):

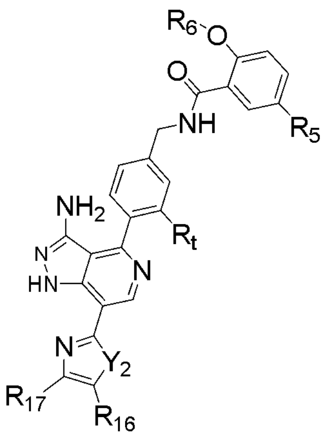

(IV-1)

wherein,

$R_5$ is halogen; alternatively, $R_5$ is F;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_6$ is methyl or $-CD_3$;

$R_t$ is H, D or halogen; alternatively, $R_t$ is H, D or F;

$Y_2$ is O or S;

$R_{16}$, $R_{17}$ and the atom to which they are attached are taken together to form $C_{3-6}$ cycloalkyl, which is optionally substituted with one or more R"; alternatively, $R_{16}$, $R_{17}$ and the atom to which they are attached are taken together to form cyclohexyl, which is optionally substituted with one or more R"; or

$R_{16}$, $R_{17}$ and the atom to which they are attached are taken together to form 3- to 7-membered monocyclic heterocyclyl, which is optionally substituted with one or more R"; alternatively, $R_{16}$, $R_{17}$ and the atom to which they are attached are taken together to form 6-membered heterocyclyl having 1 or 2 ring heteroatoms N, which is optionally substituted with one or more R"; or

$R_{16}$, $R_{17}$ and the atom to which they are attached are taken together to form 6- to 12-membered fused bicyclic heterocycle, which is optionally substituted with one or more R"; alternatively, $R_{16}$, $R_{17}$ and the atom to which they are attached are taken together to form 8- to 10-membered fused bicyclic heterocycle having 1 or 2 ring heteroatoms N, which is optionally substituted with one or more R"; or

$R_{16}$, $R_{17}$ and the atom to which they are attached are taken together to form $C_{6-10}$ aryl, which is optionally substituted with one or more R"; alternatively, $R_{16}$, $R_{17}$ and the atom to which they are attached are taken together to form phenyl, which is optionally substituted with one or more R"; or

$R_{16}$, $R_{17}$ and the atom to which they are attached are taken together to form 5- to 10-membered heteroaryl, which is optionally substituted with one or more R"; alternatively, $R_{16}$, $R_{17}$ and the atom to which they are attached are taken together to form 6-membered heteroaryl having 1 or 2 ring heteroatoms N, which is optionally substituted with one or more R";

each R" is independently D, -C(O)OR$_g$, -C(O)NR$_h$R$_j$, -NR$_g$C(O)R$_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully

deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (IV-1) above, or a tautomer, a stereoisomer, a prodrug, a crystal form, a pharmaceutically acceptable salt, a hydrate or a solvate thereof, wherein,

$R_5$ is F;
$R_6$ is methyl or $-CD_3$;
Rt is H, D or F;
$Y_2$ is O or S;
$R_{16}$, $R_{17}$ and the atom to which they are attached are taken together to form the following groups:

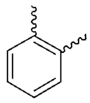 , 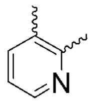 or 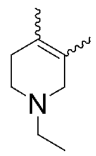 ,

wherein the groups are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (V):

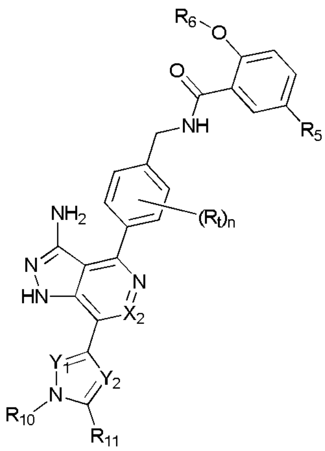
(V)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$ is H, $C_{1-6}$ alkyl, $C_{1-6}$ haloalkyl, $C_{4-6}$ cycloalkyl or 5- to 6-membered heterocyclyl, wherein the $C_{1-6}$ alkyl, $C_{1-6}$ haloalkyl, $C_{4-6}$ cycloalkyl and 5- to 6-membered heterocyclyl are optionally substituted with one or more R";
$R_{11}$, $R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl

are optionally substituted with one or more D, up to fully deuterated;

or, $R_{10}$, $R_{11}$ and the atom to which they are attached are taken together to form 5- to 10-membered heterocyclyl or 5- to 6-membered heteroaryl, wherein the 5- to 10-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";

wherein R" is H, D, halogen, oxo, $-C(O)R_g$, $-C(O)OR_g$, $-C(O)NR_hR_j$, $-NR_hR_j$, $-NR_gC(O)R_h$, $-OR_g$, $-OC(O)R_g$, $-NR_gS(O)_2R_h$, $-S(O)_2NR_hR_j$, $-S(O)R_g$, $-S(O)_2R_g$, $C_{1-6}$ alkyl, $C_{1-6}$ haloalkyl, $C_{4-6}$ cycloalkyl or 4- to 6-membered heterocyclyl, wherein the $C_{1-6}$ alkyl, $C_{1-6}$ haloalkyl, $C_{4-6}$ cycloalkyl and 4- to 6-membered heterocyclyl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

In one more specific embodiment, the present disclosure relates to a compound of formula (V) above, or a tautomer, a stereoisomer, a prodrug, a crystal form, a pharmaceutically acceptable salt, a hydrate or a solvate thereof, wherein,

$X_2$ is $CR_2$;
$R_2$ is H, D or $-CH(CH_3)(CF_3)$;
$R_5$ is F;
$R_6$ is Me;
$R_t$ is H or D;
n is 0, 1, 2, 3 or 4;
$Y_1$ is N or $CR_{12}$; alternatively, $Y_1$ is N;
$Y_2$ is N or $CR_{13}$;
$R_{10}$ is H, Me, $CHF_2$, $CH_2CF_3$ or piperidyl, which is optionally substituted with one or more R";
$R_{11}$ is H, D or Me;
$R_{12}$ and $R_{13}$ are independently H or D;
or, $R_{10}$, $R_{11}$ and the atom to which they are attached are taken together to form 5- to 10-membered heterocyclyl or 5- to 6-membered heteroaryl, wherein the 5- to 10-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is Me, Et, $-C(O)OR_g$, $-C(O)NR_hR_j$, $-NR_gC(O)R_h$ or oxetanyl;
each of $R_g$, $R_h$ and $R_j$ is independently H, D, $C_{1-6}$ alkyl or $C_{1-6}$ haloalkyl.

In one more specific embodiment, the present disclosure relates to a compound of formula (V) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$ is H, $C_{1-6}$ alkyl, $C_{1-6}$ haloalkyl, $C_{4-6}$ cycloalkyl or 5- to 6-membered heterocyclyl, wherein the $C_{1-6}$ alkyl, $C_{1-6}$ haloalkyl, $C_{4-6}$ cycloalkyl and 5- to 6-membered heterocyclyl are optionally substituted with one or more R";
$R_{11}$, $R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{10}$, $R_{11}$ and the atom to which they are attached are taken together to form 5- to 6-membered heterocyclyl or 5- to

6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";

wherein R" is H, D, halogen, oxo, $-C(O)R_g$, $-C(O)OR_g$, $-C(O)NR_hR_j$, $-NR_hR_j$, $-NR_gC(O)R_h$, $-OR_g$, $-OC(O)R_g$, $-NR_gS(O)_2R_h$, $-S(O)_2NR_hR_j$, $-S(O)R_g$, $-S(O)_2R_g$, $C_{1-6}$ alkyl, $C_{1-6}$ haloalkyl, $C_{4-6}$ cycloalkyl or 4- to 6-membered heterocyclyl, wherein the $C_{1-6}$ alkyl, $C_{1-6}$ haloalkyl, $C_{4-6}$ cycloalkyl and 4- to 6-membered heterocyclyl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

In one more specific embodiment, the present disclosure relates to a compound of formula (V) above, or a tautomer, a stereoisomer, a prodrug, a crystal form, a pharmaceutically acceptable salt, a hydrate or a solvate thereof, wherein,

$X_2$ is $CR_2$;

$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_5$ is halogen;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

n is 0, 1, 2, 3 or 4;

$Y_1$ is N or $CR_{12}$; alternatively, $Y_1$ is N;

$Y_2$ is N or $CR_{13}$;

$R_{10}$ is H, $C_{1-6}$ alkyl, $C_{1-6}$ haloalkyl or 5- to 6-membered heterocyclyl, wherein the $C_{1-6}$ alkyl, $C_{1-6}$ haloalkyl and 5- to 6-membered heterocyclyl are optionally substituted with one or more R";

$R_{11}$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_{12}$ and $R_{13}$ are independently H, D or halogen;

or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";

wherein R" is H, D, halogen, $-C(O)OR_g$, $-C(O)NR_hR_j$, $C_{1-6}$ alkyl, $C_{1-6}$ haloalkyl or 4- to 6-membered heterocyclyl, wherein the $C_{1-6}$ alkyl, $C_{1-6}$ haloalkyl and 4- to 6-membered heterocyclyl are optionally substituted with one or more D, up to fully deuterated;

wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (V) above, or a tautomer, a stereoisomer, a prodrug, a crystal form, a pharmaceutically acceptable salt, a hydrate or a solvate thereof, wherein,

$X_2$ is $CR_2$;

$R_2$ is H, D or $-CH(CH_3)(CF_3)$;

$R_5$ is F;

$R_6$ is Me;

$R_t$ is H or D;

n is 0, 1, 2, 3 or 4;

$Y_1$ is N or $CR_{12}$; alternatively, $Y_1$ is N;

$Y_2$ is N or $CR_{13}$;

$R_{10}$ is H, Me, $CHF_2$, $CH_2CF_3$ or piperidyl, which is optionally substituted with one or more R";

$R_{11}$ is H, D or Me;

$R_{12}$ and $R_{13}$ are independently H or D;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is Me, Et, $-C(O)OR_g$, $-C(O)NR_hR_j$, or oxetanyl;
wherein each of $R_g$, $R_h$ and $R_j$ is independently H, D, $C_{1-6}$ alkyl or $C_{1-6}$ haloalkyl.

In one more specific embodiment, the present disclosure relates to a compound of formula (V) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$, $R_{11}$, $R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (V) above, or a tautomer, a stereoisomer, a prodrug, a crystal form, a pharmaceutically acceptable salt, a hydrate or a solvate thereof, wherein,

$X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$ is H, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{11}$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{12}$ and $R_{13}$ are independently H, D or halogen;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered

heterocyclyl and 5- to 6-membered heteroaryl are optionally substituted with one or more R";
wherein R" is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (V) above, or a tautomer, a stereoisomer, a prodrug, a crystal form, a pharmaceutically acceptable salt, a hydrate or a solvate thereof, wherein,

$X_2$ is $CR_2$;
$R_2$ is H, D or $-CH(CH_3)(CF_3)$;
$R_5$ is F;
$R_6$ is Me;
$R_t$ is H or D;
n is 0, 1, 2, 3 or 4;
$Y_1$ is N or $CR_{12}$; alternatively, $Y_1$ is N;
$Y_2$ is N or $CR_{13}$;
$R_{10}$ is H, Me, $CHF_2$ or $CH_2CF_3$;
$R_{11}$ is H, D or Me;
$R_{12}$ and $R_{13}$ are independently H or D;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is Me or Et.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (VI):

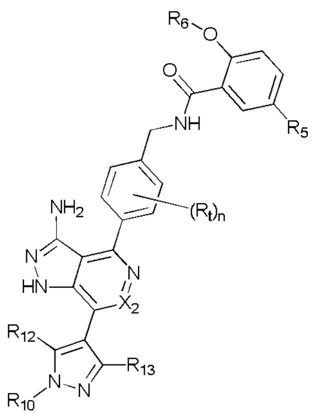
(VI)

wherein,

$X_2$ is N or $CR_2$; alternatively, $X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$R_{10}$ is H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (VI) above, or a tautomer, a stereoisomer, a prodrug, a crystal form, a pharmaceutically acceptable salt, a hydrate or a solvate thereof, wherein,

$X_2$ is $CR_2$;
$R_2$ is H, D or $-CH(CH_3)(CF_3)$;
$R_5$ is F;
$R_6$ is Me;
$R_t$ is H or D;
n is 0, 1, 2, 3 or 4;
$R_{10}$ is H, Me, $CHF_2$ or $CH_2CF_3$;
$R_{12}$ and $R_{13}$ are independently H or D.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (VII):

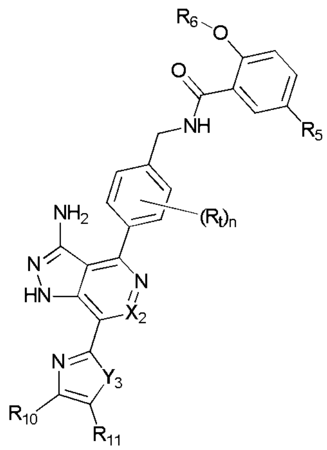

(VII)

wherein,

$X_2$ is N or $CR_2$; alternatively, $X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_3$ is O or S;
$R_{10}$ and $R_{11}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{10}$, $R_{11}$ and the atom to which they are attached are taken together to form $C_{5-8}$ cycloalkyl, 5- to 6-membered heterocyclyl, $C_{6-10}$ aryl or 5- to 6-membered heteroaryl, wherein the $C_{5-8}$ cycloalkyl, 5- to 6-membered heterocyclyl, $C_{6-10}$ aryl and 5- to 6-membered heteroaryl are optionally substituted with one or more R";
wherein R" is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (VII) above, or a tautomer, a stereoisomer, a prodrug, a crystal form, a pharmaceutically acceptable salt, a hydrate or a solvate thereof,

wherein,

$X_2$ is $CR_2$;
$R_2$ is H, D, halogen or $-CH(CH_3)(CF_3)$;
$R_5$ is F;
$R_6$ is Me;
$R_t$ is H or D;
n is 0, 1, 2, 3 or 4;
$Y_3$ is O or S;
$R_{10}$ is H, Me, $CHF_2$ or $CH_2CF_3$;
$R_{11}$ is H or D;
or, $R_{10}$, $R_{11}$ and the atom to which they are attached are taken together to form $C_{5-8}$ cycloalkyl, 5- to 6-membered heterocyclyl, $C_{6-10}$ aryl or 5- to 6-membered heteroaryl, wherein the $C_{5-8}$ cycloalkyl, 5- to 6-membered heterocyclyl, $C_{6-10}$ aryl and 5- to 6-membered heteroaryl are optionally substituted with one or more R";
wherein R" is Me or Et.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (VIII), (VIII-1) or (VIII-2):

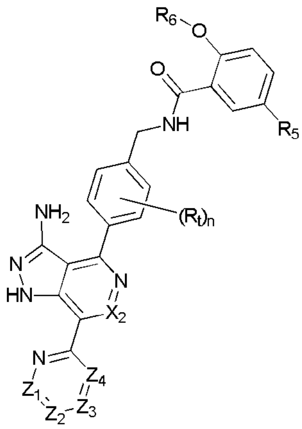
(VIII),
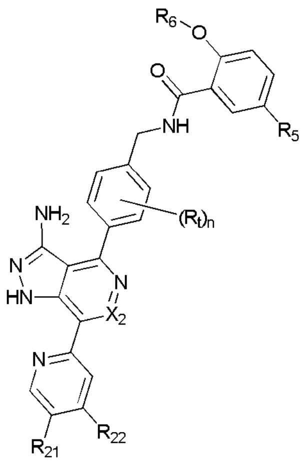
(VIII-1) or
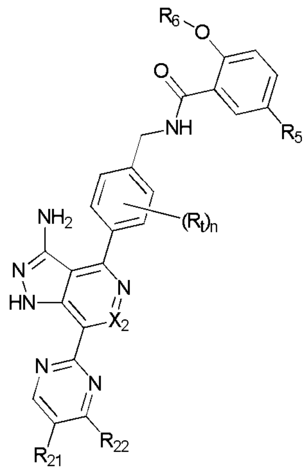
(VIII-2)

wherein,

$X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_2$ is H or D;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$, $R_{22}$ and $R_{23}$ are independently H, D, halogen, $-OC(O)R_d$, $-NR_dC(O)R_e$, $-C(O)NR_eR_f$, $-C(O)OR_d$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated, and $R_{20}$, $R_{22}$ and $R_{23}$ are independently and optionally substituted with one or more R";
$R_{21}$ is H, D, halogen, $-OR_d$, $-NR_eR_f$, $-C(O)R_d$, $-OC(O)R_d$, $-NR_dC(O)R_e$, $-C(O)NR_eR_f$, $-C(O)OR_d$, $-S(O)_2NR_eR_f$, $-S(O)R_d$, $-S(O)_2R_d$, $-P(=O)R_eR_f$, $-OP(=O)R_eR_f$, $C_{1-6}$ alkyl, $C_{1-6}$ haloalkyl, $C_{3-8}$ cycloalkyl or 4- to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with

one or more D, up to fully deuterated, and $R_{21}$ is optionally substituted with one or more R";
wherein each of $R_d$, $R_e$ and $R_f$ is independently H, D, $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl and 5- to 10-membered heteroaryl are optionally substituted with one or more D, up to fully deuterated, and optionally substituted with one or more R";
wherein R" is H, D, halogen, $-OR_g$, $-NR_hR_j$, $C_{1-6}$ alkyl, $C_{1-6}$ haloalkyl, $C_{3-8}$ cycloalkyl or 4- to 7-membered heterocyclyl, wherein R" is optionally substituted with one or more D, halogen, $C_{1-6}$ alkyl or $C_{1-6}$ haloalkyl;
each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

In one more specific embodiment, the present disclosure relates to a compound of formula (VIII), (VIII-1) or (VIII-2) above, or a tautomer, a stereoisomer, a prodrug, a crystal form, a pharmaceutically acceptable salt, a hydrate or a solvate thereof, wherein,

$X_2$ is $CR_2$;
$R_2$ is H, D, F, Me, $CHF_2$, $CF_3$ or $-CH(CH_3)(CF_3)$; alternatively, $R_2$ is H or D;
$R_5$ is F;
$R_6$ is Me;
$R_t$ is H or D;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$ is H or D;
$R_{21}$ is H, D, F, Cl, Me, $CHF_2$, $CF_3$, $CH_2CF_3$, $-C(OH)(CH_3)_2$, $-OR_d$, $-NR_dC(O)R_e$, $-S(O)R_d$, $-S(O)_2R_d$, $-P(=O)R_eR_f$ or 4- to 7-membered heterocyclyl, which is optionally substituted with one or more R";
$R_{22}$ is H, D, $-NR_dC(O)R_e$ or $-C(O)NR_eR_f$, which is optionally substituted with one or more R";
$R_{23}$ is H or D;
wherein each of $R_d$, $R_e$ and $R_f$ is independently H, D, $C_{1-6}$ alkyl, $C_{1-6}$ haloalkyl, $C_{3-8}$ cycloalkyl or 4-to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more R";
wherein R" is Me, Et, $-OR_g$, $-NR_hR_j$ or 4- to 7-membered heterocyclyl, wherein R" is optionally substituted with one or more D, halogen, $C_{1-6}$ alkyl or $C_{1-6}$ haloalkyl;
each of $R_g$, $R_h$ and $R_j$ is independently H, D, $C_{1-6}$ alkyl or $C_{1-6}$ haloalkyl.

In one more specific embodiment, the present disclosure relates to a compound of formula (VIII), (VIII-1) or (VIII-2) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_2$ is H or D;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$, $R_{22}$ and $R_{23}$ are independently H, D, halogen, $-OC(O)R_d$, $-NR_dC(O)R_e$, $-C(O)NR_eR_f$, $-C(O)OR_d$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated,

and $R_{20}$, $R_{22}$ and $R_{23}$ are independently and optionally substituted with one or more R";
$R_{21}$ is H, D, halogen, $-OR_d$, $-NR_eR_f$, $-OC(O)R_d$, $-NR_dC(O)R_e$, $-C(O)NR_eR_f$, $-C(O)OR_d$, $-S(O)R_d$, - $S(O)_2R_d$, $C_{1-6}$ alkyl, $C_{1-6}$ haloalkyl, $C_{3-8}$ cycloalkyl or 4- to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more D, up to fully deuterated, and $R_{21}$ is optionally substituted with one or more R";
wherein each of $R_d$, $R_e$ and $R_f$ is independently H, D, $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl and 5- to 10-membered heteroaryl are optionally substituted with one or more D, up to fully deuterated, which is optionally substituted with one or more R";
wherein R" is H, D, halogen, $-OR_g$, $-NR_hR_j$, $C_{1-6}$ alkyl, $C_{1-6}$ haloalkyl, $C_{3-8}$ cycloalkyl or 4- to 7-membered heterocyclyl, wherein R" is optionally substituted with one or more D, halogen, $C_{1-6}$ alkyl or $C_{1-6}$ haloalkyl;
each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

In one more specific embodiment, the present disclosure relates to a compound of formula (VIII), (VIII-1) or (VIII-2) above, or a tautomer, a stereoisomer, a prodrug, a crystal form, a pharmaceutically acceptable salt, a hydrate or a solvate thereof, wherein,

$X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_2$ is H or D;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$ and $R_{23}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{21}$ is H, D, halogen, $-OR_d$, $-NR_dC(O)R_e$, $-S(O)R_d$, $-S(O)_2R_d$, $C_{1-6}$ alkyl, $C_{1-6}$ haloalkyl or 4- to 7-membered heterocyclyl, wherein the $C_{1-6}$ alkyl, $C_{1-6}$ haloalkyl and 4- to 7-membered heterocyclyl are optionally substituted with one or more D, up to fully deuterated, and $R_{21}$ is optionally substituted with one or more R";
$R_{22}$ is H, D, $-NR_dC(O)R_e$ or $-C(O)NR_eR_f$, which is optionally substituted with one or more R";
wherein each of $R_d$, $R_e$ and $R_f$ is independently H, D, $C_{1-6}$ alkyl, $C_{1-6}$ haloalkyl, $C_{3-8}$ cycloalkyl or 4-to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more R";
wherein R" is H, D, halogen, $-OR_g$, $-NR_hR_j$, $C_{1-6}$ alkyl, $C_{1-6}$ haloalkyl, $C_{3-8}$ cycloalkyl or 4- to 7-membered heterocyclyl, wherein R" is optionally substituted with one or more D, halogen, $C_{1-6}$ alkyl or $C_{1-6}$ haloalkyl;
each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

In one more specific embodiment, the present disclosure relates to a compound of formula (VIII), (VIII-1) or (VIII-2) above, or a tautomer, a stereoisomer, a prodrug, a crystal form, a pharmaceutically acceptable salt, a hydrate or a solvate thereof, wherein,

$X_2$ is $CR_2$;
$R_2$ is H, D, Me, $CHF_2$ or $-CH(CH_3)(CF_3)$; alternatively, $R_2$ is H or D;
$R_5$ is F;
$R_6$ is Me;
$R_t$ is H or D;
n is 0, 1, 2, 3 or 4;

$Z_1$ is N or $CR_{20}$;
$Z_2$ is N or $CR_{21}$;
$Z_3$ is N or $CR_{22}$;
$Z_4$ is N or $CR_{23}$;
$R_{20}$ is H or D;
$R_{21}$ is H, D, F, Cl, Me, $CHF_2$, $CF_3$, $CH_2CF_3$, $-C(OH)(CH_3)_2$, $-OR_d$, $-NR_dC(O)R_e$, $-S(O)R_d$, $-S(O)_2R_d$, or 4- to 7-membered heterocyclyl, which is optionally substituted with one or more R";
$R_{22}$ is H, D, $-NR_dC(O)R_e$ or $-C(O)NR_eR_f$, which is optionally substituted with one or more R";
$R_{23}$ is H or D;
wherein each of $R_d$, $R_e$ and $R_f$ is independently H, D, $C_{1-6}$ alkyl, $C_{1-6}$ haloalkyl, $C_{3-8}$ cycloalkyl or 4-to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more R";
wherein R" is Me, Et, $-OR_g$, $-NR_hR_j$ or 4- to 7-membered heterocyclyl, wherein R" is optionally substituted with one or more D, halogen, $C_{1-6}$ alkyl or $C_{1-6}$ haloalkyl;
each of $R_g$, $R_h$ and $R_j$ is independently H, D, $C_{1-6}$ alkyl or $C_{1-6}$ haloalkyl.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (IX):

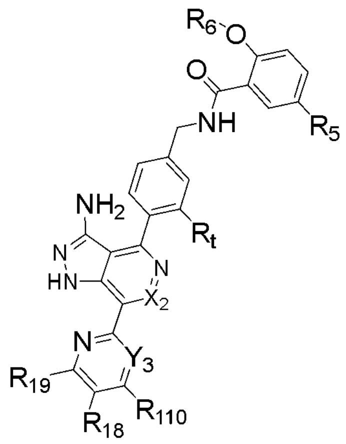

(IX)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N or $CR_{111}$;
$R_{18}$, $R_{19}$, $R_{110}$ and $R_{111}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{18}$ and $R_{19}$ or $R_{18}$ and $R_{110}$ or $R_{110}$ and $R_{111}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered

heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the N atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (IX) above, or a tautomer, a stereoisomer, a prodrug, a crystal form, a pharmaceutically acceptable salt, a hydrate or a solvate thereof, wherein,

$X_2$ is N or $CR_2$;

$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_5$ is halogen;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$Y_3$ is N or $CR_{111}$;

$R_{18}$ is H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";

$R_{110}$ is H, D, halogen, $-NR_dC(O)R_e$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_{19}$ and $R_{111}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

or, $R_{18}$ and $R_{19}$ or $R_{18}$ and $R_{110}$ or $R_{110}$ and $R_{111}$ and the atom to which they are attached are taken together to form $C_{3-6}$ cycloalkyl, 3- to 7-membered monocyclic heterocyclyl, 6- to 12-membered fused bicyclic heterocycle, $C_{6-10}$ aryl or 5- to 10-membered heteroaryl, wherein the groups are optionally substituted with one or more R"; or

wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (IX) above, or a tautomer, a stereoisomer, a prodrug, a crystal form, a pharmaceutically acceptable salt, a hydrate or a solvate thereof, wherein,

$X_2$ is N or $CR_2$;

$R_2$ is H, D or halogen; alternatively, $R_2$ is H, D or F; alternatively, $R_2$ is H;

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

$R_t$ is H, D or halogen; alternatively, $R_t$ is H, D or F;

$Y_3$ is N or $CR_{111}$;

$R_{18}$ is H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{18}$ is halogen, $-C(O)NR_eR_f$, $-OR_d$, $-S(O)_2R_d$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";

$R_{110}$ is H, D, halogen, $-NR_4C(O)R_e$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{19}$ is H or D;

$R_{19}$ and $R_{111}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{110}$ is H or D;

or, $R_{18}$ and $R_{19}$ or $R_{18}$ and $R_{110}$ or $R_{110}$ and $R_{111}$ and the atom to which they are attached are taken together to form 3- to 7-membered monocyclic heterocyclyl, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5-membered or 6-membered heterocyclyl having 1 or 2 ring heteroatoms selected from N, O and S, wherein the group is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5-membered heterocyclyl having 1 or 2 ring heteroatoms N, wherein the group is optionally substituted with one or more R"; or

$R_{18}$ and $R_{19}$ or $R_{18}$ and $R_{110}$ or $R_{110}$ and $R_{111}$ and the atom to which they are attached are taken together to form 6- to 12-membered fused bicyclic heterocycle, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 8- to 10-membered fused bicyclic heterocycle having 1 to 3 ring heteroatoms selected from N, O and S, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 8- to 10-membered fused bicyclic heterocycle having 1 or 2 ring heteroatoms N, which is optionally substituted with one or more R"; or

$R_{18}$ and $R_{19}$ or $R_{18}$ and $R_{110}$ or $R_{110}$ and $R_{111}$ and the atom to which they are attached are taken together to form 5- to 10-membered heteroaryl, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5- or 6-membered heteroaryl having 1 to 3 ring heteroatoms selected from N, O and S, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5-membered heteroaryl having 1 to 3 ring heteroatoms selected from N, O and S, wherein the group is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5-membered heteroaryl having 1 or 2 ring heteroatoms N, wherein the group is optionally substituted with one or more R";

wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (IX) above, or a tautomer, a stereoisomer, a prodrug, a crystal form, a pharmaceutically acceptable salt, a hydrate or a solvate thereof, wherein,

$X_2$ is CH;

$R_5$ is F;

$R_6$ is methyl or $-CD_3$;

$R_t$ is H, D or F;

$Y_3$ is N or $CR_{111}$;

$R_{18}$ and $R_{19}$ or $R_{18}$ and $R_{110}$ or $R_{110}$ and $R_{111}$ and the atom to which they are attached are taken together to form 5-membered heteroaryl having 1 or 2 ring heteroatoms N, wherein the group is optionally substituted with one or more R";

each R" is independently D, halogen, $C_{1-6}$ alkyl or $C_{1-6}$ haloalkyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl and 3- to 7-membered heterocyclyl are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (IX-1):

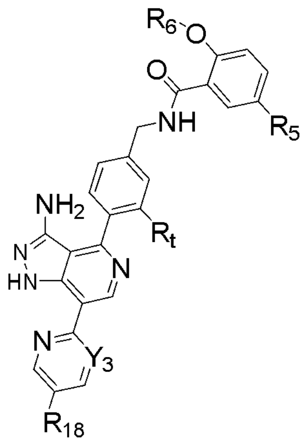

(IX-1)

wherein,

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

$R_t$ is H, D or halogen; alternatively, $R_t$ is H, D or F;

$Y_3$ is N, CH or CD; alternatively, $Y_3$ is N or CH;

$R_{18}$ is H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{18}$ is halogen, $-C(O)NR_eR_f$, $-OR_d$, $-S(O)_2R_4$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";

wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (IX-1) above, or a tautomer, a stereoisomer, a prodrug, a crystal form, a pharmaceutically acceptable salt, a hydrate or a solvate thereof, wherein,

$R_5$ is F;

$R_6$ is methyl or $-CD_3$;

$R_t$ is H, D or F;

$Y_3$ is N or CH;

$R_{18}$ is H, F, Cl, $-CH_3$, $-CHF_2$, $-CF_3$, $-OCF_3$,

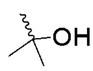, 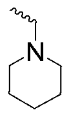, 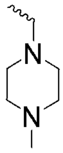, 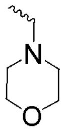, 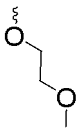, 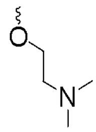,

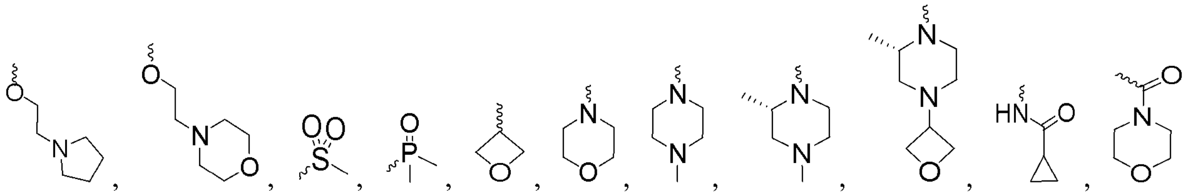

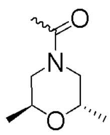 or 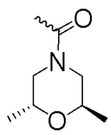,

wherein the groups are optionally substituted with one or more D, up to fully deuterated; alternatively, $R_{18}$ is F, Cl, $-CH_3$, $-CHF_2$, $-CF_3$, $-OCF_3$,

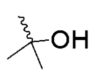, 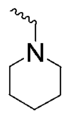, 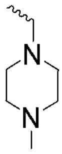, 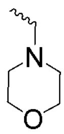, 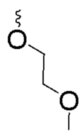,

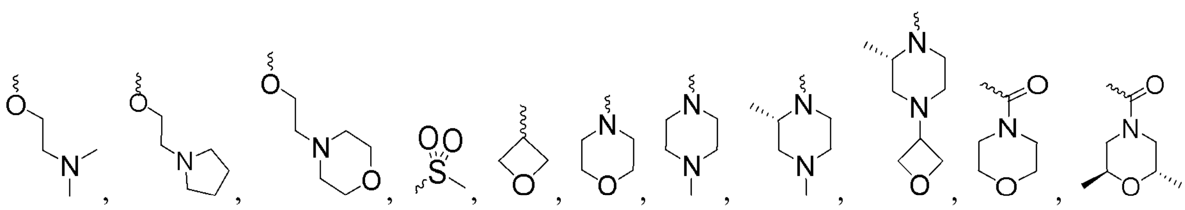

or

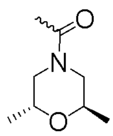,

wherein the groups are optionally substituted with one or more D, up to fully deuterated; alternatively, $R_{18}$ is $-CH_3$,

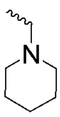, 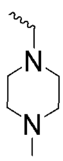, 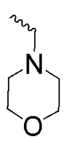, 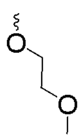, 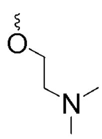, 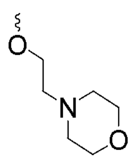, 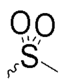, 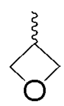,

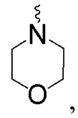 , 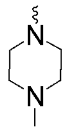 , 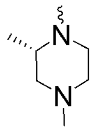 , 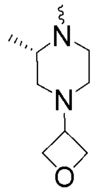 or 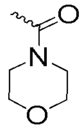 ,

wherein the groups are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (X):

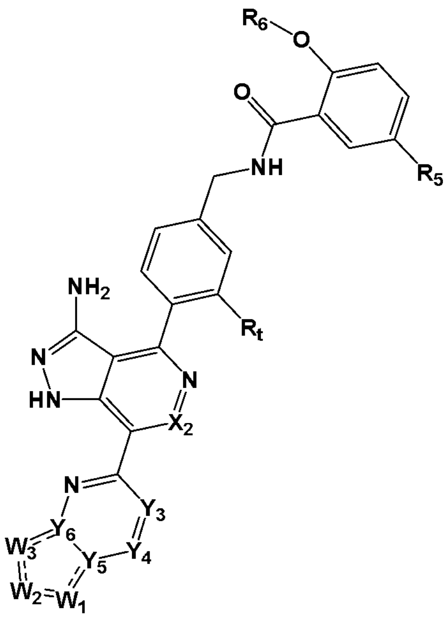

(X)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N, CH or CD;
$Y_4$ is N, CH or CD;
$Y_5$ and $Y_6$ are independently N or C, and at least one of $Y_5$ and $Y_6$ is N;
$W_1$ is N or $CR_{112}$;
$W_2$ is N or $CR_{113}$;
$W_3$ is N or $CR_{114}$;
- - - indicates single or double bonds;
with the proviso that, the bicyclic ring containing $Y_3$, $Y_4$, $Y_5$, $Y_6$, $W_1$, $W_2$ and $W_3$ is an aromatic ring;
$R_{112}$, $R_{113}$ and $R_{114}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R''; or

3) $R_{112}$ and $R_{113}$ or $R_{113}$ and $R_{114}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (X) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N, CH or CD;
$Y_4$ is N, CH or CD;
$Y_5$ and $Y_6$ are independently N or C, and at least one of $Y_5$ and $Y_6$ is N;
$W_1$ is N or $CR_{112}$;
$W_2$ is N or $CR_{113}$;
$W_3$ is N or $CR_{114}$;
- - - indicates single or double bonds;
with the proviso that, the bicyclic ring containing $Y_3$, $Y_4$, $Y_5$, $Y_6$, $W_1$, $W_2$ and $W_3$ is an aromatic ring;
$R_{112}$, $R_{113}$ and $R_{114}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";
or, $R_{112}$ and $R_{113}$ or $R_{113}$ and $R_{114}$ and the atom to which they are attached are taken together to form $C_{3-6}$ cycloalkyl, 3- to 7-membered monocyclic heterocyclyl, 6- to 12-membered fused bicyclic heterocycle, $C_{6-10}$ aryl or 5- to 10-membered heteroaryl, wherein the groups are optionally substituted with one or more R";
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are

optionally substituted with one or more D, up to fully deuterated.

In one more specific embodiment, the present disclosure relates to a compound of formula (X) above, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
$R_t$ is H, D or halogen; alternatively, $R_t$ is H, D or F;
$Y_3$ is N, CH or CD; alternatively, $Y_3$ is N or CH;
$Y_4$ is N, CH or CD; alternatively, $Y_4$ is N or CH;
$Y_5$ and $Y_6$ are independently N or C, and at least one of $Y_5$ and $Y_6$ is N;
$W_1$ is N or $CR_{112}$;
$W_2$ is N or $CR_{113}$;
$W_3$ is N or $CR_{114}$;
- - - indicates single or double bonds;
with the proviso that, the bicyclic ring containing $Y_3$, $Y_4$, $Y_5$, $Y_6$, $W_1$, $W_2$ and $W_3$ is an aromatic ring;
$R_{112}$, $R_{113}$ and $R_{114}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{112}$, $R_{113}$ and $R_{114}$ are independently H, D, $C_{1-6}$ alkyl or $C_{1-6}$ haloalkyl, wherein the $C_{1-6}$ alkyl and $C_{1-6}$ haloalkyl are optionally substituted with one or more R"; or, $R_{112}$ and $R_{113}$ or $R_{113}$ and $R_{114}$ and the atom to which they are attached are taken together to form 3- to 7-membered heterocyclyl, which is optionally substituted with one or more R"; alternatively, $R_{112}$ and $R_{113}$ or $R_{113}$ and $R_{114}$ and the atom to which they are attached are taken together to form 5-membered or 6-membered heterocyclyl having 1 or 2 ring heteroatoms independently selected from N, O and S, which is optionally substituted with one or more R";
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (X-1) or (X-2):

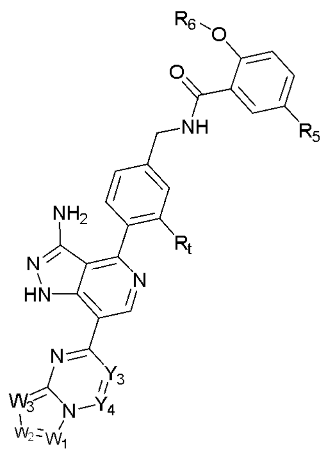
(X-1) or
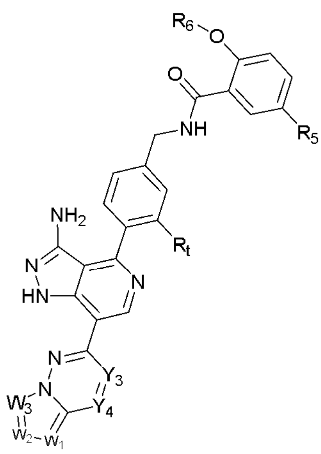
(X-2)

wherein,

$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
$R_t$ is H, D or halogen; alternatively, $R_t$ is H, D or F;
$Y_3$ is N, CH or CD; alternatively, $Y_3$ is N or CH;
$Y_4$ is N, CH or CD; alternatively, $Y_4$ is N or CH;
$W_1$ is N or $CR_{112}$;
$W_2$ is N or $CR_{113}$;
$W_3$ is N or $CR_{114}$;
$R_{112}$, $R_{113}$ and $R_{114}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{112}$, $R_{113}$ and $R_{114}$ are independently H, D, $C_{1-6}$ alkyl or $C_{1-6}$ haloalkyl, wherein the $C_{1-6}$ alkyl and $C_{1-6}$ haloalkyl are optionally substituted with one or more R";
or, $R_{112}$ and $R_{113}$ or $R_{113}$ and $R_{114}$ and the atom to which they are attached are taken together to form 3- to 7-membered heterocyclyl, which is optionally substituted with one or more R"; alternatively, $R_{112}$ and $R_{113}$ or $R_{113}$ and $R_{114}$ and the atom to which they are attached are taken together to form 5-membered or 6-membered heterocyclyl having 1 or 2 ring heteroatoms independently selected from N, O and S, wherein the group is optionally substituted with one or more R";
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (X-1) or (X-2) above, or a tautomer, a stereoisomer, a prodrug, a crystal form, a pharmaceutically acceptable salt, a hydrate or a solvate thereof, wherein,

$R_5$ is F;
$R_6$ is methyl or $-CD_3$;
$R_t$ is H, D or F;
$Y_3$ is N or CH;
$Y_4$ is N or CH;
$W_1$ is N or $CR_{112}$;
$W_2$ is N or $CR_{113}$;
$W_3$ is N or $CR_{114}$;
$R_{112}$, $R_{113}$ and $R_{114}$ are H;
or, $R_{112}$ and $R_{113}$ or $R_{113}$ and $R_{114}$ and the atom to which they are attached are taken together to form the following groups:

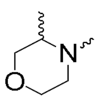
or
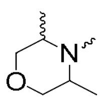
,

wherein the groups are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (XI):

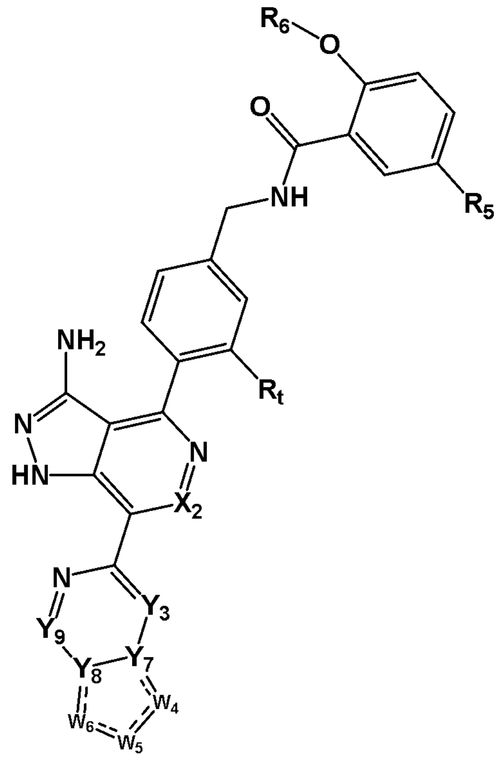

(XI)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N, CH or CD;
$Y_7$ and $Y_8$ are independently N or C, and at least one of $Y_7$ and $Y_8$ is N;
$Y_9$ is N, CH or CD;
$W_4$ is N or $CR_{115}$;
$W_5$ is N or $CR_{116}$;
$W_6$ is N or $CR_{117}$;
- - - indicates single or double bonds;
with the proviso that, the bicyclic ring containing $Y_3$, $Y_7$, $Y_8$, $Y_9$, $W_4$, $W_5$ and $W_6$ is an aromatic ring;
$R_{115}$, $R_{116}$ and $R_{117}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$, or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{115}$ and $R_{116}$ or $R_{116}$ and $R_{117}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to

10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{3-6}$ cycloalkyl, 3-to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

In one more specific embodiment, the present disclosure relates to a compound of formula (XI) above, or a tautomer, a stereoisomer, a prodrug, a crystal form, a pharmaceutically acceptable salt, a hydrate or a solvate thereof, wherein,

$X_2$ is N or $CR_2$;

$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_5$ is halogen;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_t$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$Y_3$ is N, CH or CD;

$Y_7$ and $Y_8$ are independently N or C, and at least one of $Y_7$ and $Y_8$ is N;

$Y_9$ is N, CH or CD;

$W_4$ is N or $CR_{115}$;

$W_5$ is N or $CR_{116}$;

$W_6$ is N or $CR_{117}$;

- - - indicates single or double bonds;

with the proviso that, the bicyclic ring containing $Y_3$, $Y_7$, $Y_8$, $Y_9$, $W_4$, $W_5$ and $W_6$ is an aromatic ring;

$R_{115}$, $R_{116}$ and $R_{117}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";

or, $R_{115}$ and $R_{116}$ or $R_{116}$ and $R_{117}$ and the atom to which they are attached are taken together to form $C_{3-6}$ cycloalkyl, 3- to 7-membered monocyclic heterocyclyl, 6- to 12-membered fused bicyclic heterocycle, $C_{6-10}$ aryl or 5- to 10-membered heteroaryl, wherein the groups are optionally substituted with one or more R";

wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (XI) above, or a tautomer, a stereoisomer, a prodrug, a crystal form, a pharmaceutically acceptable salt, a hydrate or a solvate thereof, wherein,

$X_2$ is N or $CR_2$;

$R_2$ is H, D or halogen; alternatively, $R_2$ is H, D or F; alternatively, $R_2$ is H;

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

$R_t$ is H, D or halogen; alternatively, $R_t$ is H, D or F;

$Y_3$ is N, CH or CD; alternatively, $Y_3$ is N or CH;

$Y_7$ and $Y_8$ are independently N or C, and at least one of $Y_7$ and $Y_8$ is N;

$Y_9$ is N, CH or CD; alternatively, $Y_4$ is N or CH;

$W_4$ is N or $CR_{115}$;

$W_5$ is N or $CR_{116}$;

$W_6$ is N or $CR_{117}$;

- - - indicates single or double bonds;

with the proviso that, the bicyclic ring containing $Y_3$, $Y_7$, $Y_8$, $Y_9$, $W_4$, $W_5$ and $W_6$ is an aromatic ring;

$R_{115}$, $R_{116}$ and $R_{117}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{115}$, $R_{116}$ and $R_{117}$ are independently H, D, $C_{1-6}$ alkyl or $C_{1-6}$ haloalkyl, wherein the $C_{1-6}$ alkyl and $C_{1-6}$ haloalkyl are optionally substituted with one or more R"; or, $R_{115}$ and $R_{116}$ or $R_{116}$ and $R_{117}$ and the atom to which they are attached are taken together to form 3- to 7-membered heterocyclyl, which is optionally substituted with one or more R"; alternatively, $R_{115}$ and $R_{116}$ or $R_{116}$ and $R_{117}$ and the atom to which they are attached are taken together to form 5-membered or 6-membered heterocyclyl, wherein the group is optionally substituted with one or more R";

wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (A) or formula (I) above, or a tautomer, a stereoisomer, a prodrug, a crystal form, a pharmaceutically acceptable salt, a hydrate or a solvate thereof, wherein the compound is a compound of formula (XI-1) or (XI-2):

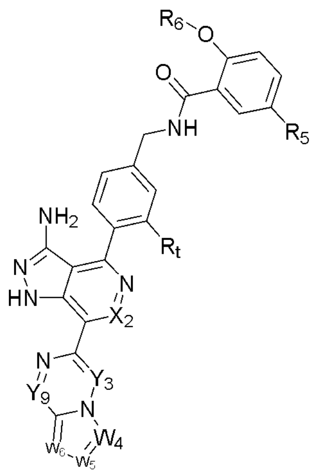

(XI-1) or

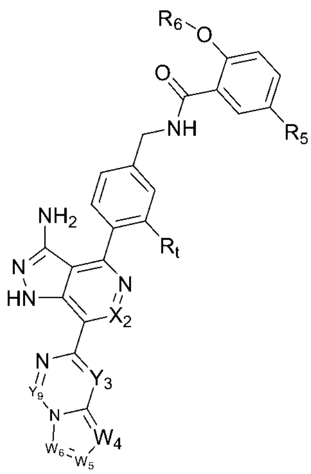

(XI-2)

wherein,

$X_2$ is N or $CR_2$;

$R_2$ is H, D or halogen; alternatively, $R_2$ is H, D or F; alternatively, $R_2$ is H;

$R_5$ is halogen; alternatively, $R_5$ is F;

$R_6$ is $C_{1-6}$ alkyl or $C_{1-6}$ haloalkyl, wherein the $C_{1-6}$ alkyl and $C_{1-6}$ haloalkyl are optionally substituted with one or more D,

up to fully deuterated; alternatively, $R_6$ is methyl or $-CD_3$;
$R_t$ is H, D or halogen; alternatively, $R_t$ is H, D or F;
$Y_3$ is N, CH or CD; alternatively, $Y_3$ is N or CH;
$Y_9$ is N, CH or CD; alternatively, $Y_9$ is N or CH;
$W_4$ is N or $CR_{115}$;
$W_5$ is N or $CR_{116}$;
$W_6$ is N or $CR_{117}$;
$R_{115}$, $R_{116}$ and $R_{117}$ are independently H, D, halogen, $-C(O)NR_eR_f$, $-NR_dC(O)R_e$, $-OR_d$, $-S(O)_2R_d$, - $P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{115}$, $R_{116}$ and $R_{117}$ are independently H, D, $C_{1-6}$ alkyl or $C_{1-6}$ haloalkyl, wherein the $C_{1-6}$ alkyl and $C_{1-6}$ haloalkyl are optionally substituted with one or more R"; or, $R_{115}$ and $R_{116}$ or $R_{116}$ and $R_{117}$ and the atom to which they are attached are taken together to form 3- to 7-membered heterocyclyl, which is optionally substituted with one or more R"; alternatively, $R_{115}$ and $R_{116}$ or $R_{116}$ and $R_{117}$ and the atom to which they are attached are taken together to form 5-membered or 6-membered heterocyclyl having 1 or 2 ring heteroatoms independently selected from N, O and S, wherein the group is optionally substituted with one or more R";
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

In one more specific embodiment, the present disclosure relates to a compound of formula (XI-1) or (XI-2), or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is F;
$R_6$ is methyl or $-CD_3$;
Rt is H, D or F;
$Y_3$ is N or CH;
$Y_9$ is N or CH;
$W_4$ is N or $CR_{115}$;
$W_5$ is N or $CR_{116}$;
$W_6$ is N or $CR_{117}$;
$R_{115}$, $R_{116}$ and $R_{117}$ are H;
or, $R_{115}$ and $R_{116}$ or $R_{116}$ and $R_{117}$ and the atom to which they are attached are taken together to form the following groups:

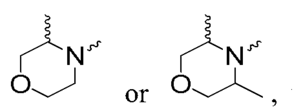

wherein the groups are optionally substituted with one or more D, up to fully deuterated.

In one more specific embodiment, the present disclosure relates to a compound of formula (I) above, or a tautomer, a stereoisomer, a prodrug, a crystal form, a pharmaceutically acceptable salt, a hydrate or a solvate thereof, wherein the compound is selected from:

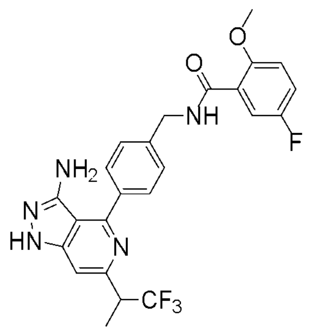
,
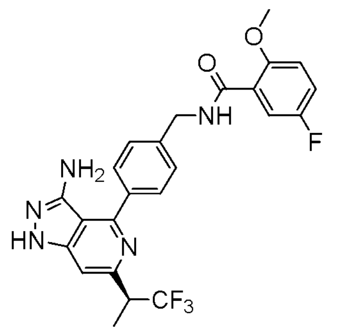
,
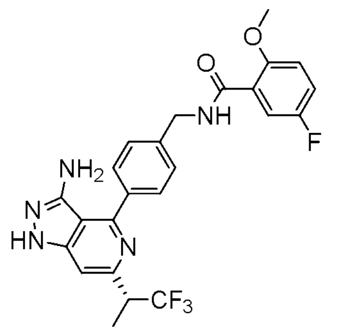
,
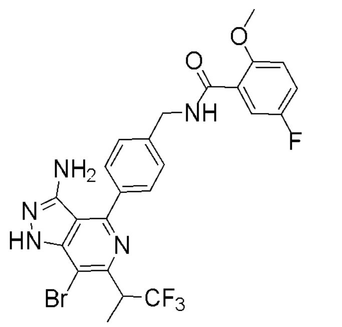
,
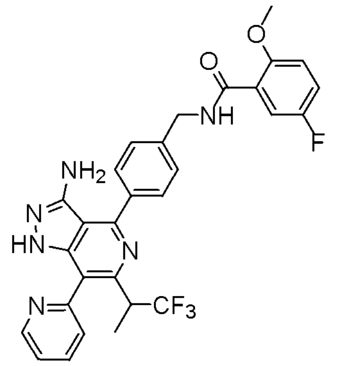
,
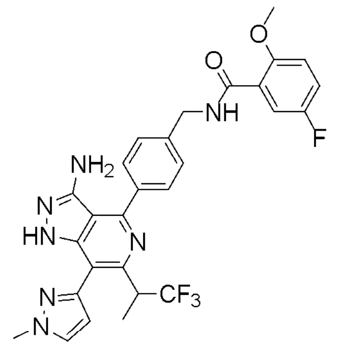
,
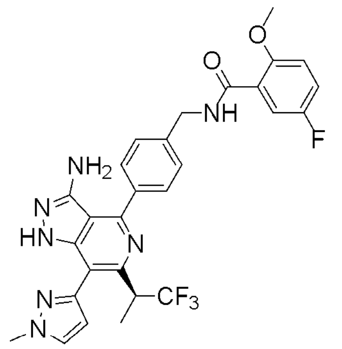
,
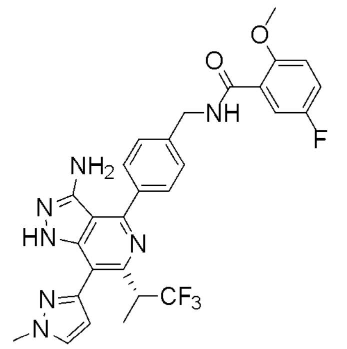
,
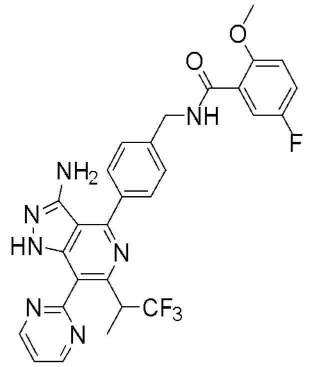
,
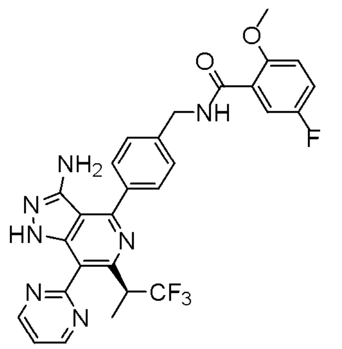
,
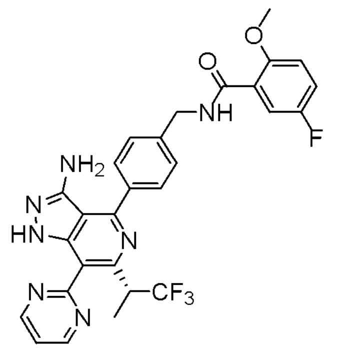
,
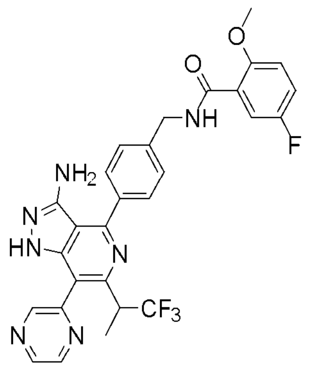
,

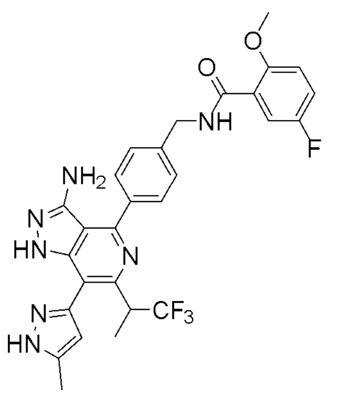
,
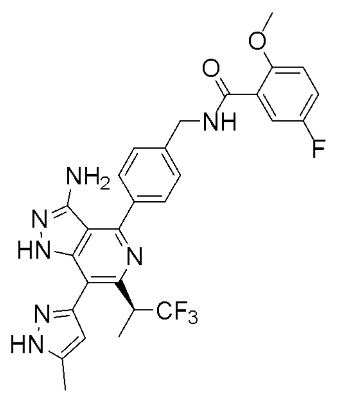
,
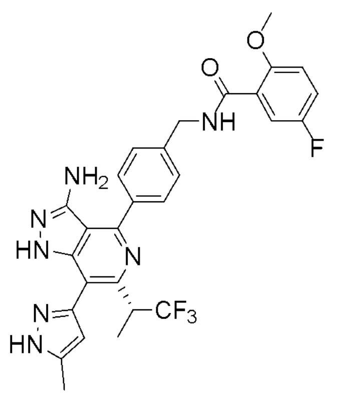
,

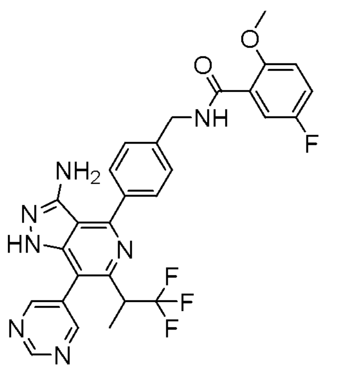
,
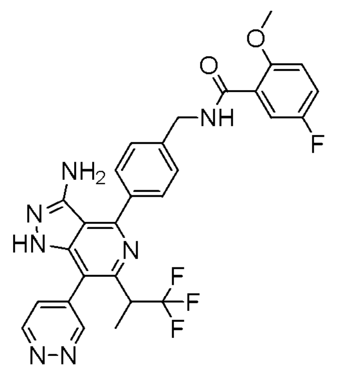
,
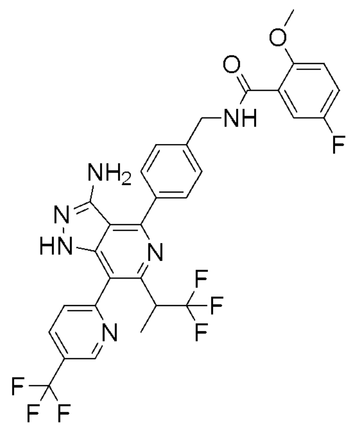
,

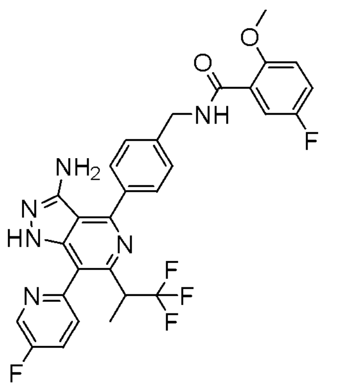
,
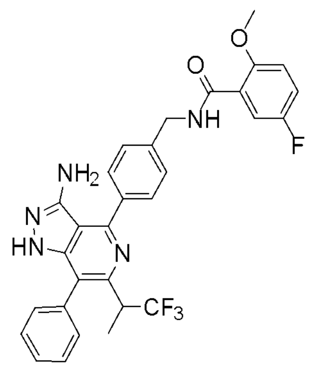
,
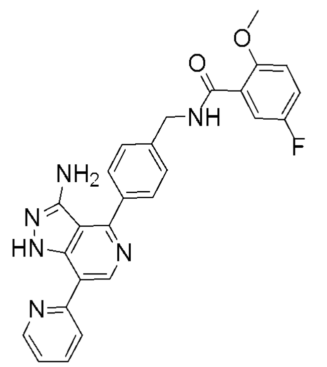
,

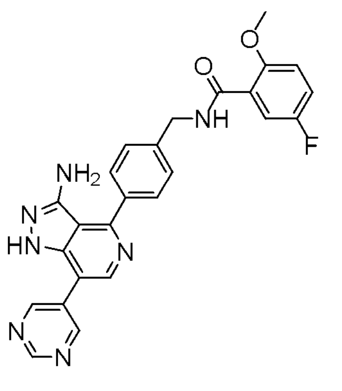
,
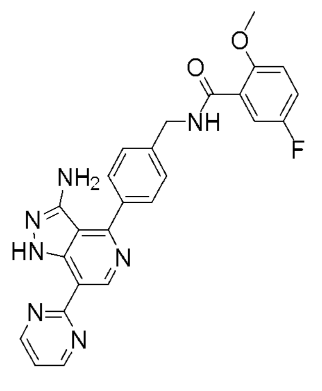
,
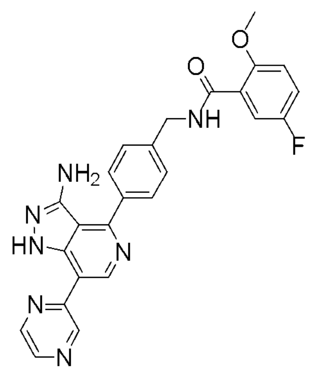
,

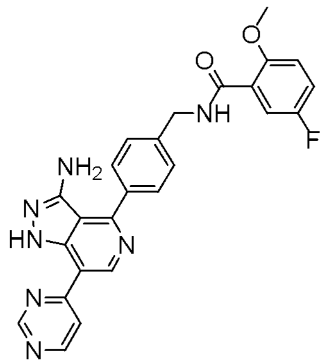,
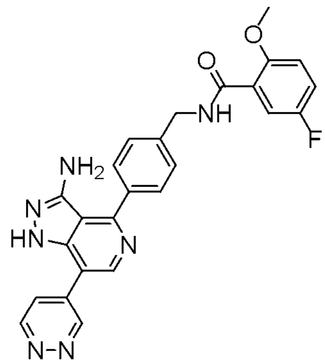,
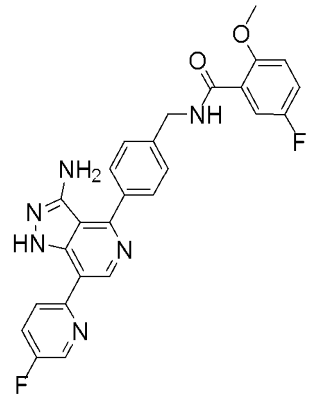,

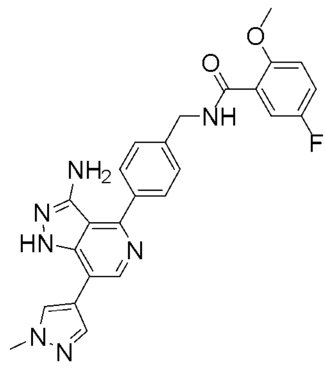,
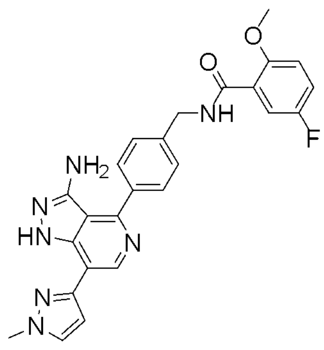,
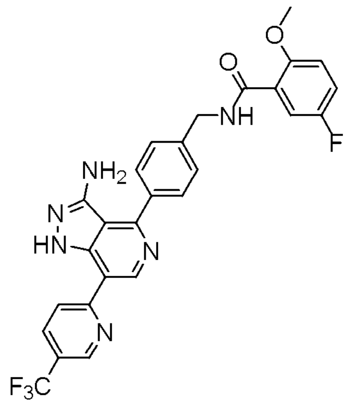,

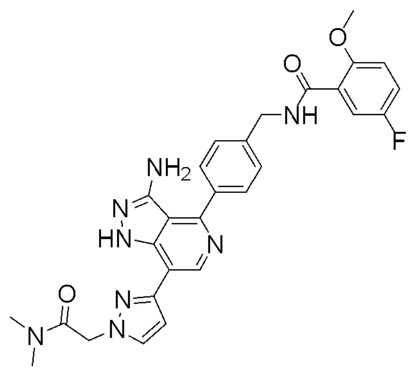,
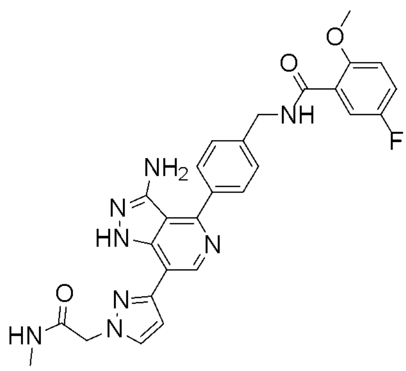,

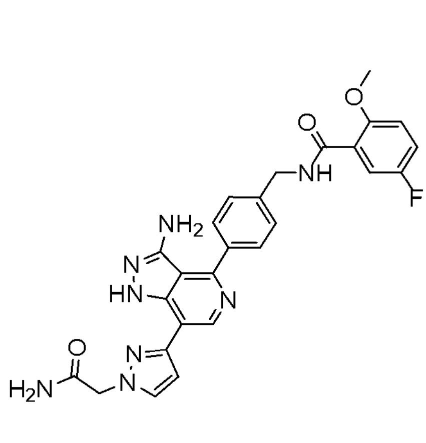,
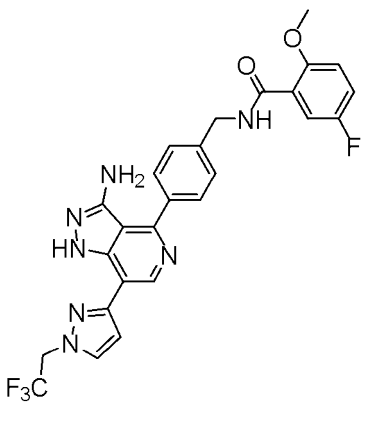,
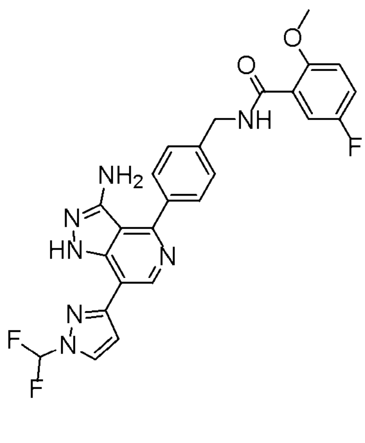,

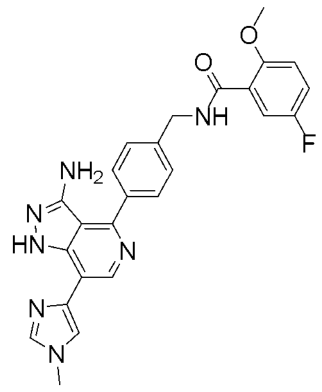 , 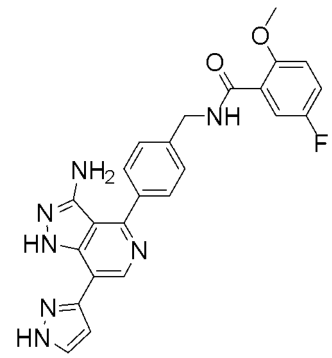 , 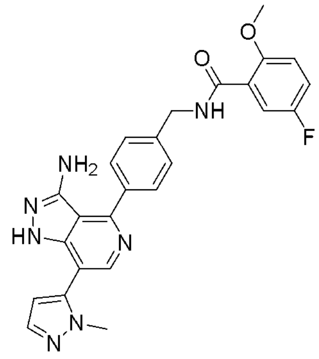 ,

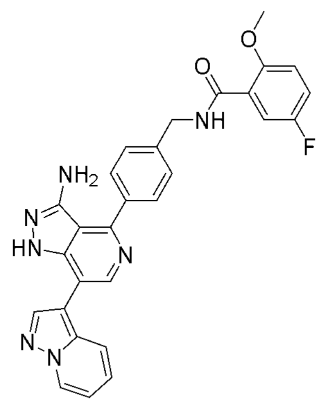 , 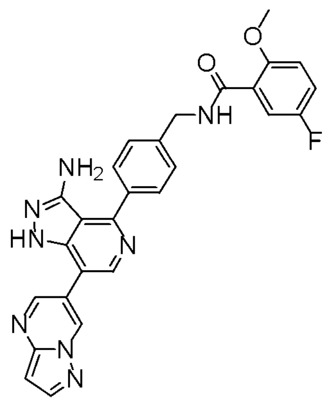 , 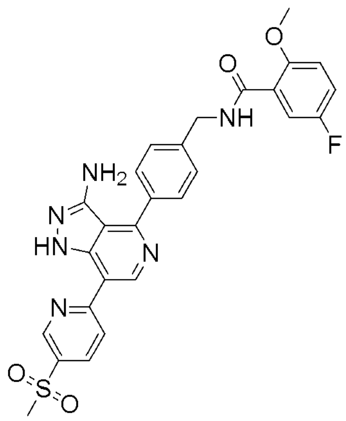 ,

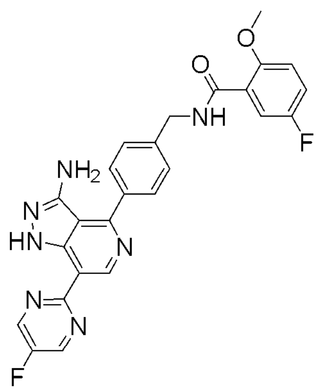 , 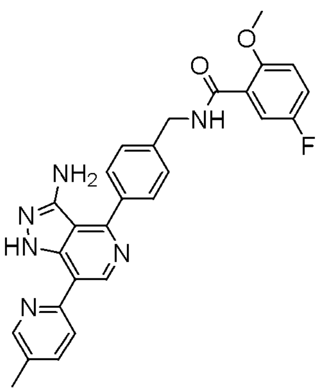 , 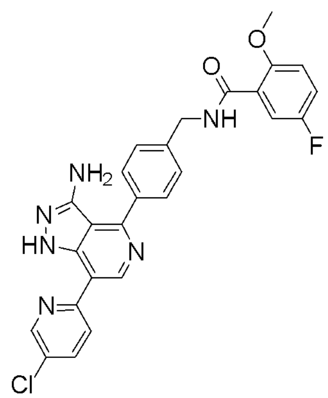 ,

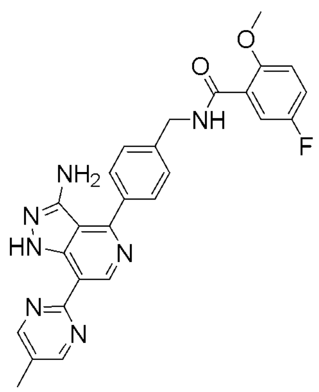 , 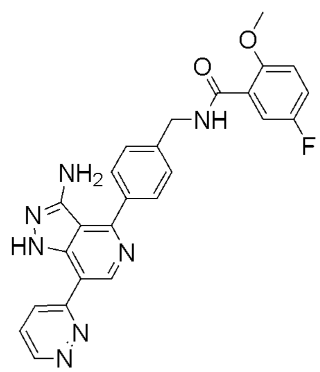 , 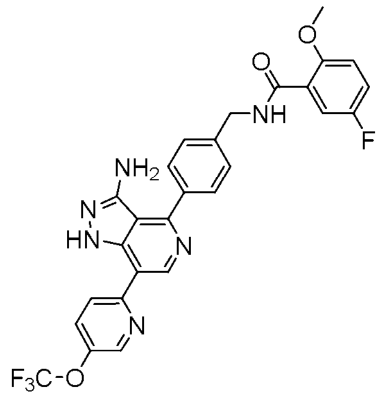 ,

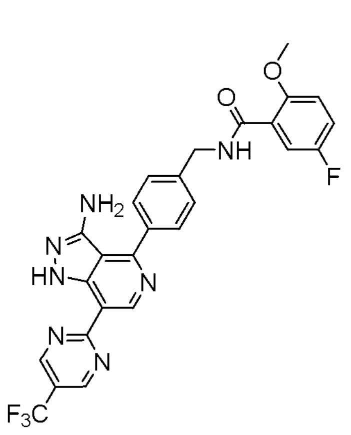 , 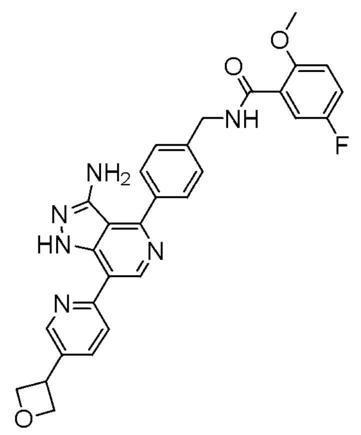 , 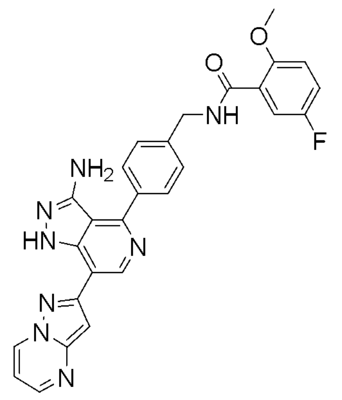 ,

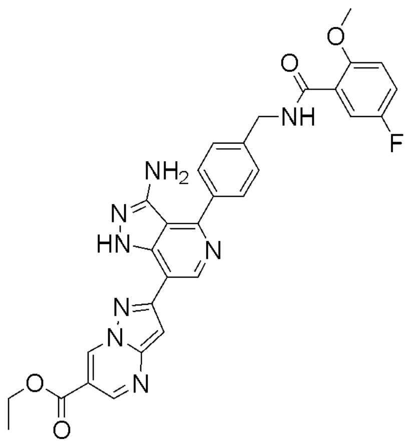 , 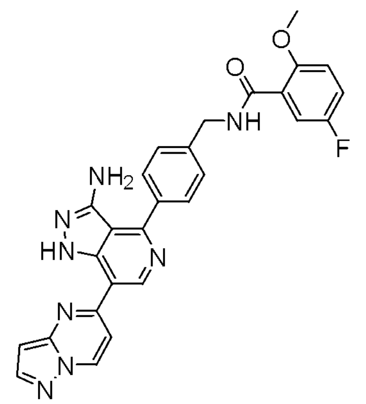 , 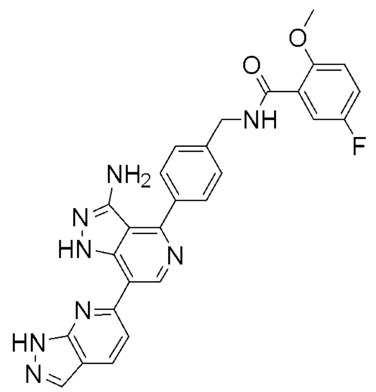 ,

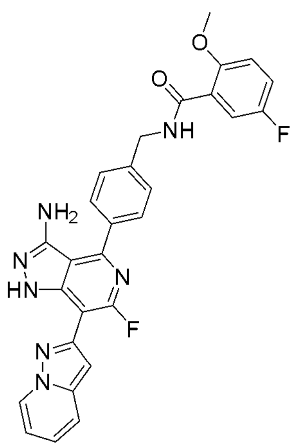 , 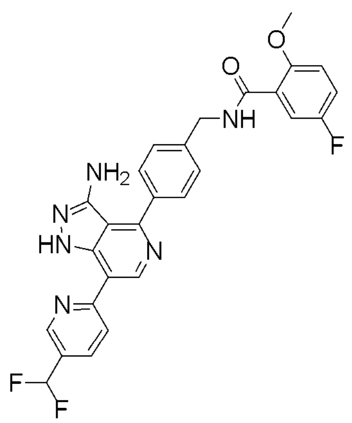 , 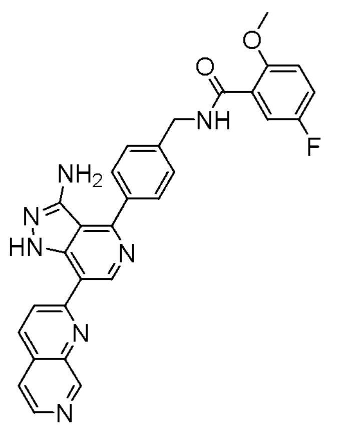 ,

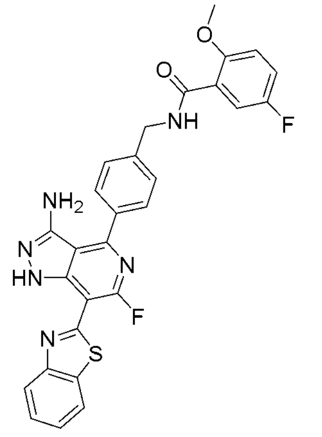 , 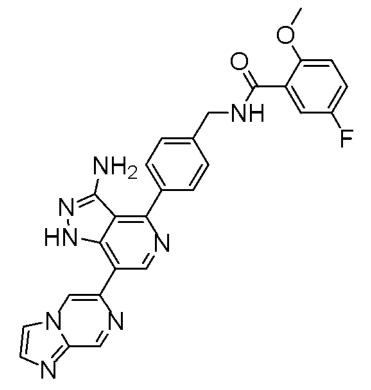 , 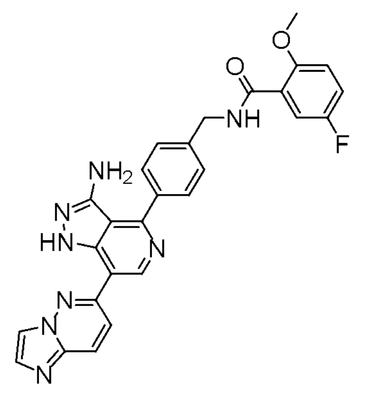 ,

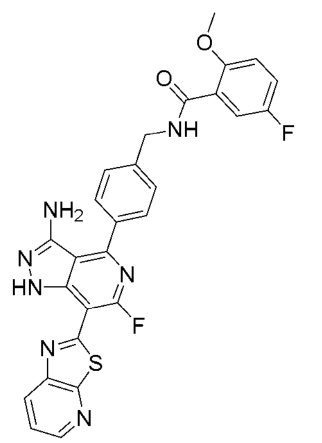 , 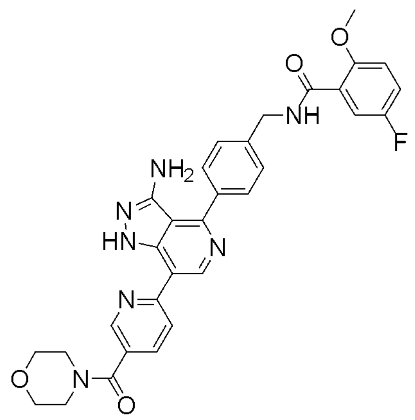 , 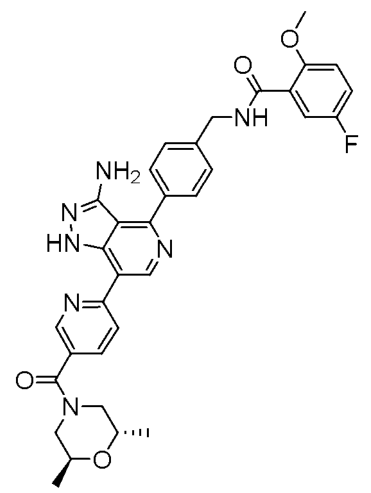 ,

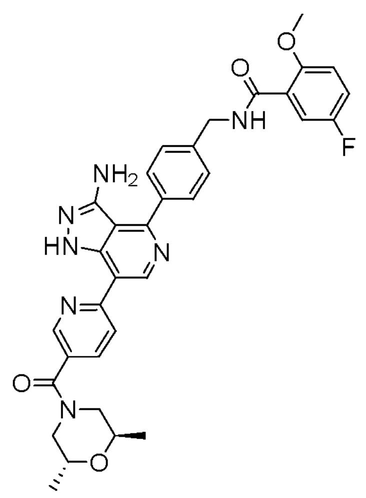 , 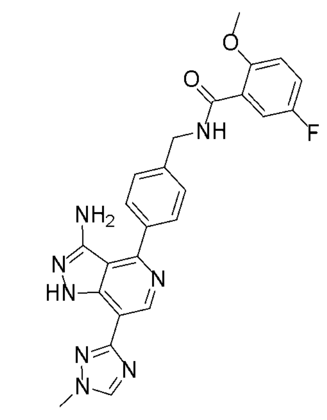 , 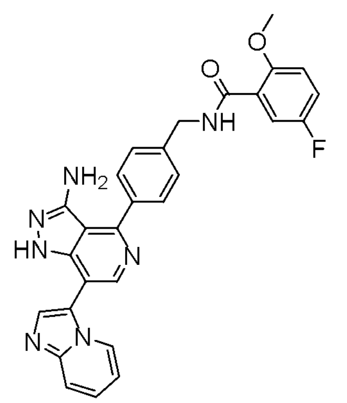 ,

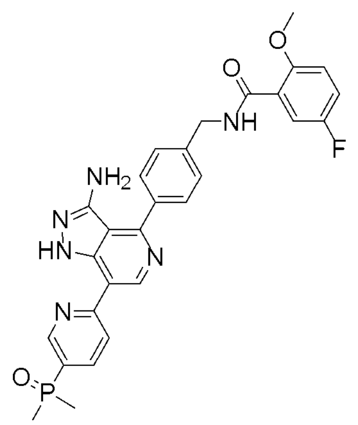
,
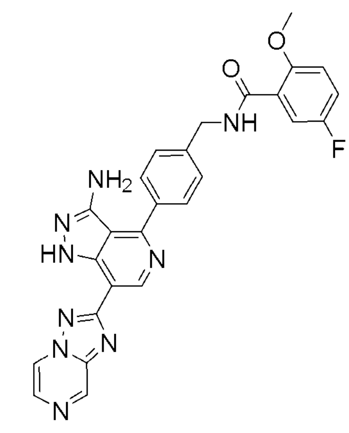
,
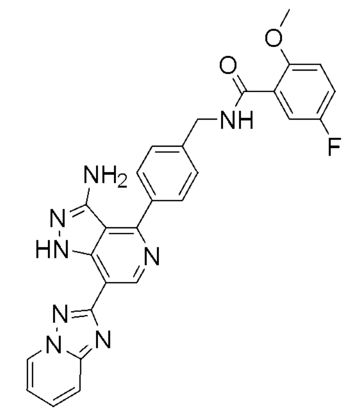
,

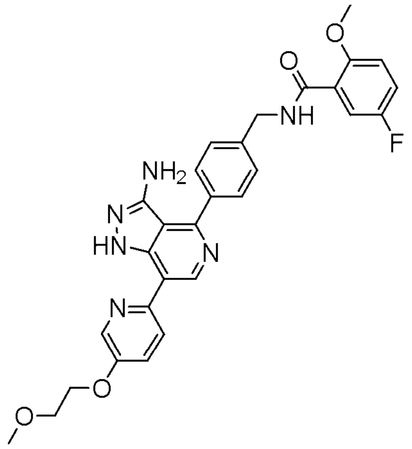
,
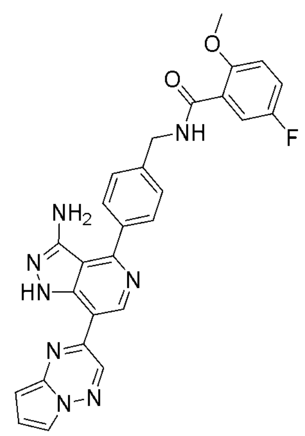
,
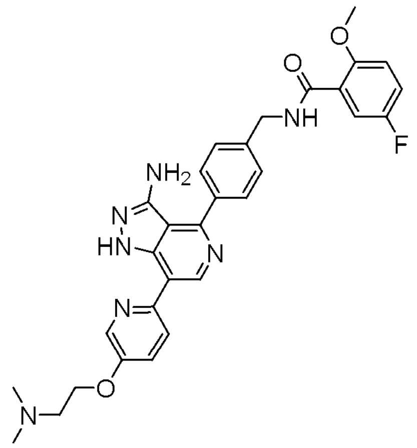
,

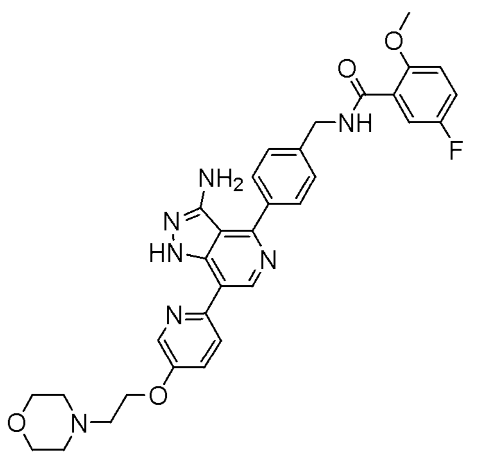
,
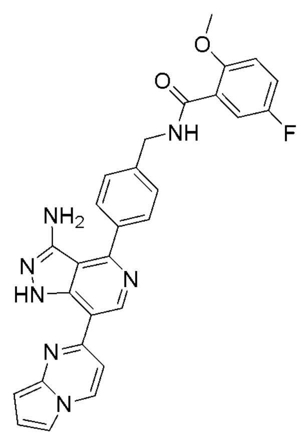
,

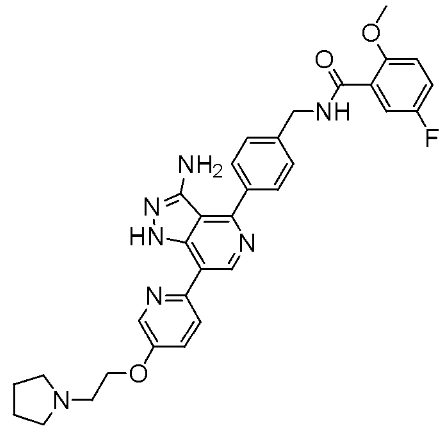

,

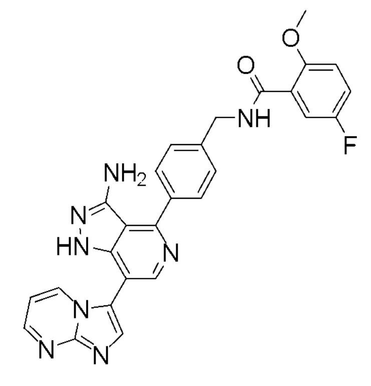

,

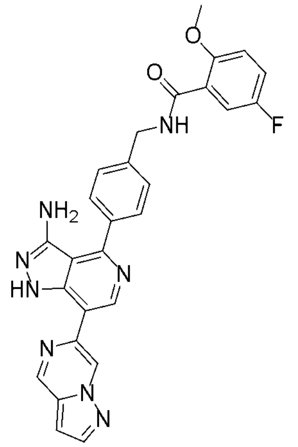

,

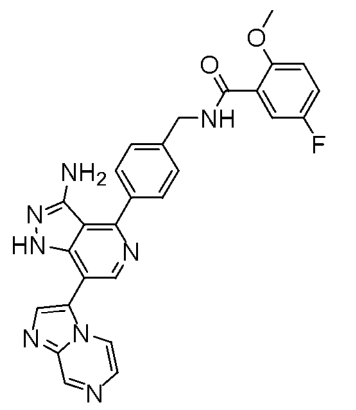

,

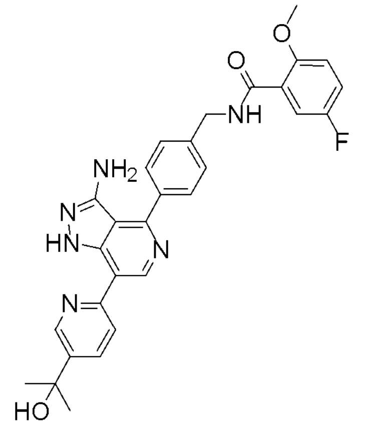

,

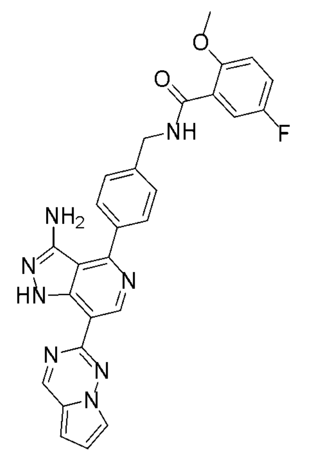

,

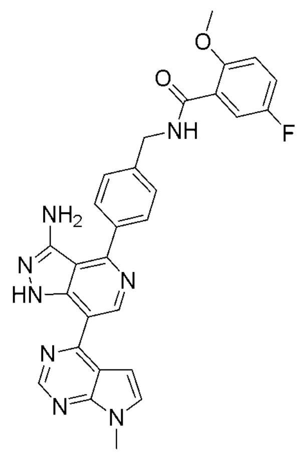

,

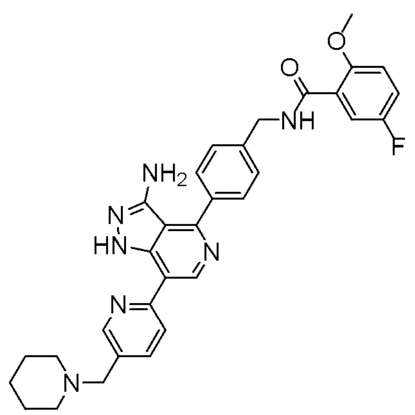

,

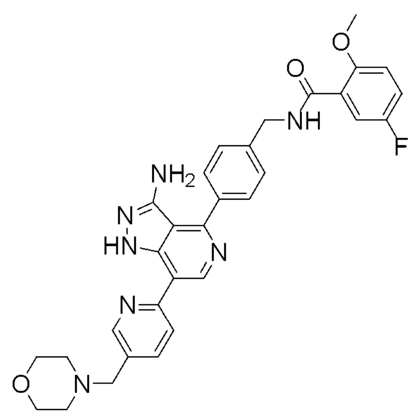

,

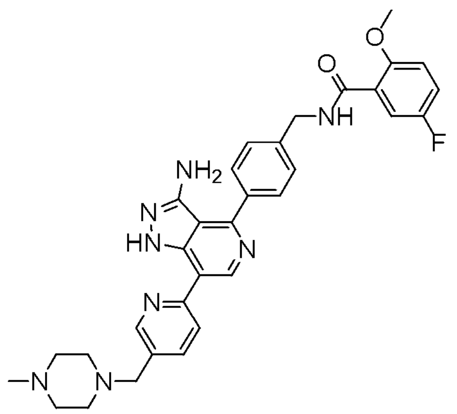
,
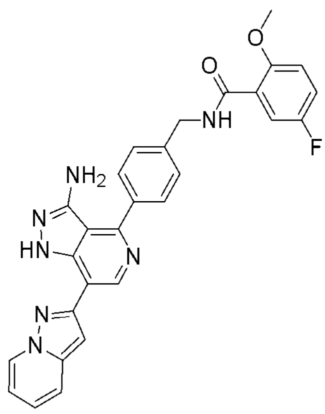
,
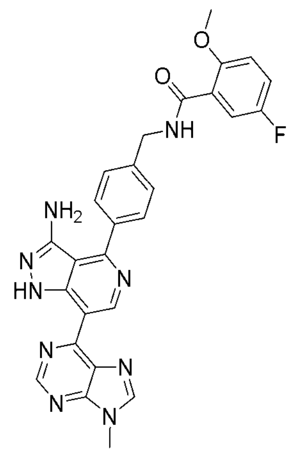
,
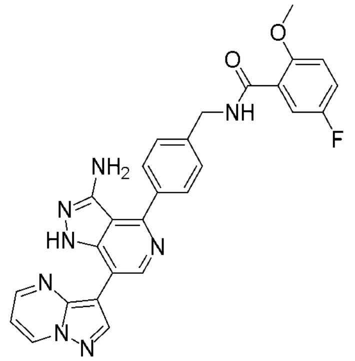
,
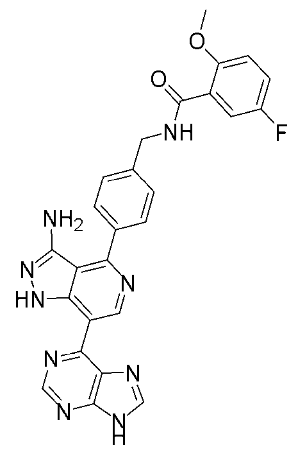
,
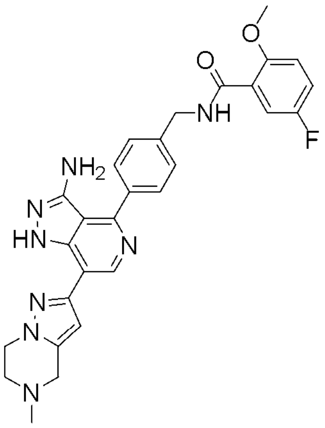
,
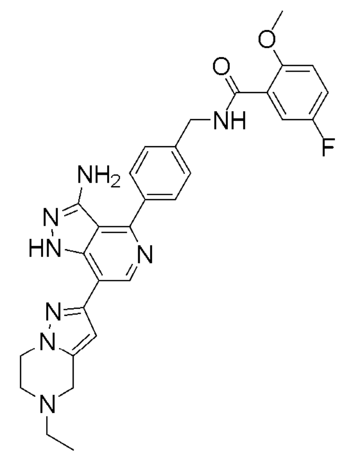
,
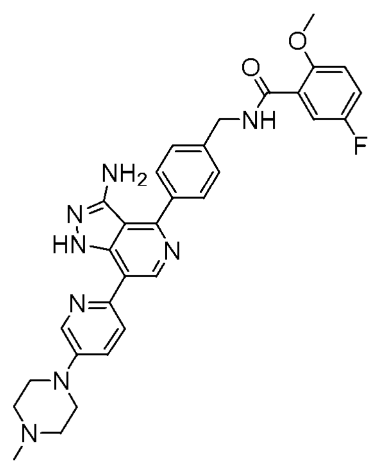
,
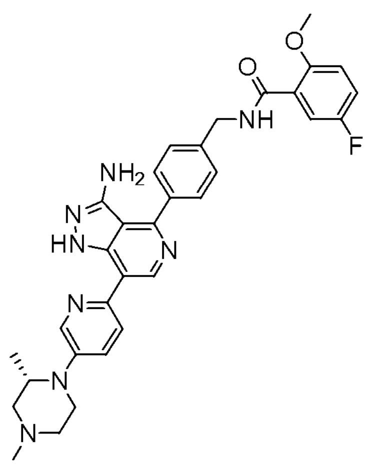
,

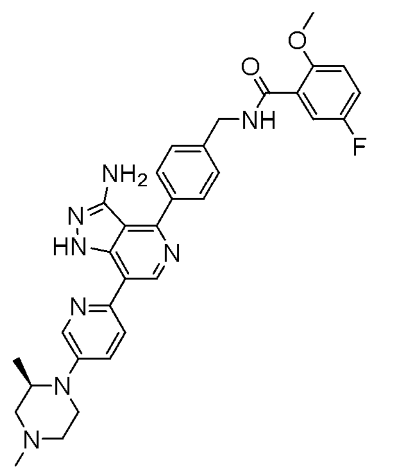
,
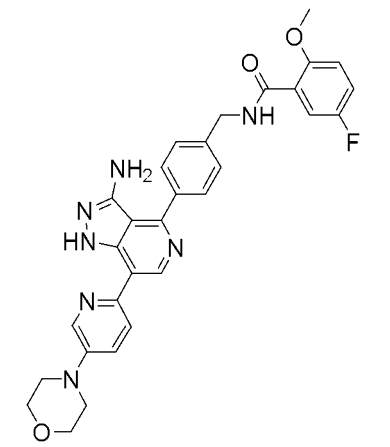
,
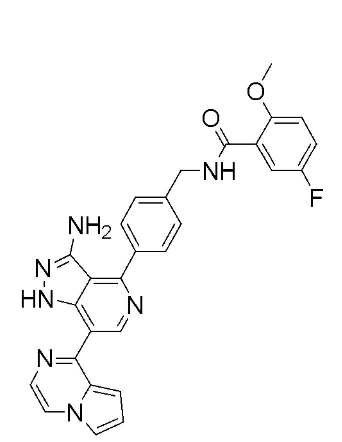
,

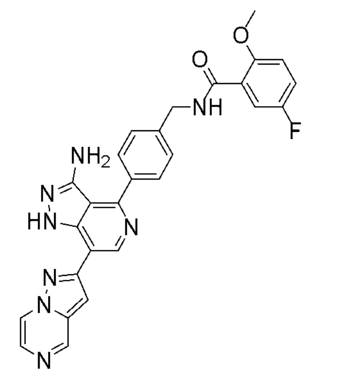
,
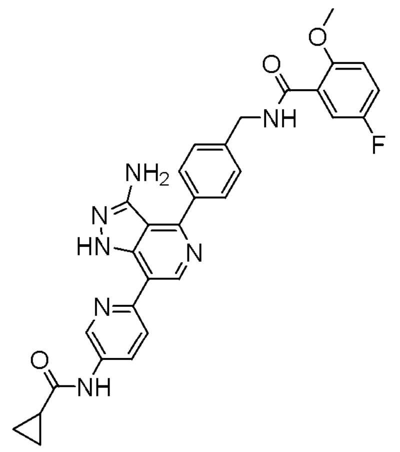
,
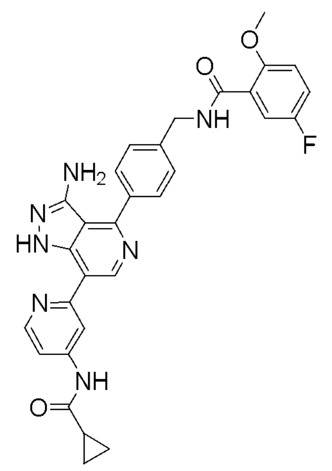
,

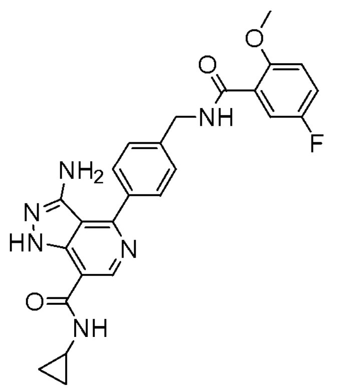
,
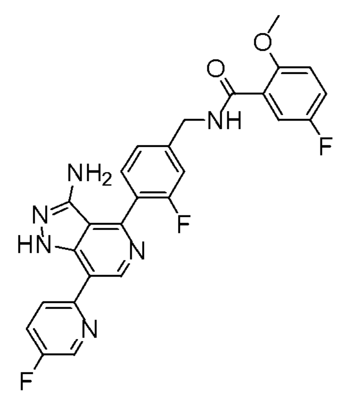
,
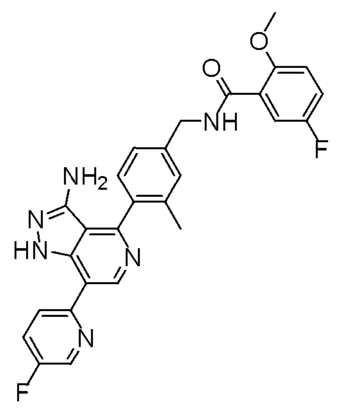
,

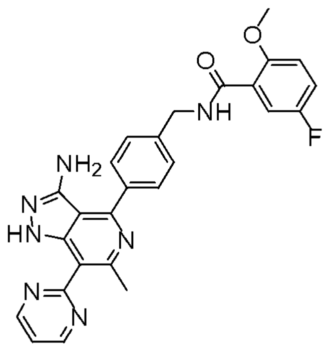,
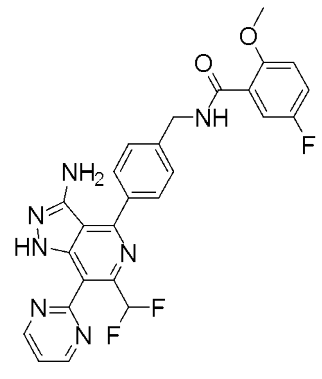,
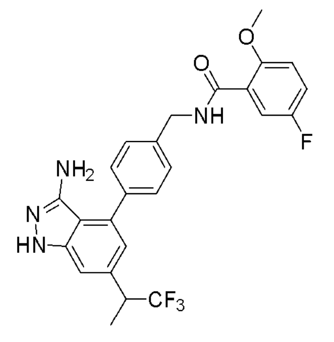,

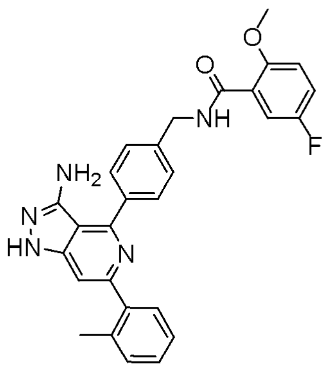,
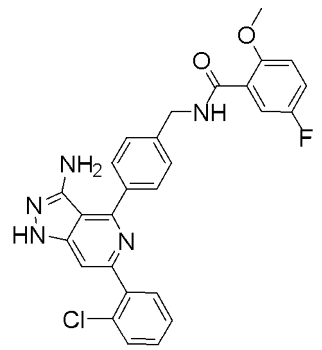,
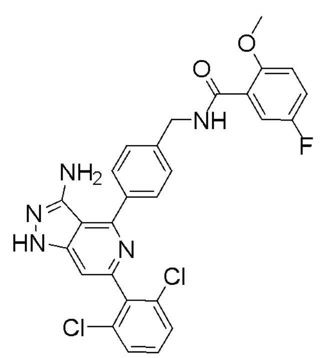,

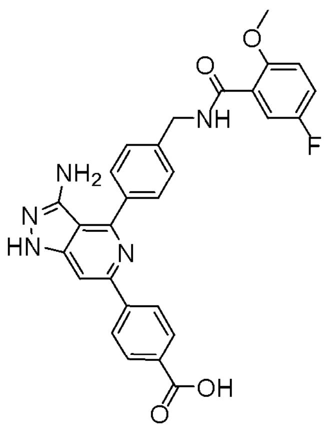,
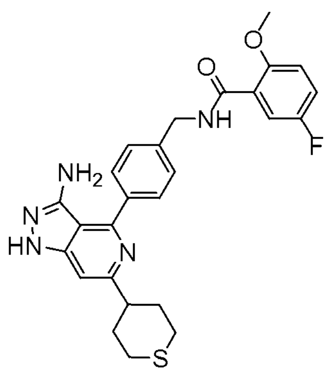,
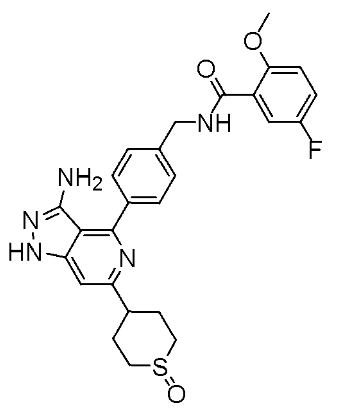,

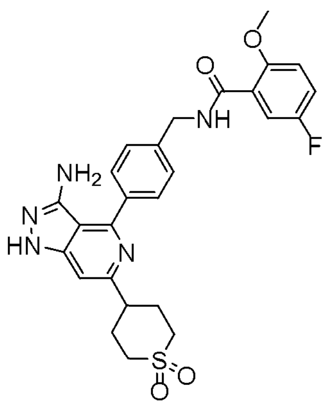,
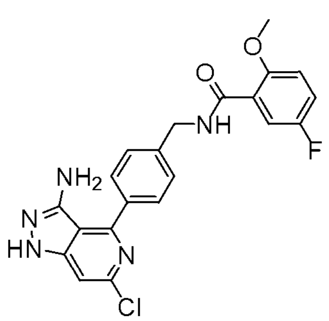,
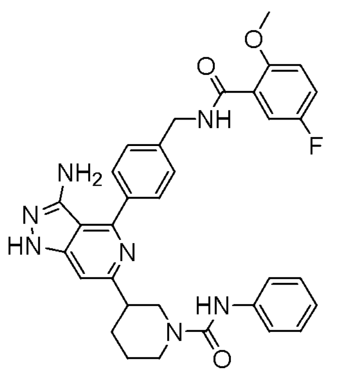,

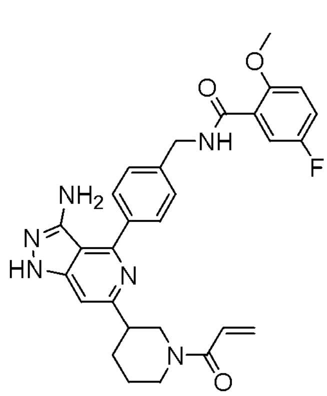,
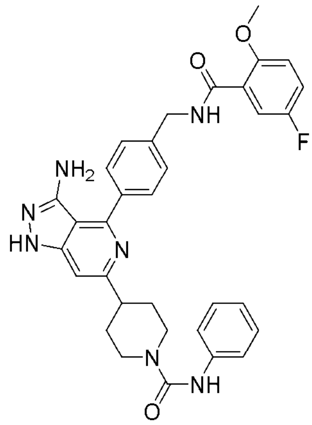,
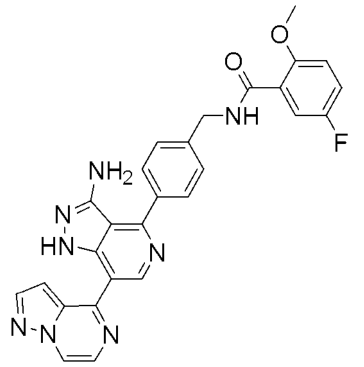,

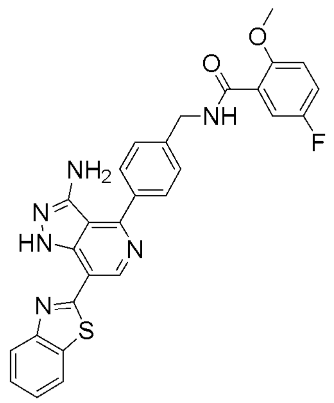,
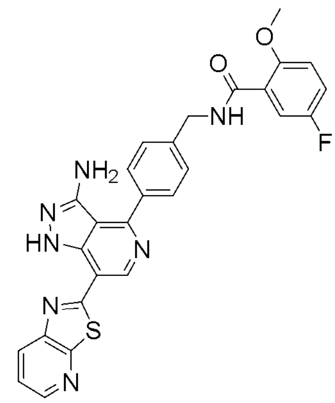,
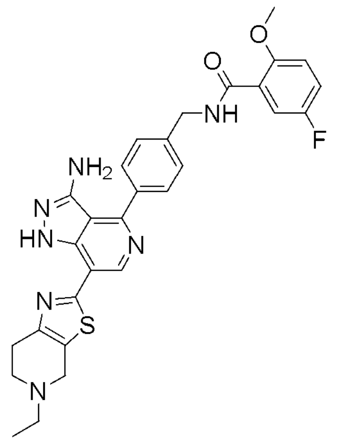,

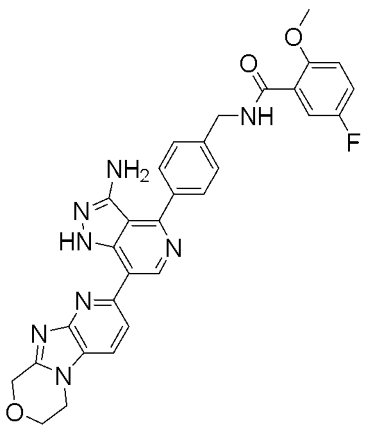,
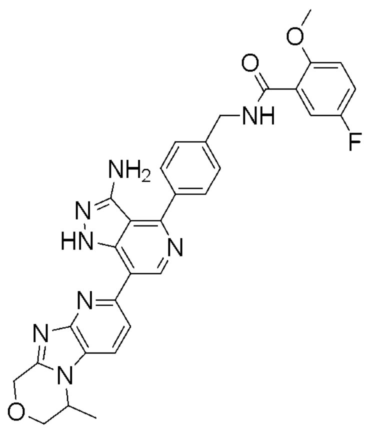,
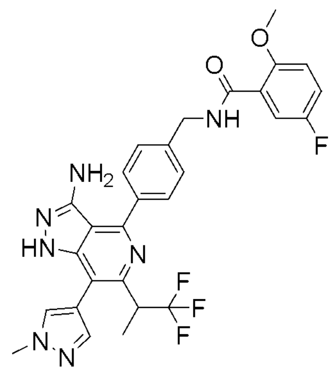,

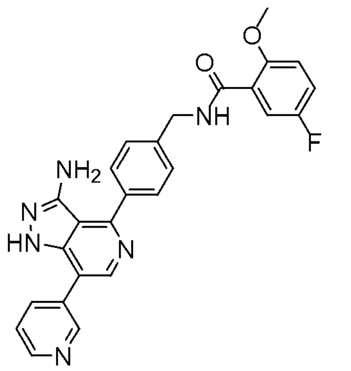
,
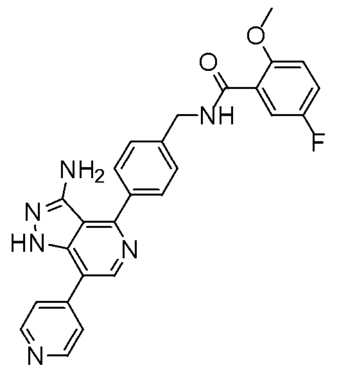
,
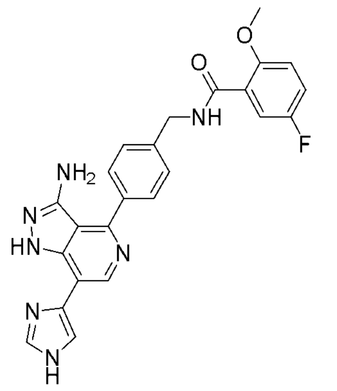
,
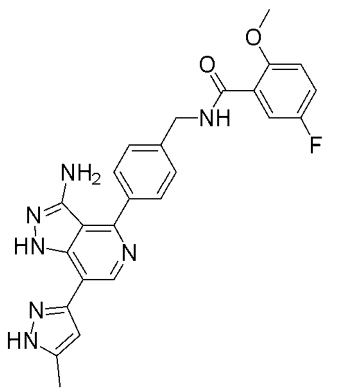
,
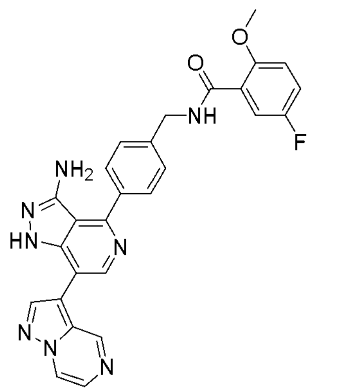
,
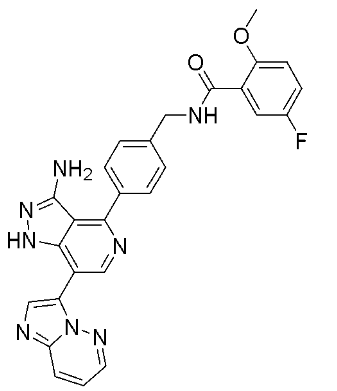
,
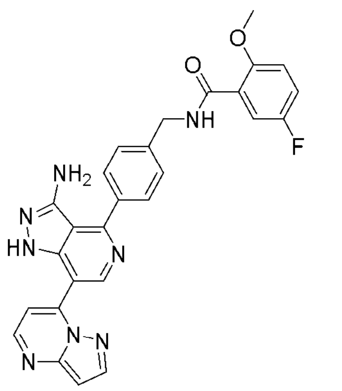
,
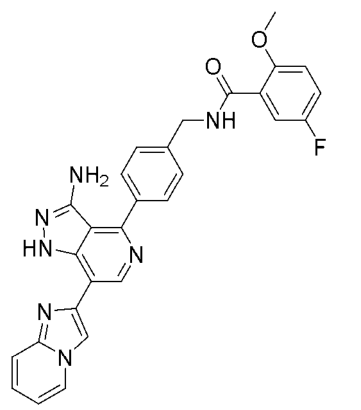
,
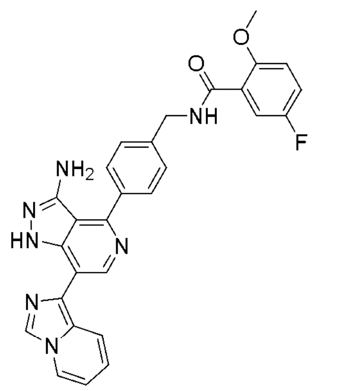
,

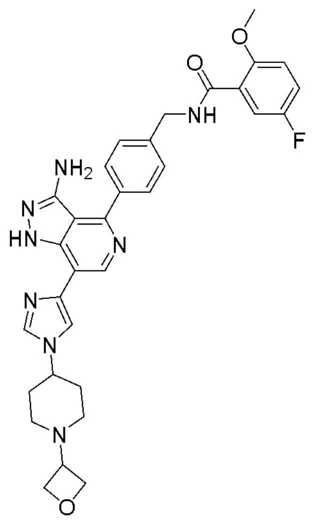,
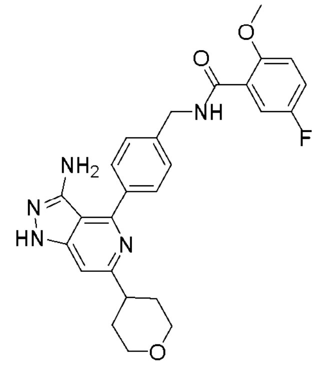,
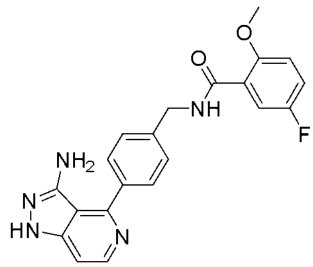,

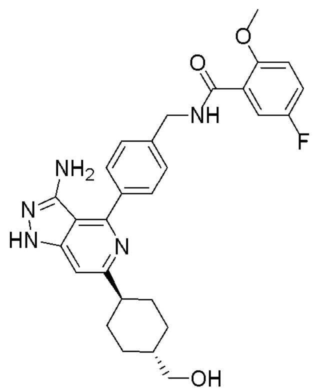,
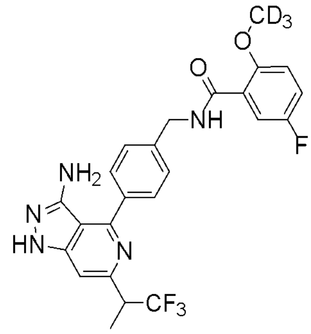,
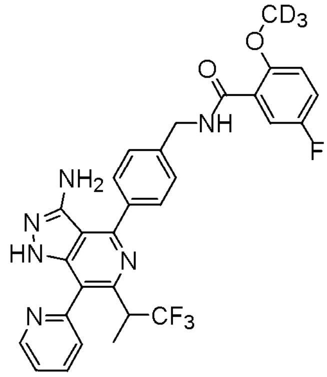,

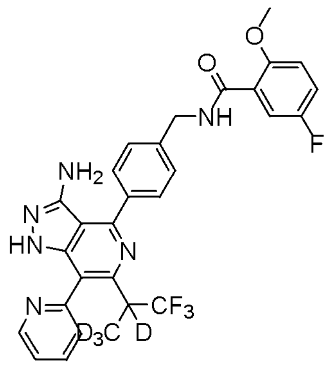,
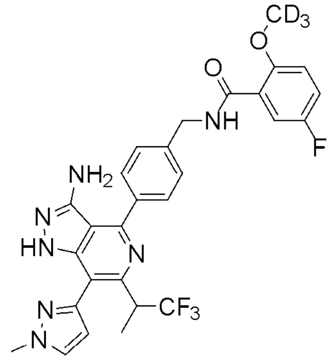,
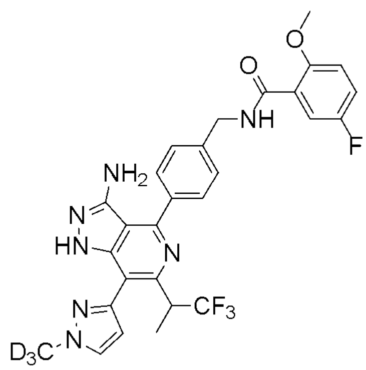,

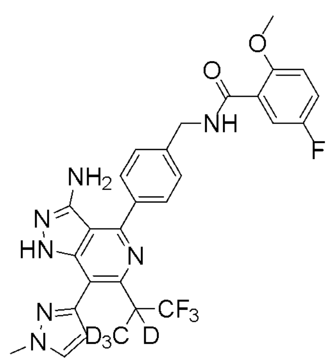,
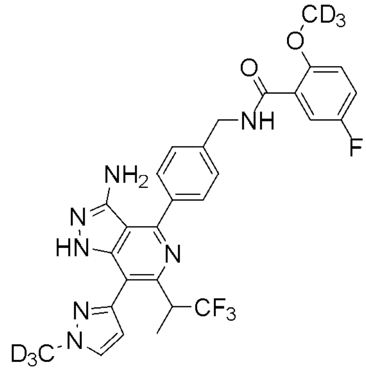,
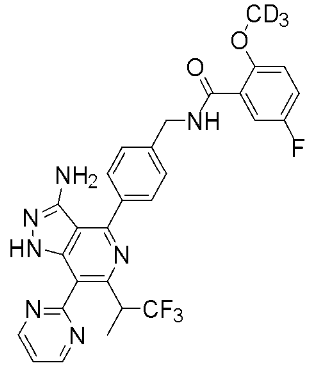,

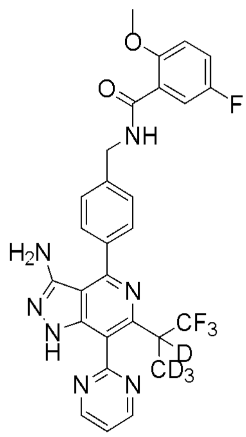
,
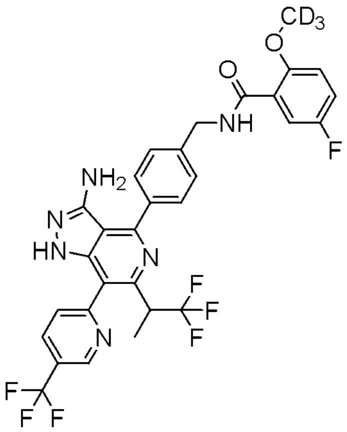
,
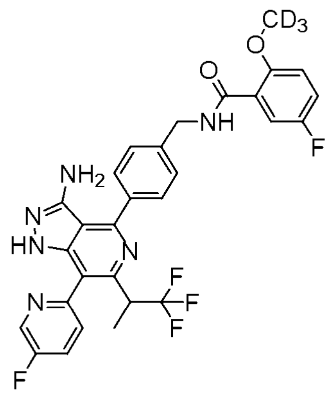
,

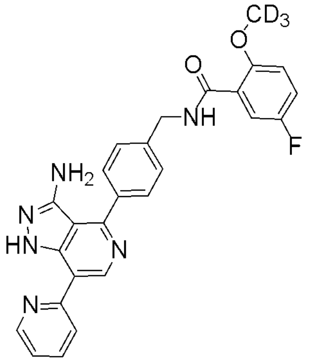
,
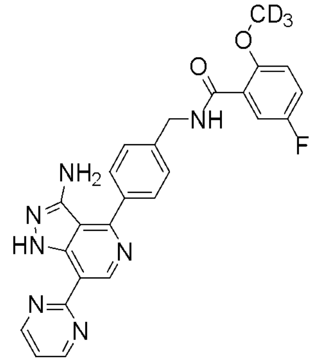
,
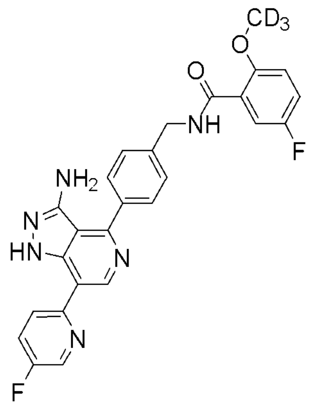
,

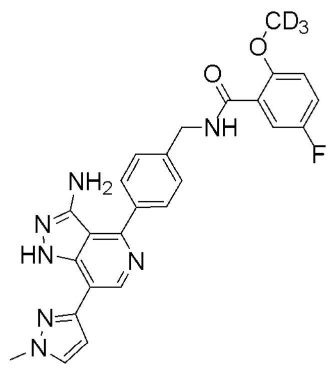
,
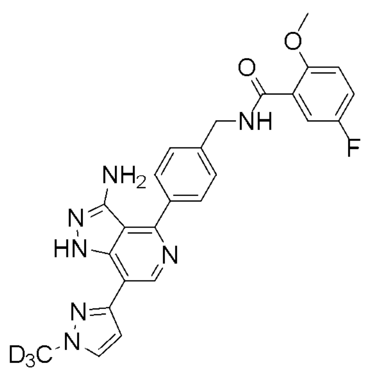
,
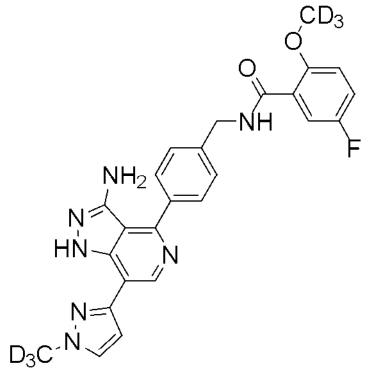
,

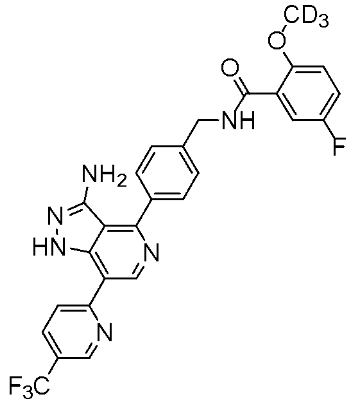
,
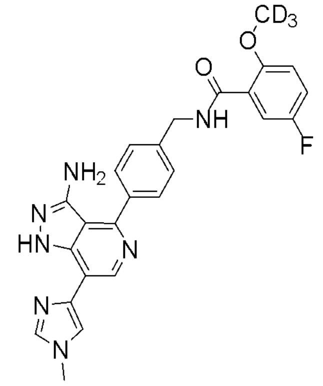
,
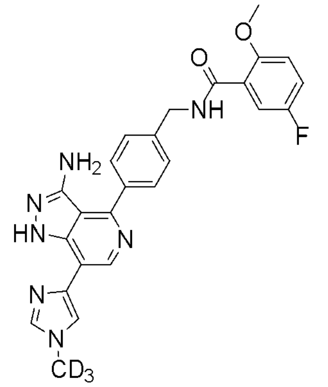
,

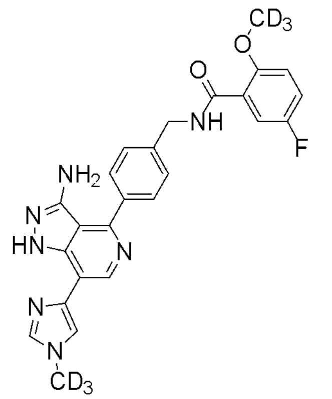
,
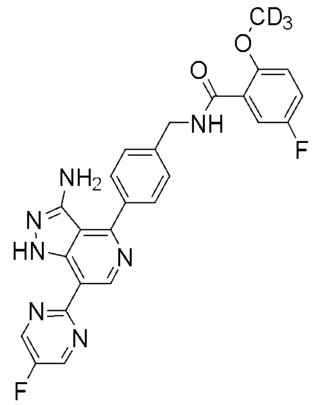
,
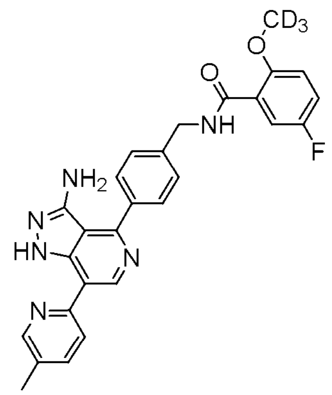
,
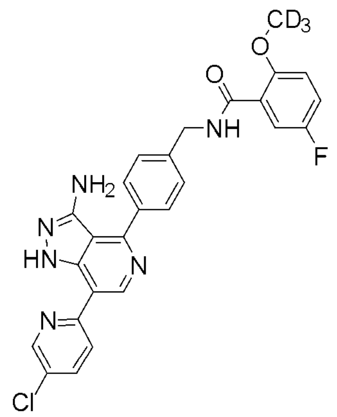
,
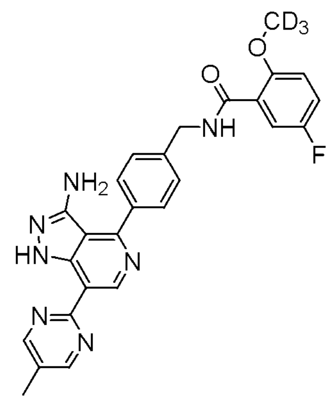
,
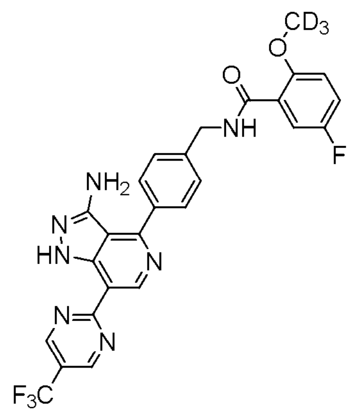
,
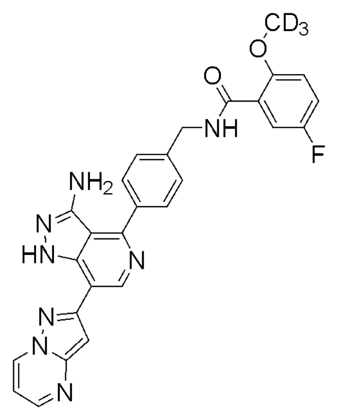
,
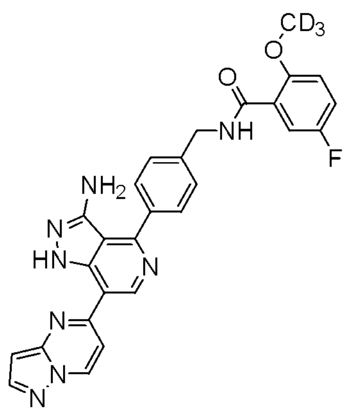
,
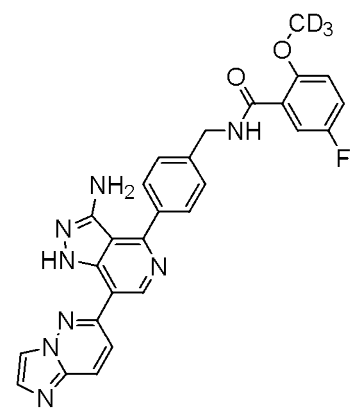
,

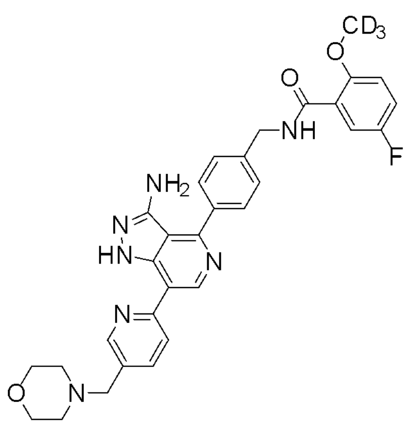
,
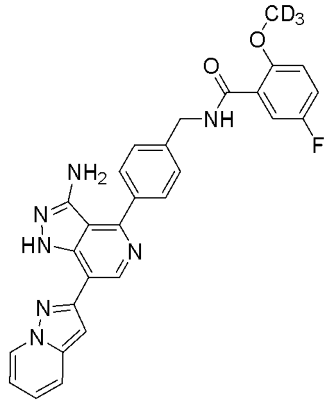
,
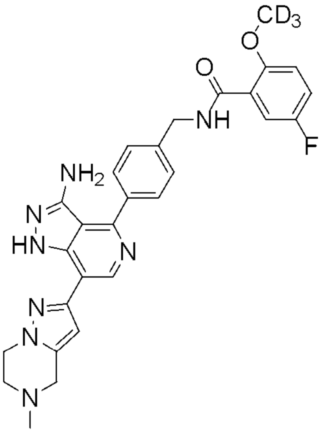
,

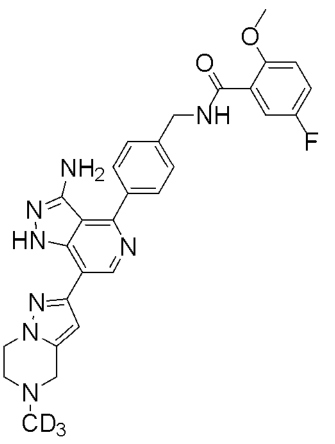
,
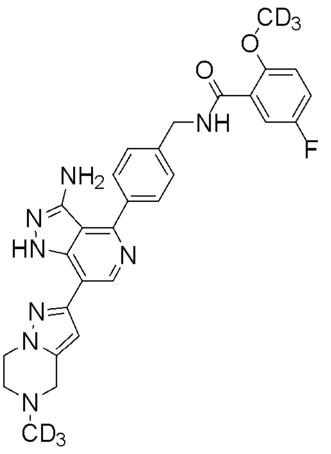
,
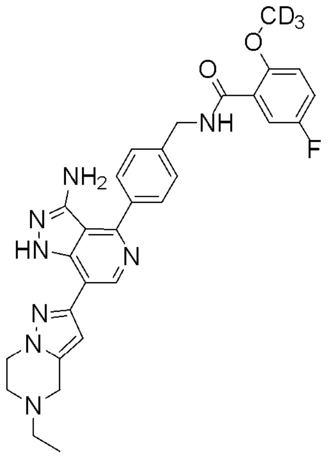
,

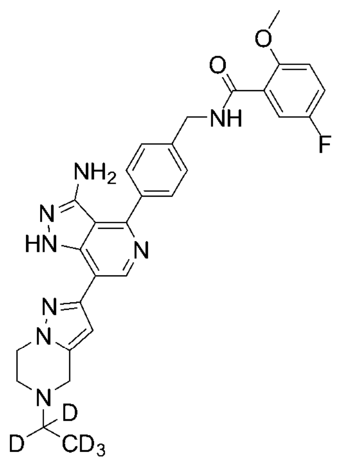
,
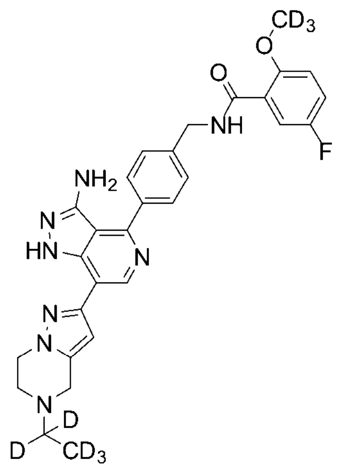
,
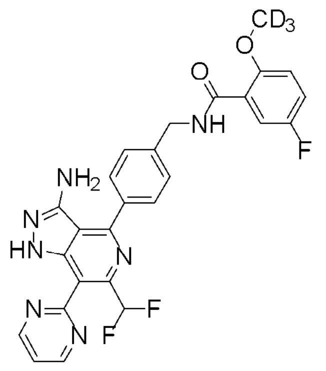
,

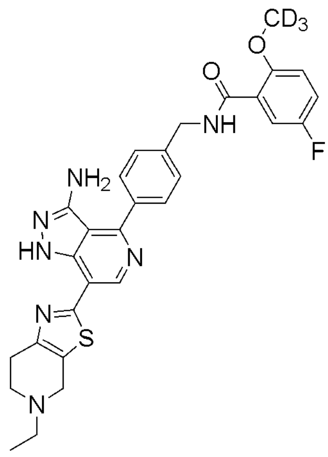,
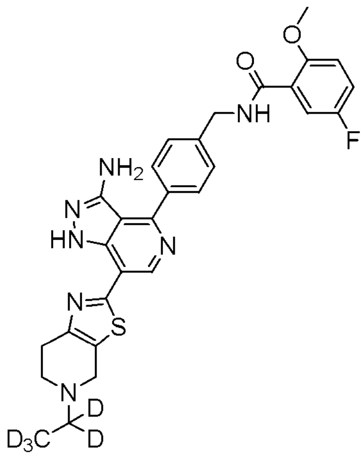,
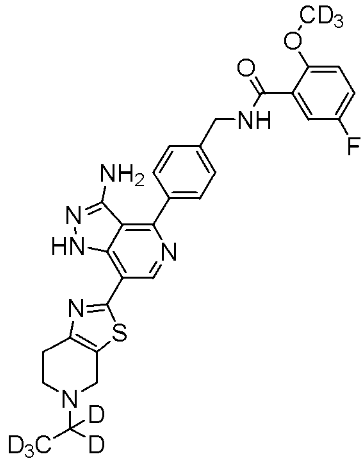,

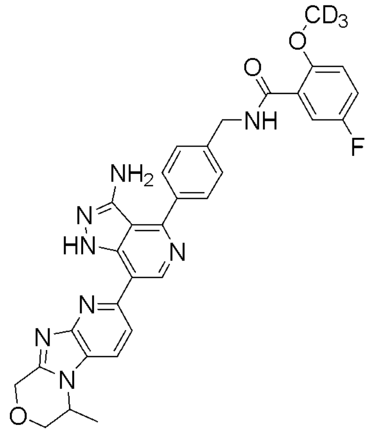,
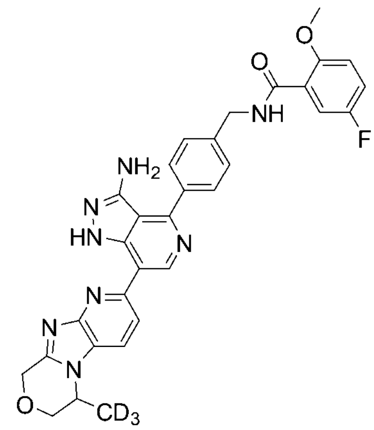,
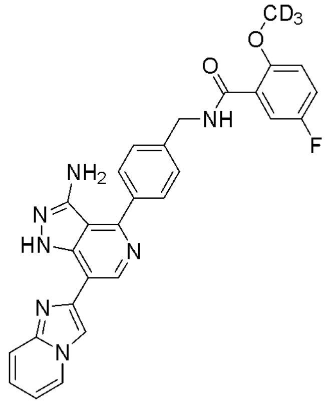,

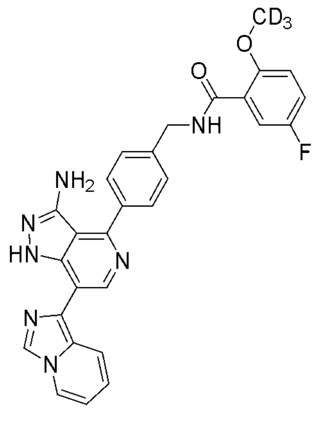,
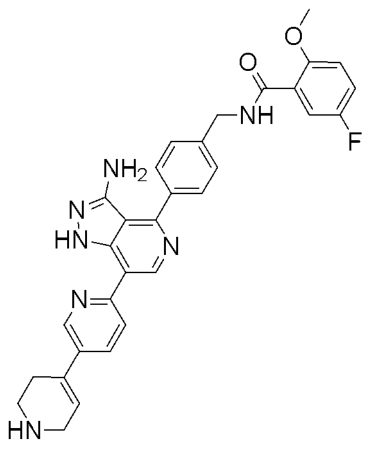,
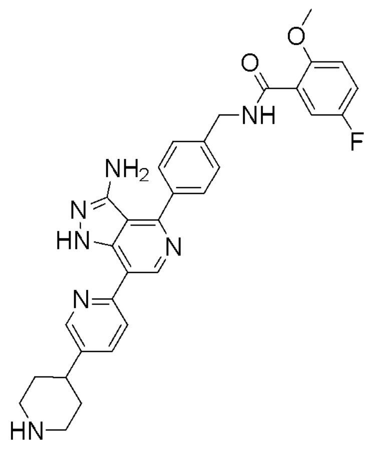,

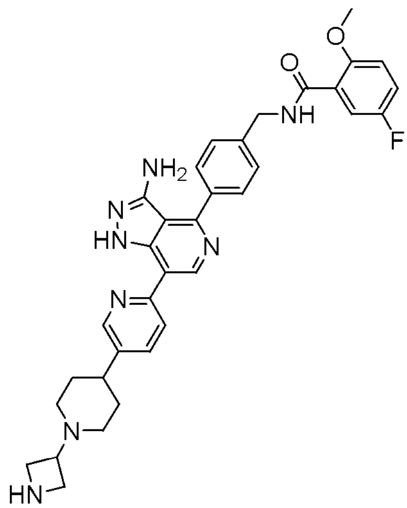

,

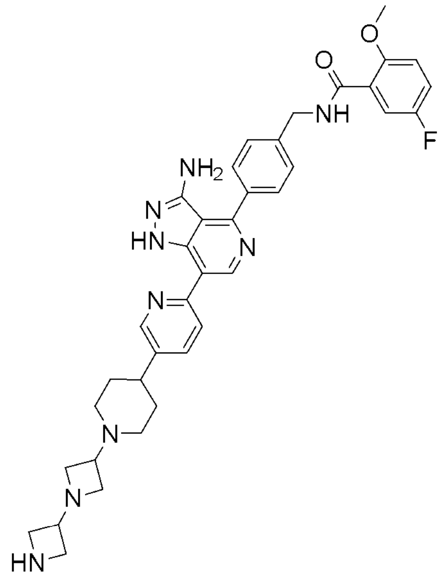

,

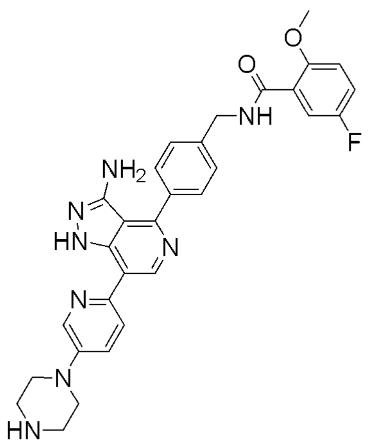

,

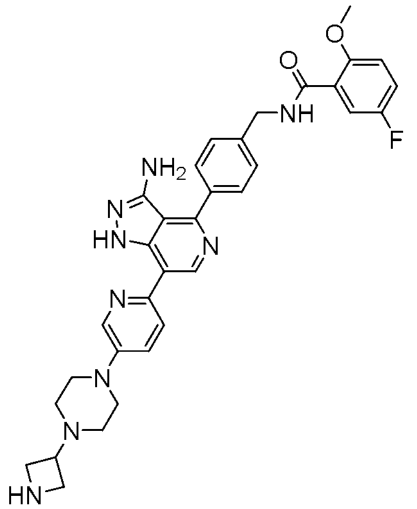

,

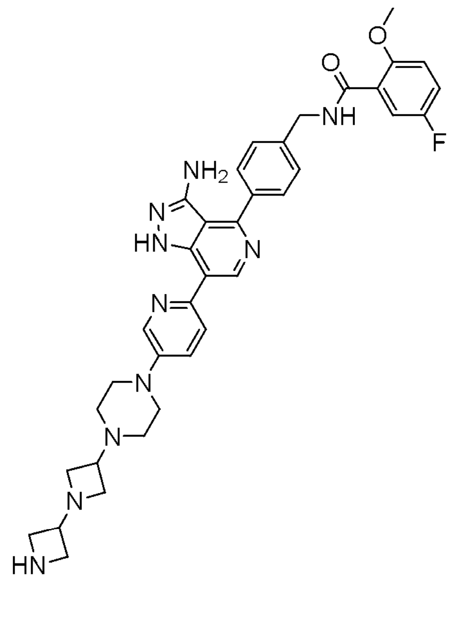

,

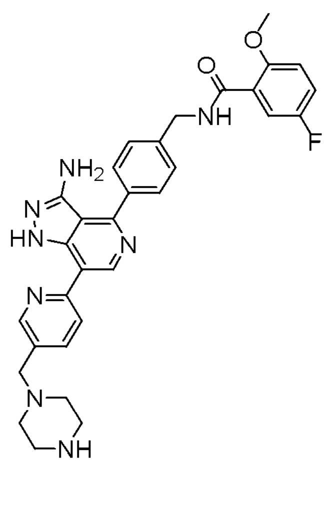

,

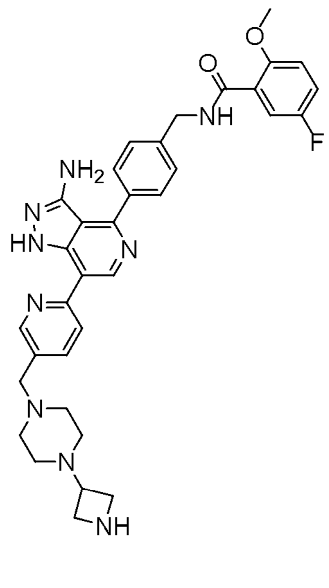

,

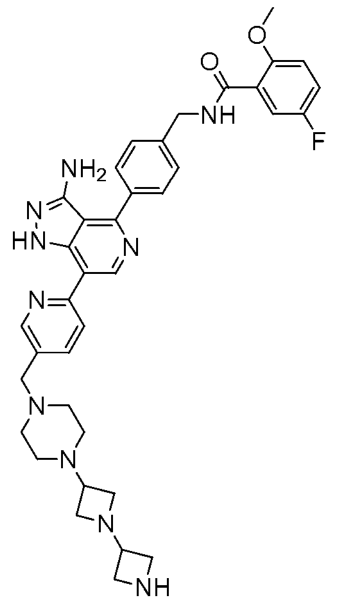,
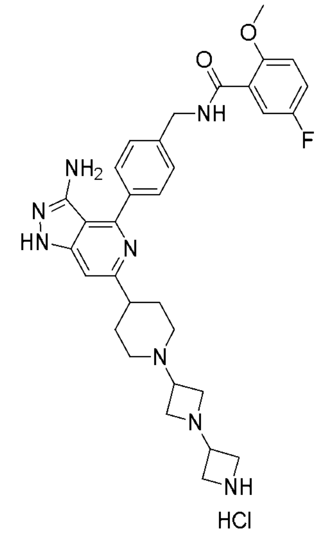,
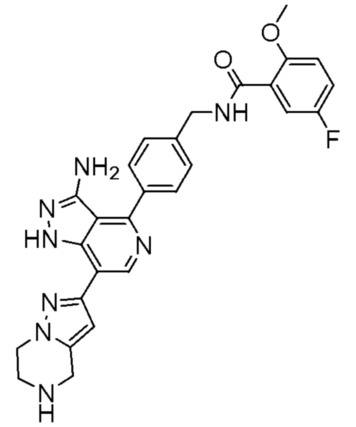,

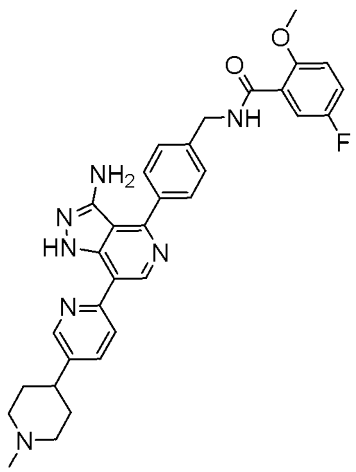,
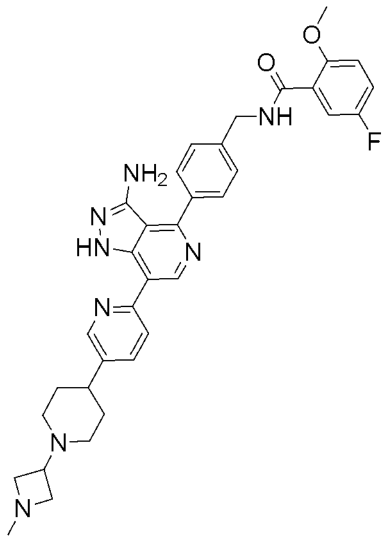,
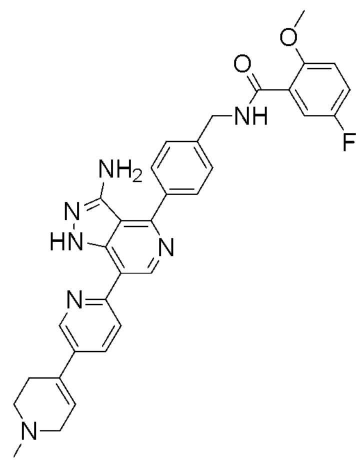,

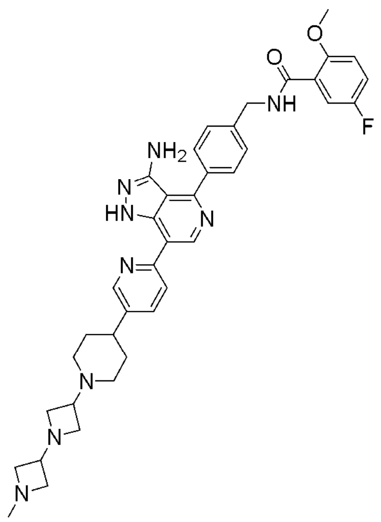,
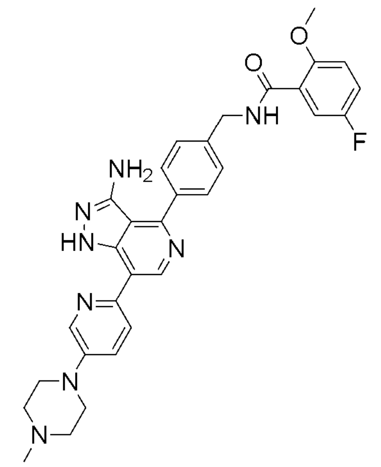,
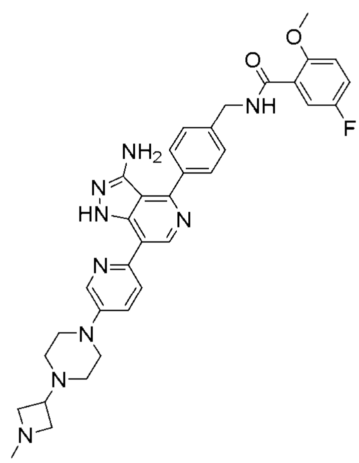,

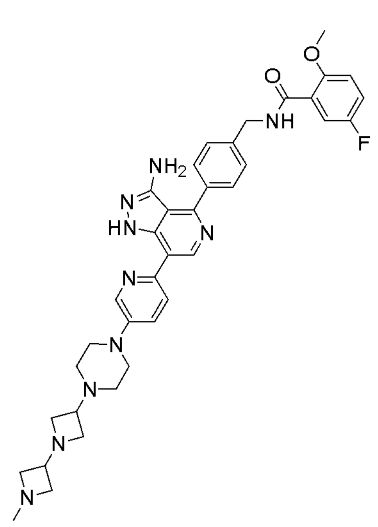
,
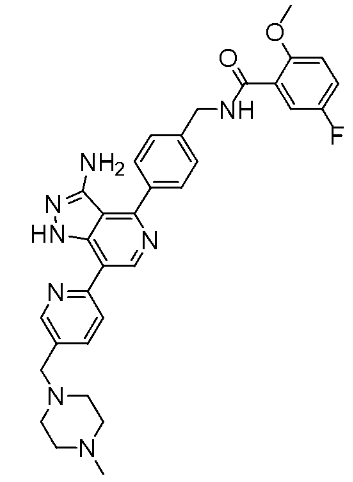
,
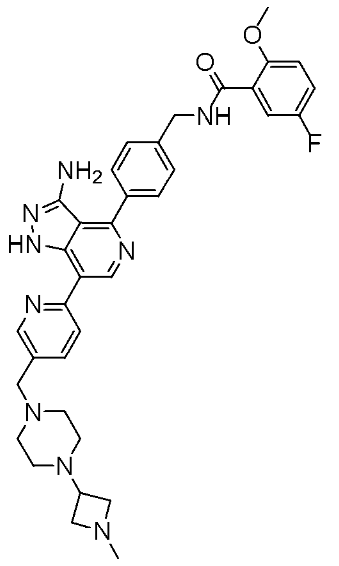
,

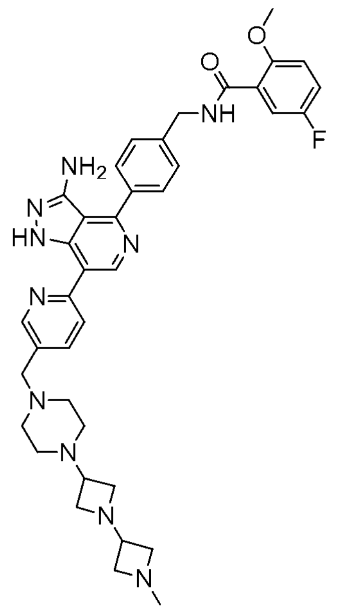
,
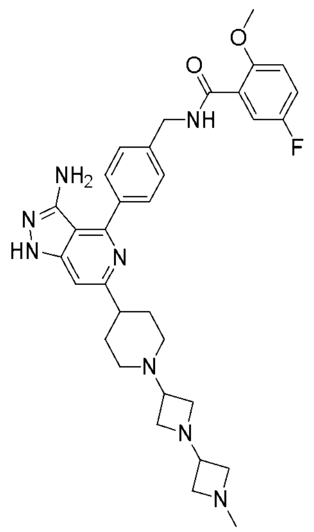
,
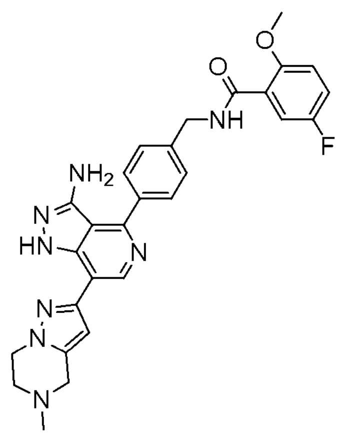
,

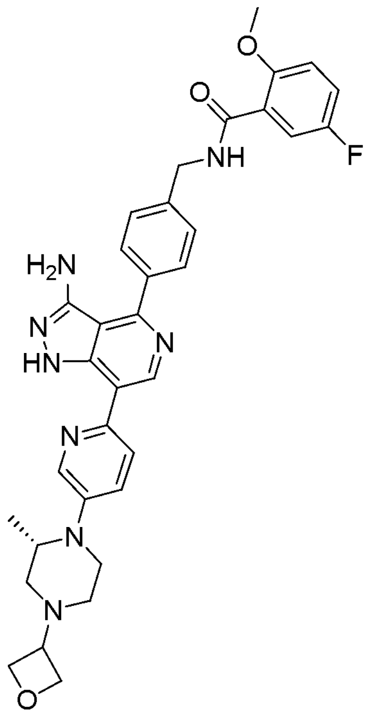

,

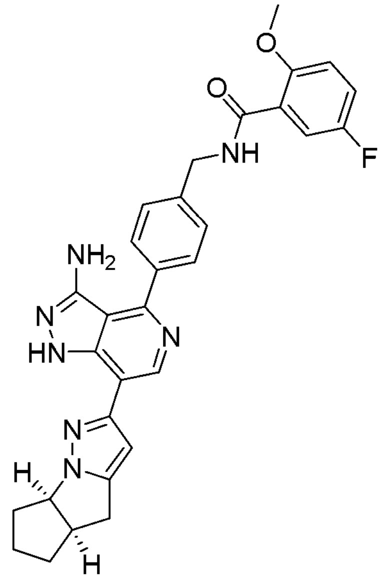

,

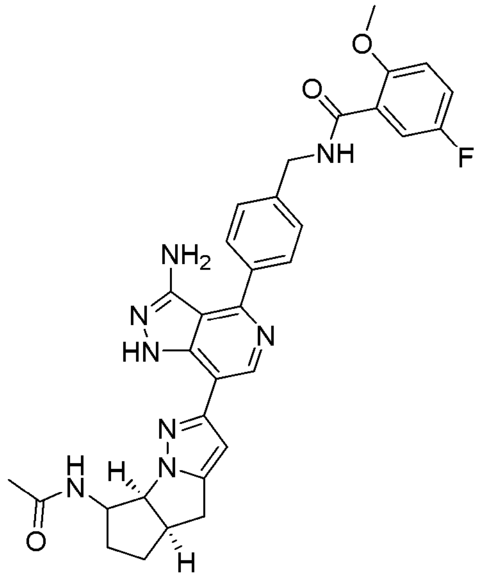

,

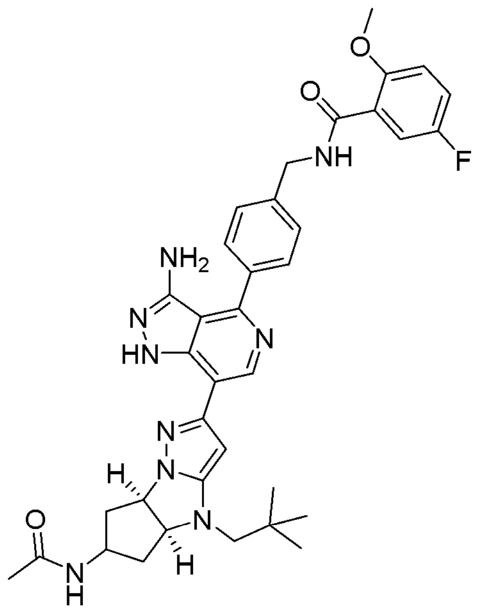

,

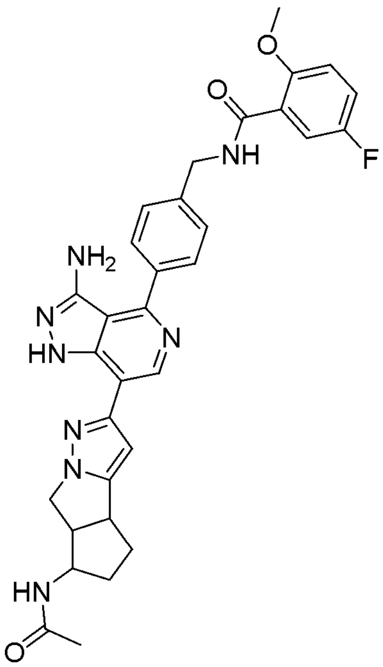
,
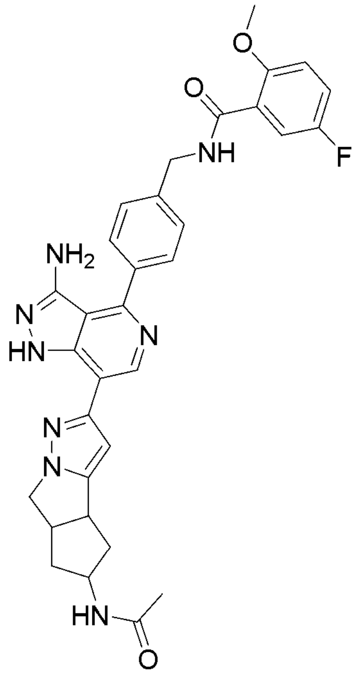
,
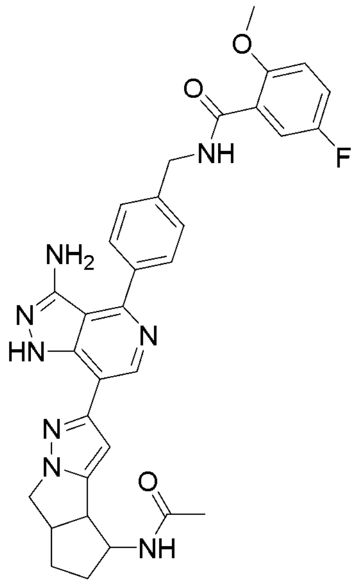

and

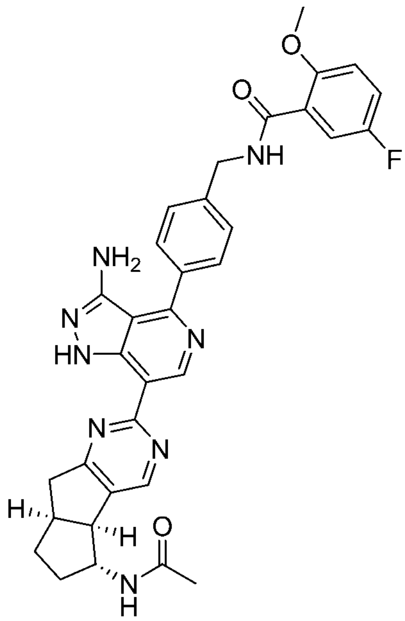
.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

"Tautomer" refers to an isomer in which one functional group in a compound changes its structure into another functional group, wherein the compound and the isomer can quickly convert between each other, thus being in dynamic equilibrium; this two isomers are called tautomers.

It will be understood by those skilled in the art that the organic compounds can form complexes with solvents in which they are reacted or from which they are precipitated or crystallized. These complexes are known as "solvates."

When the solvent is water, the complex is known as "hydrate." The present disclosure encompasses all solvates of the compounds of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds of the present disclosure may be in an amorphous or a crystalline form (crystal form or polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes, which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^2H$, $^3H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., $^3H$ and $^{14}C$), can be used for the measurement of the distribution of drug and/or substrate in tissue. Tritium, which is $^3H$ and carbon-14, which is $^{14}C$ isotope, are yet alternative, because they are easy to prepare and detect. Furthermore, replaced by heavier isotopes, such as deuterium, which is $^2H$, may provide therapeutic benefits due to the higher metabolic stability, such as prolonging the half-life in vivo or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects in vivo by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

The prodrugs are any covalently bonded compounds of the present disclosure, which release the parent compound in vivo when the prodrug is administered to a patient. Prodrugs are typically prepared by modifying functional groups in such a way that the modifications can be cleaved either by routine manipulation or decompose in vivo to yield the parent compound. Prodrugs include, for example, compounds of the present disclosure wherein the hydroxyl, amino or sulfhydryl groups are bonded to any group that, when administered to a patient, cleaves to form the hydroxyl, amino or sulfhydryl groups. Thus, representative examples of prodrugs include (but are not limited to) the acetate/acetamide, formate/formamide and benzoate/benzamide derivatives of the hydroxyl, sulfhydryl or amino functional groups of the compounds of formula (I). Furthermore, in the case of carboxylic acid (-COOH), esters such as methyl esters and ethyl esters, etc. can be employed. The ester itself may be active in their own and/or hydrolyzable under in vivo conditions in the human body. Suitable pharmaceutically acceptable in vivo hydrolysable ester groups include those groups that can readily

break down in the human body to release the parent acids or salts thereof.

Pharmaceutical compositions, formulations and kits

In another aspect, the present disclosure provides a pharmaceutical composition comprising a compound disclosed herein (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other suitable containers) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, for example, over 3 months, 6 months, 1 year, 2 years, 3 years, 5 years, etc, or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present disclosure may be further delivered using a variety of dosing methods. For example, in certain embodiments, the pharmaceutical composition may be given as a bolus, e.g., in order to raise the concentration of the compound in the blood to an effective level rapidly. The placement of the bolus dose depends on the desired systemic levels of the active ingredient, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk

powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and yet alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavours and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or formulation. All such known transdermal formulations and ingredients are included within the scope provided herein.

The compounds provided herein can also be administered by a transdermal device. Accordingly, transdermal administration may be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds disclosed herein can also be administered in sustained release forms or from sustained release drug delivery systems. A description of representative sustained release materials may be found in Remington's Pharmaceutical Sciences.

The present disclosure also relates to the pharmaceutically acceptable formulations of a compound disclosed herein. In one embodiment, the formulation comprises water. In another embodiment, the formulation comprises a cyclodextrin derivative. The most common cyclodextrins are $\alpha$-, $\beta$- and $\gamma$-cyclodextrins consisting of 6, 7 and 8 $\alpha$-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether $\beta$-cyclodextrin, e.g., for example, sulfobutyl ether $\beta$-cyclodextrin, also known as Captisol. See, e.g., U.S. 5,376,645. In certain embodiments, the formulation comprises hexapropyl-$\beta$-cyclodextrin (e.g., 10-50% in water).

## Indications

In another aspect, provided is the use of the compound of formula (I) disclosed herein (including all individual embodiments and generic subsets disclosed herein) or a tautomer, stereoisomer, prodrug, crystal form, pharmaceutically acceptable salt, hydrate or solvate thereof as a drug.

The compounds of the present disclosure exhibit potent and selective BTK inhibition. For example, the compounds of the present disclosure exhibit nanomolar potency against wild-type BTK and BTK kinase encoded by a BTK gene comprising a BTK kinase inhibitor resistance mutation (including, for example, BTK C481S, BTK T474I, or BTK L528W).

In some embodiments, the compounds of the present disclosure selectively target BTK kinase. For example, the compounds of the present disclosure can selectively target BTK kinase relative to another kinase or non-kinase target. In some embodiments, the compounds of the present disclosure can selectively target BTK kinase relative to one or more kinases of BRK, CSK, ERBB4, FYN, MEK1, MEK2, TEC, TXK, YES1, BMX, BLK, EGFR, ITK, SRC, JAK1, JAK2 and JAK3. In some other embodiments, the compounds of the present disclosure can selectively target BTK kinase relative to one or more kinases of TEC, EGFR, and ITK. In some other embodiments, the compounds of the present disclosure can selectively target BTK kinase relative to one or more kinases of TEC and EGFR.

In some embodiments, the compounds of the present disclosure exhibit at least 30-fold selectivity for BTK kinase relative to another kinase. For example, relative to another kinase, the compounds of the present disclosure exhibit at least 40-fold selectivity for BTK kinase; at least 50-fold selectivity; at least 60-fold selectivity; at least 70-fold selectivity; at least 80-fold selectivity; at least 90-fold selectivity; at least 100-fold selectivity; at least 200-fold selectivity; at least 300-fold selectivity; at least 400-fold selectivity; at least 500-fold selectivity; at least 600-fold selectivity; at least 700-fold selectivity; at least 800-fold selectivity; at least 900-fold selectivity; or at least 1000-fold selectivity. In some embodiments, the compounds of the present disclosure exhibit at least 100-fold selectivity for BTK kinase relative to another kinase. In some embodiments, the selectivity for BTK kinase relative to another kinase is measured in a cellular assay (e.g., a cellular assay provided herein).

In some embodiments, the compounds of the present disclosure can be used to treat BTK kinase-mediated diseases caused by covalent BTK kinase inhibitor resistance mutations. In some more specific embodiments, the covalent BTK kinase inhibitor resistance mutations are C481S, C481F, C481Y, C481R, C481T, C481G and C481W.

In some embodiments, the compounds of the present disclosure can be used to treat BTK kinase-mediated diseases caused by non-covalent BTK kinase inhibitor resistance mutations. In some more specific embodiments, the non-covalent BTK kinase inhibitor resistance mutations are V416L, A428D, M437R, T474I and L528W.

In some embodiments, the BTK kinase inhibitor resistance mutation is one or more point mutations in the BTK gene. In some embodiments, one or more point mutations in the BTK gene result in the translation of a BTK protein with one or more amino acid substitutions at one or more of the following amino acid positions: 117, 316, 416, 428, 437, 474, 481, 528, 560, 562 and 601. In some embodiments, one or more point mutations in the BTK gene result in the translation of a BTK protein with one or more of the following amino acid substitutions: T117P, T316A, V416L, A428D, M437R, T474I, T474M, T474S, C481S, C481F, C481T, C481G, C481R, L528W, P560L, R562W, R562G and F601L.

The compounds of the present disclosure are useful for treating diseases and conditions that are treated with BTK kinase inhibitors, such as diseases and conditions associated with BTK, such as proliferative diseases, such as cancers, including hematological cancers and solid tumors, and inflammatory conditions, immune diseases or fibrosis.

In some embodiments of any of the methods or uses described herein, the cancer (e.g., a BTK-related cancer) is a hematological cancer. In some embodiments of any of the methods or uses described herein, the cancer (e.g., a BTK-related cancer) is a solid tumor. In some embodiments of any of the methods or uses described herein, the cancer (e.g., a BTK-related cancer) is a B-cell malignancy. In some embodiments of any of the methods or uses described herein, the cancer (e.g., a BTK-related cancer) is Hodgkin's lymphoma, diffuse large B-cell lymphoma, follicular lymphoma, mantle cell lymphoma, marginal zone lymphoma (e.g., splenic marginal zone lymphoma, extranodal marginal zone B-cell lymphoma), Burkitt's lymphoma, Waldenstrom's macroglobulinemia (lymphoplasmacytic lymphoma), primary central nervous system lymphoma, small lymphocytic lymphoma, chronic lymphocytic lymphoma, acute lymphocytic leukemia, B-cell prolymphocytic leukemia, precursor B-cell lymphoblastic leukemia, hairy cell leukemia, acute myeloid leukemia, chronic myeloid leukemia, multiple myeloma, plasma cell myeloma, plasmacytoma, bone cancer, bone metastasis, breast cancer, gastroesophageal cancer, pancreatic cancer, ovarian cancer, prostate cancer, lung cancer, colon cancer, head and neck cancer or glioma.

In some embodiments, the hematological cancer is selected from leukemia, non-Hodgkin's lymphoma, Hodgkin's lymphoma or myeloma, for example, acute lymphocytic leukemia (ALL), acute myeloid leukemia (AML), acute promyelocytic leukemia (APL), chronic lymphocytic lymphoma (CLL), chronic myeloid leukemia (CML), chronic myelomonocytic leukemia (CMML), chronic neutrophilic leukemia (CNL), acute undifferentiated leukemia (AML), anaplastic large cell lymphoma (ALCL), prolymphocytic leukemia (PML), juvenile myelomonocytic leukemia (JMML), adult T-cell leukemia (ALL), acute myeloid leukemia with trilineage myelodysplasia (AML/TMDS), mixed lineage leukemia (MLL), myelodysplastic syndrome (MDS), myeloproliferative disorder (MPD), diffuse large B-cell lymphoma, follicular lymphoma, mantle cell lymphoma, marginal zone lymphoma (e.g., splenic marginal zone lymphoma, extranodal marginal zone B-cell lymphoma), Burkitt's lymphoma, Waldenstrom's macroglobulinemia (lymphoplasmacytic lymphoma), primary central nervous system lymphoma, small lymphocytic lymphoma, precursor B-cell lymphoblastic leukemia, hairy cell leukemia, mucosa-associated lymphoid tissue lymphoma, plasma cell myeloma, plasmacytoma, and multiple myeloma. Other

examples of hematological cancers include myelodysplastic disorders (MPD), such as polycythemia vera (PV), essential thrombocytopenia (ET), and idiopathic primary myelofibrosis (IMF/IPF/PMF). In some embodiments, the hematological cancer is mantle cell lymphoma, chronic lymphocytic lymphoma, small lymphocytic lymphoma, Waldenstrom's macroglobulinemia, or marginal zone lymphoma.

In some embodiments, the solid tumor is selected from bone cancer, bone metastasis, breast cancer, gastroesophageal cancer, pancreatic cancer, ovarian cancer, prostate cancer, lung cancer, colon cancer, and head and neck cancer.

In some embodiments, the immune disease is selected from arthritis, multiple sclerosis, osteoporosis, irritable bowel syndrome, inflammatory bowel disease, Crohn's disease, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, Guillain-Barré syndrome, acute disseminated encephalomyelitis, Addison's disease, opsoclonus syndrome, ankylosing spondylitis, antiphospholipid antibody syndrome, aplastic anemia, autoimmune hepatitis, celiac disease, Goodpasture's syndrome, idiopathic thrombocytopenic purpura, optic neuritis, scleroderma, primary biliary cirrhosis, Reiter's syndrome, Takayasu's arteritis, warm autoimmune hemolytic anemia, Wegener's granulomatosis, psoriasis, Alopecia universalis, Behcet's disease, chronic fatigue, dysautonomia, endometriosis, interstitial cystitis, neuromyotonia, scleroderma and vulvodynia, asthma, appendicitis, blepharitis, bronchiolitis, bronchitis, bursitis, cervicitis, cholangitis, cholecystitis, colitis, conjunctivitis, colitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, inflammatory enteritis, suppurative inflammation, laryngitis, mastitis, meningitis, myelitis, myocarditis, myositis, nephritis, oophoritis, orchitis, osteitis, otitis, pancreatitis, mumps, pericarditis, peritonitis, pharyngitis, pleurisy, phlebitis, pneumonia, pneumonitis, proctitis, prostatitis, pyelonephritis, rhinitis, salpingitis, sinusitis, stomatitis, synovitis, tendinitis, tonsillitis, uveitis, vaginitis, vasculitis, vulvitis graft-versus-host disease, transplantation, transfusion, anaphylaxis, allergic reaction, type I hypersensitivity, allergic conjunctivitis, allergic rhinitis and atopic dermatitis.

In some embodiments, the inflammatory disease is selected from arthritis, asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis fibrositis, gastritis, gastroenteritis, hepatitis, hidradenitis suppurativa, laryngitis, mastitis, meningitis, osteomyelitis myocarditis, myositis, nephritis, oophoritis, orchitis, osteitis, otitis, pancreatitis, mumps, pericarditis, peritonitis, pharyngitis, pleurisy, phlebitis, pneumonia, lung disease, proctitis, prostatitis, pyelonephritis, rhinitis, salpingitis, sinusitis, stomatitis, synovitis, tendinitis, tonsillitis, uveitis, vaginitis, vasculitis and vulvitis.

In some embodiments, the autoimmune disease is selected from lupus and Sjögren's syndrome, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, Guillain-Barre syndrome, acute disseminated encephalomyelitis, Addison's disease, opsoclonus-myoclonus syndrome, ankylosing spondylitis, antiphospholipid antibody syndrome, aplastic anemia, autoimmune hepatitis, celiac disease, Goodpasture's syndrome, idiopathic thrombocytopenic purpura, optic neuritis, scleroderma, primary biliary cirrhosis, Reiter's syndrome, Takayasu's arteritis, hemolytic anemia, Wegener's granulomatosis, psoriasis, alopecia universalis, Behcet's disease, chronic fatigue, dysautonomia, endometriosis, interstitial cystitis, neuromyotonia, scleroderma and vulvodynia.

In some embodiments, the heteroimmune disease is selected from graft-versus-host disease, transplantation, transfusion, anaphylaxis, allergic reaction, type I hypersensitivity reaction, allergic conjunctivitis, allergic rhinitis, and atopic dermatitis.

In some embodiments, fibrosis is selected from pulmonary fibrosis, idiopathic pulmonary fibrosis (IPF), usual interstitial pneumonia (UIP), interstitial lung disease, cryptogenic fibrosing alveolitis (CFA), bronchiolitis obliterans, bronchiectasis, fatty liver disease, steatosis (e.g., nonalcoholic steatohepatitis (NASH), cholestatic liver disease (e.g., primary biliary cirrhosis (PBC)), cirrhosis, alcohol-induced liver fibrosis, bile duct damage, biliary fibrosis, cholestasis, and bile duct disease.

In the treatment methods of the present disclosure, "effective amount" is intended to mean an amount or dosage sufficient to produce the desired therapeutic benefit in an individual in need of such treatment. The effective amount or dosage of the compounds of the present disclosure can be determined by conventional methods (e.g., modeling, dose escalation, or clinical trials) and conventional factors (e.g., the mode or route of drug delivery, the pharmacokinetics of the agent, the severity and course of the infection, the individual's health status and weight, and the judgment of the treating physician). Exemplary dosages are in a range of about 0.1 mg to 1 g per day, or about 1 mg to 50 mg per day, or about 50 mg to 250 mg per day, or about 250 mg to 1 g per day. The total dosage may be in single or divided dosage units (e.g., BID, TID, QID).

After improvement of the patient's disease occurs, the dosage may be adjusted for preventive or maintenance treatment. For example, the dosage or frequency of administration, or both, may be reduced to an amount that maintains the desired therapeutic or preventive effect, depending on the symptoms. Of course, if the symptoms have been alleviated

to an appropriate degree, then treatment may be discontinued. However, the patient may require long-term intermittent treatment upon recurrence of any symptom. The patient may also require long-term slow treatment.

Drug combinations

The compounds of the present disclosure described herein may be used in combination with one or more other active ingredients in pharmaceutical compositions or methods to treat the diseases and conditions described herein. Other additional active ingredients include other therapeutic agents or agents that mitigate the adverse effects of the therapeutic agent against the intended disease target. The combination may be used to increase efficacy, ameliorate other disease symptoms, reduce one or more negative effects, or reduce the required dosage of the compounds of the present disclosure. The additional active ingredients may be formulated as separate pharmaceutical compositions from the compounds of the present disclosure or may be included in a single pharmaceutical composition with the compounds of the present disclosure. The additional active ingredients may be administered simultaneously with, prior to, or after the administration of the compounds of the present disclosure.

Combination agents include those additional active ingredients known or observed to be effective in treating the diseases and conditions described herein, including those effective against another target associated with the disease. For example, the compositions and formulations of the present disclosure, and methods of treatment may further comprise other drugs or medicaments, such as other active agents useful for treating or ameliorating the target disease or associated symptoms or conditions. For cancer indications, such other agents include, but are not limited to, kinase inhibitors, such as EGFR inhibitors (e.g., erlotinib, gefitinib); Raf inhibitors (e.g., vemurafenib), VEGFR inhibitors (e.g., sunitinib); standard chemotherapeutic agents, such as alkylating agents, antimetabolites, antitumor antibiotics, topoisomerase inhibitors, platinum drugs, mitotic inhibitors, antibodies, hormone therapy or corticosteroids. For pain indications, suitable combination agents include anti-inflammatory agents, such as NSAID. The pharmaceutical compositions of the present disclosure may additionally comprise one or more of such active agents, and methods of treatment may additionally comprise administering an effective amount of one or more of such active agents.

## Examples

The present disclosure will be further elaborated with specific examples below. It should be understood that these examples are only used to illustrate the present disclosure and are not used to limit the scope of the present disclosure. The experimental methods without specific conditions in the following examples are usually in accordance with the conventional conditions or the conditions suggested by the manufacturer. Unless otherwise specified, parts and percentages are parts by weight and percentages by weight.

Generally, in the preparation process, each reaction is carried out in an inert solvent at room temperature to reflux temperature (such as 0 °C to 100 °C, alternatively 0 °C to 80 °C). The reaction time is usually 0.1 to 60 hours and alternatively 0.5 to 24 hours.

Abbreviations used herein have the following meanings:

| | | | |
|---|---|---|---|
| DCM | Dichloromethane | STAB | Sodium triacetoxyborohydride |
| DMF | N,N-Dimethylformamide | HATU | O-(7-azabenzotriazol-1-yl)-N,N,N',N'-tetramethyluronium hexafluorophosphate |
| KHMDS | Potassium bis(trimethylsilyl)amide | Xphos | 2-Dicyclohexylphosphino-2',4',6'-triisopropylbiphenyl |
| THF | Tetrahydrofuran | $Pd(OAc)_2$ | Palladium(II) acetate |
| MTBE | Methyl tert-butyl ether | NBS | N-Bromosuccinimide |
| LDA | Lithium diisopropylamide | $T_3P$ | 1-Propylphosphonic anhydride |
| CDI | N,N'-Carbonyldiimidazole | TsOH | p-Toluenesulfonic acid |
| NMP | N-Methyl-2-pyrrolidone | $Pd(PPh_3)_4$ | Tetrakis(triphenylphosphine) palladium(0) |
| TFA | Trifluoroacetic acid | NIS | N-Iodosuccinimide |
| DMSO | Dimethyl sulfoxide | EDCI | 1-Ethyl-3-(3-dimethylaminopropyl)carbodiimide |
| $B_2Pin_2$ | Bis(pinacolato)diboron | HOBT | 1-Hydroxybenzotriazole |

(continued)

| | | | |
|---|---|---|---|
| Pd(dppf)$Cl_2$ | [1,1'-Bis(diphenylphosphino)ferrocene]dichlor opalladium(II) | DCE | 1,2-Dichloroethane |
| LiHMDS | Lithium bis(trimethylsilyl)amide | m-CPBA | meta-Chloroperoxybenzoic acid |
| DIPEA | N,N-Diisopropylethylamine | Oxone | Potassium peroxymonosulfate |
| $Pd_2(dba)_3$ | Tris(dibenzylideneacetone)dipalladium(0) | $BocNHNH_2$ | tert-Butyl carbazate |
| Xantphos | 4,5-Bis(diphenylphosphino)-9,9-dimethylxanthene | $Pd(t-Bu_3P)_2$ | Bis(tri-tert-butylphosphine)palladium(0) |
| Tol | Toluene | DME | Dimethoxyethane |
| TPP | Triphenylphosphine | DIAD | Diisopropyl azodicarboxylate |
| DMP | Dess-Martin periodinane | TBAF | Tetra-n-butylammonium fluoride |
| MsCl | Methanesulfonyl chloride | | |

Synthesis of related intermediate compounds

Preparation of intermediate A-1 potassium trifluoro((5-fluoro-2-methoxybenzamido)methyl)borate

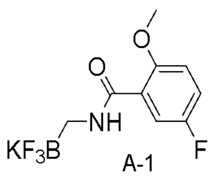

A-1

The following synthetic route was **adopted**

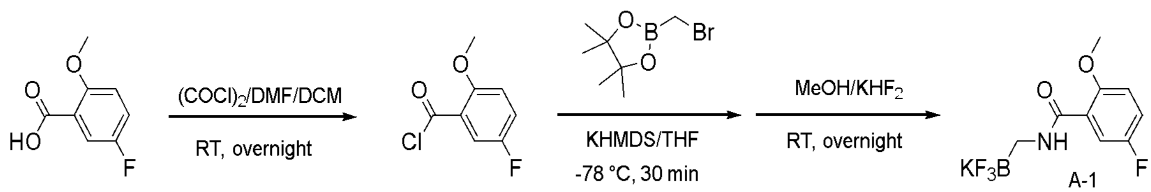


A-1

Step 1: Synthesis of compound 5-fluoro-2-methoxybenzoyl chloride

5-Fluoro-2-methoxybenzoic acid (26 g, 150 mmol) and anhydrous dichloromethane (300 mL) were added to a 100 mL three-necked flask equipped with magnetic stirring. The resulting mixture was stirred until dissolved. The mixture was cooled in an ice-water bath. Oxalyl chloride (38 g, 300 mmol) and anhydrous N,N-dimethylformamide (1.1 g, 15 mmol) were added dropwise under nitrogen. After the addition was completed, the ice bath was removed and the resulting mixture was stirred and reacted at room temperature overnight. The solvent and unreacted oxalyl chloride were removed by evaporation under reduced pressure, and the resulting mixture was set aside for later use.

Step 2: Synthesis of intermediate A-1

(Bromomethyl)boronic acid pinacol ester (36.3 g, 165 mmol) and anhydrous tetrahydrofuran (300 mL) were added to a 1000 mL three-necked flask equipped with magnetic stirring. The resulting mixture was cooled to -78 °C under nitrogen. A solution of potassium bis(trimethylsilyl)amide in tetrahydrofuran (165 mL, 1 M) was added dropwise, and the resulting mixture was stirred at the same temperature for 30 minutes. The mixture was warmed to room temperature, stirred and reacted for 30 minutes. Anhydrous methanol (21.1 g, 660 mmol) was added, and the resulting mixture was stirred at room temperature for 1 hour. The insoluble solid was filtered out. The solvent of the filtrate was removed by evaporation at a temperature not exceeding 30 °C. Then, anhydrous tetrahydrofuran (200 mL) was added, and the solvent

was removed by evaporation. The process was repeated twice. The residue was dissolved in anhydrous tetrahydrofuran (200 mL). A solution of 5-fluoro-2-methoxybenzoyl chloride in tetrahydrofuran (200 mL) was slowly added dropwise. After the addition was completed, the resulting mixture was stirred and reacted at room temperature under nitrogen overnight. The solvent was removed by evaporation, and the residue was dissolved in methanol (300 mL). An aqueous solution of potassium bifluoride (64 g, 825 mmol, 200 mL) was added, and the resulting mixture was stirred and reacted at room temperature overnight. The solvent was removed by evaporation under reduced pressure, and the resulting mixture was azeotropically refluxed with toluene (100 mL) twice to remove water. The residue was washed with methyl tert-butyl ether and filtered. The filter cake was washed with hot methanol/acetone (1/3, 1000 mL) solution. The filtrate was concentrated to dryness, and the resulting product was slurried with added methyl tert-butyl ether. A solid was precipitated, and the resulting mixture was filtered. The filter cake was dried to give 31 g of a white solid, with a yield of 70.7%. $^1H$ NMR (400 MHz, DMSO-$D_6$) δ (ppm): 7.75 (br s, 1H), 7.65-7.62 (m, 1H), 7.30-7.25 (m, 1H), 7.17-7.14 (m, 1H), 3.87 (s, 3H), 2.13-2.10 (m, 2H).

**Preparation of intermediate A-2 (Z)-3-amino-3-(4-bromophenyl)acrylonitrile**

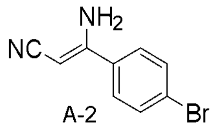

A-2

The following synthetic route was **adopted**

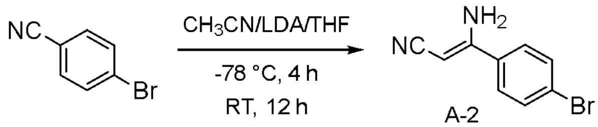


4-Bromobenzonitrile (50 g, 274.7 mmol) and anhydrous tetrahydrofuran (500 mL) were added to a 1000 mL three-necked flask equipped with magnetic stirring. The resulting mixture was stirred until dissolved. The reaction system was evacuated and purged with nitrogen three times. The resulting mixture was cooled to -78 °C, and lithium diisopropylamide (206 mL, 412.0 mmol) was added dropwise. The resulting mixture was reacted at -78 °C for 4 hours, and then anhydrous acetonitrile (17.3 g, 412.0 mmL) was added dropwise. After the addition was completed, the resulting mixture was stirred and reacted at room temperature under nitrogen for 12 hours. Water (500 mL) was added to quench the reaction. The resulting mixture was extracted with ethyl acetate (500 mL × 2). The organic phases were combined, washed with saturated brine (500 mL), dried over anhydrous sodium sulfate, filtered, concentrated, and separated by silica gel column chromatography to give 42.6 g of a white solid, with a yield of 70.0%. LC-MS (APCI): m/z = 223.0 $(M+1)^+$.

**Preparation of intermediate A-3 2-(4-bromophenyl)-4-oxo-6-(1,1,1-trifluoroprop-2-yl)-1,4-dihvdropvridine-3-carbonitrile**

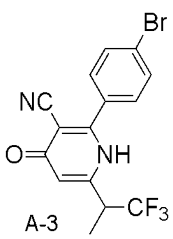

A-3

The following synthetic route was **adopted**

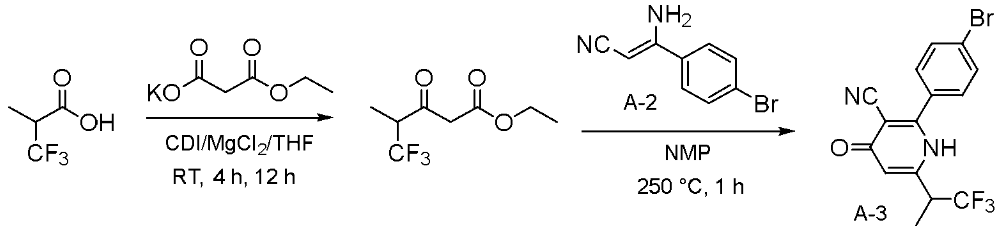


Step 1: Synthesis of compound ethyl 5,5,5-trifluoro-4-methyl-3-oxovalerate

N,N'-Carbonyldiimidazole (68.5 g, 422.3 mmol), anhydrous tetrahydrofuran (600 mL), and 3,3,3-trifluoro-2-methylpropanoic acid (50 g, 351.9 mmol) were added to a 1000 mL three-necked flask equipped with magnetic stirring. The resulting mixture was stirred until dissolved. The resulting mixture was stirred and reacted at room temperature under nitrogen for 4 hours. Anhydrous magnesium chloride (33.5 g, 351.9 mmol) and potassium 3-ethoxy-3-oxopropanoate (89.8 g, 527.8 mmol) were added, and the resulting mixture was stirred and reacted at room temperature under nitrogen for 12 hours. Hydrochloric acid (1 M) was added dropwise to the reaction solution to adjust the pH to approximately 3. The resulting mixture was extracted with ethyl acetate (500 mL × 2). The organic phases were combined, washed with saturated brine (500 mL), dried over anhydrous sodium sulfate, filtered, concentrated, and separated by silica gel column chromatography to give 44.8 g of a white solid, with a yield of 60.0%. LC-MS (APCI): m/z = 213.0 $(M+1)^+$.

Step 2 Synthesis of intermediate A-3

Intermediate A-2 (42.6 g, 200.9 mmol), ethyl 5,5,5-trifluoro-4-methyl-3-oxovalerate (22.3 g, 100.4 mmol), and N-methyl-2-pyrrolidone (60 mL) were added to a 500 mL three-necked flask equipped with magnetic stirring. The resulting mixture was stirred and reacted at 250 °C under nitrogen for 1 h. The resulting mixture was cooled to room temperature, and water (200 mL) was added. The resulting mixture was extracted with ethyl acetate (200 mL × 2). The organic phases were combined, washed with saturated brine (500 mL), dried over anhydrous sodium sulfate, filtered, concentrated, and separated by silica gel column chromatography to give 14.9 g of a white solid, with a yield of 40.0%. LC-MS (APCI): m/z = 371.0 $(M+1)^+$.

**Preparation of intermediate** A-4 **(4-((5-fluoro-2-methoxybenzamido)methyl)phenyl)boronic acid**

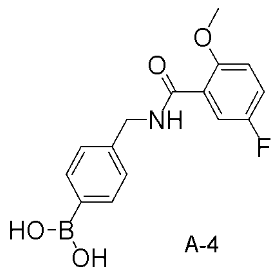

A-4

**The following synthetic route was adopted**

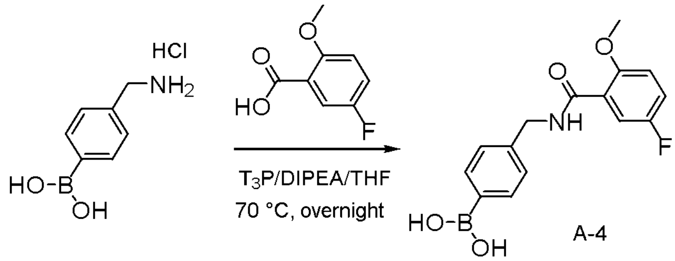


Tetrahydrofuran (120 mL), 4-aminomethylphenylboronic acid hydrochloride (12 g, 64.1 mmol), and 5-fluoro-2-methoxybenzoic acid (10.9 g, 64.1 mmol) were added to a 500 mL single-necked flask equipped with magnetic stirring and

a condenser. The resulting mixture was stirred until dissolved. A solution of 1-propylphosphonic anhydride in ethyl acetate (75.5 mL, 128.3 mmol, 1.7 M) and N,N-diisopropylethylamine (33 g, 256 mmol) were added with stirring. The reaction system was evacuated and purged with nitrogen three times. The resulting mixture was heated to 70 °C, stirred and reacted at the same temperature overnight. The mixture was cooled to room temperature and concentrated under reduced pressure. Water (120 mL) and dichloromethane (200 mL) were added. The organic phase was separated. The aqueous phase was extracted with dichloromethane (200 mL × 2). The organic phases were combined, dried over anhydrous sodium sulfate and filtered. The filtrate was concentrated to dryness, and dichloromethane (100 mL) was added. The resulting mixture was stirred for 60 minutes and filtered. The filter cake was washed with a small amount of dichloromethane, and then vacuum dried to give 17.6 g of a white solid, with a yield of 90.7%.

**Preparation of intermediate A-5 4-(benzyloxy)-2-chloronicotinonitrile**

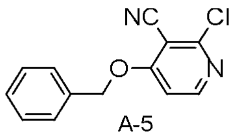

A-5

**The following synthetic route was adopted**

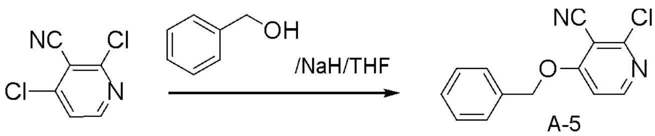


A-5

2,4-Dichloronicotinonitrile (21.7 g, 125.4 mmol), benzyl alcohol (13.55 g, 125.4 mmol), and anhydrous tetrahydrofuran (200 mL) were added to a 1 L three-necked flask equipped with magnetic stirring in an ice-water bath. The resulting mixture was stirred until homogeneous under nitrogen. Sodium hydride (6 g, 150 mmol, 60%, 0.5 g each time) was slowly added in batches over 30 minutes. After the addition was completed, the reaction mixture was reacted in an ice-water bath for 30 minutes. Then, the ice-water bath was removed, and the resulting mixture was reacted for another 1 hour. Water (100 mL) was added to quench the reaction. Tetrahydrofuran was removed by rotary evaporation. A large amount of solid was precipitated from the system. The resulting mixture was filtered, and the filter cake was washed with water. The washed solid was dissolved in dichloromethane (200 mL), washed with water (100 mL), washed with saturated brine (100 mL), dried over anhydrous sodium sulfate, filtered, and concentrated under reduced pressure to give 28.1 g of a light yellow oil. Ethyl acetate (50 mL) was added with stirring, and the resulting mixture was heated until dissolved in an oil bath. Petroleum ether (120 mL) was slowly added. The resulting mixture was slowly cooled to room temperature, and a large amount of white solid was precipitated. The mixture was filtered. The filter cake was washed with petroleum ether and vacuum dried to give 22.1 g of a white solid, with a yield of 72%. LC-MS (APCI): m/z = 245.0 $(M+1)^+$.

**Preparation of intermediate A-6 2-bromo-6-fluoro-4-(1-trifluoromethyl-vinyl)benzonitrile**

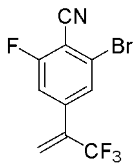

A-6

**The following synthetic route was adopted**

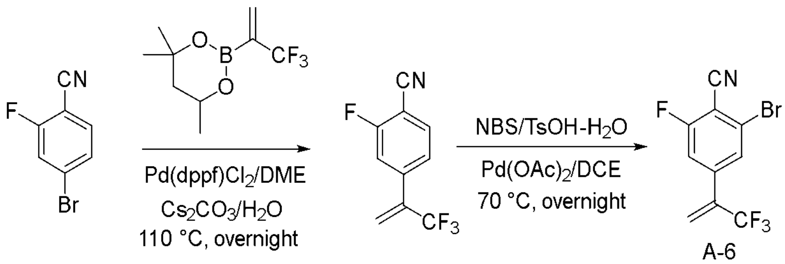

A-6

Step 1: Synthesis of compound 2-fluoro-4-(1-trifluoromethyl-vinyl)benzonitrile

4-Bromo-2-fluorobenzonitrile (5 g, 25 mmol), dimethoxyethane (100 mL), 1-(trifluoromethyl)vinylboronic acid hexylene glycol ester (8.325 g, 37.5 mmol), [1,1'-bis(diphenylphosphino)ferrocene]dichloropalladium(II) (910 mg, 1.25 mmol), cesium carbonate (24.4 g, 75 mmol), and water (25 mL) were added to a container. The reaction system was purged with nitrogen three times. The mixture was heated to 110 °C and reacted overnight. The reaction solution was cooled to room temperature, and water (100 mL) was added. The resulting mixture was extracted with ethyl acetate (150 mL × 3). The organic phase was washed with saturated brine, dried over anhydrous sodium sulfate, and concentrated. The residue was separated by silica gel column chromatography to give 4.5 g of a light yellow oil, with a yield of 83%. $^1H$ NMR (400 MHz, DMSO-$D_6$) δ (ppm): 8.05-8.01 (t, J = 8.0 Hz, 1H), 7.70 (d, J = 10.8 Hz, 1H), 7.52 (d, J = 8.0 Hz, 1H), 6.42 (t, J = 2.8 Hz, 1H), 6.33 (s, 1H).

Step 2 Synthesis of intermediate A-6

2-Fluoro-4-(1-trifluoromethyl-vinyl)benzonitrile (4.5 g, 20.93 mmol), p-toluenesulfonic acid monohydrate (4.38 g, 23.02 mmol), N-Bromosuccinimide (4.1 g, 23.02 mmol), palladium(II) acetate (235 mg, 1.05 mmol), and 1,2-dichloroethane (80 mL) were added to a container. The resulting mixture was heated to 70 °C and reacted overnight. The reaction solution was cooled to room temperature, and water (100 mL) was added. The resulting mixture was extracted with ethyl acetate (200 mL × 3). The organic phase was washed with saturated brine, dried over anhydrous sodium sulfate, and concentrated. The residue was separated by silica gel column chromatography to give 2.0 g of a light yellow solid, with a yield of 32%. LC-MS (APCI): m/z = 294.0 $(M+1)^+$.

**Preparation of intermediate A-7 2-(4-bromophenyl)-4-hydroxy-6-oxo-1,6-dihydropyridine-3-carbonitrile**

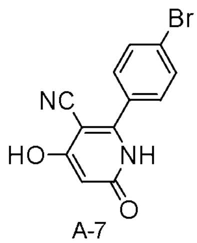
A-7

**The following synthetic route was adopted**

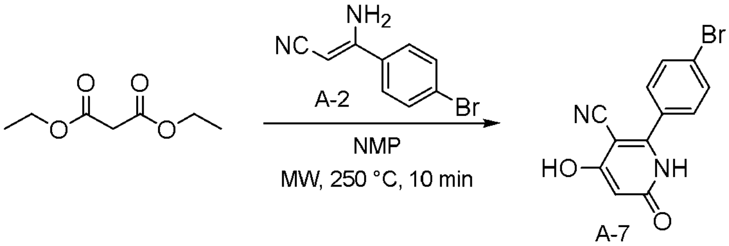

A-7

Diethyl malonate (2.16 g, 13.5 mmol), intermediate A-2 (2.0 g, 9 mmol), and N-methyl-2-pyrrolidone (10 mL) were added to a 20 mL microwave vial equipped with magnetic stirring. The vial was sealed. The mixture was microwaved and heated to 250 °C, and reacted for 10 minutes. The reaction was repeated twice. The reaction solution was cooled to room

temperature, and saturated brine (50 mL) was added to quench the reaction. The mixture was extracted with ethyl acetate (50 mL × 2). The organic phases were combined, concentrated, and separated by silica gel column chromatography to give 2.0 g of a white solid, with a yield of 25.9%. LC-MS (APCI): m/z = 291.0 $(M+1)^+$.

**Preparation of intermediate A-8 benzyl 3-(3-amino-4-(4-bromophenyl)-1H-pyrazolo[4,3-c]pyridin-6-yl)piperidine-1-carboxylate**

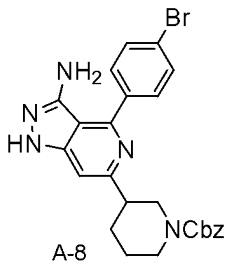

A-8

The following synthetic route was **adopted**

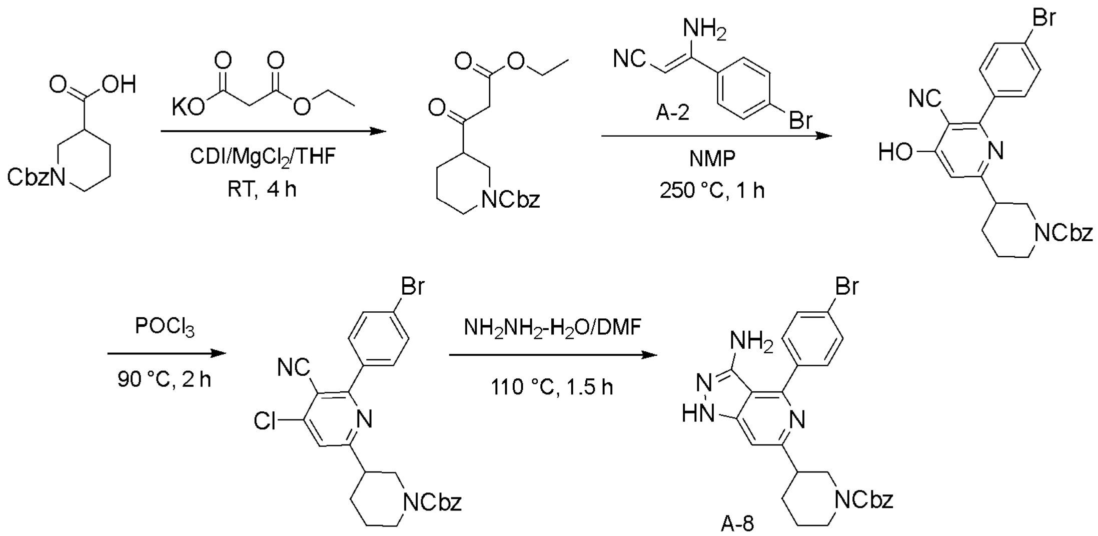


Step 1: Synthesis of compound benzyl 3-(3-ethoxy-3-oxopropanoyl)piperidine-1-carboxylate

1-((Benzyloxy)carbonyl)piperidine-3-carboxylic acid (5 g, 19.0 mmol) and tetrahydrofuran (75 mL) were added to a 250 mL three-necked flask equipped with magnetic stirring. The resulting mixture was stirred until dissolved. N,N'-carbonyldiimidazole (3.70 g, 22.8 mmol) was added, and the resulting mixture was stirred and reacted at room temperature under nitrogen for 4 hours. Compound magnesium chloride (1.81 g, 19.0 mmol) and potassium 3-ethoxy-3-oxopropanoate (4.85 g, 28.5 mmol) were added, and the resulting mixture was stirred and reacted at room temperature under nitrogen for 12 hours. 1 M hydrochloric acid was added to adjust the pH to approximately 3. The resulting mixture was extracted with ethyl acetate (50 mL × 2). The organic phases were combined, washed with saturated brine (50 mL), dried over anhydrous sodium sulfate, filtered, concentrated, and separated by silica gel column chromatography to give 4.56 g of a white solid, with a yield of 72.0%. LC-MS (APCI): m/z = 334.0 $(M+1)^+$.

Step 2: Synthesis of compound benzyl 3-(6-(4-bromophenyl)-5-cyano-4-hydroxypyridin-2-yl)piperidine-1-carboxylate

Benzyl 3-(3-ethoxy-3-oxopropanoyl)piperidine-1-carboxylate (4.5 g, 13.51 mmol), intermediate A-2 (1 g, 4.504 mmol), and N-methyl-2-pyrrolidone (5 mL) were added to a 20 mL microwave vial. The resulting mixture was stirred and reacted at 250 °C under nitrogen for 1 hour. Water (50 mL) was added to quench the reaction. The resulting mixture was extracted with ethyl acetate (50 mL × 2). The organic phases were combined, washed with saturated brine (100 mL), dried over anhydrous sodium sulfate, filtered, concentrated, and separated by silica gel column chromatography to give 1.03 g of

a white solid, with a yield of 46.6%. LC-MS (APCI): m/z = 492. 0 $(M+1)^+$.

Step 3: Synthesis of compound benzyl 3-(6-(4-bromophenyl)-4-chloro-5-cyanopyridin-2-yl)piperidine-1-carboxylate

Benzyl 3-(6-(4-bromophenyl)-5-cyano-4-hydroxypyridin-2-yl)piperidine-1-carboxylate (1.03 g, 2.098 mmol) and phosphorus oxychloride (5 mL) were added to a 25 mL single-necked flask equipped with magnetic stirring and a condenser. The resulting mixture was stirred and reacted at 90 °C under nitrogen for 2 hours. The resulting mixture was cooled in an ice-water bath, and saturated aqueous solution of sodium bicarbonate was added to adjust the pH to approximately 9. The resulting mixture was extracted with ethyl acetate (50 mL × 2). The organic phases were combined, washed with saturated brine (50 mL), dried over anhydrous sodium sulfate, filtered, concentrated, and separated by silica gel column chromatography to give 573 mg of a white solid, with a yield of 53.6%. LC-MS (APCI): m/z = 510.0 $(M+1)^+$.

Step 4: Synthesis of intermediate A-8

Benzyl 3-(6-(4-bromophenyl)-4-chloro-5-cyanopyridin-2-yl)piperidine-1-carboxylate (573 mg, 1.126 mmol), hydrazine hydrate (281.8 mg, 5.63 mmol), and N,N-dimethylformamide (3 mL) were added to a 25 mL sealed vial. The resulting mixture was stirred and reacted at 110 °C under nitrogen for 1.5 h. The resulting mixture was cooled in an ice-water bath, and saturated aqueous solution of sodium bicarbonate was added to adjust the pH to alkaline. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 500 mg of a white solid, with a yield of 87.9%. LC-MS (APCI): m/z = 506.0 $(M+1)^+$.

**Preparation of intermediate A-9 benzyl 4-(3-amino-4-(4-bromophenyl)-1H-pyrazolo[4,3-c]pyridin-6-yl)piperidine-1-carboxylate**

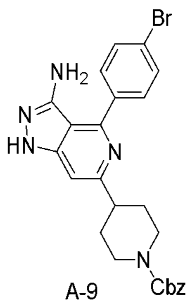

A-9

The following synthetic route was **adopted**

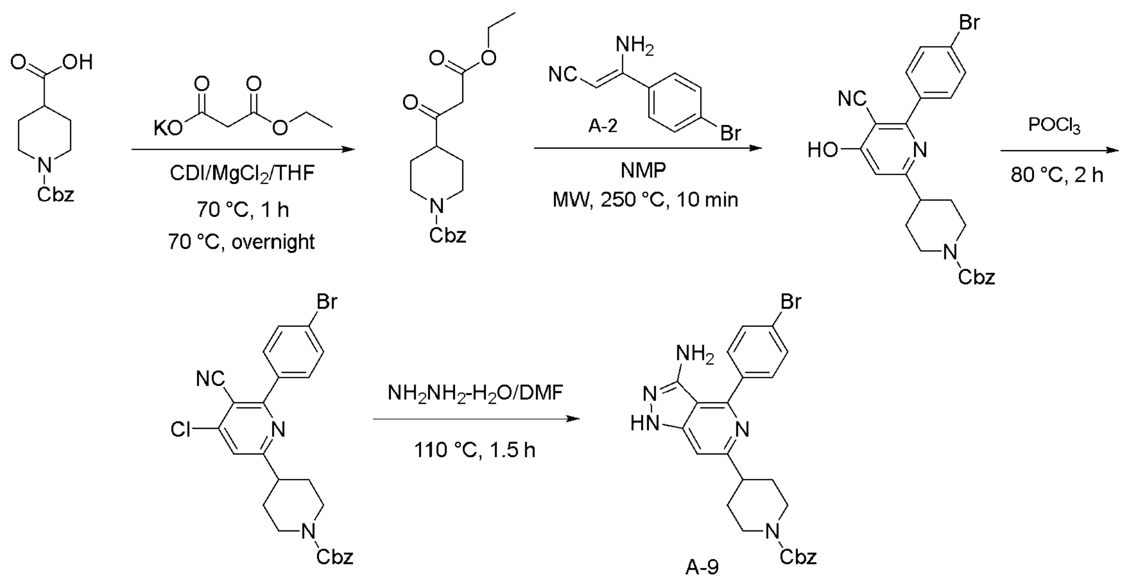


A-9

Step 1: Synthesis of compound benzyl 4-(3-ethoxy-3-oxopropanoyl)piperidine-1-carboxylate

1-((Benzyloxy)carbonyl)piperidine-4-carboxylic acid (10 g, 38 mmol), anhydrous tetrahydrofuran (200 mL), and N,N'-carbonyldiimidazole (7.4 g, 45.6 mmol) were added to a container. The resulting mixture was heated to 70 °C and reacted for 1 hour. The mixture was cooled to room temperature, and then potassium 3-ethoxy-3-oxopropanoate (5.93 g, 38 mmol) and anhydrous magnesium chloride (3.62 g, 38 mmol) were added. The resulting mixture was heated to 70 °C, stirred and reacted overnight. The reaction solution was cooled, and then 1 N hydrochloric acid was added to quench the reaction. The reaction solution was diluted with added water (100 mL). The resulting mixture was extracted with ethyl acetate (200 mL × 3). The organic phase was washed with saturated brine (200 mL), dried over anhydrous sodium sulfate, and concentrated. The residue was separated by silica gel column chromatography to give 10.5 g of a transparent oily liquid, with a yield of 82.1%. LC-MS (APCI): m/z = 334.0 $(M+1)^+$.

Step 2: Synthesis of compound benzyl 4-(6-(4-bromophenyl)-5-cyano-4-hydroxypyridin-2-yl)piperidine-1-carboxylate

Benzyl 4-(3-ethoxy-3-oxopropanoyl)piperidine-1-carboxylate (1.5 g, 4.5 mmol), intermediate A-2 (500 mg, 2.25 mmol), and N-methyl-2-pyrrolidone (15 mL) were added to a container. The resulting mixture was microwaved and reacted at 250 °C for 10 min. The reaction solution was cooled to room temperature, and water (100 mL) was added. The resulting mixture was extracted with ethyl acetate (100 mL × 3). The organic phase was washed with saturated brine, dried over anhydrous sodium sulfate, and concentrated. The residue was separated by silica gel column chromatography to give 700 mg of a light brown solid, with a yield of 63%. LC-MS (APCI): m/z = 494.1 $(M+1)^+$.

Step 3: Synthesis of compound benzyl 4-(6-(4-bromophenyl)-4-chloro-5-cyanopyridin-2-yl)piperidine-1-carboxylate

Benzyl 4-(6-(4-bromophenyl)-5-cyano-4-hydroxypyridin-2-yl)piperidine-1-carboxylate (3.9 g, 7.91 mmol) and phosphorus oxychloride (30 mL) were added to a container. The resulting mixture was heated to 80 °C and reacted for 2 hours. The reaction solution was concentrated, and warm water was added to quench the reaction. Saturated sodium bicarbonate solution was added to adjust the pH to neutral. The resulting mixture was extracted with ethyl acetate (200 mL × 3). The organic phase was washed with saturated brine, dried over anhydrous sodium sulfate, and concentrated. The residue was separated by silica gel column chromatography to give 2.5 g of a brown solid, with a yield of 61%. LC-MS (APCI): m/z = 510.1 $(M+1)^+$.

Step 4: Synthesis of intermediate A-9

Benzyl 4-(6-(4-bromophenyl)-4-chloro-5-cyanopyridin-2-yl)piperidine-1-carboxylate (2.5 g, 4.9 mmol), N,N-dimethylformamide (25 mL), and hydrazine hydrate (1.25 g, 24.51 mmol) were added to a container. The resulting mixture was heated to 110 °C and reacted for 1.5 h. The reaction solution was diluted with added water (100 mL). The resulting mixture was extracted with ethyl acetate (150 mL × 3). The organic phase was washed with saturated brine, dried over anhydrous sodium sulfate, and concentrated. The residue was separated by silica gel column chromatography to give 1.5 g of a light yellow solid, with a yield of 60%. LC-MS (APCI): m/z = 506.0 $(M+1)^+$.

**Preparation of intermediate A-10 compound tert-butyl 6-bromo-3',6'-dihydro-[3,4'-bipyridine]-1'(2'H)-carboxylate**

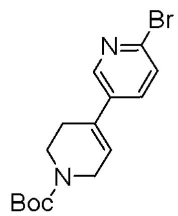

A-10

The following synthetic route was **adopted**

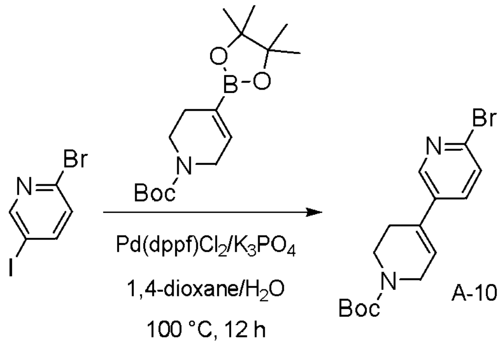


2-Bromo-5-iodopyridine (15.0 g, 53.2 mmol), N-tert-butoxycarbonyl-1,2,5,6-tetrahydropyridine-4-boronic acid pinacol ester (18 g, 18.5 mmol), 1,4-dioxane (100 mL), and water (25 mL) were successively added to a 500 mL single-necked flask equipped with magnetic stirring and a condenser. The resulting mixture was stirred until dissolved. Then, tripotassium phosphate (22 g, 133 mmol) and [1,1'-bis(diphenylphosphino)ferrocene]dichloropalladium(II) (3.8 g, 5.32 mmol) were added. The reaction system was evacuated and purged with nitrogen three times. The resulting mixture was slowly heated to 100 °C under nitrogen, stirred and reacted at the same temperature for 12 hours. The resulting mixture was cooled to room temperature, and the insoluble solid was filtered out. The resulting mixture was washed with ethyl acetate (20 mL), and the organic solvent was removed by evaporation under reduced pressure. Ethyl acetate (150 mL) was added, and the organic phase was separated. The aqueous phase was extracted with ethyl acetate (30 mL). The organic phases were combined, washed with saturated brine (50 mL), dried over anhydrous sodium sulfate, and filtered. The filtrate was concentrated to dryness and separated by silica gel column chromatography to give 15.1 g of a white solid, with a yield of 83.8%. LC-MS (APCI): m/z = 339.0 $(M+1)^+$.

**Preparation of intermediate A-11 compound tert-butyl 4-(6-bromopyridin-3-yl)piperazine-1-carboxylate**

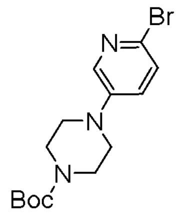

A-11

**The following synthetic route was adopted**

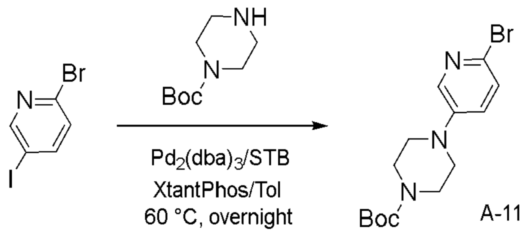


1-(tert-Butoxycarbonyl)piperazine (20.00 g, 106.38 mmol), 2-bromo-5-iodopyridine (36.24 g, 127.66 mmol), tris(dibenzylideneacetone)dipalladium(0) (2.43 g, 2.66 mmol), 4,5-bis(diphenylphosphino)-9,9-dimethylxanthene (3.08 g, 5.32 mmol), sodium tert-butoxide (30.64 g, 319.14 mmol), and toluene (200 mL) were added to a 500 mL three-necked flask equipped with magnetic stirring and a condenser. The reaction system was evacuated and purged with nitrogen three times. The mixture was stirred to disperse, stirred and reacted at 60 °C overnight. The mixture was cooled to room temperature, and saturated brine (200 mL) was added to quench the reaction. The organic phase was separated, and the aqueous phase was extracted with ethyl acetate (200 mL × 2). The organic phases were combined, washed with saturated brine (200 mL), dried over anhydrous sodium sulfate, filtered, concentrated, and separated by silica gel column chromatography to give 30.50 g of a white solid, with a yield of 84.1%. LC-MS (APCI): m/z = 342.1 $(M+1)^+$.

**Preparation of intermediate A-12 compound tert-butyl 4-((6-bromopyridin-3-yl)methyl)piperazine-1-carboxylate**

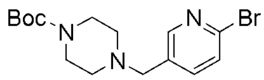

A-12

**The following synthetic route was adopted**

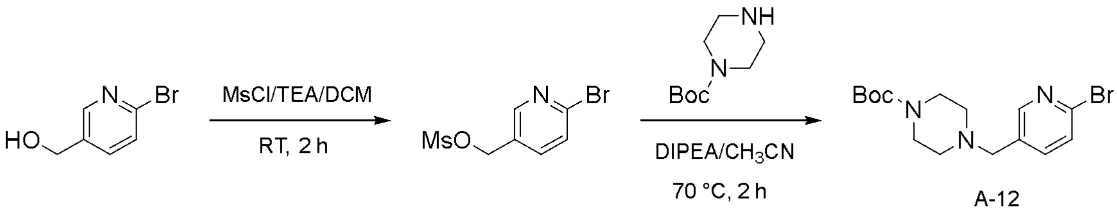


A-12

Step 1: Synthesis of compound methyl (6-bromopyridin-3-yl)methanesulfonate

6-Bromo-3-pyridinemethanol (5.0 g, 26.88 mmol), triethylamine (8.15 g, 80.64 mmol), and dichloromethane (60 mL) were successively added to a container. The resulting mixture was stirred until dissolved. Methanesulfonyl chloride (6.16 g, 53.76 mmol) was added dropwise in an ice-water bath. After the addition was completed, the mixture was stirred at room temperature under nitrogen for 2 hours. Water (50 mL) was added to quench the reaction, and the organic phase was separated. The aqueous phase was extracted with dichloromethane (100 mL × 2). The organic phases were combined, dried over anhydrous sodium sulfate, filtered, and concentrated to give a yellow oil, which was directly used in the next step reaction.

Step 2 Synthesis of intermediate A-12

The methyl (6-bromopyridin-3-yl)methanesulfonate (approximately 26.88 mmol) obtained in the previous step, acetonitrile (60 mL), 1-(tert-butoxycarbonyl)piperazine (3.22 g, 32.26 mmol), and N,N-diisopropylethylamine (10.4 g, 80.64 mmol) were successively added to a container. The resulting mixture was stirred until dissolved. The mixture was heated to 70 °C, stirred and reacted for 2 hours. The mixture was cooled to room temperature. The solvent was removed by evaporation under reduced pressure. Water (50 mL) and ethyl acetate (50 mL) were added, and the mixture was stirred for 2 minutes. The organic phase was separated, and the aqueous phase was extracted with ethyl acetate (30 mL × 2). The organic phases were combined, washed with saturated brine (100 mL), dried over anhydrous sodium sulfate, filtered, concentrated, and then mixed with silica gel and separated by column chromatography to give 4.3 g of a white solid, with a yield of 45.1%. LC-MS (APCI): m/z = 356.1 $(M+1)^+$.

**Preparation of intermediate A-13 compound tert-butyl 4-((methylsulfonyl)oxy)piperidine-1-carboxylate**

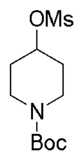

A-13

The **following synthetic route was adopted**

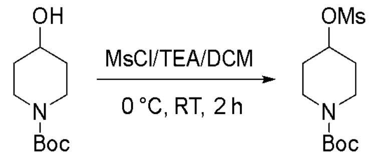

A-13

1-(tert-Butoxycarbonyl)-4-hydroxypiperidine (4.0 g, 20 mmol), triethylamine (4.0 g, 39.5 mmol), and dichloromethane (40 mL) were successively added to a container. The resulting mixture was cooled to 0 °C in an ice bath, and methanesulfonyl chloride (3.0 g, 26.2 mmol) was added dropwise. After the addition was completed, the mixture was allowed to warm to room temperature naturally. The mixture was stirred and reacted at room temperature for 2 hours. Water was added to quench the reaction. The resulting mixture was extracted with dichloromethane (100 mL × 3). The organic phase was washed with saturated brine (200 mL), dried over anhydrous sodium sulfate, and concentrated. The resulting product was directly used in the subsequent reactions.

**Preparation of intermediate B-1 4-(4-bromophenyl)-6-(1,1,1-trifluoroprop-2-yl)-1H-pyrazolo [4,3-c]pyridin-3-amine**

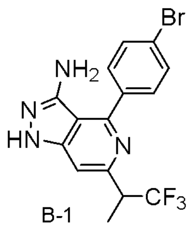
B-1

**The following synthetic route was adopted**

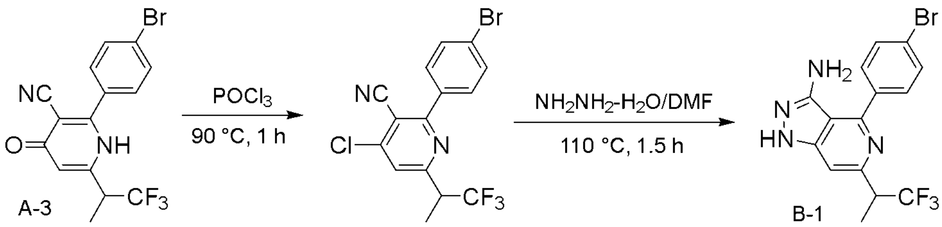

A-3 B-1

Step 1: Synthesis of compound 2-(4-bromophenyl)-4-chloro-6-(1,1,1-trifluoroprop-2-yl)nicotinonitrile

Intermediate A-3 (500 mg, 1.351 mmol) and phosphorus oxychloride (2 mL) were added to a 25 mL single-necked flask equipped with magnetic stirring and a condenser. The resulting mixture was stirred and reacted at 90 °C under nitrogen for 1 hour. The mixture was cooled to room temperature. Saturated aqueous solution of sodium bicarbonate was added to adjust the pH to approximately 9. The resulting mixture was extracted with ethyl acetate (30 mL × 2). The organic phases were combined, washed with saturated brine (30 mL), dried over anhydrous sodium sulfate, filtered, concentrated, and separated by silica gel column chromatography to give 311 mg of a white solid, with a yield of 60%. LC-MS (APCI): m/z = 389.0 $(M+1)^+$.

Step 4: Synthesis of intermediate B-1

2-(4-Bromophenyl)-4-chloro-6-(1,1,1-trifluoroprop-2-yl)nicotinonitrile (311 mg, 0.801 mmol), hydrazine hydrate (200.6 mg, 4.008 mmol), and N,N-dimethylformamide (3 mL) were added to a 25 mL sealed vial. The resulting mixture was stirred and reacted at 110 °C under nitrogen for 1.5 h. The mixture was cooled to room temperature, and saturated brine (10 mL) was added. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 240 mg of a white solid, with a yield of 78%. LC-MS (APCI): m/z = 385.0 $(M+1)^+$.

**Preparation of intermediate B-2 N-(4-(3-cyano-4-hydroxypyridin-2-yl)benzyl)-5-fluoro-2-methoxybenzamide**

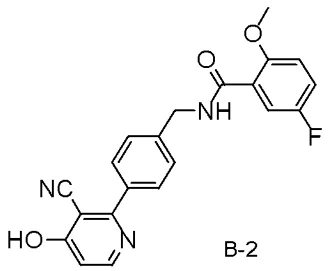

B-2

**The following synthetic route ① was adopted**

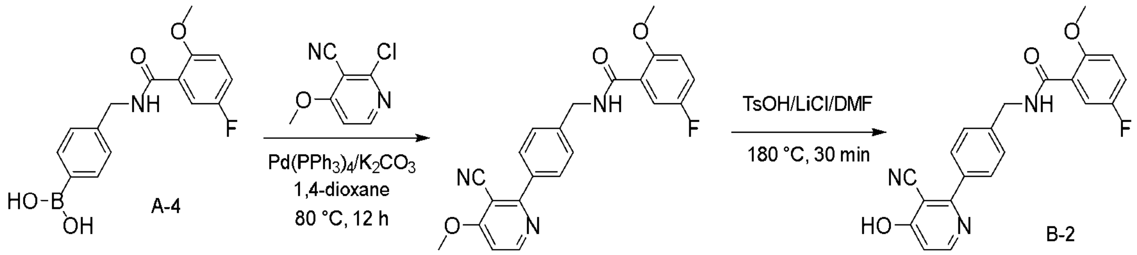


Step 1: Synthesis of compound N-(4-(3-cyano-4-methoxypyridin-2-yl)benzyl)-5-fluoro-2-methoxybenzamide

2-Chloro-4-methoxynicotinonitrile (5.0 g, 27.8 mmol), intermediate A-4 (11.0 g, 12 mmol), tetrakis(triphenylphosphine)palladium(0) (0.35 g, 0.3 mmol), potassium carbonate (10.3 g, 74.6 mmol), and 1,4-dioxane (50 mL) were added to a 100 mL single-necked flask equipped with magnetic stirring and a condenser. The reaction system was evacuated and purged with nitrogen three times. The mixture was heated to 80 °C, stirred and reacted at the same temperature under nitrogen for 12 hours. The mixture was cooled to room temperature, and ethyl acetate (100 mL) was added. The resulting mixture was filtered, concentrated, and separated by silica gel column chromatography to give 6.0 g of a white solid, with a yield of 50.1%. LC-MS (APCI): m/z = 392.14 $(M+1)^+$.

Step 2 Synthesis of intermediate B-2

N-(4-(3-Cyano-4-methoxypyridin-2-yl)benzyl)-5-fluoro-2-methoxybenzamide (6.0 g, 8.0 mmol) and N,N-dimethylformamide (60 mL) were added to a 250 mL single-necked flask equipped with magnetic stirring and a condenser. The mixture was stirred until dissolved, and p-toluenesulfonic acid (27.0 g, 69 mmol) and lithium chloride (6.5 g, 154 mmol) were added. The reaction system was evacuated and purged with nitrogen three times. The mixture was heated to 180 °C and reacted for 30 minutes. The mixture was cooled to room temperature. The reaction solution was poured into stirred ice water (400 mL), and a large amount of white solid was precipitated. The mixture was filtered and washed with ice water. The filter cake was dissolved in dichloromethane (100 mL). The mixture was dried over anhydrous sodium sulfate, filtered, concentrated, and separated by silica gel column chromatography to give 4.0 g of a white solid, with a yield of 72%. LC-MS (APCI): m/z = 378.12 $(M+1)^+$. $^1H$ NMR (400 MHz, DMSO-$D_6$) δ (ppm): 12.39 (s, 1H), 8.95 (t, J = 6.0 Hz, 1H), 7.80 (s, 1H), 7.68 (d, J = 8.0 Hz, 2H), 7.54-7.51 (m, 3H), 7.38-7.33 (m, 1H), 7.22-7.18 (m, 1H), 6.35 (br s, 1H), 4.86 (br s, 2H), 4.60 (d, J = 6.0 Hz, 2H), 3.90 (s, 3H).

**Or the following synthetic route ② was adopted**

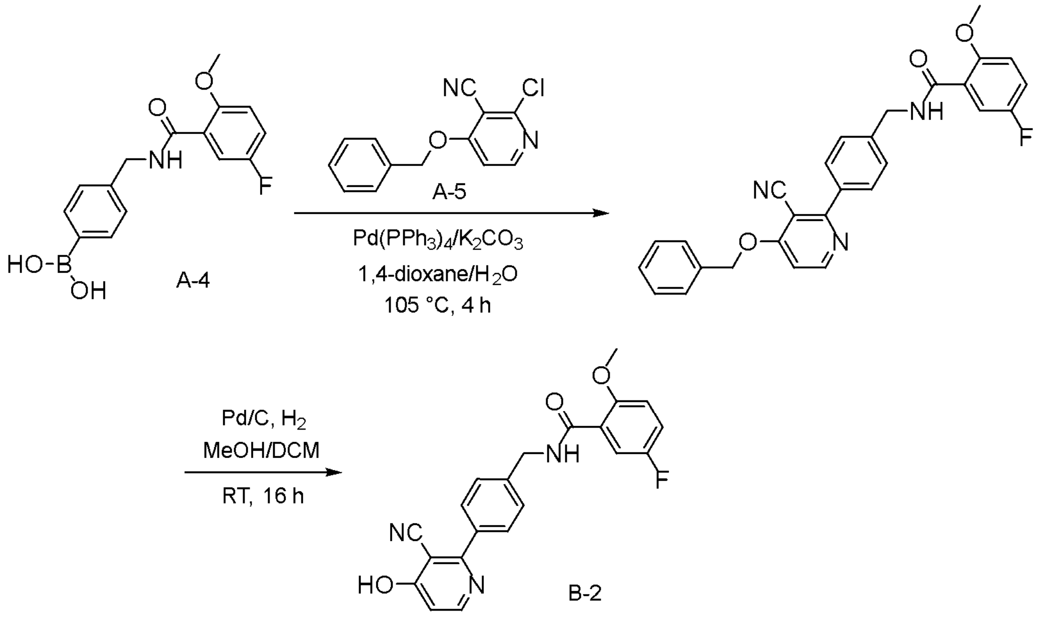


Step 1: Synthesis of compound N-(4-(4-(benzyloxy)-3-cyanopyridin-2-yl)benzyl)-5-fluoro-2-methoxybenzamide

Intermediate A-5 (9.76 g, 40 mmol), intermediate A-4 (14.5 g, 48 mmol), potassium carbonate (15.56 g, 120 mmol), tetrakis(triphenylphosphine)palladium(0) (2.3 g, 4 mmol), 1,4-dioxane (200 mL), and water (40 mL) were successively added to a 500 mL three-necked flask equipped with magnetic stirring and a condenser. The mixture was heated to 105 °C and reacted for 4 hours with stirring under nitrogen. The mixture was cooled to room temperature, and the reaction solution was diluted with added ethyl acetate (200 mL). The mixture was filtered, and the filter cake was washed with ethyl acetate (30 mL). The filtrate was washed with saturated brine (60 mL), dried over anhydrous sodium sulfate, filtered, and concentrated under reduced pressure to give 21 g of a light yellow oil. Ethyl acetate (50 mL) was added to the reaction solution with stirring, and the mixture was heated until dissolved in an oil bath. The mixture was slowly cooled to room temperature, and a large amount of white solid was precipitated from the system. The mixture was filtered. The filter cake was washed with ethyl acetate, and vacuum dried to give 12.4 g of a white solid, with a yield of 66.4%. LC-MS (APCI): m/z = 468.2 $(M+1)^+$.

Step 2 Synthesis of intermediate B-2

N-(4-(4-(Benzyloxy)-3-cyanopyridin-2-yl)benzyl)-5-fluoro-2-methoxybenzamide (40 g, 85.65 mmol), methanol (300 mL), dichloromethane (200 mL), and palladium on carbon (10%, 4 g) were added to a 1 L three-necked flask equipped with magnetic stirring. The reaction system was purged with hydrogen. The mixture was stirred and reacted at room temperature for 16 hours. The mixture was filtered, and the filtrate was collected. The filter cake was added to a reaction flask, and N,N-dimethylformamide (100 mL) was added. The mixture was heated to 60 °C and stirred for 4 hours. The mixture was filtered. The filter cake was washed with N,N-dimethylformamide, and then added back to the reaction flask. N,N-dimethylformamide (100 mL) was added, and the mixture was heated to 60 °C and stirred for 4 hours. The mixture was filtered, and the filter cake was washed with N,N-dimethylformamide. The filtrates were combined. Methanol, dichloromethane, and a small amount of toluene were removed by rotary evaporation. Water (300 mL) was slowly added dropwise with stirring at 60 °C in an oil bath. Heating was stopped, and the mixture was allowed to cool to room temperature naturally. A large amount of grayish-white solid was precipitated from the system. The mixture was filtered. The filter cake was washed with water, and vacuum dried to give 27 g of a grayish-white solid, with a yield of 83.6%. LC-MS (APCI): m/z = 378.2 $(M+1)^+$. $^1H$ NMR (400 MHz, DMSO-$D_6$) δ (ppm): 12.36 (s, 1H), 8.93 (t, J = 6.0 Hz, 1H), 7.77-7.76 (m, 1H), 7.65 (d, J = 8.0 Hz, 2H), 7.53-7.49 (m, 3H), 7.36-7.31 (m, 1H), 7.20-7.16 (m, 1H), 6.31 (d, J = 8.0 Hz, 1H), 4.58 (d, J = 6.4 Hz, 2H), 3.89 (s, 3H).

**Preparation of intermediate B-3 N-(4-(3-amino-7-bromo-1H-pyrazolo[4,3-c]pyridin-4-yl)benzyl)-5-fluoro-2-methoxybenzamide**

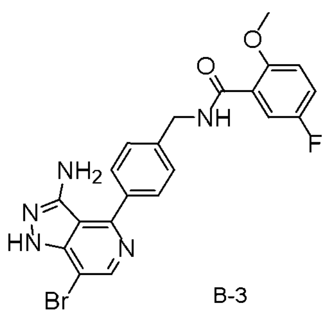

B-3

<u>**The following synthetic route was adopted**</u>

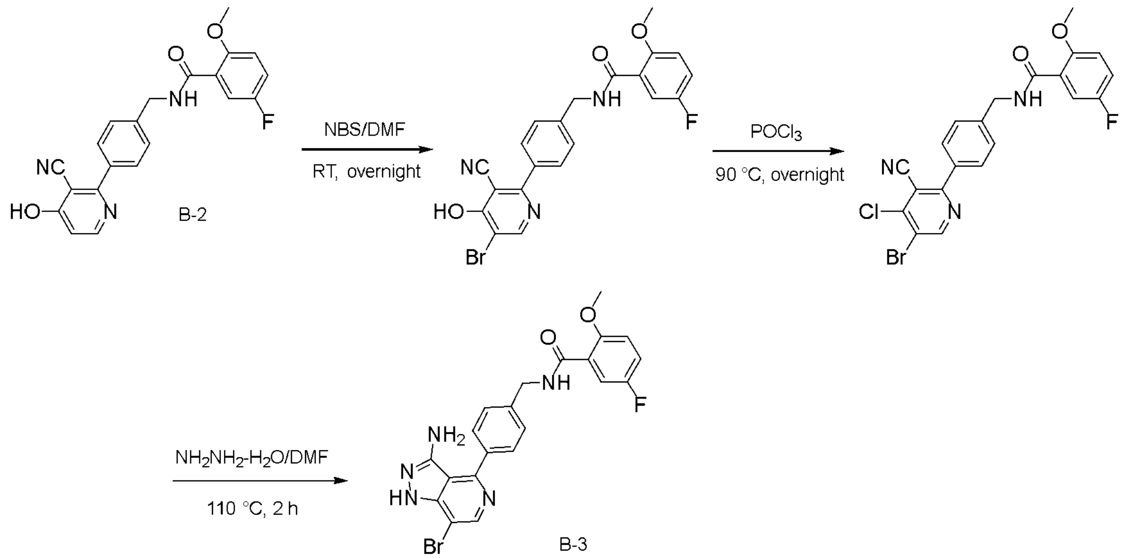


B-2

B-3

Step 1: Synthesis of compound N-(4-(5-bromo-3-cyano-4-hydroxypyridin-2-yl)benzyl)-5-fluoro-2-methoxybenzamide

Intermediate B-2 (4.0 g, 10.6 mmol, synthesized via route ① and N,N-dimethylformamide (20 mL) were added to a 250 mL single-necked flask equipped with magnetic stirring. The mixture was stirred until dissolved, and N-bromosuccinimide (2.5 g, 14 mmol) was added. The mixture was stirred and reacted at room temperature under nitrogen overnight. The reaction solution was poured into ice water (200 mL) with stirring, and a large amount of white solid was precipitated. The mixture was filtered, and washed with ice water. The filter cake was dissolved in dichloromethane (100 mL). The mixture was dried over anhydrous sodium sulfate, filtered, concentrated, and separated by silica gel column chromatography to give 3.0 g of a white solid, with a yield of 70%. LC-MS (APCI): m/z = 457.03 $(M+2)^+$. $^1H$ NMR (400 MHz, DMSO-$D_6$) δ (ppm): 12.90 (s, 1H), 8.94 (t, J = 6.0 Hz, 1H), 8.37 (s, 1H), 7.66 (d, J = 8.4 Hz, 2H), 7.54-7.49 (m, 3H), 7.37-7.32 (m, 1H), 7.21-7.18 (m, 1H), 4.59 (d, J = 6.0 Hz, 2H), 3.91 (s, 3H).

Step 2: Synthesis of compound N-(4-(5-bromo-4-chloro-3-cyanopyridin-2-yl)benzyl)-5-fluoro-2-methoxybenzamide

N-(4-(5-Bromo-3-cyano-4-hydroxypyridin-2-yl)benzyl)-5-fluoro-2-methoxybenzamide (3.0 g, 6.6 mmol) and phosphorus oxychloride (20 mL) were added to a 100 mL single-necked flask equipped with magnetic stirring. The reaction system was evacuated and purged with nitrogen three times. The mixture was heated to 90 °C, stirred and reacted overnight. The mixture was cooled to room temperature, and diluted with added ethyl acetate (100 mL). Saturated aqueous solution of sodium bicarbonate (80 mL) was carefully added with stirring. The organic phase was separated, and the aqueous phase was extracted with ethyl acetate (50 mL × 2). The organic phases were combined, washed with saturated brine (50 mL), dried over anhydrous sodium sulfate, filtered, concentrated, and separated by silica gel column chromatography to give 2.0 g of a white solid, with a yield of 65%. LC-MS (APCI): m/z = 474.99 $(M+1)^+$.

Step 3: Synthesis of intermediate B-3

N-(4-(5-Bromo-4-chloro-3-cyanopyridin-2-yl)benzyl)-5-fluoro-2-methoxybenzamide (2.0 g, 4.2 mmol) and N,N-dimethylformamide (20 mL) were added to a 100 mL single-necked flask equipped with magnetic stirring. The resulting mixture was stirred until dissolved, and hydrazine hydrate (2.1 g, 42 mmol, 80%) was added. The mixture was heated to 110 °C, stirred and reacted for 2 hours. The mixture was cooled to room temperature, and saturated brine (80 mL) was added. The organic phase was separated, and the aqueous phase was extracted with ethyl acetate (50 mL × 2). The organic phases were combined, washed with saturated brine (50 mL), dried over anhydrous sodium sulfate, filtered, concentrated, and separated by silica gel column chromatography to give 1.5 g of a white solid, with a yield of 55%. LC-MS (APCI): m/z = 471.05 $(M+2)^+$. $^1H$ NMR (400 MHz, DMSO-$D_6$) δ (ppm): 12.68 (s, 1H), 8.87 (t, J = 6.0 Hz, 1H), 8.37 (s, 1H), 7.65 (d, J = 8.0 Hz, 1H), 7.53-7.48 (m, 3H), 7.36-7.31 (m, 1H), 7.20-7.17 (m, 1H), 4.84 (br s, 2H), 4.59 (d, J = 6.0 Hz, 2H), 3.90 (s, 3H).

**Preparation of intermediate B-4 N-(4-(3-amino-7-iodo-1H-pyrazolo[4,3-c]pyridin-4 yl)benzyl)-5-fluoro-2-methoxybenzamide**

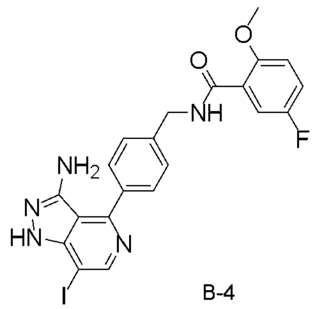

B-4

**The following synthetic route was adopted**

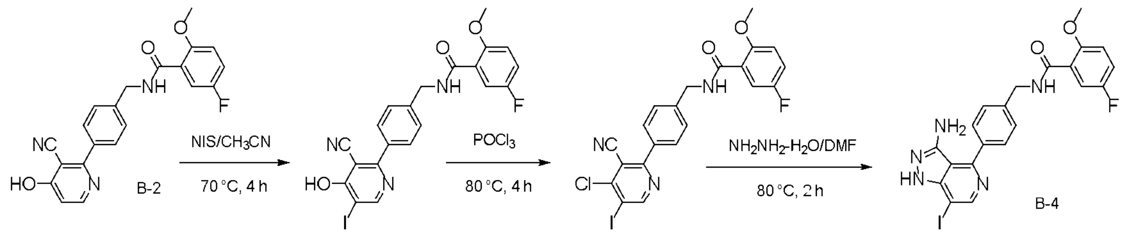


Step 1: Synthesis of compound N-(4-(3-cyano-4-hydroxy-5-iodopyridin-2-yl)benzyl)-5-fluoro-2-methoxybenzamide

Intermediate B-2 (20 g, 53 mmol, synthesized via route ②) and acetonitrile (300 mL) were added to a 500 mL three-necked flask equipped with magnetic stirring. N-iodosuccinimide (14.4 g, 48 mmol) was added with stirring. The mixture was heated to 70 °C and reacted for 4 hours with stirring under nitrogen. The mixture was cooled to room temperature, and filtered. The filter cake was washed with ethyl acetate, and vacuum dried at 80 °C to give 20.5 g of a grayish-white solid, with a yield of 76.9%. LC-MS (APCI): m/z = 504.0 $(M+1)^+$. $^1H$ NMR (400 MHz, DMSO-$D_6$) δ (ppm): 12.74 (s, 1H), 8.93 (t, J = 6.0 Hz, 1H), 8.38 (s, 1H), 7.66 (d, J = 8.4 Hz, 2H), 7.54-7.49 (m, 3H), 7.37-7.32 (m, 1H), 7.21-7.18 (m, 1H), 4.59 (d, J = 6.0 Hz, 2H), 3.90 (s, 3H).

Step 2: Synthesis of compound N-(4-(4-chloro-3-cyano-5-iodopyridin-2-yl)benzyl)-5-fluoro-2-methoxybenzamide

N-(4-(3-Cyano-4-hydroxy-5-iodopyridin-2-yl)benzyl)-5-fluoro-2-methoxybenzamide (7 g, 13.9 mmol) and phosphorus oxychloride (21 mL) were added to a 100 mL single-necked flask equipped with magnetic stirring and a condenser. The mixture was heated to 80 °C with stirring, and reacted for 4 hours, and the system was gradually dissolved. Most of the phosphorus oxychloride was removed by rotary evaporation. Ethyl acetate (70 mL) was added and the mixture was stirred until completely dissolved. The mixture was cooled to 5 °C in an ice-water bath, and ice water (50 mL) was slowly added. The mixture was stirred for 10 minutes, and the layers were separated. pH of the aqueous phase was adjusted to approximately 11 with 4% sodium hydroxide solution. The mixture was extracted with ethyl acetate (50 mL). The organic phases were combined, and saturated sodium bicarbonate solution was added to adjust the pH to approximately 9. The layers were separated. The organic phase was washed with saturated sodium bicarbonate solution (50 mL), dried over

anhydrous sodium sulfate, filtered, concentrated, and separated by silica gel column chromatography to give 6.0 g of a white solid, with a yield of 82.7%. LC-MS (APCI): m/z = 522.0 $(M+1)^+$.

Step 3: Synthesis of intermediate B-4

N-(4-(4-Chloro-3-cyano-5-iodopyridin-2-yl)benzyl)-5-fluoro-2-methoxybenzamide (6 g, 11.5 mmol) and N,N-dimethylformamide (30 mL) were added to a 100 mL single-necked flask equipped with magnetic stirring and a condenser. The mixture was stirred until dissolved, and hydrazine hydrate (5 g, 50 mmol, 50%) was added. The mixture was heated to 80 °C with stirring and reacted for 2 hours. The reaction mixture was poured into water (60 mL), and the resulting mixture was extracted with dichloromethane (30 mL × 3). The organic phases were combined, washed with water (30 mL × 3) and saturated brine (30 mL) successively, dried over anhydrous sodium sulfate, filtered, concentrated, and separated by silica gel column chromatography to give 2.88 g of a yellow solid, with a yield of 48.4%. LC-MS (APCI): m/z = 518.1 $(M+1)^+$. $^1H$ NMR (400 MHz, DMSO-$D_6$) δ (ppm): 12.64 (s, 1H), 8.88 (t, J = 6.0 Hz, 1H), 8.37 (s, 1H), 7.65 (d, J = 8.0 Hz, 1H), 7.53-7.48 (m, 3H), 7.36-7.31 (m, 1H), 7.20-7.17 (m, 1H), 4.84 (br s, 2H), 4.59 (d, J = 6.0 Hz, 2H), 3.91 (s, 3H).

**Preparation of intermediate B-5 N-(4-(3-amino-7-iodo-1H-pyrazolo[4,3-c]pyridin-4-yl)-3-fluorobenzyl)-5-fluoro-2-methoxybenzamide**

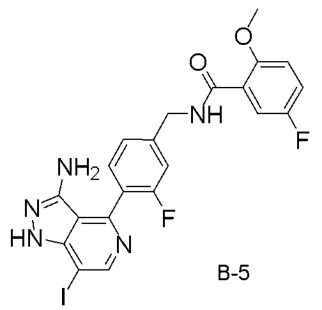

B-5

**The following synthetic route was adopted**

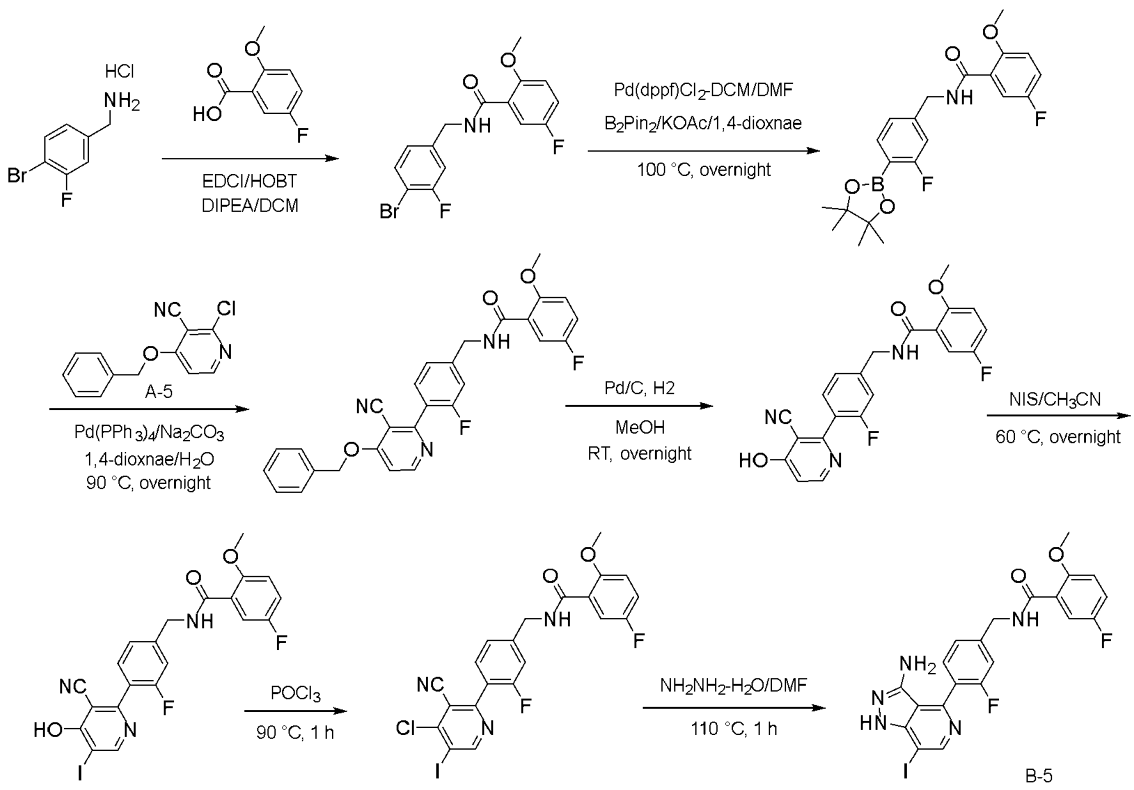

Step 1: Synthesis of compound N-(4-bromo-3-fluorobenzyl)-5-fluoro-2-methoxybenzamide

3-Fluoro-4-bromobenzylamine hydrochloride (5.0 g, 20.8 mmol), 3-fluoro-6-methoxybenzoic acid (4.2 g, 24.9 mmol), 1-ethyl-3-(3-dimethylaminopropyl)carbodiimide (5.2 g, 20.97 mmol), 1-hydroxybenzotriazole (3.65 g, 20.97 mmol), and dichloromethane (50 mL) were added to a 50 mL single-necked flask equipped with magnetic stirring. The reaction system was evacuated and purged with nitrogen three times. The mixture was stirred thoroughly in an ice bath, and N,N-diisopropylethylamine (9.0 mL, 62.25 mmol) was added. The mixture was stirred and reacted at room temperature overnight. The mixture was cooled to room temperature, and saturated brine (50 mL) was added to quench the reaction. The organic phase was separated. The aqueous phase was extracted with dichloromethane (50 mL $\times$ 2). The organic phases were combined, washed with saturated brine (50 mL $\times$ 2), dried over anhydrous sodium sulfate, filtered, concentrated and separated by silica gel column chromatography to give 6.6 g of a white solid, with a yield of 89.9%. LC-MS (APCI): m/z = 356.0 $(M+1)^+$.

Step 2: Synthesis of compound 5-fluoro-N-(3-fluoro-4-(4,4,5,5-tetramethyl-1,3,2-dioxaborolan-2-yl)benzyl)-2-methoxybenzamide

N-(4-Bromo-3-fluorobenzyl)-5-fluoro-2-methoxybenzamide (5.0 g, 14.08 mmol), bis(pinacolato)diboron (5.0 g, 19.69 mmol), [1,1'-bis(diphenylphosphino)ferrocene]palladium(II) dichloride dichloromethane adduct (1.14 g, 1.4 mmol), potassium acetate (4.1 g, 42.2 mmol), 1,4-dioxane (40 mL), and N,N-dimethylformamide (10 mL) were added to a 250 mL three-necked flask equipped with magnetic stirring and a condenser. The reaction system was evacuated and purged with nitrogen three times. The mixture was stirred and reacted at 100 °C overnight. The mixture was cooled to room temperature, and saturated brine (30 mL) was added to quench the reaction. The organic phase was separated, and the aqueous phase was extracted with ethyl acetate (40 mL $\times$ 2). The organic phases were combined, washed with saturated brine (40 mL), dried over anhydrous sodium sulfate, filtered, concentrated, and separated by silica gel column chromatography to give 5.6 g of a white solid, with a yield of 98.7%. LC-MS (APCI): m/z = 404.0 $(M+1)^+$.

Step 3: Synthesis of compound N-(4-(4-(benzyloxy)-3-cyanopyridin-2-yl)-3-fluorobenzyl)-5-fluoro-2-methoxybenzamide

5-Fluoro-N-(3-fluoro-4-(4,4,5,5-tetramethyl-1,3,2-dioxaborolan-2-yl)benzyl)-2-methoxybenzamide (3.0 g, 7.44 mmol), intermediate A-5 (2.18 g, 8.93 mmol), tetrakis(triphenylphosphine)palladium(0) (859.9 mg, 0.744 mmol), sodium carbonate (2.37 g, 22.32 mmol), 1,4-dioxane (30 mL), and water (7 mL) were added to a 100 mL three-necked flask equipped with magnetic stirring and a condenser. The reaction system was evacuated and purge with nitrogen three times. The mixture was stirred and reacted at 90 °C overnight. The mixture was cooled to room temperature, and saturated brine (30 mL) was added to quench the reaction. The organic phase was separated, and the aqueous phase was extracted with ethyl acetate (30 mL $\times$ 2). The organic phases were combined, wash with saturated brine (30 mL), dried over anhydrous sodium sulfate, filtered, concentrated, and separated by silica gel column chromatography to give 2.17 g of a white solid, with a yield of 60.1%. LC-MS (APCI): m/z = 486.0 $(M+1)^+$.

Step 4: Synthesis of compound N-(4-(3-cyano-4-hydroxypyridin-2-yl)-3-fluorobenzyl)-5-fluoro-2-methoxybenzamide

N-(4-(4-(Benzyloxy)-3-cyanopyridin-2-yl)-3-fluorobenzyl)-5-fluoro-2-methoxybenzamide (2.1 g, 4.33 mmol), palladium on carbon (5%, 210 mg), and methanol (30 mL) were added to a 100 mL single-necked flask equipped with magnetic stirring. The reaction system was evacuated and purged with hydrogen three times. The mixture was stirred and reacted at room temperature overnight. The mixture was filtered, and the filter cake was washed with methanol. The organic phases were combined, and concentrated to give 1.7 g of a white solid, with a yield of 99.4%. LC-MS (APCI): m/z = 396.0 $(M+1)^+$.

Step 5: Synthesis of compound N-(4-(3-cyano-4-hydroxy-5-iodopyridin-2-yl)-3-fluorobenzyl)-5-fluoro-2-methoxybenzamide

N-(4-(4-(Benzyloxy)-3-cyanopyridin-2-yl)-3-fluorobenzyl)-5-fluoro-2-methoxybenzamide (1.7 g, 4.3 mmol), N-iodosuccinimide (1.1 g, 4.84 mmol), and acetonitrile (40 mL) were successively added to a 100 mL single-necked flask equipped with magnetic stirring. The reaction system was evacuated and purged with hydrogen three times. The mixture was stirred and reacted at 60 °C overnight. The mixture was filtered, and washed with ethyl acetate. The filter residue was collected and dried to give 1.55 g of a white solid, with a yield of 69.2%. LC-MS (APCI): m/z = 522.0 $(M+1)^+$.

Step 6: Synthesis of compound N-(4-(4-chloro-3-cyano-5-iodopyridin-2-yl)-3-fluorobenzyl)-5-fluoro-2-methoxybenzamide

N-(4-(3-Cyano-4-hydroxy-5-iodopyridin-2-yl)-3-fluorobenzyl)-5-fluoro-2-methoxybenzamide (1.7 g, 4.3 mmol) and phosphorus oxychloride (15 mL) were added to a 50 mL single-necked flask equipped with magnetic stirring. The reaction system was evacuated and purged with hydrogen three times. The mixture was stirred and reacted at 90 °C for 1 hour. The mixture was cooled to room temperature, and concentrated under reduced pressure. Saturated aqueous solution of sodium bicarbonate (30 mL) was added to quench the reaction. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 1.07 g of a white solid, with a yield of 66.73%. LC-MS (APCI): m/z = 540.0 $(M+1)^+$.

Step 7 Synthesis of intermediate B-5

N-(4-(4-Chloro-3-cyano-5-iodopyridin-2-yl)-3-fluorobenzyl)-5-fluoro-2-methoxybenzamide (500 mg, 0.93 mmol), hydrazine hydrate (0.15 mL, 0.79 mmol), and N,N-dimethylformamide (5 mL) were added to a 20 mL sealed vial equipped with magnetic stirring. The vial was sealed, and the mixture was stirred and reacted at 110 °C for 2 hours. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 400 mg of a white solid, with a yield of 80.4%. LC-MS (APCI): m/z = 536.0 $(M+1)^+$.

**Preparation of intermediate B-6 N-(4-(3-amino-7-iodo-1H-pyrazolo[4,3-c]pyridin-4-yl)-3-methylbenzyl)-5-fluoro-2-methoxybenzamide**

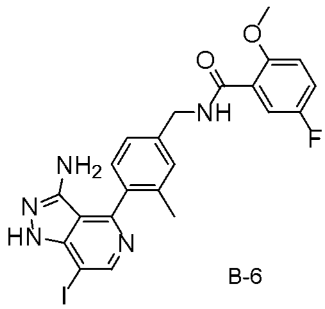

B-6

The following synthetic route was **adopted**

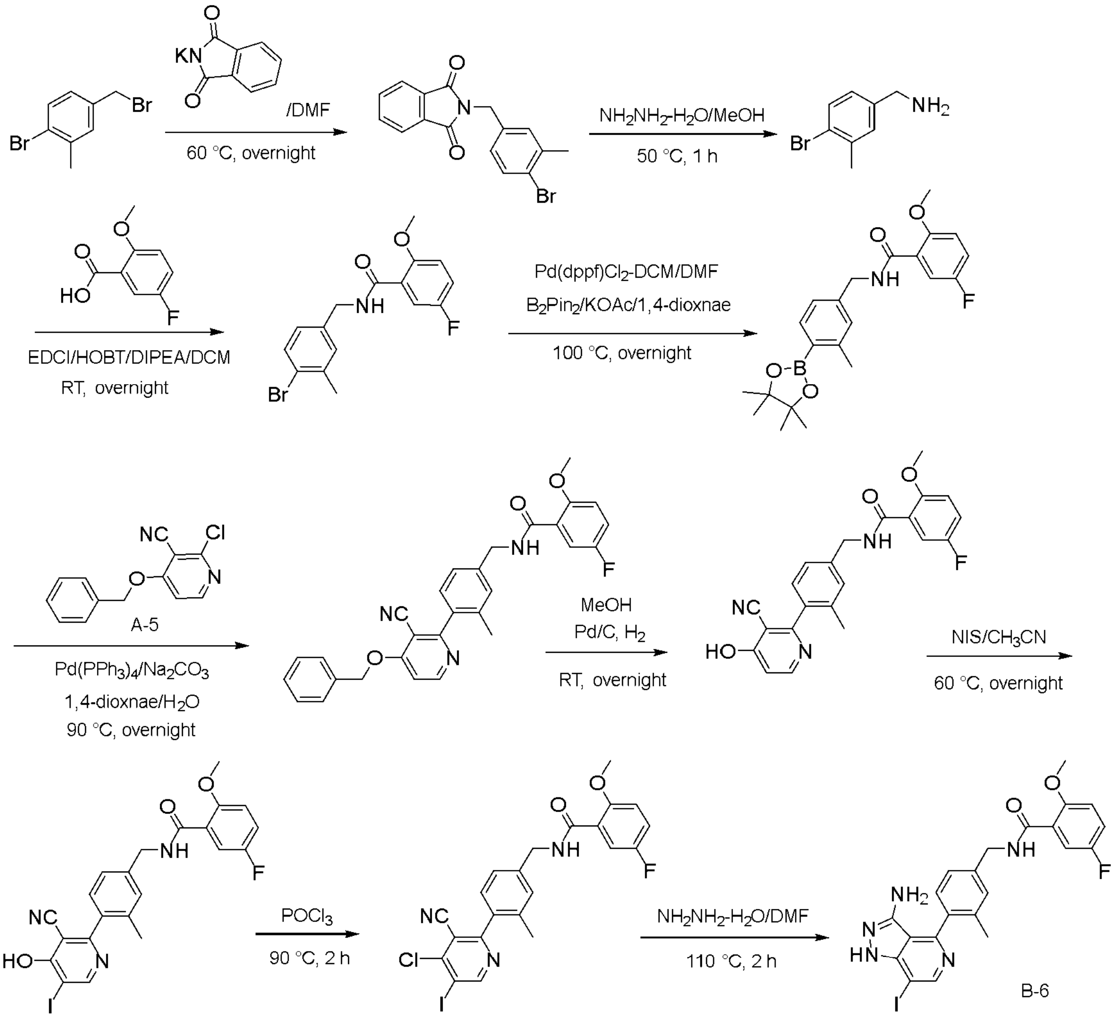

B-6

Step 1: Synthesis of compound 2-(4-bromo-3-methylbenzyl)isoindoline-1,3-dione

3-Methyl-4-bromobenzyl bromide (5.0 g, 18.9 mmol), potassium phthalimide (3.85 g, 20.8 mmol), and N,N-dimethylformamide (50 mL) were added to a 100 mL single-necked flask equipped with magnetic stirring and a condenser. The reaction system was evacuated and purged with nitrogen three times. The mixture was stirred and reacted at 60 °C overnight. The mixture was cooled to room temperature, and saturated brine (50 mL) was added to quench the reaction. The organic phase was separated, and the aqueous phase was extracted with dichloromethane (50 mL × 2). The organic phases were combined, washed with saturated brine (50 mL), dried over anhydrous sodium sulfate, filtered, concentrated, and separated by silica gel column chromatography to give 6.2 g of a white solid, with a yield of 99.7%. LC-MS (APCI): m/z = 330.0 $(M+1)^+$.

Step 2: Synthesis of compound (4-bromo-3-methylphenyl)methanamine

2-(4-Bromo-3-methylbenzyl)isoindoline-1,3-dione (5.92 g, 18 mmol) and methanol (50 mL) were added to a 250 mL three-necked flask equipped with magnetic stirring and a condenser. The reaction system was evacuated and purged with nitrogen three times. The mixture was stirred thoroughly at 50 °C. Hydrazine hydrate (2.5 mL, 39.6 mmol) was added, and the mixture was heated and refluxed for 1 hour. The mixture was cooled to room temperature, and saturated brine (30 mL) was added to quench the reaction. The organic phase was separated, and the aqueous phase was extracted with ethyl acetate (50 mL × 2). The organic phases were combined, washed with saturated brine (50 mL), dried over anhydrous sodium sulfate, filtered, concentrated, and separated by silica gel column chromatography to give 3.58 g of a colorless oily liquid, with a yield of 99.7%. LC-MS (APCI): m/z = 200.0 $(M+1)^+$.

Step 3: Synthesis of compound N-(4-bromo-3-methylbenzyl)-5-fluoro-2-methoxybenzamide

(4-Bromo-3-methylphenyl)methanamine (3.58 g, 17.9 mmol), 3-fluoro-6-methoxybenzoic acid (5.1 g, 29.9 mmol), 1-ethyl-3-(3-dimethylaminopropyl)carbodiimide (6.23 g, 32.5 mmol), 1-hydroxybenzotriazole (4.39 g, 24.07 mmol), and dichloromethane (50 mL) were added to a 250 mL three-necked flask equipped with magnetic stirring. The reaction system was evacuated, and purged with nitrogen three times. The mixture was stirred thoroughly in an ice bath, and N,N-diisopropylethylamine (10.8 mL, 75.2 mmol) was added. The mixture was stirred and reacted at room temperature overnight. The mixture was cooled to room temperature, and saturated brine (50 mL) was added to quench the reaction. The organic phase was separated, and the aqueous phase was extracted with dichloromethane (50 mL × 2). The organic phases were combined, washed with saturated brine (50 mL), dried over anhydrous sodium sulfate, filtered, concentrated and separated by silica gel column chromatography to give 4.71 g of a white solid, with a yield of 74.9%. LC-MS (APCI): m/z = 352.0 $(M+1)^+$.

Step 4: Synthesis of compound 5-fluoro-2-methoxy-N-(3-methyl-4-(4,4,5,5-tetramethyl-1,3,2-dioxaborolan-2-yl)benzyl)benzamide

N-(4-Bromo-3-methylbenzyl)-5-fluoro-2-methoxybenzamide (4.71 g, 13.4 mmol), bis(pinacolato)diboron (4.08 g, 16.08 mmol), [1,1'-bis(diphenylphosphino)ferrocene]palladium(II) dichloride dichloromethane adduct (1.09 g, 1.34 mmol), potassium acetate (3.95 g, 40.2 mmol), 1,4-dioxane (40 mL), and N,N-dimethylformamide (10 mL) were added to a 250 mL three-necked flask equipped with magnetic stirring and a condenser. The reaction system was evacuated and purged with nitrogen three times. The mixture was stirred and reacted at 100 °C overnight. The mixture was cooled to room temperature, and saturated brine (30 mL) was added to quench the reaction. The organic phase was separated, and the aqueous phase was extracted with ethyl acetate (40 mL × 2). The organic phases were combined, washed with saturated brine (40 mL), dried over anhydrous sodium sulfate, filtered, concentrated, and separated by silica gel column chromatography to give 4.5 g of a white solid, with a yield of 84.1%. LC-MS(APCI): m/z=400.0 $(M+1)^+$.

Step 5: Synthesis of compound N-(4-(4-(benzyloxy)-3-cyanopyridin-2-yl)-3-methylbenzyl)-5-fluoro-2-methoxybenzamide

5-Fluoro-2-methoxy-N-(3-methyl-4-(4,4,5,5-tetramethyl-1,3,2-dioxaborolan-2-yl)benzyl)benzamide (3.0 g, 7.5 mmol), intermediate A-5 (2.2 g, 9.0 mmol), tetrakis(triphenylphosphine)palladium(0) (866.7 mg, 0.75 mmol), sodium carbonate (2.4 g, 22.5 mmol), 1,4-dioxane (30 mL), and water (7 mL) were added to a 100 mL three-necked flask equipped with magnetic stirring and a condenser. The reaction system was evacuated and purged with nitrogen three times. The mixture was stirred and reacted at 90 °C overnight. The mixture was cooled to room temperature, and saturated brine (30 mL) was added to quench the reaction. The organic phase was separated, and the aqueous phase was extracted with ethyl acetate (30 mL × 2). The organic phases were combined, washed with saturated brine (30 mL), dried over anhydrous sodium sulfate, filtered, concentrated, and separated by silica gel column chromatography to give 1.56 g of a white solid, with a yield of 43.2%. LC-MS (APCI): m/z = 482.0 $(M+1)^+$.

Step 6: Synthesis of compound N-(4-(3-cyano-4-hydroxypyridin-2-yl)-3-methylbenzyl)-5-fluoro-2-methoxybenzamide

N-(4-(4-(Benzyloxy)-3-cyanopyridin-2-yl)-3-methylbenzyl)-5-fluoro-2-methoxybenzamide (1.56 g, 3.24 mmol), palladium on carbon (5%, 156 mg), and methanol (30 mL) were added to a 100 mL single-necked flask equipped with magnetic stirring. The reaction system was evacuated and purged with hydrogen three times. The mixture was stirred and reacted at room temperature overnight. The mixture was filtered, and the filter cake was washed with methanol. The organic phases were combined, and concentrated to give 1.26 g of a white solid, with a yield of 99.4%. LC-MS (APCI): m/z = 392.0 $(M+1)^+$.

Step 7: Synthesis of compound N-(4-(3-cyano-4-hydroxy-5-iodopyridin-2-yl)-3-methylbenzyl)-5-fluoro-2-methoxybenzamide

N-(4-(3-Cyano-4-hydroxypyridin-2-yl)-3-methylbenzyl)-5-fluoro-2-methoxybenzamide (1.26 g, 3.23 mmol), N-iodosuccinimide (801.8 g, 3.56 mmol), and acetonitrile (30 mL) were added to a 100 mL single-necked flask equipped with magnetic stirring. The reaction system was evacuated and purged with nitrogen three times. The mixture was stirred and reacted at 60 °C overnight. The mixture was filtered, and washed with ethyl acetate. The filter residue was collected and dried to give 992 mg of a white solid, with a yield of 59.4%. LC-MS (APCI): m/z = 518.0 $(M+1)^+$.

Step 8: Synthesis of compound N-(4-(4-chloro-3-cyano-5-iodopyridin-2-yl)-3-methylbenzyl)-5-fluoro-2-methoxybenzamide

N-(4-(3-Cyano-4-hydroxy-5-iodopyridin-2-yl)-3-methylbenzyl)-5-fluoro-2-methoxybenzamide (992 mg, 1.92 mmol) and phosphorus oxychloride (10 mL) were added to a 50 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (30 mL) was added to quench the reaction. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 337 mg of a white solid, with a yield of 32.8%. LC-MS (APCI): m/z = 536.0 $(M+1)^+$.

Step 9: Synthesis of intermediate B-6

N-(4-(4-Chloro-3-cyano-5-iodopyridin-2-yl)-3-methylbenzyl)-5-fluoro-2-methoxybenzamide (337 mg, 0.63 mmol), hydrazine hydrate (0.12 mL, 1.89 mmol), and N,N-dimethylformamide (5 mL) were added to a 15 mL sealed vial equipped with magnetic stirring. The vial was sealed, and the mixture was stirred and reacted at 110 °C for 2 hours. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 300 mg of a white solid, with a yield of 89.7%. LC-MS (APCI): m/z = 532.0 $(M+1)^+$.

**Preparation of intermediate B-7 N-(4-(3-amino-7-bromo-6-methyl-1H-pyrazolo[4,3-c]pyridin-4-yl)benzyl)-5-fluoro-2-methoxybenzamide**

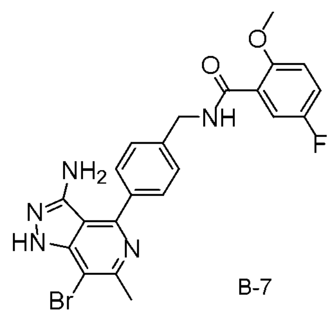

B-7

**The following synthetic route was adopted**

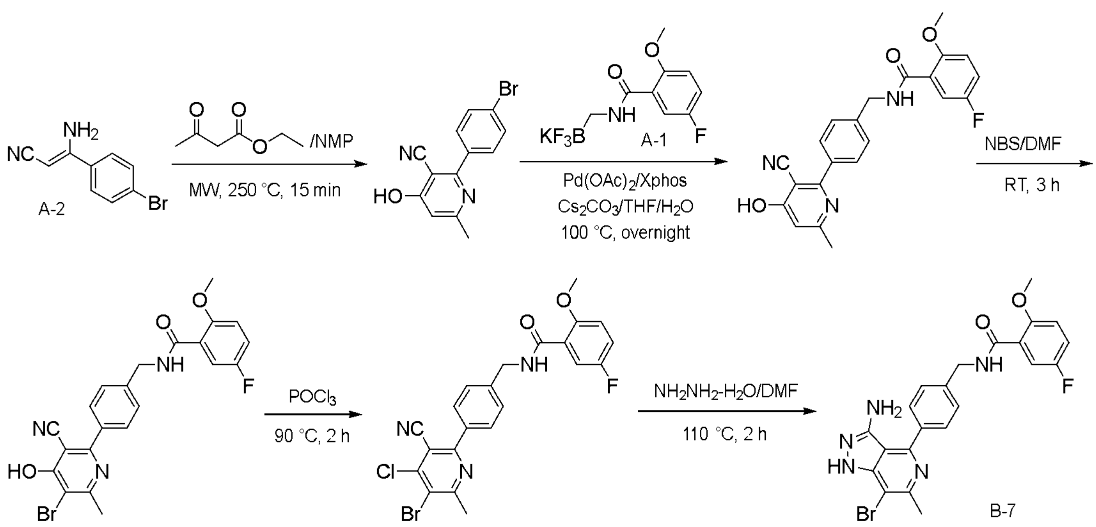

Step 1: Synthesis of compound 2-(4-bromophenyl)-4-hydroxy-6-methylnicotinonitrile

Intermediate A-2 (3 g, 12.71 mmol), ethyl acetoacetate (25.42 mmol, 3.3 g), and N-methyl-2-pyrrolidone (10 mL) were added to a container. The mixture was microwaved and heated to 250 °C, and reacted for 15 minutes. The mixture was cooled to room temperature, and the solvent was removed by evaporation under reduced pressure. The residue was separated by column chromatography to give 2.5 g of a yellow solid, with a yield of 68.3%. LC-MS (APCI): m/z = 289.0 $(M+1)^+$.

Step 2: Synthesis of compound N-(4-(3-cyano-4-hydroxy-6-methylpyridin-2-yl)benzyl)-5-fluoro-2-methoxybenzamide

2-(4-Bromophenyl)-4-hydroxy-6-methylnicotinonitrile (800 mg, 2.78 mmol), intermediate A-1 (1.04 g, 3.61 mmol), palladium(II) acetate (62 mg, 0.278 mmol), 2-dicyclohexylphosphino-2',4',6'-triisoprophylbiphenyl (132 mg, 0.278 mmol), and cesium carbonate (2.72 g, 8.33 mmol) were added to a 100 mL three-necked flask equipped with magnetic stirring and a condenser. The reaction system was evacuated and purged with nitrogen three times. Tetrahydrofuran (10 mL) and water (1 mL) were added. The mixture was stirred to disperse, stirred and reacted at 100 °C overnight. The mixture was cooled to room temperature, and saturated brine (10 mL) was added. The organic phase was separated, and the aqueous phase was extracted with ethyl acetate (20 mL × 2). The organic phases were combined, washed with saturated brine (30 mL), dried over anhydrous sodium sulfate, filtered, concentrated and separated by silica gel column chromatography to give 600 mg of a yellow solid, with a yield of 55.56%. LC-MS (APCI): m/z = 392.0 $(M+1)^+$.

Step 3: Synthesis of compound N-(4-(5-bromo-3-cyano-4-hydroxy-6-methylpyridin-2-yl)benzyl)-5-fluoro-2-methoxybenzamide

N-(4-(3-Cyano-4-hydroxy-6-methylpyridin-2-yl)benzyl)-5-fluoro-2-methoxybenzamide (600 mg, 1.538 mmol) and N,N-dimethylformamide (30 mL) were added to a 100 mL single-necked flask equipped with magnetic stirring. The mixture was stirred until dissolved. N-bromosuccinimide (288 mg, 1.615 mmol) was added, and the reaction system was evacuated and purged with nitrogen three times. The mixture was stirred at room temperature for 3 hours. A large amount of yellow solid was precipitated. The mixture was filtered, and washed with ethyl acetate (5 mL). The filter residue was collected and dried to give 630 mg of the yellow solid, with a yield of 87.2%. LC-MS (APCI): m/z = 470.0 $(M+1)^+$.

Step 4: Synthesis of compound N-(4-(5-bromo-4-chloro-3-cyano-6-methylpyridin-2-yl)benzyl)-5-fluoro-2-methoxybenzamide

N-(4-(5-Bromo-3-cyano-4-hydroxy-6-methylpyridin-2-yl)benzyl)-5-fluoro-2-methoxybenzamide (630 mg, 1.34 mmol) and phosphorus oxychloride (10 mL) were added to a 50 mL single-necked flask equipped with magnetic stirring. The reaction system was evacuated and purged with nitrogen three times. The mixture was stirred and reacted at 90 °C for 2 hours. The mixture was cooled to room temperature, and concentrated to dryness under reduced pressure. Saturated aqueous solution of sodium bicarbonate (30 mL) was added with stirring, and the mixture was extracted with ethyl acetate (30 mL × 2). The organic phases were combined, washed with saturated brine (30 mL), dried over anhydrous sodium sulfate, filtered, concentrated, and separated by silica gel column chromatography to give 430 mg of a white solid, with a yield of 65.6%. LC-MS (APCI): m/z = 488.0 $(M+1)^+$.

Step 5: Synthesis of intermediate B-7

N-(4-(5-Bromo-4-chloro-3-cyano-6-methylpyridin-2-yl)benzyl)-5-fluoro-2-methoxybenzamide (430 mg, 0.883 mmol) and N,N-dimethylformamide (5 mL) were added to a 15 mL sealed vial equipped with magnetic stirring. The mixture was stirred until dissolved, and hydrazine hydrate (0.12 mL, 1.89 mmol) was added. The vial was sealed and the mixture was stirred and reacted at **110** °C for 2 hours. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 330 mg of a white solid, with a yield of 77.5%. LC-MS (APCI): m/z = 484.0 $(M+1)^+$.

**Preparation of intermediate B-8 N-(4-(3-amino-7-bromo-6-methyl-1H-pyrazolo[4,3-c]pyridin-4-yl)benzyl)-5-fluoro-2-methoxybenzamide**

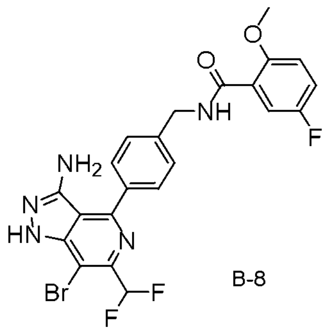

B-8

**The following synthetic route was adopted**

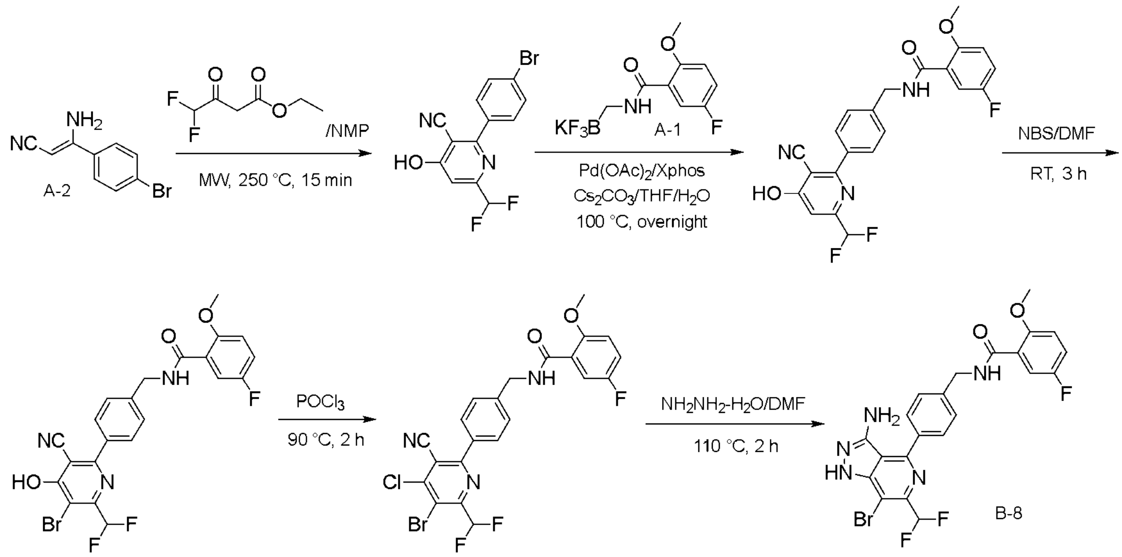


A-2 A-1 B-8

Step 1: Synthesis of compound 2-(4-bromophenyl)-6-(difluoromethyl)-4-hydroxynicotinonitrile

Intermediate A-2 (3 g, 12.71 mmol), ethyl 4,4-difluoroacetoacetate (25.42 mmol, 4.22 g), and N-methyl-2-pyrrolidone (10 mL) were added to a container. The mixture was microwaved and heated to 250 °C, and reacted for 15 minutes. The mixture was cooled to room temperature, and the solvent was removed by evaporation under reduced pressure. The residue was separated by column chromatography to give 2.5 g of a yellow solid, with a yield of 60.7%. LC-MS (APCI): m/z = 325.0 $(M+1)^+$.

Step 2: Synthesis of compound N-(4-(3-cyano-6-(difluoromethyl)-4-hydroxypyridin-2-yl)benzyl)-5-fluoro-2-methoxybenzamide

2-(4-Bromophenyl)-6-(difluoromethyl)-4-hydroxynicotinonitrile (1.5 g, 4.63 mmol), intermediate A-1 (1.74 g, 6.02 mmol), palladium(II) acetate (151 mg, 0.463 mmol), 2-dicyclohexylphosphino-2',4',6'-triisoprophylbiphenyl (220 mg, 0.463 mmol), and cesium carbonate (4.54 g, 13.89 mmol) were added to a 100 mL three-necked flask equipped with magnetic stirring and a condenser. The reaction system was evacuated and purged with nitrogen three times. Tetrahydrofuran (30 mL) and water (3 mL) were added, and the mixture was stirred to disperse. The mixture was stirred and reacted at 100 °C overnight. The mixture was cooled to room temperature, and saturated brine (50 mL) was added to quench the reaction. The organic phase was separated, and the aqueous phase was extracted with ethyl acetate (50 mL × 2). The organic phases were combined, washed with saturated brine (10 mL), dried over anhydrous sodium sulfate, filtered, concentrated, and separated by silica gel column chromatography to give 1.2 g of a yellow solid compound, with a yield of 61%. LC-MS (APCI): m/z = 428.0 $(M+1)^+$.

Step 3: Synthesis of compound N-(4-(5-bromo-3-cyano-6-(difluoromethyl)-4-hydroxypyridin-2-yl)benzyl)-5-fluoro-2-methoxybenzamide

N-(4-(3-Cyano-6-(difluoromethyl)-4-hydroxypyridin-2-yl)benzyl)-5-fluoro-2-methoxybenzamide (500 mg, 1.17 mmol) and N,N-dimethylformamide (30 mL) were added to a 100 mL single-necked flask equipped with magnetic stirring. The mixture was stirred until dissolved, and N-bromosuccinimide (313 mg, 1.76 mmol) was added. The reaction system was evacuated and purged with nitrogen three times, and the mixture was stirred at room temperature for 3 hours. A large amount of yellow solid was precipitated. The mixture was filtered, and washed with ethyl acetate (5 mL). The filter residue was collected and dried to give 530 mg of a yellow solid, with a yield of 89.5%. LC-MS (APCI): m/z = 506.0 $(M+1)^+$.

Step 4: Synthesis of compound N-(4-(5-bromo-4-chloro-3-cyano-6-(difluoromethyl)pyridin-2-yl)benzyl)-5-fluoro-2-methoxybenzamide

N-(4-(5-Bromo-3-cyano-6-(difluoromethyl)-4-hydroxypyridin-2-yl)benzyl)-5-fluoro-2-methoxybenzamide (530 mg, 1.05 mmol) and phosphorus oxychloride (10 mL) were added to a 50 mL single-necked flask equipped with magnetic stirring. The reaction system was evacuated and purged with nitrogen three times. The mixture was stirred and reacted at 90 °C for 2 hours. The mixture was cooled to room temperature, and concentrated to dryness under reduced pressure. Saturated aqueous solution of sodium bicarbonate (30 mL) was added with stirring, and the mixture was extracted with ethyl acetate (30 mL $\times$ 2). The organic phases were combined, washed with saturated brine (30 mL), dried over anhydrous sodium sulfate, filtered, concentrated, and separated by silica gel column chromatography to give 270 mg of a white solid, with a yield of 49.3%. LC-MS(APCI): m/z=524.0 $(M+1)^+$.

Step 5: Synthesis of intermediate B-8

N-(4-(5-Bromo-4-chloro-3-cyano-6-(difluoromethyl)pyridin-2-yl)benzyl)-5-fluoro-2-methoxybenzamide (270 mg, 0.516 mmol) and N,N-dimethylformamide (5 mL) were added to a 15 mL sealed vial equipped with magnetic stirring. The mixture was stirred until dissolved, and hydrazine hydrate (0.5 mL) was added. The vial was sealed, and the mixture was stirred and reacted at **110** °C for 2 hours. The mixture was cooled to room temperature, and saturated brine (10 mL) was added to quench the reaction. The organic phase was separated, and the aqueous phase was extracted with ethyl acetate (10 mL $\times$ 2). The organic phases were combined, washed with saturated brine (10 mL), dried over anhydrous sodium sulfate, filtered, concentrated, and separated by silica gel column chromatography to give 240 mg of a white solid, with a yield of 89.6%. LC-MS (APCI): m/z = 520.0 $(M+1)^+$.

**Preparation of intermediate B-9 4-(4-bromophenyl)-6-(o-tolyl)-1H-pyrazolo[4,3-c]pyridin-3-amine**

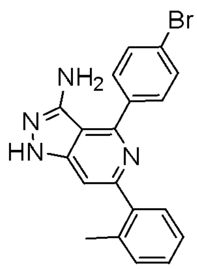

B-9

**The following synthetic route was adopted**

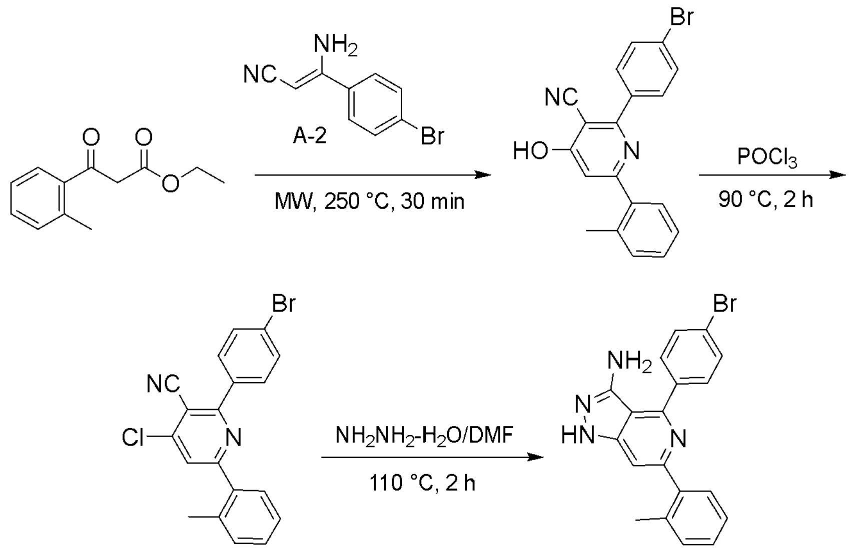


B-9

Step 1: Synthesis of compound 2-(4-bromophenyl)-4-hydroxy-6-(o-tolyl)nicotinonitrile

Ethyl 3-oxo-3-(o-tolyl)propanoate (3.7 g, 18 mmol) and intermediate A-2 (1.0 g, 4.5 mmol) were added to a 20 mL microwave vial equipped with magnetic stirring. The vial was sealed. The mixture was microwaved and heated to 250 °C, and reacted for 30 min. The mixture was cooled to room temperature, and saturated brine (20 mL) was added to quench the reaction. The mixture was extracted with ethyl acetate (20 mL × 2). The organic phases were combined, concentrated, and separated by silica gel column chromatography to give 320 mg of a white solid, with a yield of 19.5%. LC-MS (APCI): m/z = 365.0 $(M+1)^+$.

Step 2: Synthesis of compound 2-(4-bromophenyl)-4-chloro-6-(o-tolyl)nicotinonitrile

2-(4-Bromophenyl)-4-hydroxy-6-(o-tolyl)nicotinonitrile (320 mg, 0.88 mmol) and phosphorus oxychloride (5 mL) were added to a 50 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (10 mL) was added to quench the reaction. The mixture was extracted with ethyl acetate (10 mL × 2). The organic phases were combined, washed with saturated brine (10 mL), dried over anhydrous sodium sulfate, filtered, concentrated, and separated by silica gel column chromatography to give 100 mg of a white solid, with a yield of 29.7%. LC-MS (APCI): m/z = 383.0 $(M+1)^+$.

Step 3 Synthesis of intermediate B-9

2-(4-Bromophenyl)-4-chloro-6-(o-tolyl)nicotinonitrile (100 mg, 0.26 mmol), hydrazine hydrate (0.2 mL, 3.15 mmol), and N,N-dimethylformamide (3 mL) were added to a 15 mL sealed vial equipped with magnetic stirring. The vial was sealed, and the mixture was stirred and reacted at **110** °C for 2 hours. The mixture was cooled to room temperature, and saturated brine (5 mL) was added to quench the reaction. The organic phase was separated, and the aqueous phase was extracted with ethyl acetate (5 mL × 2). The organic phases were combined, washed with saturated brine (5 mL), dried over anhydrous sodium sulfate, filtered, concentrated, and separated by silica gel column chromatography to give 60 mg of a white solid, with a yield of 61.0%. LC-MS (APCI): m/z = 379.0 $(M+1)^+$.

**Preparation of intermediate B-10 4-(4-bromophenyl)-6-(2-chlorophenyl)-1H-pyrazolo[4,3-c]pyridin-3-amine**

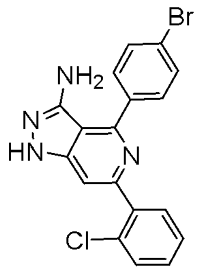

B-10

**The following synthetic route was adopted**

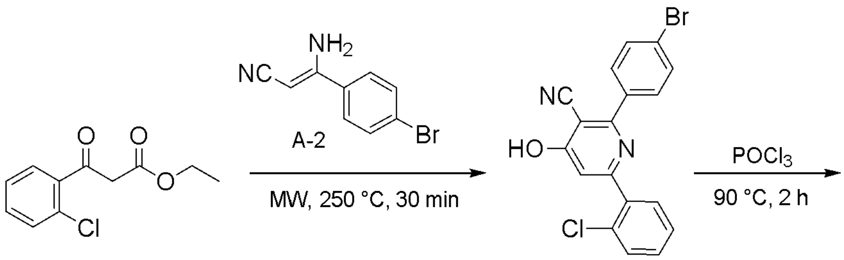


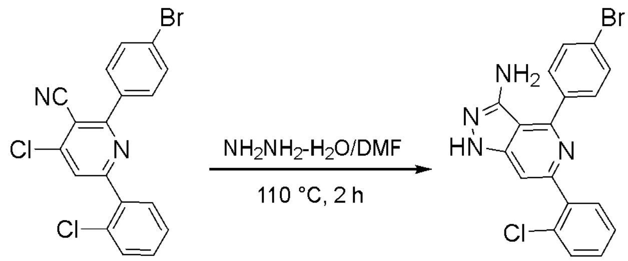


B-10

Step 1: Synthesis of compound 2-(4-bromophenyl)-6-(2-chlorophenyl)-4-hydroxynicotinonitrile

Ethyl 3-(2-chlorophenyl)-3-oxopropanoate (5.0 g, 22 mmol) and intermediate A-2 (2.0 g, 9 mmol) were added to a 20 mL microwave vial equipped with magnetic stirring. The vial was sealed. The mixture was microwaved and heated to 250 °C, and reacted for 30 min. The mixture was cooled to room temperature, and saturated brine (20 mL) was added to quench the reaction. The mixture was extracted with ethyl acetate (20 mL × 2). The organic phases were combined, concentrated, and separated by silica gel column chromatography to give 1.0 g of a white solid, with a yield of 28.9%. LC-MS (APCI): m/z = 384.0 $(M+1)^+$.

Step 2: Synthesis of compound 2-(4-bromophenyl)-4-chloro-6-(2-chlorophenyl)nicotinonitrile

2-(4-Bromophenyl)-6-(2-chlorophenyl)-4-hydroxynicotinonitrile (1.0 g, 2.6 mmol) and phosphorus oxychloride (10 mL) were added to a 50 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (10 mL) was added to quench the reaction. The mixture was extracted with ethyl acetate (10 mL × 2). The organic phases were combined, washed with saturated brine (10 mL), dried over anhydrous sodium sulfate, filtered, concentrated, and separated by silica gel column chromatography to give 710 mg of a white solid, with a yield of 67.9%. LC-MS (APCI): m/z = 403.0 $(M+1)^+$.

Step 3: Synthesis of intermediate B-10

2-(4-Bromophenyl)-4-chloro-6-(2-chlorophenyl)nicotinonitrile (710 mg, 1.77 mmol), hydrazine hydrate (0.8 mL, 3.15 mmol), and N,N-dimethylformamide (3 mL) were added to a 15 mL sealed vial equipped with magnetic stirring. The vial was sealed, and the mixture was stirred and reacted at 110 °C for 2 hours. The mixture was cooled to room temperature, and saturated brine (5 mL) was added to quench the reaction. The organic phase was separated, and the

aqueous phase was extracted with ethyl acetate (5 mL × 2). The organic phases were combined, washed with saturated brine (5 mL), dried over anhydrous sodium sulfate, filtered, concentrated, and separated by silica gel column chromatography to give 60 mg of a white solid, with a yield of 61.0%. LC-MS (APCI): m/z = 399.0 (M+1)+.

**Preparation of intermediate B-11 4-(4-bromophenyl)-6-(2-chlorophenyl)-1H-pyrazolo[4,3-c]pyridin-3-amine**

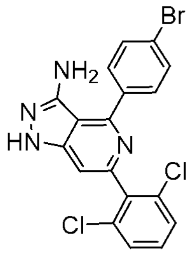

B-11

**The following synthetic route was adopted**

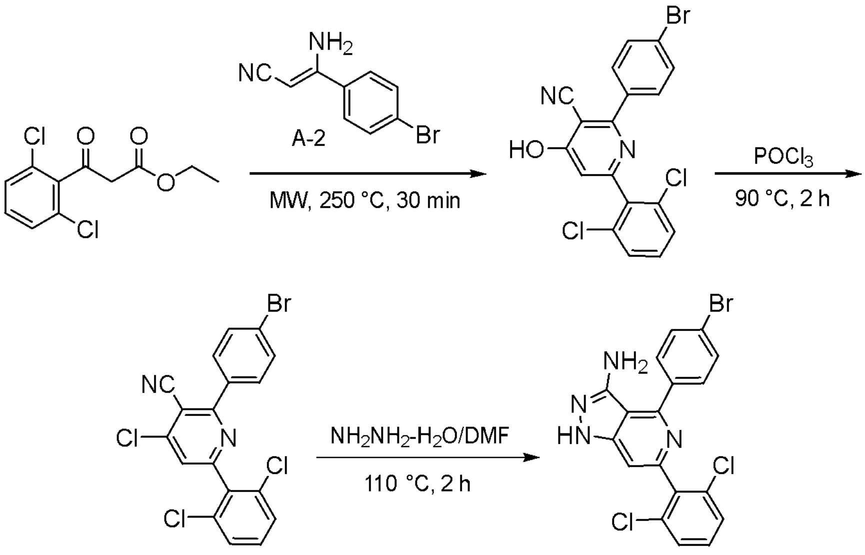


B-11

Step 1: Synthesis of compound 2-(4-bromophenyl)-6-(2,6-dichlorophenyl)-4-hydroxynicotinonitrile

Ethyl 3-(2,6-dichlorophenyl)-3-oxopropanoate (4.7 g, 18 mmol) and intermediate A-2 (1.0 g, 4.5 mmol) were added to a 20 mL microwave vial equipped with magnetic stirring. The vial was sealed. The mixture was microwaved and heated to 250 °C, and reacted for 30 min. The mixture was cooled to room temperature, and saturated brine (20 mL) was added to quench the reaction. The mixture was extracted with ethyl acetate (20 mL × 2). The organic phases were combined, concentrated, and separated by silica gel column chromatography to give 200 mg of a white solid, with a yield of 10.6%. LC-MS (APCI): m/z = 418.0 (M+1)+.

Step 2: Synthesis of compound 2-(4-bromophenyl)-4-chloro-6-(2,6-dichlorophenyl)nicotinonitrile

2-(4-Bromophenyl)-6-(2,6-dichlorophenyl)-4-hydroxynicotinonitrile (200 mg, 0.48 mmol) and phosphorus oxychloride (5 mL) were added to a 50 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (5 mL) was added to quench the reaction. The mixture was extracted with ethyl acetate (5 mL × 2). The organic phases were combined, washed with saturated brine (5 mL), dried over anhydrous sodium sulfate, filtered, concentrated, and separated by silica gel column chromatography to give 90 mg of a white solid, with a yield of 43.0%. LC-MS (APCI): m/z

= 437.0 (M+1)$^+$.

Step 3 Synthesis of intermediate B-11

2-(4-Bromophenyl)-4-chloro-6-(2,6-dichlorophenyl)nicotinonitrile (90 mg, 0.21 mmol), hydrazine hydrate (0.2 mL, 0.78 mmol), and N,N-dimethylformamide (3 mL) were added to a 15 mL sealed vial equipped with magnetic stirring. The vial was sealed, and the mixture was stirred and reacted at 110 °C for 2 hours. The mixture was cooled to room temperature, and saturated brine (5 mL) was added to quench the reaction. The organic phase was separated, and the aqueous phase was extracted with ethyl acetate (5 mL × 2). The organic phases were combined, washed with saturated brine (5 mL), dried over anhydrous sodium sulfate, filtered, concentrated, and separated by silica gel column chromatography to give 60 mg of a white solid, with a yield of 61.0%. LC-MS (APCI): m/z = 433.0 (M+1)$^+$.

**Preparation of intermediate B-12 4-(3-amino-4-(4-bromophenyl)-1H-pyrazolo[4,3-c]pyridin-6-vl)benzoic acid**

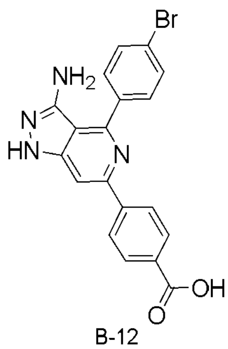

B-12

**The following synthetic route was adopted**

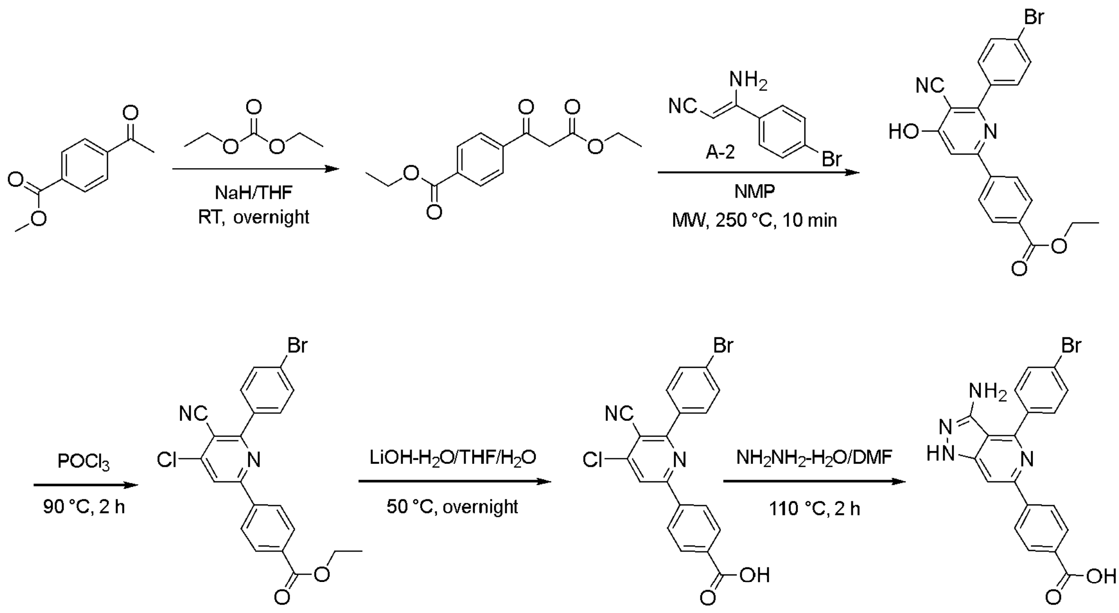


B-12

Step 1: Synthesis of compound ethyl 4-(3-ethoxy-3-oxopropanoyl)benzoate

Methyl 4-acetylbenzoate (4.0 g, 22.45 mmol) and anhydrous tetrahydrofuran (60 mL) were added to a 250 mL three-necked flask equipped with magnetic stirring. The mixture was cooled in an ice-water bath, and sodium hydride (2.5 g, 62.86 mmol) was added in batches under nitrogen. The mixture was stirred and reacted in an ice bath for 30 minutes, and diethyl carbonate (5.3 g, 44.9 mmol) was added. The mixture was stirred at room temperature overnight. The flask was

placed in an ice bath, and saturated aqueous solution of ammonium chloride (50 mL) was added to quench the reaction. The mixture was extracted with ethyl acetate (50 mL × 2). The organic phases were combined, concentrated, and separated by silica gel column chromatography to give 4.1 g of a white solid, with a yield of 69.2%. LC-MS (APCI): m/z = 265.0 $(M+1)^+$.

Step 2: Synthesis of compound ethyl 4-(6-(4-bromophenyl)-5-cyano-4-hydroxypyridin-2-yl)benzoate

Ethyl 4-(3-ethoxy-3-oxopropanoyl)benzoate (1.1 g, 4.17 mmol), intermediate A-2 (700 mg, 3.15 mmol), and N-methyl-2-pyrrolidone (5 mL) were added to a 20 mL microwave vial equipped with magnetic stirring. The vial was sealed. The mixture was microwaved and heated to 250 °C, and reacted for 10 minutes. The mixture was cooled to room temperature, and saturated brine (20 mL) was added to quench the reaction. The mixture was extracted with ethyl acetate (20 mL × 2). The organic phases were combined, concentrated, and separated by silica gel column chromatography to give 500 mg of a white solid, with a yield of 37.6%. LC-MS (APCI): m/z = 423.0 $(M+1)^+$.

Step 3: Synthesis of compound ethyl 4-(6-(4-bromophenyl)-4-chloro-5-cyanopyridin-2-yl)benzoate

Ethyl 4-(6-(4-bromophenyl)-5-cyano-4-hydroxypyridin-2-yl)benzoate (500 mg, 1.18 mmol) and phosphorus oxychloride (5 mL) were added to a 25 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (5 mL) was added to quench the reaction. The mixture was extracted with ethyl acetate (5 mL × 2). The organic phases were combined, washed with saturated brine (5 mL), dried over anhydrous sodium sulfate, filtered, concentrated, and separated by silica gel column chromatography to give 210 mg of a white solid, with a yield of 40.4%. LC-MS (APCI): m/z = 441.0 $(M+1)^+$.

Step 4: Synthesis of compound 4-(6-(4-bromophenyl)-4-chloro-5-cyanopyridin-2-yl)benzoic acid

Ethyl 4-(6-(4-bromophenyl)-4-chloro-5-cyanopyridin-2-yl)benzoate (210 mg, 0.47 mmol), lithium hydroxide monohydrate (120 mg, 2.86 mmol), tetrahydrofuran (4 mL), and water (4 mL) were added to a 50 mL three-necked flask equipped with magnetic stirring and a condenser. The reaction system was evacuated and purged with nitrogen three times. The mixture was stirred to disperse, and stirred and reacted at 50 °C overnight. The mixture was cooled to room temperature, and saturated brine (5 mL) was added to quench the reaction. The organic phase was separated, and the aqueous phase was extracted with ethyl acetate (5 mL × 2). The organic phases were combined, washed with saturated brine (5 mL), dried over anhydrous sodium sulfate, filtered, concentrated, and separated by silica gel column chromatography to give 120 mg of a white solid, with a yield of 62.0%. LC-MS (APCI): m/z = 413.0 $(M+1)^+$.

Step 5: Synthesis of intermediate B-12

4-(6-(4-Bromophenyl)-4-chloro-5-cyanopyridin-2-yl)benzoic acid (120 mg, 0.29 mmol), hydrazine hydrate (0.2 mL, 0.78 mmol) and N,N-dimethylformamide (3 mL) were added to a 15 mL sealed vial equipped with magnetic stirring. The vial was sealed, and the mixture was stirred and reacted at 110 °C for 2 hours. The mixture was cooled to room temperature, and saturated brine (5 mL) was added to quench the reaction. The organic phase was separated, and the aqueous phase was extracted with ethyl acetate (5 mL × 2). The organic phases were combined, washed with saturated brine (5 mL), dried over anhydrous sodium sulfate, filtered, concentrated, and separated by silica gel column chromatography to give 73 mg of a white solid, with a yield of 61.7 %. LC-MS (APCI): m/z = 409.0 $(M+1)^+$.

**Preparation of intermediate B-13 4-(4-bromophenyl)-6-(tetrahydro-2H-thiopyran-4-yl)-1H-pyrazolo**[4,3-c]pyridin-3-amine

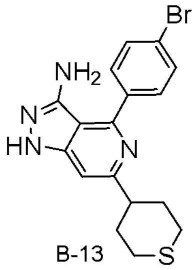

B-13

The following synthetic route was **adopted**

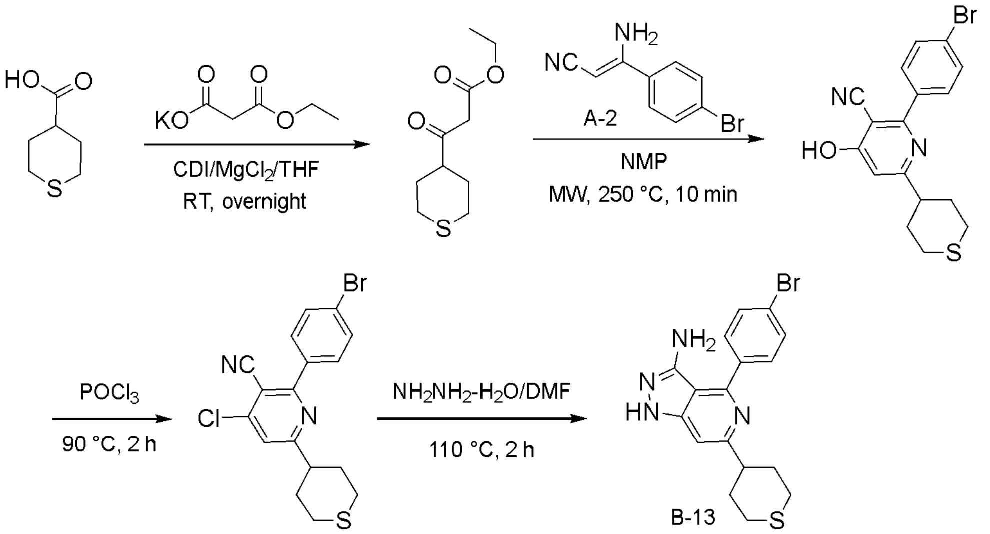


B-13

Step 1: Synthesis of compound ethyl 3-oxo-3-(tetrahydrothiopyran-4-yl)propanoate

Tetrahydrothiopyran-4-carboxylic acid (3.0 g, 20.5 mmol), N,N'-carbonyldiimidazole (3.99 g, 24.6 mmol), and anhydrous tetrahydrofuran (40 mL) were added to a 100 mL three-necked flask equipped with magnetic stirring. The mixture was stirred and reacted at room temperature for 5 hours. Potassium 3-ethoxy-3-oxopropanoate (6.98 g, 41 mmol) and magnesium chloride (1.95 g, 20.5 mmol) were added, and the mixture was stirred at room temperature overnight. The flask was placed in an ice bath, and saturated saline (40 mL) was added to quench the reaction. The mixture was extracted with ethyl acetate (40 mL × 2). The organic phases were combined, concentrated, and separated by silica gel column chromatography to give 3.1 g of a white solid, with a yield of 70.0%. LC-MS (APCI): m/z = 217.0 $(M+1)^+$.

Step 2: Synthesis of compound 2-(4-bromophenyl)-4-hydroxy-6-(tetrahydro-2H-thiopyran-4-yl)nicotinonitrile

Ethyl 3-oxo-3-(tetrahydrothiopyran-4-yl)propanoate(1.97 g, 9.12 mmol), intermediate A-2 (1.5 g, 6.75 mmol), and N-methyl-2-pyrrolidone (10 mL) were added to a 20 mL microwave vial equipped with magnetic stirring. The vial was sealed. The mixture was microwaved and heated to 250 °C, and reacted for 10 min. The mixture was cooled to room temperature, and saturated brine (20 mL) was added to quench the reaction. The mixture was extracted with ethyl acetate (20 mL × 2). The organic phases were combined, concentrated, and separated by silica gel column chromatography to give 1.02 g of a white solid, with a yield of 40.5 %. LC-MS (APCI): m/z = 375.0 $(M+1)^+$.

Step 3: Synthesis of compound 2-(4-bromophenyl)-4-chloro-6-(tetrahydro-2H-thiopyran-4-yl)nicotinonitrile

2-(4-Bromophenyl)-4-hydroxy-6-(tetrahydro-2H-thiopyran-4-yl)nicotinonitrile (1.02 g, 2.73 mmol) and phosphorus oxychloride (10 mL) were added to a 25 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (10 mL) was added to quench the reaction. The mixture was extracted with ethyl acetate (10 mL × 2). The organic phases were combined, washed with saturated brine (10 mL), dried over anhydrous sodium sulfate, filtered, concentrated, and separated by silica gel column chromatography to give 610 mg of a light yellow solid, with a yield of

57.1%. LC-MS (APCI): m/z = 393.0 $(M+1)^+$.

Step 4: Synthesis of intermediate B-13

2-(4-Bromophenyl)-4-chloro-6-(tetrahydro-2H-thiopyran-4-yl)nicotinonitrile (610 mg, 1.56 mmol), hydrazine hydrate (0.6 mL, 2.34 mmol), and N,N-dimethylformamide (6 mL) were added to a 15 mL sealed vial equipped with magnetic stirring. The vial was sealed, and the mixture was stirred and reacted at 110 °C for 2 hours. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 330 mg of a yellow solid, with a yield of 54.7%. LC-MS (APCI): m/z = 389.0 (M+1)+.

**Preparation of intermediate B-14 N-(4-(6-chloro-3-cyano-4-hydrazinylpyridin-2-yl)benzyl)-5-fluoro-2-methoxybenzamide hydrochloride**

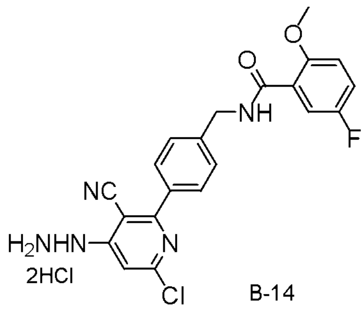

B-14

**The following synthetic route was adopted**

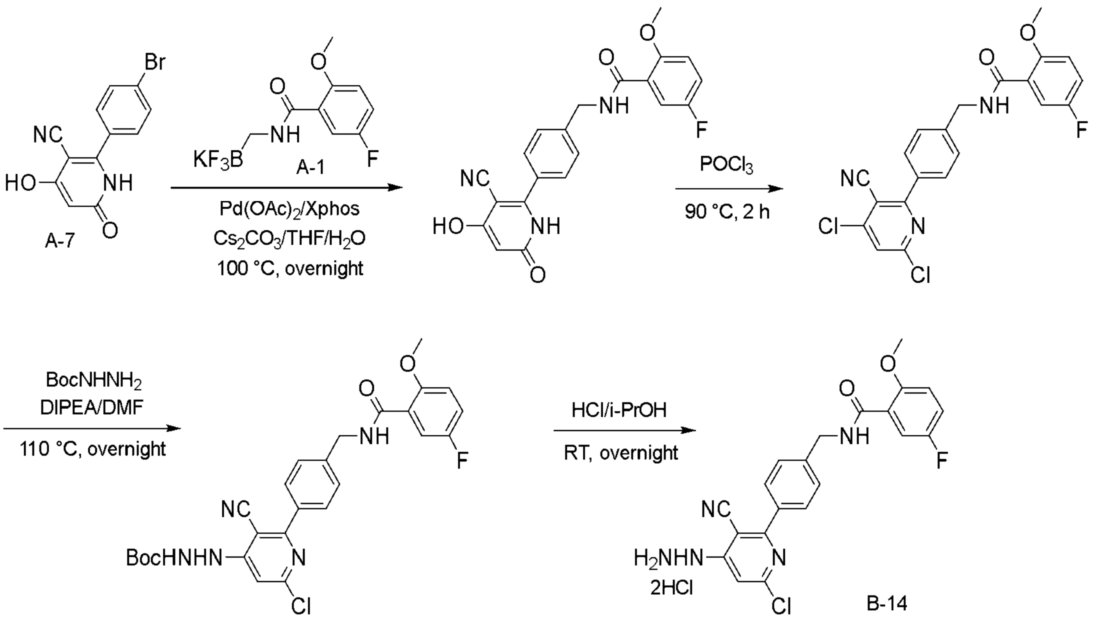


Step 1: Synthesis of compound N-(4-(3-cyano-4-hydroxy-6-oxo-1,6-dihydropyridin-2-yl)benzyl)-5-fluoro-2-methoxybenzamide

Intermediate A-7 (2.0 g, 7 mmol), intermediate A-1 (3.03 mg, 10.5 mmol), palladium(II) acetate (157.1 mg, 0.7 mmol), 2-dicyclohexylphosphino-2',4',6'-triisoprophylbiphenyl (667.4 mg, 1.4 mmol), and cesium carbonate (6.84 g, 21 mmol) were added to a 50 mL three-necked flask equipped with magnetic stirring and a condenser. The reaction system was evacuated and purged with nitrogen three times. Tetrahydrofuran (30 mL) and water (6 mL) were added, and the

mixture was stirred to disperse. The mixture was stirred and reacted at 100 °C overnight. The mixture was cooled to room temperature, and saturated brine (30 mL) was added to quench the reaction. The organic phase was separated, and the aqueous phase was extracted with ethyl acetate (30 mL × 2). The organic phases were combined, washed with saturated brine (30 mL), dried over anhydrous sodium sulfate, filtered, concentrated, and separated by silica gel column chromatography to give 1.4 g of a white solid, with a yield of 50.8 %. LC-MS (APCI): m/z = 394.0 $(M+1)^+$.

Step 2: Synthesis of compound N-(4-(4,6-dichloro-3-cyanopyridin-2-yl)benzyl)-5-fluoro-2-methoxybenzamide

N-(4-(3-Cyano-4-hydroxy-6-oxo-1,6-dihydropyridin-2-yl)benzyl)-5-fluoro-2-methoxybenzamide (1.4 g, 3.56 mmol) and phosphorus oxychloride (15 mL) were added to a 25 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (20 mL) was added to quench the reaction. The mixture was extracted with ethyl acetate (20 mL × 2). The organic phases were combined, washed with saturated brine (20 mL), dried over anhydrous sodium sulfate, filtered, concentrated, and separated by silica gel column chromatography to give 700 mg of a light yellow solid, with a yield of 74.7 %. LC-MS (APCI): m/z = 430.0 $(M+1)^+$.

Step 3: Synthesis of compound tert-butyl 2-(6-chloro-3-cyano-2-(4-((5-fluoro-2-methoxybenzoylamino)methyl)phenyl) pyridin-4-yl)hydrazine-1-carboxylate

N-(4-(4,6-Dichloro-3-cyanopyridin-2-yl)benzyl)-5-fluoro-2-methoxybenzamide (700 mg, 1.63 mmol), tert-butyl carbazate (237.2 mg, 1.79 mmol), N,N-diisopropylethylamine (0.57 mL, 4.89 mmol), and N,N-dimethylformamide (7 mL) were added to a 25 mL three-necked flask equipped with magnetic stirring and a condenser. The flask was sealed, and the mixture was stirred and reacted at 110 °C overnight. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 500 mg of a white solid, with a yield of 58.4%. LC-MS (APCI): m/z = 526.0 $(M+1)^+$.

Step 4: Synthesis of intermediate B-14

tert-Butyl 2-(6-chloro-3-cyano-2-(4-((5-fluoro-2-methoxybenzoylamino)methyl)phenyl)pyridin-4-yl)hydrazine-1-carboxylate (500 mg, 0.95 mmol) and a solution of hydrochloride in isopropanol (8 mL, 5 M) were added to a 50 mL single-necked flask equipped with magnetic stirring. The mixture was stirred and reacted at room temperature overnight. The reaction solution was concentrated to give 320 mg of a white solid, with a yield of 67.6%. LC-MS (APCI): m/z = 426.0 $(M+1)^+$.

**Preparation of intermediate B-15 N-(4-(3-amino-6-(piperidin-3-yl)-1H-pyrazolo[4,3-c]pyridin-4-yl)benzyl)-5-fluoro-2-methoxybenzamide**

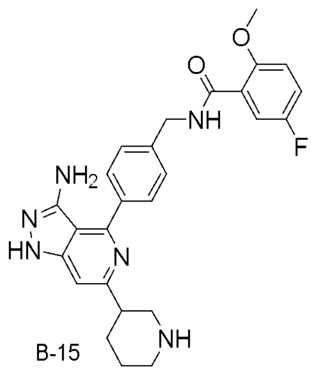

B-15

The following synthetic route was **adopted**

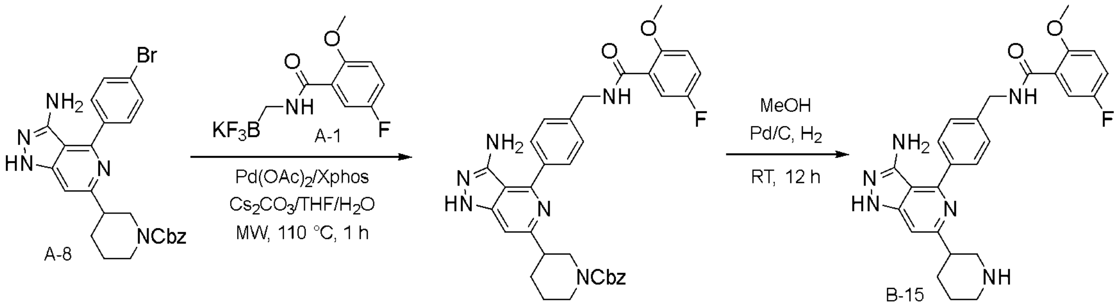


Step 1: Synthesis of compound benzyl 3-(3-amino-4-(4-((5-fluoro-2-methoxybenzoylamino)methyl)phenyl)-1H-pyrazolo[4,3-c]pyridin-6-yl)piperidine-1-carboxylate

Intermediate A-8 (500 mg, 0.990 mmol), intermediate A-1(343.3 mg, 1.188 mmol), palladium(II) acetate (11.1 mg, 0.050 mmol), 2-dicyclohexylphosphino-2',4',6'-triisoprophylbiphenyl (47.2 mg, 0.099 mmol), cesium carbonate (968.2 mg, 2.97 mmol), tetrahydrofuran (10 mL) and water (1 mL) were added to a 20 mL microwave vial equipped with magnetic stirring. The mixture was microwaved and reacted at 110 °C under nitrogen for 1 hour. Water (20 mL) was added, and the mixture was extracted with ethyl acetate (20 mL × 2). The organic phases were combined, washed with saturated brine (30 mL), dried over anhydrous sodium sulfate, filtered, concentrated, and separated by silica gel column chromatography to give 270 mg of a white solid, with a yield of 44.4%. LC-MS (APCI): m/z = 609.0 $(M+1)^+$.

Step 2 Synthesis of intermediate B-15

Benzyl 3-(3-amino-4-(4-((5-fluoro-2-methoxybenzoylamino)methyl)phenyl)-1H-pyrazolo[4,3-c]pyridin-6-yl)piperidine-1-carboxylate (270 mg, 0.444 mmol), palladium on carbon (10%, 100 mg) and methanol (15 mL) were added to a 50 mL three-necked flask equipped with magnetic stirring. The mixture was stirred and reacted at room temperature under hydrogen for 12 hours. The mixture was filtered. The filtrate was concentrated and separated by silica gel column chromatography to give 210 mg of a white solid, with a yield of 99.9%. LC-MS (APCI): m/z = 475.0 $(M+1)^+$.

**Preparation of intermediate B-16 N-(4-(3-amino-6-(piperidin-4-yl)-1H-pyrazolo[4,3-c]pyridin-4-yl)benzyl)-5-fluoro-2-methoxybenzamide**

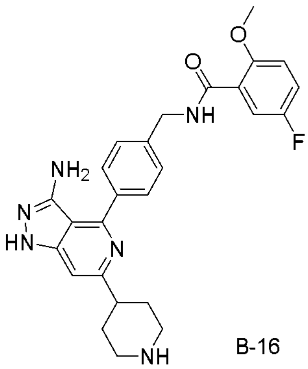

B-16

**The following synthetic route was adopted**

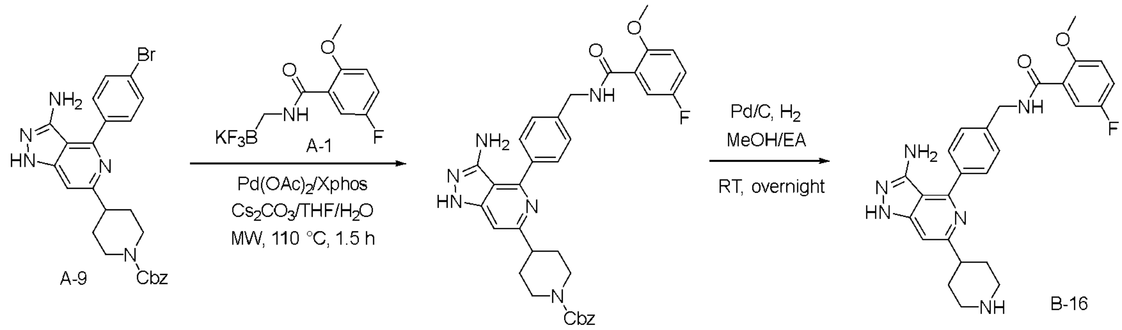


Step 1: Synthesis of compound benzyl 4-(3-amino-4-(4-((5-fluoro-2-methoxybenzoylamino)methyl)phenyl)-1H-pyrazolo[4,3-c]pyridin-6-yl)piperidine-1-carboxylate

Intermediate A-9 (1.05 g, 2.1 mmol), intermediate A-1 (605 mg, 2.1 mmol), tetrahydrofuran (15 mL), palladium(II) acetate (23.5 mg, 0.105 mmol), 2-dicyclohexylphosphino-2',4',6'-triisoprophylbiphenyl (100 mg, 0.21 mmol), cesium carbonate (2.05 g, 6.3 mmol), and water (4 mL) were added to a container. The reaction solution was purged with nitrogen for 2 minutes. The mixture was microwaved and heated to 110 °C, and reacted for 1.5 hours. The reaction solution was cooled, and then diluted with added water (50 mL). The mixture was extracted with ethyl acetate (100 mL × 3). The organic phase was washed with saturated brine (100 mL), dried over anhydrous sodium sulfate, and concentrated. The residue was separated by silica gel column chromatography to give 510 mg of a light yellow solid, with a yield of 40%. LC-MS (APCI): m/z = 609.0 $(M+1)^+$.

Step 2: Synthesis of intermediate B-16

Benzyl 4-(3-amino-4-(4-((5-fluoro-2-methoxybenzoylamino)methyl)phenyl)-1H-pyrazolo[4,3-c]pyridin-6-yl)piperidine-1-carboxylate (510 mg, 0.84 mmol), methanol/ethyl acetate (v/v = 1/1, 20 mL), and palladium on carbon (10%, 500 mg) were added to a container. The reaction system was purged with hydrogen three times. The mixture was reacted at room temperature under hydrogen overnight. The mixture was filtered, and the filter residue was washed with methanol/ethyl acetate. The filtrate was concentrated, and the residue was separated by silica gel column chromatography to give 350 mg of a white solid, with a yield of 87%. LC-MS (APCI): m/z = 475.0 $(M+1)^+$.

**Preparation of intermediate B-17 4-(4-bromophenyl)-6-(tetrahydro-2H-pyran-4-yl)-1H-pyrazolo[4,3-c]pyridin-3-amine**

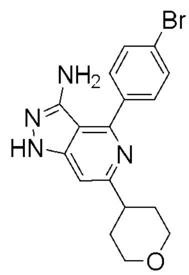

B-17

**The following synthetic route was adopted**

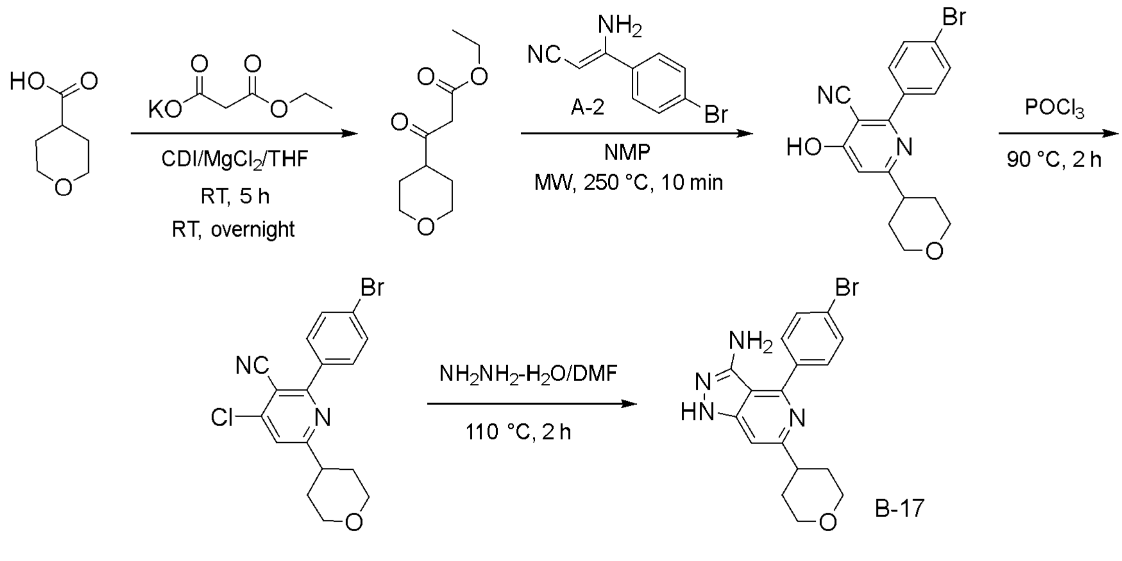


B-17

Step 1: Synthesis of compound ethyl 3-oxo-3-(tetrahydro-2H-pyran-4-yl)propanoate

Tetrahydropyran-4-carboxylic acid (2.66 g, 20.5 mmol), N,N'-carbonyldiimidazole (3.99 g, 24.6 mmol), and anhydrous tetrahydrofuran (40 mL) were added to a 100 mL three-necked flask equipped with magnetic stirring. The mixture was stirred and reacted at room temperature for 5 hours. Potassium 3-ethoxy-3-oxopropanoate (6.98 g, 41 mmol) and magnesium chloride (1.95 g, 20.5 mmol) were added, and the mixture was stirred at room temperature overnight. The flask was placed in an ice bath, and saturated saline (40 mL) was added to quench the reaction. The mixture was extracted with ethyl acetate (40 mL × 2). The organic phases were combined, concentrated, and separated by silica gel column chromatography to give 2.5 g of a white solid, with a yield of 60.9%. LC-MS (APCI): m/z = 201.0 $(M+1)^+$.

Step 2: Synthesis of compound 2-(4-bromophenyl)-4-hydroxy-6-(tetrahydro-2H-pyran-4-yl)nicotinonitrile

Ethyl 3-oxo-3-(tetrahydro-2H-pyran-4-yl)propanoate (1.82 g, 9.12 mmol), intermediate A-2 (1.5 g, 6.75 mmol), and N-methyl-2-pyrrolidone (10 mL) were successively added to a 20 mL microwave vial equipped with magnetic stirring. The vial was sealed. The mixture was microwaved and heated to 250 °C, and reacted for 10 min. The mixture was cooled to room temperature, and saturated brine (20 mL) was added to quench the reaction. The mixture was extracted with ethyl acetate (20 mL × 2). The organic phases were combined, concentrated, and separated by silica gel column chromatography to give 1.31 g of a white solid, with a yield of 54.2%. LC-MS(APCI): m/z=359.0(M+1)+.

Step 3: Synthesis of compound 2-(4-bromophenyl)-4-chloro-6-(tetrahydro-2H-pyran-4-yl)nicotinonitrile

2-(4-Bromophenyl)-4-hydroxy-6-(tetrahydro-2H-pyran-4-yl)nicotinonitrile (0.98 g, 2.73 mmol) and phosphorus oxychloride (10 mL) were added to a 25 mL single-necked flask equipped with magnetic stirring. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (10 mL) was added to quench the reaction. The mixture was extracted with ethyl acetate (10 mL × 2). The organic phases were combined, washed with saturated brine (10 mL), dried over anhydrous sodium sulfate, filtered, concentrated, and separated by silica gel column chromatography to give 650 mg of a light yellow solid, with a yield of 63.5%. LC-MS (APCI): m/z = 376.0 $(M+1)^+$.

Step 4: Synthesis of intermediate B-17

2-(4-Bromophenyl)-4-chloro-6-(tetrahydro-2H-pyran-4-yl)nicotinonitrile (585 mg, 1.56 mmol), hydrazine hydrate (0.6 mL, 2.34 mmol), and N,N-dimethylformamide (6 mL) were added to a 15 mL sealed vial equipped with magnetic stirring. The vial was sealed, and the mixture was stirred and reacted at 110 °C for 2 hours. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 320 mg of a yellow solid, with a yield of 55.0%. LC-MS (APCI): m/z = 373.2 $(M+1)^+$.

**Preparation of intermediate B-18 ((1r,4r)-4-(3-amino-4-(4-bromophenyl)-1H-pyrazolo[4,3-c]pyridin-6-yl)cyclohexyl)methanol**

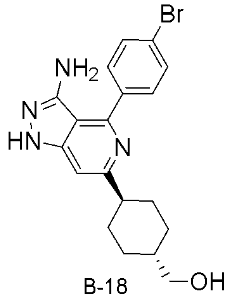

B-18

**The following synthetic route was adopted**

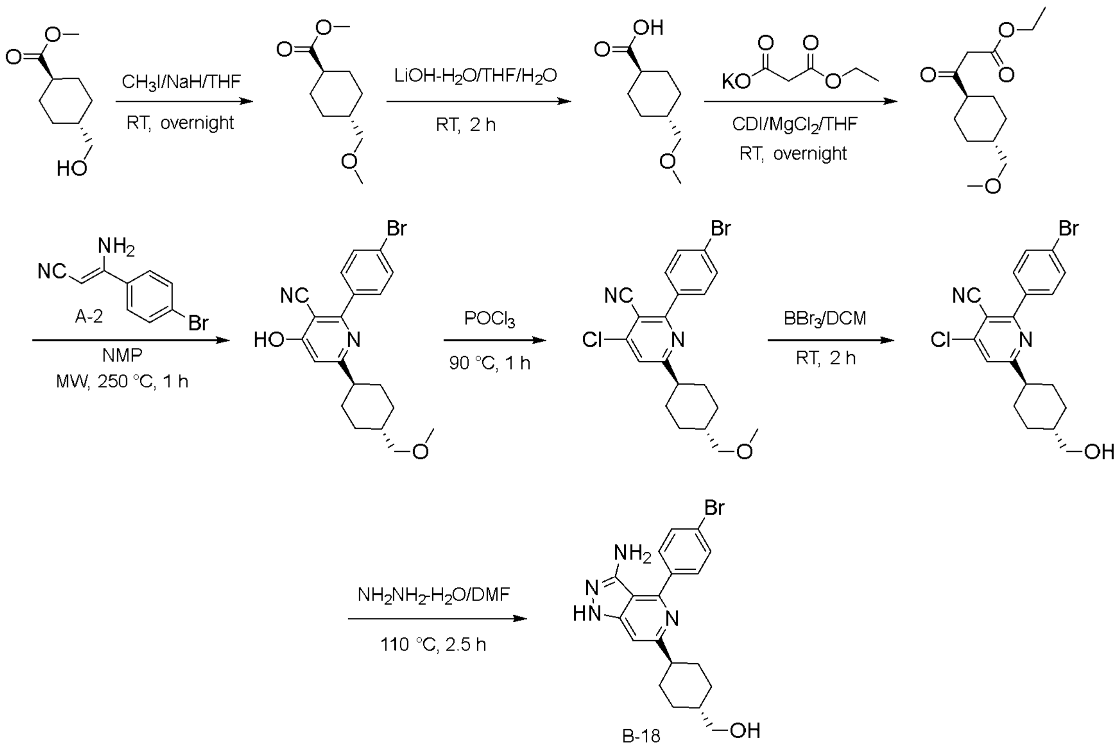


Step 1: Synthesis of compound methyl (1r,4r)-4-(methoxymethyl)cyclohexane-1-carboxylate

Methyl (1r,4r)-4-(hydroxymethyl)cyclohexane-1-carboxylate (2.0 g, 11.61 mmol) and anhydrous tetrahydrofuran (20 mL) were successively added to a 100 mL three-necked flask equipped with magnetic stirring. The mixture was stirred until dissolved, and cooled to 0 °C. Sodium hydride (0.31 g, 12.77 mmol) was added, and the mixture was stirred at the same temperature for 10 minutes. Iodomethane (1.82 g, 12.77 mmol) was added. The ice bath was removed, and the mixture was stirred at room temperature under nitrogen overnight. Saturated brine (30 mL) was added to quench the reaction, and ethyl acetate (30 mL) was added. The mixture was stirred for 5 minutes, and the organic phase was separated. The aqueous phase was extracted with ethyl acetate (30 mL). The organic phases were combined, washed with saturated brine (50 mL), dried over anhydrous sodium sulfate, filtered, concentrated, and separated by silica gel column chromatography to give 1.8 g of a solid, with a yield of 83.3%. LC-MS (APCI): m/z = 187.1 $(M+1)^+$.

Step 2: Synthesis of compound (1r,4r)-4-(methoxymethyl)cyclohexane-1-carboxylic acid

Methyl (1r,4r)-4-(methoxymethyl)cyclohexane-1-carboxylate (1.8 g, 9.67 mmol), tetrahydrofuran (15 mL), and

water (15 mL) were added to a 100 mL single-necked flask equipped with magnetic stirring. The mixture was stirred until dissolved, and lithium hydroxide monohydrate (609 mg, 14.50 mmol) was added. The mixture was stirred and reacted at room temperature for 2 hours. The organic solvent was removed by evaporation under reduced pressure, and hydrochloric acid (1 M) was added to adjust the pH to approximately 3. The mixture was extracted with ethyl acetate (20 mL × 3). The organic phases were combined, wash with saturated brine (50 mL), dried over anhydrous sodium sulfate, filtered, concentrated, and separated by silica gel column chromatography to give 1.51 g of a solid, with a yield of 90.3%. LC-MS (APCI): m/z = 173.3 $(M+1)^+$.

Step 3: Synthesis of compound ethyl 3-((1r,4r)-4-(methoxymethyl)cyclohexyl)-3-oxopropanoate

(1r,4r)-4-(Methoxymethyl)cyclohexane-1-carboxylic acid (1.5 g, 8.72 mmol), monoethyl potassium malonate (1.78 g, 10.46 mmol), N,N'-carbonyldiimidazole (1.62 g, 10.46 mmol), magnesium chloride (0.83 g, 8.72 mmol), and tetrahydrofuran (20 mL) were successively added to a 100 mL single-necked flask equipped with magnetic stirring. The mixture was stirred at room temperature under nitrogen overnight. Hydrochloric acid (1 M) was added to adjust the pH to 2-3, and water (20 mL) was added. The mixture was extracted with ethyl acetate (40 mL × 2). The organic phases were combined, washed with saturated brine (50 mL), dried over anhydrous sodium sulfate, filtered, concentrated, and separated by silica gel column chromatography to give 1.31 g of a solid, with a yield of 61.9%. LC-MS (APCI): m/z = 243.3 $(M+1)^+$.

Step 4: Synthesis of compound 2-(4-bromophenyl)-4-hydroxy-6-((1r,4r)-4-(methoxymethyl)cyclohexyl)nicotinonitrile

Ethyl 3-((1r,4r)-4-(methoxymethyl)cyclohexyl)-3-oxopropanoate (1.3 g, 5.37 mmol), intermediate A-2 (1.0 g, 4.5 mmol), and N-methyl-2-pyrrolidone (10 mL) were added to a microwave vial. The vial was sealed. The mixture was stirred and reacted at 250 °C in a microwave reactor for 1 hour. The mixture was cooled to room temperature, and saturated brine (40 mL) was added. The mixture was extracted with ethyl acetate (30 mL × 3). The organic phases were combined, washed with saturated brine (80 mL), dried over anhydrous sodium sulfate, filtered, concentrated, and separated by silica gel column chromatography to give 1.5 g of a solid, with a yield of 83.3%. LC-MS (APCI): m/z = 401.0 $(M+1)^+$.

Step 5: Synthesis of compound 2-(4-bromophenyl)-4-chloro-6-((1r,4r)-4-(methoxymethyl)cyclohexyl)nicotinonitrile

2-(4-Bromophenyl)-4-hydroxy-6-((1r,4r)-4-(methoxymethyl)cyclohexyl)nicotinonitrile (1.5 g, 3.62 mmol) and phosphorus oxychloride (10 mL) were added to a 50 mL single-necked flask equipped with magnetic stirring. The mixture was heated to 90 °C, stirred and reacted for 1 hour. The mixture was concentrated and separated by silica gel column chromatography to give 1.18 g of a solid, with a yield of 75.6%. LC-MS (APCI): m/z = 419.0 $(M+1)^+$.

Step 6: Synthesis of compound 2-(4-bromophenyl)-4-chloro-6-((1r,4r)-4-(hydroxymethyl)cyclohexyl)nicotinonitrile

2-(4-Bromophenyl)-4-chloro-6-((1r,4r)-4-(methoxymethyl)cyclohexyl)nicotinonitrile (1.18 g, 2.82 mmol) and anhydrous dichloromethane (20 mL) were added to a 100 mL single-necked flask equipped with magnetic stirring. The mixture was cooled to 0 °C, and a solution of boron tribromide in dichloromethane (2.12 mL, 4.23 mmol, 2 M) was added dropwise. The ice bath was removed, and the mixture was stirred and reacted at room temperature under nitrogen for 2 hours. An aqueous solution of sodium bicarbonate (20 mL) was added to quench the reaction. The organic phase was separated, and the aqueous phase was extracted with dichloromethane (20 mL). The organic phases were combined, dried over anhydrous sodium sulfate, filtered, concentrated, and separated by silica gel column chromatography to give 900 mg of a solid, with a yield of 78.9%. LC-MS (APCI): m/z = 405.0 $(M+1)^+$.

Step 7 Synthesis of intermediate B-18

2-(4-Bromophenyl)-4-chloro-6-((1r,4r)-4-(hydroxymethyl)cyclohexyl)nicotinonitrile (0.90 g, 2.23 mmol), hydrazine hydrate (0.668 g, 13.38 mmol), and N,N-dimethylformamide (5 mL) were added to a 50 mL sealed vial equipped with magnetic stirring. The mixture was stirred until dissolved. The vial was sealed. The mixture was heated to 110 °C, stirred and reacted at the same temperature for 2.5 hours. The mixture was cooled to room temperature, and the solvent was removed by evaporation under reduced pressure. Saturated brine (30 mL) and ethyl acetate (20 mL) were added. The organic phase was separated, and the aqueous phase was extracted with ethyl acetate (20 mL × 2). The organic phases were combined, dried over anhydrous sodium sulfate, concentrated, and separated by silica gel column chromatography to give 0.36 g of an oily product, with a yield of 40.4%. LC-MS (APCI): m/z = 401.2 $(M+1)^+$.

**Preparation of intermediate B-19 compound tert-butyl 6-(3-amino-4-(4-((5-fluoro-2-methoxybenzoylamino) methyl)phenyl)-1H-pyrazolo[4,3-c]pyridin-7-yl)-3',6'-dihydro-[3,4'-bipyridine]-1'(2')-carboxylate**

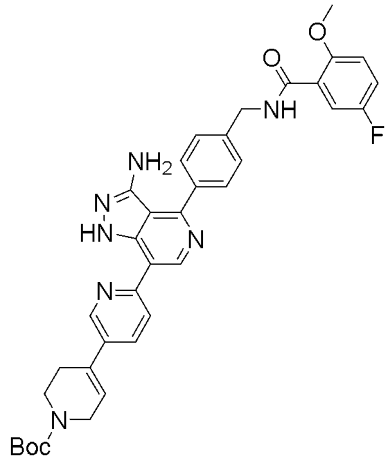

B-19

**The following synthetic route was adopted**

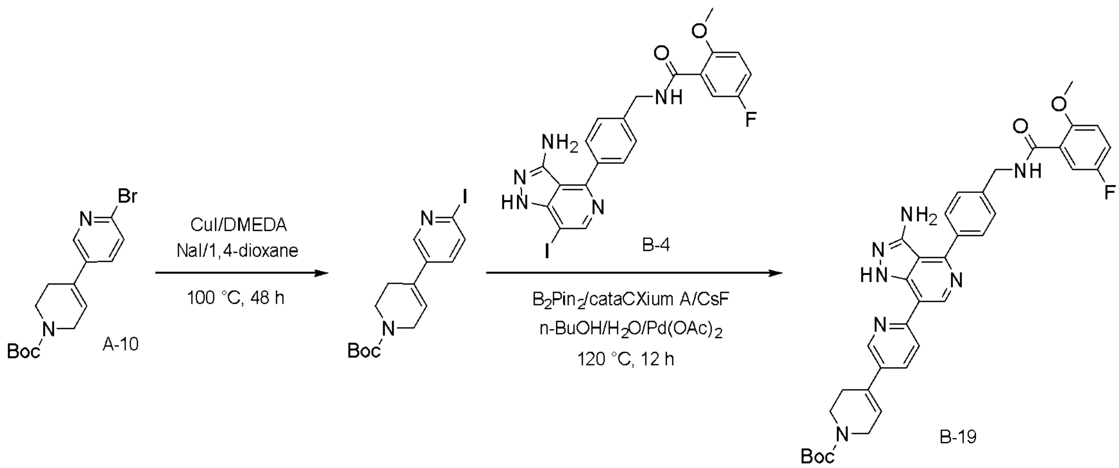


Step 1: Synthesis of compound tert-butyl 6-iodo-3',6'-dihydro-[3,4'-bipyridine]-1'(2'H)-carboxylate

Intermediate A-10 (15.1 g, 44.7 mmol), sodium iodide (13.2 g, 89.4 mmol), copper(I) iodide (2.55 g, 13.4 mmol), and 1,4-dioxane (100 mL) were successively added to a 500 mL single-necked flask equipped with magnetic stirring and a condenser. N,N'-dimethylethylenediamine (1.5 g, 17.9 mmol) was added with stirring. The reaction system was evacuated and purged with nitrogen three times. The mixture was heated to 100 °C under nitrogen, and reacted at the same temperature for 48 hours. The mixture was cooled to room temperature, and the insoluble solid was filtered out. The organic solvent was removed by evaporation under reduced pressure. Ethyl acetate (100 mL) and brine (80 mL, 10%) were added, and the organic phase was separated. The aqueous phase was extracted with ethyl acetate (50 mL × 2). The organic phases were combined, wash with saturated brine (50 mL), dried over anhydrous sodium sulfate, filtered, concentrated and separated by silica gel column chromatography to give 13.2 g of a yellow solid, with a yield of 76.7%. LC-MS (APCI): m/z = 387.0 $(M+1)^+$.

Step 2 Synthesis of intermediate B-19

tert-Butyl 6-iodo-3',6'-dihydro-[3,4'-bipyridine]-1'(2'H)-carboxylate (13.2 g, 34.1 mmol), intermediate B-4 (7.6 g, 14.8 mmol), bis(pinacolato)diboron (15.2 g, 59.2 mmol), n-butanol (100 mL), and water (20 mL) were successively added to a 500 mL single-necked flask equipped with magnetic stirring and a condenser. The mixture was stirred until dissolved. Then, cesium fluoride (8.8 g, 59.2 mmol), palladium(II) acetate (0.66 g, 2.9 mmol), and di(1-adamantyl)-n-butylphosphine

(2.0 g, 5.9 mmol) were added. The reaction system was evacuated and purged with nitrogen three times. The mixture was heated to 120 °C under nitrogen, stirred and reacted at the same temperature for 12 hours. The mixture was cooled to room temperature, and the organic solvent was removed by evaporation under reduced pressure. Ethyl acetate (100 mL) and brine (80 mL, 10%) were added, and the organic phase was separated. The mixture was extracted with ethyl acetate (50 mL × 2). The organic phases were combined, washed with saturated brine (50 mL), dried over anhydrous sodium sulfate, and filtered. The solvent was removed by evaporation under reduced pressure. The residue was separated by silica gel column chromatography to give 3.3 g of a yellow solid, with a yield of 14.8%. LC-MS (APCI): m/z = 650.2 $(M+1)^+$.

**Preparation of intermediate C-1 2-bromo-5-(oxetan-3-yl)pyridine**

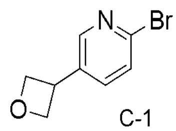

C-1

**The following synthetic route was adopted**

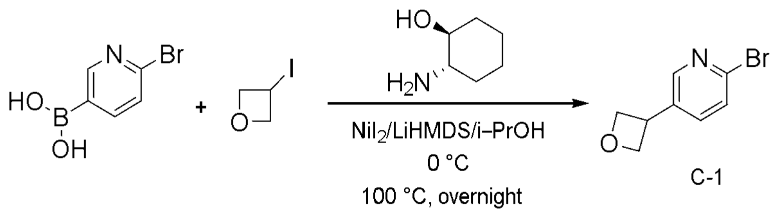


Compound 2-bromo-5-pyridineboronic acid (3.8 g, 18.8 mmol), 3-iodooxetane (1.75 g, 9.4 mmol), nickel(II) iodide (180 mg, 5.6 mmol), (1S,2S)-2-aminocyclohexanol (17 mg, 5.6 mmol), and anhydrous isopropanol (70 mL) were added to a 100 mL two-necked flask equipped with magnetic stirring. The reaction system was evacuated and purged with nitrogen three times. The mixture was cooled to 0 °C, and lithium bis(trimethylsilyl)amide (19 mL, 38 mmol, 2 M) was added dropwise. After the addition was completed, the mixture was heated to 100 °C and kept at the same temperature under nitrogen overnight. The mixture was cooled to room temperature, and water (60 mL) was added. The mixture was extracted with ethyl acetate (120 mL × 3). The organic phases were combined, washed with saturated brine (100 mL), dried over anhydrous sodium sulfate, filtered, concentrated and separated by silica gel column chromatography to give 310 mg of a yellowish-white solid, with a yield of 15.5%. LC-MS (APCI): m/z = 215 $(M+1)^+$. $^1$H NMR (400 MHz, DMSO-$D_6$) δ (ppm): 8.38 (d, J = 2.8 Hz, 1H), 7.89 (dd, J = 8.4 Hz, J = 2.8 Hz, 1H), 7.66 (d, J =8.8 Hz, 1H), 4.95-4.92 (m, 2H), 4.60 (t, J=6.8 Hz, 2H), 4.32-4.23 (m, 1H).

**Preparation of intermediate C-2 2-bromopyrazolo[1,5-a]pyrimidine**

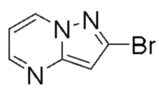

C-2

**The following synthetic route was adopted**

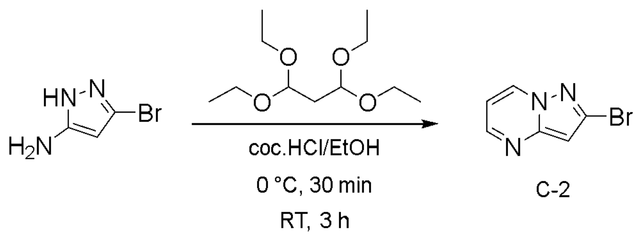

1,1,3,3-Tetraethoxypropane (1.4 g, 6.6 mmol) and ethanol (20 mL) were added to a 50 mL two-necked flask equipped with magnetic stirring. The mixture was stirred until dissolved, and cooled to 0 °C. The reaction system was evacuated and purged with nitrogen three times. Concentrated hydrochloric acid (1.1 mL, 12 M) was added, and the mixture was reacted at 0 °C for 30 minutes. 3-Bromo-5-aminopyrazole (1.0 g, 6.17 mmol) was added. The ice bath was removed, and the mixture was stirred and reacted at room temperature for 3 hours. Water (50 mL) and ethyl acetate (60 mL) were added, and the organic phase was separated. The aqueous phase was extracted with ethyl acetate (50 mL × 3). The organic phases were combined, washed with saturated brine (100 mL), dried over anhydrous sodium sulfate, filtered, concentrated, and separated by silica gel column chromatography to give 450 mg of a light yellow solid, with a yield of 37.2%. LC-MS (APCI): m/z = 198 $(M+1)^+$. $^1H$ NMR (400 MHz, DMSO-$D_6$) δ (ppm): 9.10 (d, J = 6.0 Hz, 1H), 8.59-8.58 (m, 1H), 7.10-7.07 (m, 1H), 6.92 (s, 1H).

**Preparation of intermediate C-3 ethyl 2-bromopyrazolo[1,5-a]pyrimidine-6-carboxylate**

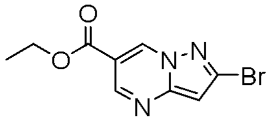

C-3

**The following synthetic route was adopted**

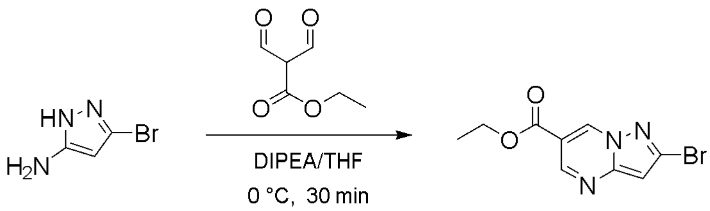


C-3

3-Bromo-5-aminopyrazole (1 g, 6.1 mmol) and tetrahydrofuran (20 mL) were added to a 50 mL two-necked flask equipped with magnetic stirring, and the mixture was stirred until dissolved. N,N-diisopropylethylamine (1.56 g, 12.2 mmol) and ethyl 2-formyl-3-oxopropanoate (1.78 g, 12.2 mmol) were added in an ice-water bath. The mixture was reacted at 0 °C for 30 min. The solvent was removed by evaporation under reduced pressure. Water (30 mL) and ethyl acetate (30 mL) were added. The mixture was stirred, and the organic phase was separated. The aqueous phase was extracted with ethyl acetate (20 mL × 2). The organic phases were combined, washed with saturated brine (50 mL), dried over anhydrous sodium sulfate, filtered, concentrated, and separated by silica gel column chromatography to give 1.2 g of a light yellow solid, with a yield of 72.2%. LC-MS (APCI): m/z = 271 $(M+1)^+$.

**Preparation of intermediate C-4 (6-bromopyridin-3-yl)(morpholino)methanone**

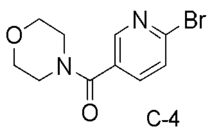

C-4

**The following synthetic route was adopted**

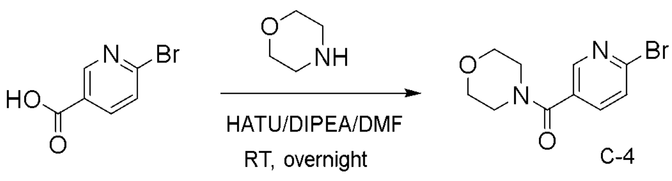


C-4

6-Bromonicotinic acid (500 mg, 2.475 mmol) and N,N-dimethylformamide (10 mL) were added to a 50 mL three-necked flask equipped with magnetic stirring, and the mixture was stirred until dissolved. Morpholine (258.7 mg, 2.97 mmol) and N,N-diisopropylethylamine (957.8 mg, 7.425 mmol) were added. The reaction system was evacuated and purged with nitrogen three times. The mixture was cooled in an ice-water bath, and O-(7-azabenzotriazol-1-yl)-N,N,N',N'-tetramethyluronium hexafluorophosphate (1.22 g, 3.217 mmol) was added. The mixture was reacted at room temperature overnight, and water (30 mL) was added to quench the reaction. The mixture was extracted with ethyl acetate (30 mL × 3). The organic phases were combined, washed with saturated brine (50 mL), dried over anhydrous sodium sulfate, filtered, concentrated, and separated by silica gel column chromatography to give 568.0 mg of a white solid, with a yield of 85.0%. LC-MS (APCI): m/z = 271.0 (M+1)+.

**Preparation of intermediate C-5 (6-bromopyridin-3-yl)((2S,6S)-2,6-dimethylmorpholino)methanone**

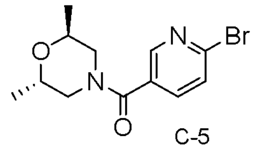

C-5

**The following synthetic route was adopted**

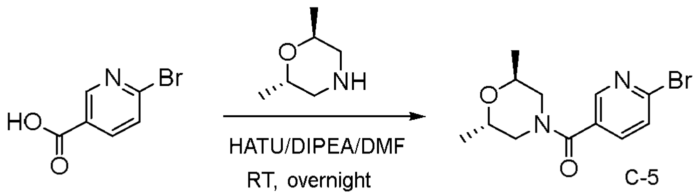


6-Bromonicotinic acid (500 mg, 2.475 mmol) and N,N-dimethylformamide (10 mL) were added to a 50 mL three-necked flask equipped with magnetic stirring, and the mixture was stirred until dissolved. (2S,6S)-2,6-Dimethylmorpholine (342.1 mg, 2.97 mmol) and N,N-diisopropylethylamine (957.8 mg, 7.425 mmol) were added. The reaction system was evacuated and purged with nitrogen three times. The mixture was cooled in an ice-water bath, and O-(7-azabenzotriazol-1-yl)-N,N,N',N'-tetramethyluronium hexafluorophosphate (1.22 g, 3.217 mmol) was added. The mixture was reacted at room temperature overnight, and water (30 mL) was added to quench the reaction. The mixture was extracted with ethyl acetate (30 mL × 3). The organic phases were combined, washed with saturated brine (50 mL), dried over anhydrous sodium sulfate, filtered, concentrated, and separated by silica gel column chromatography to give 590.1 mg of a white solid, with a yield of 80.0%. LC-MS (APCI): m/z = 299.0 (M+1)+.

**Preparation of intermediate C-6 (6-bromopyridin-3-yl)((2R,6R)-2,6-dimethylmorpholino)methanone**

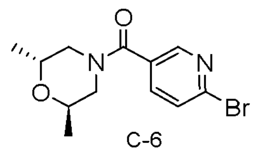

C-6

**The following synthetic route was adopted**

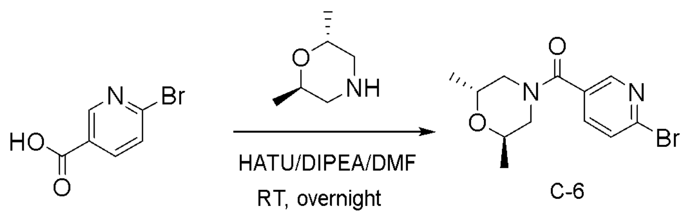

6-Bromonicotinic acid (500 mg, 2.475 mmol) and N,N-dimethylformamide (10 mL) were added to a 50 mL three-necked flask equipped with magnetic stirring, and the mixture was stirred until dissolved. (2R,6R)-2,6-Dimethylmorpholine (342.1 mg, 2.97 mmol) and N,N-diisopropylethylamine (957.8 mg, 7.425 mmol) were added. The reaction system was evacuated and purged with nitrogen three times. The mixture was cooled in an ice-water bath, and O-(7-azabenzotriazol-1-yl)-N,N,N',N'-tetramethyluronium hexafluorophosphate (1.22 g, 3.217 mmol) was added. The mixture was reacted at room temperature overnight, and water (30 mL) was added to quench the reaction. The mixture was extracted with ethyl acetate (30 mL × 3). The organic phases were combined, washed with saturated brine (50 mL), dried over anhydrous sodium sulfate, filtered, concentrated, and separated by silica gel column chromatography to give 582.1 mg of a white solid, with a yield of 78.2%. LC-MS (APCI): m/z = 299.0 $(M+1)^+$.

**Preparation of intermediate C-7 2-bromo-[1,2,4]triazolo[1,5-a]pyridine**

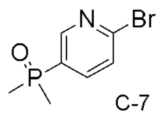

C-7

**The following synthetic route was adopted**

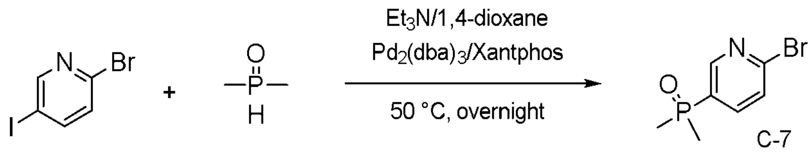


2-Bromo-5-iodopyridine (500 mg, 1.76 mmol), dimethylphosphine oxide (206 mg, 2.64 mmol), tris(dibenzylideneacetone)dipalladium(0) (322.3 mg, 0.35 mmol), 4,5-bis(diphenylphosphino)-9,9-dimethylxanthene (203 mg, 0.35 mmol), triethylamine (0.73 mL, 0.58 mmol) and 1,4-dioxane (12 mL) were added to a 50 mL single-necked flask equipped with magnetic stirring. The reaction system was evacuated and purged with nitrogen three times. The mixture was stirred and reacted at 50 °C overnight. The mixture was cooled to room temperature, and saturated brine (20 mL) was added. The organic phase was separated, and the aqueous phase was extracted with ethyl acetate (20 mL × 2). The organic phases were combined, washed with saturated brine (20 mL), dried over anhydrous sodium sulfate, filtered, concentrated, and separated by silica gel column chromatography to give 350 mg of a white solid, with a yield of 85.4%. LC-MS (APCI): m/z = 234.0 $(M+1)^+$.

**Preparation of intermediate C-8 2-bromo-[1,2,4]triazolo[1,5-a]pyrazine**

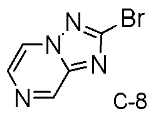

C-8

**The following synthetic route was adopted**

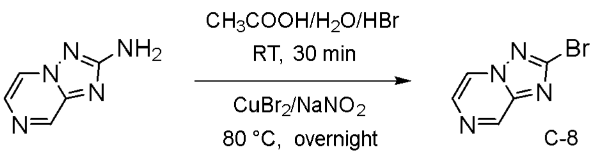


[1,2,4]Triazolo[1,5-a]pyrazin-2-amine (1.0 g, 7.4 mmol), glacial acetic acid (8 mL), water (8 mL), and hydrobromic acid (6 mL) were added to a 50 mL three-necked flask equipped with magnetic stirring and a condenser. The mixture was stirred and reacted at room temperature for 30 minutes. Copper(II) bromide (530.7 mg, 3.7 mmol) was added, and saturated aqueous solution of sodium nitrate (1.5 mL) was slowly added dropwise. The reaction system was evacuated and purged with nitrogen three times. The mixture was stirred and reacted at 80 °C overnight. The mixture was cooled to

room temperature, and concentrated under reduced pressure. Saturated aqueous solution of sodium bicarbonate (30 mL) was added to quench the reaction. The organic phase was separated, and the aqueous phase was extracted with ethyl acetate (30 mL × 2). The organic phases were combined, washed with saturated brine (30 mL), dried over anhydrous sodium sulfate, filtered, concentrated, and separated by silica gel column chromatography to give 840 mg of a white solid, with a yield of 57.3%. LC-MS (APCI): m/z = 199.0 $(M+1)^+$.

**Preparation of intermediate C-9 2-bromo-[1,2,4]triazolo[1,5-a]pyridine**

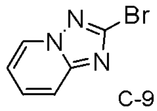

C-9

**The following synthetic route was adopted**

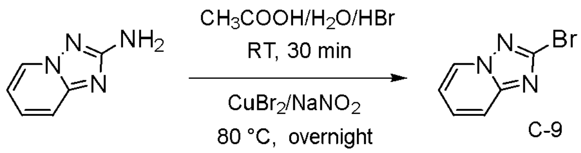


[1,2,4]Triazolo[1,5-a]pyridin-2-amine (500 mg, 3.73 mmol), glacial acetic acid (4 mL), water (4 mL), and hydrobromic acid (4 mL) were added to a 50 mL three-necked flask equipped with magnetic stirring and a condenser. The mixture was stirred and reacted at room temperature for 30 minutes. Copper(II) bromide (267.5 mg, 1.87 mmol) was added, and saturated aqueous solution of sodium nitrate (0.8 mL) was slowly added dropwise. The reaction system was evacuated and purged with nitrogen three times. The mixture was stirred and reacted at 80 °C overnight. The mixture was cooled to room temperature, and concentrated under reduced pressure. Saturated aqueous solution of sodium bicarbonate (20 mL) was added to quench the reaction. The organic phase was separated, and the aqueous phase was extracted with ethyl acetate (20 mL × 2). The organic phases were combined, washed with saturated brine (30 mL), dried over anhydrous sodium sulfate, filtered, concentrated, and separated by silica gel column chromatography to give 564 mg of a white solid, with a yield of 76.8%. LC-MS (APCI): m/z = 198.0 $(M+1)^+$.

**Preparation of intermediate C-10 2-bromo-5-(2-methoxyethoxy)pyridine**

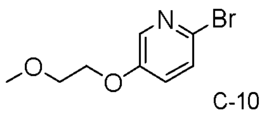

C-10

**The following synthetic route was adopted**

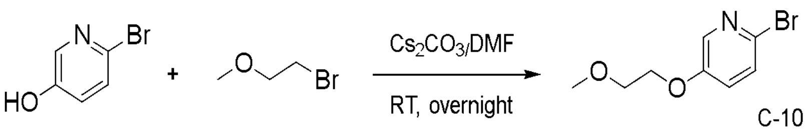


5-Hydroxy-2-bromopyridine (2.0 g, 11.56 mmol), 1-bromo-2-methoxyethane (2.41 g, 17.34 mmol), cesium carbonate (11.3 g, 34.68 mmol), and N,N-dimethylformamide (20 mL) were added to a 50 mL single-necked flask equipped with magnetic stirring. The mixture was stirred and reacted at room temperature overnight. Saturated brine (20 mL) was added, and the organic phase was separated. The aqueous phase was extracted with ethyl acetate (30 mL × 2). The organic phases were combined, washed with saturated brine (40 mL × 3), dried over anhydrous sodium sulfate, filtered, concentrated, and separated by silica gel column chromatography to give 2.4 g of a white solid, with a yield of 89.9%. LC-MS (APCI): m/z = 232.0 $(M+1)^+$.

**Preparation of intermediate C-11 2-((6-bromopyridin-3-yl)oxy)-N,N-dimethylethan-1-amine**

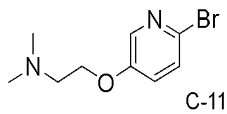

C-11

**The following synthetic route was adopted**

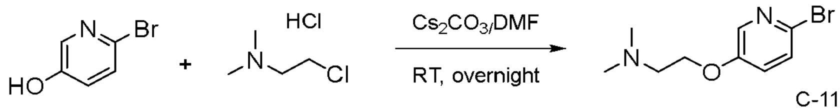


C-11

5-Hydroxy-2-bromopyridine (2.0 g, 11.56 mmol), 2-chloro-N,N-dimethylethylamine hydrochloride (2.5 g, 17.34 mmol), cesium carbonate (11.3 g, 34.68 mmol), and N,N-dimethylformamide (20 mL) were added to a 50 mL single-necked flask equipped with magnetic stirring. The mixture was stirred and reacted at room temperature overnight. Saturated brine (20 mL) was added, and the organic phase was separated. The aqueous phase was extracted with ethyl acetate (20 mL × 2). The organic phases were combined, washed with saturated brine, dried over anhydrous sodium sulfate, filtered, concentrated, and separated by silica gel column chromatography to give 734 mg of a white solid, with a yield of 26.0%. LC-MS (APCI): m/z = 245.0 $(M+1)^+$.

**Preparation of intermediate C-12 4-(2-((6-bromopyridin-3-yl)oxy)ethyl)morpholine**

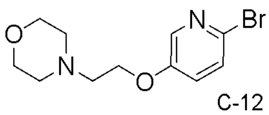

C-12

**The following synthetic route was adopted**

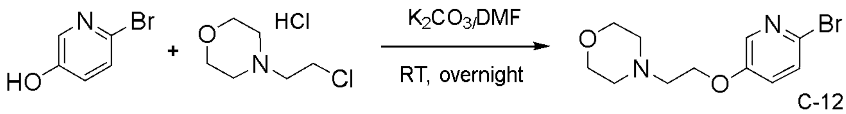


C-12

5-Hydroxy-2-bromopyridine (2.0 g, 11.56 mmol), N-(2-chloroethyl)morpholine hydrochloride (3.23 g, 17.34 mmol), potassium carbonate (6.39 g, 34.68 mmol), and N,N-dimethylformamide (20 mL) were added to a 50 mL single-necked flask equipped with magnetic stirring. The mixture was stirred and reacted at room temperature overnight. Saturated brine (20 mL) was added, and the organic phase was separated. The aqueous phase was extracted with ethyl acetate (20 mL × 2). The organic phases were combined, washed with saturated brine, dried over anhydrous sodium sulfate, filtered, concentrated, and separated by silica gel column chromatography to give 2.4 g of a white solid, with a yield of 72.6%. LC-MS (APCI): m/z = 287.0 $(M+1)^+$.

**Preparation of intermediate C-13 2-bromo-5-(2-(pyrrolidin-1-yl)ethoxy)pyridine**

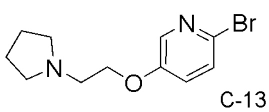

C-13

**The following synthetic route was adopted**

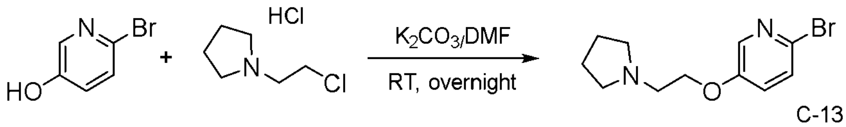


C-13

5-Hydroxy-2-bromopyridine (2.0 g, 11.56 mmol), N-(2-chloroethyl)pyrrolidine hydrochloride (2.95 g, 17.34 mmol), potassium carbonate (6.39 g, 34.68 mmol), and N,N-dimethylformamide (20 mL) were added to a 50 mL single-necked flask equipped with magnetic stirring. The mixture was stirred and reacted at room temperature overnight. Saturated brine (20 mL) was added, and the organic phase was separated. The aqueous phase was extracted with ethyl acetate (20 mL × 2). The organic phases were combined, washed with saturated brine, dried over anhydrous sodium sulfate, filtered, concentrated, and separated by silica gel column chromatography to give 1.26 g of a white solid, with a yield of 40.4%. LC-MS (APCI): m/z = 271.0 $(M+1)^+$.

**Preparation of intermediate C-14 2-bromo-5-methyl-4,5,6,7-tetrahydropyrazolo[1,5-a]pyrazine**

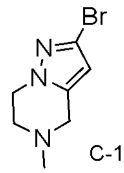

C-14

**The following synthetic route was adopted**

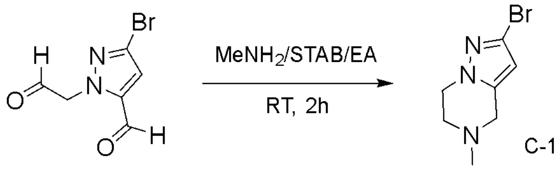


C-14

3-Bromo-1-(oxoethyl)-1H-pyrazole-5-carbaldehyde (370 mg, 1.7 mmol) and ethyl acetate (10 mL) were added to a container, and the mixture was cooled to 0 °C in an ice bath. A solution of methylamine in ethanol (0.94 mL, 1.87 mmol, 2 M) was slowly added dropwise, and then sodium triacetoxyborohydride (1.08 g, 5.1 mmol) was added. The mixture was stirred and reacted at room temperature for 2 hours, and diluted with added water (10 mL). The mixture was extracted with ethyl acetate (20 mL × 3). The organic phase was washed with saturated brine, dried over anhydrous sodium sulfate, filtered, concentrated, and separated by silica gel column chromatography to give 150 mg of a light yellow oil, with a yield of 40.8%. LC-MS (APCI): m/z = 218.0 $(M+1)^+$.

**Preparation of intermediate C-15 2-bromo-5-ethyl-4,5,6,7-tetrahydropyrazolo[1,5-a]pyrazine**

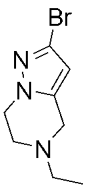

C-15

**The following synthetic route was adopted**

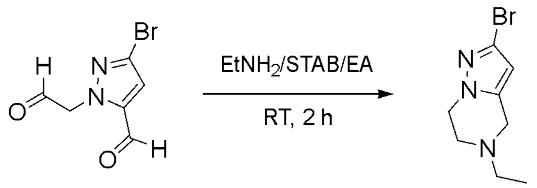

C-15

3-Bromo-1-(oxoethyl)-1H-pyrazole-5-carbaldehyde (370 mg, 1.7 mmol) and ethyl acetate (10 mL) were added to a container, and the mixture was cooled to 0 °C in an ice bath. A solution of ethylamine in ethanol (0.94 mL, 1.87 mmol, 2 M) was slowly added dropwise, and then sodium triacetoxyborohydride (1.08 g, 5.1 mmol) was added. The mixture was stirred and reacted at room temperature for 2 hours, and diluted with added water (10 mL). The mixture was extracted with ethyl acetate (20 mL × 3). The organic phase was washed with saturated brine, dried over anhydrous sodium sulfate, filtered, concentrated, and separated by silica gel column chromatography to give 180 mg of a light yellow oil, with a yield of 46.1%. LC-MS (APCI): m/z = 232.0 $(M+1)^+$.

**Preparation of intermediate C-16 1-(6-bromopyridin-3-yl)-4-methylpiperazine**

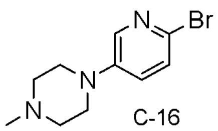
C-16

**The following synthetic route was adopted**

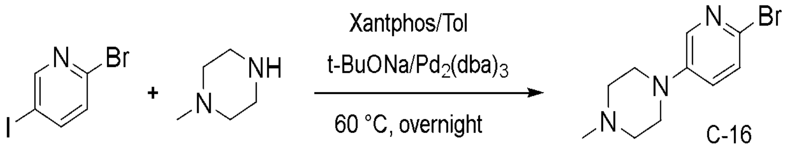

C-16

Compound 1-methylpiperazine (1 g, 10 mmol), 2-bromo-5-iodopyridine (3.41 g, 12 mmol), sodium tert-butoxide (2.88 g, 30 mmol), tris(dibenzylideneacetone)dipalladium(0) (230 mg, 0.25 mmol), 4,5-bis(diphenylphosphino)-9,9-dimethylxanthene (290 mg, 0.5 mmol), and toluene (60 mL) were added to a 50 mL single-necked flask equipped with magnetic stirring and a condenser. The mixture was stirred and reacted at 60 °C overnight under nitrogen. The mixture was cooled to room temperature, and the reaction mixture was poured into water (50 mL). The mixture was extracted with ethyl acetate (50 mL × 3). The organic phases were combined, washed with saturated brine (50 mL), dried over anhydrous sodium sulfate, filtered, concentrated, and separated by silica gel column chromatography to give 0.55 g of a brownish-yellow solid, with a yield of 21.5%. LC-MS (APCI): m/z = 256.0 $(M+1)^+$.

**Preparation of intermediate C-17 (S)-1-(6-bromopyridin-3-yl)-2,4-dimethylpiperazine**

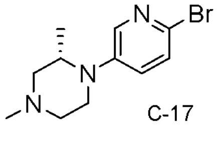
C-17

**The following synthetic route was adopted**

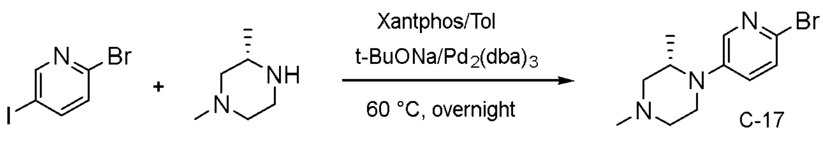

C-17

Compound (S)-1,3-dimethylpiperazine (1.14 g, 10 mmol), 2-bromo-5-iodopyridine (3.41 g, 12 mmol), sodium

tert-butoxide (2.88 g, 30 mmol), tris(dibenzylideneacetone)dipalladium(0) (230 mg, 0.25 mmol), 4,5-bis(diphenylphosphino)-9,9-dimethylxanthene (290 mg, 0.5 mmol), and toluene (60 mL) were added to a 50 mL single-necked flask equipped with magnetic stirring and a condenser. The mixture was stirred and reacted at 60 °C overnight under nitrogen. The mixture was cooled to room temperature, and the reaction mixture was poured into water (50 mL). The mixture was extracted with ethyl acetate (50 mL × 3). The organic phases were combined, washed with saturated brine (50 mL), dried over anhydrous sodium sulfate, filtered, concentrated, and separated by silica gel column chromatography to give 0.59 g of a brownish-yellow solid, with a yield of 21.8%. LC-MS (APCI): m/z = 270.0 $(M+1)^+$.

**Preparation of intermediate C-18 (R)-1-(6-bromopyridin-3-yl)-2,4-dimethylpiperazine**

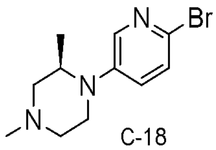

C-18

**The following synthetic route was adopted**

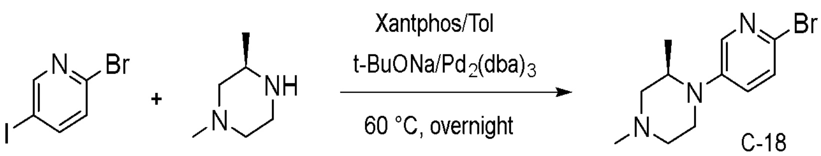


C-18

Compound (R)-1,3-dimethylpiperazine (1.14 g, 10 mmol), 2-bromo-5-iodopyridine (3.41 g, 12 mmol), sodium tert-butoxide (2.88 g, 30 mmol), tris(dibenzylideneacetone)dipalladium(0) (230 mg, 0.25 mmol), 4,5-bis(diphenylphosphino)-9,9-dimethylxanthene (290 mg, 0.5 mmol), and toluene (60 mL) were added to a 50 mL single-necked flask equipped with magnetic stirring and a condenser. The mixture was stirred and reacted at 60 °C overnight under nitrogen. The mixture was cooled to room temperature, and the reaction mixture was poured into water (50 mL). The mixture was extracted with ethyl acetate (50 mL × 3). The organic phases were combined, washed with saturated brine (50 mL), dried over anhydrous sodium sulfate, filtered, concentrated, and separated by silica gel column chromatography to give 0.53 g of a brownish-yellow solid, with a yield of 21.5%. LC-MS (APCI): m/z = 270.0 $(M+1)^+$.

**Preparation of intermediate C-19 4-(6-bromopyridin-3-yl)morpholine**

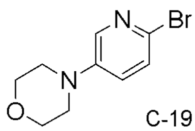

C-19

**The following synthetic route was adopted**

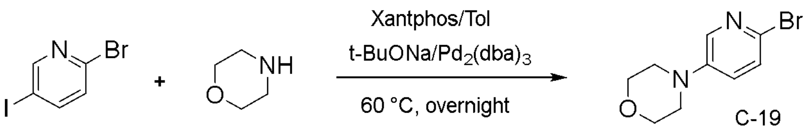


C-19

Compound morpholine (0.86 g, 10 mmol), 2-bromo-5-iodopyridine (3.41 g, 12 mmol), sodium tert-butoxide (2.88 g, 30 mmol), tris(dibenzylideneacetone)dipalladium(0) (230 mg, 0.25 mmol), 4,5-bis(diphenylphosphino)-9,9-dimethylxanthene (290 mg, 0.5 mmol), and toluene (60 mL) were added to a 50 mL single-necked flask equipped with magnetic stirring and a condenser. The mixture was stirred and reacted at 60 °C overnight under nitrogen. The mixture was cooled to room temperature, and the reaction mixture was poured into water (50 mL). The mixture was extracted with ethyl acetate (50 mL × 3). The organic phases were combined, washed with saturated brine (50 mL), dried over anhydrous sodium sulfate, filtered, concentrated, and separated by silica gel column chromatography to give 0.49 g of a brownish-yellow

solid, with a yield of 21.0%. LC-MS (APCI): m/z = 243.0 (M+1)+.

**Preparation of intermediate C-20 1-bromopyrrolo[1,2-a]pyrazine**

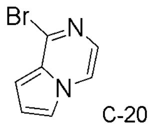

C-20

**The following synthetic route was adopted**

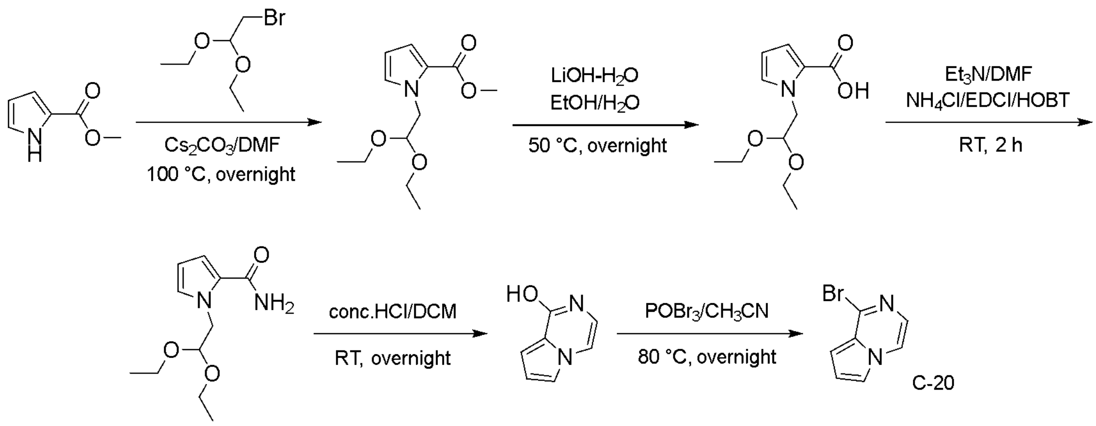


C-20

Step 1: Synthesis of compound methyl 1-(2,2-diethoxyethyl)-1H-pyrrole-2-carboxylate

Methyl 1H-pyrrole-2-carboxylate (3.0 g, 24.0 mmol) and N,N-dimethylformamide (35 mL) were added to a 100 mL single-necked flask equipped with magnetic stirring and a condenser. The mixture was stirred until dissolved. 2-Bromo-1,1-diethoxyethane (5.68 g, 28.8 mmol) and cesium carbonate (11.7 g, 30.0 mmol) were added. The reaction system was evacuated and purged with nitrogen three times. The mixture was stirred and reacted at 100 °C overnight. The mixture was cooled to room temperature, and saturated brine (50 mL) was added to quench the reaction. The organic phase was separated, and the aqueous phase was extracted with ethyl acetate (50 mL × 2). The organic phases were combined, washed with saturated brine (50 mL × 2), dried over anhydrous sodium sulfate, filtered, concentrated, and separated by silica gel column chromatography to give 2.0 g of a white solid, with a yield of 39.2%. LC-MS (APCI): m/z = 214.0 (M+1)+.

Step 2: Synthesis of compound 1-(2,2-diethoxyethyl)-1H-pyrrole-2-carboxylic acid

Methyl 1-(2,2-diethoxyethyl)-1H-pyrrole-2-carboxylate (2.0 g, 9.4 mmol), ethanol (15 mL), and water (15 mL) were added to a 100 mL single-necked flask equipped with magnetic stirring and a condenser. The mixture was stirred until dissolved, and lithium hydroxide monohydrate (1.97 g, 47 mmol) was added. The reaction system was evacuated and purged with nitrogen three times. The mixture was stirred and reacted at 50 °C overnight. The mixture was cooled to 0 °C, and hydrochloric acid (10 mL, 1 N) was added to adjust the pH to 4. The organic phase was separated, and the aqueous phase was extracted with ethyl acetate (20 mL × 2). The organic phases were combined, washed with saturated brine (20 mL), dried over anhydrous sodium sulfate, filtered, concentrated, and separated by silica gel column chromatography to give 1.35 g of a white solid, with a yield of 63.2%. LC-MS (APCI): m/z = 228.0 (M+1)+.

Step 3: Synthesis of compound 1-(2,2-diethoxyethyl)-1H-pyrrole-2-carboxamide

1-(2,2-Diethoxyethyl)-1H-pyrrole-2-carboxylic acid (350 mg, 1.54 mmol), ammonium chloride (411.9 mg, 7.7 mmol), and N,N-dimethylformamide (6 mL) were added to a 50 mL single-necked flask equipped with magnetic stirring. The mixture was stirred until dissolved. 1-Ethyl-3-(3-dimethylaminopropyl)carbodiimide (384.4 mg, 2 mmol) and 1-hydroxybenzotriazole (270.3 mg, 2 mmol) were added. The reaction system was evacuated and purged with nitrogen three times. The mixture was cooled to 0 °C, and triethylamine (641.3 mg, 4.62 mmol) was added. The mixture was reacted

at room temperature for 2 hours. Saturated brine (10 mL) was added to quench the reaction, and the organic phase was separated. The aqueous phase was extracted with ethyl acetate (10 mL × 2). The organic phases were combined, washed with saturated brine (10 mL), dried over anhydrous sodium sulfate, filtered, concentrated and separated by silica gel column chromatography to give 300 mg of a white solid, with a yield of 86.1%. LC-MS (APCI): m/z = 227.0 $(M+1)^+$.

Step 4: Synthesis of compound pyrrolo[1,2-a]pyrazin-1-ol

1-(2,2-Diethoxyethyl)-1H-pyrrole-2-carboxamide (300 mg, 1.32 mmol) and dichloromethane (6 mL) were successively added to a 50 mL single-necked flask equipped with magnetic stirring. The mixture was stirred to disperse, and 2 drops of concentrated hydrochloric acid were added dropwise. The mixture was stirred and reacted at room temperature overnight. The mixture was filtered. The filter residue was washed with ethyl acetate and dried to give 124 mg of a white solid, with a yield of 70.2%. LC-MS (APCI): m/z = 135.0 $(M+1)^+$.

Step 5: Synthesis of intermediate C-20

Pyrrolo[1,2-a]pyrazin-1-ol (124 mg, 0.93 mmol) and acetonitrile (6 mL) were added to a 50 mL single-necked flask equipped with magnetic stirring and a condenser. The mixture was stirred to disperse, and phosphorus oxybromide (1.07 g, 3.72 mmol) was added. The mixture was stirred and reacted at 80 °C overnight. The mixture was cooled to 0 °C, and saturated sodium carbonate was added to adjust the pH to 10. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 113 mg of a white solid, with a yield of 62.0%. LC-MS (APCI): m/z = 197.0 $(M+1)^+$.

**Preparation of intermediate C-21 pyrazolo[1,5-a]pyrazine-3-boronic acid pinacol ester**

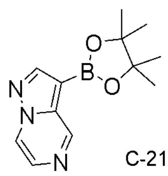

C-21

**The following synthetic route was adopted**

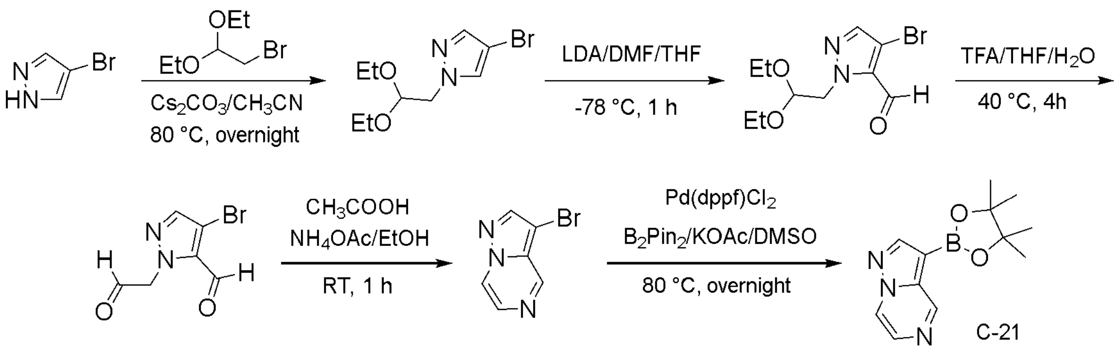


Step 1: Synthesis of compound 4-bromo-1-(2,2-ethoxyethyl)-1H-pyrazole

4-Bromopyrazole (1.0 g, 6.8 mmol), acetonitrile (20 mL), bromoacetaldehyde diethyl acetal (1.41 g, 7.14 mmol), and cesium carbonate (3.48 g, 10.7 mmol) were added to a container. The mixture was reacted at 80 °C under nitrogen overnight. The mixture was cooled to room temperature, and filtered. The filter cake was washed twice with ethyl acetate. The mixture was filtered, concentrated, and separated by silica gel column chromatography to give 1.7 g of a colorless, transparent oil, with a yield of 95%. LC-MS (APCI): m/z = 263.0 $(M+1)^+$.

Step 2: Synthesis of compound 4-bromo-1-(2,2-ethoxyethyl)-1H-pyrazole-5-carbaldehyde

4-Bromo-1-(2,2-ethoxyethyl)-1H-pyrazole (1.7 g, 6.48 mmol) and anhydrous tetrahydrofuran (30 mL) were added to a container, and the mixture was cooled to -78 °C. Lithium diisopropylamide (5 mL, 10 mmol, 2 M) was slowly added dropwise, and the mixture was reacted at -78 °C for 1 hour. N,N-dimethylformamide (850 mg, 11.6 mmol) was dissolved in anhydrous tetrahydrofuran (10 mL), and this solution was slowly added dropwise to the above reaction solution at -78 °C. Then, the resulting mixture was reacted at -78 °C for 1.5 hours. Saturated ammonium chloride solution was added to quench the reaction, and the mixture was diluted with added water at room temperature. The mixture was extracted with methyl tert-butyl ether (100 mL × 3). The organic phase was washed with saturated brine, dried over anhydrous sodium sulfate, filtered, concentrated, and separated by silica gel column chromatography to give 1.57 g of a light yellow oil, with a yield of 83%. LC-MS (APCI): m/z = 291.0 $(M+1)^+$.

Step 3: Synthesis of compound 4-bromo-1-(oxoethyl)-1H-pyrazole-5-carbaldehyde

4-Bromo-1-(2,2-ethoxyethyl)-1H-pyrazole-5-carbaldehyde (1.57 g, 5.4 mmol), trifluoroacetic acid (6 mL), tetrahydrofuran (3 mL), and water (3 mL) were added to a container. The solution was heated to 40 °C and reacted for 4 hours. The mixture was cooled to room temperature. The reaction solution was concentrated and azeotropically refluxed with toluene (3 × 10 mL) three times to remove water to give 4-bromo-1-(oxoethyl)-1H-pyrazole-5-carbaldehyde crude product, which was directly used in the next step reaction.

Step 4: Synthesis of compound 3-bromopyrazolo[1,5-a]pyrazine

The 4-bromo-1-(oxoethyl)-1H-pyrazole-5-carbaldehyde crude product from step 3, ethanol (15 mL), ammonium acetate (1.25 g, 16.2 mmol), and acetic acid (972 mg, 16.2 mmol) were added to a container. The mixture was reacted at room temperature for 1 hour. The reaction solution was concentrated, and diluted with added water (50 mL). Saturated sodium carbonate solution was added to adjust the pH to 8. The mixture was extracted with ethyl acetate (100 mL × 3). The organic phase was washed with saturated brine (200 mL), dried over anhydrous sodium sulfate, filtered, concentrated, and separated by silica gel column chromatography to give 770 mg of a colorless, transparent oil, with a two-step yield of 72%. ESI-MS: 198 $[M^+]$. $^1H$ NMR (400 MHz, DMSO-*D*6) δ (ppm): 9.10 (s, 1H), 8.81 (d, J = 5.2 Hz, 1H), 8.33 (s, 1H), 7.97 (d, J = 5.2 Hz, 1H).

Step 5: Synthesis of intermediate C-21

3-Bromopyrazolo[1,5-a]pyrazine (100 mg, 0.5 mmol), dimethyl sulfoxide (5 mL), bis(pinacolato)diboron (254 mg, 1 mmol), [1,1'-bis(diphenylphosphino)ferrocene]dichloropalladium(II) (41 mg, 0.05 mmol), and potassium acetate (150 mg, 1.5 mmol) were added to a container. The mixture was heated to 80 °C and reacted overnight. The reaction solution was cooled, and then diluted with added water (20 mL). The mixture was extracted with ethyl acetate (50 mL × 3). The organic phase was washed with saturated brine (100 mL), dried over anhydrous sodium sulfate, concentrated, and separated by silica gel column chromatography to give 70 mg of a light yellow oil, with a yield of 56%. LC-MS (APCI): m/z = 246.0 $(M+1)^+$.

**Preparation of intermediate C-22 N-(6-bromopyridin-3-yl)cyclopropanecarboxamide**

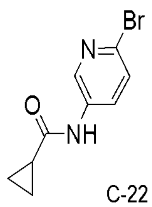

C-22

**The following synthetic route was adopted**

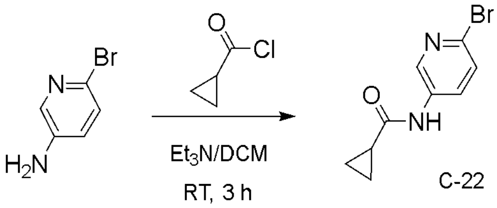

C-22

2-Bromo-5-aminopyridine (500 mg, 2.924 mmol) and dichloromethane (10 mL) were added to a 50 mL three-necked flask equipped with magnetic stirring. The mixture was stirred until dissolved, and triethylamine (894.7 mg, 8.772 mmol) was added. Cyclopropanecarbonyl chloride (336.2 mg, 3.216 mmol) was added dropwise in an ice-water bath. After the addition was completed, the ice bath was removed. The mixture was reacted at room temperature under nitrogen for 3 hours. Water (30 mL) was added to quench the reaction, and the mixture was diluted with dichloromethane (20 mL). The organic phase was separated, and the aqueous phase was extracted with dichloromethane (30 mL × 2). The organic phases were combined, dried over anhydrous sodium sulfate, filtered, concentrated, and separated by silica gel column chromatography to give 561.4 mg of a white solid, with a yield of 80.0%. LC-MS (APCI): m/z = 241.0 $(M+1)^+$.

<u>**Preparation of intermediate C-23 N-(2-bromopyridin-4-yl)cyclopropanecarboxamide**</u>

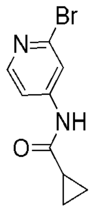
C-23

<u>**The following synthetic route was adopted**</u>

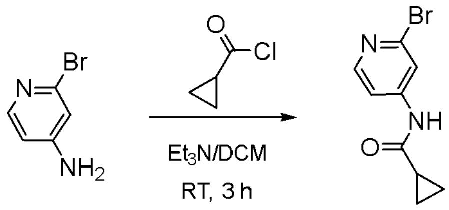

C-23

2-Bromo-4-aminopyridine (500 mg, 2.924 mmol) and dichloromethane (10 mL) were added to a 50 mL three-necked flask equipped with magnetic stirring. The mixture was stirred until dissolved, and triethylamine (894.7 mg, 8.772 mmol) was added. Cyclopropanecarbonyl chloride (336.2 mg, 3.216 mmol) was added dropwise in an ice-water bath. After the addition was completed, the ice bath was removed. The mixture was reacted at room temperature under nitrogen for 3 hours. Water (30 mL) was added to quench the reaction, and the mixture was diluted with dichloromethane (20 mL). The organic phase was separated, and the aqueous phase was extracted with dichloromethane (30 mL × 2). The organic phases were combined, dried over anhydrous sodium sulfate, filtered, concentrated, and separated by silica gel column chromatography to give 550 mg of a white solid, with a yield of 78.4%. LC-MS (APCI): m/z = 241.0 $(M+1)^+$.

<u>**Preparation of intermediate C-24 4-bromopyrazolo[1,5-a]pyrazine**</u>

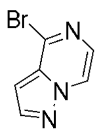
C-24

**The following synthetic route was adopted**

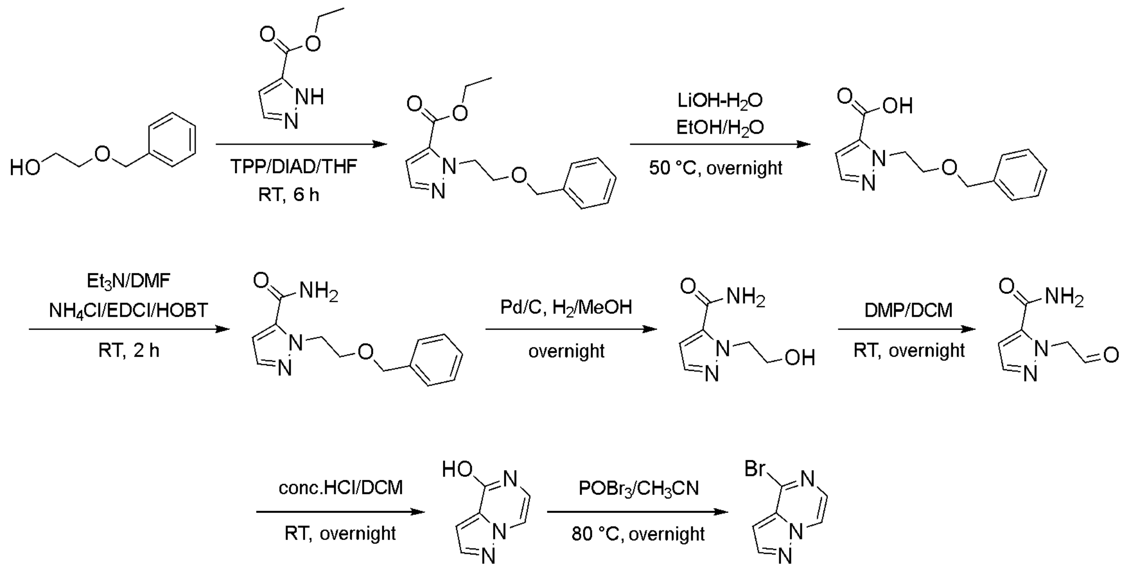

C-24

Step 1: Synthesis of compound ethyl 1-(2-(benzyloxy)ethyl)-1H-pyrazole-5-carboxylate

2-(Benzyloxy)ethanol (3.0 g, 19.1 mmol), ethyl 1H-pyrazole-5-carboxylate (3.04 g, 21.7 mmol), triphenylphosphine (10.33 g, 39.4 mmol), and anhydrous tetrahydrofuran (40 mL) were added to a 100 mL three-necked flask equipped with magnetic stirring. The reaction system was evacuated and purged with nitrogen three times. Diisopropyl azodicarboxylate (7.97 g, 39.4 mmol) was added dropwise at 0 °C with stirring. The mixture was reacted at room temperature for 6 hours. Saturated brine (40 mL) was added to quench the reaction. The organic phase was separated, and the aqueous phase was extracted with ethyl acetate (40 mL × 2). The organic phases were combined, washed with saturated brine (40 mL), dried over anhydrous sodium sulfate, filtered, concentrated, and separated by silica gel column chromatography to give 19.3 g of a white solid, with a yield of 97.9%. LC-MS (APCI): m/z = 275.0$(M+1)^+$.

Step 2: Synthesis of compound 1-(2-(benzyloxy)ethyl)-1H-pyrazole-5-carboxylic acid

Ethyl 1-(2-(benzyloxy)ethyl)-1H-pyrazole-5-carboxylate (5.3 g, 19.3 mmol), lithium hydroxide monohydrate (4.05 g, 96.5 mmol), ethanol (30 mL), and water (30 mL) were added to a 100 mL single-necked flask equipped with magnetic stirring and a condenser. The reaction system was evacuated and purged with nitrogen three times. The mixture was stirred and reacted at 50 °C overnight. The mixture was cooled to 0 °C, and hydrochloric acid (30 mL, 1 N) was added to adjust the pH to 4. The organic phase was separated, and the aqueous phase was extracted with ethyl acetate (20 mL × 2). The organic phases were combined, washed with saturated brine (20 mL), dried over anhydrous sodium sulfate, filtered, concentrated, and separated by silica gel column chromatography to give 3.3 g of a white solid, with a yield of 68.8%. LC-MS (APCI): m/z = 247.0$(M+1)^+$.

Step 3: Synthesis of compound 1-(2-(benzyloxy)ethyl)-1H-pyrazole-5-carboxamide

1-(2-(Benzyloxy)ethyl)-1H-pyrazole-5-carboxylic acid (3.3 g, 13.37 mmol), ammonium chloride (3.58 g, 66.85 mmol), 1-ethyl-3-(3-dimethylaminopropyl)carbodiimide (3.34 g, 17.38 mmol), 1-hydroxybenzotriazole (2.35 g, 17.38 mmol), and N,N-dimethylformamide (35 mL) were added to a 100 mL single-necked flask equipped with magnetic stirring and a condenser. The reaction system was evacuated and purged with nitrogen three times. The mixture was cooled to 0 °C, and triethylamine (5.6 mL, 40.11 mmol) was added. The mixture was reacted at room temperature for 2 hours. Saturated brine (30 mL) was added to quench the reaction. The organic phase was separated, and the aqueous phase was extracted with ethyl acetate (30 mL × 2). The organic phases were combined, washed with saturated brine (30 mL), dried over anhydrous sodium sulfate, filtered, concentrated and separated by silica gel column chromatography to give 3.20 g of a white solid, with a yield of 97.6%. LC-MS (APCI): m/z = 246.0 $(M+1)^+$.

Step 4: Synthesis of compound 1-(2-hydroxyethyl)-1H-pyrazole-5-carboxamide

1-(2-(Benzyloxy)ethyl)-1H-pyrazole-5-carboxamide (3.2 g, 13.2 mmol) and methanol (50 mL) were added to a 50 mL single-necked flask equipped with magnetic stirring. The mixture was stirred until dissolved, and palladium on carbon (10%, 320 mg) was added. The mixture was stirred to disperse. The reaction system was evacuated and purged with hydrogen three times. The mixture was stirred and reacted under hydrogen overnight. The mixture was filtered, and the filter residue was washed with methanol. The filtrate was concentrated to give 837 mg of a white solid, with a yield of 40.9%. LC-MS (APCI): m/z = 156.0 $(M+1)^+$.

Step 5: Synthesis of compound 1-(2-oxoethyl)-1H-pyrazole-5-carboxamide

1-(2-Hydroxyethyl)-1H-pyrazole-5-carboxamide (837 mg, 5.4 mmol) and dichloromethane (30 mL) were added to a 50 mL single-necked flask equipped with magnetic stirring. The mixture was stirred until dissolved, and Dess-Martin periodinane (5.73 g, 13.5 mmol) was added. The mixture was stirred at room temperature overnight. The mixture was filtered. The filtrate was concentrated and separated by silica gel column chromatography to give 300 mg of a white solid, with a yield of 36.3%. LC-MS (APCI): m/z = 154.0 $(M+1)^+$.

Step 6: Synthesis of compound pyrazolo[1,5-a]pyrazin-4-ol

1-(2-Oxoethyl)-1H-pyrazole-5-carboxamide (300 mg, 1.96 mmol) and dichloromethane (6 mL) were added to a 50 mL single-necked flask equipped with magnetic stirring. The mixture was stirred to disperse, and 2 drops of concentrated hydrochloric acid were added dropwise. The mixture was stirred and reacted at room temperature overnight. The mixture was filtered. The filter residue was washed with ethyl acetate and dried to give 250 mg of a white solid, with a yield of 94.4%. LC-MS (APCI): m/z =136.0 $(M+1)^+$.

Step 7 Synthesis of intermediate C-24

Pyrazolo[1,5-a]pyrazin-4-ol (250 mg, 1.85 mmol) and acetonitrile (6 mL) were added to a 50 mL single-necked flask equipped with magnetic stirring. The mixture was stirred to disperse, and phosphorus oxybromide (2.12 g, 7.4 mmol) was added. The mixture was stirred and reacted at 80 °C overnight. The mixture was cooled to 0 °C, and saturated sodium carbonate was added to adjust the pH to 10. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 171.4 mg of a white solid, with a yield of 47.0%. LC-MS (APCI): m/z = 198.0 $(M+1)^+$.

**Preparation of intermediate C-25 2-bromo-5-ethyl-4,5,6,7-tetrahydrothiazolo[5,4-c]pyridine**

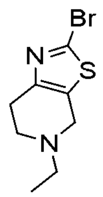

C-25

**The following synthetic route was adopted**

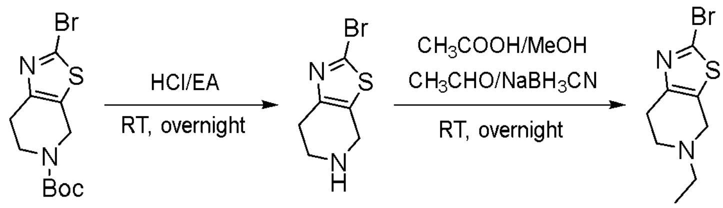


C-25

Step 1: Synthesis of compound 2-bromo-4,5,6,7-tetrahydrothiazolo[5,4-c]pyridine

tert-Butyl 2-bromo-6,7-dihydrothiazolo[5,4-c]pyridine-5(4H)-carboxylate (1.0 g, 3.14 mmol) and ethyl acetate (6 mL) were successively added to a 50 mL single-necked flask equipped with magnetic stirring. The mixture was stirred until dissolved, and a solution of hydrogen chloride in ethyl acetate (10 mL, 4 N) was added. The mixture was stirred and reacted at room temperature overnight. The mixture was filtered. The filter residue was washed with ethyl acetate (5 mL), and dried to give 686 mg of a white solid, with a yield of 85.8%. LC-MS (APCI): m/z = 219.0 $(M+1)^+$.

Step 2 Synthesis of intermediate C-25

2-Bromo-4,5,6,7-tetrahydrothiazolo[5,4-c]pyridine (686 mg, 2.7 mmol) and methanol (10 mL) were added to a 100 mL single-necked flask equipped with magnetic stirring. The mixture was stirred until dissolved. Glacial acetic acid (188 mg, 2.7 mmol) and acetaldehyde (0.63 mL, 2.97 mmol) were added. The mixture was cooled to 0 °C, and sodium cyanoborohydride (591 mg, 8.1 mmol) was added. The mixture was reacted at room temperature overnight. Saturated brine (10 mL) was added to quench the reaction. The organic phase was separated, and the aqueous phase was extracted with ethyl acetate (30 mL × 2). The organic phases were combined, washed with saturated brine (10 mL), dried over anhydrous sodium sulfate, filtered, concentrated, and separated by silica gel column chromatography to give 272.3 mg of a white solid, with a yield of 41.0%. $^1$H NMR (400 MHz, DMSO-$D_6$) δ (ppm): 3.56 (s, 2H), 2.74-2.69 (m, 4H), 2.50 (q, J = 7.2 Hz, 2H), 1.04 (t, J = 7.2 Hz, 3H). LC-MS (APCI): m/z = 247.0 $(M+1)^+$.

**Preparation of intermediate C-26 2-bromo-6,7-dihydro-9H-pyrido[2',3':4,5]imidazo[2,1-c][1,4]oxazine**

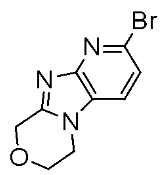

C-26

**The following synthetic route was adopted**

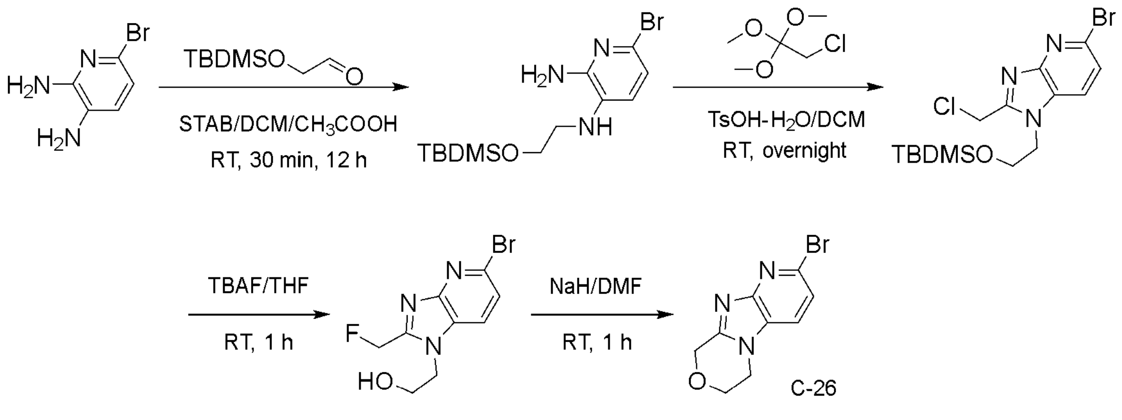


Step 1: Synthesis of compound 6-bromo-N$^3$-(2-((tert-butyldimethylsilyl)oxy)ethyl)pyridine-2,3-diamine

6-Bromopyridine-2,3-diamine (500 mg, 2.659 mmol) and dichloromethane (15 mL) were added to a 100 mL three-necked flask equipped with magnetic stirring, and the mixture was stirred until dissolved. 2-((tert-Butyldimethylsilyl)oxy)acetaldehyde (463.5 mg, 2.659 mmol) and 2 drops of glacial acetic acid were added. The reaction system was evacuated and purged with nitrogen three times. The mixture was reacted at room temperature for 30 minutes. Sodium triacetoxyborohydride (1.691 g, 7.977 mmol) was added, and the mixture was reacted at room temperature under nitrogen for 12 hours. Water (15 mL) was added to quench the reaction, and an aqueous solution of sodium bicarbonate was added to adjust the pH to alkaline. The mixture was extracted with dichloromethane (20 mL × 3). The organic phases were combined, washed with saturated brine (30 mL), dried over anhydrous sodium sulfate, filtered, concentrated, and

separated by silica gel column chromatography to give 670 mg of a white solid, with a yield of 73.0%. LC-MS (APCI): m/z = 346.0 $(M+1)^+$.

Step 2: Synthesis of compound 5-bromo-1-(2-((tert-butyldimethylsilyl)oxy)ethyl)-2-(chloromethyl)-1H-imidazo[4,5-b]pyridine

6-Bromo-$N^3$-(2-((tert-butyldimethylsilyl)oxy)ethyl)pyridine-2,3-diamine (670 mg, 1.942 mmol) and dichloromethane (15 mL) were added to a 50 mL three-necked flask equipped with magnetic stirring, and the mixture was stirred until dissolved. 2-Chloro-1,1,1-trimethoxyethane (900.65 mg, 5.826 mmol) and p-toluenesulfonic acid monohydrate (55.35 mg, 0.291 mmol) were added. The reaction system was evacuated and purged with nitrogen three times. The mixture was stirred and reacted at room temperature under nitrogen overnight. The reaction solution was concentrated and separated by silica gel column chromatography to give 600 mg of a white solid, with a yield of 76.66%. LC-MS (APCI): m/z = 404.0 $(M+1)^+$.

Step 3: Synthesis of compound 2-(5-bromo-2-(fluoromethyl)-1H-imidazo[4,5-b]pyridin-1-yl)ethan-1-ol

5-Bromo-1-(2-((tert-butyldimethylsilyl)oxy)ethyl)-2-(chloromethyl)-1H-imidazo[4,5-b]pyridine (600 mg, 1.489 mmol) and a solution of tetra-n-butylammonium fluoride in tetrahydrofuran (5 mL, 1 M) were added to a 25 mL three-necked flask equipped with magnetic stirring. The reaction system was evacuated and purged with nitrogen three times. The mixture was stirred and reacted at room temperature under nitrogen for 1 hour. The reaction solution was concentrated and separated by silica gel column chromatography to give 285 mg of a white solid, with a yield of 70.1%. LC-MS (APCI): m/z = 274.0 $(M+1)^+$.

Step 4: Synthesis of intermediate C-26

2-(5-Bromo-2-(fluoromethyl)-1H-imidazo[4,5-b]pyridin-1-yl)ethan-1-ol (285 mg, 1.044 mmol) and N,N-dimethylformamide (5 mL) were added to a 50 mL single-necked flask equipped with magnetic stirring. The reaction system was evacuated and purged with nitrogen three times. Sodium hydride (62.64 mg, 1.566 mmol) was added in batches in an ice-water bath. The mixture was stirred and reacted at room temperature under nitrogen for 1 hour. The reaction solution was concentrated to give 85 mg of a white solid, with a yield of 32.2%. $^1H$ NMR (400 MHz, DMSO-$D_6$) δ (ppm): 7.99 (d, J = 8.4 Hz, 1H), 7.45 (d, J = 8.4 Hz, 1H), 5.00 (s, 2H), 4.25 (t, J = 5.2 Hz, 2H), 4.17 (t, J = 5.2 Hz, 2H). LC-MS (APCI): m/z = 254.0 $(M+1)^+$.

**Preparation of intermediate C-27 2-bromo-6-methyl-6,7-dihydro-9H-pyrido[2',3':4,5]imidazo[2,1-c][1,4]oxazine**

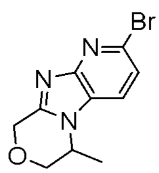

C-27

**The following synthetic route was adopted**

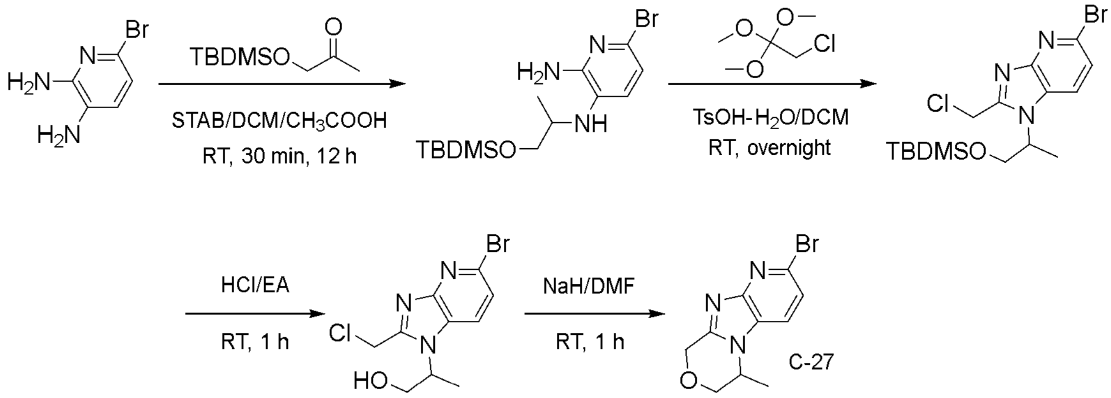


Step 1: Synthesis of compound 6-bromo-$N^3$-(1-((tert-butyldimethylsilyl)oxy)propan-2-yl)pyridine-2,3-diamine

6-Bromopyridine-2,3-diamine (1.5 g, 7.977 mmol) and dichloromethane (30 mL) were added to a 100 mL three-necked flask equipped with magnetic stirring, and the mixture was stirred until dissolved. 1-((tert-Butyldimethylsilyl)oxy)propan-2-one (1.5 g, 7.977 mmol) and 2 drops of glacial acetic acid were added. The reaction system was evacuated and purged with nitrogen three times. The mixture was reacted at room temperature for 30 minutes. Sodium triacetoxyborohydride (5.07 g, 23.931 mmol) was added, and the mixture was reacted at room temperature under nitrogen for 12 hours. Water (50 mL) was added to quench the reaction, and an aqueous solution of sodium bicarbonate was added to adjust the pH to alkaline. The mixture was extracted with dichloromethane (30 mL $\times$ 3). The organic phases were combined, washed with saturated brine (50 mL), dried over anhydrous sodium sulfate, filtered, concentrated, and separated by silica gel column chromatography to give 2.57 g of a white solid, with a yield of 89.8%. LC-MS (APCI): m/z = 360.0 $(M+1)^+$.

Step 2: Synthesis of compound 5-bromo-1-(1-((tert-butyldimethylsilyl)oxy)propan-2-yl)-2-(chloromethyl)-1H-imidazo[4,5-b]pyridine

6-Bromo-$N^3$-(1-((tert-butyldimethylsilyl)oxy)propan-2-yl)pyridine-2,3-diamine (2.57 g, 7.159 mmol) and dichloromethane (40 mL) were added to a 100 mL three-necked flask equipped with magnetic stirring, and the mixture was stirred until dissolved. 2-Chloro-1,1,1-trimethoxyethane (3.32 g, 21.476 mmol) and p-toluenesulfonic acid monohydrate (204.1 mg, 1.074 mmol) were added. The reaction system was evacuated and purged with nitrogen three times. The mixture was stirred and reacted at room temperature under nitrogen overnight. The reaction solution was concentrated and separated by silica gel column chromatography to give 1.85 g of a white solid, with a yield of 62%. LC-MS (APCI): m/z = 418.0 $(M+1)^+$.

Step 3: Synthesis of compound 2-(5-bromo-2-(chloromethyl)-1H-imidazo[4,5-b]pyridin-1-yl)propan-1-ol

5-Bromo-1-(1-((tert-butyldimethylsilyl)oxy)propan-2-yl)-2-(chloromethyl)-1H-imidazo[4,5-b]pyridine (1.85 g, 4.436 mmol) and a solution of hydrogen chloride in ethyl acetate (10 mL, 4 M) were added to a 50 mL three-necked flask equipped with magnetic stirring. The reaction system was evacuated and purged with nitrogen three times. The mixture was stirred and reacted at room temperature under nitrogen for 1 hour. The reaction solution was concentrated and separated by silica gel column chromatography to give 1.0 g of a white solid, with a yield of 75%. LC-MS (APCI): m/z = 304.0 $(M+1)^+$.

Step 4: Synthesis of intermediate C-27

2-(5-Bromo-2-(chloromethyl)-1H-imidazo[4,5-b]pyridin-1-yl)propan-1-ol (400 mg, 1.320 mmol) and N,N-dimethylformamide (5 mL) were added to a 50 mL single-necked flask equipped with magnetic stirring. The reaction system was evacuated and purged with nitrogen three times. Sodium hydride (105.6 mg, 2.640 mmol) was added in batches in an ice-water bath, and the mixture was stirred and reacted at room temperature under nitrogen for 1 hour. The reaction solution was concentrated to give 188 mg of a white solid, with a yield of 53.3%. LC-MS (APCI): m/z = 268.0 $(M+1)^+$.

**Preparation of intermediate C-28 4-(4-iodo-1H-imidazol-1-yl)-1-(oxetan-3-yl)piperidine**

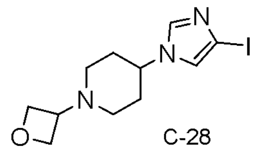

C-28

**The following synthetic route was adopted**

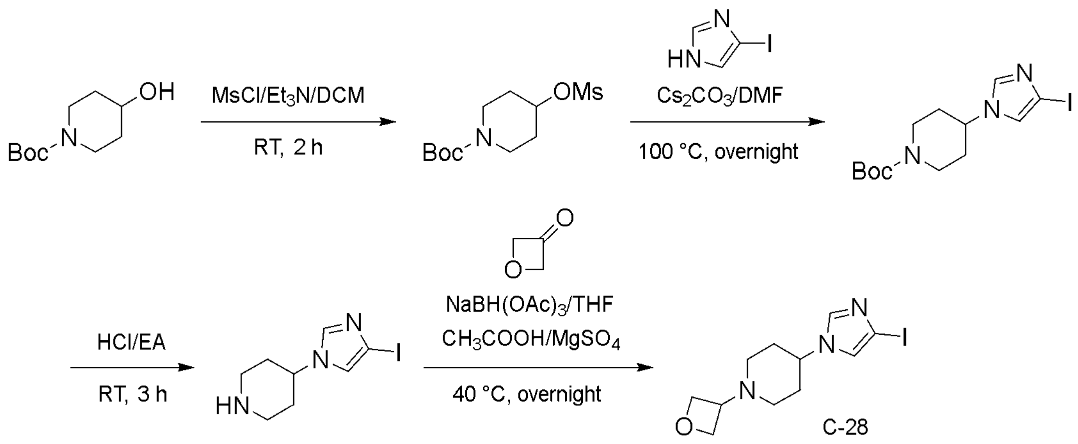


C-28

Step 1: Synthesis of compound tert-butyl 4-((methylsulfonyl)oxy)piperidine-1-carboxylate

tert-Butyl 4-hydroxypiperidine-1-carboxylate (970 mg, 5 mmol) and dichloromethane (20 mL) were added to a 100 mL three-necked flask equipped with magnetic stirring. The mixture was stirred until dissolved. The mixture was cooled to below 10 °C in an ice-water bath under nitrogen. Triethylamine (800 mg, 8 mmol) was added, and methanesulfonyl chloride (687 mg, 6 mmol) was slowly added dropwise. The mixture was warmed to room temperature, stirred and reacted for 2 hours. Water (10 mL) was added to the reaction system to quench the reaction, and the organic phase was separated. The aqueous phase was extracted with dichloromethane (20 mL). The organic phases were combined, dried over anhydrous sodium sulfate, filtered, and concentrated under reduced pressure to give a reddish-brown oil. The oil was separated by silica gel column chromatography to give 890 mg of a brown solid, with a yield of 63.8%. LC-MS (APCI): m/z = 280.0 $(M+1)^+$.

Step 2: Synthesis of compound tert-butyl 4-(4-iodo-1H-imidazol-1-yl)piperidine-1-carboxylate

tert-Butyl 4-((methylsulfonyl)oxy)piperidine-1-carboxylate (890 mg, 3.19 mmol), 4-iodoimidazole (890 mg, 3.83 mmol), and N,N-dimethylformamide (20 mL) were added to a 100 mL single-necked flask equipped with magnetic stirring, and the mixture was stirred until dissolved. Cesium carbonate (1.56 g, 4.79 mmol) was added, and the mixture was heated to 100 °C, stirred and reacted under nitrogen overnight. The mixture was cooled to room temperature, and the reaction mixture was poured into water (30 mL). The mixture was extracted with ethyl acetate (30 mL × 3). The organic phases were combined, washed with saturated brine (50 mL × 3), dried over anhydrous sodium sulfate, filtered, and concentrated under reduced pressure to give a brown oil. The oil was separated by silica gel column chromatography to give 670 mg of a brownish-yellow solid, with a yield of 55.7%. LC-MS (APCI): m/z = 378.0 $(M+1)^+$.

Step 3: Synthesis of compound 4-(4-iodo-1H-imidazol-1-yl)piperidine

tert-Butyl 4-(4-iodo-1H-imidazol-1-yl)piperidine-1-carboxylate (670 mg, 1.78 mmol) and a solution of hydrogen chloride in ethyl acetate (10 mL, 40 mmol, 4 M) were added to a 100 mL single-necked flask equipped with magnetic stirring. The mixture was stirred at room temperature under nitrogen for 3 hours. The solvent was removed by evaporation under reduced pressure to give 530 mg of a brownish-yellow solid, which was directly used in the next step reaction. LC-MS (APCI): m/z = 278.0 $(M+1)^+$.

Step 4 Synthesis of intermediate C-28

4-(4-Iodo-1H-imidazol-1-yl)piperidine crude product (530 mg, 1.78 mmol), 3-oxetanone (256 mg, 3.56 mmol), and anhydrous tetrahydrofuran (40 mL) were added to a 100 mL three-necked flask equipped with magnetic stirring, and

the mixture was stirred until dissolved. Anhydrous magnesium sulfate (427 mg, 3.56 mmol) and glacial acetic acid (107 mg, 1.78 mmol) were added. The mixture was stirred at 40 °C under nitrogen for 0.5 h. Sodium triacetoxyborohydride (1.1 g, 5.16 mmol) was added in batches with stirring, and the mixture was reacted at 40 °C overnight. The mixture was cooled to room temperature, and the reaction mixture was poured into water (30 mL). The mixture was extracted with dichloromethane (30 mL × 3). The organic phases were combined, dried over anhydrous sodium sulfate, filtered, and concentrated under reduced pressure to give a reddish-brown oil. The oil was separated by silica gel column chromatography to give 0.27 g of a brownish-yellow solid, with a two-step combined yield of 45.6%. LC-MS (APCI): m/z = 334.0 $(M+1)^+$.

**Example 1: Preparation of N-(4-(3-amino-6-(1,1,1-trifluoropropan-2-yl)-1H-pyrazolo[4,3-c]pyridin-4-yl)benzyl)-5-fluoro-2-methoxybenzamide**

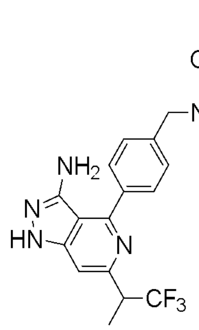

**The following synthetic route was adopted**

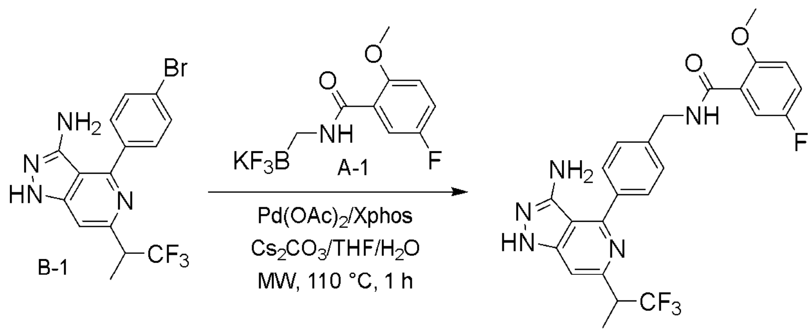


Intermediate B-1 (311 mg, 0.810 mmol), intermediate A-1 (280.9 mg, 0.972 mmol), palladium(II) acetate (9.09 mg, 0.041 mmol), 2-dicyclohexylphosphino-2',4',6'-triisoprophylbiphenyl (38.6 mg, 0.081 mmol), cesium carbonate (79.2 mg, 0.243 mmol), tetrahydrofuran (10 mL), and water (1 mL) were successively added to a 20 mL microwave vial equipped with magnetic stirring. The reaction solution was purged with nitrogen for 2 minutes. The vial was sealed. The mixture was heated to 110 °C, microwaved and reacted for 1 hour. Water (20 mL) was added. The mixture was extracted with ethyl acetate (20 mL × 2). The organic phases were combined, washed with saturated brine (30 mL), dried over anhydrous sodium sulfate, filtered, concentrated, and separated by silica gel column chromatography to give 256.4 mg of a white solid, with a yield of 65.5%. LC-MS (APCI): m/z = 488.0 $(M+1)^+$. $^1$H NMR (400 MHz, DMSO-$D_6$) δ (ppm): 12.23 (s, 1H), 8.89 (t, J = 6.0 Hz, 1H), 7.68(d, J = 8.4 Hz, 2H), 7.55-7.50 (m, 3H), 7.38-7.33 (m, 1H), 7.28 (s, 1H), 7.21-7.18 (m, 1H), 4.76 (s, 2H), 4.60 (d, J = 5.6 Hz, 2H), 4.02-3.96 (m, 1H), 3.91 (s, 3H), 1.54 (d, J = 6.8 Hz, 3H).

**Example 2 Preparation of N-(4-(3-amino-6-(1,1,1-trifluoropropan-2-yl)-1H-pyrazolo[4,3-c]pyridin-4-yl)benzyl)-5-fluoro-2-methoxybenzamide (isomer 1), and;**

**Example 3 Preparation of N-(4-(3-amino-6-(1,1,1-trifluoropropan-2-yl)-1H-pyrazolo[4,3-c]pyridin-4-yl)benzyl)-5-fluoro-2-methoxybenzamide (isomer 2)**

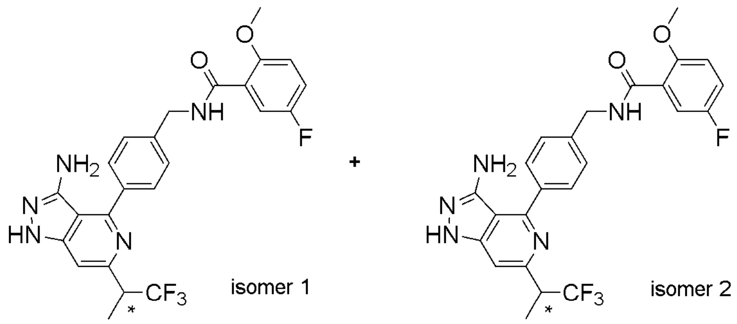

**The following synthetic route was adopted**

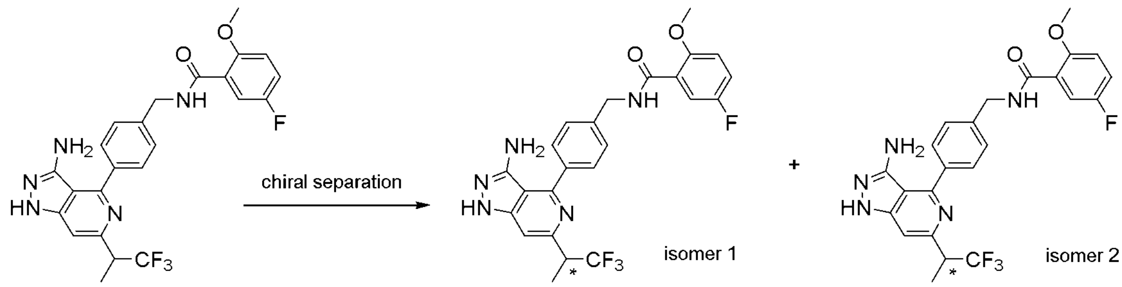

The racemic compound of example 1 (250 mg) was dissolved in methanol (10 mL) and the following conditions were used for chiral preparation: IG column;
flow rate: 4 mL/min; resolution conditions: ethyl acetate: n-hexane: ethanol = 18:72:10 (0.1% triethylamine); 110 mg of isomer example 2 (isomer 1) with a peak time of 27.053 min was obtained, and 110 mg of isomer example 3 (isomer 2) with a peak time of 36.335 min was obtained.

**Example 4 Preparation of N-(4-(3-amino-7-bromo-6-(1,1,1-trifluoropropan-2-yl)-1H-pyrazolo[4,3-c]pyridin-4-yl) benzyl)-5-fluoro-2-methoxybenzamide**

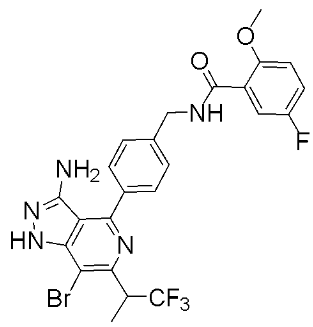

**The following synthetic route was adopted**

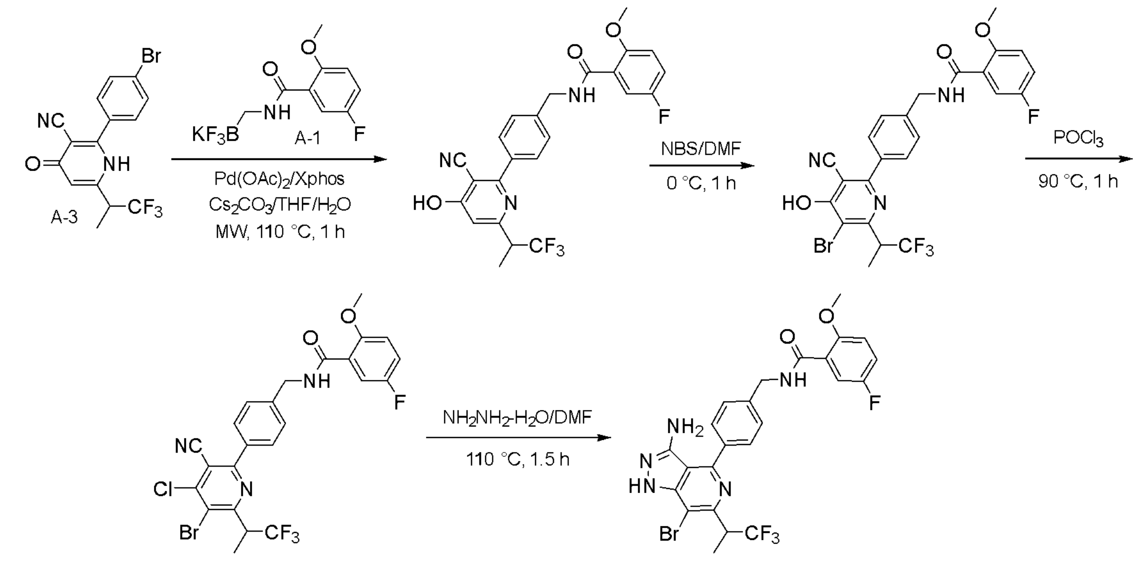


Step 1: Synthesis of compound N-(4-(3-cyano-4-hydroxy-6-(1,1,1-trifluoropropan-2-yl)pyridin-2-yl)benzyl)-5-fluoro-2-methoxybenzamide

Intermediate A-1 (936.3 mg, 3.24 mmol), intermediate A-3 (1 g, 2.70 mmol), palladium(II) acetate (30.3 mg, 0.135 mmol), 2-dicyclohexylphosphino-2',4',6'-triisoprophylbiphenyl (128.7 mg, 0.27 mmol), cesium carbonate (2.64 g, 8.1 mmol), tetrahydrofuran (15 mL), and water (1.5 mL) were successively added to a 20 mL microwave vial equipped with magnetic stirring. The reaction solution was purged with nitrogen for 2 minutes. The vial was sealed. The mixture was heated to 110 °C, microwaved and reacted for 1 hour. Water (30 mL) was added. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 640 mg of a white solid, with a yield of 50.0%. LC-MS (APCI): m/z = 474.0 $(M+1)^+$. $^1H$ NMR (400 MHz, DMSO-$D_6$) δ (ppm): 8.91 (t, J = 6.0 Hz, 1H), 7.73 (d, J = 8.0 Hz, 2H), 7.53-7.50 (m, 3H), 7.37-7.33 (m, 1H), 7.21-7.17 (m, 1H), 4.58 (d, J=6.0 Hz, 2H), 4.01-3.96 (m, 4H), 1.46 (d, J=6.8 Hz, 3H).

Step 2: Synthesis of compound N-(4-(5-bromo-3-cyano-4-hydroxy-6-(1,1,1-trifluoropropan-2-yl)pyridin-2-yl)benzyl)-5-fluoro-2-methoxybenzamide

N-(4-(3-Cyano-4-hydroxy-6-(1,1,1-trifluoropropan-2-yl)pyridin-2-yl)benzyl)-5-fluoro-2-methoxybenzamide (640 mg, 1.353 mmol) and N,N-dimethylformamide (10 mL) were added to a 50 mL three-necked flask equipped with magnetic stirring and a condenser. N-bromosuccinimide (264.89 mg, 1.488 mmol) was added in batches in an ice-water bath. The mixture was stirred and reacted at 0 °C under nitrogen for 1 hour. Water (30 mL) was added, and the mixture was extracted with ethyl acetate (30 mL × 2). The organic phases were combined, washed with saturated brine (30 mL), dried over anhydrous sodium sulfate, filtered, concentrated, and separated by silica gel column chromatography to give 560 mg of a white solid, with a yield of 75.0%. LC-MS (APCI): m/z = 552.0 $(M+1)^+$. $^1H$ NMR (400 MHz, DMSO-$D_6$) δ (ppm): 8.88-8.86 (m, 1H), 7.67 (d, J = 8.0 Hz, 2H), 7.53-7.50 (m, 3H), 7.44-7.40 (m, 1H), 7.36-7.31 (m, 1H), 7.20-7.16 (m, 1H), 4.55 (d, J=6.0 Hz, 2H), 4.47-4.13 (m, 1H), 3.90 (s, 3H), 1.49-1.46 (m, 3H).

Step 3: Synthesis of compound N-(4-(5-bromo-4-chloro-3-cyano-6-(1,1,1-trifluoropropan-2-yl)pyridin-2-yl)benzyl)-5-fluoro-2-methoxybenzamide

N-(4-(5-Bromo-3-cyano-4-hydroxy-6-(1,1,1-trifluoropropan-2-yl)pyridin-2-yl)benzyl)-5-fluoro-2-methoxybenzamide (560 mg, 1.016 mmol) and phosphorus oxychloride (2 mL) were added to a 25 mL single-necked flask equipped with magnetic stirring and a condenser. The mixture was stirred and reacted at 90 °C under nitrogen for 1 hour. The mixture was cooled to room temperature, and saturated sodium bicarbonate solution (10 mL) was added. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 347 mg of a white solid, with a yield of 60%. LC-MS (APCI): m/z = 570.0 $(M+1)^+$. $^1H$ NMR (400 MHz, DMSO-$D_6$) δ (ppm): 8.93 (t, J = 6.0 Hz, 1H), 7.89 (d, J = 8.4 Hz, 2H), 7.56-7.52 (m, 3H), 7.38-7.33 (m, 1H), 7.22-7.18 (m, 1H), 4.61-4.57 (m, 3H), 3.91 (s, 3H), 1.55 (d, J = 6.8 Hz, 3H).

Step 4: Synthesis of example 4

N-(4-(5-Bromo-4-chloro-3-cyano-6-(1,1,1-trifluoropropan-2-yl)pyridin-2-yl)benzyl)-5-fluoro-2-methoxybenzamide (347 mg, 0.610 mmol), hydrazine hydrate (152.7 mg, 3.05 mmol), and N,N-dimethylformamide (3 mL) were added to a 25 mL sealed vial. The mixture was stirred and reacted at 110 °C under nitrogen for 1.5 h. The mixture was cooled in an ice-water bath, and saturated brine was added. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 224 mg of a white solid, with a yield of 65%. LC-MS (APCI): m/z = 566.0 $(M+1)^+$. $^1$H NMR (400 MHz, DMSO-$D_6$) δ (ppm): 12.71 (s, 1H), 8.89 (t, J = 6.0 Hz, 1H), 7.68 (d, J = 8.4 Hz, 2H), 7.55-7.50 (m, 3H), 7.38-7.33 (m, 1H), 7.21-7.18 (m, 1H), 4.92 (s, 2H), 4.60 (d, J = 5.6 Hz, 2H), 4.37-4.33 (m, 1H), 3.91 (s, 3H), 1.54 (d, J = 6.8 Hz, 3H).

**Example 5 Preparation of N-(4-(3-amino-7-(pyridin-2-yl)-6-(1,1,1-trifluoropropan-2-yl)-1H-pyrazolo[4,3-c]pyridin-4-yl)benzyl)-5-fluoro-2-methoxybenzamide**

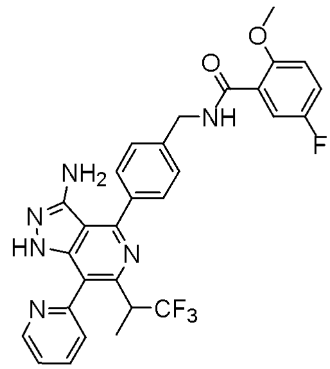

**The following synthetic route was adopted**

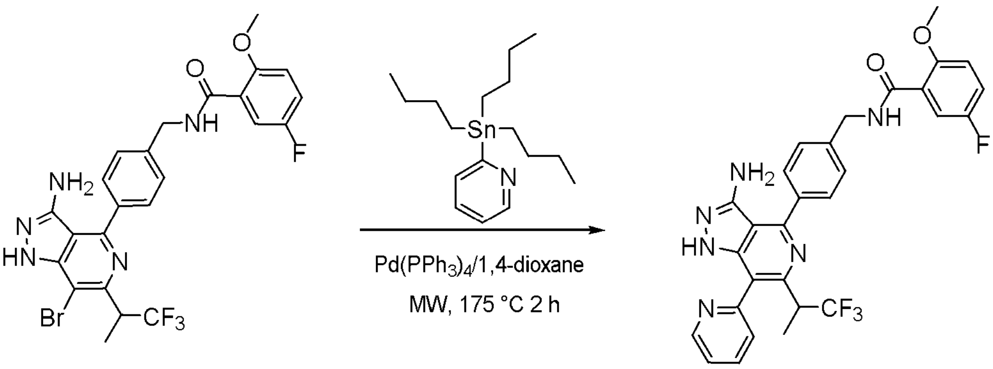


Example 4 (200 mg, 0.354 mmol), 2-(tributylstannyl)pyridine (260.6 mg, 0.708 mmol), tetrakis(triphenylphosphine)palladium(0) (40.9 mg, 0.035 mmol) and 1,4-dioxane (6 mL) were added to a 10 mL microwave vial. The reaction solution was purged with nitrogen for 2 minutes. The vial was sealed, and the mixture was microwaved and reacted at 175 °C for 2 hours. The mixture was cooled to room temperature, and saturated aqueous solution of potassium fluoride (20 mL) was added. The mixture was stirred for 10 minutes, and extracted with ethyl acetate (30 mL × 2). The organic phases were combined, washed with saturated brine (30 mL), dried over anhydrous sodium sulfate, filtered, concentrated and separated by silica gel column chromatography to give 20 mg of a white solid, with a yield of 10%. LC-MS (APCI): m/z= 565.0 $(M+1)^+$. $^1$H NMR (400 MHz, DMSO-$D_6$) δ (ppm): 12.04 (s, 1H), 8.90 (t, J = 6.4 Hz, 1H), 8.80 (d, J = 3.6 Hz, 1H), 8.03-7.98 (m, 1H), 7.76(d, J = 8.8 Hz, 2H), 7.63 (d, J = 8.0 Hz, 1H), 7.56-7.53 (m, 4H), 7.37-7.32 (m, 1H), 7.21-7.18 (m, 1H), 4.83 (s, 2H), 4.62 (d, J = 6.0 Hz, 2H), 4.08-4.05 (m, 1H), 3.91 (s, 3H), 1.51 (d, J = 7.2 Hz, 3H).

**Example 6 Preparation of N-(4-(3-amino-7-(1-methyl-1H-pyrazol-3-yl)-6-(1,1,1-trifluoropropan-2-yl)-1H-pyrazolo[4,3-c]pyridin-4-yl)benzyl)-5-fluoro-2-methoxybenzamide**

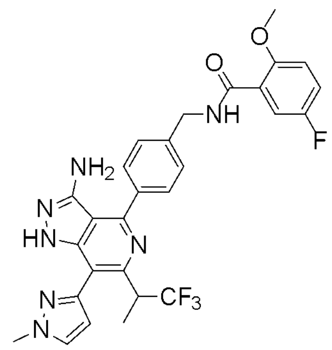

**The following synthetic route was adopted**

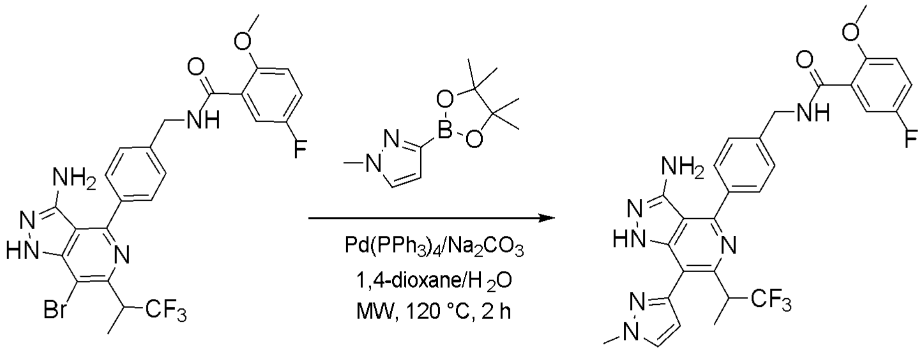


Example 4 (200 mg, 0.354 mmol), 1-methylpyrazole-3-boronic acid pinacol ester (147.3 mg, 0.708 mmol), tetrakis(triphenylphosphine)palladium(0) (40.9 mg, 0.035 mmol), sodium carbonate (112.6 mg, 1.062 mmol), 1,4-dioxane (6 mL), and water (1.5 mL) were added to a 10 mL microwave vial. The reaction solution was purged with nitrogen for 2 minutes. The mixture was heated to 120 °C, microwaved and reacted for 2 hours. The mixture was cooled to room temperature, and water (30 mL) was added to quench the reaction. The mixture was extracted with ethyl acetate (30 mL × 2). The organic phases were combined, washed with saturated brine (30 mL), dried over anhydrous sodium sulfate, filtered, concentrated, and separated by silica gel column chromatography to give 130 mg of a white solid, with a yield of 64.8%. LC-MS (APCI): m/z = 568.0 $(M+1)^+$.

**Example 6-1 Preparation of N-(4-(3-amino-7-(1-methyl-1H-pyrazol-3-yl)-6-(1,1,1-trifluoropropan-2-yl)-1H-pyrazolo[4,3-c]pyridin-4-yl)benzyl)-5-fluoro-2-methoxybenzamide (isomer 1), and**

**Example 6-2 Preparation of N-(4-(3-amino-7-(1-methyl-1H-pyrazol-3-yl)-6-(1,1,1-trifluoropropan-2-yl)-1H-pyrazolo[4,3-c]pyridin-4-yl)benzyl)-5-fluoro-2-methoxybenzamide (isomer 2)**

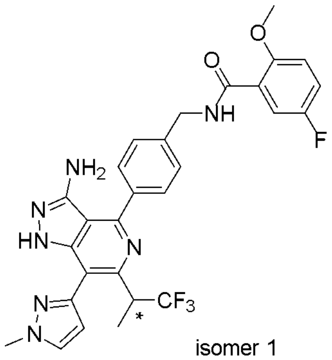

isomer 1

+

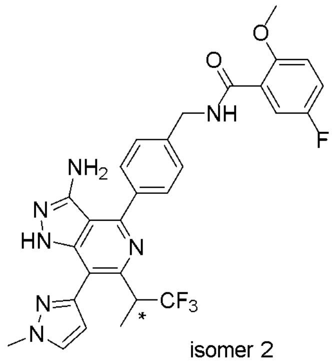

isomer 2

The racemic compound of example 6 (130 mg) was dissolved in methanol (10 mL) and the following conditions were used for chiral preparation: IG column; flow rate: 4 mL/min; resolution conditions: n-hexane:methanol:ethanol = 80:10:10 (0.1% triethylamine); 50 mg of isomer example 6-1 (isomer 1) with a peak time of 25.177 min was obtained, and 50 mg of isomer example 6-2 (isomer 2) with a peak time of 27.004 min was obtained.

**Example 7 Preparation of N-(4-(3-amino-7-(pyrimidin-2-yl)-6-(1,1,1-trifluoropropan-2-yl)-1H-pyrazolo[4,3-c]pyridin-4-yl)benzyl)-5-fluoro-2-methoxybenzamide**

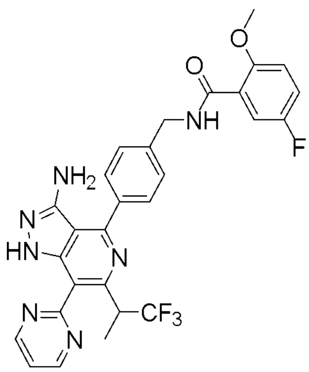

Example 7 was prepared using 2-(tributylstannyl)pyrimidine instead of 2-(tributylstannyl)pyridine as the raw material according to the method described in example 5, with a production of 100 mg and a yield of 31.2%. LC-MS (APCI): m/z = 566.0 $(M+1)^+$. $^1H$ NMR (400 MHz, DMSO-$D_6$) δ (ppm): 12.31 (s, 1H), 9.04 (t, J = 8.8 Hz, 1H), 8.91 (t, J = 6.4 Hz, 1H), 7.78 (d, J = 8.8 Hz, 2H), 7.56-7.53 (m, 4H), 7.37-7.32 (m, 1H), 7.21-7.18 (m, 1H), 5.44-5.40 (m, 1H), 4.89 (s, 2H), 4.61 (d, J = 6.0 Hz, 2H), 3.91 (s, 3H), 1.58 (d, J = 9.6 Hz, 3H).

**Example 7-1 Preparation of N-(4-(3-amino-7-(pyrimidin-2-yl)-6-(1,1,1-trifluoropropan-2-yl)-1H-pyrazolo[4,3-c]pyridin-4-yl)benzyl)-5-fluoro-2-methoxybenzamide (isomer 1), and**

**Example 7-2 Preparation of N-(4-(3-amino-7-(pyrimidin-2-yl)-6-(1,1,1-trifluoropropan-2-yl)-1H-pyrazolo[4,3-c]pyridin-4-yl)benzyl)-5-fluoro-2-methoxybenzamide (isomer 2)**

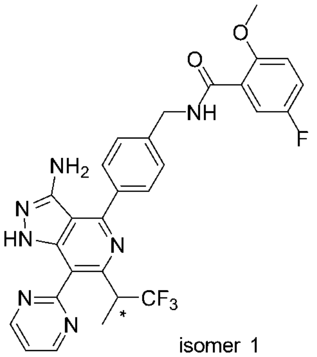

+

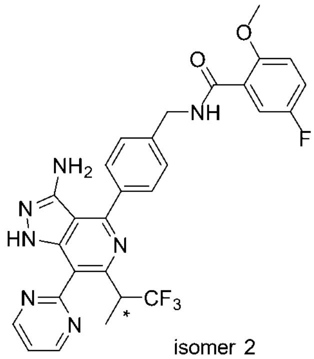

isomer 1 isomer 2

The racemic compound of example 7 (100 mg) was dissolved in methanol (10 mL) and the following conditions were used for chiral preparation: IG column; flow rate: 4 mL/min; resolution conditions: methanol; 40 mg of isomer example 7-1 (isomer 1) with a peak time of 9.667 min was obtained, and 40 mg of isomer example 7-2 (isomer 2) with a peak time of 13.098 min was obtained.

**Example 8 Preparation of N-(4-(3-amino-7-(pyrazin-2-yl)-6-(1,1,1-trifluoropropan-2-yl)-1H-pyrazolo[4,3-c]pyridin-4-yl)benzyl)-5-fluoro-2-methoxybenzamide**

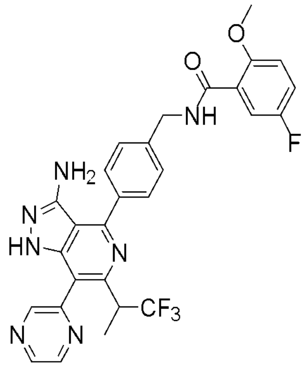

Example 8 was prepared using 2-(tributylstannyl)pyrazine instead of 2-(tributylstannyl)pyridine as the raw material according to the method described in example 5, with a production of 35 mg and a yield of 17.5%. LC-MS (APCI): m/z = 566.0 $(M+1)^+$. $^1H$ NMR (400 MHz, DMSO-$D_6$) δ (ppm): 12.20 (s, 1H), 8.90-8.87 (m, 3H), 8.77 (d, J = 2.0 Hz, 1H), 7.76 (d, J = 8.4 Hz, 2H), 7.55-7.52 (m, 3H), 7.34-7.32 (m, 1H), 7.20-7.17 (m, 1H), 4.87 (s, 2H), 4.61 (d, J = 6.0 Hz, 2H), 3.91 (s, 3H), 3.90-3.87 (m, 1H), 1.50 (d, J = 7.2 Hz, 3H).

**Example 9 Preparation of N-(4-(3-amino-7-(5-methyl-1H-pyrazol-3-yl)-6-(1,1,1-trifluoropropan-2-yl)-1H-pyrazolo [4,3-c]pyridin-4-yl)benzyl)-5-fluoro-2-methoxybenzamide**

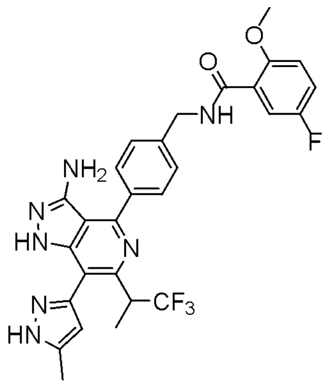

Example 9 was prepared using (5-methyl-1H-pyrazol-3-yl)boronic acid instead of 1-methylpyrazole-3-boronic acid pinacol ester as the raw material according to the method described in example 6, with a production of 100 mg and a yield of 29.5%. LC-MS (APCI): m/z = 568.0 $(M+1)^+$.

**Example 9-1 Preparation of N-(4-(3-amino-7-(5-methyl-1H-pyrazol-3-yl)-6-(1,1,1-trifluoropropan-2-yl)-1H-pyrazolo[4,3-c]pyridin-4-yl)benzyl)-5-fluoro-2-methoxybenzamide (isomer 1), and**

**Example 9-2 Preparation of N-(4-(3-amino-7-(5-methyl-1H-pyrazol-3-yl)-6-(1,1,1-trifluoropropan-2-yl)-1H-pyrazolo[4,3-c]pyridin-4-yl)benzyl)-5-fluoro-2-methoxybenzamide (isomer 2)**

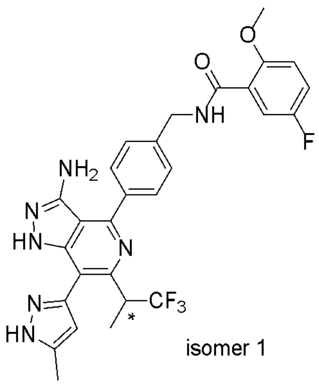

isomer 1

+

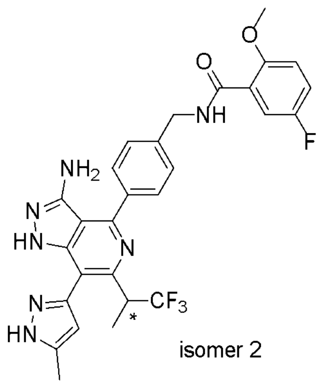

isomer 2

The racemic compound of example 9 (100 mg) was dissolved in methanol (10 mL) and the following conditions were used for chiral preparation: IG column; flow rate: 4 mL/min; resolution conditions: ethyl acetate:ethanol = 80:20 (0.1% triethylamine); 37 mg of isomer example 9-1 (isomer 1) with a peak time of 25.957 min was obtained, and 37 mg of isomer example 9-2 (isomer 2) with a peak time of 29.117 min was obtained.

**Example 10 Preparation of N-(4-(3-amino-7-(pyrimidin-5-yl)-6-(1,1,1-trifluoropropan-2-yl)-1H-pyrazolo[4,3-c]pyridin-4-yl)benzyl)-5-fluoro-2-methoxybenzamide**

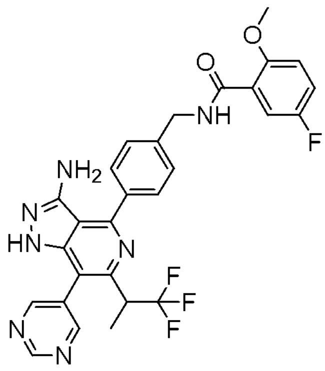

Example 10 was prepared using pyrimidine-5-boronic acid instead of 1-methylpyrazole-3-boronic acid pinacol ester as the raw material according to the method described in example 6, with a production of 115 mg and a yield of 57.5%. LC-MS (APCI): m/z = 566.0 $(M+1)^+$. $^1H$ NMR (400 MHz, DMSO-$D_6$) δ (ppm): 12.16 (s, 1H), 9.35 (s, 1H), 8.92-8.88 (m, 3H), 7.74 (d, J = 8.4 Hz, 2H), 7.55-7.52 (m, 3H), 7.34-7.32 (m, 1H), 7.20-7.17 (m, 1H), 4.87 (s, 2H), 4.61 (d, J = 6.0 Hz, 2H), 3.91 (s, 3H), 3.62-3.57 (m, 1H), 1.50 (d, J = 7.2 Hz, 3H).

**Example 11 Preparation of N-(4-(3-amino-7-(pyridazin-4-yl)-6-(1,1,1-trifluoropropan-2-yl)-1H-pyrazolo[4,3-c] pyridin-4-yl)benzyl)-5-fluoro-2-methoxybenzamide**

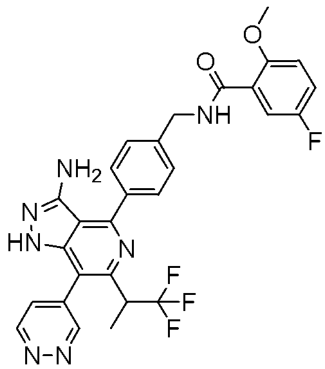

Example 11 was prepared using 4-(tributylstannyl)pyridazine instead of 2-(tributylstannyl)pyridine as the raw material according to the method described in example 5, with a production of 31 mg and a yield of 15.5%. LC-MS (APCI): m/z = 566.0 $(M+1)^+$. $^1H$ NMR (400 MHz, DMSO-$D_6$) δ (ppm): 12.16 (s, 1H), 9.42 (d, J = 4.2 Hz, 1H), 9.32 (s, 1H), 8.89 (t, J = 6.0 Hz, 1H), 7.83-7.81 (m, 1H), 7.75 (d, J = 8.0 Hz, 2H), 7.55-7.52 (m, 3H), 7.36-7.32 (m, 1H), 7.20-7.17 (m, 1H), 4.88 (s, 2H), 4.61 (d, J=6.0 Hz, 2H), 3.90 (s, 3H), 3.61-3.50 (m, 1H), 1.50 (d, J = 7.2 Hz, 3H).

**Example 12 Preparation of N-(4-(3-amino-7-(5-(trifluoromethyl)pyridin-2-yl)-6-(1,1,1-trifluoropropan-2-yl)-1H-pyrazolo[4,3-c]pyridin-4-yl)benzyl)-5-fluoro-2-methoxybenzamide**

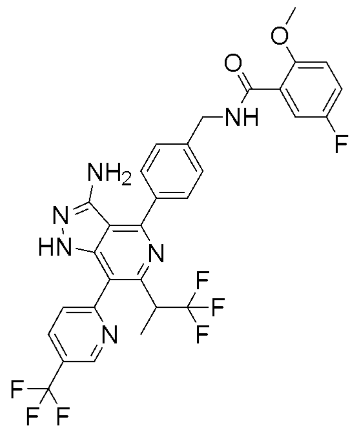

Example 12 was prepared using 2-(tributylstannyl)-5-trifluoromethylpyridine instead of 2-(tributylstannyl)pyridine as the raw material according to the method described in example 5, with a production of 36 mg and a yield of 16.0%. LC-MS (APCI): m/z = 633.0 $(M+1)^+$. $^1H$ NMR (400 MHz, DMSO-$D_6$) δ (ppm): 12.10 (s, 1H), 9.19 (s, 1H), 8.91 (t, J = 6.0 Hz, 1H), 8.43-8.41 (m, 1H), 7.88 (d, J = 8.4 Hz, 1H), 7.77 (d, J = 8.0 Hz, 2H), 7.56-7.53 (m, 3H), 7.38-7.33 (m, 1H), 7.22-7.18 (m, 1H), 4.88 (s, 2H), 4.61 (d, J = 6.0 Hz, 2H), 4.04-4.00 (m, 1H), 3.92 (s, 3H), 1.52 (d, J = 7.2 Hz, 3H).

**Example 13 Preparation of N-(4-(3-amino-7-(5-fluoropyridin-2-yl)-6-(1,1,1-trifluoropropan-2-yl)-1H-pyrazolo [4,3-c]pyridin-4-yl)benzyl)-5-fluoro-2-methoxybenzamide**

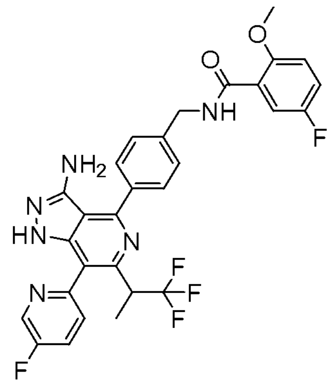

Example 13 was prepared using 5-fluoro-2-(tributylstannyl)pyridine instead of 2-(tributylstannyl)pyridine as the raw material according to the method described in example 5, with a production of 31 mg and a yield of 15.1%. LC-MS (APCI): m/z = 583.0 $(M+1)^+$. $^1H$ NMR (400 MHz, DMSO-$D_6$) δ (ppm): 12.04 (s, 1H), 8.91 (t, J = 6.0 Hz, 1H), 8.80 (d, J = 3.2 Hz, 1H), 7.97-7.92 (m, 1H), 7.88 (d, J = 8.4 Hz, 1H), 7.73-7.70 (m, 1H), 7.56-7.53 (m, 3H), 7.35-7.32 (m, 1H), 7.21-7.18 (m, 1H), 4.85 (s, 2H), 4.61 (d, J = 6.0 Hz, 2H), 4.04-4.00 (m, 1H), 3.92 (s, 3H), 1.50 (d, J = 7.2 Hz, 3H).

**Example 14 Preparation of N-(4-(3-amino-7-phenyl-6-(1,1,1-trifluoropropan-2-yl)-1H-pyrazolo[4,3-c]pyridin-4-yl)benzyl)-5-fluoro-2-methoxybenzamide**

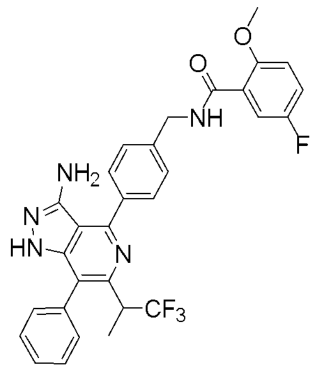

Example 14 was prepared using phenylboronic acid instead of 1-methylpyrazole-3-boronic acid pinacol ester as the raw material according to the method described in example 6, with a production of 80 mg and a yield of 58.3%. LC-MS (APCI): m/z = 564.2 $(M+1)^+$. $^1H$ NMR (400 MHz, DMSO-$D_6$) δ (ppm): 12.14 (s, 1H), 8.91 (t, J = 6.0 Hz, 1H), 7.73-7.70 (m, 3H), 7.56-7.53 (m, 3H), 7.35-7.32 (m, 1H), 7.25-7.23 (m, 3H), 7.21-7.18 (m, 1H), 4.88 (s, 2H), 4.60 (d, J = 6.0 Hz, 2H), 4.04-4.00 (m, 1H), 3.90 (s, 3H), 1.49 (d, J = 7.2 Hz, 3H).

**Example 15 Preparation of N-(4-(3-amino-7-(pyridin-2-yl)-1H-pyrazolo[4,3-c]pyridin-4-yl)benzyl)-5-fluoro-2-methoxybenzamide**

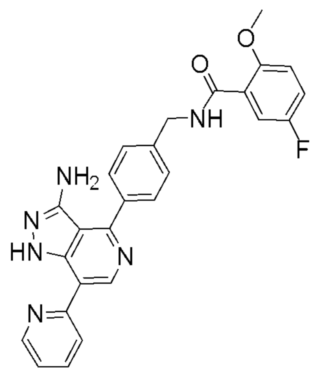

**The following synthetic route was adopted**

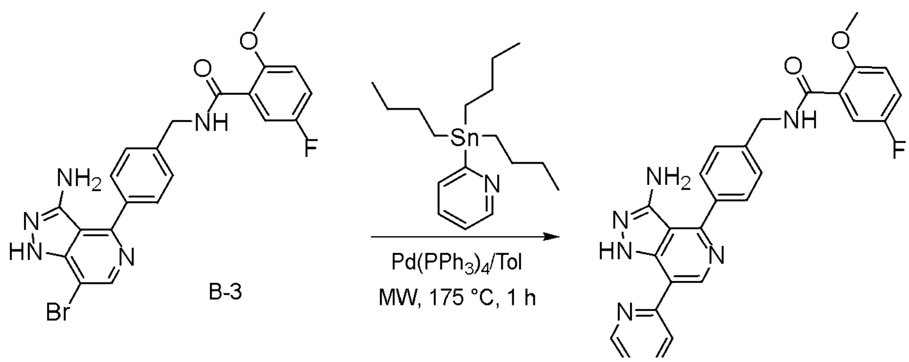


Intermediate B-3 (200 mg, 0.43 mmol), 2-(tributylstannyl)pyridine (290 mg, 0.64 mmol), tetrakis(triphenylphosphine)palladium(0) (50 mg, 0.043 mmol) and toluene (12 mL) were added to a 20 mL microwave vial equipped with magnetic stirring. The reaction solution was purged with nitrogen for 2 minutes. The mixture was heated to 175 °C in a microwave reactor, and reacted for 1 hour. The mixture was cooled to room temperature. Ethyl acetate (30 mL) and saturated aqueous solution of potassium fluoride (20 mL) were added. The mixture was stirred for 10 minutes, and the organic phase was separated. The aqueous phase was extracted with ethyl acetate (20 mL × 2). The organic phases were combined, dried over anhydrous sodium sulfate, filtered, concentrated and separated by silica gel column chromatography to give 30 mg of a white solid, with a yield of 18.6%. LC-MS (APCI): m/z = 469 $(M+1)^+$. $^1H$ NMR (400 MHz, DMSO-$D_6$) δ (ppm): 12.36 (s, 1H), 8.92 (s, 1H), 8.83 (t, J = 6.0 Hz, 1H), 8.70 (d, J = 8.4 Hz, 1H), 8.17 (d, J = 8.0 Hz, 1H), 7.90-7.86 (m, 1H), 7.65 (d, J = 8.0 Hz, 2H), 7.48-7.45 (m, 3H), 7.35-7.25 (m, 2H), 7.14-7.11 (m, 1H), 4.75 (br s, 2H), 4.55 (d, J = 6.0 Hz, 2H), 3.84 (s, 3H).

**Example 16 Preparation of N-(4-(3-amino-7-(pyrimidin-5-yl)-1H-pyrazolo[4,3-c]pyridin-4-yl)benzyl)-5-fluoro-2-methoxybenzamide**

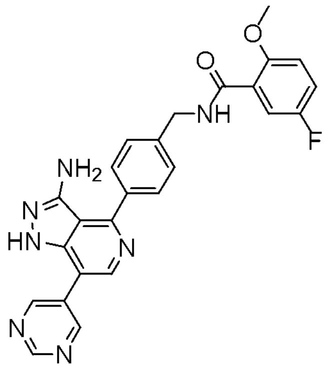

**The following synthetic route was adopted**

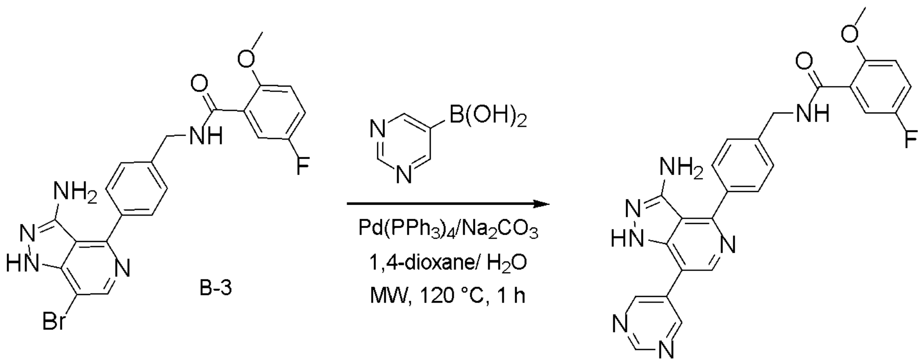


Intermediate B-3 (200 mg, 0.43 mmol), pyrimidine-5-boronic acid (80 mg, 0.64 mmol), tetrakis(triphenylphosphine)palladium(0) (50 mg, 0.043 mmol), sodium carbonate (137 mg, 1.29 mmol), 1,4-dioxane (5 mL), and water (1 mL) were added to a 10 mL microwave reaction vial equipped with magnetic stirring. The reaction solution was purged with nitrogen for two minutes. The vial was sealed. The mixture was heated to 120 °C in a microwave reactor, and reacted for 1 hour. The solvent was removed by evaporation under reduced pressure. The residue was separated by silica gel column chromatography to give 80 mg of a white solid, with a yield of 40%. LC-MS (APCI): m/z = 470 $(M+1)^+$. $^1H$ NMR (400 MHz, DMSO-$D_6$) δ (ppm): 12.61 (s, 1H), 9.27 (s, 1H), 9.16 (s, 2H), 8.90 (t, J = 6.0 Hz, 1H), 8.42 (s, 1H), 7.69 (d, J = 8.0 Hz, 1H), 7.54-7.51 (m, 3H), 7.36-7.31 (m, 1H), 7.20-7.17 (m, 1H), 4.83 (br s, 2H), 4.60 (d, J = 6.0 Hz, 2H), 3.90 (s, 3H).

**Example 17 Preparation of N-(4-(3-amino-7-(pyrimidin-2-yl)-1H-pyrazolo[4,3-c]pyridin-4-yl)benzyl)-5-fluoro-2-methoxybenzamide**

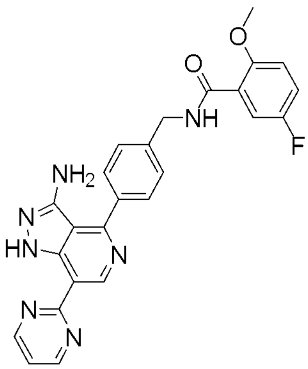

Example 17 was prepared using 2-(tributylstannyl)pyrimidine instead of 2-(tributylstannyl)pyridine as the raw material according to the method described in example 15, with a production of 35 mg and a yield of 23.4%. LC-MS (APCI): m/z = 470.2 $(M+1)^+$. $^1H$ NMR (400 MHz, DMSO-$D_6$) δ (ppm): 12.40 (s, 1H), 9.27 (s, 1H), 8.96 (s, 1H), 8.94 (s, 1H), 8.89 (t, J = 6.0 Hz, 1H), 7.72 (d, J = 8.0 Hz, 1H), 7.54-7.51 (m, 3H), 7.46 (t, J = 4.8 Hz, 1H), 7.36-7.31 (m, 1H), 7.20-7.17 (m, 1H), 4.85 (br s, 2H), 4.61 (d, J = 6.0 Hz, 2H), 3.89 (s, 3H).

**Example 18 Preparation of N-(4-(3-amino-7-(pyrazin-2-yl)-1H-pyrazolo[4,3-c]pyridin-4-yl)benzyl)-5-fluoro-2-methoxybenzamide**

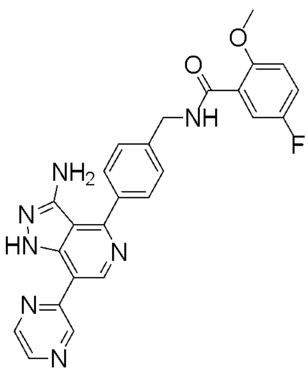

Example 18 was prepared using 2-(tributylstannyl)pyrazine instead of 2-(tributylstannyl)pyridine as the raw material according to the method described in example 15, with a production of 30 mg and a yield of 35.1%. LC-MS (APCI): m/z = 470.2 $(M+1)^+$. $^1H$ NMR (400 MHz, DMSO-$D_6$) δ (ppm): 12.48 (s, 1H), 9.52 (s, 1H), 9.11 (s, 1H), 8.89 (t, J = 6.0 Hz, 1H), 8.78-8.76 (m, 1H), 8.62 (d, J = 2.4 Hz, 1H), 7.72 (d, J = 8.0 Hz, 1H), 7.54-7.51 (m, 3H), 7.36-7.31 (m, 1H), 7.20-7.17 (m, 1H), 4.86 (br s, 2H), 4.61 (d, J = 6.0 Hz, 2H), 3.90 (s, 3H).

**Example 19 Preparation of N-(4-(3-amino-7-(pyrimidin-4-yl)-1H-pyrazolo[4,3-c]pyrimidin-4-yl)benzyl)-5-fluoro-2-methoxybenzamide**

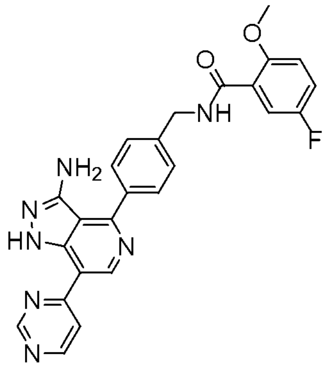

Example 19 was prepared using 4-(tributylstannyl)pyrimidine instead of 2-(tributylstannyl)pyridine as the raw material according to the method described in example 15, with a production of 26 mg and a yield of 30.2%. LC-MS (APCI): m/z = 470.2 $(M+1)^+$. $^1$H NMR (400 MHz, DMSO-$D_6$) δ (ppm): 12.55 (s, 1H), 9.29 (d, J = 1.6 Hz, 1H), 9.14 (s, 1H), 8.92-8.88 (m, 2H), 8.33 (d, J = 5.6 Hz, 1H), 7.72 (d, J = 8.0 Hz, 1H), 7.54-7.51 (m, 3H), 7.36-7.31 (m, 1H), 7.20-7.17 (m, 1H), 4.89 (br s, 2H), 4.62 (d, J = 6.0 Hz, 2H), 3.90 (s, 3H).

**Example 20 Preparation of N-(4-(3-amino-7-(pyridazin-4-yl)-1H-pyrazolo[4,3-c]pyridin-4-yl)benzyl)-5-fluoro-2-methoxybenzamide**

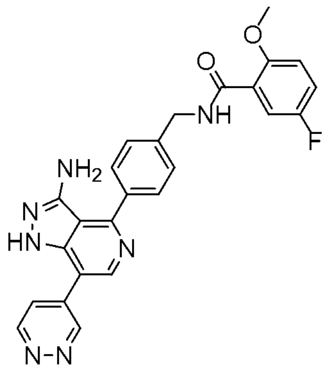

Example 20 was prepared using pyridazine-4-boronic acid pinacol ester instead of pyrimidine-5-boronic acid as the raw material according to the method described in example 16, with a production of 80 mg and a yield of 65.2%. LC-MS (APCI): m/z = 470 $(M+1)^+$. $^1$H NMR (400 MHz, DMSO-$D_6$) δ (ppm): 12.68 (s, 1H), 8.95 (s, 1H), 9.35 (d, J = 5.6 Hz, 1H), 8.91 (t, J = 6.0 Hz, 1H), 8.58 (s, 1H), 8.07-8.05 (m, 1H), 7.71 (d, J = 8.0 Hz, 2H), 7.55-7.52 (m, 3H), 7.37-7.32 (m, 1H), 7.21-7.18 (m, 1H), 4.88 (br s, 2H), 4.62 (d, J = 6.0 Hz, 2H), 3.91 (s, 3H).

**Example 21 Preparation of N-(4-(3-amino-7-(5-fluoropyridin-2-yl)-1H-pyrazolo[4,3-c]pyridin-4-yl)benzyl)-5-fluoro-2-methoxybenzamide**

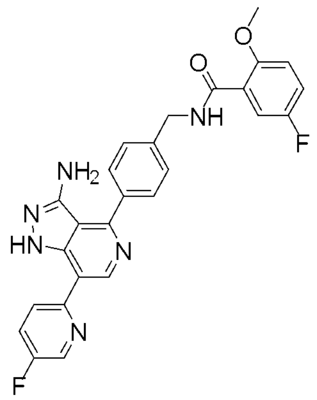

Example 21 was prepared using 5-fluoro-2-(tributylstannyl)pyridine instead of 2-(tributylstannyl)pyridine as the raw material according to the method described in example 15, with a production of 60 mg and a yield of 39.1%. LC-MS (APCI): m/z = 487 $(M+1)^+$. $^1H$ NMR (400 MHz, DMSO-$D_6$) δ (ppm): 12.44 (s, 1H), 8.96 (s, 1H), 8.91 (t, J = 6.0 Hz, 1H), 8.70 (d, J = 2.8 Hz, 1H), 8.35-8.32 (m, 1H), 7.71 (d, J = 8.0 Hz, 2H), 7.55-7.52 (m, 3H), 7.37-7.32 (m, 1H), 7.21-7.18 (m, 1H), 4.84 (br s, 2H), 4.62 (d, J = 6.0 Hz, 2H), 3.91 (s, 3H).

**Example 22 Preparation of N-(4-(3-amino-7-(1-methyl-1H-pyrazol-4-yl)-1H-pyrazolo[4,3-c]pyridin-4-yl)benzyl)-5-fluoro-2-oxybenzamide**

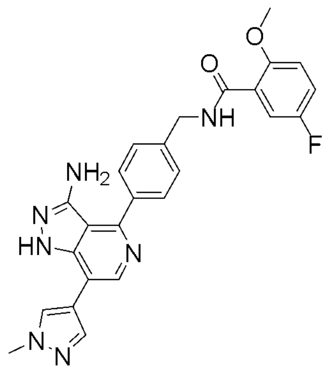

Example 22 was prepared using 1-methylpyrazole-4-boronic acid pinacol ester instead of pyrimidine-5-boronic acid as the raw material according to the method described in example 16, with a production of 70 mg and a yield of 46.0%. LC-MS (APCI): m/z = 472 $(M+1)^+$. $^1H$ NMR (400 MHz, DMSO-$D_6$) δ (ppm): 12.18 (s, 1H), 8.89 (t, J = 6.0 Hz, 1H), 8.49 (s, 1H), 8.38 (s, 1H), 8.09 (s, 1H), 7.65 (d, J = 8.4 Hz, 2H), 7.55-7.49 (m, 3H), 7.37-7.32 (m, 1H), 7.21-7.18 (m, 1H), 4.76 (br s, 2H), 4.60 (d, J = 6.0 Hz, 2H), 3.94 (s, 3H), 3.91 (s, 3H).

**Example 23 Preparation of N-(4-(3-amino-7-(1-methyl-1H-pyrazol-3-yl)-1H-pyrazolo[4,3-c]pyridin-4-yl)benzyl)-5-fluoro-2-methoxybenzamide**

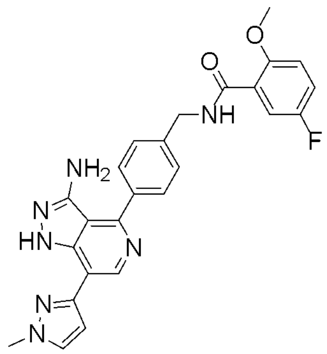

Example 23 was prepared using 1-methylpyrazole-3-boronic acid pinacol ester instead of pyrimidine-5-boronic acid as the raw material according to the method described in example 16, with a production of 50 mg and a yield of 35.2%. LC-MS (APCI): m/z = 472 $(M+1)^+$. $^1$H NMR (400 MHz, DMSO-$D_6$) δ (ppm): 11.88 (s, 1H), 8.90 (t, J=6.0 Hz, 1H), 8.70 (s, 1H), 7.84 (d, J = 2.4 Hz, 1H), 7.71 (d, J = 8.0 Hz, 2H), 7.55-7.52 (m, 3H), 7.37-7.32 (m, 1H), 7.21-7.18 (m, 1H), 6.96 (d, J = 2.4 Hz, 1H), 4.79 (br s, 2H), 4.61 (d, J = 6.0 Hz, 2H), 4.00 (s, 3H), 3.91 (s, 3H).

**Example 24 Preparation of N-(4-(3-amino-7-(5-(trifluoromethyl)pyridin-2-yl)-1H-pyrazolo[4,3-c]pyridin-4-yl)benzyl)-5-fluoro-2-methoxybenzamide**

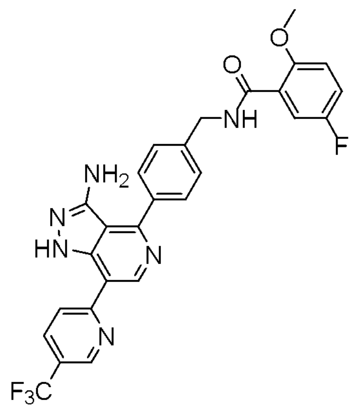

Example 24 was prepared using 2-(tributylstannyl)-5-(trifluoromethyl)pyridine instead of 2-(tributylstannyl)pyridine as the raw material according to the method described in example 15, with a production of 56 mg and a yield of 33.2%. LC-MS (APCI): m/z = 537 $(M+1)^+$. $^1$H NMR (400 MHz, DMSO-$D_6$) δ (ppm): 11.58 (s, 1H), 9.12 (s, 1H), 9.06 (s, 1H), 8.91 (t, J = 6.0 Hz, 1H), 8.49 (d, J = 8.4 Hz, 1H), 8.34 (dd, J = 8.4 Hz, J = 2.0 Hz, 1H), 7.73 (d, J = 8.0 Hz, 2H), 7.55-7.52 (m, 3H), 7.37-7.32 (m, 1H), 7.21-7.18 (m, 1H), 4.88 (br s, 2H), 4.63 (d, J = 6.0 Hz, 2H), 3.91 (s, 3H).

**Example 25 Preparation of N-(4-(3-amino-7-(1-(2-(dimethylamino)-2-oxoethyl)-1H-pyrazol-3-yl)-1H-pyrazolo[4,3-c]pyridin-4-yl)benzyl)-5-fluoro-2-methoxybenzamide**

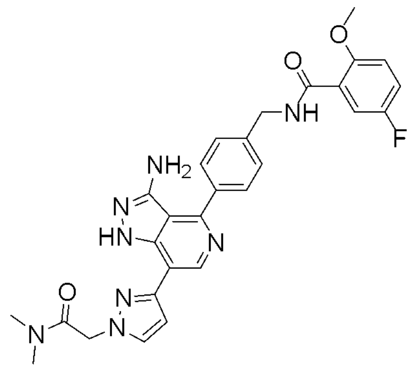

Example 25 was prepared using N,N-dimethyl-2-(3-(4,4,5,5-tetramethyl-1,3,2-dioxaborolan-2-yl)-1H-pyrazol-1-yl)acetamide instead of pyrimidine-5-boronic acid as the raw material according to the method described in example 16, with a production of 50 mg and a yield of 51.6%. LC-MS (APCI): m/z = 543.2 $(M+1)^+$. $^1H$ NMR (400 MHz, DMSO-$D_6$) δ (ppm): 11.85 (s, 1H), 8.88 (t, J = 6.4 Hz, 1H), 8.71 (s, 1H), 7.78 (d, J = 2.0 Hz, 1H), 7.70 (d, J = 12.4 Hz, 2H), 7.54-7.49 (m, 3H), 7.36-7.31 (m, 1H), 7.20-7.17 (m, 1H), 6.98 (d, J = 2.4 Hz, 1H), 5.31 (s, 2H), 4.78 (s, 2H), 4.60 (d, J = 6.0 Hz, 2H), 3.90 (s, 3H), 3.09 (s, 3H), 2.88 (s, 3H).

**Example 26 Preparation of N-(4-(3-amino-7-(1-(2-(methylamino)-2-oxoethyl)-1H-pyrazol-3-yl)-1H-pyrazolo[4,3-c]pyridin-4-yl)benzyl)-5-fluoro-2-methoxybenzamide**

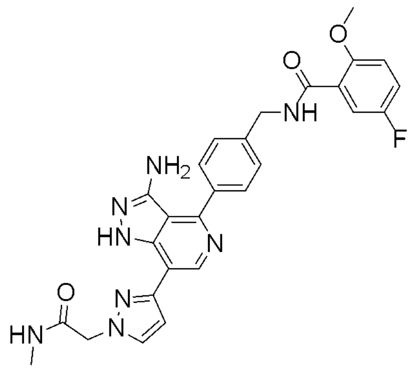

Example 26 was prepared using N-methyl-2-(3-(4,4,5,5-tetramethyl-1,3,2-dioxaborolan-2-yl)-1H-pyrazol-1-yl)acetamide instead of pyrimidine-5-boronic acid as the raw material according to the method described in example 16, with a production of 45 mg and a yield of 46.7%. LC-MS (APCI): m/z = 529.2 $(M+1)^+$. $^1H$ NMR (400 MHz, DMSO-$D_6$) δ (ppm): 11.90 (s, 1H), 8.95 (t, J = 6.4 Hz, 1H), 8.74 (s, 1H), 8.10-8.08 (m, 1H), 7.93 (d, J = 2.4 Hz, 1H), 7.78 (d, J = 8.4 Hz, 2H), 7.64 (d, J = 8.4 Hz, 2H), 7.53-7.50 (m, 1H), 7.38-7.32 (m, 1H), 7.21-7.17 (m, 2H), 5.32-5.30 (m, 2H), 4.99 (s, 2H), 4.64 (d, J = 6.0 Hz, 2H), 3.91 (s, 3H), 2.65 (d, J = 4.8 Hz, 3H).

**Example 27 Preparation of N-(4-(3-amino-7-(1-(2-amino-2-oxoethyl)-1H-pyrazol-3-yl)-1H-pyrazolo[4,3-c]pyridin-4-yl)benzyl)-5-fluoro-2-methoxybenzamide**

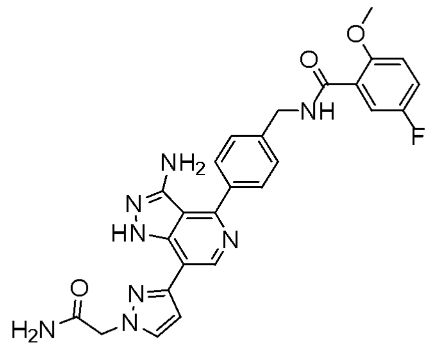

Example 27 was prepared using 2-(3-(4,4,5,5-tetramethyl-1,3,2-dioxaborolan-2-yl)-1H-pyrazole-1-yl)acetamide instead of pyrimidine-5-boronic acid as the raw material according to the method described in example 16, with a production of 40 mg and a yield of 42.7%. LC-MS (APCI): m/z = 515.19 $(M+1)^+$. $^1$H NMR (400 MHz, DMSO-$D_6$) δ (ppm): 11.88 (s, 1H), 8.88 (t, J = 6.4 Hz, 1H), 8.70 (s, 1H), 7.84 (d, J = 2.4 Hz, 1H), 7.67 (d, J = 8.4 Hz, 2H), 7.56-7.48 (m, 4H), 7.36-7.28 (m, 2H), 7.19-7.16 (m, 1H), 6.97 (d, J = 2.0 Hz, 1H), 4.92 (s, 2H), 4.77 (s, 2H), 4.59 (d, J = 6.0 Hz, 2H), 3.89 (s, 3H).

**Example 28 Preparation of N-(4-(3-amino-7-(1-(2,2,2-trifluoroethyl)-1H-pyrazol-3-yl)-1H-pyrazolo[4,3-c]pyridin-4-yl)benzyl)-5-fluoro-2-methoxybenzamide**

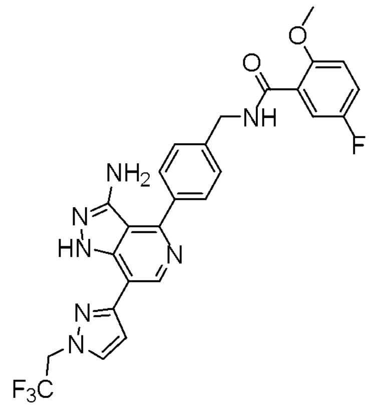

Example 28 was prepared using 1-(2,2,2-trifluoroethyl)-1H-pyrazole-3-boronic acid pinacol ester instead of pyrimidine-5-boronic acid as the raw material according to the method described in example 16, with a production of 55 mg and a yield of 61.5%. LC-MS (APCI): m/z = 540.17 $(M+1)^+$. $^1$H NMR (400 MHz, DMSO-$D_6$) δ (ppm): 11.92 (s, 1H), 8.90 (t, J = 6.4 Hz, 1H), 8.76 (s, 1H), 8.02 (d, J = 2.8 Hz, 1H), 7.70 (d, J = 8.0 Hz, 2H), 7.55-7.52 (m, 3H), 7.36-7.31 (m, 2H), 7.20-7.17 (m, 1H), 7.12 (d, J = 2.8 Hz, 1H), 5.34-5.27 (m, 2H), 4.83 (s, 2H), 4.61 (d, J = 6.0 Hz, 2H), 3.91 (s, 3H).

**Example 29 Preparation of N-(4-(3-amino-7-(1-(difluoromethyl)-1H-pyrazol-3-yl)-1H-pyrazolo[4,3-c]pyridin-4-yl)benzyl)-5-fluoro-2-methoxybenzamide**

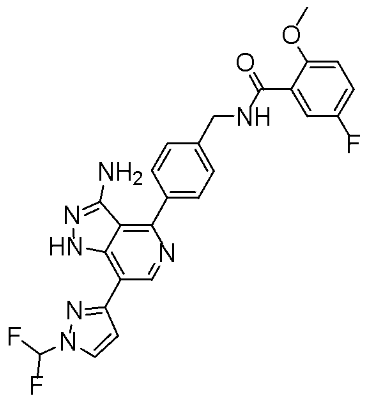

Example 29 was prepared using 1-(difluoromethyl)-1H-pyrazole-3-boronic acid pinacol ester instead of pyrimidine-5-boronic acid as the raw material according to the method described in example 16, with a production of 46 mg and a yield of 52.3%. LC-MS (APCI): m/z = 522.18 $(M+1)^+$. $^1$H NMR (400 MHz, DMSO-$D_6$) δ (ppm): 12.06 (s, 1H), 8.90 (t, J = 6.4 Hz, 1H), 8.83 (s, 1H), 8.45 (d, J = 2.8 Hz, 1H), 7.90 (d, J = 15.0 Hz, 1H), 7.71 (d, J = 8.0 Hz, 2H), 7.55-7.52 (m, 3H), 7.36-7.31 (m, 2H), 7.20-7.17 (m, 1H), 4.86 (s, 2H), 4.62 (d, J = 6.0 Hz, 2H), 3.90 (s, 3H).

## Example 30 Preparation of N-(4-(3-amino-7-(1-methyl-1H-imidazol-4-yl)-1H-pyrazolo[4,3-c]pyridin-4-yl)benzyl)-5-fluoro-2-methoxybenzamide

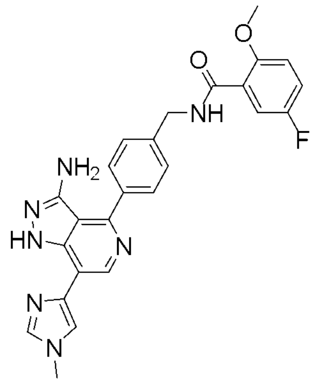

Example 30 was prepared using N-methyl-4-(tributylstannyl)imidazole instead of 2-(tributylstannyl)pyridine as the raw material according to the method described in example 15, with a production of 20 mg and a yield of 24.3%. LC-MS (APCI): m/z = 472.2 $(M+1)^+$. $^1$H NMR (400 MHz, DMSO-$D_6$) δ (ppm): 8.98 (t, J = 6.0 Hz, 1H), 8.51 (br s, 1H), 8.20 (s, 1H), 8.09 (br s, 1H), 7.80(d, J = 8.8 Hz, 2H), 7.65(d, J = 8.8 Hz, 2H), 7.55-7.35(m, 1H), 7.24-7.20(m, 1H), 4.66(d, J = 6.0 Hz, 2H), 3.92(s, 3H).

## Example 31 Preparation of N-(4-(3-amino-7-(1H-pyrazol-3-yl)-1H-pyrazolo[4,3-c]pyridin-4-yl)benzyl)-5-fluoro-2-methoxybenzamide

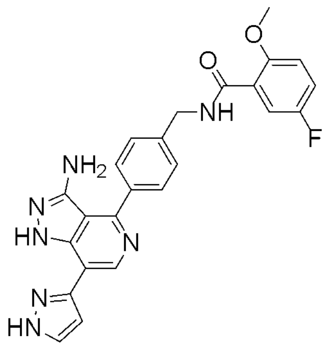

Example 31 was prepared using pyrazole-3-boronic acid instead of pyrimidine-5-boronic acid as the raw material according to the method described in example 16, with a production of 35 mg and a yield of 30.1%. LC-MS (APCI): m/z = 458.2 $(M+1)^+$. $^1H$ NMR (400 MHz, DMSO-$D_6$) δ (ppm): 12.51 (s, 1H), 11.88 (s, 1H), 8.90 (t, J = 6.0 Hz, 1H), 8.70 (s, 1H), 7.84 (d, J = 2.4 Hz, 1H), 7.71 (d, J = 8.0 Hz, 2H), 7.55-7.52 (m, 3H), 7.37-7.32 (m, 1H), 7.21-7.18 (m, 1H), 6.96 (d, J = 2.4 Hz, 1H), 4.79 (br s, 2H), 4.61 (d, J = 6.0 Hz, 2H), 3.91 (s, 3H).

**Example 32 Preparation of N-(4-(3-amino-7-(1-methyl-1H-pyrazol-5-yl)-1H-pyrazolo[4,3-c]pyridin-4-yl)benzyl)-5-fluoro-2-methoxybenzamide**

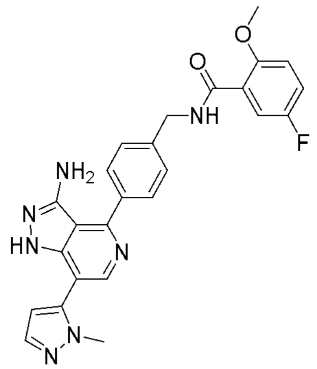

Example 32 was prepared using 1-methylpyrazole-5-boronic acid instead of pyrimidine-5-boronic acid as the raw material according to the method described in example 16, with a production of 40 mg and a yield of 52.6%. LC-MS (APCI): m/z = 472.19 $(M+1)^+$. $^1H$ NMR (400 MHz, DMSO-$D_6$) δ (ppm): 12.06 (s, 1H), 8.89 (t, J = 6.4 Hz, 1H), 8.24 (s, 1H), 7.68 (d, J = 8.0 Hz, 2H), 7.58 (d, J = 2.4 Hz, 1H), 7.53-7.49 (m, 3H), 7.36-7.31 (m, 1H), 7.19-7.17 (m, 1H), 6.53 (d, J = 2.0 Hz, 1H), 4.80 (s, 2H), 4.60 (d, J = 6.0 Hz, 2H), 3.88 (s, 3H), 3.79 (s, 3H).

**Example 33 Preparation of N-(4-(3-amino-7-(pyrazolo[1,5-a]pyridin-3-yl)-1H-pyrazolo[4,3-c]pyridin-4-yl)benzyl)-5-fluoro-2-methoxybenzamide**

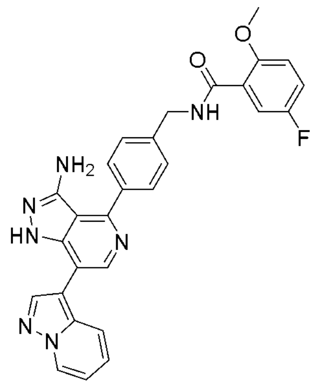

Example 33 was prepared using pyrazolo[1,5-a]pyridine-3-boronic acid pinacol ester instead of pyrimidine-5-boronic acid as the raw material according to the method described in example 16, with a production of 65 mg and a yield of 64.7%. LC-MS (APCI): m/z = 508.19 $(M+1)^+$. $^1H$ NMR (400 MHz, DMSO-$D_6$) δ (ppm): 12.26 (s, 1H), 8.95 (t, J = 6.4 Hz, 1H), 8.85 (d, J = 6.8 Hz, 1H), 8.56-8.50 (m, 1H), 7.78 (d, J = 2.0 Hz, 1H), 7.63 (d, J = 8.0 Hz, 2H), 7.54-7.51 (m, 1H), 7.45-7.41 (m, 1H), 7.36-7.31 (m, 1H), 7.20-7.17 (m, 1H), 7.09-7.06 (t, J = 6.4 Hz, 1H), 4.78 (s, 2H), 4.64 (d, J = 6.0 Hz, 2H), 3.91 (s, 3H).

## Example 34 Preparation of N-(4-(3-amino-7-(pyrazolo[1,5-a]pyrimidin-6-yl)-1H-pyrazolo[4,3-c]pyridin-4-yl)benzyl)-5-fluoro-2-methoxybenzamide

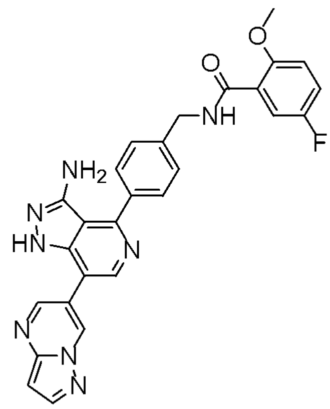

Example 34 was prepared using pyrazolo[1,5-a]pyrimidine-6-boronic acid pinacol ester instead of pyrimidine-5-boronic acid as the raw material according to the method described in example 16, with a production of 58 mg and a yield of 61.5%. LC-MS (APCI): m/z = 509.0 $(M+1)^+$. $^1H$ NMR (400 MHz, DMSO-$D_6$) δ (ppm): 12.61 (s, 1H), 9.45 (s, 1H), 8.93-8.87 (m, 2H), 8.46 (s, 1H), 8.32 (d, J = 3.0 Hz, 1H), 7.73 (d, J = 8.4 Hz, 1H), 7.56-7.52 (m, 3H), 7.38-7.33 (m, 1H), 7.22-7.19 (m, 1H), 6.85 (d, J = 3.0 Hz, 1H), 4.83 (s, 2H), 4.62 (d, J = 6.0 Hz, 2H), 3.92(s, 3H).

## Example 35 Preparation of N-(4-(3-amino-7-(5-(methylsulfonyl)pyridin-2-yl)-1H-pyrazolo[4,3-c]pyridin-4-yl)benzyl)-5-fluoro-2-methoxybenzamide

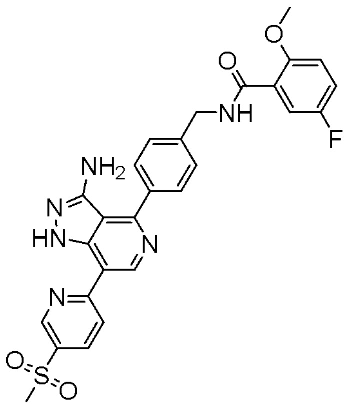

<u>**The following synthetic route was adopted**</u>

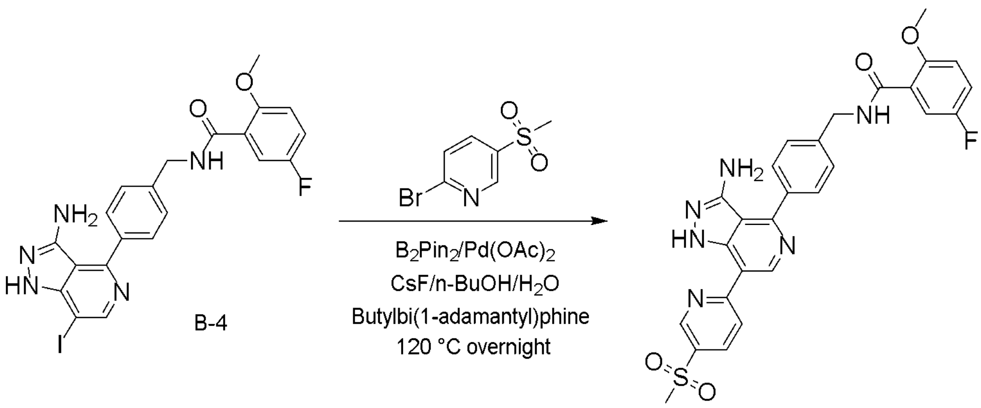


Intermediate B-4 (200 mg, 0.38 mmol), 2-bromo-5-(methylsulfonyl)pyridine (181 mg, 0.77 mmol), bis(pinacolato) diboron (233.7 mg, 0.92 mmol), di(1-adamantyl)-n-butylphosphine (53.7 mg, 0.15 mmol), cesium fluoride (234 mg, 1.54 mmol), palladium(II) acetate (18 mg, 0.08 mmol), n-butanol (10 mL), and water (2 mL) were added to a 25 mL two-necked flask equipped with magnetic stirring. The reaction system was evacuated and purged with nitrogen three times. The mixture was heated to 120 °C, and kept at the same temperature overnight. The mixture was cooled to room temperature, and diluted with added ethyl acetate (30 mL). The insoluble solid was filtered out. The filtrate was concentrated to dryness. The residue was separated by silica gel column chromatography to give 26 mg of a yellow solid , with a yield of 12.2%. LC-MS (APCI): m/z = 547 $(M+1)^+$. $^1H$ NMR (400 MHz, DMSO-$D_6$) δ (ppm): 12.64 (s, 1H), 9.19 (d, J = 2.0 Hz, 1H), 9.15 (s, 1H), 8.89 (t, J = 6.0 Hz, 1H) ,8.55 (d, J = 8.4 Hz, 1H), 8.44 (dd, J = 8.4 Hz, J = 2.0 Hz, 1H), 7.74 (d, J = 8.0 Hz, 2H), 7.55-7.52 (m, 3H), 7.37-7.32 (m, 1H), 7.21-7.18 (m, 1H), 4.88 (br s, 2H), 4.63 (d, J = 6.0 Hz, 2H), 3.91 (s, 3H), 3.34 (s, 3H).

<u>**Example 36 Preparation of N-(4-(3-amino-7-(5-fluoropyrimidin-2-yl)-1H-pyrazolo[4,3-c]pyridin-4-yl)benzyl)-5-fluoro-2-methoxybenzamide**</u>

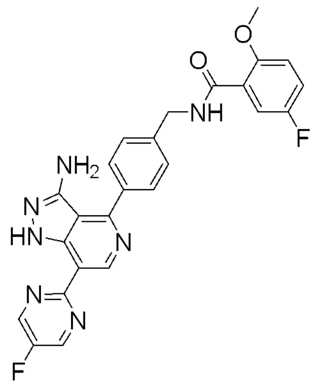

Example 36 was prepared using 2-bromo-5-fluoropyrimidine instead of 2-bromo-5-(methylsulfonyl)pyridine as the raw material according to the method described in example 35, with a production of 20 mg and a yield of 15.6%. LC-MS (APCI): m/z=488.2 $(M+1)^+$. $^1H$ NMR (400 MHz, DMSO-$D_6$) δ (ppm): 12.44 (s, 1H), 9.23 (s, 1H), 9.02 (s, 2H), 8.89 (t, J = 6.0 Hz, 1H), 7.74 (d, J = 8.8 Hz, 2H), 7.55-7.52 (m, 3H), 7.37-7.32 (m, 1H), 7.21-7.18 (m, 1H), 4.88 (br s, 2H), 4.63 (d, J = 6.0 Hz, 2H), 3.91 (s, 3H).

**Example 37 Preparation of N-(4-(3-amino-7-(5-methylpyridin-2-yl)-1H-pyrazolo[4,3-c]pyridin-4-yl)benzyl)-5-fluoro-2-methoxybenzamide**

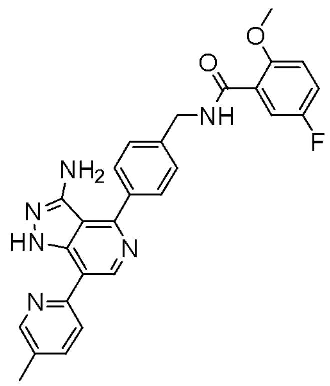

Example 37 was prepared using 2-bromo-5-methylpyridine instead of 2-bromo-5-(methylsulfonyl)pyridine as the raw material according to the method described in example 35, with a production of 25 mg and a yield of 21.1%. LC-MS (APCI): m/z = 483.1 $(M+1)^+$. $^1H$ NMR (400 MHz, DMSO-$D_6$) δ (ppm): 12.38 (s, 1H), 8.95 (s, 1H), 8.89 (t, J = 6.0 Hz, 1H) ,8.61 (s, 1H), 8.14 (d, J = 8.4 Hz, 1H), 7.76 (dd, J = 8.0 Hz, J=2.0 Hz, 1H), 7.71 (d, J = 8.0 Hz, 2H), 7.55-7.52 (m, 3H), 7.37-7.32 (m, 1H), 7.21-7.18 (m, 1H), 4.80 (br s, 2H), 4.62 (d, J = 6.0 Hz, 2H), 3.91 (s, 3H), 2.39 (s, 3H).

**Example 38 Preparation of N-(4-(3-amino-7-(5-chloropyridin-2-yl)-1H-pyrazolo[4,3-c]pyridin-4-yl)benzyl)-5-fluoro-2-methoxybenzamide**

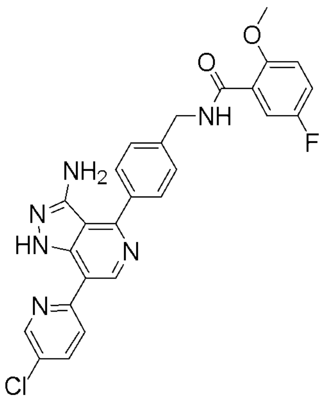

Example 38 was prepared using 5-chloro-2-iodopyridine instead of 2-bromo-5-(methylsulfonyl)pyridine as the raw material according to the method described in example 35, with a production of 55 mg and a yield of 56.8%. LC-MS (APCI): m/z = 503.1 $(M+1)^+$. $^1H$ NMR (400 MHz, DMSO-$D_6$) δ (ppm): 12.43 (s, 1H), 8.98 (s, 1H), 8.87 (t, J = 6.0 Hz, 1H), 8.72 (d, J = 2.4 Hz, 1H), 8.27 (d, J = 8.4 Hz, 1H), 8.08-8.05 (m, 1H), 7.70 (d, J = 8.0 Hz, 2H), 7.53-7.51 (m, 3H), 7.33-7.30 (m, 1H), 7.20-7.17 (m, 1H), 4.82 (s, 2H), 4.60 (d, J = 6.0 Hz, 2H), 3.89 (s, 3H).

## Example 39 Preparation of N-(4-(3-amino-7-(5-methylpyrimidin-2-yl)-1H-pyrazolo[4,3-c]pyridin-4-yl)benzyl)-5-fluoro-2-methoxybenzamide

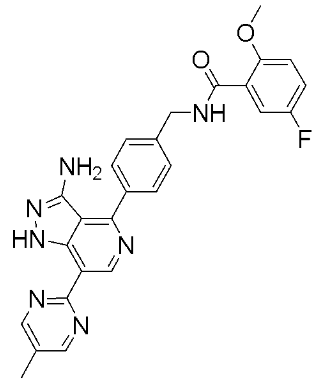

Example 39 was prepared using 2-bromo-5-methylpyrimidine instead of 2-bromo-5-(methylsulfonyl)pyridine as the raw material according to the method described in example 35, with a production of 20 mg and a yield of 18.6%. LC-MS (APCI): m/z = 484.1 $(M+1)^+$. $^1H$ NMR (400 MHz, DMSO-$D_6$) δ (ppm): 12.37 (s, 1H), 9.26 (s, 1H), 8.89 (t, J = 6.0 Hz, 1H), 8.82 (s, 2H), 7.73 (d, J = 8.4 Hz, 2H), 7.55-7.52 (m, 3H), 7.37-7.32 (m, 1H), 7.21-7.18 (m, 1H), 4.86 (br s, 2H), 4.62 (d, J = 6.0 Hz, 2H), 3.91 (s, 3H), 2.37 (s, 3H).

## Example 40 Preparation of N-(4-(3-amino-7-(pyridazin-3-yl)-1H-pyrazolo[4,3-c]pyridin-4-yl)benzyl)-5-fluoro-2-methoxybenzamide

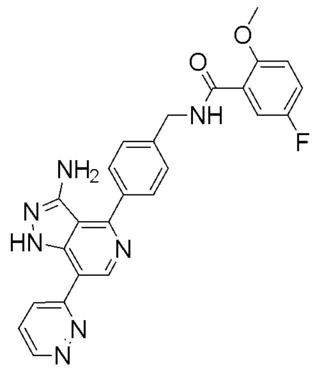

Example 40 was prepared using 3-bromopyridazine instead of 2-bromo-5-(methylsulfonyl)pyridine as the raw material according to the method described in example 35, with a production of 16 mg and a yield of 15.4%. LC-MS (APCI): m/z = 470.2 $(M+1)^+$. $^1$H NMR (400 MHz, DMSO-$D_6$) δ (ppm): 12.42 (s, 1H), 9.26 (d, J = 4.8 Hz, 1H), 9.02 (s, 1H), 8.91 (t, J = 6.0 Hz, 1H), 8.53 (d, J = 8.0 Hz, 1H), 7.88-7.85 (m, 1H), 7.74 (d, J = 8.0 Hz, 2H), 7.55-7.52 (m, 3H), 7.37-7.32 (m, 1H), 7.22-7.18 (m, 1H), 4.88 (br s, 2H), 4.62 (d, J = 6.0 Hz, 2H), 3.91 (s, 3H).

**Example 41 Preparation of N-(4-(3-amino-7-(5-(trifluoromethoxy)pyridin-2-yl)-1H-pyrazolo[4,3-c]pyridin-4-yl) benzyl)-5-fluoro-2-methoxybenzamide**

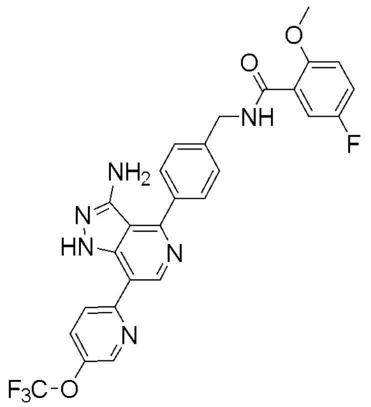

Example 41 was prepared using 2-bromo-5-(trifluoromethoxy)pyridine instead of 2-bromo-5-(methylsulfonyl) pyridine as the raw material according to the method described in example 35, with a production of 15 mg and a yield of 13.1%. LC-MS (APCI): m/z = 553.1 $(M+1)^+$. $^1$H NMR (400 MHz, DMSO-$D_6$) δ (ppm): 12.49 (s, 1H), 9.02 (s, 1H), 8.90 (t, J = 6.0 Hz, 1H), 8.79 (d, J = 2.8 Hz, 1H), 8.41 (d, J = 8.8 Hz, 1H), 8.08-8.05 (m, 1H), 7.72 (d, J = 8.4 Hz, 2H), 7.55-7.52 (m, 3H), 7.37-7.32 (m, 1H), 7.22-7.18 (m, 1H), 4.85 (br s, 2H), 4.62 (d, J = 6.0 Hz, 2H), 3.91 (s, 3H).

**Example 42 Preparation of N-(4-(3-amino-7-(5-(trifluoromethyl)pyrimidin-2-yl)-1H-pyrazolo[4,3-c]pyridin-4-yl) benzyl)-5-fluoro-2-methoxybenzamide**

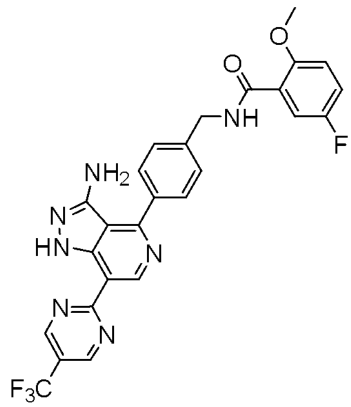

Example 42 was prepared using 2-bromo-5-(trifluoromethyl)pyrimidine instead of 2-bromo-5-(methylsulfonyl) pyridine as the raw material according to the method described in example 35, with a production of 18 mg and a yield of 20.4%. LC-MS (APCI): m/z = 538.1 $(M+1)^+$. $^1H$ NMR (400 MHz, DMSO-$D_6$) δ (ppm): 12.66 (s, 1H), 9.47 (s, 1H), 9.43 (s, 2H), 9.02 (t, J = 6.0 Hz, 1H), 7.86 (d, J = 8.4 Hz, 2H), 7.66-7.62 (m, 3H), 7.48-7.43 (m, 1H), 7.32-7.29 (m, 1H), 5.06 (br s, 2H), 4.73 (d, J = 6.0 Hz, 2H), 4.02 (s, 3H).

## Example 43 Preparation of N-(4-(3-amino-7-(5-(oxetan-3-yl)pyridin-2-yl)-1H-pyrazolo[4,3-c]pyridin-4-yl)benzyl)-5-fluoro-2-methoxybenzamide

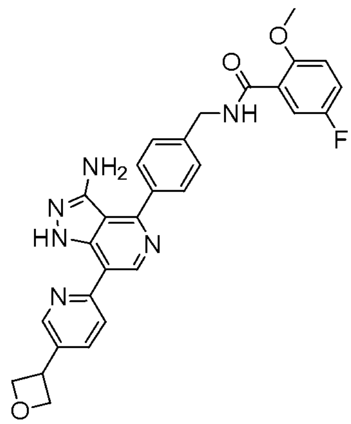

Example 43 was prepared using intermediate C-1 instead of 2-bromo-5-(methylsulfonyl)pyridine as the raw material according to the method described in example 35, with a production of 15 mg and a yield of 16.2%. LC-MS (APCI): m/z = 525.1 $(M+1)^+$. $^1H$ NMR (400 MHz, DMSO-$D_6$) δ (ppm): 12.46 (s, 1H), 9.04 (s, 1H), 8.95 (t, J = 6.0 Hz, 1H), 8.78 (d, J = 2.0 Hz, 1H), 8.32 (d, J = 8.8 Hz, 1H), 8.12 (dd, J = 8.8 Hz, J = 2.0 Hz, 1H), 7.75 (d, J = 8.4 Hz, 2H), 7.59-7.55 (m, 3H), 7.41-7.36 (m, 1H), 7.25-7.22 (m, 1H), 5.07-5.03 (m, 2H), 4.86 (br s, 2H), 4.74 (t, J = 6.0 Hz, 2H), 4.65 (d, J = 6.0 Hz, 2H), 4.45-4.43 (m, 1H), 3.95 (s, 3H).

## Example 44 Preparation of N-(4-(3-amino-7-(pyrazolo[1,5-a]pyrimidin-2-yl)-1H-pyrazolo[4,3-c]pyridin-4-yl)benzyl)-5-fluoro-2-methoxybenzamide

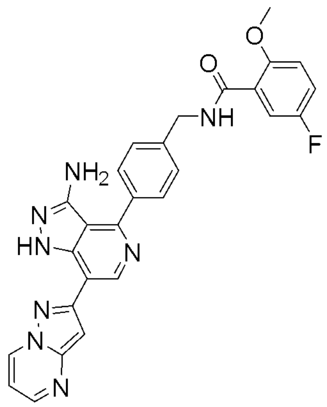

Example 44 was prepared using intermediate C-2 instead of 2-bromo-5-(methylsulfonyl)pyridine as the raw material according to the method described in example 35, with a production of 10 mg and a yield of 10.9%. LC-MS (APCI): m/z = 509.2 $(M+1)^+$. $^1H$ NMR (400 MHz, DMSO-$D_6$) δ (ppm): 12.26 (s, 1H), 9.11 (d, J = 6.8 Hz, 1H), 8.98 (s, 1H), 8.91 (t, J = 6.0 Hz, 1H), 8.62-8.60 (m, 1H), 7.72 (d, J = 8.4 Hz, 2H), 7.55-7.48 (m, 4H), 7.35-7.33 (m, 1H), 7.21-7.18 (m, 1H), 7.13-7.11 (m, 1H), 4.86 (br s, 2H), 4.62 (d, J = 6.0 Hz, 2H), 3.91 (s, 3H).

**Example 45 Preparation of ethyl 2-(3-amino-4-(4-((5-fluoro-2-methoxybenzoylamino)methyl)phenyl)-1H-pyrazolo[4,3-c]pyridin-7-yl)pyrazolo[1,5-a]pyrimidine-6-carboxylate**

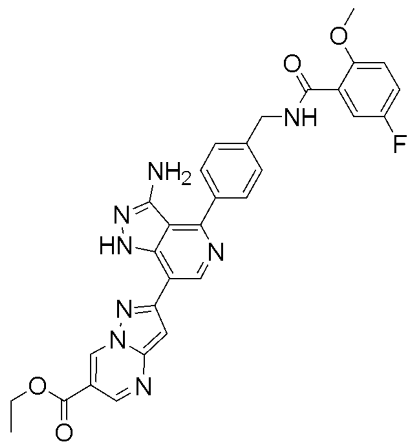

Example 45 was prepared using intermediate C-3 instead of 2-bromo-5-(methylsulfonyl)pyridine as the raw material according to the method described in example 35, with a production of 12 mg and a yield of 11.6%. LC-MS (APCI): m/z = 581.1 $(M+1)^+$. $^1H$ NMR (400 MHz, DMSO-$D_6$) δ (ppm): 12.37 (s, 1H), 9.62 (d, J = 1.6 Hz, 1H), 9.09 (s, 1H), 8.98 (d, J = 1.6 Hz, 1H), 8.95 (t, J = 6.0 Hz, 1H), 7.77 (d, J = 8.4 Hz, 2H), 7.69(s, 1H), 7.59-7.57 (m, 3H), 7.39-7.36 (m, 1H), 7.25-7.22 (m, 1H), 4.95 (br s, 2H), 4.66 (d, J = 6.0 Hz, 2H), 4.45 (q, J = 6.8 Hz, 2H), 3.95 (s, 3H), 1.43 (t, J = 6.8 Hz, 3H).

**Example 46 Preparation of N-(4-(3-amino-7-(pyrazolo[1,5-a]pyrimidin-5-yl)-1H-pyrazolo[4,3-c]pyridin-4-yl)benzyl)-5-fluoro-2-methoxybenzamide**

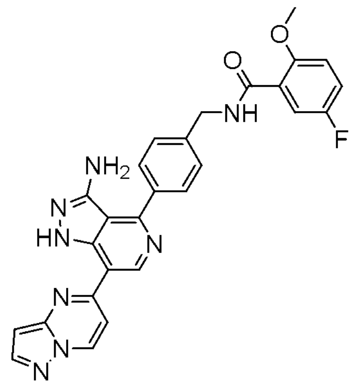

Example 46 was prepared using 5-bromopyrazolo[1,5-a]pyrimidine instead of 2-bromo-5-(methylsulfonyl) pyridine as the raw material according to the method described in example 35, with a production of 10 mg and a yield of 12.5%. LC-MS (APCI): m/z = 509.1 $(M+1)^+$. $^1$H NMR (400 MHz, DMSO-$D_6$) δ (ppm): 12.64 (s, 1H), 9.18-9.16 (m, 1H), 8.89 (t, J = 6.0 Hz, 1H), 8.21 (d, J = 2.0 Hz, 1H), 7.89 (d, J = 8.0 Hz, 1H), 7.72 (d, J = 8.4 Hz, 2H), 7.53-7.50 (m, 3H), 7.35-7.33 (m, 1H), 7.19-7.16 (m, 1H), 7.13-7.11 (m, 1H), 4.90 (br s, 2H), 4.61 (d, J = 6.0 Hz, 2H), 3.89 (s, 3H).

**Example 47 Preparation of N-(4-(3-amino-7-(1H-pyrazolo[3,4-b]pyridin-6-yl)-1H-pyrazolo[4,3-c]pyridin-4-yl) benzyl)-5-fluoro-2-methoxybenzamide**

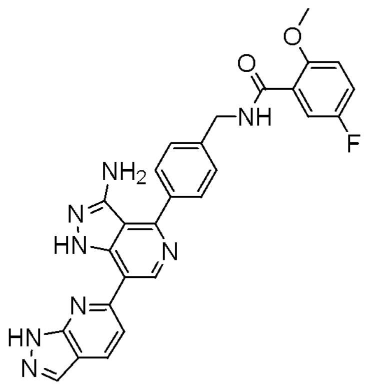

Example 47 was prepared using 6-bromo-1H-pyrazolo[3,4-b]pyridine instead of 2-bromo-5-(methylsulfonyl) pyridine as the raw material according to the method described in example 35, with a production of 8 mg and a yield of 10.1%. LC-MS (APCI): m/z = 509.1 $(M+1)^+$. $^1$H NMR (400 MHz, DMSO-$D_6$) δ (ppm): 13.19 (s, 1H), 12.44 (s, 1H), 9.08 (s, 1H), 8.89 (t, J = 6.0 Hz, 1H), 8.35 (d, J = 8.8 Hz, 1H), 8.17 (d, J = 1.6 Hz, 1H), 8.05 (d, J = 8.4 Hz, 1H), 7.72 (d, J = 8.0 Hz, 2H), 7.53-7.50 (m, 3H), 7.35-7.33 (m, 1H), 7.19-7.16 (m, 1H), 4.88 (br s, 2H), 4.61 (d, J = 6.0 Hz, 2H), 3.89 (s, 3H).

**Example 48 Preparation of N-(4-(3-amino-7-(5-(difluoromethyl)pyridin-2-yl)-1H-pyrazolo[4,3-c]pyridin-4-yl)benzyl)-5-fluoro-2-methoxybenzamide**

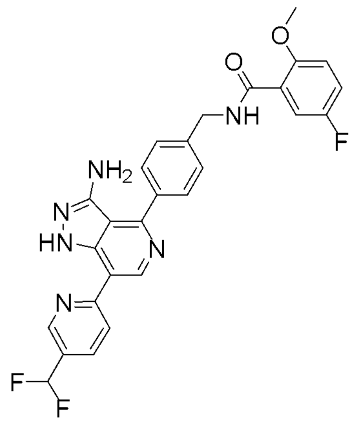

Example 48 was prepared using 2-bromo-5-(difluoromethyl)pyridine instead of 2-bromo-5-(methylsulfonyl) pyridine as the raw material according to the method described in example 35, with a production of 20 mg and a yield of 18.7%. LC-MS(APCI): m/z = 519.1 $(M+1)^+$. $^1H$ NMR (400 MHz, DMSO-$D_6$) δ (ppm): 12.53 (s, 1H), 9.04 (s, 1H), 8.92 (s, 1H), 8.89 (t, J = 6.0 Hz, 1H), 8.38 (d, J = 8.4 Hz, 1H), 8.12 (d J = 8.0 Hz, 1H), 7.70 (d, J = 8.0 Hz, 2H), 7.53-7.50 (m, 3H), 7.37-7.31 (m, 1H), 7.23-7.10 (m, 2H), 4.84 (br s, 2H), 4.60 (d, J = 6.0 Hz, 2H), 3.89 (s, 3H).

**Example 49 Preparation of N-(4-(3-amino-7-(1,7-naphthyridin-2-yl)-1H-pyrazolo[4,3-c]pyridin-4-yl)benzyl)-5-fluoro-2-methoxybenzamide**

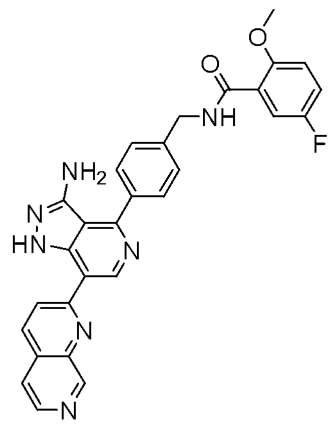

Example 49 was prepared using 2-bromo-1,7-naphthyridine instead of 2-bromo-5-(methylsulfonyl)pyridine as the raw material according to the method described in example 35, with a production of 15 mg and a yield of 15.3%. LC-MS (APCI): m/z = 519.1 $(M+1)^+$.

**Example 50 Preparation of N-(4-(3-amino-7-(imidazo[1,2-a]pyrazin-6-yl)-1H-pyrazolo[4,3-c]pyridin-4-yl)benzyl)-5-fluoro-2-methoxybenzamide**

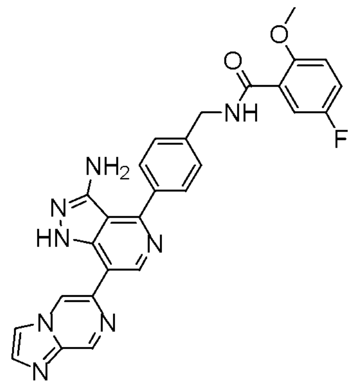

Example 50 was prepared using 6-bromoimidazo[1,2-a]pyrazine instead of 2-bromo-5-(methylsulfonyl)pyridine as the raw material according to the method described in example 35, with a production of 12 mg and a yield of 14.6%. LC-MS (APCI): m/z = 509.1 $(M+1)^+$. $^1H$ NMR (400 MHz, DMSO-$D_6$) δ (ppm): 12.37 (s, 1H), 9.31 (s, 1H), 9.19 (s, 1H), 8.88 (t, J = 6.0 Hz, 1H), 8.79 (s, 1H), 8.17 (s, 1H), 7.89 (s, 1H), 7.69 (d, J = 8.0 Hz, 2H), 7.53-7.50 (m, 3H), 7.37-7.31 (m, 1H), 7.19-7.16 (m, 1H), 4.80 (br s, 2H), 4.60 (d, J = 6.0 Hz, 2H), 3.88 (s, 3H).

**Example 51 Preparation of N-(4-(3-amino-7-(imidazo[1,2-b]pyridazin-6-yl)-1H-pyrazolo[4,3-c]pyridin-4-yl)benzyl)-5-fluoro-2-methoxybenzamide**

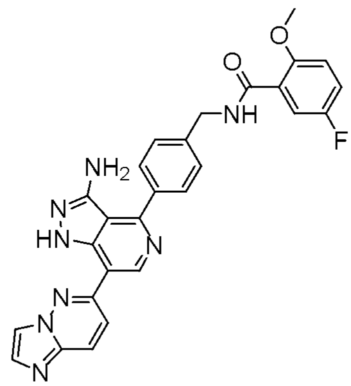

Example 51 was prepared using 6-bromoimidazo[1,2-b]pyridazine instead of 2-bromo-5-(methylsulfonyl)pyridine as the raw material according to the method described in example 35, with a production of 10 mg and a yield of 12.1%. LC-MS (APCI): m/z = 509.1 $(M+1)^+$. $^1H$ NMR (400 MHz, DMSO-$D_6$) δ (ppm): 12.63 (s, 1H), 9.12 (s, 1H), 8.89 (t, J = 6.0 Hz, 1H), 8.61 (s, 1H), 8.22 (d, J = 9.6 Hz, 1H), 8.13 (d, J = 9.6 Hz, 1H), 7.79 (s, 1H), 7.69 (d, J = 8.0 Hz, 2H), 7.53-7.50 (m, 3H), 7.35-7.30 (m, 1H), 7.19-7.16 (m, 1H), 4.90 (br s, 2H), 4.60 (d, J = 6.0 Hz, 2H), 3.88 (s, 3H).

**Example 52 Preparation of N-(4-(3-amino-7-(5-(morpholine-4-carbonyl)pyridin-2-yl)-1H-pyrazolo[4,3-c]pyridin-4-yl)benzyl)-5-fluoro-2-methoxybenzamide**

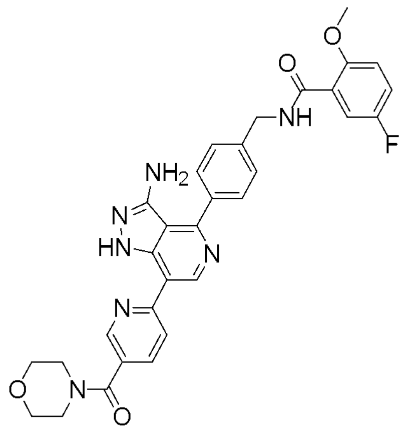

Example 52 was prepared using intermediate C-4 instead of 2-bromo-5-(methylsulfonyl)pyridine as the raw material according to the method described in example 35, with a production of 50 mg and a yield of 29.6%. LC-MS (APCI): m/z = 582.0 $(M+1)^+$. $^1H$ NMR (400 MHz, DMSO-$D_6$) δ (ppm): 12.50 (s, 1H), 9.04 (s, 1H), 8.91 (t, J = 6.0 Hz, 1H), 8.80 (d, J = 1.6 Hz, 1H), 8.32 (d, J = 8.4 Hz, 1H), 8.00 (dd, J = 8.4 Hz, J=2.4 Hz, 1H), 7.73 (d, J = 8.4 Hz, 1H), 7.56-7.52 (m, 3H), 7.35-7.32 (m, 1H), 7.21-7.18 (m, 1H), 4.85 (s, 2H), 4.62 (d, J = 6.0 Hz, 2H), 3.91 (s, 3H), 3.66-3.50 (m, 8H).

**Example** 53 **Preparation** of **N-(4-(3-amino-7-(5-((2S,6S)-2,6-dimethylmorpholine-4-carbonyl)pyridin-2-yl)-1H-pyrazolo[4,3-c]pyridin-4-yl)benzyl)-5-fluoro-2-methoxybenzamide**

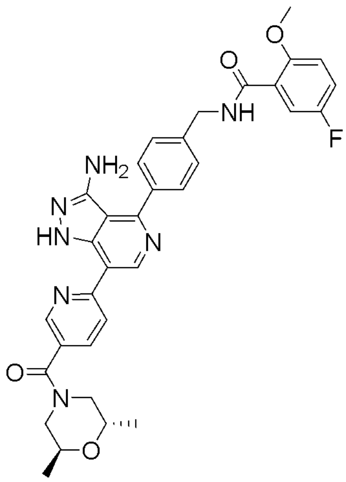

Example 53 was prepared using intermediate C-5 instead of 2-bromo-5-(methylsulfonyl)pyridine as the raw material according to the method described in example 35, with a production of 50 mg and a yield of 28.3%. LC-MS (APCI): m/z = 610.0 $(M+1)^+$.

**Example** 54 **Preparation** of **N-(4-(3-amino-7-(5-((2R,6R)-2,6-dimethylmoroholine-4-carbonyl)pyridin-2-yl)-1H-pyrazolo[4,3-c]pyridin-4-yl)benzyl)-5-fluoro-2-methoxybenzamide**

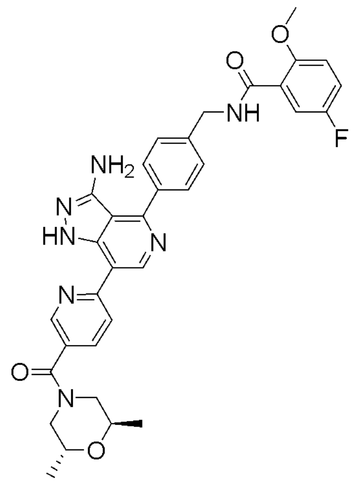

Example 54 was prepared using intermediate C-6 instead of 2-bromo-5-(methylsulfonyl)pyridine as the raw material according to the method described in example 35, with a production of 60 mg and a yield of 34.0%. LC-MS (APCI): m/z = 610.0 $(M+1)^+$. $^1H$ NMR (400 MHz, DMSO-$D_6$) δ (ppm): 12.50 (s, 1H), 9.05 (s, 1H), 8.91 (t, J = 6.0 Hz, 1H), 8.80 (d, J = 1.6 Hz, 1H), 8.32 (d, J = 8.4 Hz, 1H), 8.00 (d, J = 8.0 Hz, 1H), 7.73 (d, J = 8.4 Hz, 1H), 7.56-7.52 (m, 3H), 7.35-7.32 (m, 1H), 7.21-7.18 (m, 1H), 4.85 (s, 2H), 4.62 (d, J = 6.0 Hz, 2H), 4.02-3.95 (m, 1H), 3.91 (s, 3H), 3.71-3.45 (m, 3H), 3.27-3.15 (m, 1H), 1.71-1.10 (m, 6H).

**Example 55 Preparation of N-(4-(3-amino-7-(imidazo[1,2-a]pyridin-3-yl)-1H-pyrazolo[4,3-c]pyridin-4-yl)benzyl)-5-fluoro-2-methoxybenzamide**

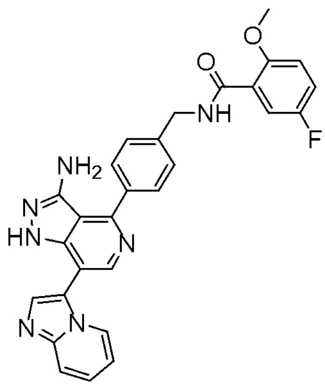

Example 55 was prepared using 3-bromoimidazo[1,2-a]pyridine instead of 2-bromo-5-(methylsulfonyl)pyridine as the raw material according to the method described in example 35, with a production of 15 mg and a yield of 15.2%. LC-MS (APCI): m/z = 508.0 $(M+1)^+$. $^1H$ NMR (400 MHz, DMSO-$D_6$) δ (ppm): 12.32 (s, 1H), 8.93 (t, J = 1.6 Hz, 1H), 8.46 (s, 1H), 8.24 (d, J = 5.2 Hz, 1H), 7.99 (s, 1H), 7.79-7.74 (m, 3H), 7.57-7.53 (m, 3H), 7.46 (t, J = 8.8 Hz, 1H), 7.38-7.33 (m, 1H), 7.22-7.19 (m, 1H), 7.03 (t, J = 6.8 Hz, 1H), 4.90 (br s, 2H), 4.63 (d, J = 6.0 Hz, 2H), 3.92 (s, 3H).

**Example 56 Preparation of N-(4-(3-amino-7-(5-(dimethylphosphoryl)pyridin-2-yl)-1H-pyrazolo[4,3-c]pyridin-4-yl)benzyl)-5-fluoro-2-methoxybenzamide**

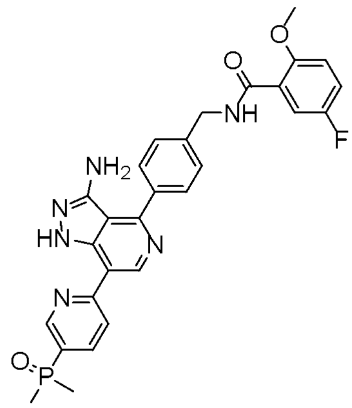

Example 56 was prepared using intermediate C-7 instead of 2-bromo-5-(methylsulfonyl)pyridine as the raw material according to the method described in example 35, with a production of 30 mg and a yield of 18.9%. LC-MS (APCI): m/z = 545.0 $(M+1)^+$. $^1$H NMR (400 MHz, DMSO-$D_6$) δ (ppm): 12.50 (s, 1H), 9.06-9.04 (m, 2H), 8.88 (t, J = 6.0 Hz, 1H), 8.36 (d, J = 8.0 Hz, 1H), 8.27-8.22 (m, 1H), 7.70 (d, J = 8.4 Hz, 1H), 7.53-7.50 (m, 3H), 7.35-7.32 (m, 1H), 7.21-7.18 (m, 1H), 4.83 (s, 2H), 4.60 (d, J = 6.0 Hz, 2H), 3.88 (s, 3H), 1.76 (s, 3H), 1.73 (s, 3H).

## Example 57 Preparation of N-(4-(7-([1,2,4]triazolo[1,5-a]pyrazin-2-yl)-3-amino-1H-pyrazolo[4,3-c]pyridin-4-yl)benzyl)-5-fluoro-2-methoxybenzamide

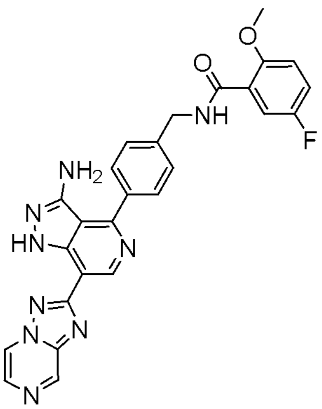

Example 57 was prepared using intermediate C-8 instead of 2-bromo-5-(methylsulfonyl)pyridine as the raw material according to the method described in example 35, with a production of 30 mg and a yield of 20.3%. LC-MS (APCI): m/z = 510.0 $(M+1)^+$. $^1$H NMR (400 MHz, DMSO-$D_6$) δ (ppm): 12.36 (s, 1H), 9.49 (d, J = 1.2 Hz, 1H), 9.15 (s, 1H), 9.11 (dd, J = 4.8 Hz, J = 1.2 Hz, 1H), 8.92 (d, J = 6.0 Hz, 1H), 8.37 (d, J = 4.0 Hz, 1H), 7.75 (d, J = 8.8 Hz, 2H), 7.56-7.52 (m, 3H), 7.38-7.33 (m, 1H), 7.21-7.18 (m, 1H), 4.93 (s, 2H), 4.64 (d, J = 6.0 Hz, 2H), 3.92 (s, 3H).

## Example 58 Preparation of N-(4-(7-([1,2,4]triazolo[1,5-a]pyridin-2-yl)-3-amino-1H-pyrazolo[4,3-c]pyridin-4-yl)benzyl)-5-fluoro-2-methoxybenzamide

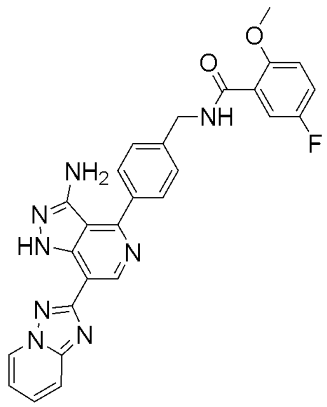

Example 58 was prepared using intermediate C-9 instead of 2-bromo-5-(methylsulfonyl)pyridine as the raw material according to the method described in example 35, with a production of 34 mg and a yield of 23.1%. LC-MS (APCI): m/z = 509.0 $(M+1)^+$. $^1$H NMR (400 MHz, DMSO-$D_6$) δ (ppm): 12.21 (s, 1H), 9.09 (s, 1H), 8.99 (d, J = 7.2 Hz, 1H), 8.91 (t, J = 6.0 Hz, 1H), 7.93 (d, J = 8.4 Hz, 1H), 7.79-7.74 (m, 3H), 7.56-7.52 (m, 3H), 7.37-7.33 (m, 2H), 7.21-7.18 (m, 1H), 4.89 (s, 2H), 4.62 (d, J = 6.0 Hz, 2H), 3.91 (s, 3H).

**Example 59 Preparation of N-(4-(3-amino-7-(5-(2-methoxyethoxy)pyridin-2-yl)-1H-pyrazolo[4,3-c]pyridin-4-yl)benzyl)-5-fluoro-2-methoxybenzamide**

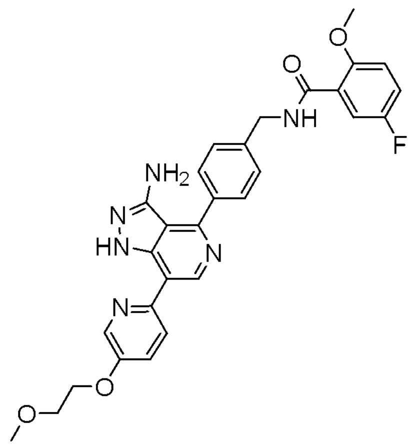

Example 59 was prepared using intermediate C-10 instead of 2-bromo-5-(methylsulfonyl)pyridine as the raw material according to the method described in example 35, with a production of 21 mg and a yield of 13.5%. LC-MS (APCI): m/z = 543.0 $(M+1)^+$. $^1$H NMR (400 MHz, DMSO-$D_6$) δ (ppm): 12.35 (s, 1H), 8.92-8.90 (d, J = 6.0 Hz, 1H), 8.46 (d, J = 2.8 Hz, 1H), 8.19 (d, J = 8.8 Hz, 1H), 7.71 (d, J = 8.0 Hz, 2H), 7.60-7.52 (m, 4H), 7.38-7.33 (m, 1H), 7.21-7.18 (m, 1H), 4.80 (s, 2H), 4.63 (d, J = 6.0 Hz, 2H), 4.29-4.27 (m, 2H), 3.92 (s, 3H), 3.74-3.72 (m, 2H), 3.34 (s, 3H).

**Example 60 Preparation of N-(4-(3-amino-7-(5-(2-(dimethylamino)ethoxy)pyridin-2-yl)-1H-pyrazolo[4,3-c]pyridin-4-yl)benzyl)-5-fluoro-2-methoxybenzamide**

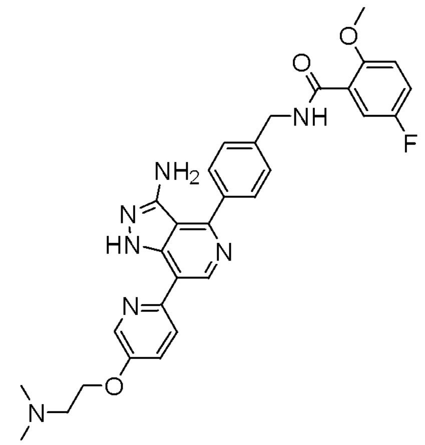

Example 60 was prepared using intermediate C-11 instead of 2-bromo-5-(methylsulfonyl)pyridine as the raw material according to the method described in example 35, with a production of 35 mg and a yield of 21.7%. LC-MS (APCI): m/z = 556.0 $(M+1)^+$. $^1H$ NMR (400 MHz, DMSO-$D_6$) δ (ppm): 12.35 (s, 1H), 8.92-8.90 (d, J = 6.0 Hz, 1H), 8.46 (d, J = 2.8 Hz, 1H), 8.19 (d, J = 8.8 Hz, 1H), 7.71 (d, J = 8.0 Hz, 2H), 7.60-7.52 (m, 4H), 7.38-7.33 (m, 1H), 7.21-7.18 (m, 1H), 4.80 (s, 2H), 4.63 (d, J = 6.0 Hz, 2H), 3.92 (s, 3H), 3.65-3.60 (m, 2H), 3.35-3.30 (m, 2H), 2.85 (s, 6H).

**Example 61 Preparation of N-(4-(3-amino-7-(5-(2-morpholinoethoxy)pyridin-2-yl)-1H-pyrazolo[4,3-c]pyridin-4-yl)benzyl)-5-fluoro-2-methoxybenzamide**

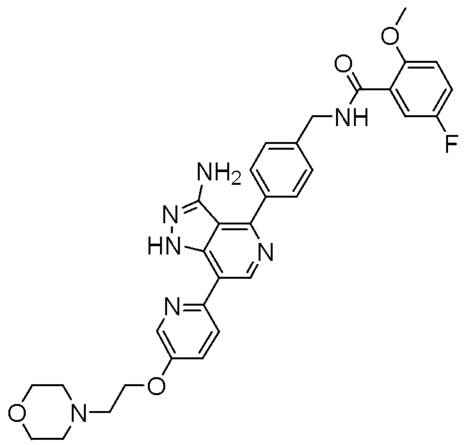

Example 61 was prepared using intermediate C-12 instead of 2-bromo-5-(methylsulfonyl)pyridine as the raw material according to the method described in example 35, with a production of 39 mg and a yield of 22.5%. LC-MS (APCI): m/z = 598.0 $(M+1)^+$. $^1H$ NMR (400 MHz, DMSO-$D_6$) δ (ppm): 12.33 (s, 1H), 8.92-8.88 (m, 2H), 8.46 (d, J = 2.8 Hz, 1H), 8.19 (d, J = 8.8 Hz, 1H), 7.71 (d, J = 8.0 Hz, 2H), 7.60-7.52 (m, 4H), 7.38-7.33 (m, 1H), 7.21-7.18 (m, 1H), 4.79 (s, 2H), 4.62 (d, J = 6.0 Hz, 2H), 4.27 (t, J = 5.6 Hz, 2H), 4.16 (t, J = 6.0 Hz, 1H), 3.92 (s, 3H), 3.62-3.57 (m, 6H), 2.77-2.68 (m, 3H).

**Example 62 Preparation of N-(4-(3-amino-7-(5-(2-(pyrrolidin-1-yl)ethoxy)pyridin-2-yl)-1H-pyrazolo[4,3-c]pyridin-4-yl)benzyl)-5-fluoro-2-methoxybenzamide**

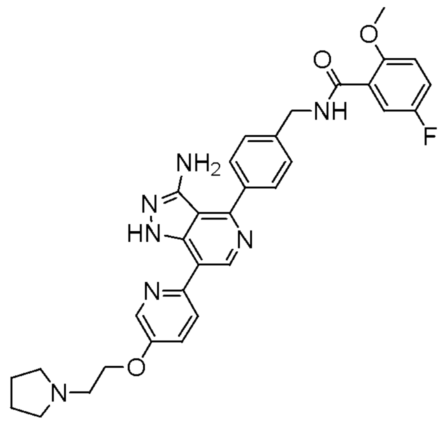

Example 62 was prepared using intermediate C-13 instead of 2-bromo-5-(methylsulfonyl)pyridine as the raw material according to the method described in example 35, with a production of 17 mg and a yield of 14.6%. LC-MS (APCI): m/z = 582.0 $(M+1)^+$. $^1H$ NMR (400 MHz, DMSO-$D_6$) δ (ppm): 12.33 (s, 1H), 8.92-8.88 (m, 2H), 8.46 (d, J = 2.8 Hz, 1H), 8.19 (d, J = 8.8 Hz, 1H), 7.71 (d, J = 8.0 Hz, 2H), 7.60-7.52 (m, 4H), 7.38-7.33 (m, 1H), 7.21-7.18 (m, 1H), 4.79 (s, 2H), 4.62 (d, J = 6.0 Hz, 2H), 3.92 (s, 3H), 3.68-3.65 (m, 4H), 3.19-3.14 (m, 4), 1.77-1.68 (m, 4H).

**Example 63 Preparation of N-(4-(3-amino-7-(imidazo[1,2-a]pyrimidin-3-yl)-1H-pyrazolo[4,3-c]pyridin-4-yl)benzyl)-5-fluoro-2-methoxybenzamide**

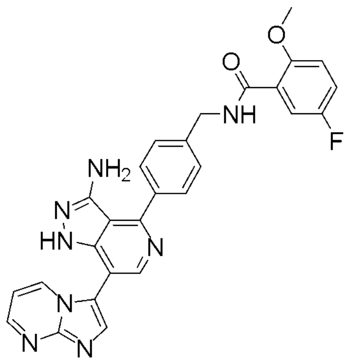

Example 63 was prepared using 3-bromoimidazo[1,2-a]pyrimidine instead of 2-bromo-5-(methylsulfonyl)pyridine as the raw material according to the method described in example 35, with a production of 17 mg and a yield of 11.5%. LC-MS(APCI): m/z = 509.2 $(M+1)^+$. $^1H$ NMR (400 MHz, DMSO-$D_6$) δ (ppm): 12.22 (s, 1H), 8.92 (t, J = 6.0 Hz, 1H), 8.65-8.63 (m, 2H), 8.46 (s, 1H), 8.06 (s, 1H), 7.74 (d, J = 8.8 Hz, 1H), 7.56-7.53 (m, 3H), 7.38-7.33 (m, 1H), 7.22-7.19 (m, 1H), 7.12-7.09 (m, 1H), 4.87 (s, 2H), 4.63 (d, J = 6.0 Hz, 2H), 3.92 (s, 3H).

**Example 64 Preparation of N-(4-(3-amino-7-(imidazo[1,2-a]pyrazin-3-yl)-1H-pyrazolo[4,3-c]pyridin-4-yl)benzyl)-5-fluoro-2-methoxybenzamide**

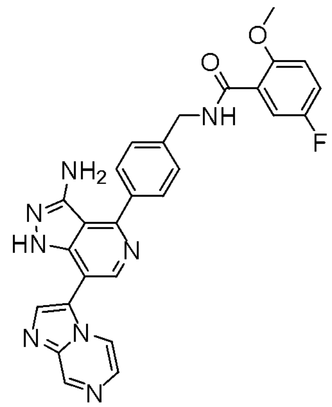

Example 64 was prepared using 3-bromoimidazo[1,2-a]pyrazine instead of 2-bromo-5-(methylsulfonyl)pyridine as the raw material according to the method described in example 35, with a production of 21 mg and a yield of 14.2%. LC-MS (APCI): m/z = 509.2 $(M+1)^+$. $^1$H NMR (400 MHz, DMSO-$D_6$) δ (ppm): 12.26 (s, 1H), 9.22 (s, 1H), 8.92 (t, J = 6.0 Hz, 1H), 8.50 (s, 1H), 8.26 (d, J = 4.8 Hz, 1H), 8.14 (s, 1H), 7.94 (d, J = 4.8 Hz, 1H), 7.74 (d, J = 8.8 Hz, 2H), 7.56-7.53 (m, 3H), 7.38-7.33 (m, 1H), 7.22-7.19 (m, 1H), 4.90 (s, 2H), 4.63 (d, J = 6.0 Hz, 2H), 3.92 (s, 3H).

## Example 65 Preparation of N-(4-(3-amino-7-(5-(2-hydroxypropan-2-yl)pyridin-2-yl)-1H-pyrazolo[4,3-c]pyridin-4-yl)benzyl)-5-fluoro-2-methoxybenzamide

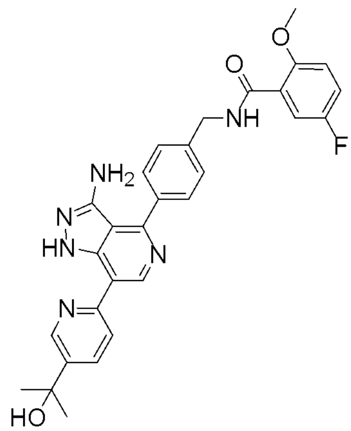

Example 65 was prepared using 2-(6-bromopyridin-3-yl)propan-2-ol instead of 2-bromo-5-(methylsulfonyl) pyridine as the raw material according to the method described in example 35, with a production of 20 mg and a yield of 13.1%. LC-MS (APCI): m/z = 527.2 $(M+1)^+$.

## Example 66 Preparation of N-(4-(3-amino-7-(5-(piperidin-1-ylmethyl)pyridin-2-yl)-1H-pyrazolo[4,3-c]pyridin-4-yl)benzyl)-5-fluoro-2-methoxybenzamide

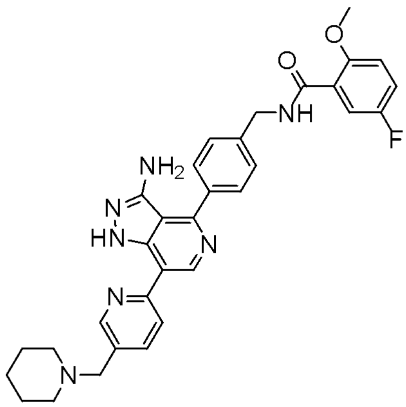

Example 66 was prepared using 2-bromo-5-(piperidin-1-ylmethyl)pyridine instead of 2-bromo-5-(methylsulfonyl)pyridine as the raw material according to the method described in example 35, with a production of 25 mg and a yield of 15.2%. LC-MS (APCI): m/z = 566.2 $(M+1)^+$. $^1H$ NMR (400 MHz, DMSO-$D_6$) δ (ppm): 12.38 (br s, 1H), 8.95 (s, 1H), 8.89 (t, J = 6.0 Hz, 1H), 8.64 (d, J = 1.6 Hz, 1H), 8.18 (d, J = 8.4 Hz, 1H), 7.80 (dd, J = 8.0 Hz, J = 2.0 Hz, 1H), 7.70 (d, J = 8.0 Hz, 2H), 7.53-7.50 (m, 3H), 7.35-7.30 (m, 1H), 7.19-7.16 (m, 1H), 4.79 (br s, 2H), 4.58 (d, J = 6.0 Hz, 2H), 3.88 (s, 3H), 3.50 (s, 2H), 2.36-2.30 (m, 4H), 1.51-1.47 (m, 4H), 1.39-1.31 (m, 2H).

**Example 67 Preparation of N-(4-(3-amino-7-(5-(morpholinomethyl)pyridin-2-yl)-1H-pyrazolo[4,3-c]pyridin-4-yl)benzyl)-5-fluoro-2-methoxybenzamide**

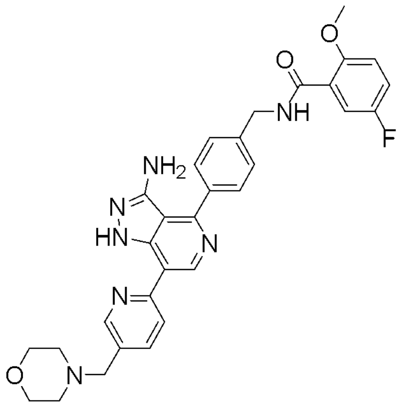

Example 67 was prepared using 4-((6-bromopyridin-3-yl)methyl)morpholine instead of 2-bromo-5-(methylsulfonyl)pyridine as the raw material according to the method described in example 35, with a production of 20 mg and a yield of 12.2%. LC-MS (APCI): m/z = 568.1 $(M+1)^+$. $^1H$ NMR (400 MHz, DMSO-$D_6$) δ (ppm): 12.43 (br s, 1H), 8.98 (s, 1H), 8.90 (t, J = 6.0 Hz, 1H), 8.70 (br s, 1H), 8.22 (d, J = 4.4 Hz, 1H), 7.86 (d, J = 8.6 Hz, 1H), 7.72 (d, J=8.0 Hz, 2H), 7.55-7.52 (m, 3H), 7.37-7.33 (m, 1H), 7.22-7.19 (m, 1H), 4.82 (br s, 2H), 4.64 (d, J = 6.0 Hz, 2H), 3.92 (s, 3H), 3.60-3.58 (m, 6H), 2.44-2.42 (m, 4H).

**Example 68 Preparation of N-(4-(3-amino-7-(5-((4-methylpiperazin-1-yl)methyl)pyridin-2-yl)-1H-pyrazolo[4,3-c]pyridin-4-yl)benzyl)-5-fluoro-2-methoxybenzamide**

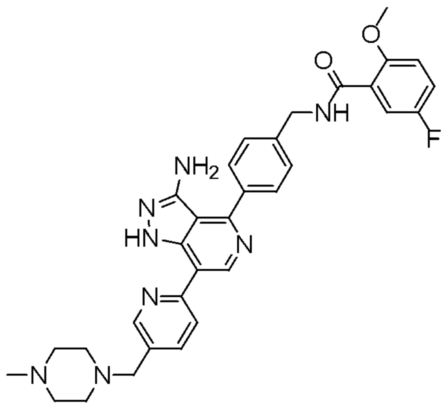

Example 68 was prepared using 1-((6-bromopyridin-3-yl)methyl)-4-methylpiperazine instead of 2-bromo-5-(methylsulfonyl)pyridine as the raw material according to the method described in example 35, with a production of 25 mg and a yield of 14.8%. LC-MS (APCI): m/z = 581.0 $(M+1)^+$. $^1$H NMR (400 MHz, DMSO-$D_6$) δ (ppm): 12.38 (br s, 1H), 8.95 (s, 1H), 8.89 (t, J = 6.0 Hz, 1H), 8.65 (s, 1H), 8.18 (d, J = 8.4 Hz, 1H), 7.80 (d, J = 8.0 Hz, 1H), 7.70 (d, J = 8.0 Hz, 2H), 7.53-7.50 (m, 3H), 7.35-7.30 (m, 1H), 7.19-7.16 (m, 1H), 4.79 (br s, 2H), 4.58 (d, J = 6.0 Hz, 2H), 3.88 (s, 3H), 3.54 (s, 2H), 2.49-2.26 (m, 8H), 2.13 (s, 3H).

**Example 69 Preparation of N-(4-(3-amino-7-(pyrazolo[1,5-a]pyridin-2-yl)-1H-pyrazolo[4,3-c]pyridin-4-yl)benzyl)-5-fluoro-2-methoxybenzamide**

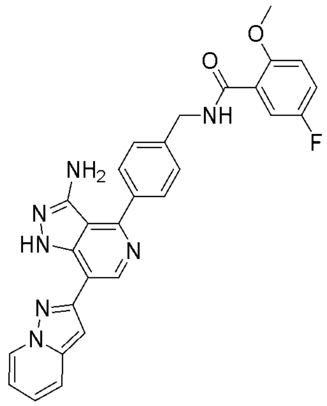

Example 69 was prepared using 2-bromopyrazolo[1,5-a]pyridine instead of 2-bromo-5-(methylsulfonyl)pyridine as the raw material according to the method described in example 35, with a production of 15 mg and a yield of 10.1%. LC-MS (APCI): m/z = 508.2 $(M+1)^+$. $^1$H NMR (400 MHz, DMSO-$D_6$) δ (ppm): 12.22 (s, 1H), 8.91-8.89 (m, 2H), 8.76 (d, J = 6.8 Hz, 1H), 7.77-7.72 (m, 3H), 7.55-7.52 (m, 3H), 7.36-7.31 (m, 3H), 7.20-7.17 (m, 1H), 7.00-6.96 (m, 1H), 4.86 (s, 2H), 4.62 (d, J = 6.0 Hz, 2H), 3.90 (s, 3H).

**Example 70 Preparation of N-(4-(3-amino-7-(pyrazolo[1,5-a]pyrimidin-3-yl)-1H-pyrazolo[4,3-c]pyridin-4-yl)benzyl)-5-fluoro-2-methoxybenzamide**

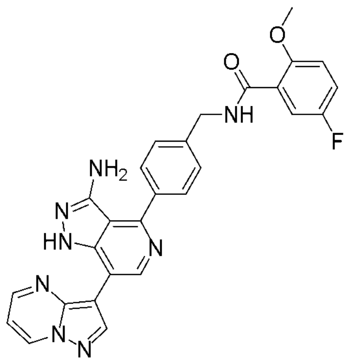

Example 70 was prepared using 3-bromopyrazolo[1,5-a]pyrimidine instead of 2-bromo-5-(methylsulfonyl) pyridine as the raw material according to the method described in example 35, with a production of 45 mg and a yield of 30.4%. LC-MS (APCI): m/z = 509.2 $(M+1)^+$. $^1H$ NMR (400 MHz, DMSO-$D_6$) δ (ppm): 12.42 (s, 1H), 9.26-9.24 (m, 1H), 8.93-8.87 (m, 2H), 8.74-8.72 (m, 1H), 7.68 (d, J = 8.0 Hz, 2H), 7.53-7.49 (m, 2H), 7.36-7.31 (m, 1H), 7.19-7.17 (m, 2H), 6.65 (s, 1H), 4.78 (s, 2H), 4.60 (d, J = 6.0 Hz, 2H), 3.90 (s, 3H).

**Example 71 Preparation of N-(4-(3-amino-7-(5-methyl-4,5,6,7-tetrahydropyrazolo[1,5-a]pyrazin-2-yl)-1H-pyrazolo[4,3-c]pyridin-4-yl)benzyl)-5-fluoro-2-methoxybenzamide**

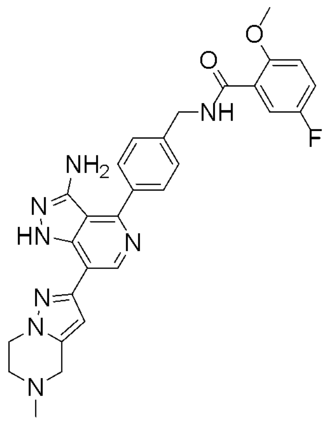

Example 71 was prepared using intermediate C-14 instead of 2-bromo-5-(methylsulfonyl)pyridine as the raw material according to the method described in example 35, with a production of 21 mg and a yield of 13.7%. LC-MS (APCI): m/z = 527.2 $(M+1)^+$. $^1H$ NMR (400 MHz, DMSO-$D_6$) δ (ppm): 12.25 (br s, 1H), 8.97 (t, J = 6.0 Hz, 1H), 8.76 (br s, 1H), 7.79 (d, J = 8.0 Hz, 2H), 7.65 (d, J = 8.0 Hz, 2H), 7.55-7.52 (m, 1H), 7.40-7.35 (m, 1H), 7.24-7.20 (m, 1H), 7.13 (s, 1H), 4.66 (d, J = 6.0 Hz, 2H), 4.52-4.49 (m, 4H), 3.92 (s, 3H), 3.74-3.71 (m, 2H), 2.97 (s, 3H).

**Example 72 Preparation of N-(4-(3-amino-7-(5-ethyl-4,5,6,7-tetrahydropyrazolo[1,5-a]pyrazin-2-yl)-1H-pyrazolo [4,3-c]pyridin-4-yl)benzyl)-5-fluoro-2-methoxybenzamide**

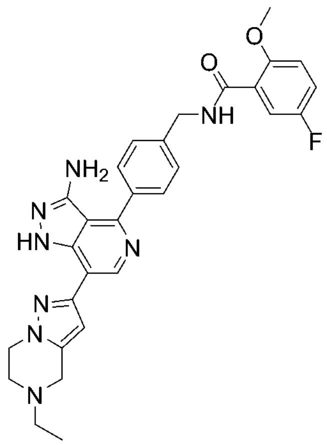

Example 72 was prepared using intermediate C-15 instead of 2-bromo-5-(methylsulfonyl)pyridine as the raw material according to the method described in example 35, with a production of 25 mg and a yield of 15.9%. LC-MS (APCI): m/z = 541.2 $(M+1)^+$. $^1H$ NMR (400 MHz, DMSO-$D_6$) δ (ppm): 11.81 (s, 1H), 8.89 (t, J = 6.0 Hz, 1H), 8.66 (s, 1H), 7.69 (d, J = 8.0 Hz, 2H), 7.55-7.45 (m, 4H), 7.37-7.32 (m, 1H), 7.21-7.18 (m, 1H), 6.73 (s, 1H), 4.78 (br s, 2H), 4.66 (d, J=6.0 Hz, 2H), 4.22 (t, J = 5.6 Hz, 2H), 3.92 (s, 3H), 3.71 (s, 2H), 2.96 (t, J = 6.0 Hz, 2H), 2.60 (q, J = 6.4 Hz, 2H), 1.12 (t, J = 6.4 Hz, 3H).

**Example 73 Preparation of N-(4-(3-amino-7-(5-(4-methylpiperazin-1-yl)pyridin-2-yl)-1H-pyrazolo[4,3-c]pyridin-4-yl)benzyl)-5-fluoro-2-methoxybenzamide**

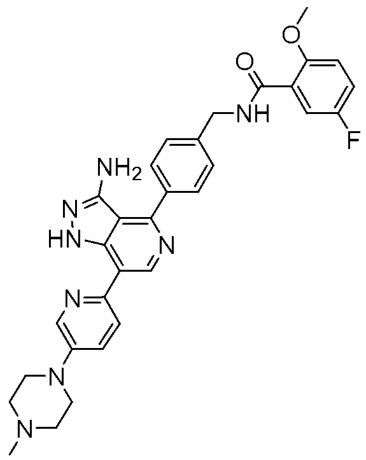

Example 73 was prepared using intermediate C-16 instead of 2-bromo-5-(methylsulfonyl)pyridine as the raw material according to the method described in example 35, with a production of 29 mg and a yield of 13.0%. LC-MS (APCI): m/z = 567.2 $(M+1)^+$. $^1H$ NMR (400 MHz, DMSO-$D_6$) δ (ppm): 12.25 (s, 1H), 8.95 (t, J = 6.0 Hz, 1H), 8.84 (s, 1H), 8.38 (d, J = 2.8 Hz, 1H), 8.06 (d, J = 8.8 Hz, 1H), 7.67 (d, J = 8.0 Hz, 2H), 7.53-7.49 (m, 3H), 7.45-7.42 (m, 1H), 7.37-7.32 (m, 1H), 7.21-7.18 (m, 1H), 4.76 (s, 2H), 4.60 (d, J = 6.0 Hz, 2H), 4.17-4.15 (m, 1H), 3.91 (s, 3H), 3.30-3.24 (m, 8H), 2.85 (s, 3H).

**Example 74 Preparation of (S)-N-(-4-(3-amino-7-(5-(2,4-dimethylpiperazin-1-yl)pyridin-2-yl) 1H-pyrazolo[4,3-c]pyridin-4-yl)benzyl)-5-fluoro-2-methoxybenzamide**

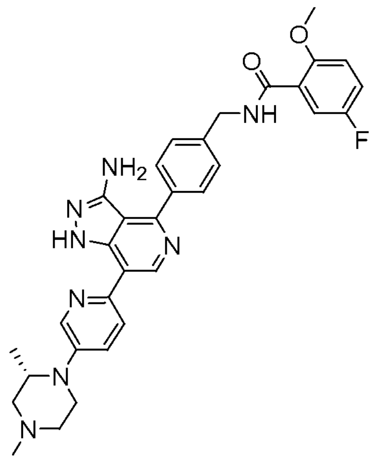

Example 74 was prepared using intermediate C-17 instead of 2-bromo-5-(methylsulfonyl)pyridine as the raw material according to the method described in example 35, with a production of 21 mg and a yield of 11.2%. LC-MS (APCI): m/z = 581.3 $(M+1)^+$. $^1$H NMR (400 MHz, DMSO-$D_6$) δ (ppm): 12.25 (s, 1H), 8.95 (t, J = 6.0 Hz, 1H), 8.84 (s, 1H), 8.38 (d, J = 2.8 Hz, 1H), 8.06 (d, J = 8.8 Hz, 1H), 7.67 (d, J = 8.0 Hz, 2H), 7.53-7.49 (m, 3H), 7.45-7.42 (m, 1H), 7.37-7.32 (m, 1H), 7.21-7.18 (m, 1H), 4.76 (s, 2H), 4.60 (d, J = 6.0 Hz, 2H), 4.17-4.15 (m, 1H), 3.91 (s, 3H), 3.49 (d, J = 16.4 Hz, 1H), 3.10-3.05 (m, 1H), 2.86 (d, J = 10.8 Hz, 1H), 2.70 (d, J = 10.8 Hz, 1H), 2.28-2.24 (m, 1H), 2.22 (s, 3H), 2.10-1.98 (m, 1H), 1.12 (d, J = 6.8 Hz, 3H).

**Example 75 Preparation of (R)-N-(-4-(3-amino-7-(5-(2,4-dimethylpiperazin-1-yl)pyridin-2-yl) 1H-pyrazolo[4,3-c] pyridin-4-yl)benzyl)-5-fluoro-2-methoxybenzamide**

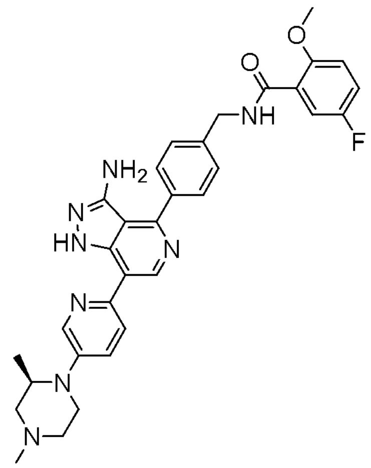

Example 75 was prepared using intermediate C-18 instead of 2-bromo-5-(methylsulfonyl)pyridine as the raw material according to the method described in example 35, with a production of 21 mg and a yield of 11.2%. LC-MS (APCI): m/z = 581.3 $(M+1)^+$. $^1$H NMR (400 MHz, DMSO-$D_6$) δ (ppm): 12.25 (s, 1H), 8.95 (t, J = 6.0 Hz, 1H), 8.84 (s, 1H), 8.38 (d, J = 2.8 Hz, 1H), 8.06 (d, J = 8.8 Hz, 1H), 7.67 (d, J = 8.0 Hz, 2H), 7.53-7.49 (m, 3H), 7.45-7.42 (m, 1H), 7.37-7.32 (m, 1H), 7.21-7.18 (m, 1H), 4.76 (s, 2H), 4.60 (d, J = 6.0 Hz, 2H), 4.17-4.15 (m, 1H), 3.91 (s, 3H), 3.51-3.42 (m, 1H), 3.10-3.05 (m, 1H), 2.88-2.83 (m, 1H), 2.72-2.68 (m, 1H), 2.28-2.24 (m, 1H), 2.22 (s, 3H), 2.10-1.98 (m, 1H), 1.12 (d, J=6.8 Hz, 3H).

**Example 76 Preparation of N-(4-(3-amino-7-(5-morpholinopyridin-2-yl)-1H-pyrazolo[4,3-c]pyridin-4-yl)benzyl)-5-fluoro-2-methoxybenzamide**

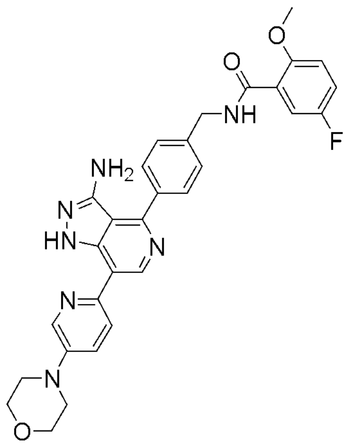

Example 76 was prepared using intermediate C-19 instead of 2-bromo-5-(methylsulfonyl)pyridine as the raw material according to the method described in example 35, with a production of 15 mg and a yield of 13.4%. LC-MS (APCI): m/z = 554.1 $(M+1)^+$. $^1H$ NMR (400 MHz, DMSO-$D_6$) δ (ppm): 12.26 (s, 1H), 8.86 (t, J = 6.0 Hz, 1H), 8.84 (s, 1H), 8.42 (d, J = 2.4 Hz, 1H), 8.07 (d, J = 8.8 Hz, 1H), 7.67 (d, J = 8.0 Hz, 2H), 7.53-7.49 (m, 4H), 7.37-7.32 (m, 1H), 7.21-7.18 (m, 1H), 4.76 (s, 2H), 4.58 (d, J = 6.0 Hz, 2H), 3.89 (s, 3H), 3.77 (t, J = 4.0 Hz, 4H), 3.25 (t, J = 4.0 Hz, 4H).

**Example 77 Preparation of N-(4-(3-amino-7-(pyrrolo[1,2-a]pyrazin-1-yl)-1H-pyrazolo[4,3-c]pyridin-4-yl)benzyl)-5-fluoro-2-methoxybenzamide**

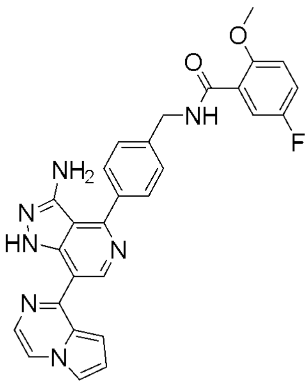

Example 77 was prepared using intermediate C-20 instead of 2-bromo-5-(methylsulfonyl)pyridine as the raw material according to the method described in example 35, with a production of 20 mg and a yield of 15.1%. LC-MS (APCI): m/z = 508.2 $(M+1)^+$. $^1H$ NMR (400 MHz, DMSO-$D_6$) δ (ppm): 12.33 (s, 1H), 9.04 (s, 1H), 8.90 (t, J = 6.0 Hz, 1H), 8.32 (d, J = 4.8 Hz, 1H), 7.89 (d, J = 1.2 Hz, 1H), 7.74 (d, J = 8.4 Hz, 2H), 7.70 (d, J = 4.8 Hz, 1H), 7.53-7.51 (m, 3H), 7.35-7.31 (m, 1H), 7.20-7.17 (m, 1H), 7.00-6.98 (m, 1H), 4.81 (s, 2H), 4.61 (d, J = 6.0 Hz, 2H), 3.90 (s, 3H).

**Example 78 Preparation of N-(4-(3-amino-7-(pyrazolo[1,5-a]pyrazin-2-yl)-1H-pyrazolo[4,3-c]pyridin-4-yl)benzyl)-5-fluoro-2-methoxybenzamide**

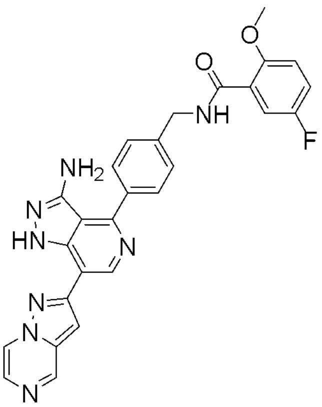

Example 78 was prepared using 2-bromopyrazolo[1,5-a]pyrazine instead of 2-bromo-5-(methylsulfonyl)pyridine as the raw material according to the method described in example 35, with a production of 18 mg and a yield of 13.5%. LC-MS (APCI): m/z = 509.2 $(M+1)^+$. $^1H$ NMR (400 MHz, DMSO-$D_6$) δ (ppm): 9.30 (s, 1H), 9.00 (s, 1H), 8.92 (t, J = 6.4 Hz, 1H), 8.79-8.78 (m, 1H), 8.01 (d, J = 5.2 Hz, 1H), 7.77-7.75 (m, 3H), 7.59-7.67 (m, 2H), 7.54-7.51 (m, 1H), 7.37-7.32 (m, 1H), 7.21-7.18 (m, 1H), 4.63 (d, J = 6.0 Hz, 2H), 3.91 (s, 3H).

**Example 79 Preparation of N-(4-(3-amino-7-(5-(cyclopropaneformamido)pyridin-2-yl)-1H-pyrazolo[4,3-c]pyridin-4-yl)benzyl)-5-fluoro-2-methoxybenzamide**

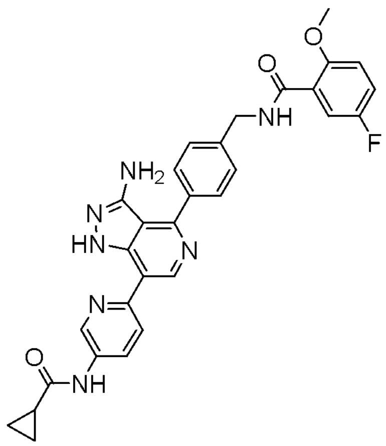

Example 79 was prepared using intermediate C-22 instead of 2-bromo-5-(methylsulfonyl)pyridine as the raw material according to the method described in example 35, with a production of 20 mg and a yield of 12.5%. LC-MS (APCI): m/z = 551.0 $(M+1)^+$. $^1H$ NMR (400 MHz, DMSO-$D_6$) δ (ppm): 12.39 (s, 1H), 10.57 (s, 1H), 8.95 (t, J = 1.6 Hz, 1H), 8.89-8.86 (m, 2H), 8.17 (d, J = 2.0 Hz, 1H), 7.67 (d, J = 8.0 Hz, 1H), 7.53-7.49 (m, 3H), 7.35-7.32 (m, 1H), 7.19-7.16 (m, 1H), 4.77 (s, 2H), 4.60 (d, J = 6.0 Hz, 2H), 3.89 (s, 3H), 1.98-1.96 (m, 1H), 1.82-1.80 (m, 1H), 0.85-0.81 (m, 3H).

**Example 80 Preparation of N-(4-(3-amino-7-(4-(cyclopropaneformamido)pyridin-2-yl)-1H-pyrazolo[4,3-c]pyridin-4-yl)benzyl)-5-fluoro-2-methoxybenzamide**

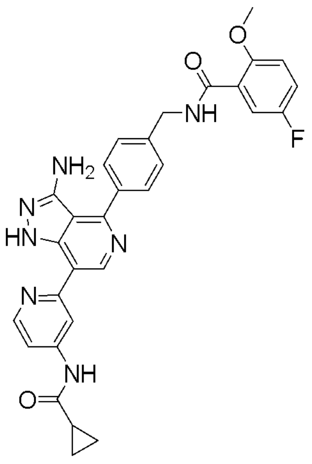

Example 80 was prepared using intermediate C-23 instead of 2-bromo-5-(methylsulfonyl)pyridine as the raw material according to the method described in example 35, with a production of 16 mg and a yield of 10.0%. LC-MS (APCI): m/z = 551.0 $(M+1)^+$. $^1H$ NMR (400 MHz, DMSO-$D_6$) δ (ppm): 12.43 (s, 1H), 10.60 (s, 1H), 8.95 (t, J = 1.6 Hz, 1H), 8.89-8.85 (m, 2H), 8.15 (d, J = 2.0 Hz, 1H), 7.67-7.65 (m, 1H), 7.53-7.49 (m, 3H), 7.35-7.32 (m, 1H), 7.20-7.16 (m, 1H), 4.79 (s, 2H), 4.61 (d, J = 6.0 Hz, 2H), 3.90 (s, 3H), 1.95-1.92 (m, 1H), 1.80-1.77 (m, 1H), 0.90-0.85 (m, 3H).

**Example 81 Preparation of 3-amino-N-cyclopropyl-4-(4-((5-fluoro-2-methoxybenzoylamino)methyl)phenyl)-1H-pyrazolo[4,3-c]pyridine-7-carboxamide**

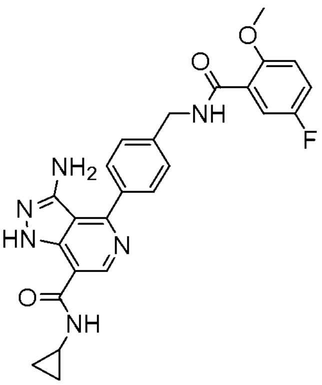

**The following synthetic route was adopted**

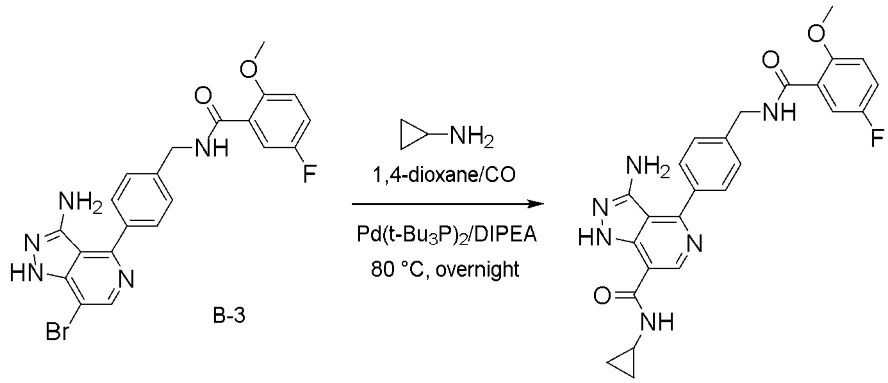


Intermediate B-3 (200 mg, 0.42 mmol), cyclopropylamine (33 mg, 0.63 mmol), bis(tri-tert-butylphosphine) palladium(0) (59 mg, 0.13 mmol), N,N-diisopropylethylamine (50 mg, 0.42 mmol), and 1,4-dioxane (10 mL) were added to a 50 mL two-necked flask equipped with magnetic stirring. The reaction system was evacuated and purged with carbon

monoxide three times. The mixture was heated to 80 °C and kept at the same temperature overnight. The mixture was cooled to room temperature, and diluted with added ethyl acetate (20 mL). The insoluble solid was filtered out. The filtrate was concentrated to dryness. The residue was separated by silica gel column chromatography to give 73 mg of a yellow solid, with a yield of 40%. LC-MS (APCI): m/z = 475 $(M+1)^+$.

**Example** 82 **Preparation** of **N-(4-(3-amino-7-(5-fluoropyridin-2-yl)-1H-pyrazolo[4,3-c]pyridin-4-yl)-3-fluorobenzyl)-5-fluoro-2-methoxybenzamide**

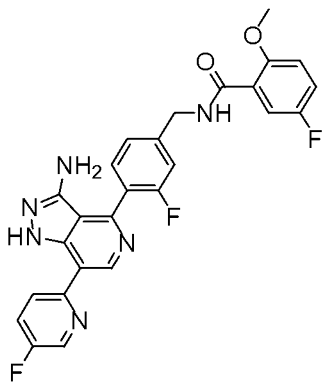

**The following synthetic route was adopted**

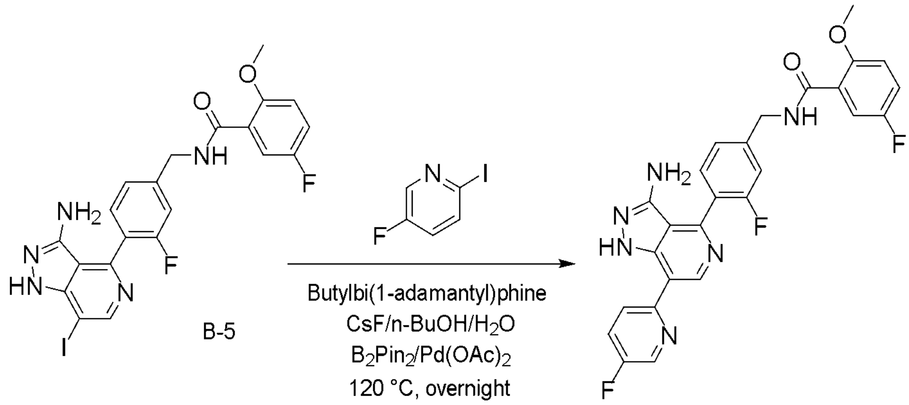


Intermediate B-5 (150 mg, 0.28 mmol), 5-fluoro-2-iodopyridine (187.3 mg, 0.84 mmol), di(1-adamantyl)-n-butylphosphine (40.15 mg, 0.112 mmol), palladium(II) acetate (12.6 mg, 0.056 mmol), bis(pinacolato)diboron (284.4 mg, 1.12 mmol), and cesium fluoride (170.2 mg, 1.12 mmol) were added to a 25 mL three-necked flask equipped with magnetic stirring and a condenser. The reaction system was evacuated and purged with nitrogen three times. n-Butanol (10 mL) and water (2 mL) were added, and the mixture was stirred to disperse. The mixture was stirred and reacted at 120 °C overnight. The mixture was cooled to room temperature, and saturated brine (10 mL) was added to quench the reaction. The organic phase was separated, and the aqueous phase was extracted with ethyl acetate (10 mL $\times$ 2). The organic phases were combined, washed with saturated brine (10 mL), dried over anhydrous sodium sulfate, filtered, concentrated, and separated by silica gel column chromatography to give 75 mg of a white solid, with a yield of 53.1%. LC-MS (APCI): m/z = 505.0 $(M+1)^+$. $^1H$ NMR (400 MHz, DMSO-$D_6$) $\delta$ (ppm): 12.45 (s, 1H), 8.97-8.94 (m, 2H), 8.71 (t, J = 3.2 Hz, 1H), 8.37-8.34 (m, 1H), 7.95-7.90 (m, 1H), 7.56-7.52 (m, 2H), 7.38-7.33 (m, 3H), 7.21-7.18 (m, 1H), 4.66 (s, 2H), 4.63 (d, J = 6.0 Hz, 2H), 3.92 (s, 3H).

**Example 83 Preparation of N-(4-(3-amino-7-(5-fluoropyridin-2-yl)-1H-pyrazolo[4,3-c]pyridin-4-yl)-3-methylbenzyl)-5-fluoro-2-methoxybenzamide**

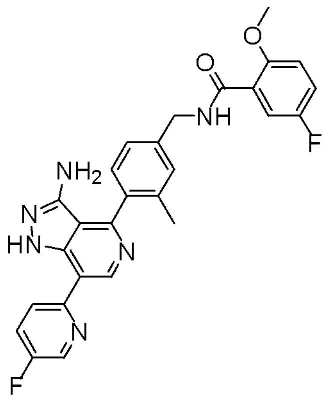

Example 83 was prepared using intermediate B-6 instead of intermediate B-5 as the raw material according to the method described in example 82. 19 mg of a white solid was obtained, with a yield of 13.6%. LC-MS (APCI): m/z = 501.0 $(M+1)^+$. $^1H$ NMR (400 MHz, DMSO-$D_6$) δ (ppm): 12.43 (s, 1H), 8.95 (s, 1H), 8.87 (t, J = 6.0 Hz, 1H), 8.70 (d, J = 2.4 Hz, 1H), 8.36-8.35 (m, 1H), 7.95-7.90 (m, 1H), 7.56-7.52 (m, 1H), 7.37-7.29 (m, 4H), 7.21-7.18 (m, 1H), 4.58 (d, J = 6.0 Hz, 2H), 4.52 (s, 2H), 3.89 (s, 3H), 2.14 (s, 3H).

**Example 84 Preparation of N-(4-(3-amino-6-methyl-7-(pyrimidin-2-yl)-1H-pyrazolo[4,3-c]pyridin-4-yl)benzyl)-5-fluoro-2-methoxybenzamide**

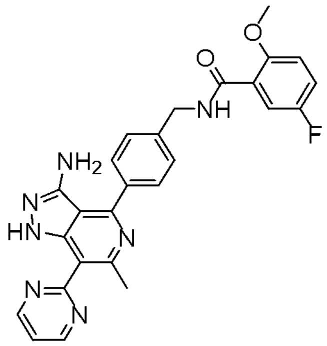

**The following synthetic route was adopted**

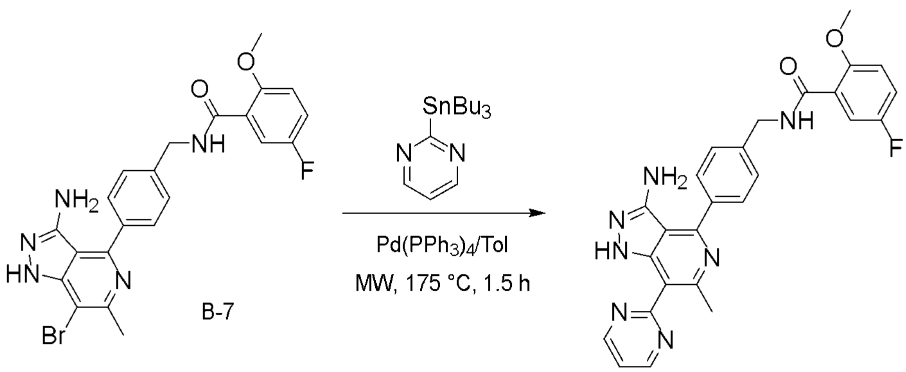


Intermediate B-7 (270 mg, 0.56 mmol), 2-(tributylstannyl)pyrimidine (413 mg, 1.12 mmol), tetrakis(triphenylphosphine)palladium(0) (65 mg, 0.056 mmol), and toluene (5 mL) were added to a 20 mL microwave vial. The reaction solution was purged with nitrogen for 2 minutes. The vial was sealed. The mixture was stirred and reacted at 175 °C under microwave for 1.5 hours. The mixture was cooled to room temperature. Saturated brine (10 mL) was added, and the organic phase was separated. The aqueous phase was extracted with ethyl acetate (10 mL × 2). The organic phases were combined, washed with saturated brine (10 mL), dried over anhydrous sodium sulfate, filtered, concentrated, and

separated by silica gel column chromatography to give 55 mg of a yellow solid, with a yield of 20.3%. LC-MS (APCI): m/z = 484.0 $(M+1)^+$. $^1H$ NMR (400 MHz, DMSO-$D_6$) δ (ppm): 12.07 (s, 1H), 8.93 (d, J = 4.8 Hz, 1H), 8.82 (t, J = 6.0 Hz, 1H), 7.64 (d, J = 8.4 Hz, 1H), 7.48-7.40 (m, 4H), 7.28-7.26 (m, 1H), 7.14-7.11 (m, 1H), 4.68 (s, 2H), 4.54 (d, J = 5.6 Hz, 2H), 3.84 (s, 3H), 2.79 (s, 3H).

**Example 85 Preparation of N-(4-(3-amino-6-(difluoromethyl)-7-(pyrimidin-2-yl)-1H-pyrazolo[4,3-c]pyridin-4-yl)benzyl)-5-fluoro-2-methoxybenzamide**

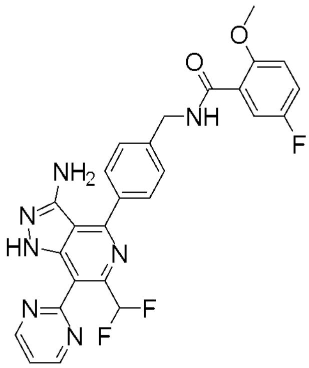

Example 85 was prepared using intermediate B-8 instead of intermediate B-7 as the raw material according to the method described in example 84. 45 mg of a yellow solid was obtained, with a yield of 18.8%. LC-MS (APCI): m/z = 520.0 $(M+1)^+$. $^1H$ NMR (400 MHz, DMSO-$D_6$) δ (ppm): 12.57 (s, 1H), 9.04 (s, 1H), 9.02 (s, 1H), 8.89 (t, J = 6.0 Hz, 1H), 7.83 (t, J = 14.4 Hz, 1H), 7.74 (d, J = 8.4 Hz, 1H), 7.58-7.51 (m, 4H), 7.36-7.31 (m, 1H), 7.20-7.16 (m, 1H), 4.92 (s, 2H), 4.61 (d, J = 5.6 Hz, 2H), 3.90 (s, 3H).

**Example 86 Preparation of N-(4-(3-amino-6-(1,1,1-trifluoropropan-2-yl)-1H-indazol-4-yl)benzyl)-5-fluoro-2-methoxybenzamide**

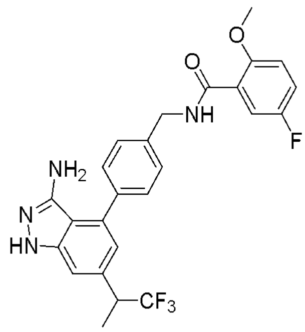

**The following synthetic route was adopted**

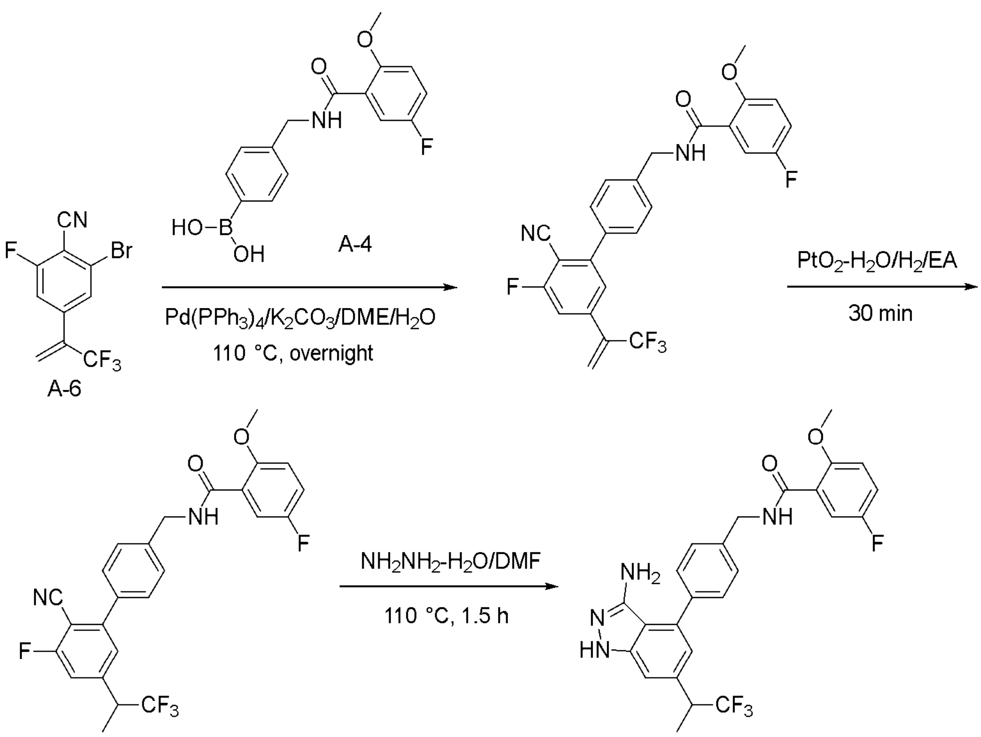


Step 1: Synthesis of compound N-((2'-cyano-3'-fluoro-5'-(3,3,3-trifluoroprop-1-en-2-yl)-[1,1'-biphenyl]-4-yl)methyl)-5-fluoro-2-methoxybenzamide

Intermediate A-6 (2.0 g, 6.8 mmol), intermediate A-4 (2.47 g, 8.16 mmol), dimethoxyethane (30 mL), tetrakis(triphenylphosphine)palladium(0) (785 mg, 0.68 mmol), potassium carbonate (2.82 g, 20.4 mmol), and water (6 mL) were added to a container. The reaction system was purged with nitrogen three times. The mixture was heated to 110 °C and reacted overnight. The reaction solution was cooled to room temperature, and water (100 mL) was added. The mixture was extracted with ethyl acetate (150 mL × 3). The organic phase was washed with saturated brine, dried over anhydrous sodium sulfate, and concentrated. The residue was separated by silica gel column chromatography to give 2.1 g of a brown solid, with a yield of 65%. LC-MS (APCI): m/z = 473.1 $(M+1)^+$. $^1H$ NMR (400 MHz, DMSO-$D_6$) δ (ppm): 8.91 (t, J = 6.4 Hz, 1H), 7.70-7.63 (m, 3H), 7.54-7.51 (m, 4H), 7.37-7.32 (m, 1H), 7.21-7.17 (m, 1H), 6.52 (s, 1H), 6.38 (s, 1H), 4.59 (d, J = 6.0 Hz, 2H), 3.90 (s, 3H).

Step 2: Synthesis of compound N-((2'-cyano-3'-fluoro-5'-(1,1,1-trifluoropropan-2-yl)-[1,1'-biphenyl]-4-yl)methyl)-5-fluoro-2-methoxybenzamide

Ethyl acetate (50 mL), N-((2'-cyano-3'-fluoro-5'-(3,3,3-trifluoroprop-1-en-2-yl)-[1,1'-biphenyl]-4-yl)methyl)-5-fluoro-2-methoxybenzamide (1.0 g, 2.12 mmol) and platinum(IV) oxide hydrate (500 mg) were added to a container. The reaction system was purged with hydrogen three times. The mixture was reacted under hydrogen for 30 minutes. The mixture was filtered, and the filter residue was washed with ethyl acetate. The filtrate was concentrated, and the residue was separated by silica gel column chromatography to give 720 mg of a light yellow solid, with a yield of 72%. LC-MS (APCI): m/z = 475.2 $(M+1)^+$.

Step 3: Synthesis of example 86

N-((2'-Cyano-3'-fluoro-5'-(1,1,1-trifluoropropan-2-yl)-[1,1'-biphenyl]-4-yl)methyl)-5-fluoro-2-methoxybenzamide (400 mg, 0.844 mmol), N,N-dimethylformamide (5 mL), and hydrazine hydrate (210 mg, 4.22 mmol) were added to a container. The reaction mixture was heated to 110 °C and reacted for 1.5 hours. The reaction solution was diluted with added water (50 mL). The mixture was extracted with ethyl acetate (150 mL × 3). The organic phase was washed with saturated brine, dried over anhydrous sodium sulfate, and concentrated. The residue was separated by silica gel column chromatography to give 200 mg of a light yellow solid, with a yield of 48%. LC-MS (APCI): m/z = 487.0 $(M+1)^+$. $^1H$ NMR (400 MHz, $CDCl_3$) δ (ppm): 8.38-8.36 (m, 1H), 7.98 (dd, J = 8.8 Hz, J = 2.8 Hz, 1H), 7.52-7.44 (m, 4H), 7.19-7.14 (m, 1H), 6.97-6.94 (m, 1H), 6.87 (s, 1H), 4.76 (d, J = 5.6 Hz, 2H), 3.95 (s, 3H), 3.56-3.51 (m, 1H), 1.56 (d, J = 6.8 Hz, 3H).

**Example 87 Preparation of N-(4-(3-amino-6-(o-tolyl)-1H-pyrazolo[4,3-c]pyridin-4-yl)benzyl)-5-fluoro-2-methoxybenzamide**

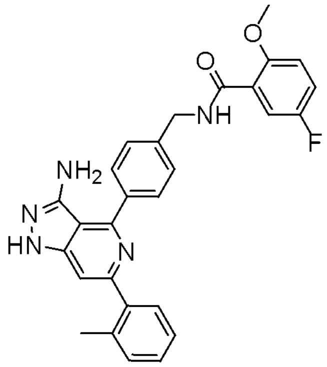

**The following synthetic route was adopted**

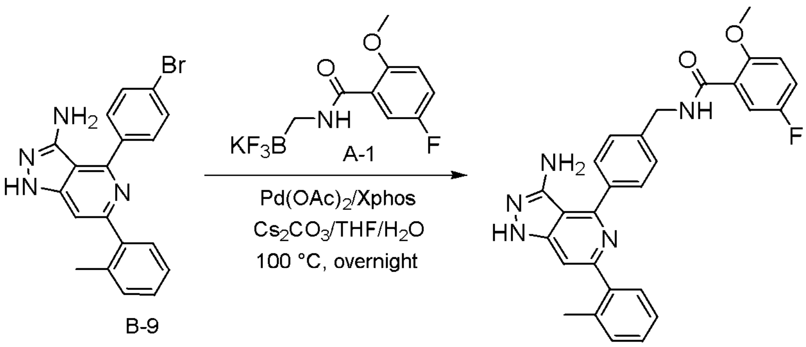


Intermediate B-9 (60 mg, 0.16 mmol), intermediate A-1 (69.4 mg, 0.24 mmol), palladium(II) acetate (4 mg, 0.016 mmol), 2-dicyclohexylphosphino-2',4',6'-triisoprophylbiphenyl (15.3 mg, 0.032 mmol), and cesium carbonate (156.4 mg, 0.48 mmol) were added to a 25 mL three-necked flask equipped with magnetic stirring and a condenser. The reaction system was evacuated and purged with nitrogen three times. Tetrahydrofuran (3 mL) and water (0.7 mL) were added, and the mixture was stirred to disperse. The mixture was stirred and reacted at 100 °C overnight. The mixture was cooled to room temperature, and saturated brine (5 mL) was added to quench the reaction. The organic phase was separated, and the aqueous phase was extracted with ethyl acetate (5 mL × 2). The organic phases were combined, washed with saturated brine (10 mL), dried over anhydrous sodium sulfate, filtered, concentrated, and separated by silica gel column chromatography to give 50 mg of a white solid, with a yield of 64.9%. LC-MS (APCI): m/z = 482.0 $(M+1)^+$. $^1H$ NMR (400 MHz, DMSO-$D_6$) δ (ppm): 12.17 (s, 1H), 8.86 (t, J=6.0 Hz, 1H), 7.69-7.67 (m, 2H), 7.52-7.47 (m, 3H), 7.45-7.43 (m, 1H), 7.36-7.24 (m, 4H), 7.23 (s, 1H), 7.18-7.15 (m, 1H), 4.73 (s, 2H), 4.58 (d, J = 6.0 Hz, 2H), 3.86 (s, 3H), 2.35 (s, 3H).

**Example** 88 **Preparation** of **N-(4-(3-amino-6-(2-chlorophenyl)-1H-pyrazolo[4,3-c]pyridin-4-yl)benzyl)-5-fluoro-2-methoxybenzamide**

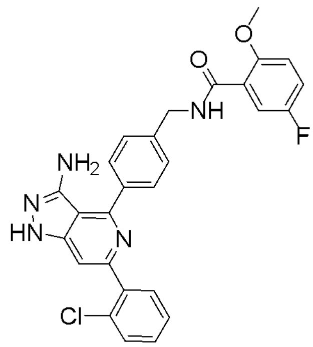

The **following synthetic route was adopted**

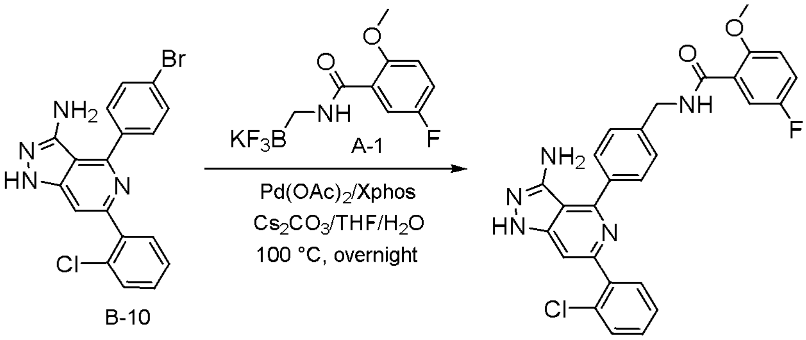


Intermediate B-10 (200 mg, 0.5 mmol), intermediate A-1 (216.8 mg, 0.75 mmol), palladium(II) acetate (11.2 mg, 0.05 mmol), 2-dicyclohexylphosphino-2',4',6'-triisoprophylbiphenyl (47.7 mg, 0.1 mmol), and cesium carbonate (488.7 mg, 1.5 mmol) were added to a 25 mL three-necked flask equipped with magnetic stirring and a condenser. The reaction system was evacuated and purged with nitrogen three times. Tetrahydrofuran (15 mL) and water (3 mL) were added, and the mixture was stirred to disperse. The mixture was stirred and reacted at 100 °C overnight. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 180 mg of a white solid, with a yield of 71.8%. LC-MS (APCI): m/z = 502.0 $(M+1)^+$. $^1H$ NMR (400 MHz, DMSO-$D_6$) δ (ppm): 12.26 (s, 1H), 8.86 (t, J = 6.0 Hz, 1H), 7.72-7.67 (m, 3H), 7.56-7.47 (m, 4H), 7.43-7.40 (m, 3H), 7.34-7.29 (m, 1H), 7.18-7.15 (m, 1H), 4.77 (s, 2H), 4.58 (d, J = 6.0 Hz, 2H), 3.88 (s, 3H).

**Example 89 Preparation of N-(4-(3-amino-6-(2,6-dichlorophenyl)-1H-pyrazolo[4,3-c]pyridin-4-yl)benzyl)-5-fluoro-2-methoxybenzamide**

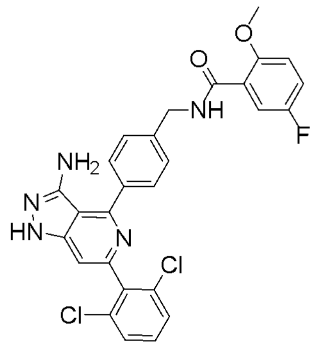

**The following synthetic route was adopted**

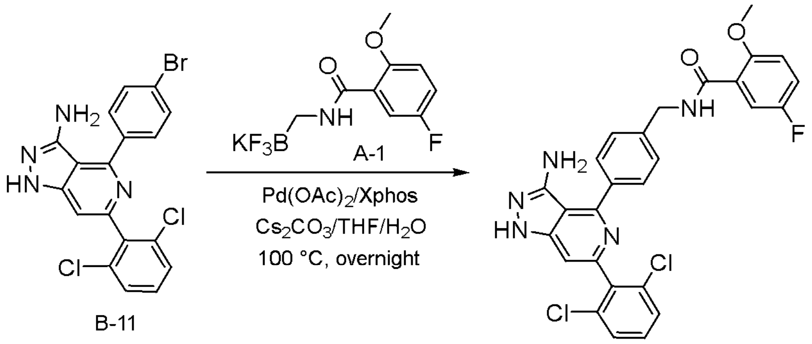


Intermediate B-11 (60 mg, 0.14 mmol), intermediate A-1 (60.7 mg, 0.21 mmol), palladium(II) acetate (3 mg, 0.014 mmol), 2-dicyclohexylphosphino-2',4',6'-triisoprophylbiphenyl (13.3 mg, 0.028 mmol), and cesium carbonate (136.8 mg, 0.42 mmol) were added to a 25 mL three-necked flask equipped with magnetic stirring and a condenser. The reaction system was evacuated and purged with nitrogen three times. Tetrahydrofuran (3 mL) and water (0.7 mL) were added, and the mixture was stirred to disperse. The mixture was stirred and reacted at 100 °C overnight. The mixture was cooled to room temperature, and saturated brine (5 mL) was added to quench the reaction. The organic phase was separated, and the aqueous phase was extracted with ethyl acetate (5 mL × 2). The organic phases were combined, washed with saturated brine (5 mL), dried over anhydrous sodium sulfate, filtered, concentrated, and separated by silica gel column chromatography to give 50 mg of a yellow solid, with a yield of 66.7%. LC-MS (APCI): m/z = 536.0 $(M+1)^+$. $^1H$ NMR (400 MHz, DMSO-$D_6$) δ (ppm): 12.26 (s, 1H), 8.86 (t, J = 6.0 Hz, 1H), 7.72-7.67 (m, 3H), 7.56-7.47 (m, 3H), 7.43-7.40 (m, 3H), 7.34-7.29 (m, 1H), 7.18-7.15 (m, 1H), 4.77 (s, 2H), 4.58 (d, J = 6.0 Hz, 2H), 3.88 (s, 3H).

**Example 90 Preparation of 4-(3-amino-4-(4-((5-fluoro-2-methoxybenzoylamino)methyl)phenyl)-1H-pyrazolo [4,3-c]pyridin-6-yl)benzoic acid**

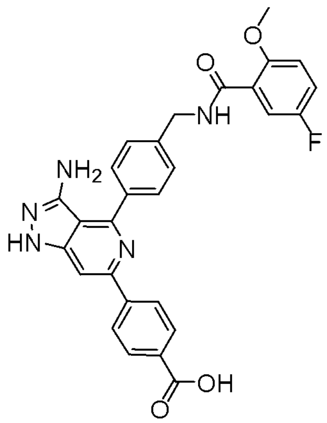

**The following synthetic route was adopted**

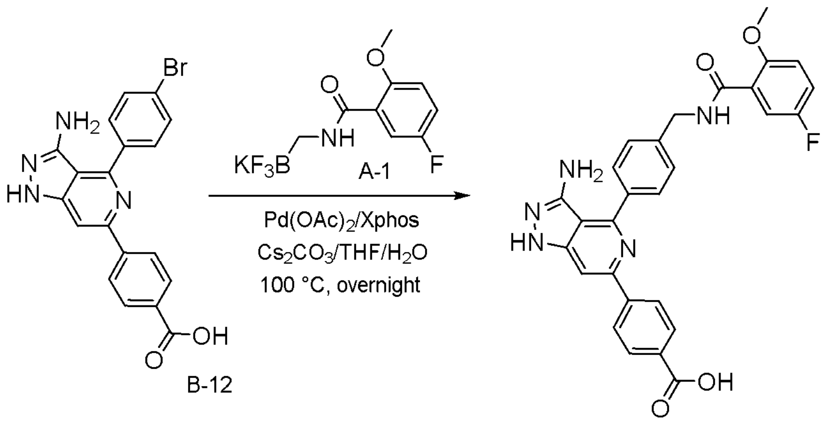


B-12

Intermediate B-12 (73 mg, 0.18 mmol), intermediate A-1 (78 mg, 0.27 mmol), palladium(II) acetate (4 mg, 0.018 mmol), 2-dicyclohexylphosphino-2',4',6'-triisoprophylbiphenyl (17.2 mg, 0.036 mmol), and cesium carbonate (175.9 mg, 0.54 mmol) were added to a 25 mL three-necked flask equipped with magnetic stirring and a condenser. The reaction system was evacuated and purged with nitrogen three times. Tetrahydrofuran (4 mL) and water (1 mL) were added, and the mixture was stirred to disperse. The mixture was stirred and reacted at 100 °C overnight. The mixture was cooled to room temperature, and saturated brine (5 mL) was added to quench the reaction. The organic phase was separated, and the aqueous phase was extracted with ethyl acetate (5 mL × 2). The organic phases were combined, washed with saturated brine (5 mL), dried over anhydrous sodium sulfate, filtered, concentrated, and separated by silica gel column chromatography to give 90 mg of a white solid, with a yield of 97.8%. LC-MS (APCI): m/z = 512.0 $(M+1)^+$. $^1H$ NMR (400 MHz, DMSO-$D_6$) δ (ppm): 8.93 (t, J = 6.0 Hz, 1H), 8.26-8.24 (m, 2H), 8.05 (d, J = 8.4 Hz, 2H), 7.86 (s, 1H), 7.80 (d, J = 8.4 Hz, 2H), 7.58-7.53 (m, 4H), 7.36-7.33 (m, 1H), 7.22-7.19 (m, 1H), 4.63 (d, J = 6.0 Hz, 2H), 3.92 (s, 3H).

**Example 91 Preparation of N-(4-(3-amino-6-(tetrahydro-2H-thiopyran-4-yl)-1H-pyrazolo[4,3-c]pyridin-4-yl)benzyl)-5-fluoro-2-methoxybenzamide**

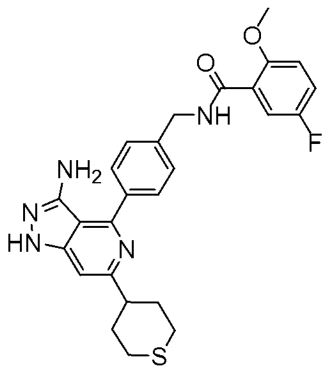

**The following synthetic route was adopted**

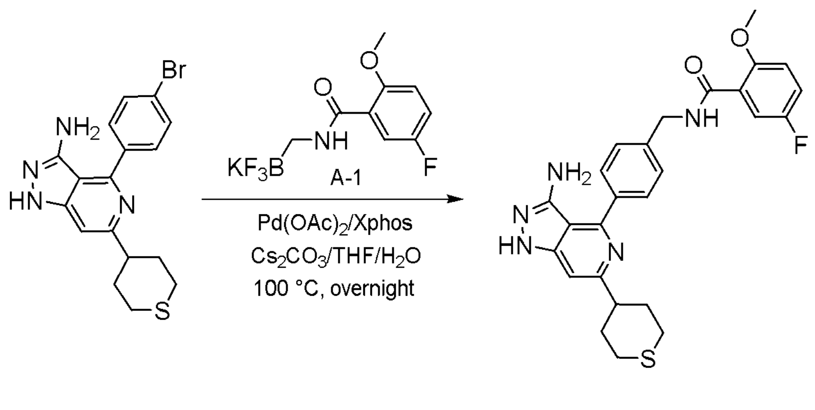

Intermediate B-13 (330 mg, 0.85 mmol), intermediate A-1 (491.3 mg, 1.7 mmol), palladium(II) acetate (19.08 mg, 0.085 mmol), 2-dicyclohexylphosphino-2',4',6'-triisoprophylbiphenyl (80.04 mg, 0.17 mmol), and cesium carbonate (830.8 mg, 2.55 mmol) were added to a 25 mL three-necked flask equipped with magnetic stirring and a condenser. The reaction system was evacuated and purged with nitrogen three times. Tetrahydrofuran (10 mL) and water (2 mL) were added, and the mixture was stirred to disperse. The mixture was stirred and reacted at 100 °C overnight. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 370 mg of a light yellow solid, with a yield of 88.6%. LC-MS (APCI): m/z = 492.0 $(M+1)^+$.

**Example 92 Preparation of N-(4-(3-amino-6-(1-oxidotetrahydro-2H-thiopyran-4-yl)-1H-pyrazolo[4,3-c]pyridin-4-yl)benzyl)-5-fluoro-2-hydroxybenzamide**

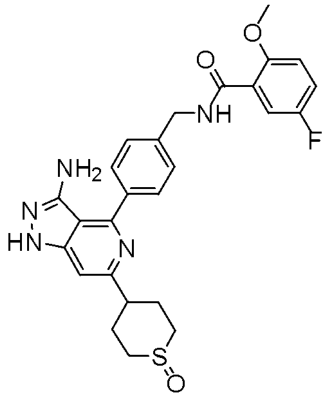

The following synthetic route was adopted

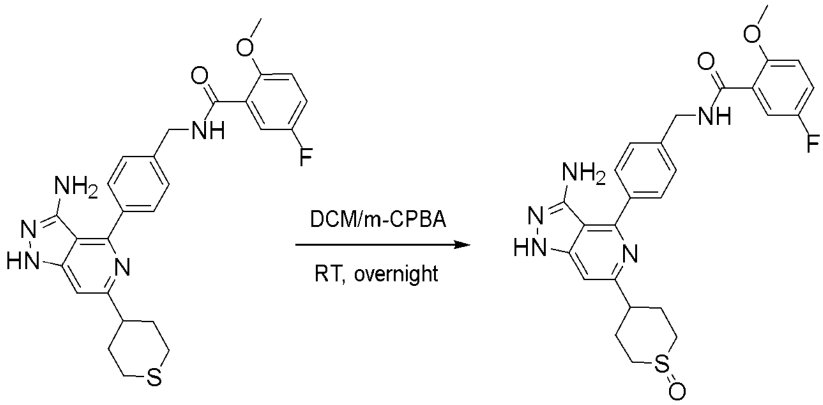


Example 91 (100 mg, 0.2 mmol) and dichloromethane (6 mL) were added to a 25 mL single-necked flask equipped with magnetic stirring, and the mixture was stirred until dissolved. m-Chloroperoxybenzoic acid (103.54 mg, 0.6 mmol) was added. The reaction system was evacuated and purged with nitrogen three times. The mixture was stirred and reacted at room temperature overnight. Saturated brine (10 mL) was added to quench the reaction. The mixture was extracted with ethyl acetate (10 mL × 2). The organic phases were combined, washed with saturated brine (10 mL), dried over anhydrous sodium sulfate, filtered, concentrated, and separated by silica gel column chromatography to give 93 mg of a light yellow solid, with a yield of 91.7%. LC-MS (APCI): m/z=494.0$(M+1)^+$. $^1$H NMR (400 MHz, DMSO-$D_6$) δ (ppm): 8.95 (t, J = 6.0 Hz, 1H), 7.75-7.73 (m, 2H), 7.63-7.61 (m, 2H), 7.51-7.48 (m, 2H), 7.36-7.33 (m, 1H), 7.22-7.19 (m, 1H), 4.62 (d, J = 6.0 Hz, 2H), 3.90 (s, 3H), 3.28-3.21 (m, 1H), 3.03-2.99 (m, 2H), 2.84-2.77 (m, 2H), 2.48-2.40 (m, 2H), 1.98-1.95 (m, 2H).

**Example 93 Preparation of N-(4-(3-amino-6-(1,1-dioxidotetrahydro-2H-thiopyran-4-yl)-1H-pyrazolo[4,3-c]pyridin-4-yl)benzyl)-5-fluoro-2-methoxybenzamide**

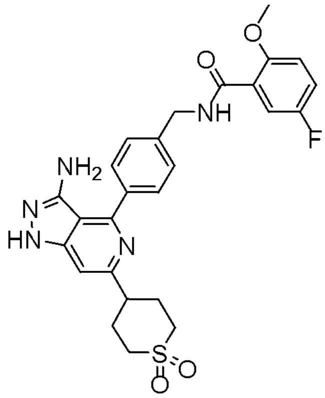

The **following synthetic route was adopted**

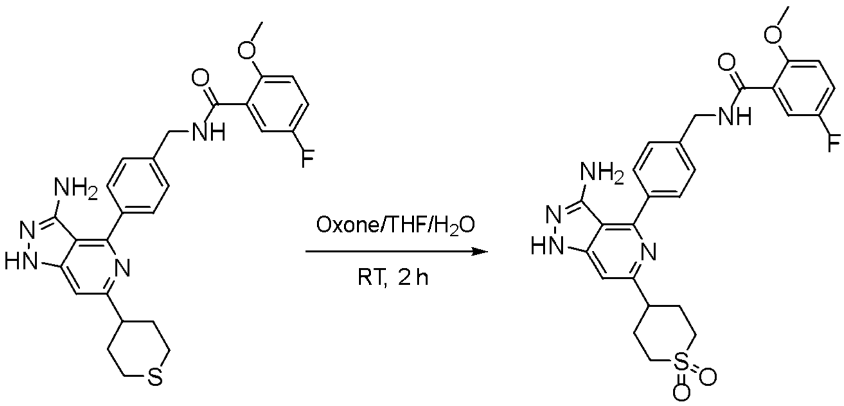


Example 91 (50 mg, 0.1 mmol), tetrahydrofuran (5 mL) and water (1 mL) were added to a 25 mL single-necked flask equipped with magnetic stirring, and the mixture was stirred thoroughly at 0 °C. Potassium peroxymonosulfate (61.4 mg, 0.1 mmol) was added in batches, and the mixture was stirred and reacted at room temperature for 2 hours. Saturated brine (5 mL) was added to quench the reaction. The mixture was extracted with ethyl acetate (5 mL × 2). The organic phases were combined, washed with saturated brine (5 mL), dried over anhydrous sodium sulfate, filtered, concentrated, and separated by silica gel column chromatography to give 33 mg of a light yellow solid, with a yield of 63.1%. LC-MS (APCI): m/z = 524.0 $(M+1)^+$.

**Example 94 Preparation of N-(4-(3-amino-6-chloro-1H-pyrazolo[4,3-c]pyridin-4-yl)benzyl)-5-fluoro-2-methoxybenzamide**

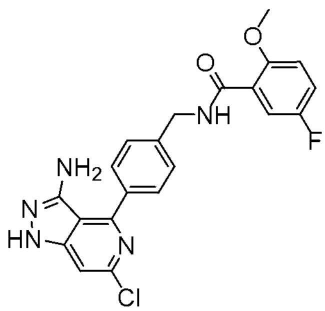

**The following synthetic route was adopted**

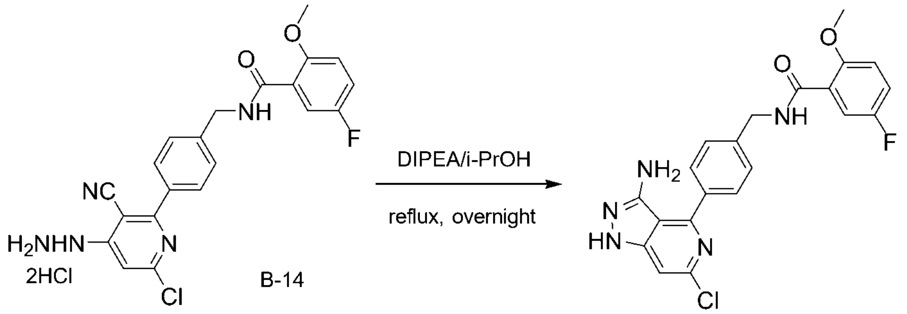


Intermediate B-14 (320 mg, 0.64 mmol), N,N-diisopropylethylamine (0.3 mL, 1.84 mmol), and isopropanol (5 mL) were successively added to a 25 mL single-necked flask equipped with magnetic stirring, and the mixture was heated and refluxed overnight. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 200 mg of a white solid, with a yield of 73.5%. LC-MS (APCI): m/z = 426.0 $(M+1)^+$. $^1H$ NMR (400 MHz, DMSO-$D_6$) δ (ppm): 12.30 (s, 1H), 8.87 (t, J = 6.0 Hz, 1H), 7.64 (d, J = 8.0 Hz, 2H), 7.51-7.48 (m, 3H), 7.35-7.30 (m, 1H), 7.28 (s, 1H), 7.19-7.16 (m, 1H), 4.81 (s, 2H), 4.59 (d, J = 6.0 Hz, 2H), 3.90 (s, 3H).

**Example 95 Preparation of 3-(3-amino-4-(4-((5-fluoro-2-methoxybenzoylamino)methyl)phenyl)-1H-pyrazolo [4,3-c]pyridin-6-yl)-N-phenylpiperidine-1-carboxamide**

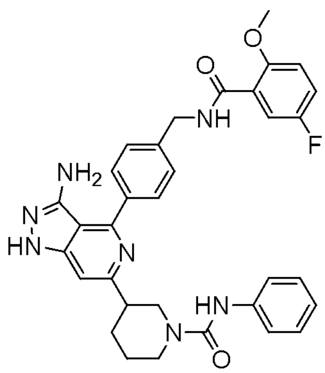

**The following synthetic route was adopted**

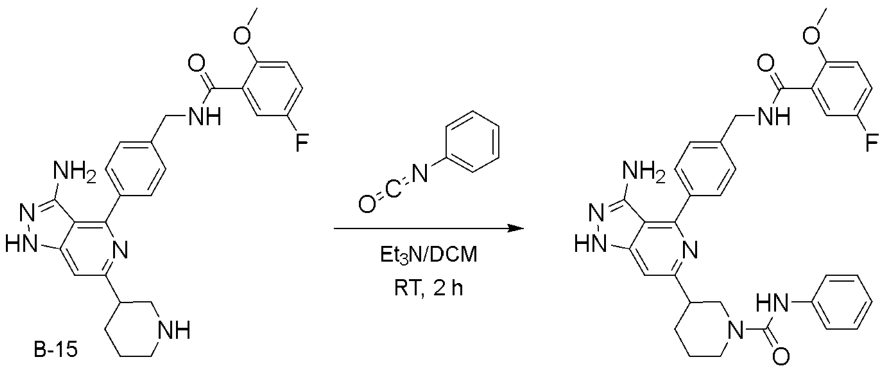


Intermediate B-15 (130 mg, 0.274 mmol), triethylamine (83.8 mg, 0.822 mmol), and dichloromethane (10 mL) were added to a 50 mL three-necked flask equipped with magnetic stirring. Phenyl isocyanate (35.9 mg, 0.301 mmol) was

added in an ice-water bath. The mixture was stirred and reacted at room temperature under nitrogen for 2 hours. Saturated aqueous solution of sodium bicarbonate was added to adjust the pH to alkaline in an ice-water bath. The mixture was extracted with ethyl acetate (50 mL × 2). The organic phases were combined, washed with saturated brine (50 mL), dried over anhydrous sodium sulfate, filtered, concentrated, and separated by silica gel column chromatography to give 50 mg of a white solid, with a yield of 30.7%. LC-MS (APCI): m/z = 594.0 $(M+1)^+$. $^1H$ NMR (400 MHz, DMSO-$D_6$) δ (ppm): 12.06 (s, 1H), 8.87 (t, J = 5.6 Hz, 1H), 8.47 (s, 1H), 7.78 (d, J = 8.8 Hz, 2H), 7.68-7.66 (d, J = 8.0 Hz, 2H), 7.53-7.48 (m, 3H), 7.43 (d, J = 8.0 Hz, 2H), 7.36-7.31 (m, 1H), 7.21-7.18 (m, 3H), 7.11 (s, 1H), 6.89 (t, J = 8.0 Hz, 1H), 4.67 (s, 2H), 4.59 (d, J = 6.0 Hz, 2H), 4.29 (d, J = 11.2 Hz, 1H), 4.15 (d, J = 13.6 Hz, 1H), 3.88 (s, 3H), 3.13 (t, J = 12.8 Hz, 1H), 2.91-2.80 (m, 2H), 2.03-2.00 (m, 1H), 1.87-1.84 (m, 1H), 1.76-1.73 (m, 1H), 1.56-1.53 (m, 1H).

**Example 96 Preparation of N-(4-(6-(1-acryloylpiperidin-3-yl)-3-amino-1H-pyrazolo[4,3-c]pyridin-4-yl)benzyl)-5-fluoro-2-methoxybenzamide**

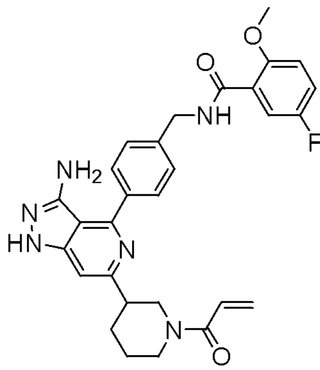

**The following synthetic route was adopted**

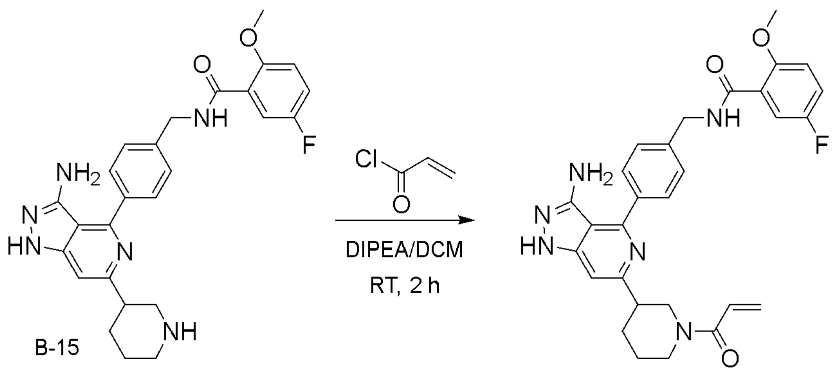


Intermediate B-15 (80 mg, 0.169 mmol), N,N-diisopropylethylamine (65.3 mg, 0.506 mmol), and dichloromethane (10 mL) were added to a 50 mL three-necked flask equipped with magnetic stirring. Acryloyl chloride (15.3 mg, 0.169 mmol) was added in an ice-water bath. The mixture was stirred and reacted at room temperature under nitrogen for 2 hours. Water (20 mL) was added, and the mixture was extracted with ethyl acetate (20 mL × 2). The organic phases were combined, washed with saturated brine (30 mL), dried over anhydrous sodium sulfate, filtered, concentrated, and separated by silica gel column chromatography to give 40 mg of a white solid, with a yield of 44.8%. LC-MS (APCI): m/z = 529.0 $(M+1)^+$.

**Example 97 Preparation of 4-(3-amino-4-(4-((5-fluoro-2-methoxvbenzoylamino)methyl)phenyl)-1H-pyrazolo [4,3-c]pyridin-6-yl)-N-phenylpiperidine-1-carboxamide**

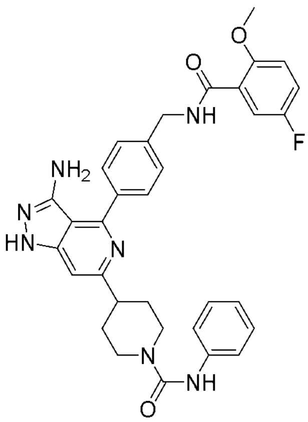

The following synthetic route was adopted

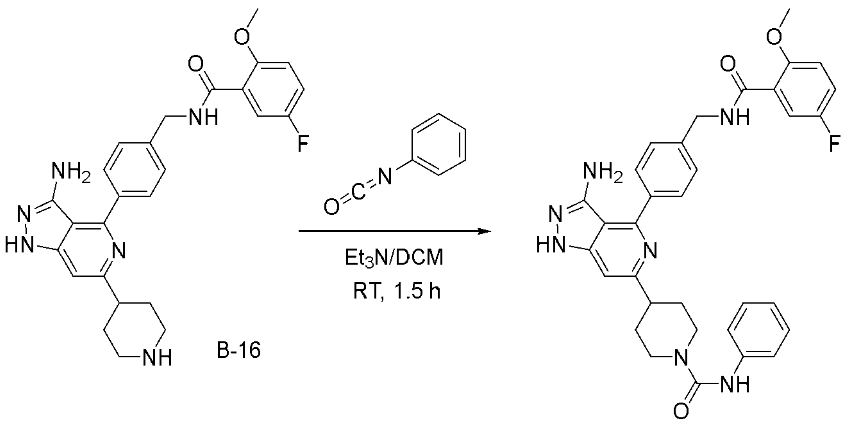


Intermediate B-16 (100 mg, 0.21 mmol), dichloromethane (5 mL), and triethylamine (64 mg, 0.63 mmol) were added to a container. The mixture was cooled to 0 °C, and phenyl isocyanate (37 mg, 0.315 mmol) was added dropwise. The mixture was reacted at room temperature for 1.5 hours. Water (50 mL) was added to quench the reaction and dilute the mixture. The mixture was extracted with dichloromethane (50 mL × 3). The organic phase was washed with saturated brine (100 mL), dried over anhydrous sodium sulfate, and concentrated. The residue was separated by silica gel column chromatography to give 40 mg of a light yellow solid, with a yield of 32%. ESI-MS: 594 [M+1]$^+$.

**Example 98 Preparation of N-(4-(3-amino-7-(pyrazolo[1,5-a]pyrazin-4-yl)-1H-pyrazolo[4,3-c]pyridin-4-yl)benzyl)-5-fluoro-2-methoxybenzamide**

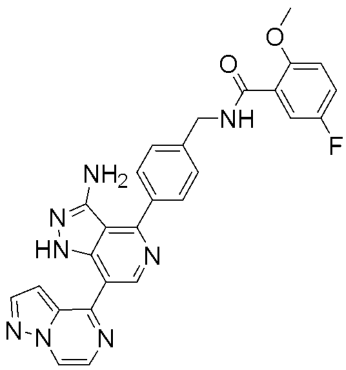

Example 98 was prepared using intermediate C-24 instead of 2-bromo-5-(methylsulfonyl)pyridine as the raw material according to the method described in example 35, with a production of 16 mg and a yield of 10.9%. LC-MS (APCI):

m/z =509.0 (M+1)+. 1H NMR (400 MHz, DMSO-$D_6$) δ (ppm): 12.59 (s, 1H), 9.18 (s, 1H), 8.93 (t, J = 6.0 Hz, 1H), 8.81 (d, J = 4.4 Hz, 1H), 8.31 (d, J = 2.0 Hz, 1H), 8.11 (d, J = 5.2 Hz, 1H), 7.77 (d, J = 8.4 Hz, 2H), 7.57-7.53 (m, 4H), 7.37-7.34 (m, 1H), 7.23-7.19 (m, 1H), 4.91 (s, 2H), 4.64 (d, J = 6.0 Hz, 2H), 3.92 (s, 3H).

**Example 99 Preparation of N-(4-(3-amino-7-(benzo[d]thiazol-2-yl)-1H-pyrazolo[4,3-c]pyridin-4-yl)benzyl)-5-fluoro-2-methoxybenzamide**

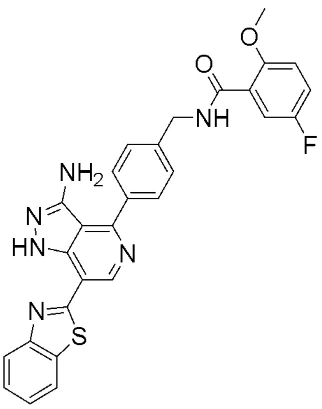

Example 99 was prepared using 2-bromobenzo[d]thiazole instead of 2-bromo-5-(methylsulfonyl)pyridine as the raw material according to the method described in example 35, with a production of 22 mg and a yield of 14.5 %. LC-MS (APCI): m/z =525.0 (M+1)+. 1H NMR (400 MHz, DMSO-$D_6$) δ (ppm): 12.57 (s, 1H), 8.91 (t, J = 6.0 Hz, 2H), 8.20 (t, J = 8.4 Hz, 2H), 7.75 (d, J = 8.0 Hz, 2H), 7.60-7.50 (m, 5H), 7.35-7.32 (m, 1H), 7.21-7.18 (m, 1H), 4.98 (s, 2H), 4.62 (d, J = 6.0 Hz, 2H), 3.91 (s, 3H).

**Example 100 Preparation of N-(4-(3-amino-7-(thiazolo[5,4-b]pyridin-2-yl)-1H-pyrazolo[4,3-c]pyridin-4-yl)benzyl)-5-fluoro-2-methoxybenzamide**

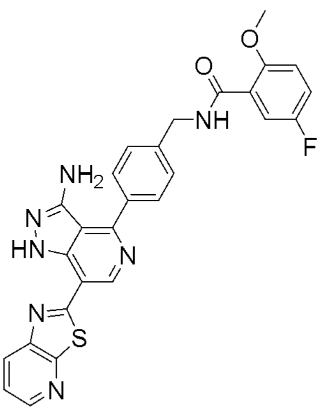

Example 100 was prepared using 2-bromothiazolo[5,4-b]pyridine instead of 2-bromo-5-(methylsulfonyl)pyridine as the raw material according to the method described in example 35, with a production of 14 mg and a yield of 9.2 %. LC-MS (APCI): m/z =526.0 (M+1)+. 1H NMR (400 MHz, DMSO-$D_6$) δ (ppm): 12.67 (s, 1H), 8.96 (s, 1H), 8.92 (t, J = 6.4 Hz, 1H), 8.65 (d, J = 4.4 Hz, 1H), 8.50 (d, J = 8.0 Hz, 1H), 7.76 (d, J = 8.4 Hz, 2H), 7.69-7.66 (m, 1H), 7.56-7.52 (m, 3H), 7.36-7.33 (m, 1H), 7.21-7.18 (m, 1H), 5.01 (s, 1H), 4.63 (d, J = 6.0 Hz, 2H), 3.91 (s, 3H).

**Example 101 Preparation of N-(4-(3-amino-7-(5-ethyl-4,5,6,7-tetrahydrothiazolo[5,4-c]pyridin-2-yl)-1H-pyrazolo[4,3-c]pyridin-4-yl)benzyl)-5-fluoro-2-methoxybenzamide**

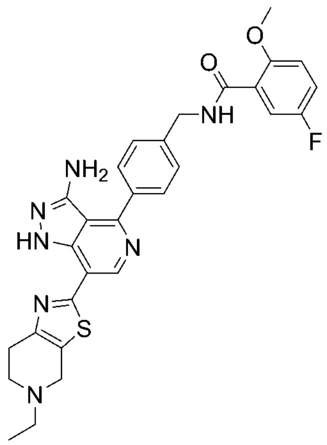

Example 101 was prepared using intermediate C-25 instead of 2-bromo-5-(methylsulfonyl)pyridine as the raw material according to the method described in example 35, with a production of 19 mg and a yield of 11.8 %. LC-MS (APCI): m/z =558.0 $(M+1)^+$. $^1H$ NMR (400 MHz, DMSO-$D_6$) δ (ppm): 12.19 (s, 1H), 8.90 (t, J = 6.0 Hz, 1H), 8.71 (s, 1H), 7.71 (d, J = 8.0 Hz, 2H), 7.55-7.51 (m, 3H), 7.37-7.32 (m, 1H), 7.21-7.18 (m, 1H), 4.91 (br s, 2H), 4.61 (d, J = 6.0 Hz, 2H), 3.91 (s, 3H), 3.75 (s, 2H), 2.93-2.91 (m, 2H), 2.86-2.83 (m, 2H), 2.61 (q, J = 7.2 Hz, 2H), 1.11 (t, J = 7.2 Hz, 3H).

**Example 102 Preparation of N-(4-(3-amino-7-(6,7-dihydro-9H-pyrido[2',3':4,5]imidazo[2,1-c][1,4]oxazin-2-yl)-1H-pyrazolo[4,3-c]pyridin-4-yl)benzyl)-5-fluoro-2-methoxybenzamide**

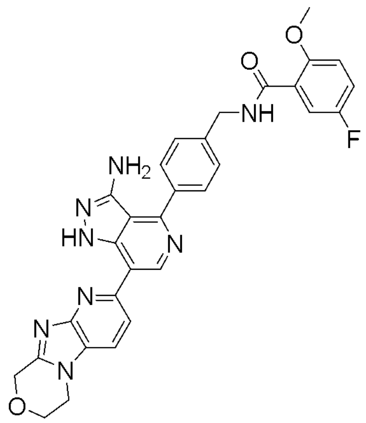

Example 102 was prepared using intermediate C-26 instead of 2-bromo-5-(methylsulfonyl)pyridine as the raw material according to the method described in example 35, with a production of 20 mg and a yield of 21.1%. LC-MS (APCI): m/z =565.0 $(M+1)^+$. $^1H$ NMR (400 MHz, DMSO-$D_6$) δ (ppm): 12.20 (s, 1H), 8.97 (s, 1H), 8.89 (t, J = 6.0 Hz, 1H), 8.23 (d, J = 8.0 Hz, 1H), 8.10 (d, J = 8.8 Hz, 1H), 7.71 (d, J = 8.0 Hz, 2H), 7.54-7.47 (m, 3H), 7.36-7.31 (m, 1H), 7.22-7.19 (m, 1H), 4.90 (br s, 2H), 5.03 (s, 2H), 4.88 (s, 2H), 4.71-4.68 (m, 2H), 4.19-4.15 (m, 2H), 3.88 (s, 3H).

**Example 103 Preparation of N-(4-(3-amino-7-(6-methyl-6,7-dihydro-9H-pyrido[2',3':4,5]imidazo[2,1-c][1,4]oxazin-2-yl)-1H-pyrazolo[4,3-c]pyridin-4-yl)benzyl)-5-fluoro-2-methoxybenzamide**

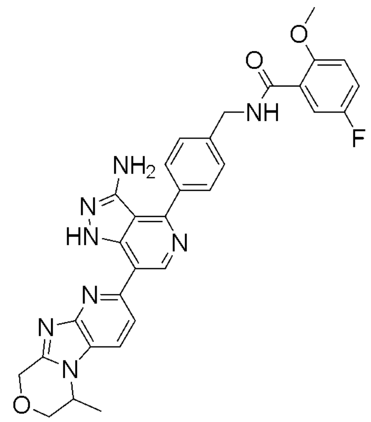

Example 103 was prepared using intermediate C-27 instead of 2-bromo-5-(methylsulfonyl)pyridine as the raw material according to the method described in example 35, with a production of 20 mg and a yield of 18.8%. LC-MS (APCI): m/z =579.0 $(M+1)^+$. $^1H$ NMR (400 MHz, DMSO-$D_6$) δ (ppm): 12.20 (s, 1H), 8.97 (s, 1H), 8.89 (t, J = 6.0 Hz, 1H), 8.23 (d, J = 8.0 Hz, 1H), 8.10 (d, J = 8.8 Hz, 1H), 7.71 (d, J = 8.0 Hz, 2H), 7.54-7.47 (m, 3H), 7.36-7.31 (m, 1H), 7.22-7.19 (m, 1H), 4.90(br s, 2H), 5.03(dd, J = 36.8 Hz, J = 16.8 Hz, 2H), 4.88 (s, 2H), 4.71-4.68 (m, 1H), 4.60 (d, J = 6.0 Hz, 2H), 4.19-4.15 (m, 1H), 4.02-3.98 (m, 1H), 3.88 (s, 3H), 1.52 (d, J-6.4 Hz, 3H).

**Example 104 Preparation of N-(4-(3-amino-7-(1-methyl-1H-pyrazol-4-yl)-6-(1,1,1-trifluoropropan-2-yl)-1H-pyrazolo[4,3-c]pyridin-4-yl)benzyl)-5-fluoro-2-methoxybenzamide**

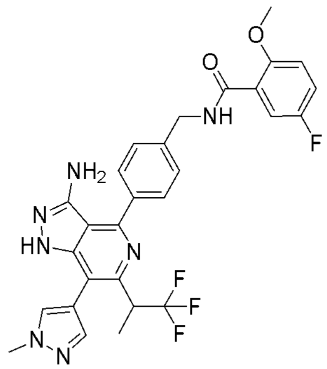

Example 104 was prepared using 1-methylpyrazole-4-boronic acid pinacol ester instead of 1-methylpyrazole-3-boronic acid pinacol ester as the raw material according to the method described in example 6, with a production of 65 mg and a yield of 55.6%. LC-MS (APCI): m/z = 568.2 $(M+1)^+$. $^1H$ NMR (400 MHz, DMSO-$D_6$) δ (ppm): 11.99 (s, 1H), 8.89 (t, J = 6.0 Hz, 1H), 7.96 (s, 1H), 7.72 (d, J = 8.0 Hz, 2H), 7.59 (s, 1H), 7.56-7.51 (m, 3H), 7.37-7.32 (m, 1H), 7.21-7.18 (m, 1H), 7.28 (s, 1H), 7.21-7.18 (m, 1H), 4.77 (s, 2H), 4.60 (d, J = 6.0 Hz, 2H), 4.02-3.96 (m, 1H), 3.95 (s, 3H), 3.91 (s, 3H), 1.49 (d, J = 6.8 Hz, 3H).

**Example 105 Preparation of N-(4-(3-amino-7-(pyridin-3-yl)-1H-pyrazolo[4,3-c]pyridin-4-yl)benzyl)-5-fluoro-2-methoxybenzamide**

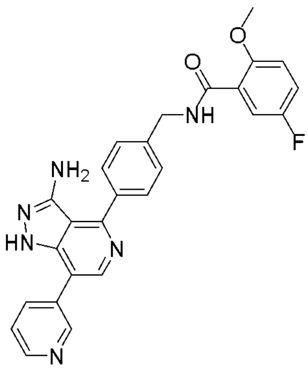

Example 105 was prepared using pyridine-3-boronic acid pinacol ester instead of pyrimidine-5-boronic acid as the raw material according to the method described in example 16, with a production of 61 mg and a yield of 56.2%. LC-MS (APCI): m/z = 469.1 $(M+1)^+$. $^1$H NMR (400 MHz, DMSO-$D_6$) δ (ppm): 12.50 (s, 1H), 8.94-8.89 (m, 2H), 8.66 (d, J = 6.0 Hz, 1H), 8.37 (s, 1H), 8.16-8.14 (m, 1H), 7.70 (d, J = 8.4 Hz, 2H), 7.59-7.52 (m, 4H), 7.37-7.32 (m, 1H), 7.21-7.18 (m, 1H), 4.81 (br s, 2H), 4.60 (d, J = 6.0 Hz, 2H), 3.91 (s, 3H).

**Example 106 Preparation of N-(4-(3-amino-7-(pyridin-4-yl)-1H-pyrazolo[4,3-c]pyridin-4-yl)benzyl)-5-fluoro-2-methoxybenzamide**

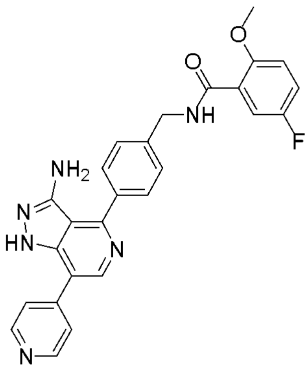

Example 106 was prepared using pyridine-4-boronic acid pinacol ester instead of pyrimidine-5-boronic acid as the raw material according to the method described in example 16, with a production of 60 mg and a yield of 60.1%. LC-MS (APCI): m/z = 469.1 $(M+1)^+$. $^1$H NMR (400 MHz, DMSO-$D_6$) δ (ppm): 12.53 (s, 1H), 8.91 (t, J = 6.0 Hz, 1H), 8.47 (s, 1H), 7.78-7.76 (m, 1H), 7.69 (d, J = 8.4 Hz, 2H), 7.55-7.52 (m, 3H), 7.37-7.32 (m, 1H), 7.21-7.18 (m, 1H), 4.84 (br s, 2H), 4.62 (d, J = 6.0 Hz, 2H), 3.91 (s, 3H).

**Example 107 Preparation of N-(4-(3-amino-7-(1H-imidazol-4-yl)-1H-pyrazolo[4,3-c]pyridin-4-yl)benzyl)-5-fluoro-2-methoxybenzamide**

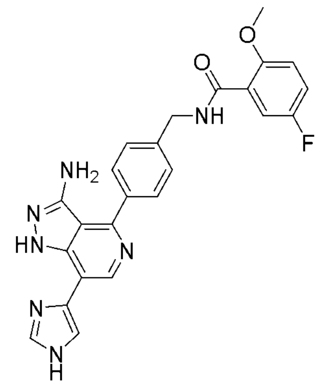

Example 107 was prepared using (1H-imidazol-4-yl)boronic acid instead of pyrimidine-5-boronic acid as the raw material according to the method described in example 16, with a production of 22 mg and a yield of 15.3%. LC-MS (APCI): m/z = 458.1 $(M+1)^+$. $^1H$ NMR (400 MHz, DMSO-$D_6$) δ (ppm): 12.47 (s, 1H), 11.93 (s, 1H), 8.89 (t, J = 6.0 Hz, 1H), 8.78 (s, 1H), 7.95-7.94 (m, 1H), 7.67 (d, J = 8.0 Hz, 2H), 7.55-7.52 (m, 3H), 7.37-7.32 (m, 1H), 7.21-7.18 (m, 1H), 4.75 (br s, 2H), 4.61 (d, J = 6.0 Hz, 2H), 3.91 (s, 3H).

**Example 108 Preparation of N-(4-(3-amino-7-(5-methyl-1H-pyrazol-3-yl)-1H-pyrazolo[4,3-c]pyridin-4-yl)benzyl)-5-fluoro-2-methoxybenzamide**

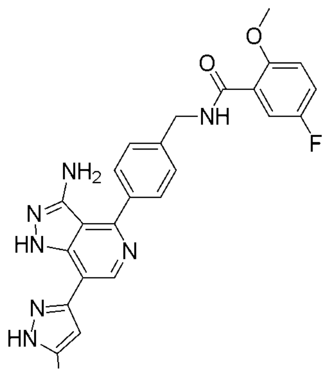

Example 108 was prepared using (5-methyl-1H-pyrazol-3-yl)boronic acid instead of pyrimidine-5-boronic acid as the raw material according to the method described in example 16, with a production of 15 mg and a yield of 14.2%. LC-MS (APCI): m/z = 472.1 $(M+1)^+$. $^1H$ NMR (400 MHz, DMSO-$D_6$) δ (ppm): 12.47 (s, 1H), 11.32 (s, 1H), 8.89 (t, J = 6.0 Hz, 1H), 8.74 (s, 1H), 7.96-7.94 (m, 1H), 7.67 (d, J = 8.0 Hz, 2H), 7.52-7.48 (m, 3H), 7.37-7.32 (m, 1H), 7.23-7.19 (m, 1H), 4.75 (br s, 2H), 4.61 (d, J = 6.0 Hz, 2H), 3.91 (s, 3H), 2.56 (s, 3H).

**Example 109 Preparation of N-(4-(3-amino-7-(pyrazolo[1,5-a]pyrazin-3-yl)-1H-pyrazolo[4,3-c]pyridin-4-yl)benzyl)-5-fluoro-2-methoxybenzamide**

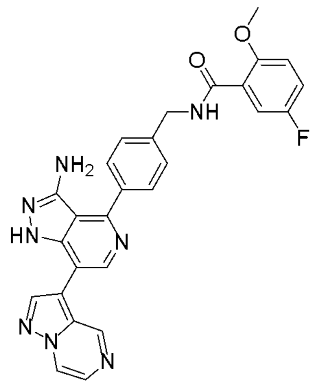

Example 109 was prepared using intermediate C-21 instead of pyrimidine-5-boronic acid as the raw material according to the method described in example 16, with a production of 40 mg and a yield of 62.7%. LC-MS (APCI): m/z = 509.1 $(M+1)^+$. $^1$H NMR (400 MHz, DMSO-$D_6$) δ (ppm): 8.95-8.91 (m, 2H), 8.60-8.53 (m, 1H), 8.04 (d, J = 4.4 Hz, 1H), 7.75 (d, J = 8.0 Hz, 2H), 7.63-7.60 (m, 2H), 7.54-7.51 (m, 1H), 7.37-7.32 (m, 1H), 7.21-7.18 (m, 1H), 4.63(d, J = 6.0 Hz, 2H), 3.91 (s, 3H).

**Example 110 Preparation of N-(4-(3-amino-7-(imidazo[1,2-b]pyridazin-3-yl)-1H-pyrazolo[4,3-c]pyridin-4-yl)benzyl)-5-fluoro-2-methoxybenzamide**

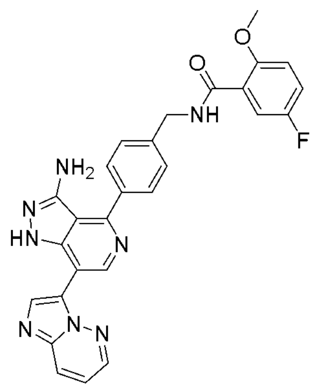

Example 110 was prepared using 3-bromoimidazo[1,2-b]pyridazine instead of 2-bromo-5-(methylsulfonyl) pyridine as the raw material according to the method described in example 35, with a production of 10 mg and a yield of 12.2%. LC-MS (APCI): m/z = 509.0 $(M+1)^+$. $^1$H NMR (400 MHz, DMSO-$D_6$) δ (ppm): 12.59 (s, 1H), 9.18 (s, 1H), 8.93 (t, J = 6.0 Hz, 1H), 8.85 (d, J = 4.4 Hz, 1H), 8.23 (d, J = 2.0 Hz, 1H), 8.11 (d, J = 5.2 Hz, 1H), 7.77 (d, J = 8.4 Hz, 2H), 7.57-7.53 (m, 4H), 7.35-7.32 (m, 1H), 7.15-7.08 (m, 1H), 4.91 (s, 2H), 4.64 (d, J = 6.0 Hz, 2H), 3.92 (s, 3H).

**Example 111 Preparation of N-(4-(3-amino-7-(pyrazolo[1,5-a]pyrimidin-7-yl)-1H-pyrazolo[4,3-c]pyridin-4-yl)benzyl)-5-fluoro-2-methoxybenzamide**

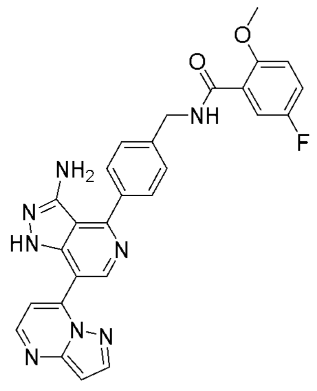

Example 111 was prepared using 7-bromopyrazolo[1,5-a]pyrimidine instead of 2-bromo-5-(methylsulfonyl) pyridine as the raw material according to the method described in example 35, with a production of 12 mg and a yield of 11.8%. LC-MS (APCI): m/z = 509.1 $(M+1)^+$. $^1H$ NMR (400 MHz, DMSO-$D_6$) δ (ppm): 12.54 (s, 1H), 8.95-8.91 (m, 2H), 8.63-8.60 (m, 1H), 8.04 (d, J = 4.4 Hz, 1H), 7.75 (d, J = 8.0 Hz, 2H), 7.63-7.60 (m, 2H), 7.54-7.51 (m, 1H), 7.41-7.38 (m, 1H), 7.21-7.18 (m, 1H), 4.63 (d, J = 6.0 Hz, 2H), 3.91 (s, 3H).

## Example 112 Preparation of N-(4-(3-amino-7-(imidazo[1,2-a]pyridin-2-yl)-1H-pyrazolo[4,3-c]pyridin-4-yl)benzyl)-5-fluoro-2-methoxybenzamide

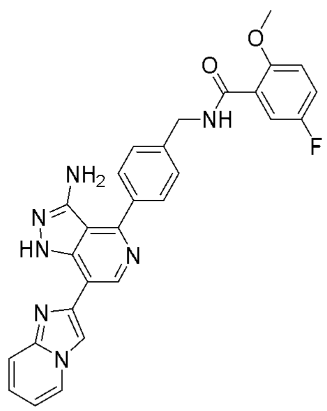

Example 112 was prepared using 2-bromoimidazo[1,2-a]pyridine instead of 2-bromo-5-(methylsulfonyl)pyridine as the raw material according to the method described in example 35, with a production of 10 mg and a yield of 9.8%. LC-MS (APCI): m/z = 508.2 $(M+1)^+$. $^1H$ NMR (400 MHz, DMSO-$D_6$) δ (ppm): 12.17 (s, 1H), 9.95-9.91 (m, 2H), 8.69 (s, 1H), 8.60 (d, J = 6.4 Hz, 1H), 7.73-7.68 (m, 3H), 7.56-7.52 (m, 3H), 7.38-7.32 (m, 2H), 7.22-7.19 (m, 1H), 6.98 (t, J = 7.2 Hz, 1H), 4.84 (br s, 2H), 4.63 (d, J = 6.0 Hz, 2H), 3.92 (s, 3H).

## Example 113 Preparation of N-(4-(3-amino-7-(imidazo[1,5-a]pyridin-1-yl)-1H-pyrazolo[4,3-c]pyridin-4-yl)benzyl)-5-fluoro-2-methoxybenzamide

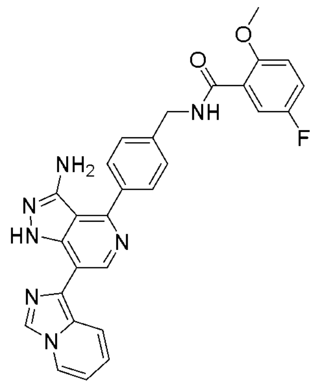

Example 113 was prepared using 1-bromoimidazo[1,5-a]pyridine instead of 2-bromo-5-(methylsulfonyl)pyridine as the raw material according to the method described in example 35, with a production of 15 mg and a yield of 12.6%. LC-MS (APCI): m/z = 508.2 $(M+1)^+$. $^1H$ NMR (400 MHz, DMSO-$D_6$) δ (ppm): 1.65 (br s, 1H), 8.95 (t, J = 6.0 Hz, 1H), 8.74 (s, 1H), 8.60 (s, 1H), 8.54 (d, J = 5.6 Hz, 1H), 8.11-8.08 (m, 1H), 7.77 (d, J = 8.8 Hz, 2H), 7.62 (d, J = 8.0 Hz, 2H), 7.54-7.51 (m, 1H), 7.37-7.32 (m, 1H), 7.21-7.18 (m, 1H), 7.12-7.08 (m, 1H), 6.92-6.90 (m, 1H), 5.16 (br s, 2H), 4.63 (d, J = 6.0 Hz, 2H), 3.90 (s, 3H).

**Example 114 Preparation of N-(4-(3-amino-7-(1-(1-(oxetan-3-yl)piperidin-4-yl)-1H-imidazol-4-yl)-1H-pyrazolo [4,3-c]pyridin-4-yl)benzyl)-5-fluoro-2-methoxybenzamide**

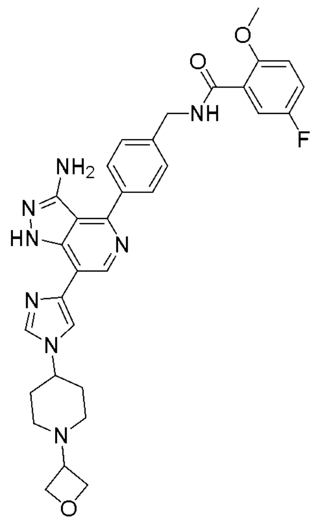

Example 114 was prepared using intermediate C-28 instead of 2-bromo-5-(methylsulfonyl)pyridine as the raw material according to the method described in example 35, with a production of 10 mg and a yield of 16.7%. LC-MS (APCI): m/z = 597.2 $(M+1)^+$. $^1H$ NMR (400 MHz, DMSO-$D_6$) δ (ppm): 8.91 (t, J = 6.0 Hz, 1H), 8.65 (s, 1H), 8.26-8.18 (m, 2H), 8.02 (s, 1H), 7.71-7.68 (m, 2H), 7.57-7.54 (m, 1H), 7.53-7.50 (m, 1H), 7.37-7.32 (m, 1H), 7.21-7.18 (m, 1H), 6.64 (s, 1H), 5.31 (t, J = 4.8 Hz, 1H), 4.61 (d, J = 6.0 Hz, 2H), 4.48-4.45 (m, 2H), 3.89 (s, 3H), 3.45-2.40 (m, 5H), 2.87-2.84 (m, 2H), 2.05-2.00 (m, 4H).

**Example 115 Preparation of N-(4-(3-amino-6-(tetrahydro-2H-pyran-4-yl)-1H-pyrazolo[4,3-c]pyridin-4-yl)benzyl)-5-fluoro-2-methoxybenzamide**

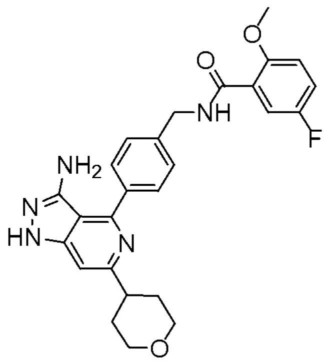

**The following synthetic route was adopted**

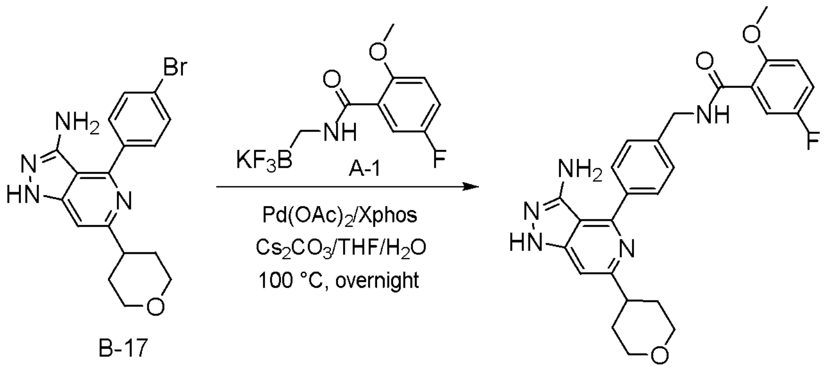


Intermediate B-17 (316 mg, 0.85 mmol), intermediate A-1 (491.3 mg, 1.7 mmol), palladium(II) acetate (19.08 mg, 0.085 mmol), 2-dicyclohexylphosphino-2',4',6'-triisoprophylbiphenyl (80.04 mg, 0.17 mmol), and cesium carbonate (830.8 mg, 2.55 mmol) were added to a 25 mL three-necked flask equipped with magnetic stirring and a condenser. The reaction system was evacuated and purged with nitrogen three times. Tetrahydrofuran (10 mL) and water (2 mL) were added, and the mixture was stirred to disperse. The mixture was stirred and reacted at 100 °C overnight. The mixture was cooled to room temperature, and saturated brine (10 mL) was added to quench the reaction. The organic phase was separated, and the aqueous phase was extracted with ethyl acetate (5 mL × 2). The organic phases were combined, washed with saturated brine (10 mL), dried over anhydrous sodium sulfate, filtered, concentrated, and separated by silica gel column chromatography to give 300 mg of a light yellow solid, with a yield of 74.2%. LC-MS (APCI): m/z = 476.0 $(M+1)^+$. $^1H$ NMR (400 MHz, DMSO-$D_6$) δ (ppm): 12.03 (s, 1H), 8.89 (t, J = 5.6 Hz, 1H), 7.67 (d, J = 8.4 Hz, 2H), 7.54-7.51 (m, 3H), 7.36-7.31 (m, 1H), 7.20-7.17 (m, 1H), 7.03 (s, 1H), 4.68 (br s, 2H), 4.61 (d, J = 6.0 Hz, 2H), 4.00-3.95 (m, 2H), 3.91 (s, 3H), 3.49-3.44 (m, 2H), 3.01-2.95 (m, 1H), 1.86-1.80 (m, 4H).

**Example 116 Preparation of N-(4-(3-amino-1H-pyrazolo[4,3-c]pyridin-4-yl)benzyl)-5-fluoro-**2-methoxvbenzamide

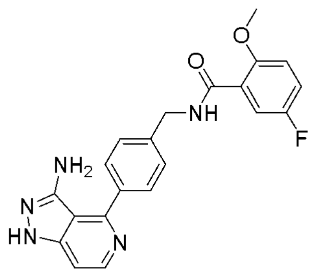

**The following synthetic route was adopted**

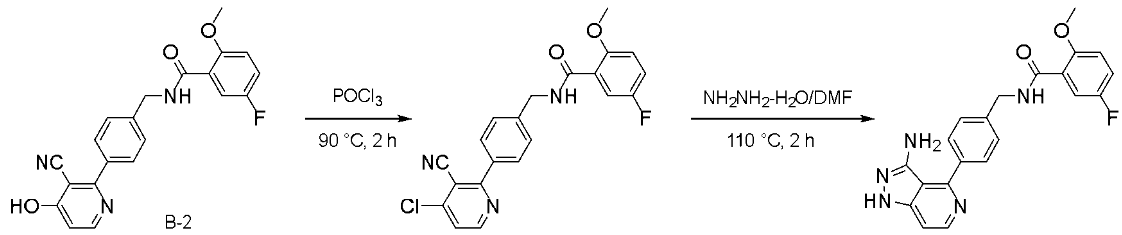


Step 1: Synthesis of compound N-(4-(4-chloro-3-cyanopyridin-2-yl)benzyl)-5-fluoro-2-methoxybenzamide

Intermediate B-2(0.38 g, 1 mmol) and phosphorus oxychloride (5 mL) were added to a 25 mL single-necked flask equipped with magnetic stirring and a condenser. The reaction system was evacuated and purged with nitrogen three times. The mixture was stirred and reacted at 90 °C for 2 hours. The mixture was cooled to room temperature, and concentrated under reduced pressure. Saturated aqueous solution of sodium bicarbonate (10 mL) was added to quench the reaction. The mixture was extracted with ethyl acetate (10 mL × 2). The organic phases were combined, washed with saturated brine (10 mL), dried over anhydrous sodium sulfate, filtered, concentrated, and separated by silica gel column chromatography to give 150 mg of a light yellow solid, with a yield of 38.0%. LC-MS (APCI): m/z = 396.0 $(M+1)^+$.

Step 2: Synthesis of example 116

N-(4-(4-Chloro-3-cyanopyridin-2-yl)benzyl)-5-fluoro-2-methoxybenzamide (150 mg, 0.38 mmol), hydrazine hydrate (0.6 mL, 2.34 mmol), and N,N-dimethylformamide (6 mL) were added to a 15 mL sealed vial equipped with magnetic stirring. The vial was sealed, and the mixture was stirred and reacted at **110** °C for 2 hours. The mixture was cooled to room temperature, and saturated brine (10 mL) was added to quench the reaction. The organic phase was separated, and the aqueous phase was extracted with ethyl acetate (10 mL × 2). The organic phases were combined, washed with saturated brine (10 mL), dried over anhydrous sodium sulfate, filtered, concentrated, and separated by silica gel column chromatography to give 80 mg of a yellow solid, with a yield of 55.0%. LC-MS(APCI): m/z = 392.2 $(M+1)^+$. $^1H$ NMR (400 MHz, DMSO-$D_6$) δ (ppm): 8.89 (t, J = 6.0 Hz, 1H), 8.31 (s, 1H), 8.28 (d, J = 6.0 Hz, 1H), 7.69 (d, J = 8.0 Hz, 2H), 7.54-7.51 (m, 1H), 7.43 (d, J = 8.0 Hz, 2H), 7.35-7.32 (m, 1H), 7.20-7.17 (m, 1H), 7.09 (d, J = 6.0 Hz, 1H), 4.57 (d, J = 6.0 Hz, 2H), 4.54 (br s, 2H), 3.91 (s, 3H).

**Example 117 Preparation of N-(4-(3-amino-6-((1r,4r)-4-(hydroxymethyl)cyclohexyl)-1H-pyrazolo[4,3-c]pyridin-4-yl)benzyl)-5-fluoro-2-methoxybenzamide**

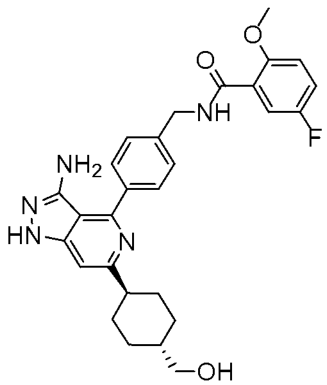

**The following synthetic route was adopted**

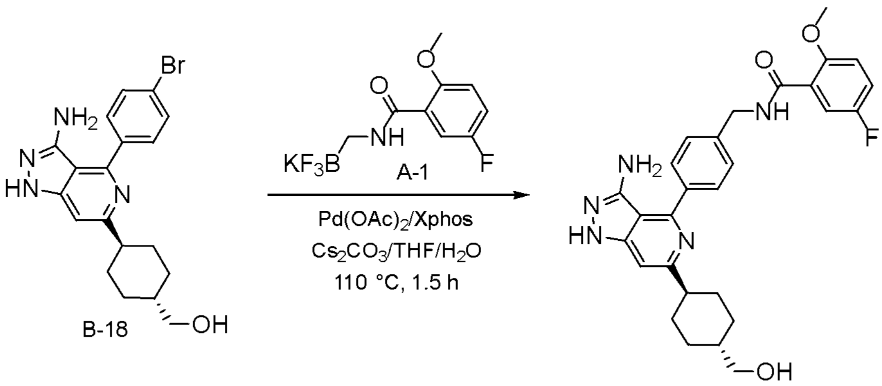


Intermediate B-18(360 mg, 0.9 mmol), intermediate A-1 (211 mg, 1.02 mmol), 2-dicyclohexylphosphino-2',4',6'-triisoprophylbiphenyl (44 mg, 0.09 mmol), palladium(II) acetate (7 mg, 0.03 mmol), cesium carbonate (908 mg, 2.79 mmol), tetrahydrofuran (10 mL) and water (1 mL) were added to a microwave vial equipped with magnetic stirring. The mixture was stirred until dissolved. The reaction system was purged with nitrogen three times. The mixture was heated to 110 °C, and stirred at the same temperature for 1.5 hours. The mixture was cooled to room temperature, and the reaction solution was diluted with added ethyl acetate (30 mL). The insoluble solid was filtered out. The filtrate was concentrated to dryness, and the residue was separated by silica gel column chromatography to give 220 mg of a yellowish-white solid, with a yield of 48.6%. LC-MS (APCI): m/z = 504.2 $(M+1)^+$. $^1H$ NMR (400 MHz, DMSO-$D_6$) δ (ppm): 12.04 (s, 1H), 8.91 (t, J = 5.6 Hz, 1H), 7.67 (d, J = 8.4 Hz, 2H), 7.54-7.51 (m, 3H), 7.36-7.31 (m, 1H), 7.20-7.17 (m, 1H), 7.03 (s, 1H), 4.68 (br s, 2H), 4.60 (d, J = 6.0 Hz, 2H), 4.45 (t, J = 6.0 Hz, 1H), 4.24-4.18 (m, 1H), 3.90 (s, 3H), 3.28-3.24 (m, 2H), 2.38-2.29 (m, 2H), 1.87 (d, J = 12.8 Hz, 2H), 1.78 (d, J = 10.4 Hz, 2H), 1.45-1.42 (m, 1H), 1.10-1.01 (m, 2H).

**Example 118 Preparation of compound N-(4-(3-amino-7-(1',2',3',6'-tetrahydro-[3,4'-bipyridin]-6-yl)-1H-pyrazolo[4,3-c]pyridin-4-yl)benzyl)-5-fluoro-2-methoxybenzamide**

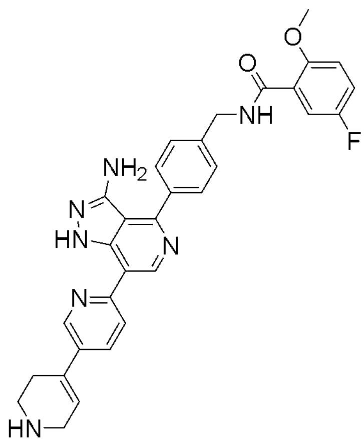

The following synthetic route was adopted

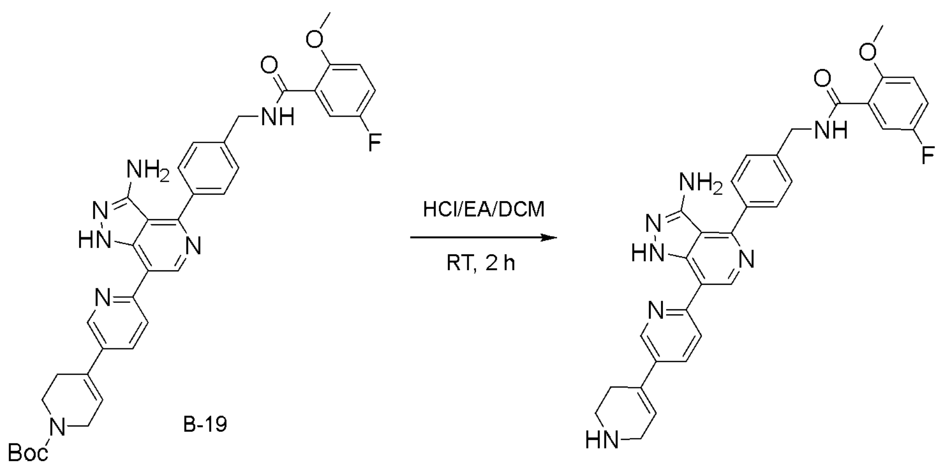


Intermediate B-19 (500 mg, 0.77 mmol) and dichloromethane (10 mL) were added to a 100 mL single-necked flask equipped with magnetic stirring. The mixture was stirred until dissolved, and a solution of hydrogen chloride in ethyl acetate (4 mL, 4 M) was added. The mixture was reacted at room temperature under nitrogen for 2 hours. The mixture was concentrated to dryness under reduced pressure to give 500 mg of a white solid, with a yield of 100%. LC-MS (APCI): m/z = 550.2 $(M+1)^+$.

**Example 119 Preparation of compound N-(4-(3-amino-7-(5-(piperidin-4-yl)pyridin-2-yl)-1H-pyrazolo[4,3-c]pyridin-4-yl)benzyl)-5-fluoro-2-methoxybenzamide**

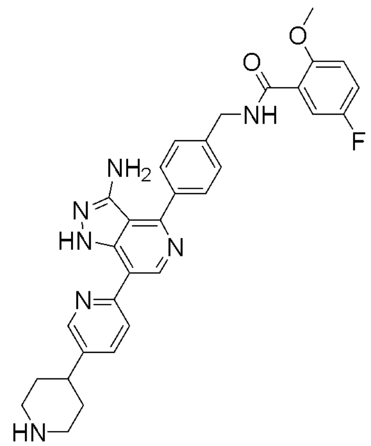

**The following synthetic route was adopted**

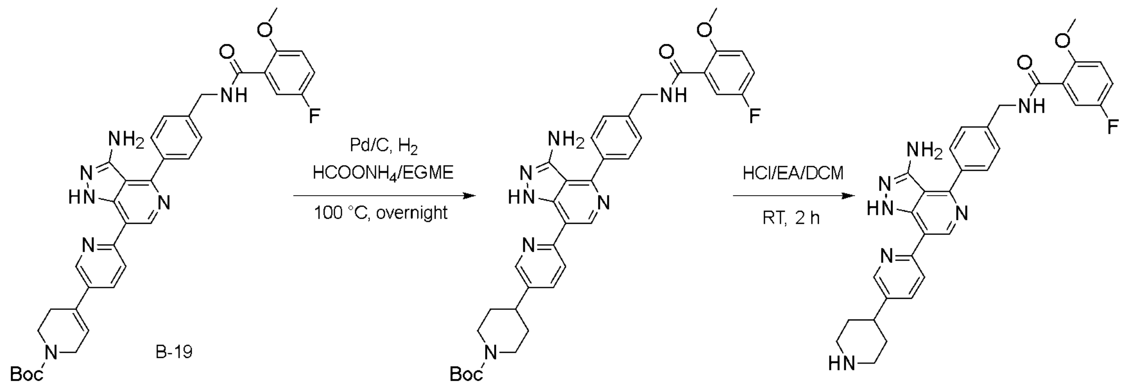

Step 1: Synthesis of compound tert-butyl 4-(6-(3-amino-4-(4-((5-fluoro-2-methoxybenzoylamino)methyl)phenyl)-1H-pyrazolo[4,3-c]pyridin-7-yl)pyridin-3-yl)piperidine-1-carboxylate

Intermediate B-19 (2.8 g, 4.31 mmol), ammonium formate (2.0 g, 31.7 mmol), ethylene glycol monomethyl ether (30 mL), and wet palladium on carbon (280 mg, 10%) were successively added to a 100 mL stainless steel jar equipped with magnetic stirring. The mixture was purged with hydrogen for 30 seconds. The jar was sealed, and placed in an oil pan. The mixture was heated to 100 °C, and stirred and reacted at the same temperature overnight. The mixture was cooled to room temperature, and the lid was carefully opened. Ethyl acetate (50 mL) was added to dilute the reaction solution. The insoluble solid was filtered out, and the filter cake was washed with ethyl acetate (20 mL). The filtrate was concentrated to dryness, and the residue was separated by silica gel column chromatography to give 2.6 g of a yellow solid, with a yield of 92.8%. LC-MS (APCI): m/z = 652.3 $(M+1)^+$.

Step 2: Synthesis of example 119

tert-Butyl 4-(6-(3-amino-4-(4-((5-fluoro-2-methoxybenzoylamino)methyl)phenyl)-1H-pyrazolo[4,3-c]pyridin-7-yl)pyridin-3-yl)piperidine-1-carboxylate (2.6 g, 3.9 mmol) and dichloromethane (20 mL) were added to a 100 mL single-necked flask equipped with magnetic stirring. The mixture was stirred until dissolved, and a solution of hydrogen chloride in ethyl acetate (20 mL, 4 M) was added. The mixture was reacted at room temperature under nitrogen for 2 hours. The mixture was concentrated to dryness under reduced pressure to give 2.6 g of a yellowish-green solid, with a yield of 100%. LC-MS (APCI): m/z = 552.3 $(M+1)^+$.

$^1$H NMR (400MHz, DMSO-$d_6$) δ (ppm): 12.40 (br s, 1H), 8.96 (s, 1H), 8.90 (t, J = 6.0 Hz, 1H), 8.68 (d, J = 1.2 Hz, 1H), 8.18 (d, J = 8.4 Hz, 1H), 7.86 (dd, J = 8.4 Hz, J = 2.4 Hz, 1H), 7.72-7.69 (m, 3H), 7.55-7.51 (m, 3H), 7.35-7.30 (m, 1H), 7.19-7.16 (m, 1H), 4.79 (s, 2H), 4.61 (d, J = 6.0 Hz, 2H), 3.89 (s, 3H), 3.42-3.35 (m, 4H), 3.06-2.96 (m, 3H), 2.04-1.95 (m, 3H).

**<u>Example 120 Preparation of compound N-(4-(3-amino-7-(5-(1-(azetidin-3-yl)piperidin-4-yl)pyridin-2-yl)-1H-pyrazolo[4,3-c]pyridin-4-yl)benzyl)-5-fluoro-2-methoxybenzamide</u>**

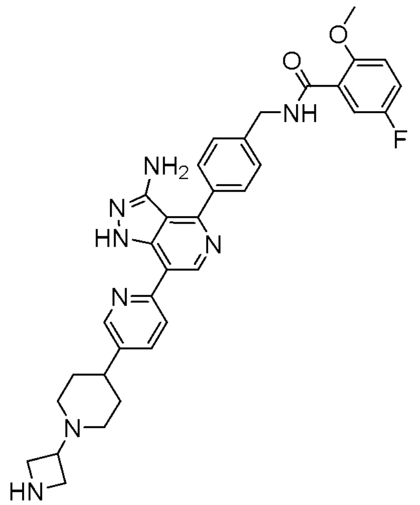

**The following synthetic route was adopted**

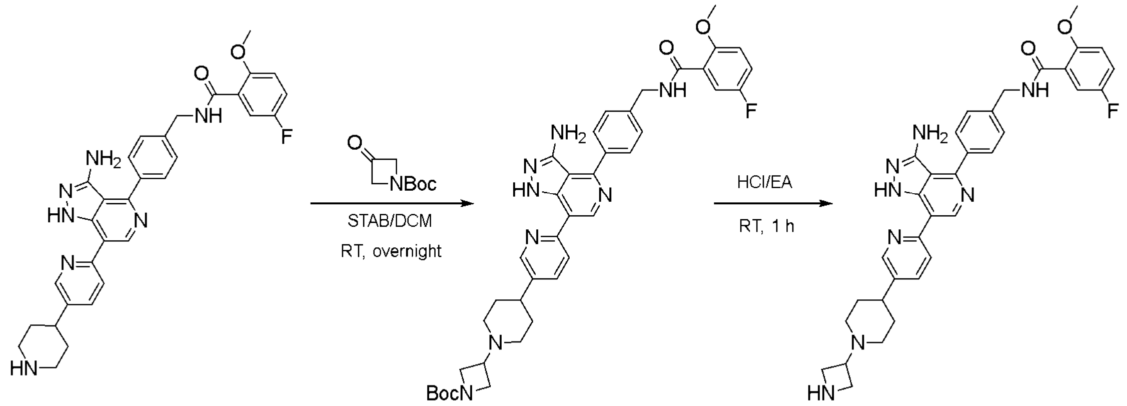


Step 1: Synthesis of compound tert-butyl 3-(4-(6-(3-amino-4-(4-((5-fluoro-2-methoxybenzoylamino)methyl)phenyl)-1H-pyrazolo[4,3-c]pyridin-7-yl)pyridin-3-yl)piperidin-1-yl)azetidine-1-carboxylate

Example 119 (165 mg, 0.30 mmol), 1-tert-butoxycarbonyl-3-azetidinone (2.0 g, 11.7 mmol), and dichloromethane (5 mL) were successively added to a 50 mL round-bottom flask. The mixture was stirred until dissolved. Glacial acetic acid (102 mg, 0.60 mmol) and dichloromethane (5 mL) were added. Powdered 4A molecular sieve (200 mg) was added. The mixture was cooled in an ice-water bath. Sodium triacetoxyborohydride (127 mg, 0.60 mmol) was added. After the addition was completed, the ice bath was removed, and the mixture was reacted at room temperature under nitrogen overnight. The reaction was completed, and saturated aqueous solution of sodium bicarbonate (15 mL) was added to quench the reaction. The mixture was extracted with dichloromethane (10 mL × 3). The organic phases were combined, dried over anhydrous sodium sulfate, filtered, concentrated, and separated by silica gel column chromatography to give 120 mg of a yellowish-green solid, with a yield of 56.6%. LC-MS: m/z = 707.2 $(M+1)^+$.

Step 2: Synthesis of example 120

tert-Butyl 3-(4-(6-(3-amino-4-(4-((5-fluoro-2-methoxybenzoylamino)methyl)phenyl)-1H-pyrazolo[4,3-c]pyridin-7-yl)pyridin-3-yl)piperidin-1-yl)azetidine-1-carboxylate (120 mg, 0.17 mmol) and ethyl acetate (2 mL) were successively added to a 50 mL round-bottom flask. The mixture was stirred until dissolved, and cooled in an ice-water bath. A solution of hydrogen chloride in ethyl acetate (4 M, 5 mL) was added. After the addition was completed, the ice bath was removed, and the mixture was reacted at room temperature under nitrogen for 1 hour. The reaction was completed, and the mixture was concentrated to dryness under reduced pressure. Saturated aqueous solution of sodium bicarbonate (2 mL) and dichloromethane (5 mL) were added. The organic phase was separated, and the aqueous phase was extracted with dichloromethane (5 mL × 2). The organic phases were combined, dried over anhydrous sodium sulfate, filtered, and concentrated to give 95 mg of a yellowish-green solid, with a yield of 92.2%. LC-MS: m/z = 607.2 $(M+1)^+$.

$^1$H NMR (400MHz, DMSO-$d_6$) δ (ppm): 12.40 (br s, 1H), 8.96 (s, 1H), 8.91 (t, J = 6.0 Hz, 1H), 8.66 (s, 1H), 8.18 (d, J = 8.4 Hz, 1H), 7.85 (d, J = 8.4 Hz, 1H), 7.72 (d, J = 8.0 Hz, 2H), 7.55-7.51 (m, 3H), 7.38-7.33 (m, 1H), 7.22-7.19 (m, 1H), 4.82 (s, 2H), 4.63 (d, J = 6.0 Hz, 2H), 3.89 (s, 3H), 3.73-3.45 (m, 5H), 2.88-2.85 (m, 2H), 2.68-2.63 (m, 1H), 1.92-1.83 (m, 4H), 1.76-1.71 (m, 2H).

**Example 121 Preparation** of **compound N-(4-(7-(5-(1-([1,3'-biazetidin]-3-yl)piperidin-4-yl)pyridin-2-yl)-3-amino-1H-pyrazolo[4,3-c]pyridin-4-yl)benzyl)-5-fluoro-2-methoxybenzamide**

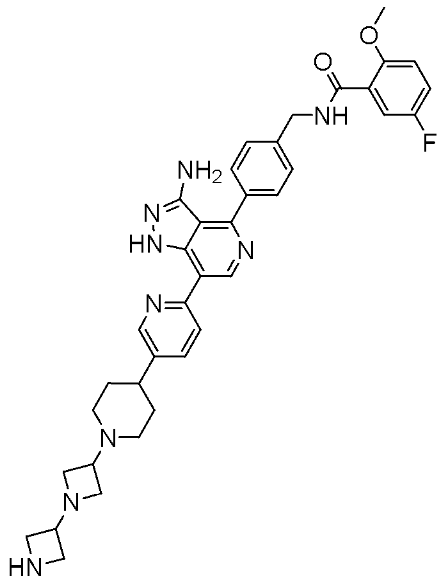

The compound was prepared using example 120 instead of example 119 as the raw material according to the preparation method described in example 120.

**Example 122 Preparation of compound N-(4-(3-amino-7-(5-(piperazin-1-yl)pyridin-2-yl)-1H-pyrazolo[4,3-c]pyridin-4-yl)benzyl)-5-fluoro-2-methoxybenzamide**

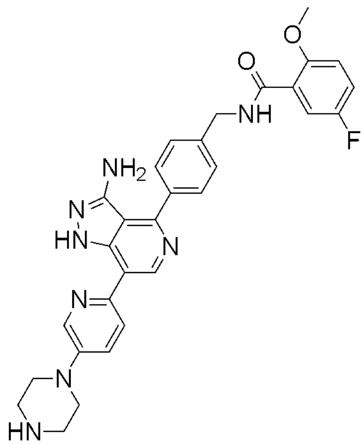

The **following** synthetic route was **adopted**

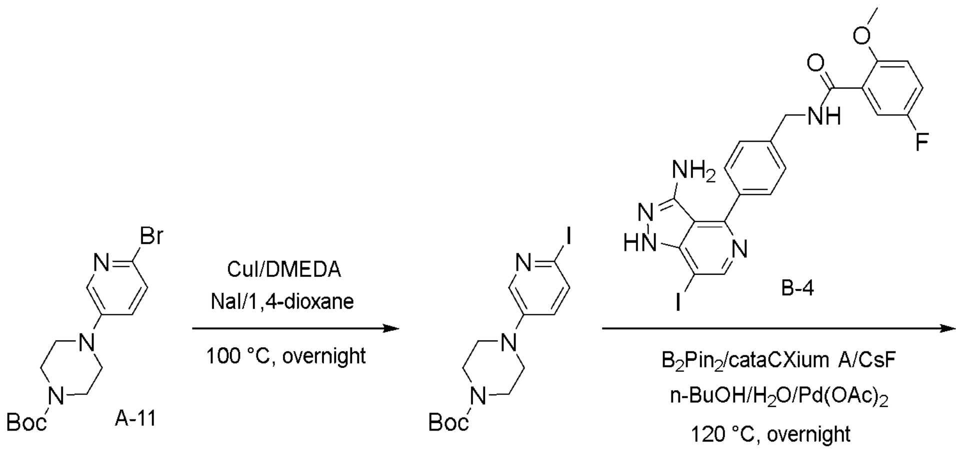


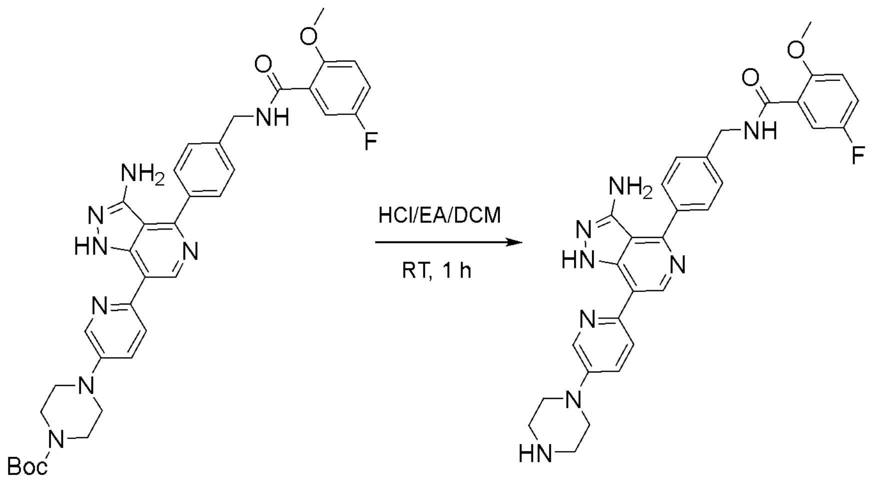


Step 1: Synthesis of compound tert-butyl 4-(6-iodopyridin-3-yl)piperazine-1-carboxylate

Intermediate A-11 (20.00 g, 58.56 mmol), copper(I) iodide (1.12 g, 5.86 mmol), sodium iodide (17.58 g, 117.28 mmol), N,N'-dimethylethylenediamine (1.03 g, 11.72 mmol), and 1,4-dioxane (200 mL) were successively added to a 500 mL three-necked flask equipped with magnetic stirring and a condenser. The reaction system was evacuated and purged with nitrogen three times. The mixture was stirred to disperse, and stirred and reacted at 100 °C overnight. The mixture was cooled to room temperature, and saturated brine (200 mL) was added to quench the reaction. The organic phase was separated, and the aqueous phase was extracted with ethyl acetate (200 mL × 2). The organic phases were combined, washed with saturated brine (200 mL), dried over anhydrous sodium sulfate, filtered, concentrated, and separated by silica gel column chromatography to give 17.60 g of a white solid, with a yield of 77.1%. LC-MS (APCI): m/z = 390.1 $(M+1)^+$.

Step 2: Synthesis of compound tert-butyl 4-(6-(3-amino-4-(4-((5-fluoro-2-methoxybenzoylamino)methyl)phenyl)-1H-pyrazolo[4,3-c]pyridin-7-yl)pyridin-3-yl)piperazine-1-carboxylate

Intermediate B-4 (7.80 g, 15.08 mmol), tert-butyl 4-(6-iodopyridin-3-yl)piperazine-1-carboxylate (17.60 g, 45.24 mmol), di(1-adamantyl)-n-butylphosphine (2.16 g, 6.04 mmol), palladium(II) acetate (678.02 mg, 3.02 mmol), bis(pinacolato)diboron (15.32 g, 60.32 mmol), and cesium fluoride (9.16 g, 60.32 mmol) were successively added to a 250 mL three-necked flask equipped with magnetic stirring and a condenser. The reaction system was evacuated and purged with nitrogen three times. n-Butanol (100 mL) and water (20 mL) were added, and the mixture was stirred to disperse. The mixture was stirred and reacted at 120 °C overnight. The mixture was cooled to room temperature, and saturated brine (100 mL) was added to quench the reaction. The organic phase was separated, and the aqueous phase was extracted with ethyl acetate (100 mL × 2). The organic phases were combined, washed with saturated brine (100 mL), dried over anhydrous sodium sulfate, filtered, concentrated, and separated by silica gel column chromatography to give 2.66 g of a yellow solid, with a yield of 27.1%. LC-MS (APCI): m/z = 653.3 $(M+1)^+$.

Step 3: Synthesis of example 122

tert-Butyl 4-(6-(3-amino-4-(4-((5-fluoro-2-methoxybenzoylamino)methyl)phenyl)-1H-pyrazolo[4,3-c]pyridin-7-yl)pyridin-3-yl)piperazine-1-carboxylate (2.66 g, 4.08 mmol), dichloromethane (30 mL), and a solution of hydrogen chloride in ethyl acetate (20 mL, 4 M) were successively added to a 100 mL single-necked flask equipped with magnetic stirring. The mixture was stirred to disperse. The mixture was stirred and reacted at room temperature for 1 hour. The mixture was concentrated to give 2.64 g of a yellow solid, with a yield of 99.9%. LC-MS (APCI): m/z = 553.3 $(M+1)^+$.

**Example 123 Preparation of compound N-(4-(3-amino-7-(5-(4-(azetidin-3-yl)piperazin-1-yl)pyridin-2-yl)-1H-pyrazolo[4,3-c]pyridin-4-yl)benzyl)-5-fluoro-2-methoxybenzamide**

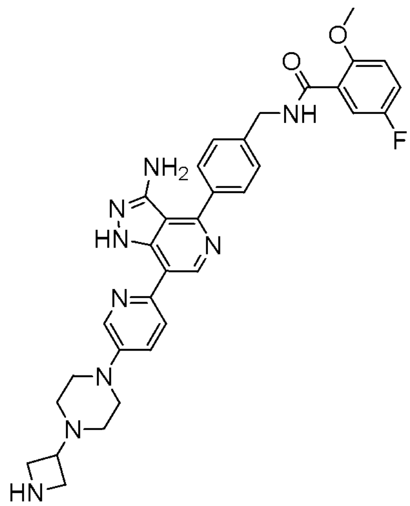

1.36 g of a yellow solid was prepared using example 122 (2.40 g, 4.08 mmol) instead of example 119 as the raw material according to the preparation method described in example 120. LC-MS (APCI): m/z = 608.3 $(M+1)^+$.

**Example 124 Preparation of compound N-(4-(7-(5-(4-([1,3'-biazetidin]-3-yl)piperazin-1-yl)pyridin-2-yl)-3-amino-1H-pyrazolo[4,3-c]pyridin-4-yl)benzyl)-5-fluoro-2-methoxybenzamide**

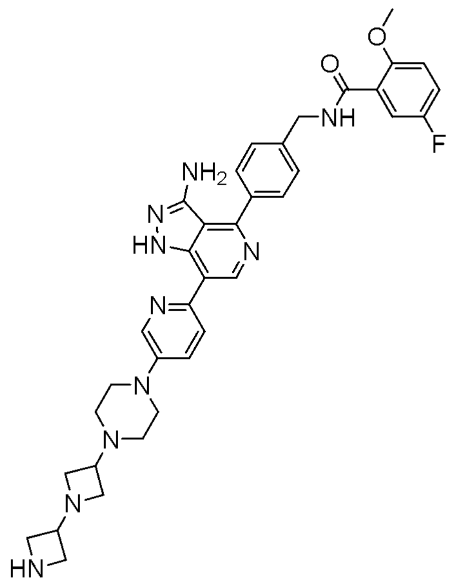

442.3 mg of a yellow solid was prepared using example 123 (1.12 g, 1.65 mmol) instead of example 119 as the raw material according to the preparation method described in example 120. LC-MS (APCI): m/z = 663.4 $(M+1)^+$.

**Example 125 Preparation of compound N-(4-(3-amino-7-(5-(piperazin-1-ylmethyl)pyridin-2-yl)-1H-pyrazolo[4,3-c]pyridin-4-yl)benzyl)-5-fluoro-2-methoxybenzamide**

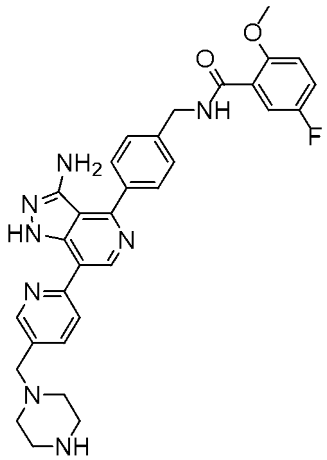

400 mg of a yellow solid was prepared using intermediate A-12 (2.0 g, 5.602 mmol) instead of intermediate A-11 as the raw material according to the preparation method described in example 122. LC-MS (APCI): m/z = 567.3 $(M+1)^+$.

**Example 126 Preparation of compound N-(4-(3-amino-7-(5-((4-(azetidin-3-yl)piperazin-1-yl)methyl)pyridin-2-yl)-1H-pyrazolo[4,3-c]pyridin-4-yl)benzyl)-5-fluoro-2-methoxybenzamide**

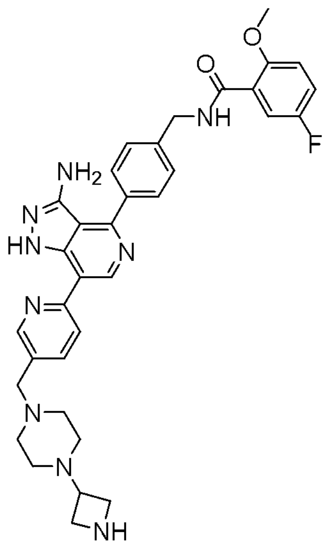

1.0 g of a yellow solid was prepared using example 125 (2.0 g, 3.54 mmol) instead of example 119 as the raw material according to the preparation method described in example 120. LC-MS (APCI): m/z = 622.3 $(M+1)^+$.

**Example 127 Preparation of compound N-(4-(7-(5-((4-([1,3'-biazetidin]-3-yl)piperazin-1-yl)methyl)pyridin-2-yl)-3-amino-1H-pyrazolo[4,3-c]pyridin-4-yl)benzyl)-5-fluoro-2-methoxybenzamide**

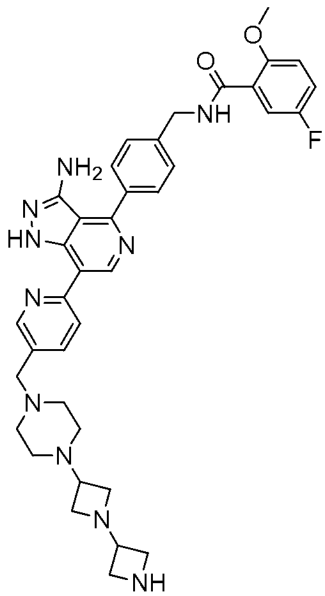

A yellow solid was prepared using example 126 (1.0 g, 1.61 mmol) instead of example 119 as the raw material according to the preparation method described in example 120. LC-MS (APCI): m/z = 677.4 $(M+1)^+$.

**Example 128 Preparation of compound N-(4-(6-(1-([1,3'-biazetidin]-3-yl)piperidin-4-yl)-3-amino-1H-pyrazolo [4,3-c]pyridin-4-ylbenzyl)-5-fluoro-2-methoxybenzamide hydrochloride**

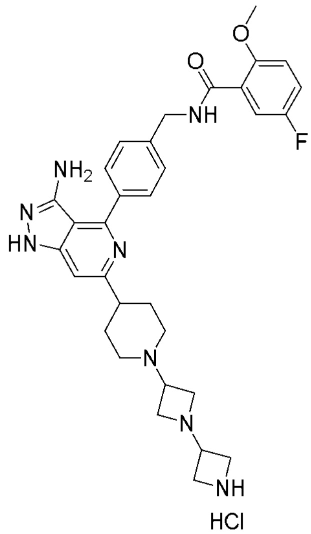

The **following synthetic route was adopted**

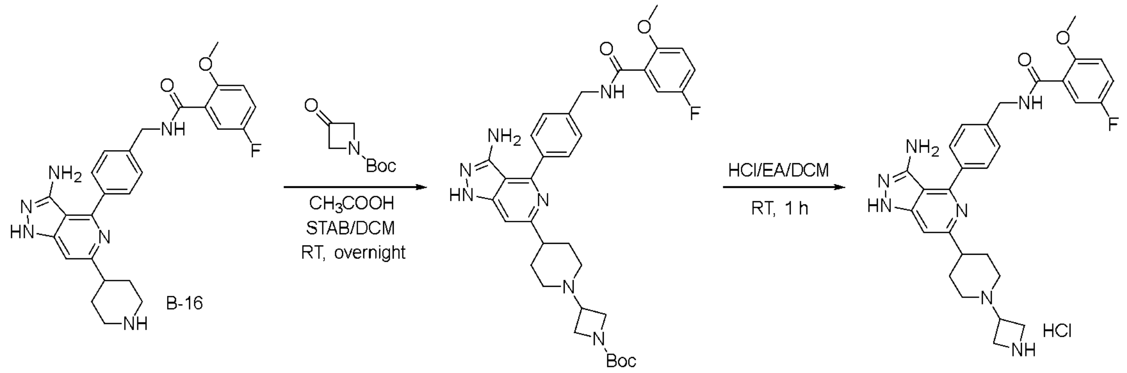


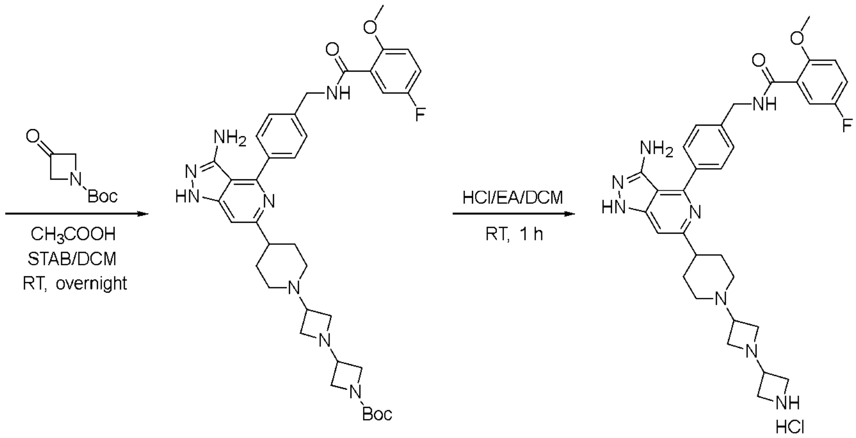


Step 1: Synthesis of compound tert-butyl 3-(4-(3-amino-4-(4-((5-fluoro-2-methoxybenzoylamino)methyl)phenyl)-1H-pyrazolo[4,3-c]pyridin-6-yl)piperidin-1-yl)azetidine-1-carboxylate

Intermediate B-16 (200 mg, 0.42 mmol), N-tert-butoxycarbonyl-3-azetidinone (108 mg, 0.63 mmol), sodium triacetoxyborohydride (267 mg, 1.26 mmol), acetic acid (76 mg, 1.26 mmol), and dichloromethane (10 mL) were successively added to a container. The mixture was stirred until dissolved, and stirred at room temperature overnight. The mixture was diluted with added water, and the resulting mixture was extracted with dichloromethane (50 mL $\times$ 3). The organic phase was washed with saturated brine (100 mL), dried over anhydrous sodium sulfate, concentrated, and separated by silica gel column chromatography to give 215 mg of a light yellow solid, with a yield of 80%. ESI-MS: 630 [$M^+$ +1].

Step 2: Synthesis of compound N-(4-(3-amino-6-(1-(azetidin-3-yl)piperidin-4-yl)-1H-pyrazolo[4,3-c]pyridin-4-yl)benzyl)-5-fluoro-2-methoxybenzamide hydrochloride

tert-Butyl 3-(4-(3-amino-4-(4-((5-fluoro-2-methoxybenzoylamino)methyl)phenyl)-1H-pyrazolo[4,3-c]pyridin-6-yl)piperidin-1-yl)azetidine-1-carboxylate and dichloromethane (10 mL) were successively added to a container, and the mixture was stirred until dissolved. A solution of hydrogen chloride in ethyl acetate (5 mL, 4 M) was added, and the mixture was stirred at room temperature for 1 hour. The mixture was concentrated to dryness to give a white solid, which was directly used in the subsequent reactions. ESI-MS: 530 [$M^+$+1].

Step 3: Synthesis of compound tert-butyl 3-(4-(3-amino-4-(4-((5-fluoro-2-methoxybenzoylamino)methyl)phenyl)-1H-pyrazolo[4,3-c]pyridin-6-yl)piperidin-1-yl)-[1,3'-biazetidine]-1'-carboxylate

N-(4-(3-Amino-6-(1-(azetidin-3-yl)piperidin-4-yl)-1H-pyrazolo[4,3-c]pyridin-4-yl)benzyl)-5-fluoro-2-methoxybenzamide hydrochloride, N-tert-butoxycarbonyl-3-azetidinone (108 mg, 0.63 mmol), sodium triacetoxyborohydride (267 mg, 1.26 mmol), acetic acid (76 mg, 1.26 mmol), and dichloromethane (10 mL) were successively added to a container. The mixture was stirred until dissolved, and stirred at room temperature overnight. The mixture was diluted with added water, and the resulting mixture was extracted with dichloromethane (50 mL $\times$ 3). The organic phase was washed with saturated brine (100 mL), dried over anhydrous sodium sulfate, concentrated, and separated by silica gel column chromatography to give 150 mg of a light yellow solid, with a yield of 64%. ESI-MS: 685 [$M^+$+1].

Step 4: Synthesis of example 128

tert-Butyl 3-(4-(3-amino-4-(4-((5-fluoro-2-methoxybenzoylamino)methyl)phenyl)-1H-pyrazolo[4,3-c]pyridin-6-yl)piperidin-1-yl)-[1,3'-biazetidine]-1'-carboxylate and dichloromethane (10 mL) were successively added to a container, and the mixture was stirred until dissolved. A solution of hydrogen chloride in ethyl acetate (5 mL, 4 M) was added. The mixture was stirred at room temperature for 1 hour, and concentrated to dryness to give a white solid. ESI-MS: 585 [$M^{+}$+1].

**Example 129 Preparation of compound N-(4-(3-amino-7-(4,5,6,7-tetrahydropyrazolo[1,5-a]pyrazin-2-yl)-1H-pyrazolo[4,3-c]pyridin-4-yl)benzyl)-5-fluoro-2-methoxybenzamide**

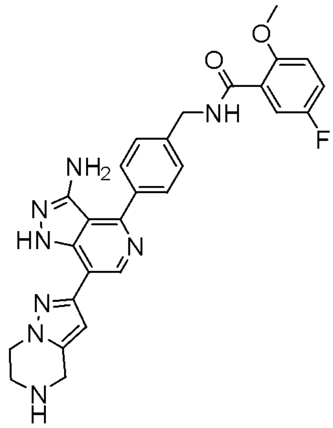

The **following** synthetic route was **adopted**

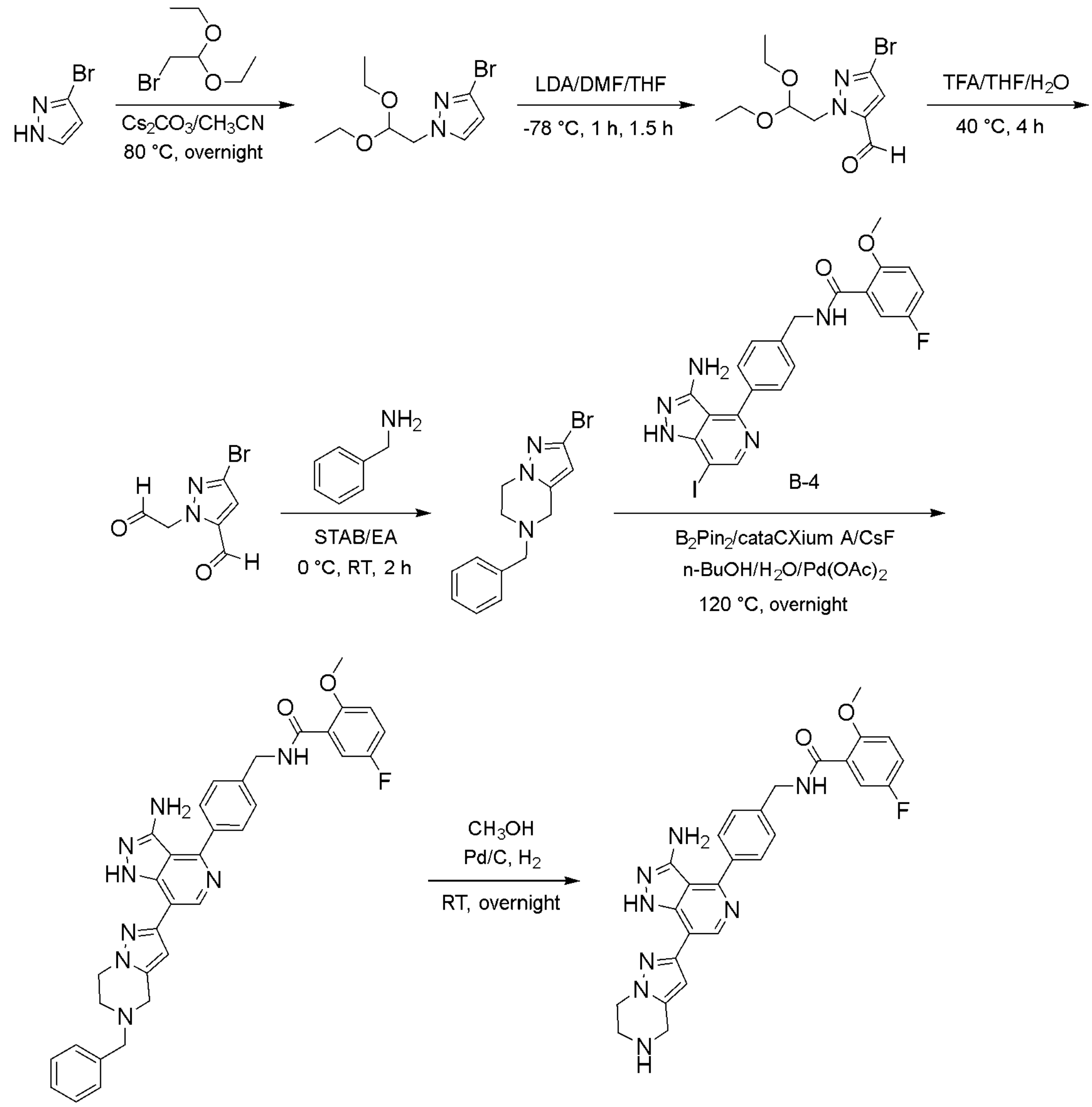


Step 1: Synthesis of compound 3-bromo-1-(2,2-diethoxyethyl)-1H-pyrazole

3-Bromopyrazole (1.0 g, 6.8 mmol), bromoacetaldehyde diethyl acetal (1.41 g, 7.14 mmol), cesium carbonate (3.48 g, 10.7 mmol), and acetonitrile (20 mL) were successively added to a container, and the mixture was stirred until dissolved. The mixture was heated to 80 °C, and reacted overnight. The reaction solution was cooled to room temperature, and filtered. The filter cake was washed with ethyl acetate twice. The filtrate was concentrated and separated by silica gel column chromatography to give 1.7 g of a colorless, transparent oil, with a yield of 95%. LC-MS (APCI): m/z = 263.1 $(M+1)^+$.

Step 2: Synthesis of compound 3-bromo-1-(2,2-diethoxyethyl)-1H-pyrazole-5-carbaldehyde

3-Bromo-1-(2,2-diethoxyethyl)-1H-pyrazole (1.7 g, 6.48 mmol) and anhydrous tetrahydrofuran (30 mL) were successively added to a container, and the mixture was stirred until dissolved. The mixture was cooled to -78 °C, and lithium diisopropylamide (5 mL, 10 mmol, 2 M) was slowly added dropwise. The mixture was reacted at -78 °C for 1 hour. Then, N,N-dimethylformamide (850 mg, 11.6 mmol) was dissolved in anhydrous tetrahydrofuran (10 mL), and this solution was slowly added dropwise to the above reaction solution at -78 °C. The resulting mixture was reacted at -78 °C for 1.5 hours. Saturated ammonium chloride solution was added to quench the reaction, and the mixture was diluted with added water at room temperature. The mixture was extracted with methyl tert-butyl ether (100 mL $\times$ 3). The organic phase was washed with saturated brine, dried over anhydrous sodium sulfate, concentrated, and separated by silica gel column chromatography to give 1.57 g of a light yellow oil, with a yield of 83%. LC-MS (APCI): m/z = 291.1 $(M+1)^+$.

Step 3: Synthesis of compound 3-bromo-1-(2-oxoethyl)-1H-pyrazole-5-carbaldehyde

3-Bromo-1-(2,2-diethoxyethyl)-1H-pyrazole-5-carbaldehyde (1.57 g, 5.4 mmol), trifluoroacetic acid (6 mL), tetrahydrofuran (3 mL), and water (3 mL) were successively added to a container, and the mixture was stirred until dissolved. The solution was heated to 40 °C, and reacted for 4 hours. The mixture was cooled to room temperature, and the reaction solution was concentrated. The resulting mixture was azeotropically refluxed with toluene (10 mL × 3) three times to remove water. The resulting mixture was concentrated and then directly used in the next step reaction. LC-MS (APCI): m/z = 191.1 $(M+1)^+$.

Step 4: Synthesis of compound 5-benzyl-2-bromo-4,5,6,7-tetrahydropyrazolo[1,5-a]pyrazine

3-Bromo-1-(2-oxoethyl)-1H-pyrazole-5-carbaldehyde (the concentrated solution from the previous step reaction) and ethyl acetate (50 mL) were successively added to a container, and the mixture was cooled to 0 °C in an ice bath. Benzylamine (694 mg, 6.48 mmol) was slowly added dropwise, and then sodium triacetoxyborohydride (3.4 g, 16.2 mmol) was added. The mixture was stirred and reacted at room temperature for 2 hours. The mixture was diluted with added water (30 mL). The resulting mixture was extracted with ethyl acetate (20 **mL** × 3). The organic phase was washed with saturated brine, dried over anhydrous sodium sulfate, concentrated, and separated by silica gel column chromatography to give 475 mg of a light yellow oil, with a yield of 30.2%. LC-MS (APCI): m/z = 292.1 $(M+1)^+$.

Step 5: Synthesis of compound N-(4-(3-amino-7-(5-benzyl-4,5,6,7-tetrahydropyrazolo[1,5-a]pyrazin-2-yl)-1H-pyrazolo [4,3-c]pyridin-4-yl)benzyl)-5-fluoro-2-methoxybenzamide

5-Benzyl-2-bromo-4,5,6,7-tetrahydropyrazolo[1,5-a]pyrazine (475 mg, 1.63 mmol), intermediate B-4 (450 mg, 0.87 mmol), di(1-adamantyl)-n-butylphosphine (129 mg, 0.36 mmol), bis(pinacolato)diboron (555 mg, 2.19 mmol), palladium(II) acetate (42 mg, 0.18 mmol), cesium fluoride (540 mg, 3.6 mmol), water (3 mL), and n-butanol (15 mL) were successively added to a container. The reaction system was purged with nitrogen three times. The mixture was heated to 120 °C under nitrogen, and refluxed and reacted overnight. The reaction solution was cooled to room temperature, concentrated, and separated by silica gel column chromatography to give 110 mg of a light yellow solid, with a yield of 21.0%. LC-MS (APCI): m/z = 603.1 $(M+1)^+$.

Step 5: Synthesis of example 129

N-(4-(3-Amino-7-(5-benzyl-4,5,6,7-tetrahydropyrazolo[1,5-a]pyrazin-2-yl)-1H-pyrazolo[4,3-c]pyridin-4-yl)benzyl)-5-fluoro-2-methoxybenzamide (110 mg, 0.18 mmol) and methanol (10 mL) were successively added to a container, and the mixture was stirred until dissolved. Palladium on carbon (100 mg, 10%) was added, and the reaction system was purged with hydrogen three times. The mixture was stirred at room temperature under hydrogen overnight, and filtered. The filtrate was concentrated to give 80 mg of a colorless, transparent solid, with a yield of 86.9%. LC-MS (APCI): m/z = 513.1 $(M+1)^+$.

**Example 130 Preparation of compound N-(4-(3-amino-7-(5-(1-methylpiperidin-4-yl)pyridin-2-yl)-1H-pyrazolo [4,3-c]pyridin-4-yl)benzyl)-5-fluoro-2-methoxybenzamide**

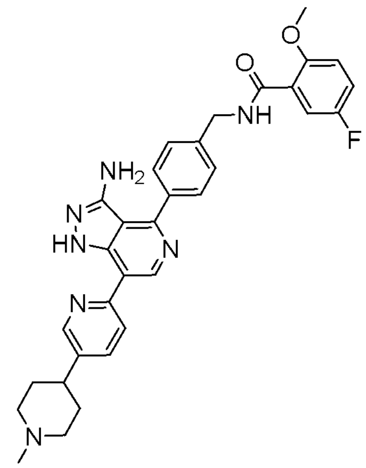

The **following** synthetic route was **adopted**

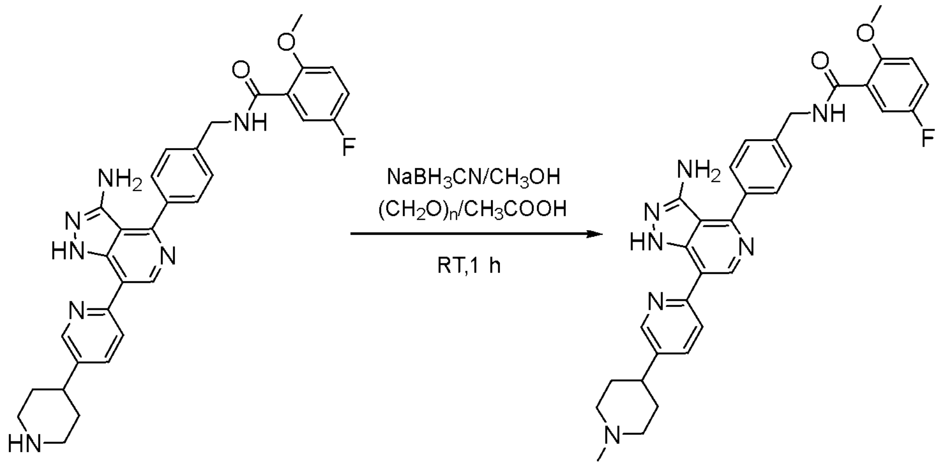


Example 119 (55 mg, 0.10 mmol) and methanol (3 mL) were successively added to a 50 mL roundbottom flask, and the mixture was stirred until dissolved. The mixture was cooled in an ice-water bath. Glacial acetic acid (9 mg, 0.15 mmol) and paraformaldehyde (6 mg, 0.20 mmol) were added, and then sodium cyanoborohydride (12 mg, 0.20 mmol) was added. After the addition was completed, the ice bath was removed. The mixture was reacted at room temperature under nitrogen for 1 hour. The reaction was completed, and saturated aqueous solution of sodium bicarbonate (15 mL) was added to quench the reaction. The mixture was extracted with ethyl acetate (10 mL × 3). The organic phases were combined, dried over anhydrous sodium sulfate, filtered, concentrated, and separated by silica gel column chromatography to give 30 mg of a yellowish-green solid, with a yield of 53.1%. LC-MS: m/z = 566.2 $(M+1)^+$.

$^1H$ NMR (400MHz, DMSO-$d_6$) δ (ppm):12.39 (br s, 1H), 8.96-8.89 (m, 2H), 8.66 (s, 1H), 8.20-8.18 (m, 1H), 7.84-7.82 (m, 1H), 7.72-7.69 (m, 2H), 7.55-7.51 (m, 3H), 7.35-7.30 (m, 1H), 7.21-7.19 (m, 1H), 4.81 (s, 2H), 4.62-4.60 (m, 2H), 3.91 (s, 3H), 3.07-3.02 (m, 2H), 2.74-2.69 (m, 2H), 2.38-2.33 (m, 3H), 1.98-1.75 (m, 5H).

**Example** 131 **Preparation** of **compound N-(4-(3-amino-7-(5-(1-(1-methylazetidin-3-yl)piperidin-4-yl)pyridin-2-yl)-1H-pyrazolo[4,3-c]pyridin-4-yl)benzyl)-5-fluoro-2-methoxybenzamide**

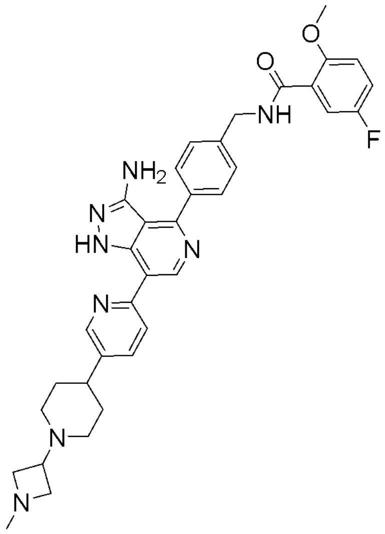

25 mg of a yellowish-green solid was prepared using example 120 (61 mg, 0.10 mmol) instead of example 119 as the raw material according to the preparation method described in example 130, with a yield of 40.3%. LC-MS (APCI): m/z = 677.4 $(M+1)^+$.

$^1H$ NMR(400MHz, DMSO-d6) δ (ppm): 12.37 (br s, 1H), 8.96 (s, 1H), 8.90 (t, J = 6.0 Hz, 1H), 8.67 (s, 1H), 8.18 (d, J = 8.4 Hz, 1H), 7.85 (d, J = 8.4 Hz, 1H), 7.72 (d, J = 8.0 Hz, 2H), 7.55-7.51 (m, 3H), 7.38-7.33 (m, 1H), 7.22-7.19 (m, 1H), 4.81 (s, 2H), 4.63 (d, J = 6.0 Hz, 2H), 3.92 (s, 3H), 3.69-3.62 (m, 4H), 3.00-2.96 (m, 2H), 2.89-2.86 (m, 4H), 2.68 (s, 3H), 2.03-1.96 (m, 4H), 1.92-1.84 (m, 2H).

**Preparation of example 132 to example 141**

A corresponding example was prepared using the corresponding raw material in Table A instead of example 119 as the raw material according to the preparation method described in example 130.

Table A

| Example | Compound and its name | Raw material |
|---|---|---|
| 132 | 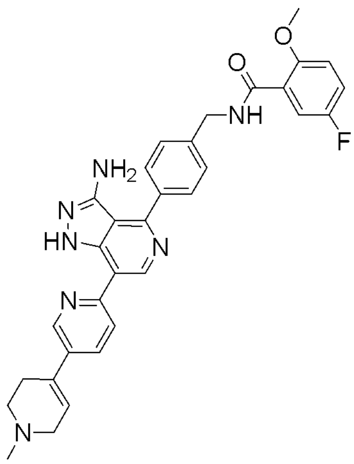 N-(4-(3-Amino-7-(1'-methyl-1',2',3',6'-tetrahydro-[3,4'-bipyridin]-6-yl)-1H-pyrazolo[4,3-c]pyridin-4-yl)benzyl)-5-fluoro-2-methoxybenzamide | 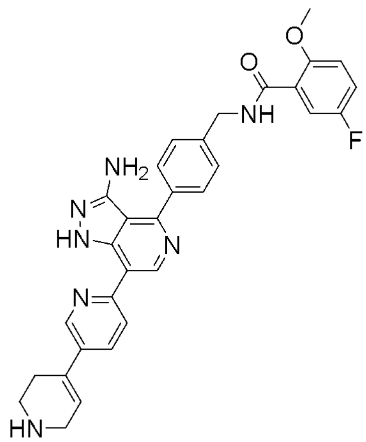 Example 118 |

(continued)

| Example | Compound and its name | Raw material |
| --- | --- | --- |
| 133 | 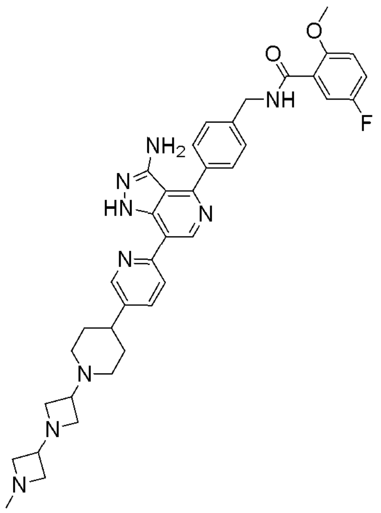 N-(4-(3-Amino-7-(5-(1-(1'-methyl-[1,3'-biazetidin]-3-yl)piperidin-4-yl)pyridin-2-yl)-1H-pyrazolo[4,3-clpyridin-4-yl)benzyl)-5-fluoro-2-methoxybenzamide | 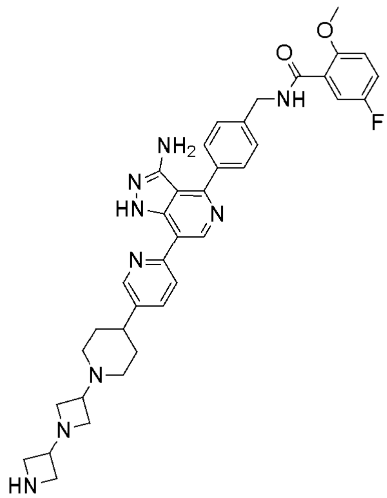 Example 121 |
| 134 | 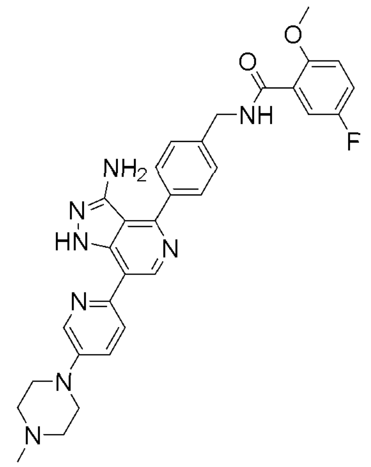 N-(4-(3-Amino-7-(5-(4-methylpiperazin-1-yl)pyridin-2-yl)-1H-pyrazolo[4,3-c]pyridin-4-yl)benzyl)-5-fluoro-2-methoxybenzamide | 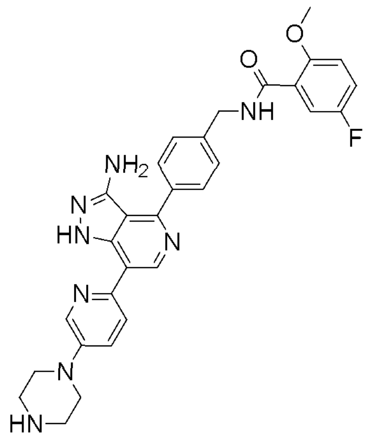 Example 122 |

(continued)

| Example | Compound and its name | Raw material |
|---|---|---|
| 135 | 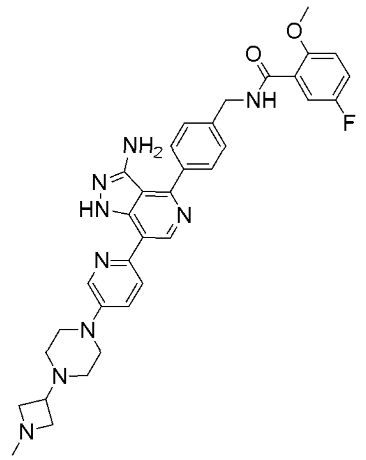 N-(4-(3-Amino-7-(5-(4-(1-methylazetidin-3-yl)piperazin-1-yl) pyridin-2-yl)-1H-pyrazolo[4,3-c]pyridin-4-yl)benzyl)-5-fluoro-2-methoxybenzamide | 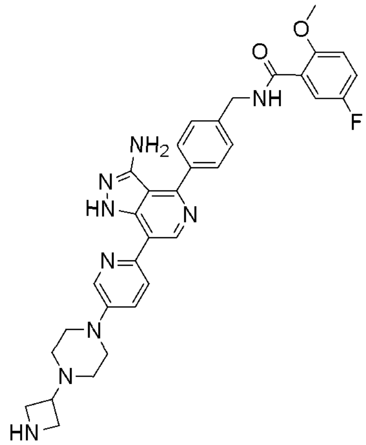 Example 123 |
| 136 | 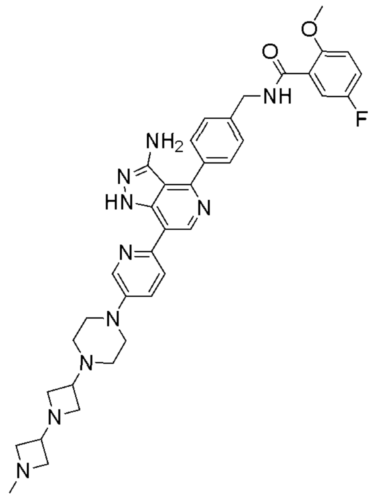 N-(4-(3-Amino-7-(5-(4-(1'-methyl-[1,3'-diazetidin]-3-yl)piperazin-1-yl)pyridin-2-yl)-1H-pyrazolo[4,3-c]pyridin-4-yl)benzyl)-5-fluoro-2-methoxybenzamide | 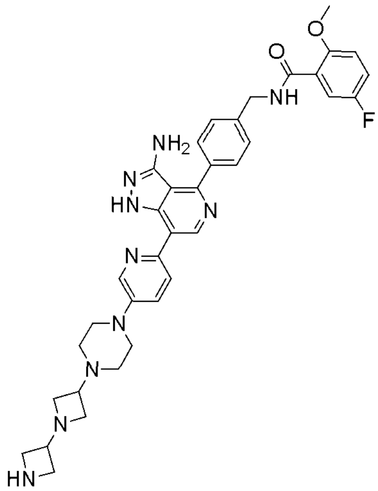 Example 124 |

(continued)

| Example | Compound and its name | Raw material |
|---|---|---|
| 137 | 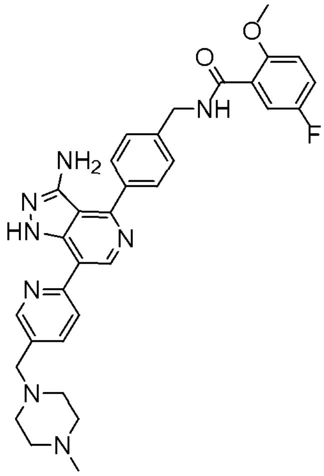 N-(4-(3-Amino-7-(5-((4-methylpiperazin-1-yl)methyl)pyridin-2-yl)-1H-pyrazolo[4,3-c]pyridin-4-yl)benzyl)-5-fluoro-2-methoxybenzamide | 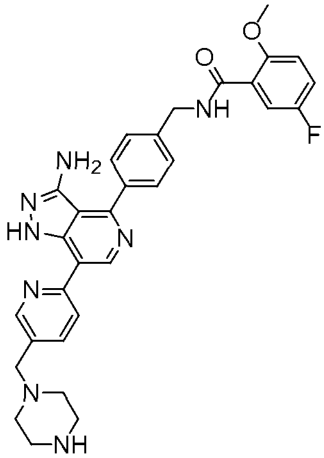 Example 125 |
| 138 | 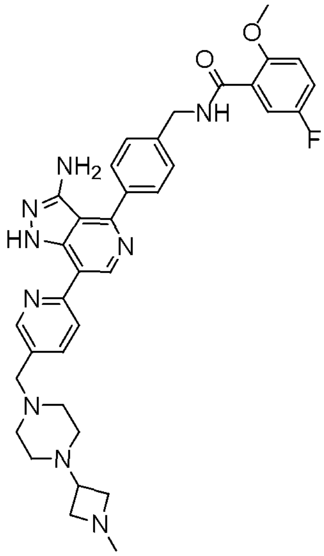 N-(4-(3-Amino-7-(5-((4-(1-methylazetidin-3-yl)piperazin-1-yl)methyl)pyridin-2-yl)-1H-pyrazolo[4,3-c]pyridin-4-yl)benzyl)-5-fluoro-2-methoxybenzamide | 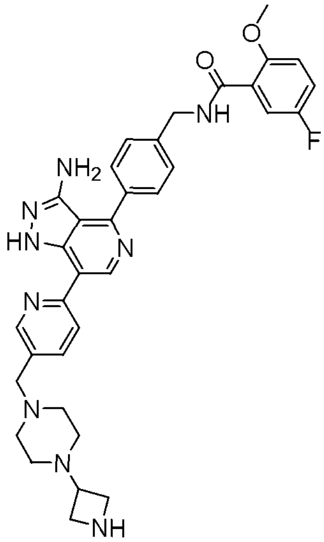 Example 126 |

(continued)

| Example | Compound and its name | Raw material |
|---|---|---|
| 139 | 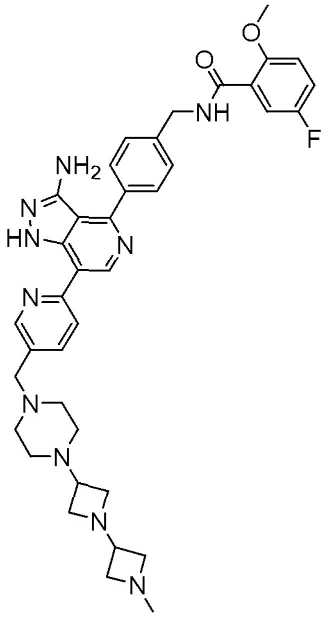 N-(4-(3-Amino-7-(5-((4-(1'-methyl-[1,3'-diazetidin]-3-yl)piperazin-1-yl)methyl)pyridin-2-yl)-1H-pyrazolo[4,3-c]pyridin-4-yl) benzyl)-5-fluoro-2-methoxybenzamide | 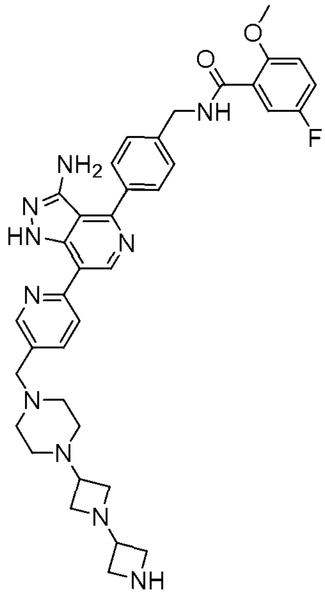 Example 127 |
| 140 | 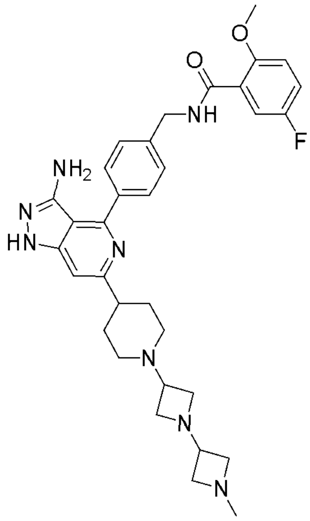 N-(4-(3-Amino-6-(1-(1'-methyl-[1,3'-biazetidin]-3-yl)piperidin-4-yl)-1H-pyrazolo[4,3-c]pyridin-4-yl)benzyl)-5-fluoro-2-methoxybenzamide | 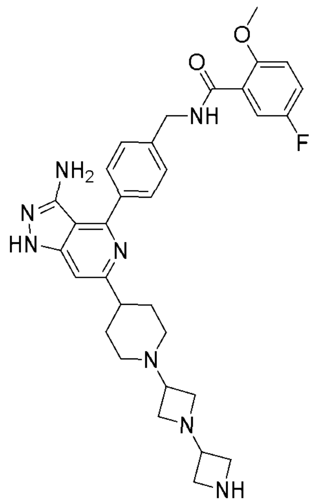 HCl Example 128 |

(continued)

| Example | Compound and its name | Raw material |
|---|---|---|
| 141 | 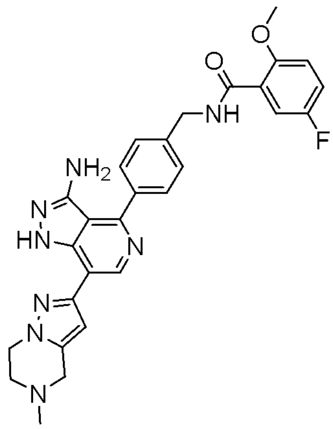 N-(4-(3-Amino-7-(5-methyl-4,5,6,7-tetrahydropyrazolo[1,5-a] pyrazin-2-yl)-1H-pyrazolo[4,3-c]pyridin-4-yl)benzyl)-5-fluoro-2-methoxybenzamide | 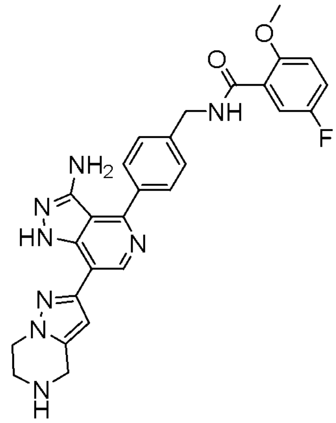 Example 129 |

**Biological activity** assay

(1) Kinase activity inhibition assay

In this assay, the HTRF TK Kit (Catalog No. 62TK0PEC) provided by Cisbio was used to detect the inhibitory activity of compounds on BTK kinase, BTK T474I kinase and BTK C481 S kinase and the selectivity of compounds relative to EGFR, ITK and TEC kinases.

Reagents and consumables: BTK kinase (Invitrogen, Catalog No. PV3587), BTK C481S (Carna, Product No. 08-547), BTK T474I(SignalChem Product No. B10-12HH), ITK (Cama, Product No. 08-181), EGFR (Cama, Product No. 08-115), TEC (Carna, Product No. 08-182), HTRF-TK kit (Cisbio, Product No. 62TK0PEC), $MgCl_2$ (Sigma, Product No. M1028), $MnCl_2$ (Sigma, Product No. M1787), DTT (Sigma, Product No. D0632), ATP (Sigma, Product No. A7699-1g), DMSO (Sigma, Product No. D2650), 384-well plate (PE, Product No. 6008280).

Kinase activity analysis: the required compounds were prepared, and the final concentration of DMSO in the kinase assay system did not exceed 1%. The entire operation steps were carried out on ice, and the total reaction volume was 10μL. A compound (4μL), substrate and ATP (4μL), and kinase (2μL) were added in sequence to a 384-well plate, and the plate was shaken for 30 seconds. The mixture was reacted at room temperature for an appropriate time, and then the detection reagent was added. The resulting mixture was reacted at room temperature for 1 hour, and then the plate was assayed with a microplate reader. The specific steps were as follows:

a) a 1 × kinase buffer solution required for the kinase assay was calculated according to the amount of the test compound, and prepared as needed;
b) 2.5 × working concentration of a compound was prepared, 100-fold gradient concentration solutions of a compound were diluted by 40 × to the desired concentration, respectively, mixed well, and added to each well at 4 μL per well;
c) 5 × working concentration of the substrate and ATP were prepared, mixed in a ratio of 1:1 before being added to the 384-well plate, and added to each well at 4 μL per well;
d) 5 × working concentration of a kinase was prepared, 2 μL of which was added to each well;
e) the plate was shaken on a microplate shaker for 30 seconds, sealed with a sealing membrane, and the mixture was reacted at room temperature for an appropriate time;
f) 4 × working concentration of streptavidin XL-665 and TK antibody-cryptate compound were prepared, mixed in a ratio of 1:1 before being added to the 384-well plate, and added to each well at 10 μL per well;
g) the mixture was reacted at room temperature for 1 hour, and then the plate was read;
h) the wells with only substrate, ATP and 1% DMSO added but no kinase were blank control wells, and the wells with only substrate, ATP, 1% DMSO and kinase were positive control wells.

Readings: The values of 620 nm (cryptate) and 665 nm (XL665) were read with a BioTek Synerg Neo2 microplate

reader, the ratio of 665/620 for each well was calculated, and the data were exported.

Data analysis: $IC_{50}$ was calculated using GraphPad Prism 7.0 software.

The $IC_{50}$ (half-maximal inhibitory concentration) of the compound was obtained using the following nonlinear fitting formula:

$$Y=Bottom + (Top-Bottom)/(1+10^{\wedge}((LogIC_{50}-X)*HillSlope))$$

X: Log value of the compound concentration
Y: Inhibition rate (% inhibition)
Top: Maximum response
Bottom: Baseline response
HillSlope: Slope of the curve

The formula for calculating kinase inhibition rate (inhibition rate %) was:

Inhibition rate % = (1 - (each concentration well-blank well)/(positive control well-blank well)) * 100%

The assay results show that the compounds of the present disclosure have strong activity against all the wild-type BTK kinase and drug-resistant mutated BTK C481S and BTK T474I kinases and excellent selectivity over EGFR, ITK and TEC kinases. The results of representative example compounds are summarized in Table 1 below, wherein, A represents an $IC_{50} \leq 1$ nM, B represents an $IC_{50}$ of 1-5 nM, C represents an $IC_{50}$ of 5-50 nM, D represents an $IC_{50}$ of 50-500 nM, and E represents an $IC_{50} > 500$ nM.

| Example number | BTK | BTK C481S | BTK T474I | EGFR | ITK | TEC |
|---|---|---|---|---|---|---|
| 1 | C | C | E | D | E | E |
| 2 | B | B | D | D | E | D |
| 3 | B | B | D | E | E | D |
| 6 | B | B | C | D | E | C |
| 6-1 | B | B | C | E | E | D |
| 6-2 | B | B | C | D | E | C |
| 7 | B | B | D | D | E | C |
| 7-1 | B | B | D | E | E | C |
| 7-2 | B | B | D | E | E | D |
| 9 | B | C | D | D | E | D |
| 15 | A | A | D | D | E | C |
| 17 | B | B | D | E | E | C |
| 18 | C | B | D | E | E | D |
| 19 | B | B | D | E | E | D |
| 21 | B | C | D | E | E | D |
| 22 | C | C | D | E | E | E |
| 23 | A | A | D | D | E | C |
| 24 | C | C | D | E | E | D |
| 25 | B | B | D | D | E | D |
| 26 | B | B | D | D | E | D |
| 28 | B | B | D | C | D | C |
| 29 | A | A | C | C | D | C |

(continued)

| Example number | BTK | BTK C481S | BTK T474I | EGFR | ITK | TEC |
|---|---|---|---|---|---|---|
| 30 | B | B | D | D | E | D |
| 31 | B | B | D | D | E | D |
| 35 | A | A | C | D | D | C |
| 36 | B | B | D | D | E | D |
| 37 | A | A | D | D | E | C |
| 38 | C | B | D | D | E | C |
| 39 | A | A | C | D | D | C |
| 41 | B | A | C | D | D | C |
| 42 | B | B | E | E | E | D |
| 43 | A | A | C | C | D | B |
| 44 | A | A | C | C | D | C |
| 45 | C | C | E | D | E | D |
| 46 | B | B | D | C | E | C |
| 47 | B | B | D | D | E | C |
| 48 | B | B | C | D | D | C |
| 50 | B | B | D | D | E | C |
| 51 | B | A | D | D | E | C |
| 52 | A | A | C | D | D | C |
| 53 | B | B | D | D | D | C |
| 54 | B | B | D | D | D | C |
| 56 | B | B | C | D | D | C |
| 57 | B | A | D | D | E | D |
| 58 | B | B | C | D | E | C |
| 59 | A | A | C | C | D | C |
| 60 | A | A | C | C | D | C |
| 61 | A | A | C | C | D | C |
| 62 | B | B | C | C | D | D |
| 65 | B | B | B | C | D | B |
| 66 | A | A | C | C | D | C |
| 67 | A | A | D | C | D | C |
| 68 | B | B | C | C | D | C |
| 69 | A | A | C | C | D | B |
| 70 | B | B | D | D | E | C |
| 71 | B | A | C | C | D | C |
| 72 | A | A | C | C | D | C |
| 73 | A | A | C | C | D | C |
| 74 | B | B | C | C | D | E |
| 75 | B | A | C | C | C | C |
| 76 | A | A | B | C | D | B |

(continued)

| Example number | BTK | BTK C481S | BTK T474I | EGFR | ITK | TEC |
|---|---|---|---|---|---|---|
| 77 | B | B | D | D | E | C |
| 78 | A | A | C | C | D | C |
| 79 | C | C | E | E | E | E |
| 80 | B | B | D | D | E | C |
| 82 | B | B | D | E | E | D |
| 84 | C | C | D | E | E | E |
| 85 | B | B | D | E | E | D |
| 91 | B | B | D | D | E | D |
| 96 | B | B | C | D | E | C |
| 98 | B | B | D | D | E | C |
| 99 | C | C | D | D | E | C |
| 100 | B | B | D | D | E | C |
| 101 | B | B | C | D | D | C |
| 102 | C | C | E | D | E | E |
| 103 | C | C | D | D | E | E |
| 105 | C | D | E | E | E | E |
| 106 | D | D | E | E | E | E |
| 115 | C | B | D | D | E | D |
| 117 | B | B | D | C | E | D |

(2) Viability assay of REC1 cells, DOHH2 cells, Pfeiffer cells, Raji cells, and RPMI-8226 cells.

In this assay, the CellTiter-Glo® luminescent cell viability assay kit (a homogeneous cell viability assay method) provided by Promega was used. The cell viability of cultured cells (REC1 cells: human mantle cell lymphoma cells, DOHH2 cells: human follicular lymphoma cells, Pfeiffer cells: human diffuse large cell lymphoma cells, Raji cells: human Burkitt lymphoma cells, RPMI-8226 cells: human multiple myeloma cells) was measured by quantifying ATP. The specific assay steps were as follows:

a) Cells in logarithmic growth phase were collected. Cell suspension was prepared and the cells were counted. Concentration of the cells was calculated.
b) The cell suspension was diluted to the required concentration and then added to the middle wells of a 96-well plate, and PBS was added to the edge wells. The wells with no cells and only culture medium were used as blank controls.
c) In addition to the cell plate used for compound analysis, another 96-well plate was prepared as a T0 plate, and 3-6 blank wells and 3-6 cell wells were added. The next day, CTG was used to test the cell viability as the T0 viability value of the cells.
d) The cell plate was incubated at 37 °C in a 5% $CO_2$ incubator overnight.
e) The next day, each concentration of a compound subjected to a gradient dilution was added, and the cell plate was incubated at 37 °C in a 5% $CO_2$ incubator for 72 h. The wells with no compound and only 0.5% DMSO were used as positive controls for cell growth.
f) CTG reagent (CellTiter-Glo kit) was added according to the manufacturer's instructions to test the cell viability value.
g) The plate was read using Biotek cytation3.0 microplate reader and the data were exported.
h) $IC_{50}$ was calculated using GraphPad Prism 7.0 software.

The $IC_{50}$ (half-maximal inhibitory concentration) of the compound was obtained using the following nonlinear fitting formula:

$$Y=Bottom + (Top-Bottom)/(1+10^{((LogIC_{50}-X)*HillSlope)})$$

X: Log value of the compound concentration
Y: Inhibition rate (% inhibition)
Top: Maximum response
Bottom: Baseline response
HillSlope: Slope of the curve

The formula for calculating cell inhibition rate (inhibition rate %) was:

Inhibition rate % = 1 - (Lum of drug to be tested - Lum of vehicle control)/(Lum of cell control - Lum of vehicle control) × 100%.

The assay results show that the compounds of the present disclosure have stronger activity on REC1 cells, DOHH2 cells, Pfeiffer cells, Raji cells and RPMI-8226 cells.

(3) Phosphor-BTK(Y223) assay

The purpose of this assay was to simply, quickly and directly detect the level of endogenous BTK phosphorylation in cells.

The steps for handling adherent cells were as follows:

1) NIH/3T3 BTK WT cells (7.5 k/well) and NIH/3T3 BTK C481S cells (10 k/well) were plated in a 96-well plate, respectively, and cultured at 37 °C and 5% $CO_2$ overnight.
2) A 3-fold final concentration gradient dilution of a compound was prepared, and 50 μL of it was taken and added to 100 μL of cell culture. 0.5% DMSO was used as a positive control for cell growth.
3) The plate was incubated at 37 °C and 5% $CO_2$ for 0.5 h.
4) $Na_3VO_4$ at a final concentration of 1mM was added, and the plate was incubated at 37 °C and 5% $CO_2$ for 2 hours.
5) During the incubation, the following reagents were prepared:

1x added lysis buffer: 3 vol ddH2O + 1 vol 4x lysis buffer + 100x blocking reagent.
p-BTK (Y223) cryptate-antibody and p-BTK (Y223) d2-antibody working solutions: 1 volume of each cryopreserved antibody (stored at - 20 °C) was added to 19 times the volume of assay buffer (stored at 4 °C).

6) After the incubation was completed, the medium was removed and 50 μL of 1x added lysis buffer was added. The plate was centrifuged at 500 rpm at room temperature for 30 min.
7) The lysate was mixed well and 16 μL of it was transferred to a 384-well plate. 1 × added lysis buffer was used as a blank control.
8) 4 μL of pre-mixed working solutions of the two antibodies (cryptate Ab:d2 Ab = 1:1) was added, and the plate was sealed.
9) The plate was incubated at room temperature overnight. 10) The plate was read at 665nm and 620nm using Biotek cytation3.0 microplate reader, and the ratio of 665/620nm of each well was calculated.

The steps for handling suspended cells were as follows:

1) Ramos cells with BTK WT (2.5 k/well), BTK C481S (2.5 k/well), or BTK T474I (2.5 k/well) were plated in a 96-well plate, respectively. A compound of different concentration gradients was added using Tecan D300e, and the plate was incubated at 37 °C and 5% $CO_2$ for 2 hours.
2) 8.33 μL of 20 mM Na3VO4 was added to bring the final concentration to 5 mM, and the plate was incubated at 37 °C and 5% $CO_2$ for 3 h.
3) During the incubation, the following reagents were prepared:

4x added lysis buffer: 24 vol 4x lysis buffer + 1 vol 25x blocking reagent.
p-BTK (Y223) cryptate-antibody and p-BTK (Y223) d2-antibody working solutions: 1 volume of each cryopreserved antibody (stored at - 20 °C) was added to 38 times the volume of assay buffer (stored at 4 °C), and mixed well.

4) After the incubation was completed, 11.1 μL of 4x added lysis buffer was directly added. The plate was shaken at 800 rpm at room temperature for 30 min.
5) The lysate was mixed well and 16 μL of it was transferred to a 384-well plate. 1 × added lysis buffer was used as a blank control.
6) 4 μL of pre-mixed working solutions of the two antibodies (cryptate Ab:d2 Ab = 1:1) was added, and the plate was sealed.
7) The plate was incubated at room temperature overnight.
8) The plate was read at 665nm and 620nm using Biotek cytation3.0 microplate reader, and the ratio of 665/620nm of each well was calculated. Data analysis:
$IC_{50}$ was calculated using GraphPad Prism 7.0 software.

The $IC_{50}$ (half-maximal phosphorylation inhibitory concentration) of the phosphorylated BTK Y223 of the compound was obtained using the following nonlinear fitting formula:

$$Y=Bottom + (Top-Bottom)/(1+10^{((LogIC50-X)*HillSlope)})$$

X: Log value of the compound concentration
Y: Inhibition rate (% inhibition)
Top: Maximum response
Bottom: Baseline response
HillSlope: Slope of the curve

The formula for calculating cell phosphorylation inhibition rate (inhibition rate %) was:

$$\text{Inhibition rate \%} = 1 - (\text{each concentration well-blank well})/(\text{positive control well-blank well})$$

(4) Viability assay of TMD8 cells and OCI-LY10 cells

The in vitro antiproliferative activity of the compounds of the present disclosure against TMD8 cells and OCI-LY10 cells was assayed using CTG (Cell Titer-Glo) luminescent cell viability assay. The specific assay methods were as follows.

a) TMD8 cells were cultured at 37 °C and 5% $CO_2$ in complete medium [RPMI 1640 (supplier Gibco, product number 22400-089) + 10% FBS (supplier Gibco, product number 10091-148) + 1% P/S (supplier Hyclone, product number SV30010)]; OCI-LY10 cells were cultured at 37 °C and 5% $CO_2$ in medium [IMDM (supplier Gibco, product number 31980-030) + 20% FBS (supplier Gibco, product number 10091-148) + 1% P/S (supplier Hyclone, product number SV30010) + 0.05 mM 2-Mercaptoethanol (supplier Sigma, product number M6250)].
b) Cells in the logarithmic growth phase were harvested, and the total cell count and viable cell count were determined using a Vi-cell cell counter (supplier: BECKMAN COULTER, product number 2785198).
c) Cell density was adjusted using complete culture medium. Then, the cells were inoculated at 1500 cells per well in a 384-well cell culture plate (supplier Greiner, product number 781090).
d) The cells in the 384-well plate were cultured at 37 °C and 5% $CO_2$ overnight.
e) A 10-fold drug solution was prepared, and then the serially diluted compound was transferred to the corresponding assay wells of the 384-well cell plate so that the final assay concentration was 1000 nM, with 9 concentrations, 3-fold dilutions, and triplicates for each drug concentration.
f) The cells in the 384-well plate with the drug added were cultured at 37 °C and 5% $CO_2$ for another 120 hours.
g) The cells were removed from the incubator, and allowed to equilibrate at room temperature for 30 minutes. The cell state and compound solubility were observed under a microscope.
h) An equal volume of CellTiterGlo solution (supplier Promega, product number G7573) was added to each well, and the plate was incubated at room temperature for 30 minutes.
i) Then, the luminescent signal value was detected on an EnVision instrument (supplier PerkinElmer, product number 2441715).

The data were analyzed using XL-fit software (supplier: ID Business Solutions Ltd., software version: XL fit 5.5.0.5). Nonlinear regression was performed using X=Log(X), and the X values were transformed using a four-parameter Logistic model 205, from which the $IC_{50}$ values were calculated.

Inhibition rate % = (Max - sample value) / Max * 100. (Max: DMSO negative control)

(5) Viability assay of Ba/F3-ETV6-BTK-T474I cells

The in vitro antiproliferative activity of the compounds of the present disclosure against Ba/F3-ETV6-BTK-T474I cells was assayed using CTG luminescent cell viability assay. The specific assay methods were as follows:

a) Ba/F3-ETV6-BTK-T474I cells were cultured at 37 °C and 5% $CO_2$ in complete medium [RPMI 1640 (supplier Gibco, product number 22400-089) + 10% FBS (supplier Gibco, product number 10091-148)].
b) Cells in the logarithmic growth phase were harvested, and cell counting was performed using a platelet counter. Trypan blue exclusion test was used to determine the cell viability, ensuring that the cell viability was above 90%.
c) Cell density was adjusted using complete culture medium. Then, the cells were inoculated at 90 μL, a total of 3000 cells per well in a 96-well cell culture plate (supplier: Shanghai Jing An Biotechnology Co., Ltd., product number J09601).
d) The cells in the 96-well plate were cultured at 37 °C and 5% $CO_2$.
e) A 10-fold drug solution was prepared, and then 10 μL of each serially diluted compound was transferred to the corresponding assay wells of the 96-well cell plate so that the final assay concentration was 1000 nM, with 9 concentrations, 3.16-fold dilutions, and triplicates for each drug concentration.
f) The cells in the 96-well plate with the drug added were cultured at 37 °C and 5% $CO_2$ for another 72 hours. Then, CTG analysis was performed.
g) The CTG reagent was melted, and the cell plate was equilibrated at room temperature for 30 minutes.
h) An equal volume of CTG solution was added to each well.
i) The plate was shaken on an orbital shaker for 5 minutes to lyse the cells.
j) The cell plate was placed at room temperature for 20 minutes to stabilize the luminescent signal.
k) The luminescent signal values were read and the data were collected.

The data were analyzed using GraphPad Prism 7.0 software. Nonlinear S-curve regression was used to fit the data to give a dose-response curve, from which the $IC_{50}$ values were calculated.

Cell viability (%) = (Lum of drug to be tested - Lum of culture medium control)/(Lum of cell control - Lum of culture medium control) × 100%.

The compounds of the present disclosure were tested in the above assays, and it was found that the compounds of the present disclosure significantly inhibited phosphorylated BTK Y223 in NIH/3T3 BTK WT, NIH/3T3 BTK C481S, Ramos BTK WT, Ramos BTK C481S, and Ramos BTK T474I cells, while exhibiting potent activity on Ba/F3 ETV6-BTK-T4741, TMD8 and OCl-Ly10 cells. The assay results of the representative examples are summarized in the table below, wherein, A represents an $IC_{50} \leq 5$ nM, B represents an $IC_{50}$ of 5-10 nM, C represents an $IC_{50}$ of 10-50 nM, and D represents an $IC_{50}$ > 50 nM.

| Example No. | NIH/3T3 BTK-WT pBTK | NIH/3T3 BTK-C481S pBTK | Ba/F3 ETV6-BTK-T474I | Ramos BTK-WT | TMD8 | OCl-Ly10 |
|---|---|---|---|---|---|---|
| 1 | C | C | | | | |
| 2 | B | C | | B | B | C |
| 3 | A | B | | B | B | C |
| 4 | D | D | | D | | |
| 5 | D | D | | D | | |
| 6 | A | B | | A | | |
| 6-1 | A | C | | A | A | B |
| 6-2 | A | C | | A | A | A |
| 7 | A | B | | B | | |
| 7-1 | A | B | | A | A | A |

(continued)

| Example No. | NIH/3T3 BTK-WT pBTK | NIH/3T3 BTK-C481S pBTK | Ba/F3 ETV6-BTK-T474I | Ramos BTK-WT | TMD8 | OCI-Ly10 |
|---|---|---|---|---|---|---|
| 7-2 | A | B | | B | A | A |
| 8 | D | D | | D | | |
| 9 | B | C | | B | A | B |
| 10 | D | D | | D | | |
| 11 | D | D | | D | | |
| 12 | D | D | | D | | |
| 13 | D | D | | D | | |
| 14 | D | D | | D | | |
| 15 | A | B | | B | A | B |
| 16 | D | D | | D | | |
| 17 | A | B | | B | B | C |
| 18 | A | C | | C | B | C |
| 19 | A | B | | B | B | C |
| 20 | D | D | | D | | |
| 21 | A | A | | A | A | B |
| 22 | D | D | | C | | |
| 23 | A | A | | A | A | A |
| 24 | A | A | | A | A | C |
| 25 | C | C | | B | | |
| 26 | C | C | | A | | |
| 28 | A | C | | C | | |
| 29 | A | B | | A | | |
| 30 | A | B | | C | | |
| 31 | B | C | | C | | |
| 32 | D | D | | D | | |
| 33 | D | D | | D | | |
| 34 | D | D | | D | | |
| 35 | A | A | | A | A | A |
| 36 | B | B | | C | | |
| 37 | A | A | C | A | A | A |
| 38 | A | A | | A | | |
| 39 | A | A | | A | | |
| 40 | C | D | | C | | |
| 41 | A | B | | A | | |
| 42 | A | C | | C | | |
| 43 | A | A | B | A | A | A |
| 44 | A | A | B | A | A | A |
| 45 | C | C | | D | | |
| 46 | A | B | D | A | A | B |

(continued)

| Example No. | NIH/3T3 BTK-WT pBTK | NIH/3T3 BTK-C481S pBTK | Ba/F3 ETV6-BTK-T474I | Ramos BTK-WT | TMD8 | OCI-Ly10 |
|---|---|---|---|---|---|---|
| 47 | B | C | | B | | |
| 48 | A | A | | A | | |
| 49 | D | D | | D | | |
| 50 | A | B | | B | | |
| 51 | A | B | | B | | |
| 52 | A | A | A | A | A | A |
| 53 | A | A | A | B | A | A |
| 54 | A | A | | A | | |
| 55 | D | D | | D | | |
| 56 | D | D | | A | | |
| 57 | A | B | | C | | |
| 58 | A | A | | A | | |
| 59 | A | A | B | A | A | A |
| 60 | A | A | B | A | A | A |
| 61 | A | A | A | A | A | A |
| 62 | A | A | C | B | A | A |
| 63 | D | D | | D | | |
| 64 | D | D | | D | | |
| 65 | A | B | A | A | A | A |
| 66 | A | A | A | B | A | A |
| 67 | A | A | A | A | A | A |
| 68 | A | A | A | A | A | A |
| 69 | A | A | C | A | A | A |
| 70 | A | A | | A | | |
| 71 | A | A | B | A | A | A |
| 72 | A | A | B | A | A | A |
| 73 | A | A | | A | | |
| 74 | A | A | A | A | A | A |
| 75 | A | A | A | A | A | A |
| 76 | A | A | A | A | A | A |
| 77 | A | A | | A | | |
| 78 | A | A | | A | | |
| 79 | B | C | | B | | |
| 80 | B | B | | A | | |
| 81 | D | D | | D | | |
| 82 | A | A | | C | | |
| 83 | C | D | | D | | |
| 84 | B | C | | B | B | C |
| 85 | A | B | | A | A | C |

(continued)

| Example No. | NIH/3T3 BTK-WT pBTK | NIH/3T3 BTK-C481S pBTK | Ba/F3 ETV6-BTK-T474I | Ramos BTK-WT | TMD8 | OCI-Ly10 |
|---|---|---|---|---|---|---|
| 86 | D | D | | | | |
| 87 | D | D | | D | | |
| 88 | C | D | | B | | |
| 89 | C | D | | C | | |
| 90 | D | D | | D | | |
| 91 | A | B | | B | A | A |
| 92 | D | D | | C | | |
| 93 | D | D | | D | | |
| 94 | D | D | | D | | |
| 95 | D | D | | B | | |
| 96 | C | D | | A | A | A |
| 97 | D | D | | B | | |
| 98 | A | A | | A | | |
| 99 | A | B | | A | | |
| 100 | A | B | | A | | |
| 101 | A | A | | A | | |
| 102 | C | C | | C | | |
| 103 | B | C | | C | | |
| 105 | D | D | | D | | |
| 106 | D | D | | D | | |
| 109 | D | D | | D | | |
| 110 | D | D | | D | | |
| 111 | D | D | | D | | |
| 113 | A | A | | A | | |
| 115 | A | B | D | A | C | C |
| 116 | D | D | | D | | |
| 117 | C | C | | A | A | A |

(6) Assay of the effect of compounds on BTK L528W

(A) Steps for detecting the B cell signaling pathways:

1) Mice were euthanized, then soaked in alcohol for 2-3 minutes, and dissected in the right lateral decubitus position. The spleen was removed and placed in a culture medium.
2) The spleen was ground on a 200-mesh stainless steel mesh using the inner core of a syringe until only connective tissue remains. The spleen cells were collected.
3) 3-5 times the cell volume of erythrocyte lysis buffer (Beyotime C3702) was added, and the mixture was gently pipetted to mix well. The cells were lysed for 1-2 minutes, and then processed according to the product instructions to give the mice spleen cells. The cells were counted.
4) The spleen cells were diluted to 100w/mL with cell culture medium, and 100μL of the cells were inoculated into each well of a 96-well plate, with 10w cells per well.

5) A compound of various concentrations was added, and the plate was incubated at 37 °C in a 5% $CO_2$ incubator for 1 hour.
6) Mouse anti-IgM was added to each treatment well to a final concentration of 50ug/mL, and the plate was incubated at 37 °C in a 5% $CO_2$ incubator for 18 hours.
7) The next day, the supernatant was removed from the 96-well plate, and the plate was centrifuged at 1000g for 10 minutes.
8) The cells were resuspended in 200 μL of FACS staining buffer. The plate was centrifuged at 1000 g for 10 min, and the supernatant was carefully aspirated.
9) All the antibodies were prepared in the FACS staining buffer and mixed well, and added to the cells in each well, respectively, and mixed well. The plate was incubated at 4 °C in the dark for 40 min.
10) Step 8 was repeated, and then the cells were resuspended in 100 μL of FACS staining buffer after being rinsed.
11) Flow cytometry analysis was performed using single-staining single-tube cells for automated fluorescence compensation, and the compensation matrix was applied to all the analyzed samples. B cells (CD19+, CD3-) were labeled using FITC-H and Pacific Blue-H, and the sample stained with IgG1-PE was used as a negative control. CD69+ and CD69- cells were distinguished based on the fluorescence threshold value of PE-H.
12) Data Analysis. In the B cells, the percentage of CD69+ in the PE-H channel was statistically analyzed. Each treatment was normalized using the DMSO + anti-IgM sample, and $IC_{50}$ analysis of CD69+% was performed using GraphPad Prism 7.

The percentage of relative CD69 mean fluorescence intensity = (mean fluorescence intensity of the drug treatment group - blank stimulation control) / (mean fluorescence intensity of DMSO control treatment group - blank stimulation control).

(B) Assay steps of calcium flux assay:

1) Ramos (BTK L528W) cells were collected, and centrifuged. The culture medium was discarded.
2) The cells were washed twice with 10 mL of HBSS, washed once with 10 mL of phenol red-free RPMI 1640 + 25 mM HEPES, and centrifuged. The supernatant was discarded.
3) The cells were resuspended in 5 mL of phenol red-free RPMI 1640 + 25 mM HEPES.
4) 5 μL of calcium indicator was taken and added to 5 mL of signal enhancer (purple), and mixed well. Then, the mixture was added to 5 mL of cell suspension. The resulting mixture was mixed well by pipetting and incubated in a 37 °C incubator for 1 h.
5) Step 2 was repeated.
6) An appropriate amount of phenol red-free RPMI 1640 + 25mM HEPES + 10% FBS were added to resuspend the cells. The cells were counted, and diluted to 200k cells per well.
7) The diluted cells were added to a 96-well plate at 100 μL per well.
8) A compound of various concentrations was added, and then the plate was incubated in a 37 °C incubator for 2 hours.
9) Anti-IgM was added to each well to a final concentration of 50ug/mL. A same volume of culture medium was added to the blank control. The plate was assayed using a microplate reader (Excitation: 485, Emission: 525, the assay was performed every 2 seconds, for a total of 3 minutes).
10) Data analysis

The unstimulated well (with only culture medium added) was used as the baseline. Each peak concentration was calculated by subtracting the baseline from the maximum reading of each well. The kinetic data were imported into Graphpad Prism 7 to calculate the calcium flux AUC for each treatment.

Relative peak calcium flux % = Peak calcium flux (anti-IgM stimulated compound treatment group) / Peak calcium flux (anti-IgM stimulated DMSO treatment group) × 100%

Relative calcium flux AUC % = [AUC (anti-IgM stimulated compound treatment group) - AUC (unstimulated DMSO treatment group)]/[AUC (anti-IgM stimulated DMSO treatment group) - AUC (unstimulated DMSO treatment group)] × 100%. The $IC_{50}$ value of calcium flux was calculated with Peak % or AUC % of each concentration treatment using GraphPad Prism 7.0 software, respectively.

(C) Cellular thermal shift assay

1) Ramos (BTK L528W) cells were cultured and plated in a 6-well plate at 5 million cells/well. 0.5% DMSO or 10 μM compound was added, and the cells were treated for 10-30 min.
2) The above treated cells were divided into small portions and subjected to a brief heat treatment at 37-70 °C, respectively.
3) The cells were lysed, and centrifuged respectively, and the supernatant was collected to prepare a sample, which was subjected to SDS-PAGE protein electrophoresis.
4) After transferring to a PVDF membrane, western blotting was performed using BTK antibody. The differences in band grayscale changes between the compound treatment groups and the 0.5% DMSO treatment group at different temperatures were compared. The compounds showing an increase in the grayscale value of the BTK band in the cell lysis supernatant were identified as those binding to BTK L528W.

The compounds of the present disclosure were tested in the above assay, and it was found that the compounds of the present disclosure significantly overcome the BTK L528W drug resistance mutation.

(7) Metabolic stability evaluation

Metabolic stability is generally used to describe the rate and degree of compounds being metabolized, and is one of the main factors affecting pharmacokinetic properties. Many compounds are substrates of CYP450 enzymes and other drug metabolizing enzymes, and liver microsomes are systems rich in CYP450. The purpose of this assay is to study the in vitro metabolic stability by incubating the compounds of the present disclosure with human and SD rat liver microsomes respectively and detecting the remaining proportion of the compounds by LC-MS/MS.

1) Preparation of solutions

Phosphate-buffered saline (PBS): 150mL of pre-prepared $K_2HPO_4$ (0.5 M) solution was mixed with 700 mL of $K_2HPO_4$ (0.5 M) solution; $K_2HPO_4$ (0.5 M) solution was then used to adjust the pH value of the mixed solution to 7.4. The mixture was used as 5-fold concentration of PBS, and stored at 4 °C for later use. The mixture was diluted 5 times with ultrapure water before use, and 3.3 mM magnesium chloride was added to give the phosphate-buffered saline PBS (100 mM).
NADPH regeneration system solution: A NADPH solution containing 6.5 mM NADP, 16.5 mM G-6-P, and 3U/mL G-6-P D was prepared with 5 mL of PBS.
Internal standard stop solution: 50 ng/mL propranolol hydrochloride and 200 ng/mL tolbutamide were prepared with acetonitrile as an internal standard working solution.
Human liver microsome solution: 0.31 mL of human liver microsomes (25mg/mL) were added to 0.961 mL of PBS (pH7.4) and mixed well to give a human liver microsome dilution with a protein concentration of 0.625 mg/mL.
SD rat liver microsome solution: 0.31 mL of SD rat liver microsomes (25 mg/mL) were added to 0.961 mL of PBS (pH7.4) and mixed well to give SD rat liver microsome dilution with a protein concentration of 0.625 mg/mL.
Sample working solution: The powder of a compound of the present disclosure, the positive control dextromethorphan powder and omeprazole powder were prepared with DMSO to 10 mM as the sample stock solution, and then diluted with 70% acetonitrile-water to give a 0.25 mM sample working solution.

2) Sample incubation

398 μL of human liver microsome dilution was added to a 96-well incubation plate (N = 2); 2 μL of 0.25 mM test compound and dextromethorphan were added respectively, and mixed well.
398 μL of SD rat liver microsome dilution was added to a 96-well incubation plate (N = 2); 2 μL of 0.25 mM test compound and omeprazole were added respectively, and mixed well.
300 μL of pre-cooled stop solution per well was added to a 96-well deep well plate as a stop plate and placed on ice.
The 96-well incubation plate and the NADPH regeneration system were placed in a 37 °C water bath, shaken at 100 rpm, and pre-incubated for 5 minutes. 80μL of incubation solution was taken out from each well of the incubation plate, added to the stop plate, and mixed well. 20μL of NADPH regeneration system solution was supplemented, as a 0 min sample. Then 80μL of NADPH regeneration system solution was added to each well of the incubation plate. The reaction was initiated, and timing was started. The reaction concentration of the compound to be tested was 1μM, and the protein

concentration was 0.5 mg/mL.

At 10, 30, and 90 minutes of the reaction, respectively, 100 μL of the reaction solution was taken and added to the stop plate, and vortexed for 3 minutes to stop the reaction.

The stop plate was centrifuged at 5000rpm and 4 °C for 15 minutes. 200μL of the supernatant was added into a 96-well plate pre-added with 200μL of ultrapure water, and mixed well. LC-MS/MS was used for sample analysis, and 10 μL of a sample was injected.

3) Sample analysis method

In this assay, LC-MS/MS system was used to detect the peak areas of the compound to be tested, dextromethorphan, omeprazole and internal standard, and the ratio of the peak areas of the compound to the internal standard was calculated.

4) Data processing

The peak areas of the sample and internal standard were obtained by mass spectrometer and Analyst software. The substrate elimination rate constant K was obtained by plotting the residual amount of compound (R%) against time using the single exponential degradation model of Graphpad prism7.0 software.

$$Ct/C0 = \exp(-K*t)$$

The half-life $T_{1/2}$ and intrinsic clearance rate $CL_{int}$ were calculated according to the following formula, where V/M is equal to 1/C (protein).

$$T_{1/2} = -\frac{0.693}{\text{Slope}},\ CL_{\text{int}} = \frac{0.693}{t_{1/2}} \cdot \frac{V}{M},\ t_{1/2}(\text{min});\ CL_{\text{int}}(\mu L/\text{min}/\text{mg}).$$

The assay results show that the compounds of the present disclosure have excellent metabolic stability.

(8) Pharmacokinetic assays in rats

For each drug to be tested, six male Sprague-Dawley rats, 7-8 weeks old and weighing approximately 210g, were prepared and divided into two groups, with three rats in each group. A single dose of the compound was administered via single intravenous injection or single oral gavage (1 mg/kg intravenously, 10 mg/kg orally). The pharmacokinetic differences were compared.

Fasting was initiated 10-14 hours prior to the assay, and feeding was resumed 4 hours after drug administration. All animals had free access to drinking water throughout the study. The drug was dissolved in 5% DMSO + 10% Solutol + 85% (20% HP-β-CD). Blood was collected from the jugular vein, and the time points of blood collection were 0.083 hours, 0.25 hours, 0.5 hours, 1 hour, 2 hours, 4 hours, 8 hours, 12 hours and 24 hours after administration.

Blood was collected via the jugular vein, with each sample collected containing approximately 0.2 mL. Heparin sodium was used for anticoagulation, and the collected samples were placed on wet ice. Blood samples were placed on ice after collection and centrifuged within 1 hour to separate the plasma (centrifugation conditions: 6000g, 3 minutes, 2-8 °C). Plasma samples were stored in a -80 °C freezer before analysis.

Pharmacokinetic parameters were calculated using blood drug concentration data at different time points with Phoenix WinNonlin 8.2.0., and parameters such as AUC0-t, AUC0-∞, MRT0-∞, Cmax, Tmax, and T1/2, along with their mean and standard deviation were provided.

The compounds of the present disclosure were tested in the above assay and it was found that the compounds of the present disclosure have better pharmacokinetic properties.

(9) Pharmacokinetic and brain tissue distribution study of male SD rats after a single oral gavage administration

This assay aimed to evaluate the pharmacokinetics and blood-brain barrier permeability of a drug in rats after oral gavage administration of the compound to the male Sprague-Dawley (SD) rats. Plasma, cerebrospinal fluid, and brain tissue were collected at different time points, and concentrations of the compound were determined by LC-MS/MS.

Male SD rats (n=3 for each time point) were administered a compound at 10 mg/kg by gavage in a solvent of 5% DMSO + 15% Solutol HS 15 + 80% (20% Hp-β-CD in DW). The animals were fasted overnight starting at 18:00 the day before the administration, and were fed 4 hours after the administration on the day of the assay, with free access to water.

Whole blood, cerebrospinal fluid, and brain tissue were collected at 30 min, 1 h, 2 h, 8 h, 24 h, and 32 h after the

administration.

The whole blood samples (approximately 0.25 mL at each time point) were collected via puncture of jugular sinus before euthanasia and transferred to K2EDTA anticoagulant tubes, and shaken well. The tubes were placed on wet ice, and centrifuged for 5 min (6000 rpm, 4 °C). Then, plasma was collected.

The cerebrospinal fluid (approximately 0.10 mL at each time point) was collected via puncture of cerebellomedullary cistern and placed in a collection tube.

After each animal was euthanized by inhaling excessive $CO_2$, systemic perfusion was performed via the heart using physiological saline. Then, the animals were dissected, and brain tissue was collected. Each tissue sample was rinsed with physiological saline, then dried with filter paper, and then weighed using a balance. The weight was recorded. The tissue was transferred to a centrifuge tube and placed in an ultra-low temperature freezer until homogenized. A homogenizing buffer of methanol:10 mM PBS (containing 0.2% formic acid) = 3:7 was used, and homogenization was performed using 9 times the volume of the homogenizing buffer (i.e., 9 mL of homogenizing buffer was added to 1 g of tissue). All the above samples were stored at -80 °C for analysis after collection.

Concentrations of the compound in the formulation and plasma samples were determined using LC-MS/MS method.

The data acquisition and integration software used was Analyst, Version 1.6.3, and the data processing software used for plotting the plasma drug concentration-time curves, calculating the mean and standard deviation of pharmacokinetic parameters, and the Brain Plasma ratio was Microsoft Office 2016 (Microsoft, USA).

Based on the mean plasma concentration-time data, pharmacokinetic parameters were calculated using the non-compartmental model statistical moment method in Phoenix WinNonlin 8.0 software. Reported parameters include, but are not limited to, Tmax, Cmax, AUC0-t, AUC0-inf, T1/2, and MRT0-inf.

The compounds of the present disclosure were tested in the above assay, and it was found that the compounds of the present disclosure had higher concentrations in the cerebrospinal fluid and brain tissue of SD rats.

The above content is a further detailed description of the present disclosure in conjunction with specific alternative embodiments, and it cannot be considered that the specific implementation of the present disclosure is limited to these descriptions. For those skilled in the art to which the present disclosure belongs, some simple deductions or substitutions can be made without departing from the concept of the present disclosure, and should be regarded as belonging to the scope of protection of the present disclosure.

## Claims

1. A compound of formula (A), or a tautomer, a stereoisomer, a prodrug, a crystal form, a pharmaceutically acceptable salt, a hydrate or a solvate thereof:

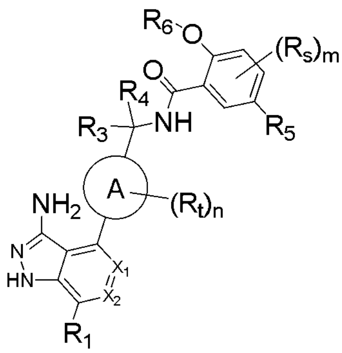

(A)

wherein,

ring A is a benzene ring or a 5- to 6-membered heteroaromatic ring;
$X_1$ is N, CD or CH;
$X_2$ is N or $CR_2$;
$R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted

with one or more R;

wherein each of $R_a$, $R_b$ and $R_c$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;

$R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R';

$R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_5$ is halogen;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

$R_s$ and $R_t$ are independently H, D, halogen, $-OR_a$, $-NR_bR_c$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

m is 0, 1, 2 or 3;

n is 0, 1, 2, 3 or 4;

each R is independently:

1) H, D, halogen, oxo, $-C(O)R_a$, $-C(O)OR_a$, $-C(O)NR_eR_f$, $-NR_eR_f$, $-NR_dC(O)R_e$, $-NR_dC(O)OR_e$, $-NR_dC(O)NR_eR_f$, $-OR_a$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or

2) $C_{1-6}$ alkyl, $C_{1-6}$ haloalkyl, $C_{3-6}$ cycloalkyl, 3- to 7-membered heterocyclyl, $-C_{1-6}$ alkylene-$C_{3-6}$ cycloalkyl, $-C_{1-6}$ alkylene-3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or

3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl, wherein the groups are optionally substituted with one or more R";

each R' is independently H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_a$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R‴;

each R‴ is independently H, D, halogen, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl is optionally substituted with one or more D, halogen, $C_{1-6}$ alkyl, $C_{1-6}$ haloalkyl, $C_{3-6}$ cycloalkyl, 3- to 7-membered heterocyclyl, $C_{6-10}$ aryl or 5-to 10-membered heteroaryl;

wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered

heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

**2.** A compound of formula (I), or a tautomer, a stereoisomer, a prodrug, a crystal form, a pharmaceutically acceptable salt, a hydrate or a solvate thereof:

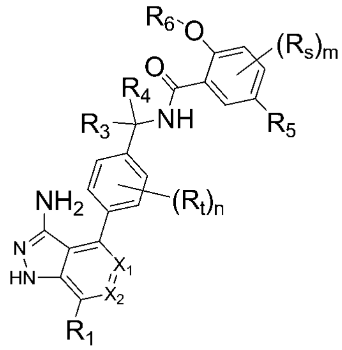

(I)

wherein,

$X_1$ is N, CD or CH;
$X_2$ is N or $CR_2$;
$R_1$ is H, D, halogen, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_bR_c$, $-S(O)R_a$, $-S(O)_2R_a$, $-P(=O)R_bR_c$, $-OP(=O)R_bR_c$, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R;
wherein each of $R_a$, $R_b$ and $R_c$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_b$, $R_c$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R;
$R_2$ is H, D, halogen, $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 12-membered heterocyclyl, $C_{2-6}$ alkenyl, $C_{2-6}$ alkynyl, $C_{6-10}$ aryl and 5- to 10-membered heteroaryl are optionally substituted with one or more R';
$R_3$ and $R_4$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_s$ and Rt are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
m is 0, 1, 2 or 3;
n is 0, 1, 2, 3 or 4;
each R is independently:

1) H, D, halogen, oxo, $-C(O)R_a$, $-C(O)OR_a$, $-C(O)NR_eR_f$, $-NR_eR_f$, $-NR_dC(O)R_e$, $-NR_dC(O)OR_e$, $-NR_dC(O)NR_eR_f$, $-OR_a$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) two R on the same atom or on two adjacent atoms are taken together with the atom(s) to which they are attached to form $C_{3-6}$ cycloalkyl, 3- to 12-membered heterocyclyl, $C_{6-10}$ aryl or 5- to 10-membered heteroaryl,

wherein the groups are optionally substituted with one or more R";

each R' is independently H, D, halogen, oxo, -C(O)$R_d$, -C(O)O$R_d$, -C(O)N$R_eR_f$, -N$R_eR_f$, -N$R_d$C(O)$R_e$, - N$R_d$C(O)O$R_e$, -N$R_d$C(O)N$R_eR_f$, -O$R_a$, -OC(O)$R_d$, -OC(O)N$R_eR_f$, -N$R_d$S$(O)_2R_e$, -S$(O)_2$N$R_eR_f$, -S(O)$R_d$, - S$(O)_2R_d$, -P(=O)$R_eR_f$, -OP(=O)$R_eR_f$; $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R' on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";

each R" is independently H, D, halogen, oxo, -C(O)$R_g$, -C(O)O$R_g$, -C(O)N$R_hR_j$, -N$R_hR_j$, -N$R_g$C(O)$R_h$, - N$R_g$C(O)O$R_h$, -N$R_g$C(O)N$R_hR_j$, -O$R_g$, -OC(O)$R_g$, -OC(O)N$R_hR_j$, -N$R_g$S$(O)_2R_h$, -S$(O)_2$N$R_hR_j$, -S(O)$R_g$, - S$(O)_2R_g$, -P(=O)$R_hR_j$, -OP(=O)$R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;

wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

**3.** The compound according to claim 1 or 2, or a tautomer, a stereoisomer, a prodrug, a crystal form, a pharmaceutically acceptable salt, a hydrate or a solvate thereof, wherein $R_1$ is H, D, $C_{6-10}$ aryl or 5- to 10-membered heteroaryl, wherein the $C_{6-10}$ aryl and 5- to 10-membered heteroaryl are optionally substituted with one or more R;

alternatively, $R_1$ is H, D,

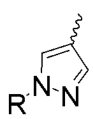, 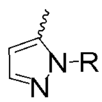, 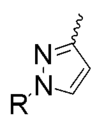, 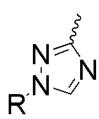, 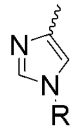, 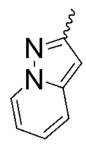, 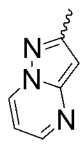, 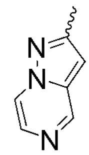,

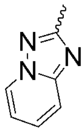, 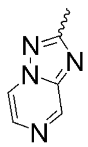, 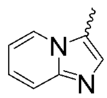, 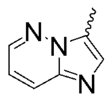, 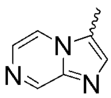, 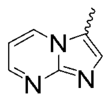, 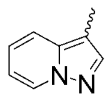, 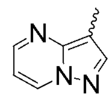, 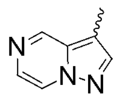,

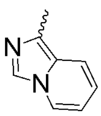, 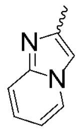, 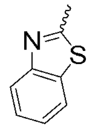, 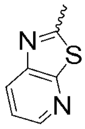, 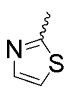, 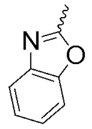, 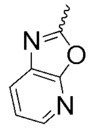, 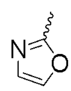, 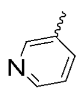, 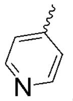, 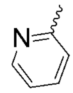,

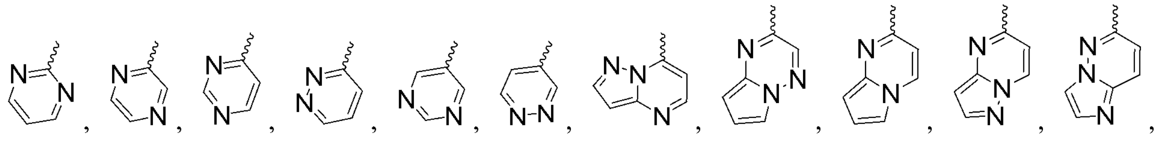

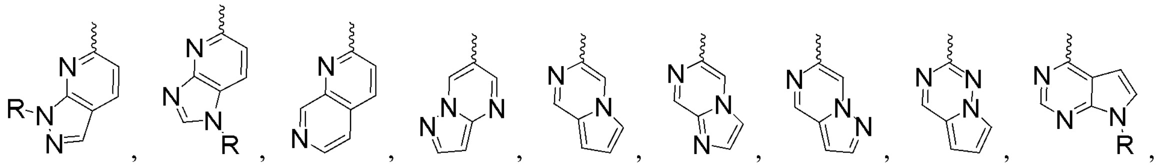

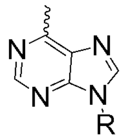 or 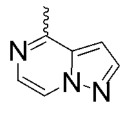,

wherein the above groups are optionally substituted with one or more R; alternatively, $R_1$ is

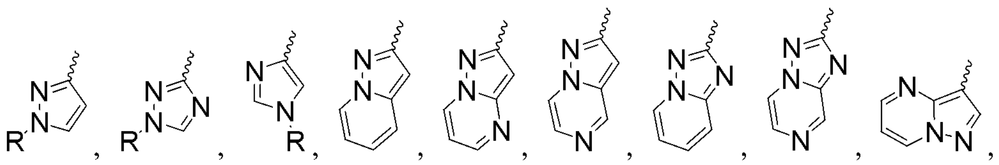

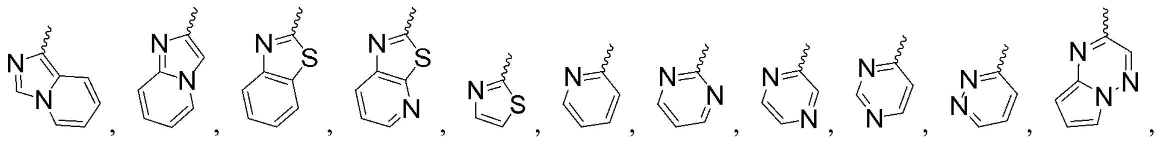

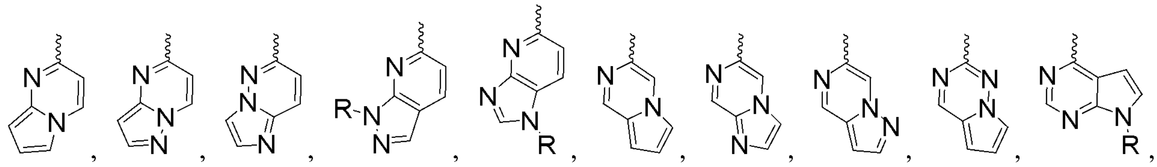

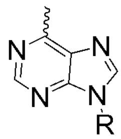 or 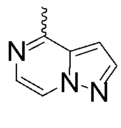,

wherein the above groups are optionally substituted with one or more R; alternatively, $R_1$ is

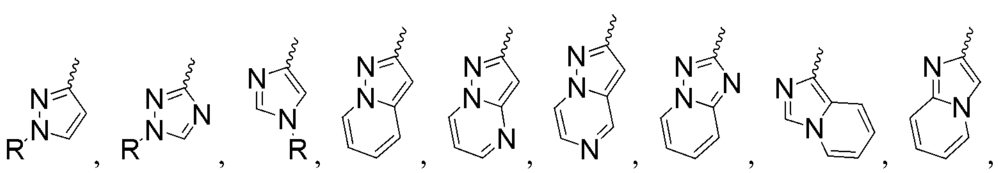

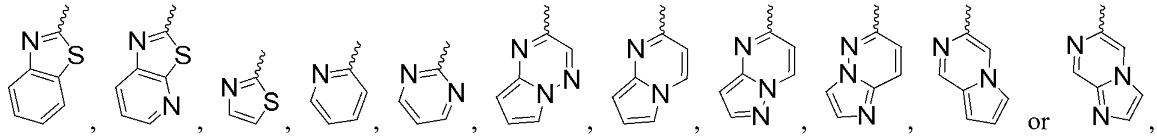

, , , , , , , , , , or ,

wherein the above groups are optionally substituted with one or more R; alternatively, $R_1$ is H.

4. The compound according to any one of claims 1 to 3, or a tautomer, a stereoisomer, a prodrug, a crystal form, a pharmaceutically acceptable salt, a hydrate or a solvate thereof, wherein $R_2$ is H, D, halogen, $C_{1-6}$ alkyl, $C_{1-6}$ haloalkyl, $C_{3-6}$ cycloalkyl, 3- to 7-membered monocyclic heterocyclyl, 6- to 12-membered fused bicyclic heterocycle, 7- to 10-membered spiro heterocycle, $C_{6-10}$ aryl or 5- to 10-membered heteroaryl, wherein the $C_{1-6}$ alkyl, $C_{1-6}$ haloalkyl, $C_{3-6}$ cycloalkyl, 3- to 7-membered monocyclic heterocyclyl, 6- to 12-membered fused bicyclic heterocycle, 7- to 10-membered spiro heterocycle, $C_{6-10}$ aryl and 5- to 10-membered heteroaryl are optionally substituted with one or more R';

alternatively, $R_2$ is H, D, halogen, methyl, $-CHF_2$, ethyl, isopropyl,

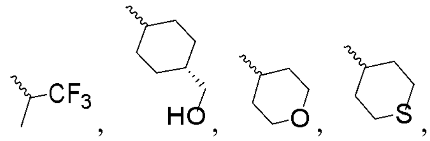

, , , ,

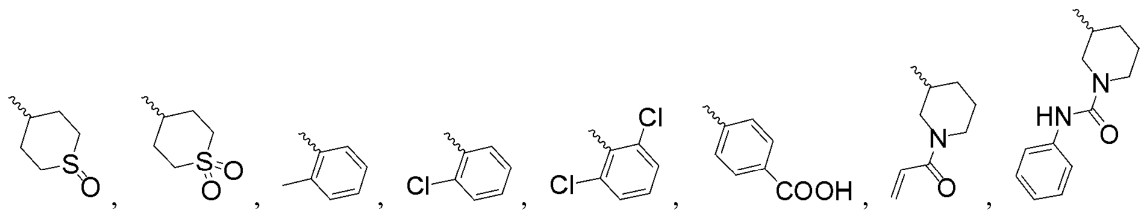

, , , , , , , ,

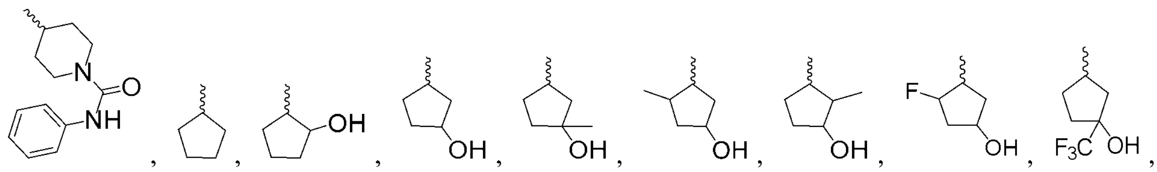

, , , , , , , , , ,

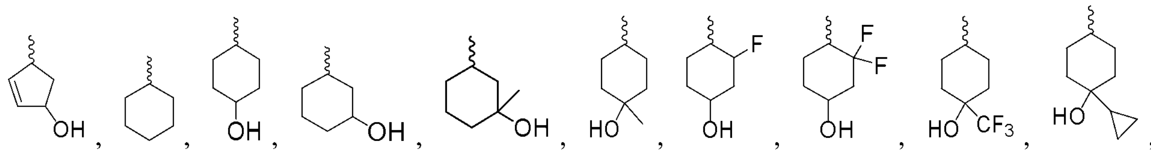

, , , , , , , , , ,

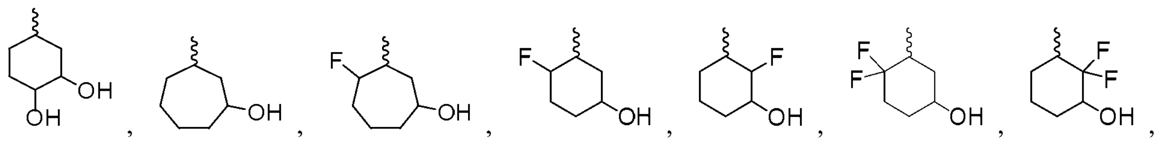

, , , , , , ,

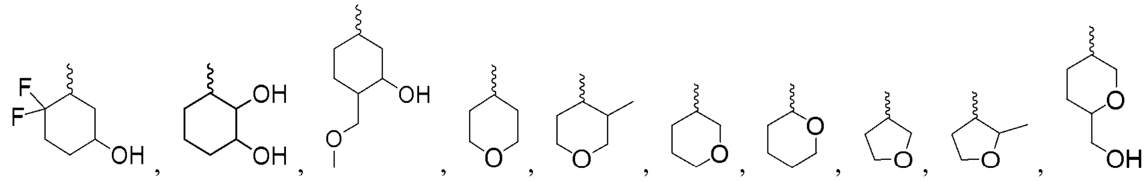

, , , , , , , , , ,

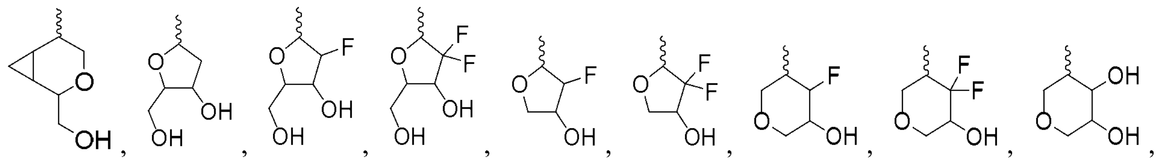

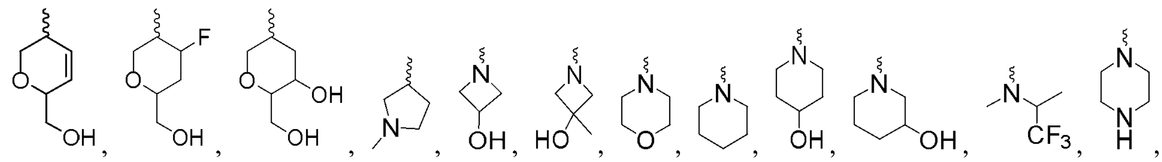

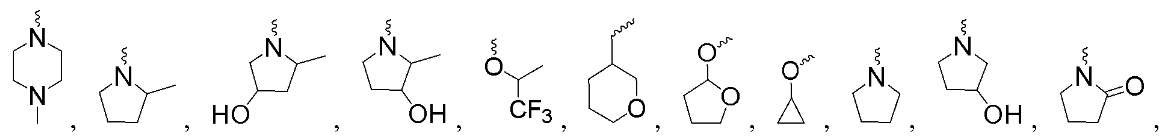

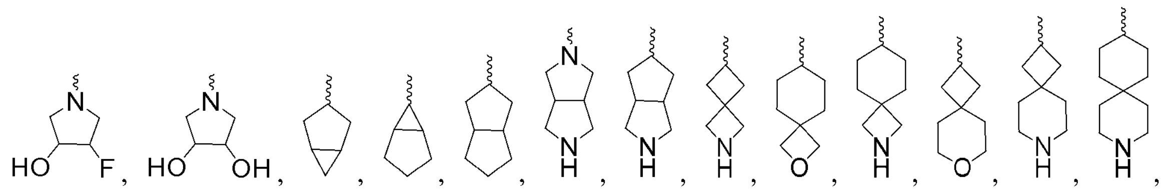

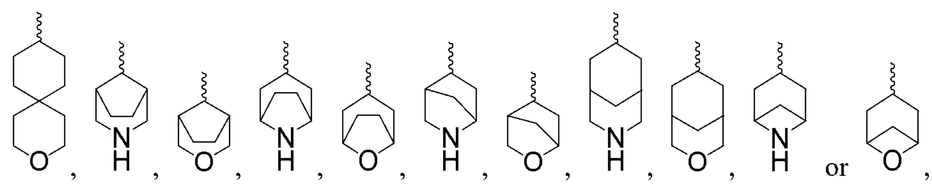

wherein the above groups are optionally substituted with one or more D, up to fully deuterated;
alternatively, $R_2$ is H, D, halogen, methyl, $-CHF_2$, ethyl, isopropyl,

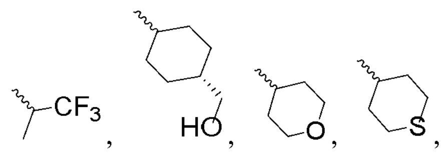

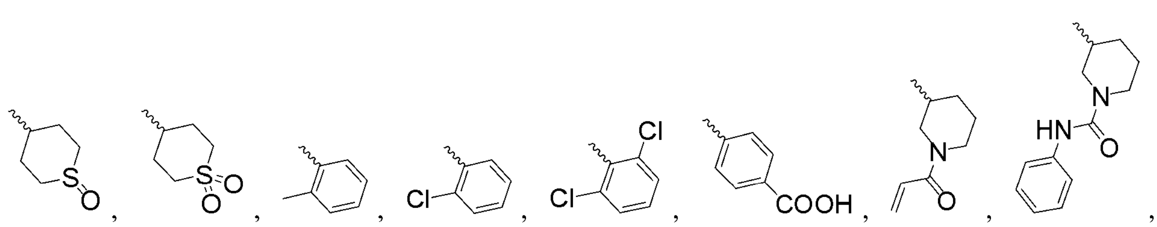

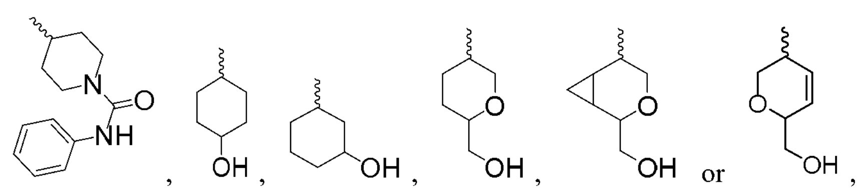

wherein the above groups are optionally substituted with one or more D, up to fully deuterated;

alternatively, $R_2$ is H, D, methyl, $-CHF_2$,

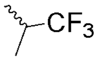, 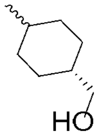, 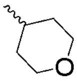, 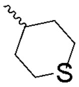, 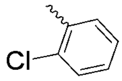, 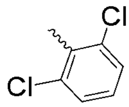,

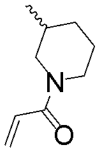, 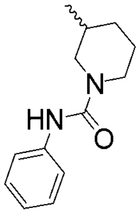, 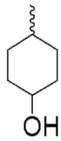, 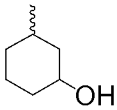, 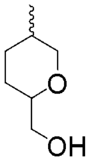, 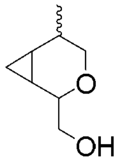 or 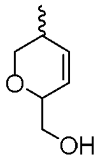,

wherein the above groups are optionally substituted with one or more D, up to fully deuterated;
alternatively, $R_2$ is H, D, $-CHF_2$ or

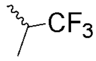,

wherein the above groups are optionally substituted with one or more D, up to fully deuterated;
alternatively, $R_2$ is H or D;
alternatively, $R_2$ is $-CHF_2$ or

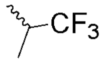,

wherein the above groups are optionally substituted with one or more D, up to fully deuterated;
alternatively, $R_2$ is

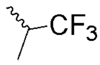,

wherein the above group is optionally substituted with one or more D, up to fully deuterated.

5. The compound according to any one of claims 1 to 4, or a tautomer, a stereoisomer, a prodrug, a crystal form, a pharmaceutically acceptable salt, a hydrate or a solvate thereof, wherein Rs is F.

6. The compound according to any one of claims 1 to 5, or a tautomer, a stereoisomer, a prodrug, a crystal form, a pharmaceutically acceptable salt, a hydrate or a solvate thereof, wherein $R_6$ is methyl or $-CD_3$.

7. The compound according to claim 1 or 2, or a tautomer, a stereoisomer, a prodrug, a crystal form, a pharmaceutically acceptable salt, a hydrate or a solvate thereof, wherein the compound is a compound of formula (II):

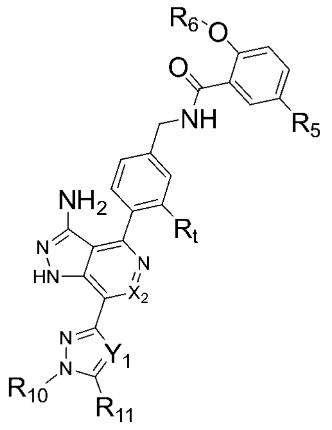

(II)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_1$ is N or $CR_{12}$;
$R_{10}$, $R_{11}$ and $R_{12}$ are independently:

1) H, D, halogen, oxo, $-C(O)R_a$, $-C(O)OR_a$, $-C(O)NR_eR_f$, $-NR_eR_f$, $-NR_dC(O)R_e$, $-NR_dC(O)OR_e$, $-NR_dC(O)NR_eR_f$, $-OR_a$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{11}$, $R_{12}$ and the atom to which they are attached are taken together to form $C_{3-6}$ cycloalkyl, which is optionally substituted with one or more R"; or
4) $R_{10}$ and $R_{11}$ or $R_{11}$ and $R_{12}$ and the atom to which they are attached are taken together to form 3- to 12-membered heterocyclyl or 5- to 10-membered heteroaryl, wherein the groups are optionally substituted with one or more R"; or
5) $R_{11}$, $R_{12}$ and the atom to which they are attached are taken together to form $C_{6-10}$ aryl, which is optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl,

$C_{6-10}$ aryl and 5- to 10-membered heteroaryl are optionally substituted with one or more D, up to fully deuterated.

**8.** The compound according to claim 7, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_1$ is N or $CR_{12}$;
$R_{10}$ is H, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more R";
$R_{11}$ and $R_{12}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{11}$, $R_{12}$ and the atom to which they are attached are taken together to form $C_{3-6}$ cycloalkyl, which is optionally substituted with one or more R"; or
$R_{10}$ and $R_{11}$ or $R_{11}$ and $R_{12}$ and the atom to which they are attached are taken together to form 3- to 7-membered monocyclic heterocyclyl, 6- to 12-membered fused bicyclic heterocycle or 5- to 10-membered heteroaryl, wherein the groups are optionally substituted with one or more R"; or
$R_{11}$, $R_{12}$ and the atom to which they are attached are taken together to form $C_{6-10}$ aryl, which is optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**9.** The compound according to claim 7 or 8, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is halogen; alternatively, $R_5$ is F;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_6$ is methyl or $-CD_3$;
$R_t$ is H, D or halogen; alternatively, $R_t$ is H, D or F;
$Y_1$ is N or $CR_{12}$;
$R_{10}$ is H, $C_{1-6}$ alkyl or $C_{1-6}$ haloalkyl, wherein the $C_{1-6}$ alkyl and $C_{1-6}$ haloalkyl are optionally substituted with one or more R"; alternatively, $R_{10}$ is H, $C_{1-3}$ alkyl or $C_{1-3}$ haloalkyl, wherein the $C_{1-3}$ alkyl and $C_{1-3}$ haloalkyl are optionally substituted with one or more D, up to fully deuterated;
$R_{11}$ and $R_{12}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{11}$ and $R_{12}$ are independently H or D;
or, $R_{11}$, $R_{12}$ and the atom to which they are attached are taken together to form $C_{3-6}$ cycloalkyl, which is optionally substituted with one or more R"; alternatively, $R_{11}$, $R_{12}$ and the atom to which they are attached are taken together to form cyclohexyl, which is optionally substituted with one or more R"; or
$R_{10}$ and $R_{11}$ or $R_{11}$ and $R_{12}$ and the atom to which they are attached are taken together to form 3- to 7-membered monocyclic heterocyclyl, which is optionally substituted with one or more R"; alternatively, $R_{10}$ and $R_{11}$ or $R_{11}$ and $R_{12}$ and the atom to which they are attached are taken together to form 6-membered heterocyclyl having 1 or 2 ring heteroatoms N, which is optionally substituted with one or more R"; or
$R_{10}$ and $R_{11}$ or $R_{11}$ and $R_{12}$ and the atom to which they are attached are taken together to form 6- to 12-membered fused bicyclic heterocycle, which is optionally substituted with one or more R"; alternatively, $R_{10}$ and $R_{11}$ or $R_{11}$ and $R_{12}$ and the atom to which they are attached are taken together to form 8- to 10-membered fused bicyclic heterocycle having 1 or 2 ring heteroatoms N, which is optionally substituted with one or more R"; or

$R_{11}$, $R_{12}$ and the atom to which they are attached are taken together to form $C_{6-10}$ aryl, which is optionally substituted with one or more R"; alternatively, $R_{11}$, $R_{12}$ and the atom to which they are attached are taken together to form phenyl, which is optionally substituted with one or more R"; or

$R_{10}$ and $R_{11}$ or $R_{11}$ and $R_{12}$ and the atom to which they are attached are taken together to form 5- to 10-membered heteroaryl, which is optionally substituted with one or more R"; alternatively, $R_{10}$ and $R_{11}$ or $R_{11}$ and $R_{12}$ and the atom to which they are attached are taken together to form 5-membered or 6-membered heteroaryl having 1 or 2 ring heteroatoms N, wherein the group is optionally substituted with one or more R"; each R" is independently D, -C(O)O$R_g$, -C(O)N$R_h$$R_j$, -N$R_g$C(O)$R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**10.** The compound according to claim 1 or 2, or a tautomer, a stereoisomer, a prodrug, a crystal form, a pharmaceutically acceptable salt, a hydrate or a solvate thereof, wherein the compound is a compound of formula (II-1):

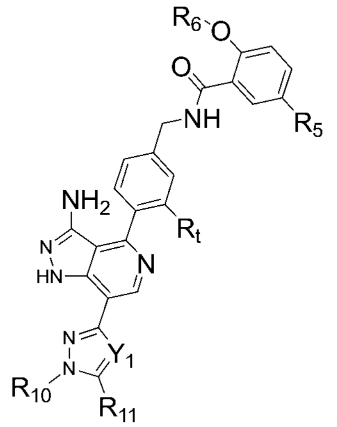

(II-1)

wherein,

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or -$CD_3$;

$R_t$ is H, D or halogen; alternatively, $R_t$ is H, D or F;

$Y_1$ is N, CH or CD;

$R_{10}$, $R_{11}$ and the atom to which they are attached are taken together to form 3- to 7-membered monocyclic heterocyclyl, which is optionally substituted with one or more R"; alternatively, $R_{10}$, $R_{11}$ and the atom to which they are attached are taken together to form 6-membered heterocyclyl having 1 or 2 ring heteroatoms N, which is optionally substituted with one or more R"; or

$R_{10}$, $R_{11}$ and the atom to which they are attached are taken together to form 6- to 12-membered fused bicyclic heterocycle, which is optionally substituted with one or more R"; alternatively, $R_{10}$, $R_{11}$ and the atom to which they are attached are taken together to form 8- to 10-membered fused bicyclic heterocycle having 1 or 2 ring heteroatoms N, which is optionally substituted with one or more R"; or

$R_{10}$, $R_{11}$ and the atom to which they are attached are taken together to form 5- to 10-membered heteroaryl, which is optionally substituted with one or more R"; alternatively, $R_{10}$, $R_{11}$ and the atom to which they are attached are taken together to form 6-membered heteroaryl having 1 or 2 ring heteroatoms N, which is optionally substituted with one or more R";

each R" is independently D, -C(O)O$R_g$, -C(O)N$R_h$$R_j$, -N$R_g$C(O)$R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**11.** The compound according to claim 10, or a tautomer, a stereoisomer, a prodrug, a crystal form, a pharmaceutically acceptable salt, a hydrate or a solvate thereof, wherein,

$R_5$ is F;
$R_6$ is methyl or $-CD_3$;
Rt is H, D or F;
$Y_1$ is N, CH or CD;
$R_{10}$, $R_{11}$ and the atom to which they are attached are taken together to form the following groups:

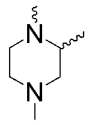 , 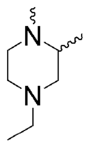 , 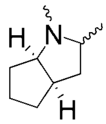 , 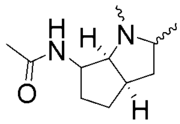 , 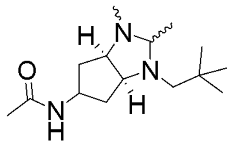 , 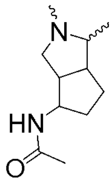 , 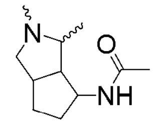 ,

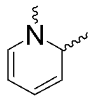 , 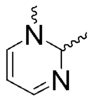 , 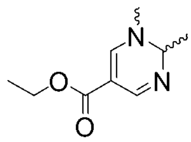 or 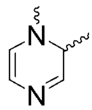

, wherein the groups are optionally substituted with one or more D, up to fully deuterated;
alternatively, $R_{10}$, $R_{11}$ and the atom to which they are attached are taken together to form the following groups:

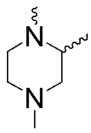 , 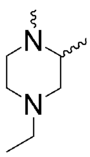 , 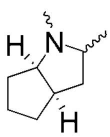 , 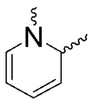 , 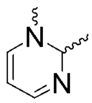 or 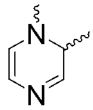 ,

wherein the groups are optionally substituted with one or more D, up to fully deuterated.

**12.** The compound according to claim 1 or 2, or a tautomer, a stereoisomer, a prodrug, a crystal form, a pharmaceutically acceptable salt, a hydrate or a solvate thereof, wherein the compound is a compound of formula (III):

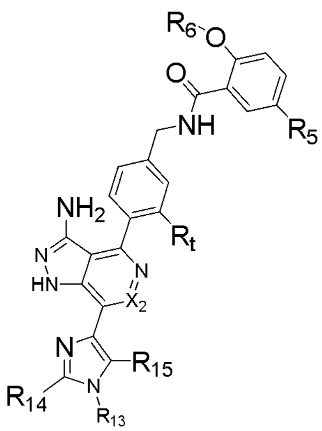

(III)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;

$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{13}$, $R_{14}$ and $R_{15}$ are independently:

1) H, D, halogen, oxo, -C(O)$R_d$, -C(O)O$R_d$, -C(O)N$R_eR_f$, -N$R_eR_f$, -N$R_d$C(O)$R_e$, -N$R_d$C(O)O$R_e$, - N$R_d$C(O)N$R_eR_f$, -O$R_d$, -OC(O)$R_d$, -OC(O)N$R_eR_f$, -N$R_d$S(O)$_2R_e$, -S(O)$_2$N$R_eR_f$, -S(O)$R_d$, -S(O)$_2R_d$, - P(=O)$R_eR_f$, -OP(=O)$R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{13}$ and $R_{14}$ or $R_{13}$ and $R_{15}$ and the atom to which they are attached are taken together to form 3- to 12-membered heterocyclyl or 5- to 10-membered heteroaryl, wherein the groups are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, -C(O)$R_g$, -C(O)O$R_g$, -C(O)N$R_hR_j$, -N$R_hR_j$, -N$R_g$C(O)$R_h$, - N$R_g$C(O)O$R_h$, -N$R_g$C(O)N$R_hR_j$, -O$R_g$, -OC(O)$R_g$, -OC(O)N$R_hR_j$, -N$R_g$S(O)$_2R_h$, -S(O)$_2$N$R_hR_j$, -S(O)$R_g$, - S(O)$_2R_g$, -P(=O)$R_hR_j$, -OP(=O)$R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

**13.** The compound according to claim 12, or a tautomer, a stereoisomer, a prodrug, a crystal form, a pharmaceutically acceptable salt, a hydrate or a solvate thereof, wherein,

$X_2$ is N or C$R_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{13}$ is H, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more R";
$R_{14}$ and $R_{15}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{13}$ and $R_{14}$ or $R_{13}$ and $R_{15}$ and the atom to which they are attached are taken together to form 3- to 7-membered monocyclic heterocyclyl, 6- to 12-membered fused bicyclic heterocycle or 5- to 10-membered heteroaryl, wherein the groups are optionally substituted with one or more R";
each R" is independently D, -C(O)O$R_g$, -C(O)N$R_hR_j$, -N$R_g$C(O)$R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**14.** The compound according to claim 12 or 13, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
$R_t$ is H, D or halogen; alternatively, $R_t$ is H, D or F;
$R_{13}$ is H, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{13}$ is H, $C_{1-3}$ alkyl, $C_{1-3}$ haloalkyl or 6-membered heterocyclyl having 1 or 2 ring heteroatoms N, wherein the $C_{1-3}$ alkyl, $C_{1-3}$ haloalkyl and 6-membered heterocyclyl are optionally substituted with one or more R";
$R_{14}$ and $R_{15}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{14}$ and $R_{15}$ are independently H or D;
or, $R_{13}$ and $R_{14}$ or $R_{13}$ and $R_{15}$ and the atom to which they are attached are taken together to form 3- to 7-membered monocyclic heterocyclyl, which is optionally substituted with one or more R"; alternatively, $R_{13}$ and $R_{14}$ or $R_{13}$ and $R_{15}$ and the atom to which they are attached are taken together to form 6-membered heterocyclyl having 1 or 2 ring heteroatoms N, which is optionally substituted with one or more R"; or
$R_{13}$ and $R_{14}$ or $R_{13}$ and $R_{15}$ and the atom to which they are attached are taken together to form 6- to 12-membered fused bicyclic heterocycle, which is optionally substituted with one or more R"; alternatively, $R_{13}$ and $R_{14}$ or $R_{13}$ and $R_{15}$ and the atom to which they are attached are taken together to form 8- to 10-membered fused bicyclic heterocycle having 1 or 2 ring heteroatoms N, which is optionally substituted with one or more R"; or
$R_{13}$ and $R_{14}$ or $R_{13}$ and $R_{15}$ and the atom to which they are attached are taken together to form 5- to 10-membered heteroaryl, which is optionally substituted with one or more R"; alternatively, $R_{13}$ and $R_{14}$ or $R_{13}$ and $R_{15}$ and the atom to which they are attached are taken together to form 6-membered heteroaryl having 1 or 2 ring heteroatoms N, which is optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**15.** The compound according to claim 1 or 2, or a tautomer, a stereoisomer, a prodrug, a crystal form, a pharmaceutically acceptable salt, a hydrate or a solvate thereof, wherein the compound is a compound of formula (III-1):

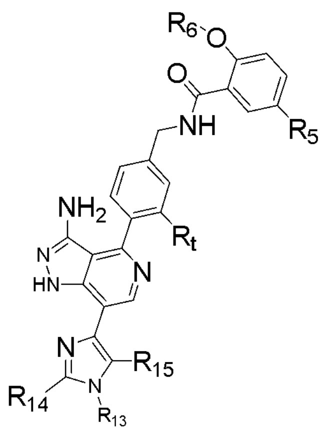

(III-1)

wherein,

$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more

D, up to fully deuterated; alternatively, $R_6$ is methyl or $-CD_3$;
$R_t$ is H, D or halogen; alternatively, $R_t$ is H, D or F;
$R_{13}$ and $R_{14}$ or $R_{13}$ and $R_{15}$ and the atom to which they are attached are taken together to form 3- to 7-membered monocyclic heterocyclyl, which is optionally substituted with one or more R"; alternatively, $R_{13}$ and $R_{14}$ or $R_{13}$ and $R_{15}$ and the atom to which they are attached are taken together to form 6-membered heterocyclyl having 1 or 2 ring heteroatoms N, which is optionally substituted with one or more R"; or
$R_{13}$ and $R_{14}$ or $R_{13}$ and $R_{15}$ and the atom to which they are attached are taken together to form 6- to 12-membered fused bicyclic heterocycle, which is optionally substituted with one or more R"; alternatively, $R_{13}$ and $R_{14}$ or $R_{13}$ and $R_{15}$ and the atom to which they are attached are taken together to form 8- to 10-membered fused bicyclic heterocycle having 1 or 2 ring heteroatoms N, which is optionally substituted with one or more R"; or
$R_{13}$ and $R_{14}$ or $R_{13}$ and $R_{15}$ and the atom to which they are attached are taken together to form 5- to 10-membered heteroaryl, which is optionally substituted with one or more R"; alternatively, $R_{13}$ and $R_{14}$ or $R_{13}$ and $R_{15}$ and the atom to which they are attached are taken together to form 6-membered heteroaryl having 1 or 2 ring heteroatoms N, which is optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**16.** The compound according to claim 15, or a tautomer, a stereoisomer, a prodrug, a crystal form, a pharmaceutically acceptable salt, a hydrate or a solvate thereof, wherein,

$R_5$ is F;
$R_6$ is methyl or $-CD_3$;
Rt is H, D or F;
$R_{13}$ and $R_{14}$ or $R_{13}$ and $R_{15}$ and the atom to which they are attached are taken together to form

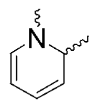,

wherein the group is optionally substituted with one or more D, up to fully deuterated.

**17.** The compound according to claim 1 or 2, or a tautomer, a stereoisomer, a prodrug, a crystal form, a pharmaceutically acceptable salt, a hydrate or a solvate thereof, wherein the compound is a compound of formula (IV):

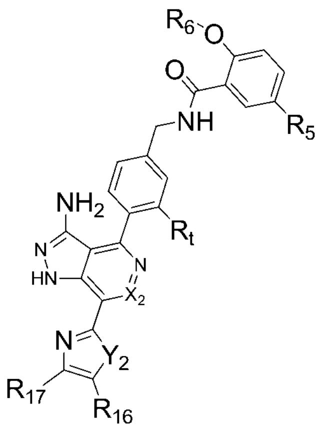

(IV)

wherein,

$X_2$ is N or $CR_2$;

$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_2$ is O or S;
$R_{16}$ and $R_{17}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, - $NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, - $P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{16}$, $R_{17}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, $-C(O)R_g$, $-C(O)OR_g$, $-C(O)NR_hR_j$, $-NR_hR_j$, $-NR_gC(O)R_h$, - $NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, - $S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

**18.** The compound according to claim 17, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_2$ is O or S;
$R_{16}$ and $R_{17}$ are independently H, D, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more R";
or, $R_{16}$, $R_{17}$ and the atom to which they are attached are taken together to form $C_{3-6}$ cycloalkyl, which is optionally substituted with one or more R"; or
$R_{16}$, $R_{17}$ and the atom to which they are attached are taken together to form 3- to 7-membered monocyclic heterocyclyl, which is optionally substituted with one or more R"; or
$R_{16}$, $R_{17}$ and the atom to which they are attached are taken together to form 6- to 12-membered fused bicyclic

heterocycle, which is optionally substituted with one or more R"; or

$R_{16}$, $R_{17}$ and the atom to which they are attached are taken together to form $C_{6-10}$ aryl, which is optionally substituted with one or more R"; or

$R_{16}$, $R_{17}$ and the atom to which they are attached are taken together to form 5- to 10-membered heteroaryl, which is optionally substituted with one or more R";

each R" is independently D, -C(O)OR$_g$, -C(O)NR$_h$R$_j$, -NR$_g$C(O)R$_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**19.** The compound according to claim 17 or 18, or a tautomer, a stereoisomer, a prodrug, a crystal form, a pharmaceutically acceptable salt, a hydrate or a solvate thereof, wherein,

$X_2$ is N or $CR_2$;

$R_2$ is H, D or halogen; alternatively, $R_2$ is H, D or F; alternatively, $R_2$ is H;

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

$R_t$ is H, D or halogen; alternatively, $R_t$ is H, D or F;

$Y_2$ is O or S;

$R_{16}$ and $R_{17}$ are independently H, D, $C_{1-6}$ alkyl or $C_{1-6}$ haloalkyl, wherein the $C_{1-6}$ alkyl and $C_{1-6}$ haloalkyl are optionally substituted with one or more R"; alternatively, $R_{10}$ is H, D, $C_{1-3}$ alkyl or $C_{1-3}$ haloalkyl, wherein the $C_{1-3}$ alkyl and $C_{1-3}$ haloalkyl are optionally substituted with one or more D, up to fully deuterated;

or, $R_{16}$, $R_{17}$ and the atom to which they are attached are taken together to form $C_{3-6}$ cycloalkyl, which is optionally substituted with one or more R"; alternatively, $R_{16}$, $R_{17}$ and the atom to which they are attached are taken together to form cyclohexyl, which is optionally substituted with one or more R"; or

$R_{16}$, $R_{17}$ and the atom to which they are attached are taken together to form 3- to 7-membered monocyclic heterocyclyl, which is optionally substituted with one or more R"; alternatively, $R_{16}$, $R_{17}$ and the atom to which they are attached are taken together to form 6-membered heterocyclyl having 1 or 2 ring heteroatoms N, which is optionally substituted with one or more R"; or

$R_{16}$, $R_{17}$ and the atom to which they are attached are taken together to form 6- to 12-membered fused bicyclic heterocycle, which is optionally substituted with one or more R"; alternatively, $R_{16}$, $R_{17}$ and the atom to which they are attached are taken together to form 8- to 10-membered fused bicyclic heterocycle having 1 or 2 ring heteroatoms N, which is optionally substituted with one or more R"; or

$R_{16}$, $R_{17}$ and the atom to which they are attached are taken together to form $C_{6-10}$ aryl, which is optionally substituted with one or more R"; alternatively, $R_{16}$, $R_{17}$ and the atom to which they are attached are taken together to form phenyl, which is optionally substituted with one or more R"; or

$R_{16}$, $R_{17}$ and the atom to which they are attached are taken together to form 5- to 10-membered heteroaryl, which is optionally substituted with one or more R"; alternatively, $R_{16}$, $R_{17}$ and the atom to which they are attached are taken together to form 6-membered heteroaryl having 1 or 2 ring heteroatoms N, which is optionally substituted with one or more R";

each R" is independently D, -C(O)OR$_g$, -C(O)NR$_h$R$_j$, -NR$_g$C(O)R$_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;

wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**20.** The compound according to claim 1 or 2, or a tautomer, a stereoisomer, a prodrug, a crystal form, a pharmaceutically acceptable salt, a hydrate or a solvate thereof, wherein the compound is a compound of formula (IV-1):

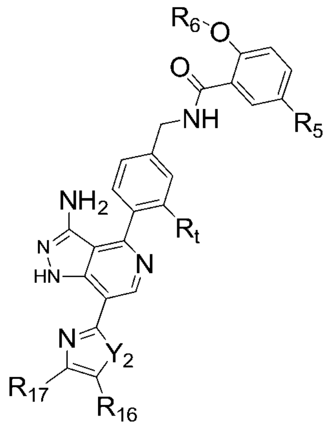

(IV-1)

wherein,

$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
$R_t$ is H, D or halogen; alternatively, $R_t$ is H, D or F;
$Y_2$ is O or S;
$R_{16}$, $R_{17}$ and the atom to which they are attached are taken together to form $C_{3-6}$ cycloalkyl, which is optionally substituted with one or more R"; alternatively, $R_{16}$, $R_{17}$ and the atom to which they are attached are taken together to form cyclohexyl, which is optionally substituted with one or more R"; or
$R_{16}$, $R_{17}$ and the atom to which they are attached are taken together to form 3- to 7-membered monocyclic heterocyclyl, which is optionally substituted with one or more R"; alternatively, $R_{16}$, $R_{17}$ and the atom to which they are attached are taken together to form 6-membered heterocyclyl having 1 or 2 ring heteroatoms N, which is optionally substituted with one or more R"; or
$R_{16}$, $R_{17}$ and the atom to which they are attached are taken together to form 6- to 12-membered fused bicyclic heterocycle, which is optionally substituted with one or more R"; alternatively, $R_{16}$, $R_{17}$ and the atom to which they are attached are taken together to form 8- to 10-membered fused bicyclic heterocycle having 1 or 2 ring heteroatoms N, which is optionally substituted with one or more R"; or
$R_{16}$, $R_{17}$ and the atom to which they are attached are taken together to form $C_{6-10}$ aryl, which is optionally substituted with one or more R"; alternatively, $R_{16}$, $R_{17}$ and the atom to which they are attached are taken together to form phenyl, which is optionally substituted with one or more R"; or
$R_{16}$, $R_{17}$ and the atom to which they are attached are taken together to form 5- to 10-membered heteroaryl, which is optionally substituted with one or more R"; alternatively, $R_{16}$, $R_{17}$ and the atom to which they are attached are taken together to form 6-membered heteroaryl having 1 or 2 ring heteroatoms N, which is optionally substituted with one or more R";
each R" is independently D, $-C(O)OR_g$, $-C(O)NR_hR_j$, $-NR_gC(O)R_h$, $C_{1-6}$ alkyl, $C_{1-6}$ haloalkyl, $C_{3-6}$ cycloalkyl or 3- to 7-membered heterocyclyl, wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated; alternatively, each R" is independently D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**21.** The compound according to claim 20, or a tautomer, a stereoisomer, a prodrug, a crystal form, a pharmaceutically acceptable salt, a hydrate or a solvate thereof, wherein,

$R_5$ is F;
$R_6$ is methyl or $-CD_3$;
Rt is H, D or F;
$Y_2$ is O or S;
$R_{16}$, $R_{17}$ and the atom to which they are attached are taken together to form the following groups:

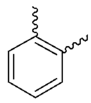, 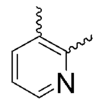 or 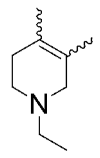

, wherein the groups are optionally substituted with one or more D, up to fully deuterated.

**22.** The compound according to claim 1 or 2, or a tautomer, a stereoisomer, a prodrug, a crystal form, a pharmaceutically acceptable salt, a hydrate or a solvate thereof, wherein the compound is a compound of formula (V):

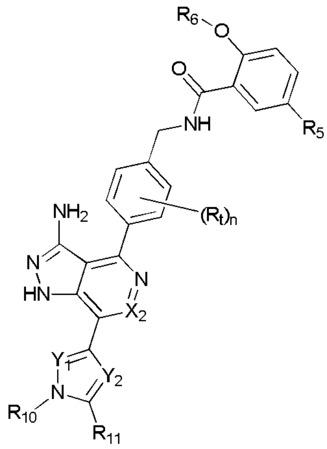

(V)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$ is H, $C_{1-6}$ alkyl, $C_{1-6}$ haloalkyl, $C_{4-6}$ cycloalkyl or 5- to 6-membered heterocyclyl, wherein the $C_{1-6}$ alkyl, $C_{1-6}$ haloalkyl, $C_{4-6}$ cycloalkyl and 5- to 6-membered heterocyclyl are optionally substituted with one or more R";
$R_{11}$, $R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{10}$, $R_{11}$ and the atom to which they are attached are taken together to form 5- to 10-membered heterocyclyl or 5- to 6-membered heteroaryl, wherein the 5- to 10-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is H, D, halogen, oxo, $-C(O)R_g$, $-C(O)OR_g$, $-C(O)NR_hR_j$, $-NR_hR_j$, $-NR_gC(O)R_h$, $-OR_g$, $-OC(O)R_g$, $-NR_gS(O)_2R_h$, $-S(O)_2NR_hR_j$, $-S(O)R_g$, $-S(O)_2R_g$, $C_{1-6}$ alkyl, $C_{1-6}$ haloalkyl, $C_{4-6}$ cycloalkyl or 4- to 6-membered heterocyclyl, wherein the $C_{1-6}$ alkyl, $C_{1-6}$ haloalkyl, $C_{4-6}$ cycloalkyl and 4- to 6-membered heterocyclyl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

**23.** The compound according to claim 22, or a tautomer, a stereoisomer, a prodrug, a crystal form, a pharmaceutically acceptable salt, a hydrate or a solvate thereof, wherein,

$X_2$ is $CR_2$;
$R_2$ is H, D or $-CH(CH_3)(CF_3)$;
$R_5$ is F;
$R_6$ is Me;
$R_t$ is H or D;
n is 0, 1, 2, 3 or 4;
$Y_1$ is N or $CR_{12}$; alternatively, $Y_1$ is N;
$Y_2$ is N or $CR_{13}$;
$R_{10}$ is H, Me, $CHF_2$, $CH_2CF_3$ or piperidyl, which is optionally substituted with one or more R";
$R_{11}$ is H, D or Me;
$R_{12}$ and $R_{13}$ are independently H or D;
or, $R_{10}$, $R_{11}$ and the atom to which they are attached are taken together to form 5- to 10-membered heterocyclyl or 5- to 6-membered heteroaryl, wherein the 5- to 10-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is Me, Et, $-C(O)OR_g$, $-C(O)NR_hR_j$, $-NR_gC(O)R_h$ or oxetanyl;
each of $R_g$, $R_h$ and $R_j$ is independently H, D, $C_{1-6}$ alkyl or $C_{1-6}$ haloalkyl.

**24.** The compound according to claim 22, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$ is H, $C_{1-6}$ alkyl, $C_{1-6}$ haloalkyl, $C_{4-6}$ cycloalkyl or 5- to 6-membered heterocyclyl, wherein the $C_{1-6}$ alkyl, $C_{1-6}$ haloalkyl, $C_{4-6}$ cycloalkyl and 5- to 6-membered heterocyclyl are optionally substituted with one or more R";
$R_{11}$, $R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is H, D, halogen, oxo, $-C(O)R_g$, $-C(O)OR_g$, $-C(O)NR_hR_j$, $-NR_hR_j$, $-NR_gC(O)R_h$, $-OR_g$, $-OC(O)R_g$, $-NR_gS(O)_2R_h$, $-S(O)_2NR_hR_j$, $-S(O)R_g$, $-S(O)_2R_g$, $C_{1-6}$ alkyl, $C_{1-6}$ haloalkyl, $C_{4-6}$ cycloalkyl or 4- to 6-membered heterocyclyl, wherein the $C_{1-6}$ alkyl, $C_{1-6}$ haloalkyl, $C_{4-6}$ cycloalkyl and 4- to 6-membered heterocyclyl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

**25.** The compound according to claim 22, or a tautomer, a stereoisomer, a prodrug, a crystal form, a pharmaceutically acceptable salt, a hydrate or a solvate thereof, wherein,

$X_2$ is $CR_2$;

$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$ is H, $C_{1-6}$ alkyl, $C_{1-6}$ haloalkyl or 5- to 6-membered heterocyclyl, wherein the $C_{1-6}$ alkyl, $C_{1-6}$ haloalkyl and 5- to 6-membered heterocyclyl are optionally substituted with one or more R";
$R_{11}$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{12}$ and $R_{13}$ are independently H, D or halogen;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is H, D, halogen, -C(O)$OR_g$, -C(O)$NR_hR_j$, $C_{1-6}$ alkyl, $C_{1-6}$ haloalkyl or 4- to 6-membered heterocyclyl, wherein the $C_{1-6}$ alkyl, $C_{1-6}$ haloalkyl and 4- to 6-membered heterocyclyl are optionally substituted with one or more D, up to fully deuterated;
wherein each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**26.** The compound according to claim 22, or a tautomer, a stereoisomer, a prodrug, a crystal form, a pharmaceutically acceptable salt, a hydrate or a solvate thereof, wherein,

$X_2$ is $CR_2$;
$R_2$ is H, D or $-CH(CH_3)(CF_3)$;
$R_5$ is F;
$R_6$ is Me;
$R_t$ is H or D;
n is 0, 1, 2, 3 or 4;
$Y_1$ is N or $CR_{12}$; alternatively, $Y_1$ is N;
$Y_2$ is N or $CR_{13}$;
$R_{10}$ is H, Me, $CHF_2$, $CH_2CF_3$ or piperidyl, which is optionally substituted with one or more R";
$R_{11}$ is H, D or Me;
$R_{12}$ and $R_{13}$ are independently H or D;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is Me, Et, -C(O)$OR_g$, -C(O)$NR_hR_j$, or oxetanyl;
wherein each of $R_g$, $R_h$ and $R_j$ is independently H, D, $C_{1-6}$ alkyl or $C_{1-6}$ haloalkyl.

**27.** The compound according to claim 22, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted

with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$, $R_{11}$, $R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**28.** The compound according to claim 22, or a tautomer, a stereoisomer, a prodrug, a crystal form, a pharmaceutically acceptable salt, a hydrate or a solvate thereof, wherein,

$X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_1$ is N or $CR_{12}$; alternatively, $Y_1$ is N;
$Y_2$ is N or $CR_{13}$;
$R_{10}$ is H, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{11}$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{12}$ and $R_{13}$ are independently H, D or halogen;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R";
wherein R" is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**29.** The compound according to claim 22, or a tautomer, a stereoisomer, a prodrug, a crystal form, a pharmaceutically acceptable salt, a hydrate or a solvate thereof, wherein,

$X_2$ is $CR_2$;
$R_2$ is H, D or $-CH(CH_3)(CF_3)$;
$R_5$ is F;
$R_6$ is Me;
$R_t$ is H or D;
n is 0, 1, 2, 3 or 4;
$Y_1$ is N or $CR_{12}$; alternatively, $Y_1$ is N;
$Y_2$ is N or $CR_{13}$;
$R_{10}$ is H, Me, $CHF_2$ or $CH_2CF_3$;
$R_{11}$ is H, D or Me;
$R_{12}$ and $R_{13}$ are independently H or D;
or, $R_{10}$, $R_{11}$ and the atom to which they are attached are taken together to form 5- to 6-membered heterocyclyl or 5- to 6-membered heteroaryl, wherein the 5- to 6-membered heterocyclyl and 5- to 6-membered heteroaryl are optionally substituted with one or more R"; or, when $Y_1$ is $CR_{12}$, $R_{10}$, $R_{12}$ and the atom to which they are attached

are taken together to form 5- to 6-membered heterocyclyl or 5- to 6-membered heteroaryl, wherein the 5- to 6-membered heterocyclyl and 5- to 6-membered heteroaryl are optionally substituted with one or more R"; wherein R" is Me or Et.

**30.** The compound according to claim 1 or 2, or a tautomer, a stereoisomer, a prodrug, a crystal form, a pharmaceutically acceptable salt, a hydrate or a solvate thereof, wherein the compound is a compound of formula (VI):

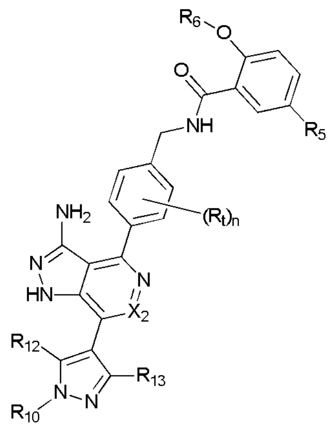

(VI)

wherein,

$X_2$ is N or $CR_2$; alternatively, $X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$R_{10}$ is H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{12}$ and $R_{13}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**31.** The compound according to claim 30, or a tautomer, a stereoisomer, a prodrug, a crystal form, a pharmaceutically acceptable salt, a hydrate or a solvate thereof, wherein,

$X_2$ is $CR_2$;
$R_2$ is H, D or $-CH(CH_3)(CF_3)$;
$R_5$ is F;
$R_6$ is Me;
$R_t$ is H or D;
n is 0, 1, 2, 3 or 4;
$R_{10}$ is H, Me, $CHF_2$ or $CH_2CF_3$;
$R_{12}$ and $R_{13}$ are independently H or D.

**32.** The compound according to claim 1 or 2, or a tautomer, a stereoisomer, a prodrug, a crystal form, a pharmaceutically acceptable salt, a hydrate or a solvate thereof, wherein the compound is a compound of formula (VII):

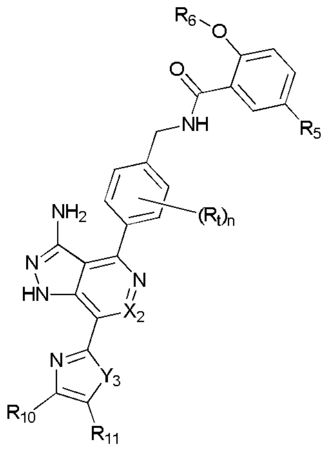

(VII)

wherein,

$X_2$ is N or $CR_2$; alternatively, $X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Y_3$ is O or S;
$R_{10}$ and $R_{11}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{10}$, $R_{11}$ and the atom to which they are attached are taken together to form $C_{5-8}$ cycloalkyl, 5- to 6-membered heterocyclyl, $C_{6-10}$ aryl or 5- to 6-membered heteroaryl, wherein the $C_{5-8}$ cycloalkyl, 5- to 6-membered heterocyclyl, $C_{6-10}$ aryl and 5- to 6-membered heteroaryl are optionally substituted with one or more R";
wherein R" is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**33.** The compound according to claim 32, or a tautomer, a stereoisomer, a prodrug, a crystal form, a pharmaceutically acceptable salt, a hydrate or a solvate thereof, wherein,

$X_2$ is $CR_2$;
$R_2$ is H, D, halogen or $-CH(CH_3)(CF_3)$;
$R_5$ is F;
$R_6$ is Me;
$R_t$ is H or D;
n is 0, 1, 2, 3 or 4;
$Y_3$ is O or S;
$R_{10}$ is H, Me, $CHF_2$ or $CH_2CF_3$;
$R_{11}$ is H or D;
or, $R_{10}$, $R_{11}$ and the atom to which they are attached are taken together to form $C_{5-8}$ cycloalkyl, 5- to 6-membered heterocyclyl, $C_{6-10}$ aryl or 5- to 6-membered heteroaryl, wherein the $C_{5-8}$ cycloalkyl, 5- to 6-membered heterocyclyl, $C_{6-10}$ aryl and 5- to 6-membered heteroaryl are optionally substituted with one or more R";
wherein R" is Me or Et.

**34.** The compound according to claim 1 or 2, or a tautomer, a stereoisomer, a prodrug, a crystal form, a pharmaceutically acceptable salt, a hydrate or a solvate thereof, wherein the compound is a compound of formula (VIII), (VIII-1) or (VIII-2):

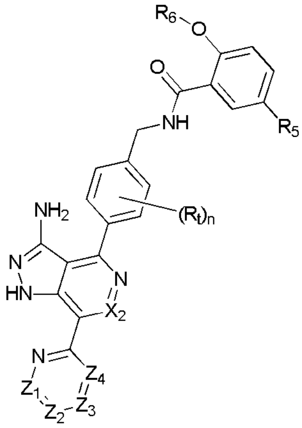 (VIII), 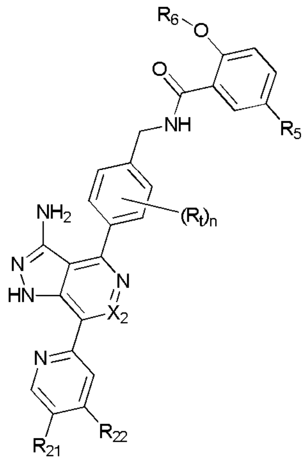 (VIII-1) or 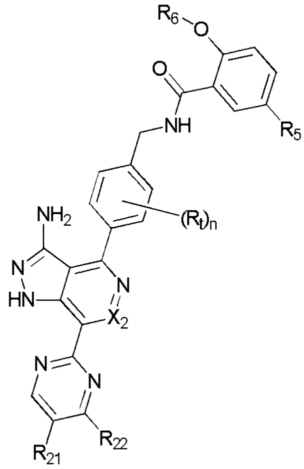 (VIII-2)

wherein,

$X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_2$ is H or D;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$, $R_{22}$ and $R_{23}$ are independently H, D, halogen, $-OC(O)R_d$, $-NR_dC(O)R_e$, $-C(O)NR_eR_f$, $-C(O)OR_d$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated, and $R_{20}$, $R_{22}$ and $R_{23}$ are independently and optionally substituted with one or more R"; $R_{21}$ is H, D, halogen, $-OR_d$, $-NR_eR_f$, $-C(O)R_d$, $-OC(O)R_d$, $-NR_dC(O)R_e$, $-C(O)NR_eR_f$, $-C(O)OR_d$, $-S(O)_2NR_eR_f$, $-S(O)R_d$, $-S(O)_2R_d$, $-P(=O)R_eR_f$, $-OP(=O)R_eR_f$, $C_{1-6}$ alkyl, $C_{1-6}$ haloalkyl, $C_{3-8}$ cycloalkyl or 4- to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more D, up to fully deuterated, and $R_{21}$ is optionally substituted with one or more R";
wherein each of $R_d$, $R_e$ and $R_f$ is independently H, D, $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl and 5- to 10-membered heteroaryl are optionally substituted with one or more D, up to fully deuterated, and optionally substituted with one or more R";
wherein R" is H, D, halogen, $-OR_g$, $-NR_hR_j$, $C_{1-6}$ alkyl, $C_{1-6}$ haloalkyl, $C_{3-8}$ cycloalkyl or 4- to 7-membered heterocyclyl, wherein R" is optionally substituted with one or more D, halogen, $C_{1-6}$ alkyl or $C_{1-6}$ haloalkyl; each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

**35.** The compound according to claim 34, or a tautomer, a stereoisomer, a prodrug, a crystal form, a pharmaceutically acceptable salt, a hydrate or a solvate thereof, wherein,

$X_2$ is $CR_2$;
$R_2$ is H, D, F, Me, $CHF_2$, $CF_3$ or $-CH(CH_3)(CF_3)$; alternatively, $R_2$ is H or D;
$R_5$ is F;
$R_6$ is Me;

$R_t$ is H or D;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$ is H or D;
$R_{21}$ is H, D, F, Cl, Me, $CHF_2$, $CF_3$, $CH_2CF_3$, -C(OH)$(CH_3)_2$, -$OR_d$, -$NR_dC(O)R_e$, -$S(O)R_d$, -$S(O)_2R_d$, - $P(=O)R_eR_f$ or 4- to 7-membered heterocyclyl, which is optionally substituted with one or more R";
$R_{22}$ is H, D, -$NR_dC(O)R_e$ or -$C(O)NR_eR_f$, which is optionally substituted with one or more R";
$R_{23}$ is H or D;
wherein each of $R_d$, $R_e$ and $R_f$ is independently H, D, $C_{1-6}$ alkyl, $C_{1-6}$ haloalkyl, $C_{3-8}$ cycloalkyl or 4- to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more R";
wherein R" is Me, Et, -$OR_g$, -$NR_hR_j$ or 4- to 7-membered heterocyclyl, wherein R" is optionally substituted with one or more D, halogen, $C_{1-6}$ alkyl or $C_{1-6}$ haloalkyl;
each of $R_g$, $R_h$ and $R_j$ is independently H, D, $C_{1-6}$ alkyl or $C_{1-6}$ haloalkyl.

**36.** The compound according to claim 34, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_2$ is H or D;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$, $R_{22}$ and $R_{23}$ are independently H, D, halogen, -$OC(O)R_d$, -$NR_dC(O)R_e$, -$C(O)NR_eR_f$, -$C(O)OR_d$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated, and $R_{20}$, $R_{22}$ and $R_{23}$ are independently and optionally substituted with one or more R"; $R_{21}$ is H, D, halogen, -$OR_d$, -$NR_eR_f$, -$OC(O)R_d$, -$NR_dC(O)R_e$, -$C(O)NR_eR_f$, -$C(O)OR_d$, -$S(O)R_d$, -$S(O)_2R_d$, $C_{1-6}$ alkyl, $C_{1-6}$ haloalkyl, $C_{3-8}$ cycloalkyl or 4- to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more D, up to fully deuterated, and $R_{21}$ is optionally substituted with one or more R";
wherein each of $R_d$, $R_e$ and $R_f$ is independently H, D, $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-8}$ cycloalkyl, 3- to 7-membered heterocyclyl, $C_{6-10}$ aryl and 5- to 10-membered heteroaryl are optionally substituted with one or more D, up to fully deuterated, and optionally substituted with one or more R";
wherein R" is H, D, halogen, -$OR_g$, -$NR_hR_j$, $C_{1-6}$ alkyl, $C_{1-6}$ haloalkyl, $C_{3-8}$ cycloalkyl or 4- to 7-membered heterocyclyl, wherein R" is optionally substituted with one or more D, halogen, $C_{1-6}$ alkyl or $C_{1-6}$ haloalkyl; each of $R_g$, $R_h$ and $R_j$ is independently H, D, $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl or 5- to 10-membered heteroaryl, wherein the $C_{1-6}$ alkyl, $C_{1-6}$ haloalkyl, $C_{2-6}$ alkenyl, $C_{2-6}$ alkynyl, $C_{3-6}$ cycloalkyl, 3- to 7-membered heterocyclyl, $C_{6-10}$ aryl and 5- to 10-membered heteroaryl are optionally substituted with one or more D, up to fully deuterated.

**37.** The compound according to claim 34, or a tautomer, a stereoisomer, a prodrug, a crystal form, a pharmaceutically acceptable salt, a hydrate or a solvate thereof, wherein,

$X_2$ is $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted

with one or more D, up to fully deuterated; alternatively, $R_2$ is H or D;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
n is 0, 1, 2, 3 or 4;
$Z_1$ is N or $CR_{20}$;
$Z_2$ is N or $CR_{21}$;
$Z_3$ is N or $CR_{22}$;
$Z_4$ is N or $CR_{23}$;
$R_{20}$ and $R_{23}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{21}$ is H, D, halogen, $-OR_d$, $-NR_dC(O)R_e$, $-S(O)R_d$, $-S(O)_2R_d$, $C_{1-6}$ alkyl, $C_{1-6}$ haloalkyl or 4- to 7-membered heterocyclyl, wherein the $C_{1-6}$ alkyl, $C_{1-6}$ haloalkyl and 4- to 7-membered heterocyclyl are optionally substituted with one or more D, up to fully deuterated, and $R_{21}$ is optionally substituted with one or more R"; $R_{22}$ is H, D, $-NR_dC(O)R_e$ or $-C(O)NR_eR_f$, which is optionally substituted with one or more R";
wherein each of $R_d$, $R_e$ and $R_f$ is independently H, D, $C_{1-6}$ alkyl, $C_{1-6}$ haloalkyl, $C_{3-8}$ cycloalkyl or 4- to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more R";
wherein R" is H, D, halogen, $-OR_g$, $-NR_hR_j$, $C_{1-6}$ alkyl, $C_{1-6}$ haloalkyl, $C_{3-8}$ cycloalkyl or 4- to 7-membered heterocyclyl, wherein R" is optionally substituted with one or more D, halogen, $C_{1-6}$ alkyl or $C_{1-6}$ haloalkyl; each of $R_g$, $R_h$ and $R_j$ is independently H, D, $C_{1-6}$ alkyl or $C_{1-6}$ haloalkyl, wherein the $C_{1-6}$ alkyl and $C_{1-6}$ haloalkyl are optionally substituted with one or more D, up to fully deuterated.

**38.** The compound according to claim 34, or a tautomer, a stereoisomer, a prodrug, a crystal form, a pharmaceutically acceptable salt, a hydrate or a solvate thereof, wherein,

$X_2$ is $CR_2$;
$R_2$ is H, D, Me, $CHF_2$ or $-CH(CH_3)(CF_3)$; alternatively, $R_2$ is H or D;
$R_5$ is F;
$R_6$ is Me;
$R_t$ is H or D;
n is 0, 1, 2, 3 or 4;
Zi is N or $CR_{20}$;
$Z_2$ is N or $CR_{21}$;
$Z_3$ is N or $CR_{22}$;
$Z_4$ is N or $CR_{23}$;
$R_{20}$ is H or D;
$R_{21}$ is H, D, F, Cl, Me, $CHF_2$, $CF_3$, $CH_2CF_3$, $-C(OH)(CH_3)_2$, $-OR_d$, $-NR_dC(O)R_e$, $-S(O)R_d$, $-S(O)_2R_d$ or 4- to 7-membered heterocyclyl, which is optionally substituted with one or more R";
$R_{22}$ is H, D, $-NR_dC(O)R_e$ or $-C(O)NR_eR_f$, which is optionally substituted with one or more R";
$R_{23}$ is H or D;
wherein each of $R_d$, $R_e$ and $R_f$ is independently H, D, $C_{1-6}$ alkyl, $C_{1-6}$ haloalkyl, $C_{3-8}$ cycloalkyl or 4- to 7-membered heterocyclyl, wherein the $C_{1-6}$ alkyl, $C_{1-6}$ haloalkyl, $C_{3-8}$ cycloalkyl and 4- to 7-membered heterocyclyl are optionally substituted with one or more R";
wherein R" is Me, Et, $-OR_g$, $-NR_hR_j$ or 4- to 7-membered heterocyclyl, wherein R" is optionally substituted with one or more D, halogen, $C_{1-6}$ alkyl or $C_{1-6}$ haloalkyl;
each of $R_g$, $R_h$ and $R_j$ is independently H, D, $C_{1-6}$ alkyl or $C_{1-6}$ haloalkyl.

**39.** The compound according to claim 1 or 2, or a tautomer, a stereoisomer, a prodrug, a crystal form, a pharmaceutically acceptable salt, a hydrate or a solvate thereof, wherein the compound is a compound of formula (IX):

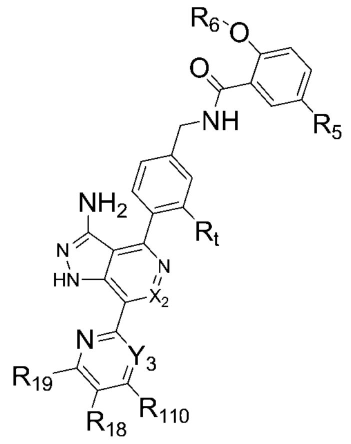

(IX)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N or $CR_{111}$;
$R_{18}$, $R_{19}$, $R_{110}$ and $R_{111}$ are independently:

1) H, D, halogen, oxo, -C(O)$R_d$, -C(O)O$R_d$, -C(O)N$R_eR_f$, -N$R_eR_f$, -N$R_d$C(O)$R_e$, -N$R_d$C(O)O$R_e$, - N$R_d$C(O)N$R_eR_f$, -O$R_d$, -OC(O)$R_d$, -OC(O)N$R_eR_f$, -N$R_d$S(O)$_2R_e$, -S(O)$_2$N$R_eR_f$, -S(O)$R_d$, -S(O)$_2R_d$, - P(=O)$R_eR_f$, -OP(=O)$R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{18}$ and $R_{19}$ or $R_{18}$ and $R_{110}$ or $R_{110}$ and $R_{111}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, -C(O)$R_g$, -C(O)O$R_g$, -C(O)N$R_hR_j$, -N$R_hR_j$, -N$R_g$C(O)$R_h$, - N$R_g$C(O)O$R_h$, -N$R_g$C(O)N$R_hR_j$, -O$R_g$, -OC(O)$R_g$, -OC(O)N$R_hR_j$, -N$R_g$S(O)$_2R_h$, -S(O)$_2$N$R_hR_j$, -S(O)$R_g$, - S(O)$_2R_g$, -P(=O)$R_hR_j$, -OP(=O)$R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the N atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

**40.** The compound according to claim 39, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N or $CR_{111}$;
$R_{18}$ is H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3-to 7-membered heterocyclyl, wherein the $C_{1-6}$ alkyl, $C_{1-6}$ haloalkyl and 3- to 7-membered heterocyclyl are optionally substituted with one or more R";
$R_{110}$ is H, D, halogen, $-NR_dC(O)R_e$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_{19}$ and $R_{111}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
or, $R_{18}$ and $R_{19}$ or $R_{18}$ and $R_{110}$ or $R_{110}$ and $R_{111}$ and the atom to which they are attached are taken together to form $C_{3-6}$ cycloalkyl, 3- to 7-membered monocyclic heterocyclyl, 6- to 12-membered fused bicyclic heterocycle, $C_{6-10}$ aryl or 5- to 10-membered heteroaryl, wherein the groups are optionally substituted with one or more R"; or
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**41.** The compound according to claim 39 or 40, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
Rt is H, D or halogen; alternatively, $R_t$ is H, D or F;
$Y_3$ is N or $CR_{111}$;
$R_{18}$ is H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3-to 7-membered heterocyclyl, wherein the $C_{1-6}$ alkyl, $C_{1-6}$ haloalkyl and 3- to 7-membered heterocyclyl are optionally substituted with one or more R"; alternatively, $R_{18}$ is halogen, $-C(O)NR_eR_f$, $-OR_d$, $-S(O)_2R_d$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";
$R_{110}$ is H, D, halogen, $-NR_dC(O)R_e$, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{19}$ is H or D;
$R_{19}$ and $R_{111}$ are independently H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated; alternatively, $R_{110}$ is H or D; or, $R_{18}$ and $R_{19}$ or $R_{18}$ and $R_{110}$ or $R_{110}$ and $R_{111}$ and the atom to which they are attached are taken together to form 3- to 7-membered monocyclic heterocyclyl, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5-membered or 6-membered heterocyclyl having 1 or 2 ring heteroatoms selected from N, O and S, wherein the group is optionally substituted with one or more R"; alternatively, $R_{18}$ and $R_{19}$ or $R_{18}$ and $R_{110}$ or $R_{110}$ and $R_{111}$ and the atom

to which they are attached are taken together to form 5-membered heterocyclyl having 1 or 2 ring heteroatoms N, wherein the group is optionally substituted with one or more R"; or

$R_{18}$ and $R_{19}$ or $R_{18}$ and $R_{110}$ or $R_{110}$ and $R_{111}$ and the atom to which they are attached are taken together to form 6- to 12-membered fused bicyclic heterocycle, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 8- to 10-membered fused bicyclic heterocycle having 1 to 3 ring heteroatoms selected from N, O and S, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 8- to 10-membered fused bicyclic heterocycle having 1 or 2 ring heteroatoms N, which is optionally substituted with one or more R"; or

$R_{18}$ and $R_{19}$ or $R_{18}$ and $R_{110}$ or $R_{110}$ and $R_{111}$ and the atom to which they are attached are taken together to form 5- to 10-membered heteroaryl, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5- or 6-membered heteroaryl having 1 to 3 ring heteroatoms selected from N, O and S, which is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5-membered heteroaryl having 1 to 3 ring heteroatoms selected from N, O and S, wherein the group is optionally substituted with one or more R"; alternatively, $R_{18}$ and $R_{19}$ or $R_{18}$ and $R_{110}$ or $R_{110}$ and $R_{111}$ and the atom to which they are attached are taken together to form 5-membered heteroaryl having 1 or 2 ring heteroatoms N, wherein the group is optionally substituted with one or more R"; wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**42.** The compound according to any one of claims 39 to 41, or a tautomer, a stereoisomer, a prodrug, a crystal form, a pharmaceutically acceptable salt, a hydrate or a solvate thereof, wherein,

$X_2$ is CH;
$R_5$ is F;
$R_6$ is methyl or $-CD_3$;
Rt is H, D or F;
$Y_3$ is N or $CR_{111}$;
$R_{18}$ and $R_{19}$ or $R_{18}$ and $R_{110}$ or $R_{110}$ and $R_{111}$ and the atom to which they are attached are taken together to form 5-membered heteroaryl having 1 or 2 ring heteroatoms N, wherein the group is optionally substituted with one or more R";
each R" is independently D, halogen, $C_{1-6}$ alkyl or $C_{1-6}$ haloalkyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl and 3- to 7-membered heterocyclyl are optionally substituted with one or more D, up to fully deuterated.

**43.** The compound according to claim 1 or 2, or a tautomer, a stereoisomer, a prodrug, a crystal form, a pharmaceutically acceptable salt, a hydrate or a solvate thereof, wherein the compound is a compound of formula (IX-1):

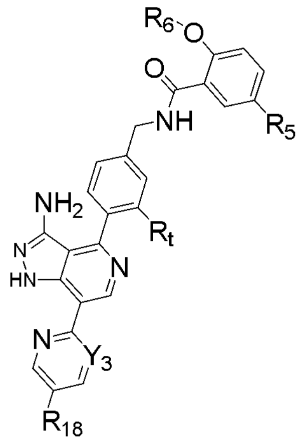

(IX-1)

wherein,

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

Rt is H, D or halogen; alternatively, $R_t$ is H, D or F;

$Y_3$ is N, CH or CD; alternatively, $Y_3$ is N or CH;

$R_{18}$ is H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3-to 7-membered heterocyclyl, wherein the $C_{1-6}$ alkyl, $C_{1-6}$ haloalkyl and 3- to 7-membered heterocyclyl are optionally substituted with one or more R"; alternatively, $R_{18}$ is halogen, $-C(O)NR_eR_f$, $-OR_d$, $-S(O)_2R_d$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";

wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**44.** The compound according to claim 43, or a tautomer, a stereoisomer, a prodrug, a crystal form, a pharmaceutically acceptable salt, a hydrate or a solvate thereof, wherein,

$R_5$ is F;

$R_6$ is methyl or $-CD_3$;

Rt is H, D or F;

$Y_3$ is N or CH;

$R_{18}$ is H, F, Cl, $-CH_3$, $-CHF_2$, $-CF_3$, $-OCF_3$,

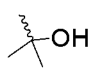, 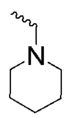, 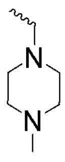, 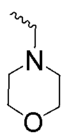, 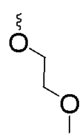, 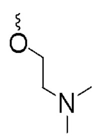, 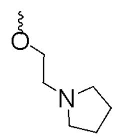,

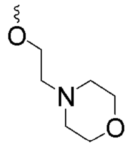, 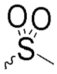, 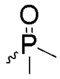, 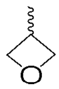, 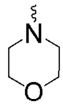, 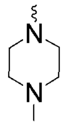, 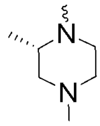, 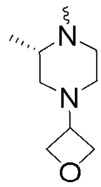, 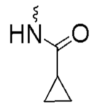, 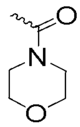, 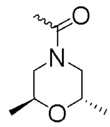

or

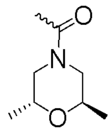,

wherein the groups are optionally substituted with one or more D, up to fully deuterated; alternatively, $R_{18}$ is F, Cl, $-CH_3$, $-CHF_2$, $-CF_3$, $-OCF_3$,

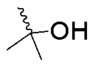, 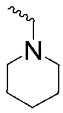, 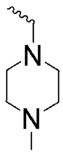, 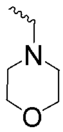, 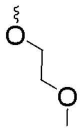, 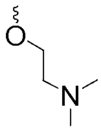,

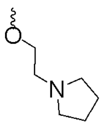, 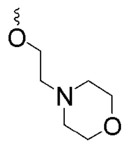, 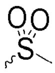, 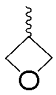, 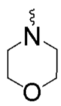, 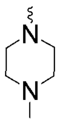, 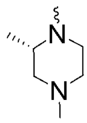, 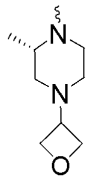, 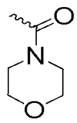, 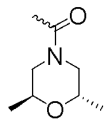 or 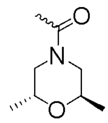,

wherein the groups are optionally substituted with one or more D, up to fully deuterated; alternatively, $R_{18}$ is $-CH_3$,

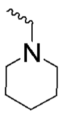, 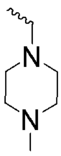, 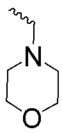, 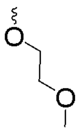, 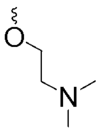, 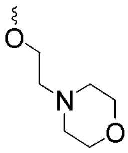, 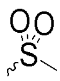, 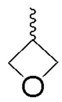, 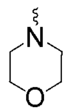,

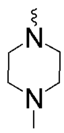, 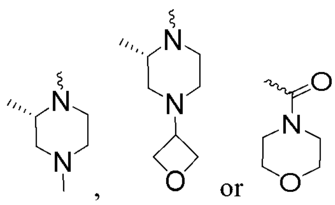, or ,

wherein the groups are optionally substituted with one or more D, up to fully deuterated.

**45.** The compound according to claim 1 or 2, or a tautomer, a stereoisomer, a prodrug, a crystal form, a pharmaceutically acceptable salt, a hydrate or a solvate thereof, wherein the compound is a compound of formula (X):

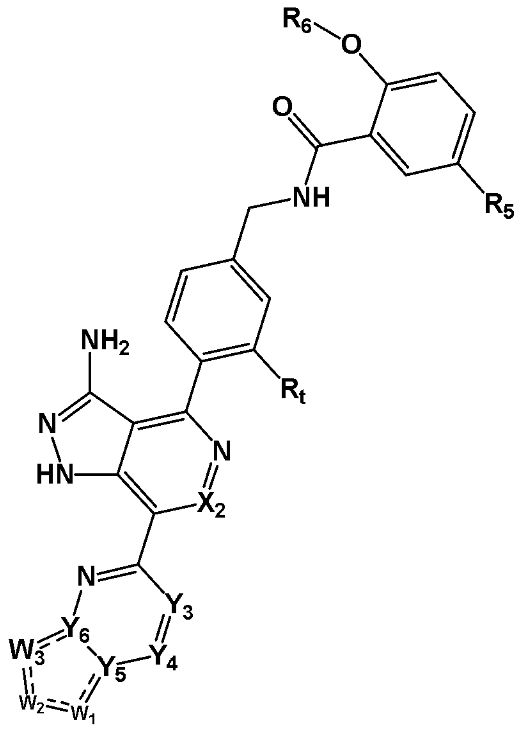

(X)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N, CH or CD;
$Y_4$ is N, CH or CD;
$Y_5$ and $Y_6$ are independently N or C, and at least one of $Y_5$ and $Y_6$ is N;
$W_1$ is N or $CR_{112}$;
$W_2$ is N or $CR_{113}$;
$W_3$ is N or $CR_{114}$;
--- indicates single or double bonds;
with the proviso that, the bicyclic ring containing $Y_3$, $Y_4$, $Y_5$, $Y_6$, $W_1$, $W_2$ and $W_3$ is an aromatic ring;
$R_{112}$, $R_{113}$ and $R_{114}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, - $NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, - $P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{112}$ and $R_{113}$ or $R_{113}$ and $R_{114}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl,

$C_{6-10}$ aryl and 5- to 10-membered heteroaryl are optionally substituted with one or more R";
each R" is independently H, D, halogen, oxo, -C(O)$R_g$, -C(O)O$R_g$, -C(O)N$R_hR_j$, -N$R_hR_j$, -N$R_g$C(O)$R_h$, - N$R_g$C(O)O$R_h$, -N$R_g$C(O)N$R_hR_j$, -O$R_g$, -OC(O)$R_g$, -OC(O)N$R_hR_j$, -N$R_g$S(O)$_2R_h$, -S(O)$_2$N$R_hR_j$, -S(O)$R_g$, - S(O)$_2R_g$, -P(=O)$R_hR_j$, -OP(=O)$R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

**46.** The compound according to claim 45, or a tautomer, a stereoisomer, a prodrug, a crystal form, a pharmaceutically acceptable salt, a hydrate or a solvate thereof, wherein,

$X_2$ is N or C$R_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N, CH or CD;
$Y_4$ is N, CH or CD;
$Y_5$ and $Y_6$ are independently N or C, and at least one of $Y_5$ and $Y_6$ is N;
$W_1$ is N or C$R_{112}$;
$W_2$ is N or C$R_{113}$;
$W_3$ is N or C$R_{114}$;
--- indicates single or double bonds;
with the proviso that, the bicyclic ring containing $Y_3$, $Y_4$, $Y_5$, $Y_6$, $W_1$, $W_2$ and $W_3$ is an aromatic ring;
$R_{112}$, $R_{113}$ and $R_{114}$ are independently H, D, halogen, -C(O)N$R_eR_f$, -N$R_d$C(O)$R_e$, -O$R_d$, -S(O)$_2R_d$, -P(=O)$R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";
or, $R_{112}$ and $R_{113}$ or $R_{113}$ and $R_{114}$ and the atom to which they are attached are taken together to form $C_{3-6}$ cycloalkyl, 3- to 7-membered monocyclic heterocyclyl, 6- to 12-membered fused bicyclic heterocycle, $C_{6-10}$ aryl or 5- to 10-membered heteroaryl, wherein the groups are optionally substituted with one or more R"; wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, -C(O)O$R_g$, -N$R_hR_j$, -N$R_g$C(O)$R_h$, -O$R_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**47.** The compound according to claim 45 or 46, or a tautomer, a stereoisomer, a prodrug, a crystal form, a pharmaceutically acceptable salt, a hydrate or a solvate thereof, wherein,

$X_2$ is N or C$R_2$;
$R_2$ is H, D or halogen; alternatively, $R_2$ is H, D or F; alternatively, $R_2$ is H;
$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

Rt is H, D or halogen; alternatively, $R_t$ is H, D or F;

$Y_3$ is N, CH or CD; alternatively, $Y_3$ is N or CH;

$Y_4$ is N, CH or CD; alternatively, $Y_4$ is N or CH;

$Y_5$ and $Y_6$ are independently N or C, and at least one of $Y_5$ and $Y_6$ is N;

$W_1$ is N or $CR_{112}$;

$W_2$ is N or $CR_{113}$;

$W_3$ is N or $CR_{114}$;

- - - indicates single or double bonds;

with the proviso that, the bicyclic ring containing $Y_3$, $Y_4$, $Y_5$, $Y_6$, $W_1$, $W_2$ and $W_3$ is an aromatic ring;

$R_{112}$, $R_{113}$ and $R_{114}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{112}$, $R_{113}$ and $R_{114}$ are independently H, D, $C_{1-6}$ alkyl or $C_{1-6}$ haloalkyl, wherein the $C_{1-6}$ alkyl and $C_{1-6}$ haloalkyl are optionally substituted with one or more R";

or, $R_{112}$ and $R_{113}$ or $R_{113}$ and $R_{114}$ and the atom to which they are attached are taken together to form 3- to 7-membered heterocyclyl, which is optionally substituted with one or more R"; alternatively, $R_{112}$ and $R_{113}$ or $R_{113}$ and $R_{114}$ and the atom to which they are attached are taken together to form 5-membered or 6-membered heterocyclyl having 1 or 2 ring heteroatoms independently selected from N, O and S, which is optionally substituted with one or more R";

wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";

each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;

wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**48.** The compound according to claim 1 or 2, or a tautomer, a stereoisomer, a prodrug, a crystal form, a pharmaceutically acceptable salt, a hydrate or a solvate thereof, wherein the compound is a compound of formula (X-1) or (X-2):

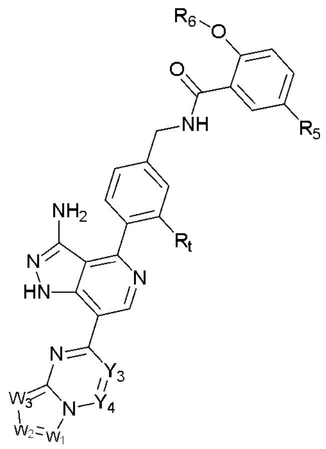

(X-1) or

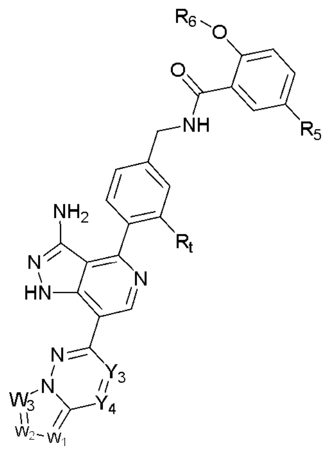

(X-2)

wherein,

$R_5$ is halogen; alternatively, $R_5$ is F;

$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;

Rt is H, D or halogen; alternatively, $R_t$ is H, D or F;

$Y_3$ is N, CH or CD; alternatively, $Y_3$ is N or CH;
$Y_4$ is N, CH or CD; alternatively, $Y_4$ is N or CH;
$W_1$ is N or $CR_{112}$;
$W_2$ is N or $CR_{113}$;
$W_3$ is N or $CR_{114}$;
$R_{112}$, $R_{113}$ and $R_{114}$ are independently H, D, halogen, -C(O)NR$_e$R$_f$, -NR$_d$C(O)R$_e$, -OR$_d$, -S(O)$_2$R$_d$, -P(=O)R$_e$R$_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{112}$, $R_{113}$ and $R_{114}$ are independently H, D, $C_{1-6}$ alkyl or $C_{1-6}$ haloalkyl, wherein the $C_{1-6}$ alkyl and $C_{1-6}$ haloalkyl are optionally substituted with one or more R";
or, $R_{112}$ and $R_{113}$ or $R_{113}$ and $R_{114}$ and the atom to which they are attached are taken together to form 3- to 7-membered heterocyclyl, which is optionally substituted with one or more R"; alternatively, $R_{112}$ and $R_{113}$ or $R_{113}$ and $R_{114}$ and the atom to which they are attached are taken together to form 5-membered or 6-membered heterocyclyl having 1 or 2 ring heteroatoms independently selected from N, O and S, wherein the group is optionally substituted with one or more R";
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, -C(O)OR$_g$, -NR$_h$R$_j$, -NR$_g$C(O)R$_h$, -OR$_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**49.** The compound according to claim 48, or a tautomer, a stereoisomer, a prodrug, a crystal form, a pharmaceutically acceptable salt, a hydrate or a solvate thereof, wherein,

$R_5$ is F;
$R_6$ is methyl or -CD$_3$;
Rt is H, D or F;
$Y_3$ is N or CH;
$Y_4$ is N or CH;
$W_1$ is N or $CR_{112}$;
$W_2$ is N or $CR_{113}$;
$W_3$ is N or $CR_{114}$;
$R_{112}$, $R_{113}$ and $R_{114}$ are H;
or, $R_{112}$ and $R_{113}$ or $R_{113}$ and $R_{114}$ and the atom to which they are attached are taken together to form the following groups:

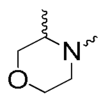
or
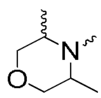
,

wherein the groups are optionally substituted with one or more D, up to fully deuterated.

**50.** The compound according to claim 1 or 2, or a tautomer, a stereoisomer, a prodrug, a crystal form, a pharmaceutically acceptable salt, a hydrate or a solvate thereof, wherein the compound is a compound of formula (XI):

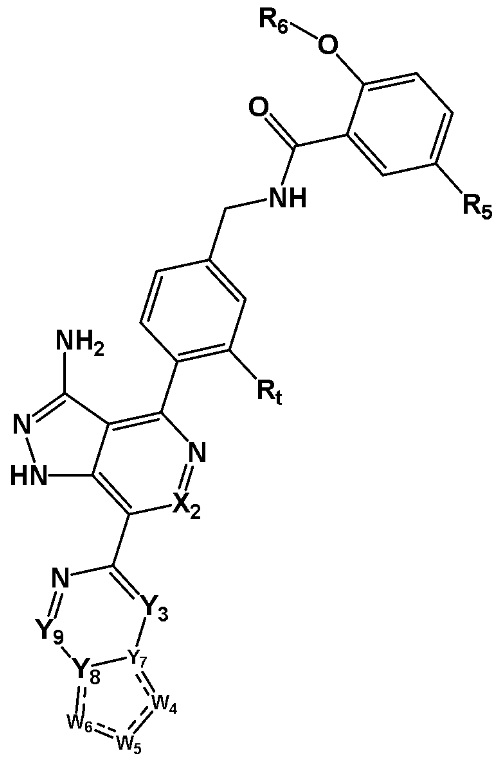

(XI)

wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N, CH or CD;
$Y_7$ and $Y_8$ are independently N or C, and at least one of $Y_7$ and $Y_8$ is N;
$Y_9$ is N, CH or CD;
$W_4$ is N or $CR_{115}$;
$W_5$ is N or $CR_{116}$;
$W_6$ is N or $CR_{117}$;
- - - indicates single or double bonds;
with the proviso that, the bicyclic ring containing $Y_3$, $Y_7$, $Y_8$, $Y_9$, $W_4$, $W_5$ and $W_6$ is an aromatic ring;
$R_{115}$, $R_{116}$ and $R_{117}$ are independently:

1) H, D, halogen, oxo, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_eR_f$, $-S(O)R_d$, $-S(O)_2R_d$, $-P(=O)R_eR_f$, $-OP(=O)R_eR_f$; or
2) $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, wherein the groups are optionally substituted with one or more R"; or
3) $R_{115}$ and $R_{116}$ or $R_{116}$ and $R_{117}$ and the atom to which they are attached are taken together to form $C_{3-6}$ cycloalkyl, 3- to 12-membered heterocyclyl, $C_{6-10}$ aryl or 5- to 10-membered heteroaryl, wherein the groups are optionally substituted with one or more R"; or

wherein each of $R_d$, $R_e$ and $R_f$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_e$, $R_f$ and the atom to which they are attached are taken together to form 3- to 7-membered heterocyclyl or 5- to 10-membered heteroaryl;

wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more R";
each R" is independently H, D, halogen, oxo, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_hR_j$, $-S(O)R_g$, $-S(O)_2R_g$, $-P(=O)R_hR_j$, $-OP(=O)R_hR_j$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or two R" on the same atom or on two adjacent atoms are taken together with the atom(s) to which they are attached to form $C_{3-6}$ cycloalkyl, 3- to 7-membered heterocyclyl, $C_{6-10}$ aryl or 5- to 10-membered heteroaryl; wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated;
wherein each of $R_g$, $R_h$ and $R_j$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_h$, $R_j$ and the atom to which they are attached are taken together to form 3- to 7-membered heterocyclyl or 5- to 10-membered heteroaryl;
wherein the $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl and 5- to 10-membered heteroaryl are optionally substituted with one or more D, up to fully deuterated.

**51.** The compound according to claim 50, or a tautomer, a stereoisomer, a prodrug, a crystal form, a pharmaceutically acceptable salt, a hydrate or a solvate thereof, wherein,

$X_2$ is N or $CR_2$;
$R_2$ is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$R_5$ is halogen;
$R_6$ is $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
Rt is H, D, halogen, $C_{1-6}$ alkyl or $C_{1-6}$ haloalkyl, wherein the $C_{1-6}$ alkyl and $C_{1-6}$ haloalkyl are optionally substituted with one or more D, up to fully deuterated;
$Y_3$ is N, CH or CD;
$Y_7$ and $Y_8$ are independently N or C, and at least one of $Y_7$ and $Y_8$ is N;
$Y_9$ is N, CH or CD;
$W_4$ is N or $CR_{115}$;
$W_5$ is N or $CR_{116}$;
$W_6$ is N or $CR_{117}$;
- - - indicates single or double bonds;
with the proviso that, the bicyclic ring containing $Y_3$, $Y_7$, $Y_8$, $Y_9$, $W_4$, $W_5$ and $W_6$ is an aromatic ring;
$R_{115}$, $R_{116}$ and $R_{117}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R";
or, $R_{115}$ and $R_{116}$ or $R_{116}$ and $R_{117}$ and the atom to which they are attached are taken together to form $C_{3-6}$ cycloalkyl, 3- to 7-membered monocyclic heterocyclyl, 6- to 12-membered fused bicyclic heterocycle, $C_{6-10}$ aryl or 5- to 10-membered heteroaryl, wherein the groups are optionally substituted with one or more R"; wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**52.** The compound according to claim 50 or 51, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;

$R_5$ is halogen; alternatively, $R_5$ is F;
$R_6$ is $C_{1-6}$ alkyl or $C_{1-6}$ haloalkyl, wherein the $C_{1-6}$ alkyl or $C_{1-6}$ haloalkyl is optionally substituted with one or more D, up to fully deuterated; alternatively, $R_6$ is methyl or $-CD_3$;
Rt is H, D or halogen; alternatively, $R_t$ is H, D or F;
$Y_3$ is N, CH or CD; alternatively, $Y_3$ is N or CH;
$Y_7$ and $Y_8$ are independently N or C, and at least one of $Y_7$ and $Y_8$ is N;
$Y_9$ is N, CH or CD; alternatively, $Y_4$ is N or CH;
$W_4$ is N or $CR_{115}$;
$W_5$ is N or $CR_{116}$;
$W_6$ is N or $CR_{117}$;
- - - indicates single or double bonds;
with the proviso that, the bicyclic ring containing $Y_3$, $Y_7$, $Y_8$, $Y_9$, $W_4$, $W_5$ and $W_6$ is an aromatic ring;
$R_{115}$, $R_{116}$ and $R_{117}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{115}$, $R_{116}$ and $R_{117}$ are independently H, D, $C_{1-6}$ alkyl or $C_{1-6}$ haloalkyl, wherein the $C_{1-6}$ alkyl and $C_{1-6}$ haloalkyl are optionally substituted with one or more R";
or, $R_{115}$ and $R_{116}$ or $R_{116}$ and $R_{117}$ and the atom to which they are attached are taken together to form 3- to 7-membered heterocyclyl, which is optionally substituted with one or more R"; alternatively, $R_{115}$ and $R_{116}$ or $R_{116}$ and $R_{117}$ and the atom to which they are attached are taken together to form 5-membered or 6-membered heterocyclyl, wherein the group is optionally substituted with one or more R";
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**53.** The compound according to claim 1 or 2, or a tautomer, a stereoisomer, a prodrug, a crystal form, a pharmaceutically acceptable salt, a hydrate or a solvate thereof, wherein the compound is a compound of formula (XI-1) or (XI-2): wherein,

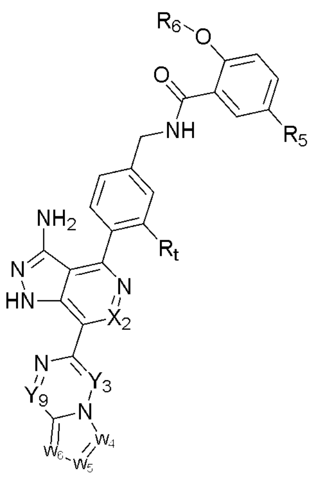

(XI-1) or

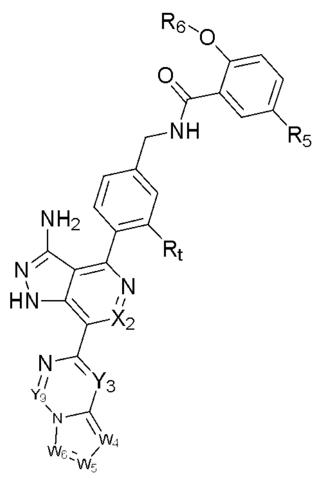

(XI-2)

$X_2$ is N or $CR_2$;
$R_2$ is H, D or halogen; alternatively, $R_2$ is H, D or F; alternatively, $R_2$ is H;
$R_5$ is halogen; alternatively, $R_5$ is F;
$R_6$ is $C_{1-6}$ alkyl or $C_{1-6}$ haloalkyl, wherein the $C_{1-6}$ alkyl and $C_{1-6}$ haloalkyl are optionally substituted with one or

more D, up to fully deuterated; alternatively, $R_6$ is methyl or $-CD_3$;
Rt is H, D or halogen; alternatively, $R_t$ is H, D or F;
$Y_3$ is N, CH or CD; alternatively, $Y_3$ is N or CH;
$Y_9$ is N, CH or CD; alternatively, $Y_9$ is N or CH;
$W_4$ is N or $CR_{115}$;
$W_5$ is N or $CR_{116}$;
$W_6$ is N or $CR_{117}$;
$R_{115}$, $R_{116}$ and $R_{117}$ are independently H, D, halogen, $-C(O)NR_eR_f$, $-NR_dC(O)R_e$, $-OR_d$, $-S(O)_2R_d$, $-P(=O)R_eR_f$, $C_{1-6}$ alkyl, $C_{1-6}$ haloalkyl or 3- to 7-membered heterocyclyl, wherein the $C_{1-6}$ alkyl, $C_{1-6}$ haloalkyl and 3- to 7-membered heterocyclyl are optionally substituted with one or more R"; alternatively, $R_{115}$, $R_{116}$ and $R_{117}$ are independently H, D, $C_{1-6}$ alkyl or $C_{1-6}$ haloalkyl, wherein the $C_{1-6}$ alkyl and $C_{1-6}$ haloalkyl are optionally substituted with one or more R";
or, $R_{115}$ and $R_{116}$ or $R_{116}$ and $R_{117}$ and the atom to which they are attached are taken together to form 3- to 7-membered heterocyclyl, which is optionally substituted with one or more R"; alternatively, $R_{115}$ and $R_{116}$ or $R_{116}$ and $R_{117}$ and the atom to which they are attached are taken together to form 5-membered or 6-membered heterocyclyl having 1 or 2 ring heteroatoms independently selected from N, O and S, wherein the group is optionally substituted with one or more R";
wherein each of $R_d$, $R_e$ and $R_f$ is independently H, $C_{1-6}$ alkyl, $C_{1-6}$ haloalkyl or $C_{3-6}$ cycloalkyl, or $R_e$, $R_f$ and the atom to which they are attached are taken together to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more R";
each R" is independently D, halogen, $-C(O)OR_g$, $-NR_hR_j$, $-NR_gC(O)R_h$, $-OR_g$, $C_{1-6}$ alkyl, $C_{1-6}$ haloalkyl, $C_{3-6}$ cycloalkyl or 3- to 7-membered heterocyclyl, or two R" on the same atom or on two adjacent atoms are taken together with the atom(s) to which they are attached to form 3- to 7-membered heterocyclyl; wherein the $C_{1-6}$ alkyl, $C_{1-6}$ haloalkyl, $C_{3-6}$ cycloalkyl and 3- to 7-membered heterocyclyl are optionally substituted with one or more D, up to fully deuterated;
wherein each of $R_g$, $R_h$ and $R_j$ is independently H or $C_{1-6}$ alkyl, or $R_h$, $R_j$ and the atom to which they are attached are taken together to form 3- to 7-membered heterocyclyl; wherein the $C_{1-6}$ alkyl and 3- to 7-membered heterocyclyl are optionally substituted with one or more D, up to fully deuterated.

**54.** The compound according to claim 53, or a tautomer, a stereoisomer, a prodrug, a crystal form, a pharmaceutically acceptable salt, a hydrate or a solvate thereof, wherein,

$X_2$ is N or $CR_2$;
$R_2$ is H, D or halogen; alternatively, $R_2$ is H, D or F; alternatively, $R_2$ is H;
$R_5$ is F;
$R_6$ is methyl or $-CD_3$;
Rt is H, D or F;
$Y_3$ is N or CH;
$Y_9$ is N or CH;
$W_4$ is N or $CR_{115}$;
$W_5$ is N or $CR_{116}$;
$W_6$ is N or $CR_{117}$;
$R_{115}$, $R_{116}$ and $R_{117}$ are H;
or, $R_{115}$ and $R_{116}$ or $R_{116}$ and $R_{117}$ and the atom to which they are attached are taken together to form the following groups:

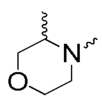 or 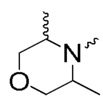 ,

wherein the groups are optionally substituted with one or more D, up to fully deuterated.

**55.** A compound, or a tautomer, a stereoisomer, a prodrug, a crystal form, a pharmaceutically acceptable salt, a hydrate or a solvate thereof, wherein the compound is selected from:

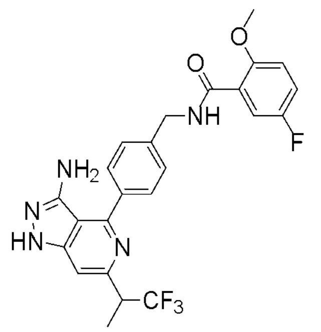

,

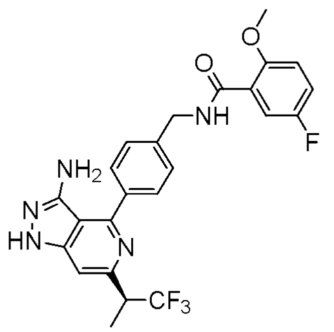

,

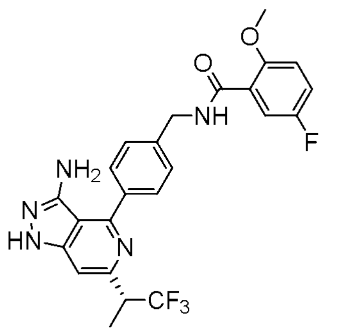

,

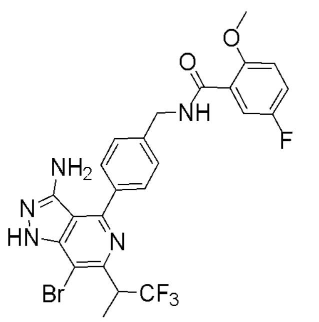

,

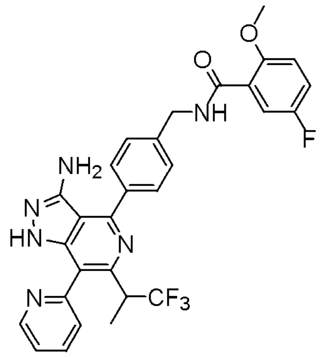

,

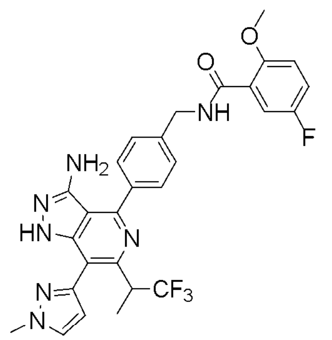

,

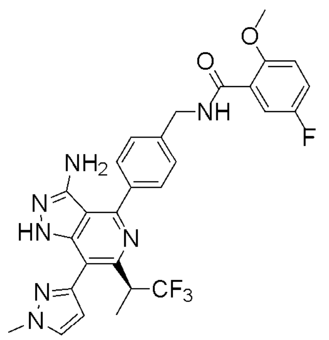

,

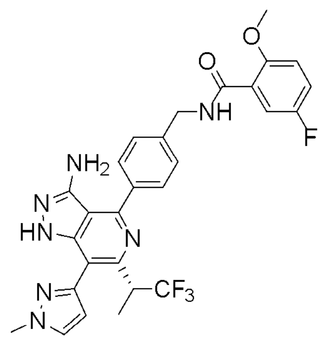

,

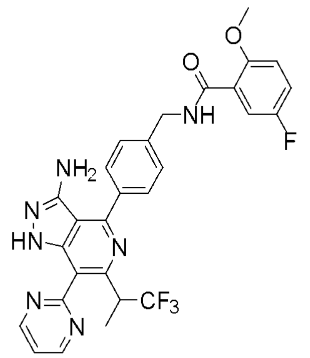

,

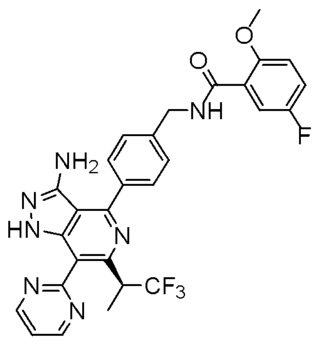

,

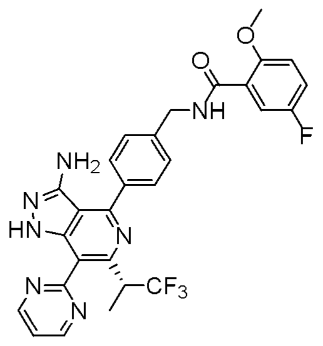

,

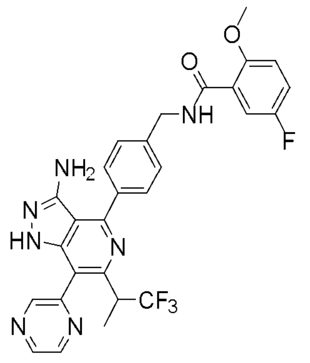

,

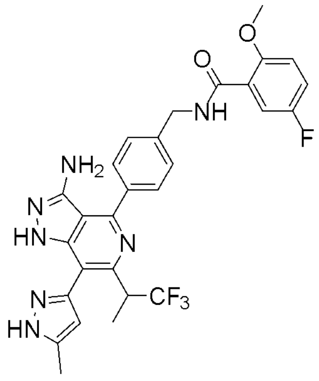
,
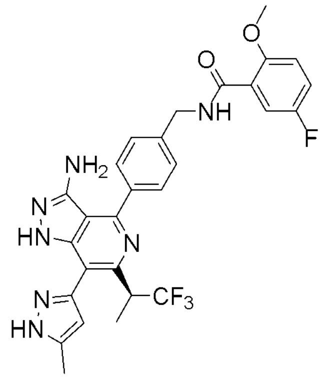
,
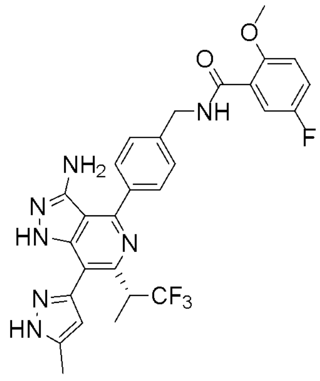
,

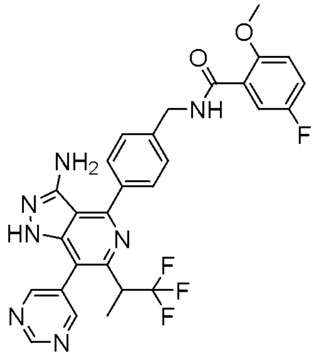
,
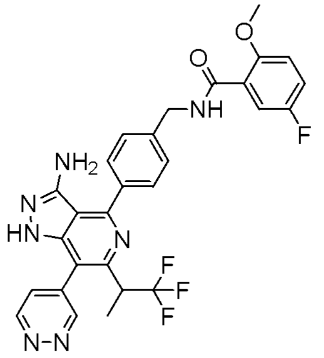
,
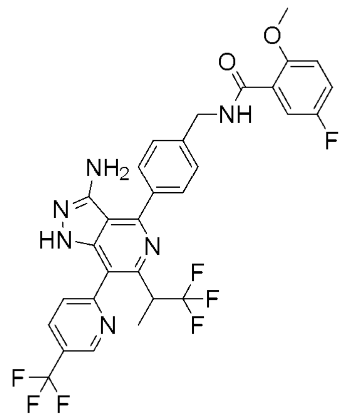
,

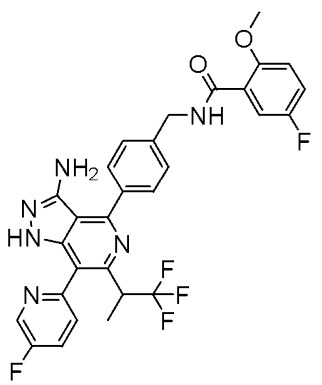
,
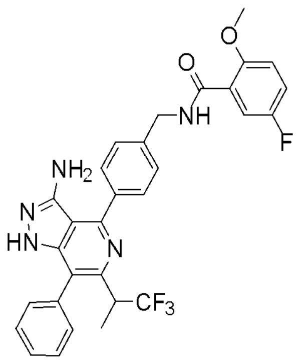
,
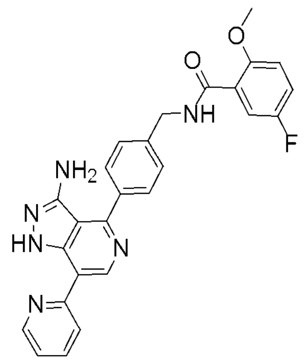
,

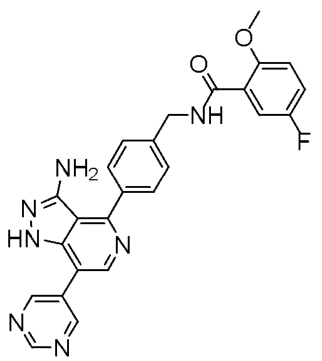
,
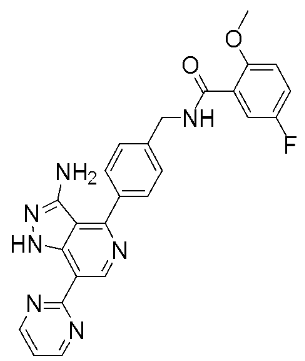
,
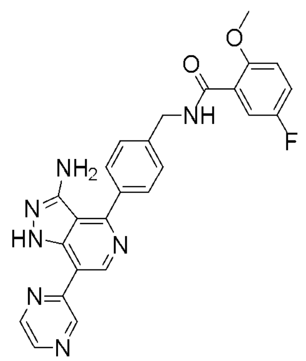
,

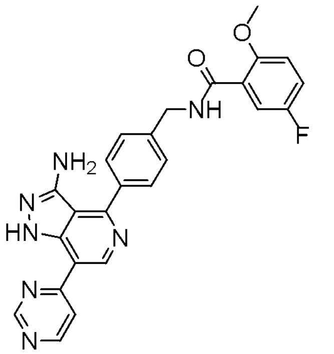
,
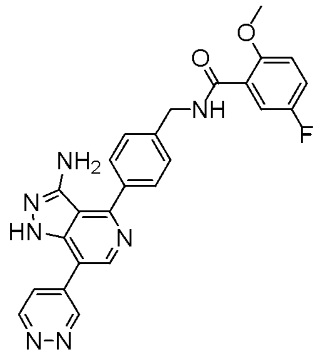
,
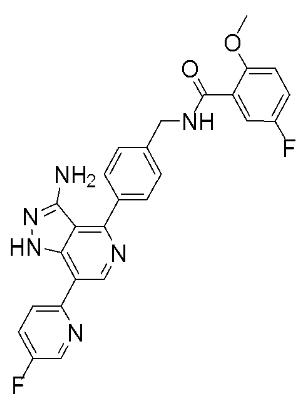
,

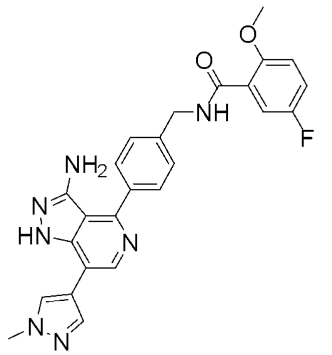
,
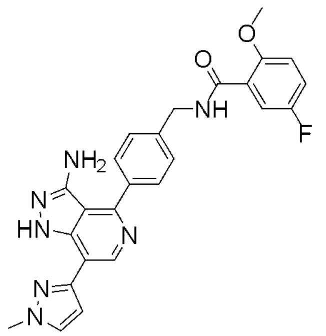
,
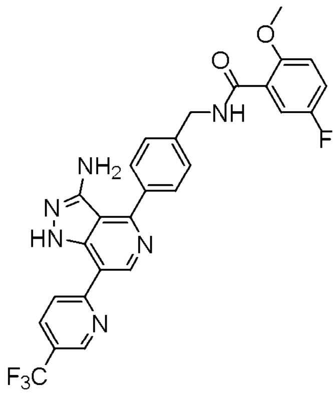
,

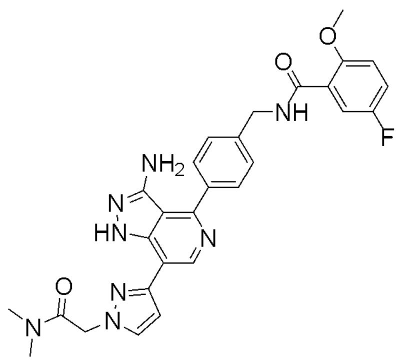
,
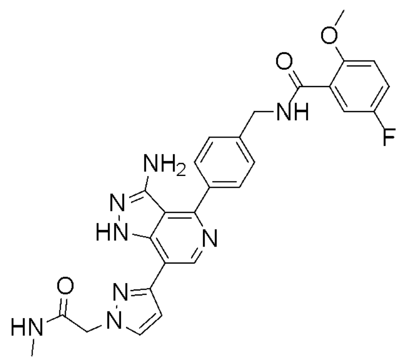
,

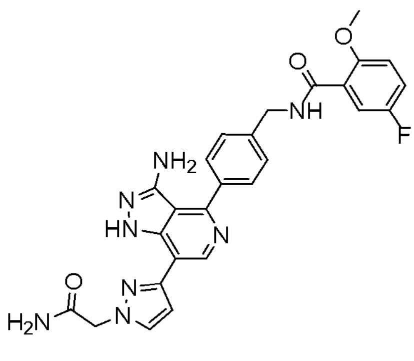
,
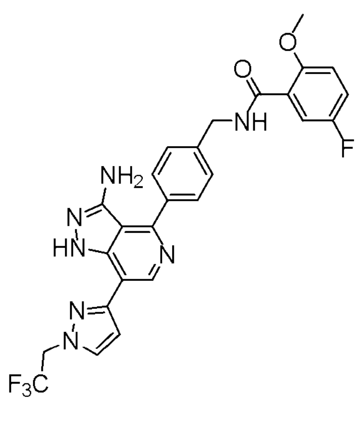
,
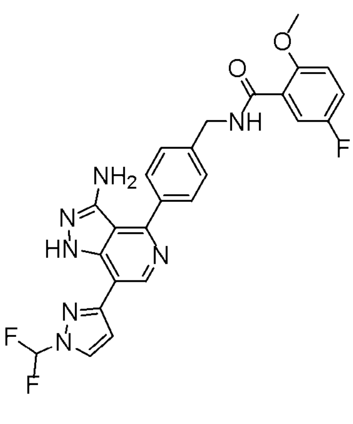
,

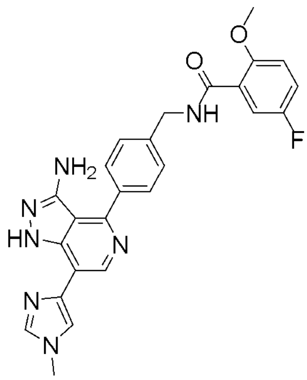 , 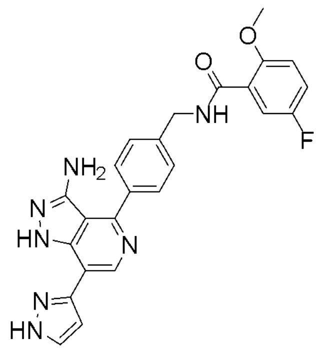 , 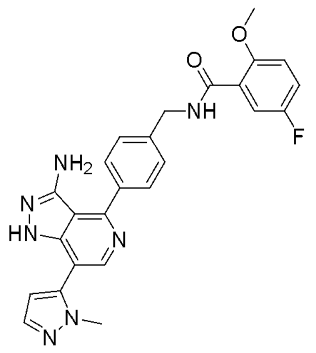 ,

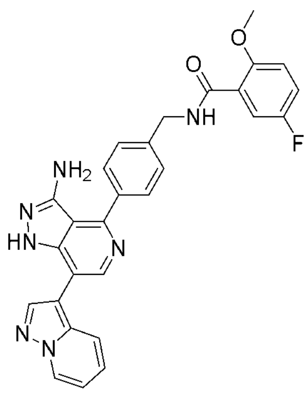 , 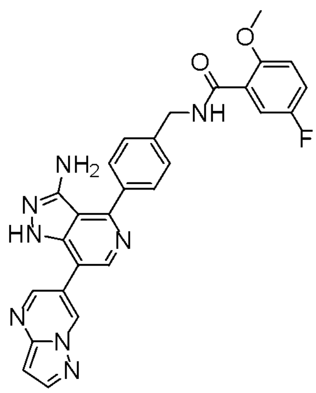 , 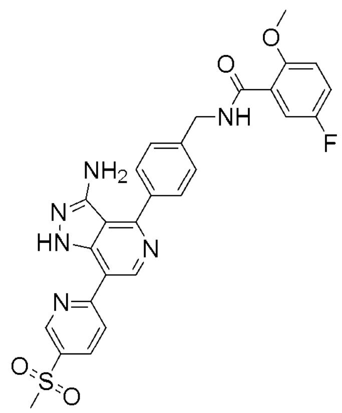 ,

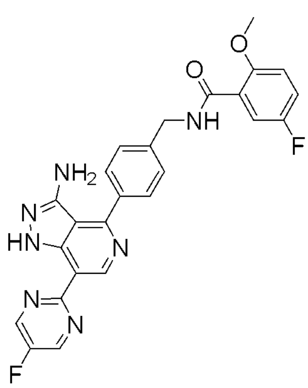 , 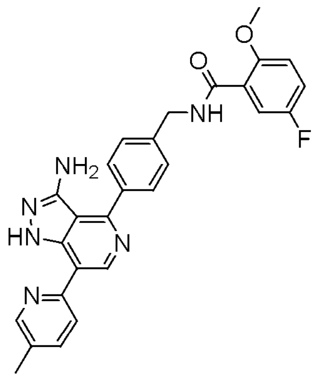 , 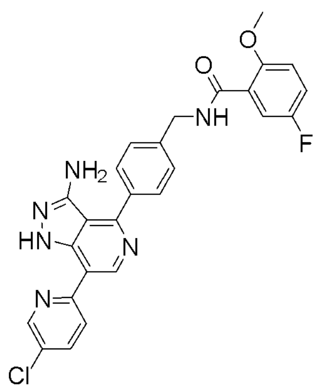 ,

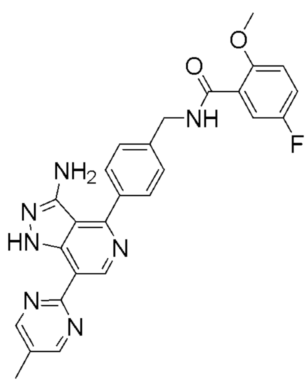 , 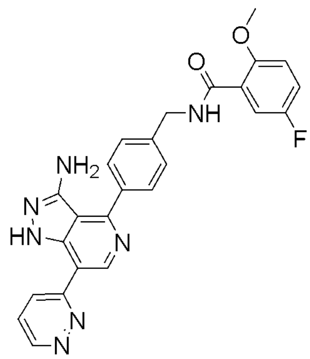 , 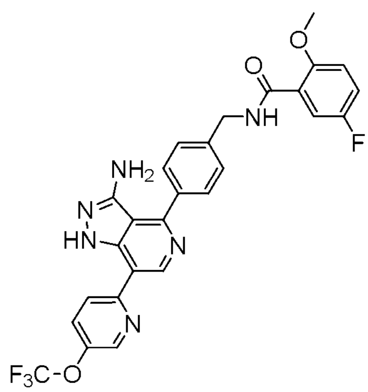 ,

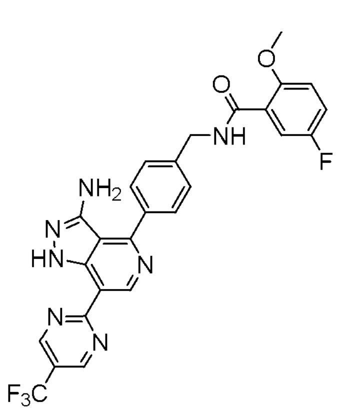
,
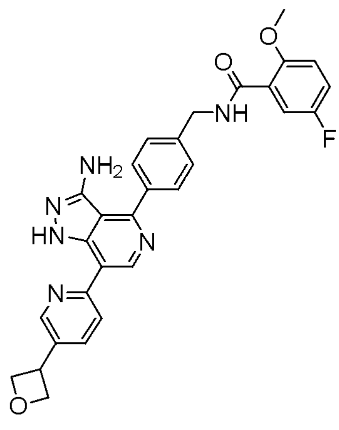
,
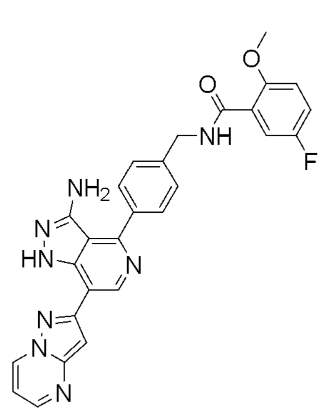
,

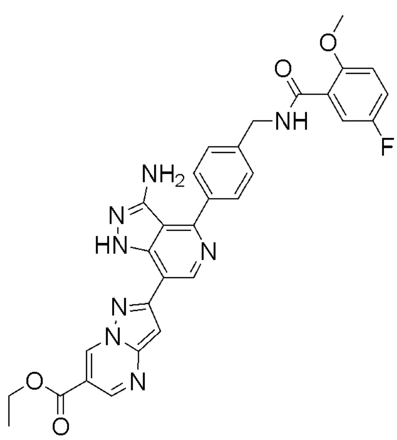
,
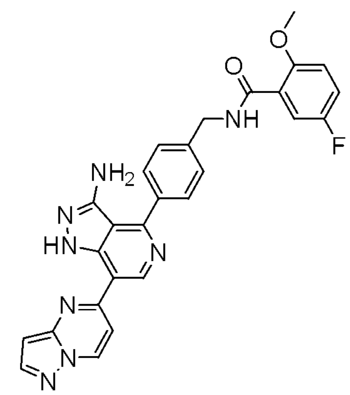
,
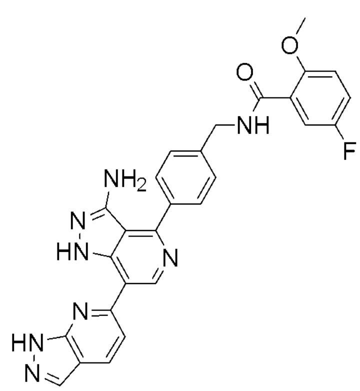
,

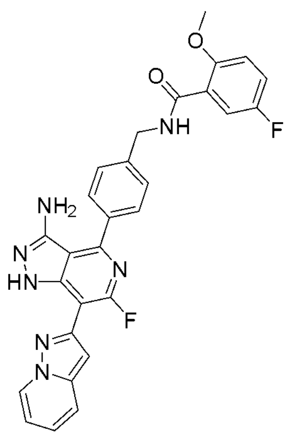
,
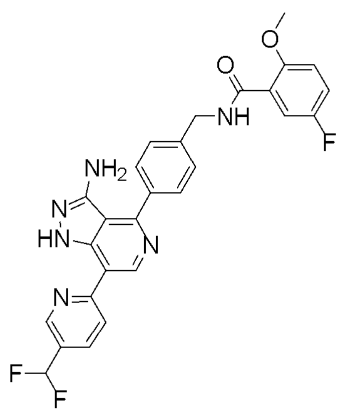
,
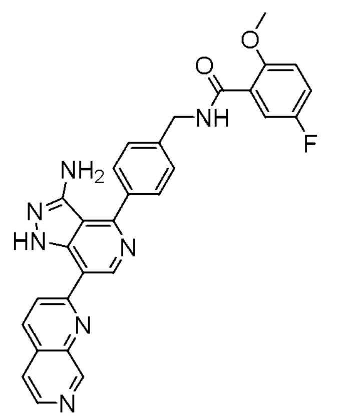
,

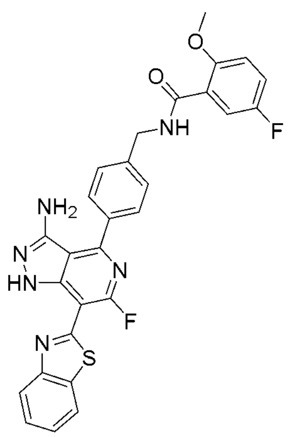
,
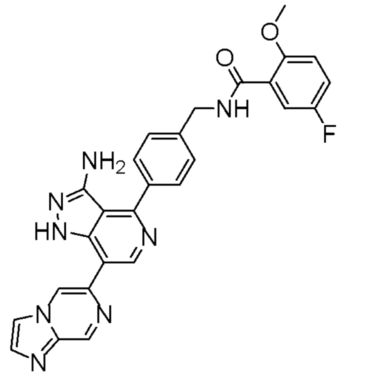
,
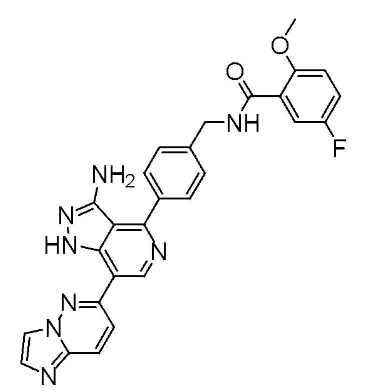
,

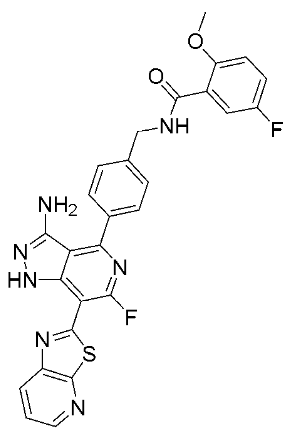
,
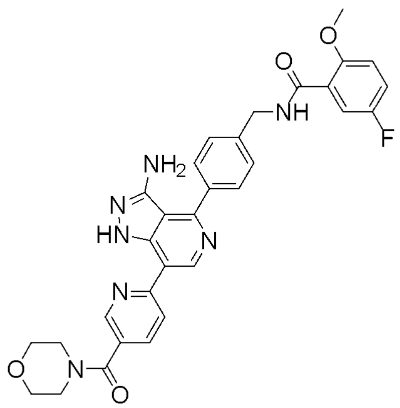
,
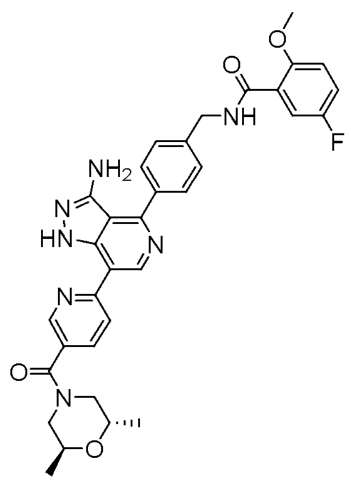
,

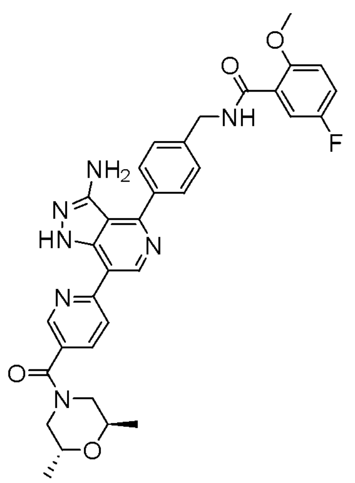
,
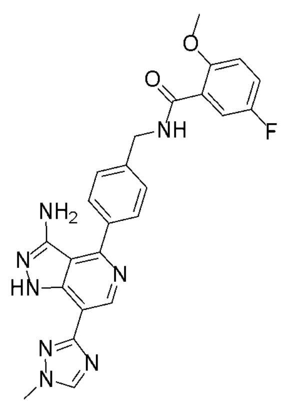
,
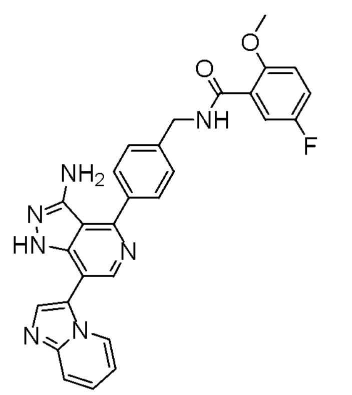
,

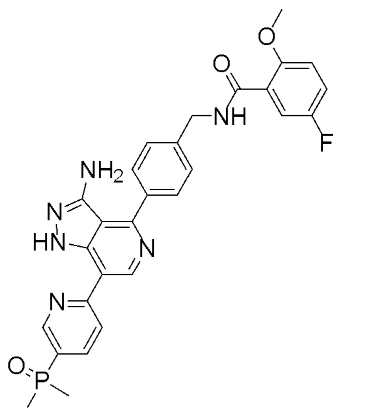 , 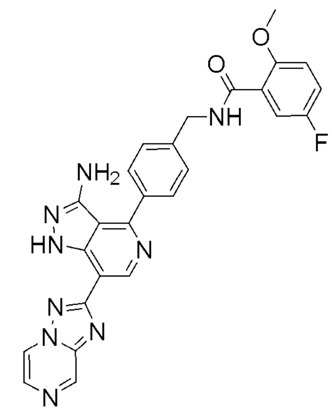 , 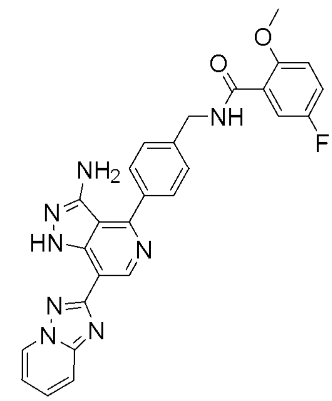 ,

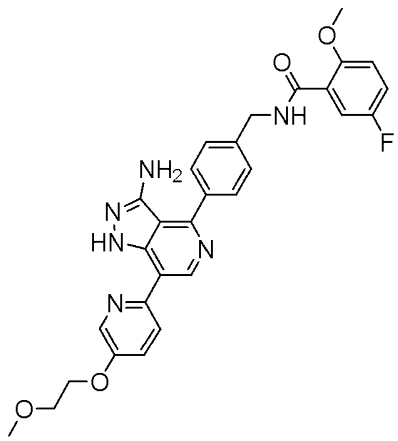 , 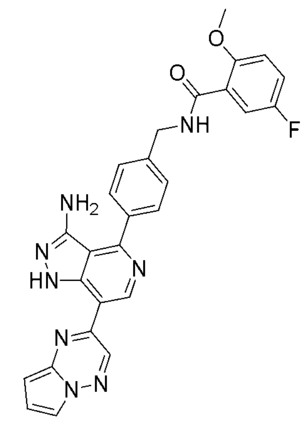 , 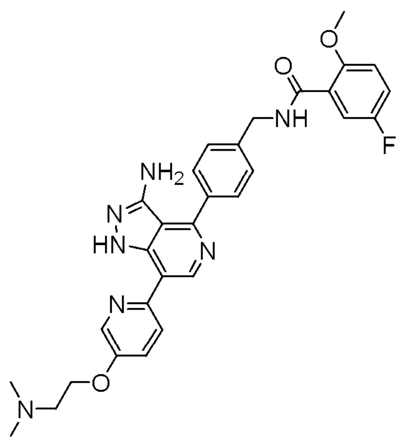 ,

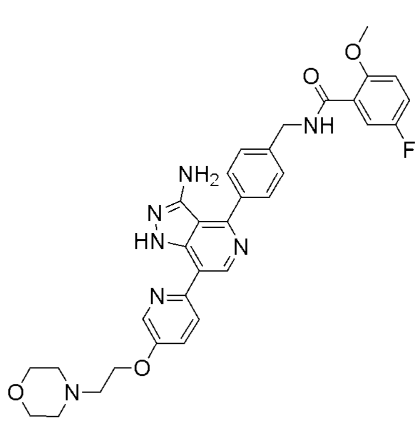 , 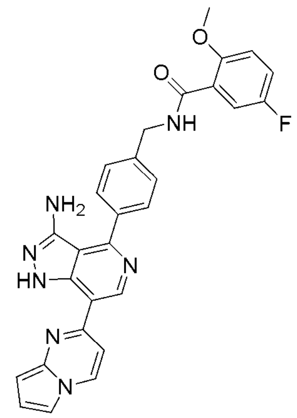 , 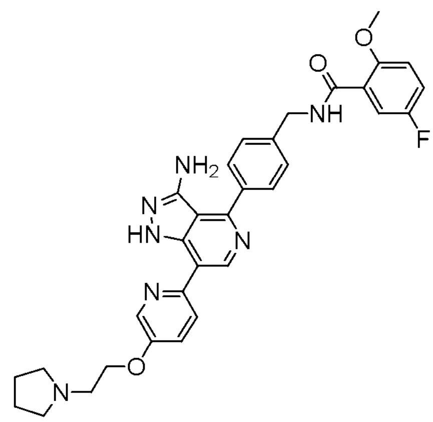 ,

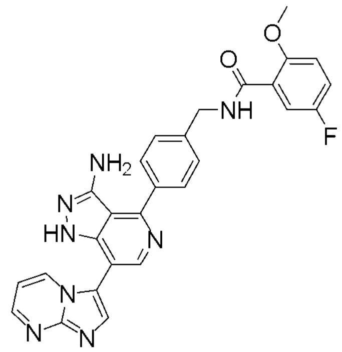
,
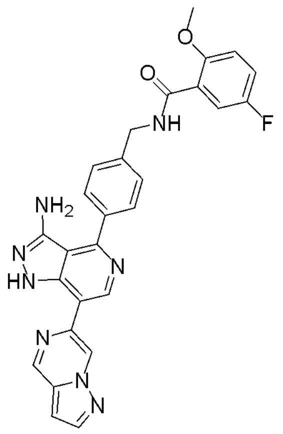
,
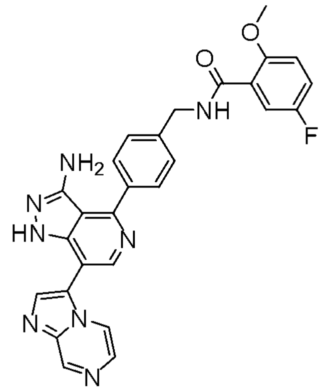
,
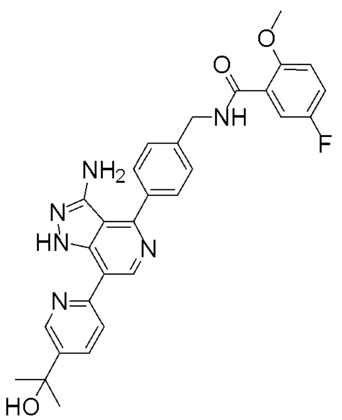
,
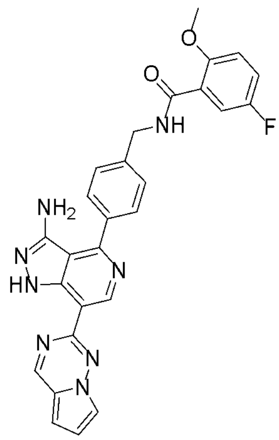
,
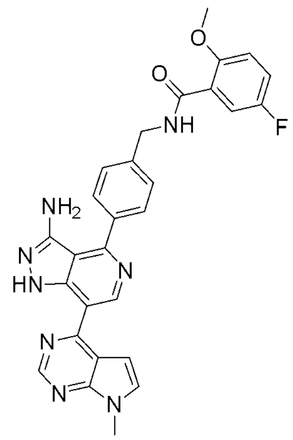
,
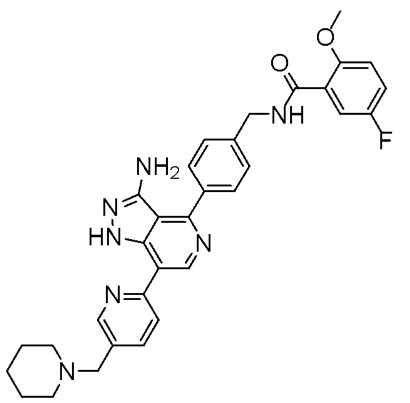
,
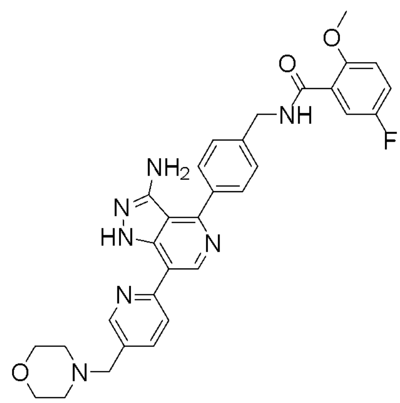
,

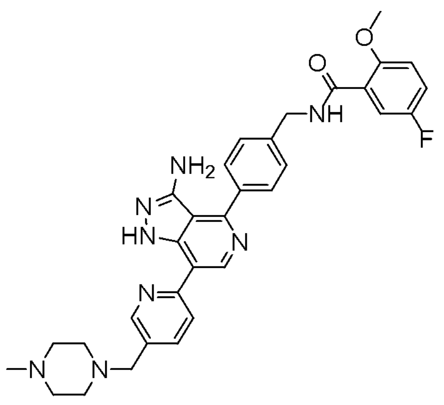 , 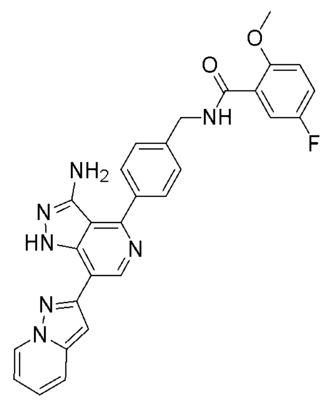 , 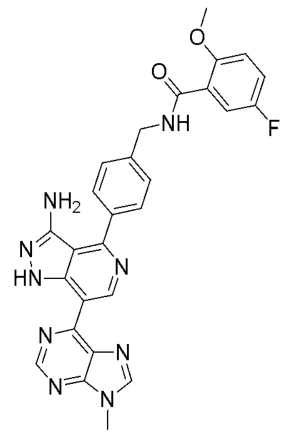 ,

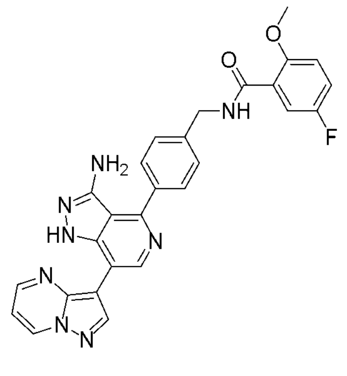 , 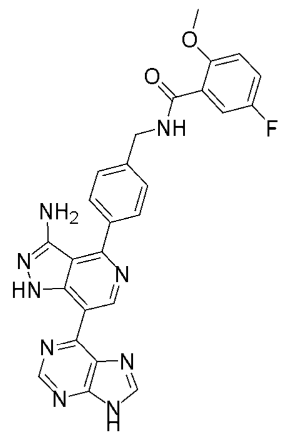 , 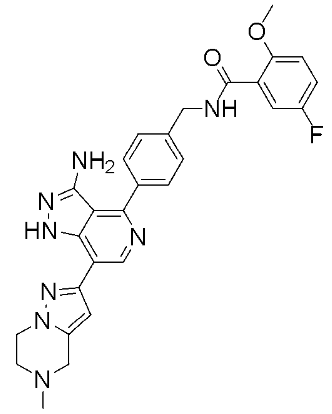 ,

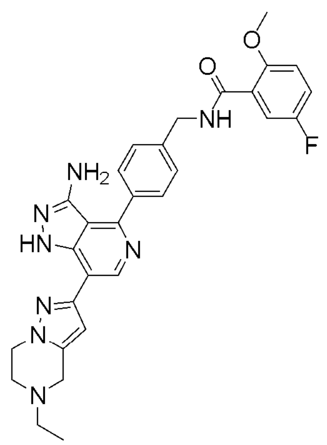 , 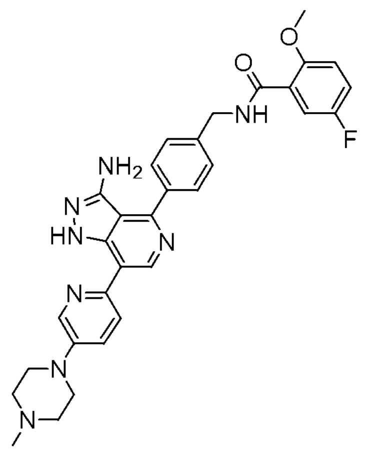 , 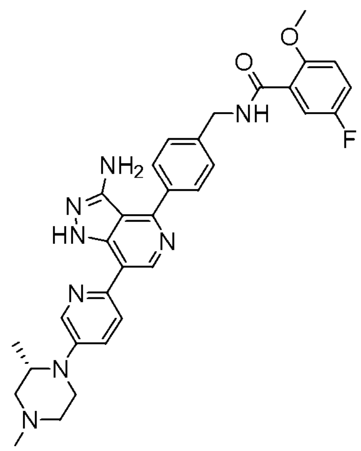 ,

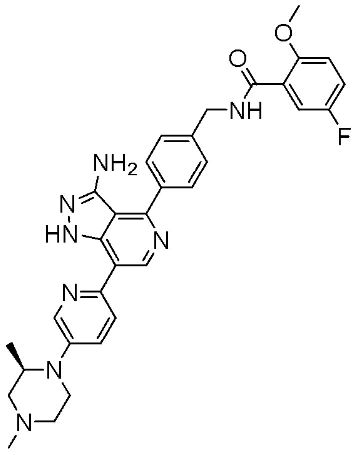
,
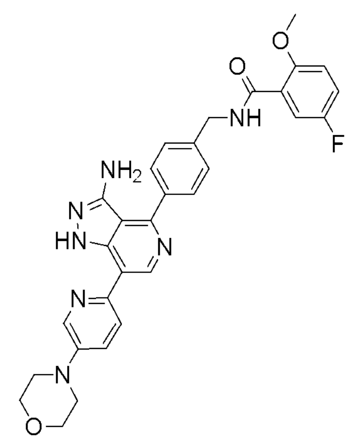
,
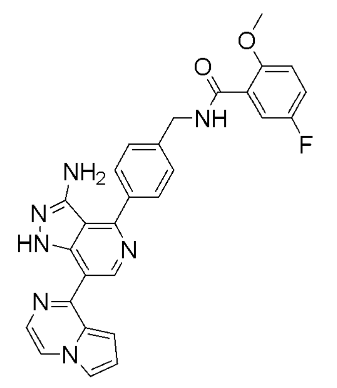
,
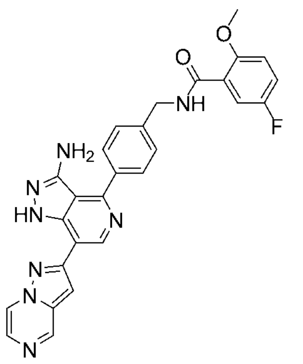
,
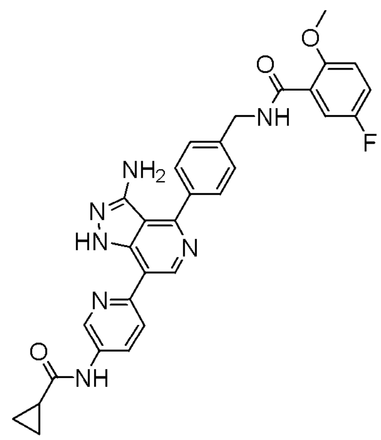
,
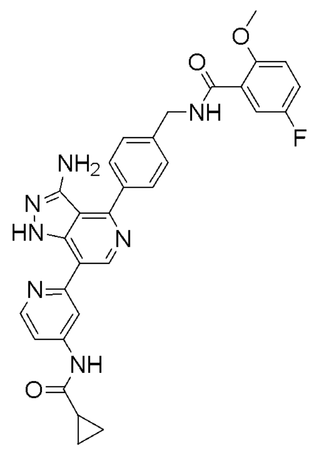
,
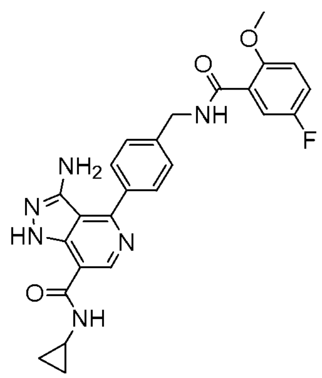
,
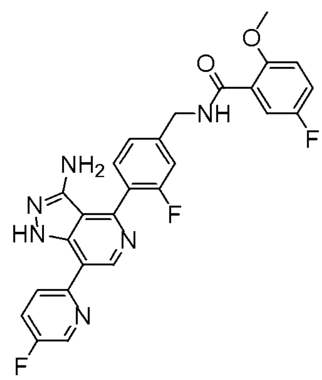
,
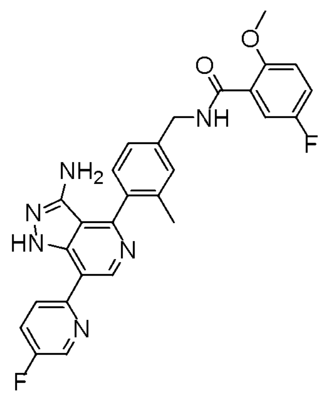
,
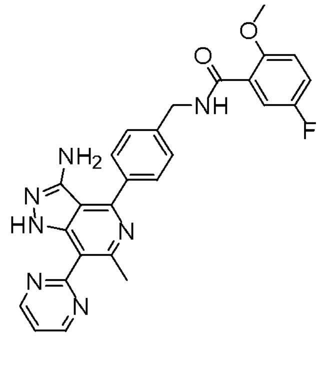
,
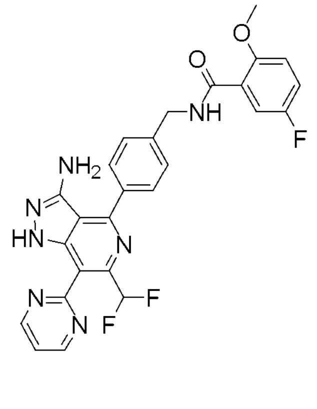
,
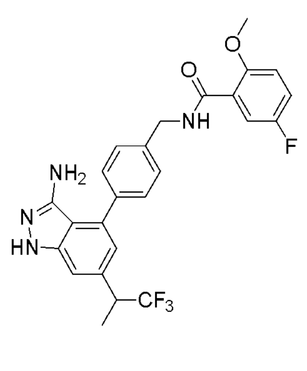
,

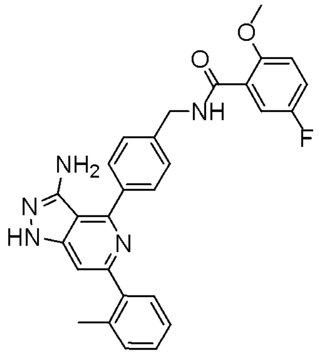
,
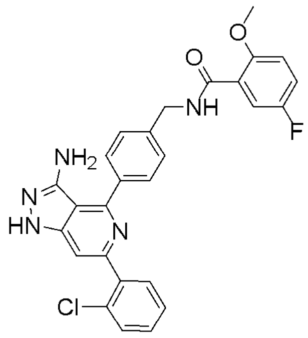
,
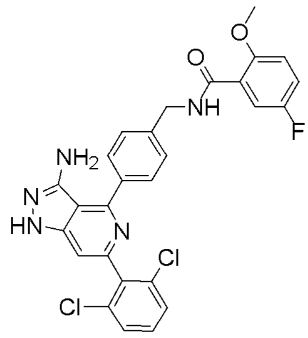
,

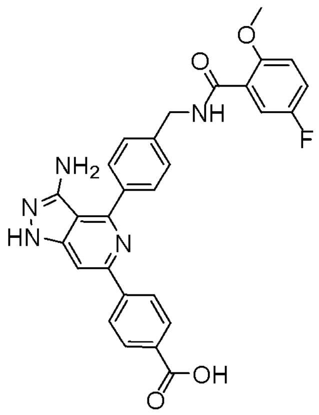
,
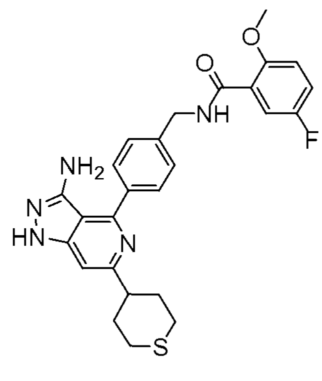
,
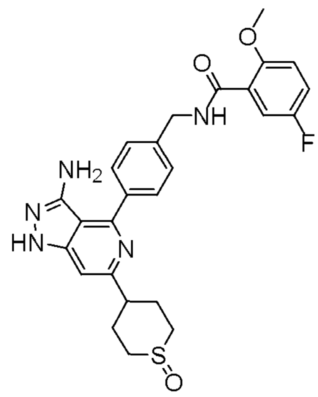
,

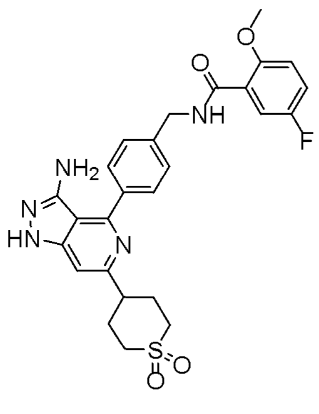
,
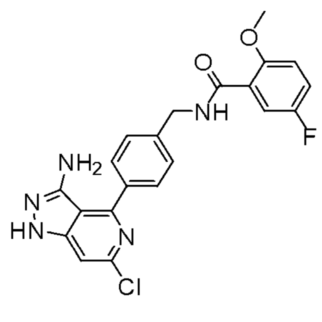
,
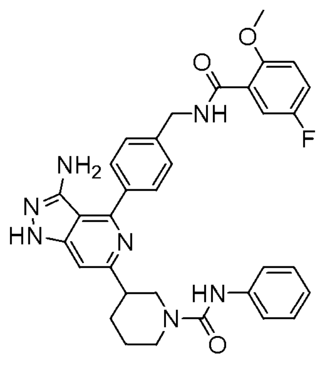
,

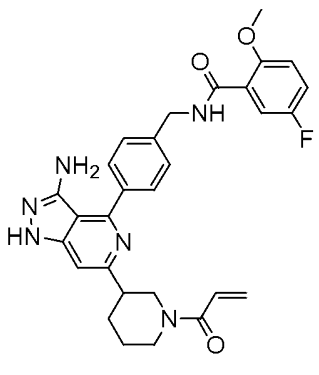
,
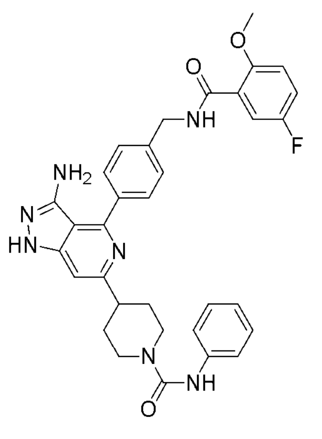
,
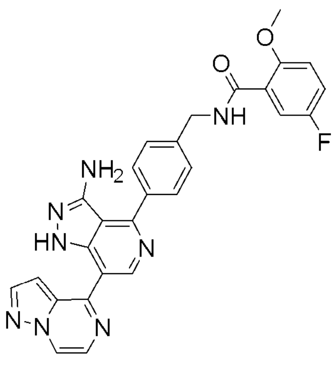
,

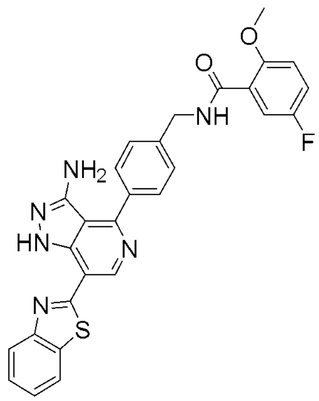
,
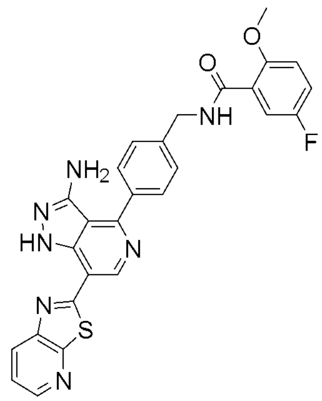
,
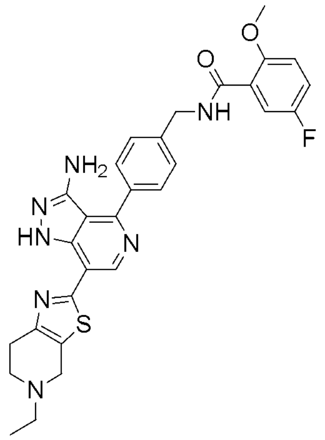
,

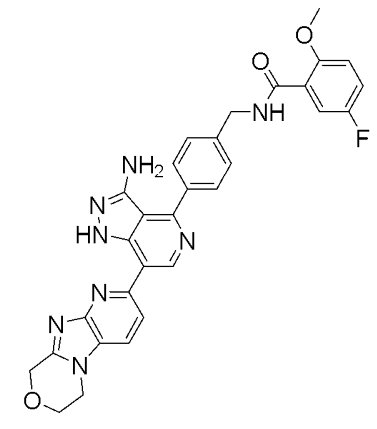
,
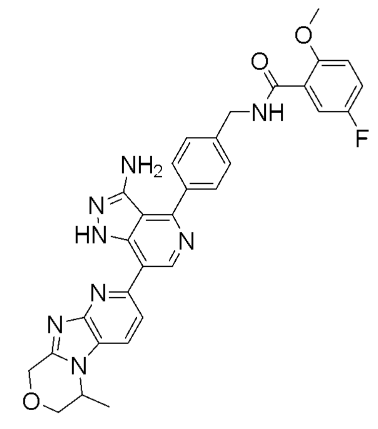
,
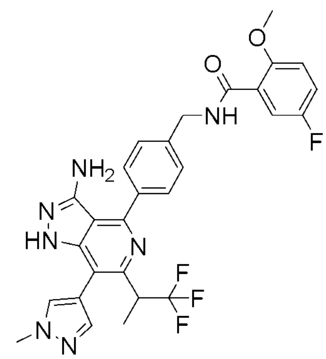
,

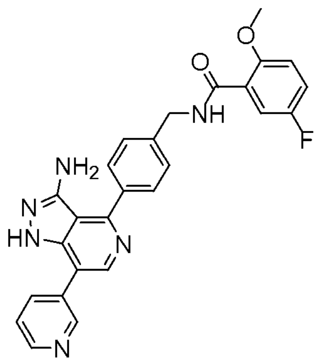
,
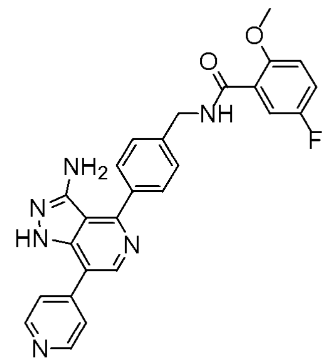
,
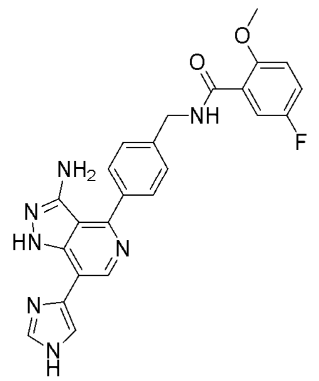
,

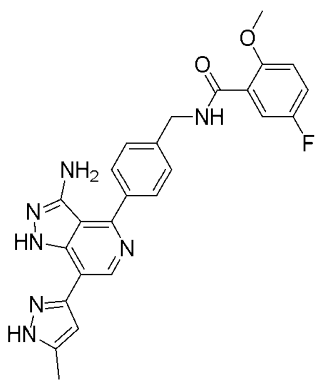
,
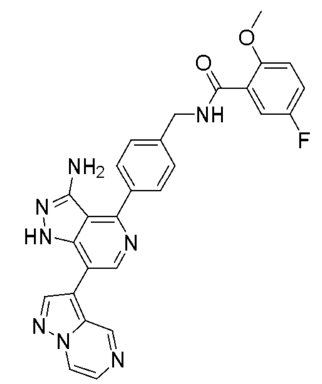
,
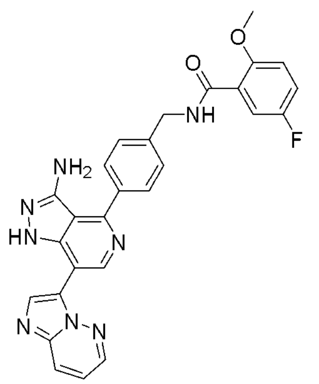
,

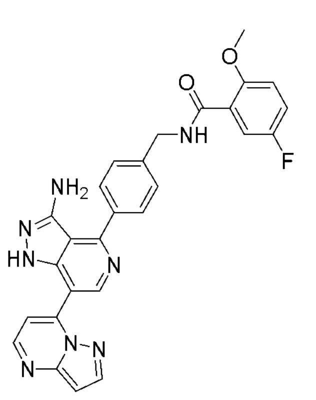
,
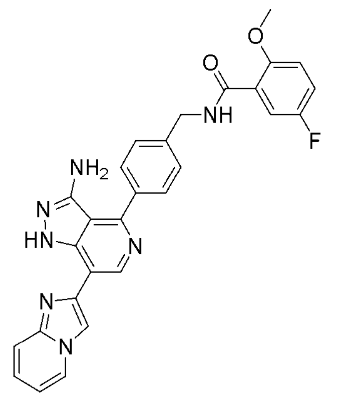
,
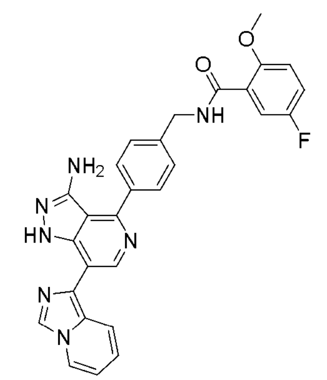
,
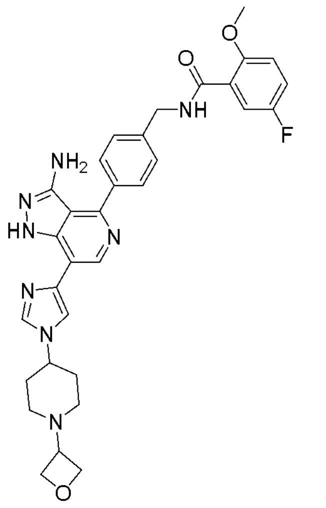
,
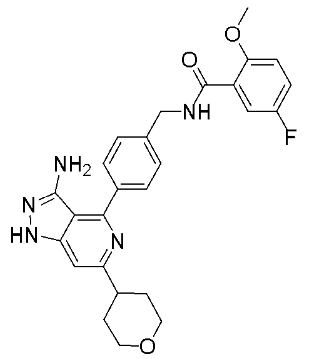
,
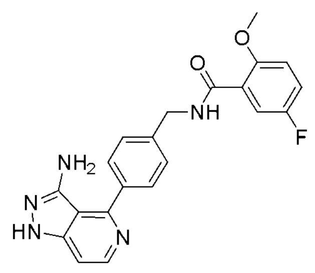
,
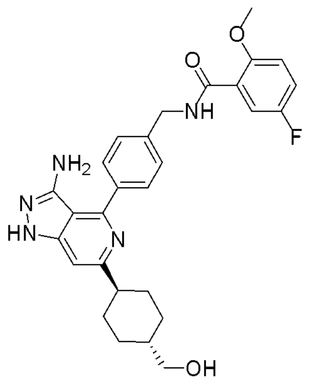
,
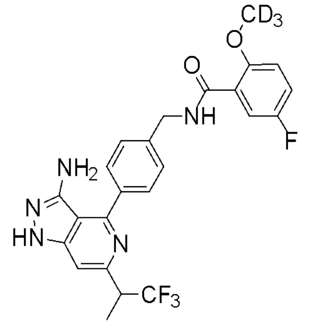
,
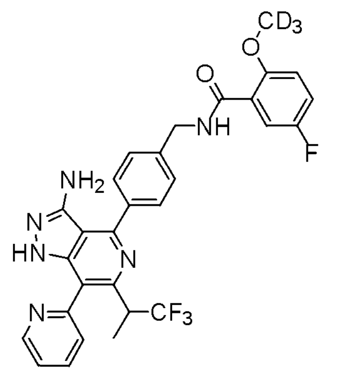
,
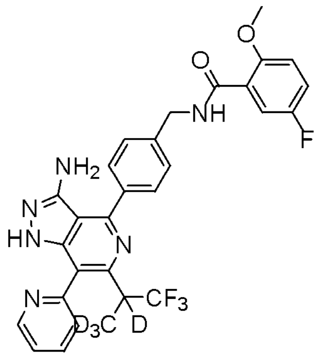
,
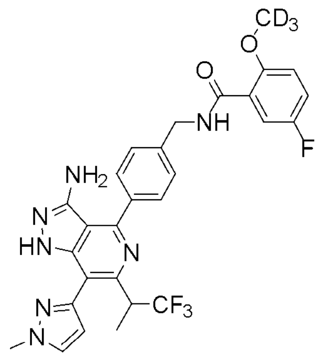
,
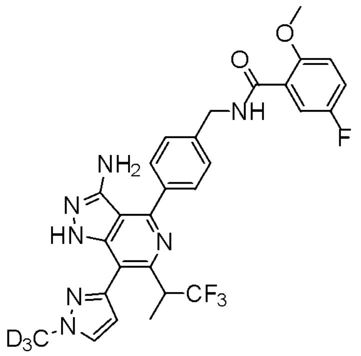
,

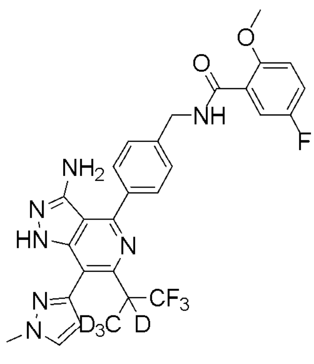,
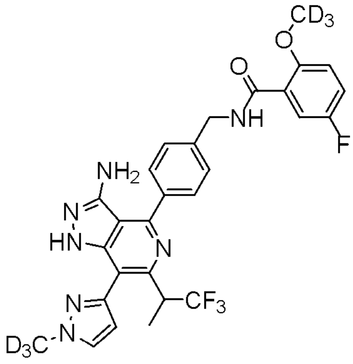,
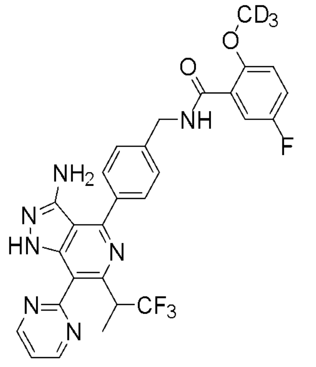,

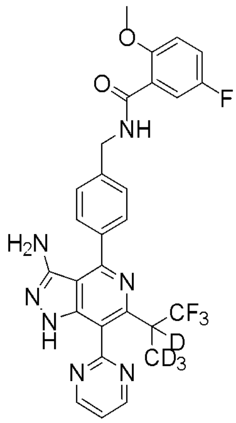,
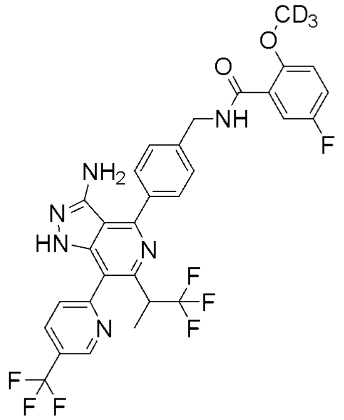,
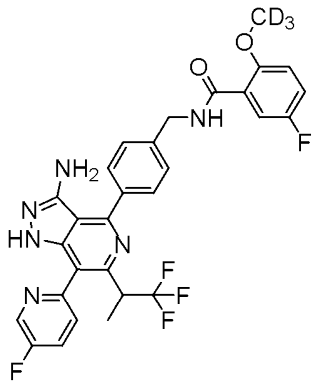,

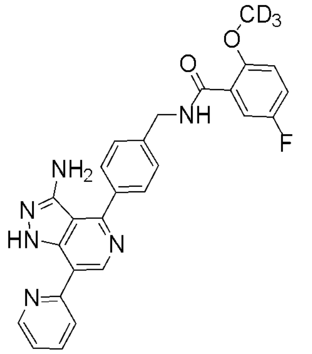,
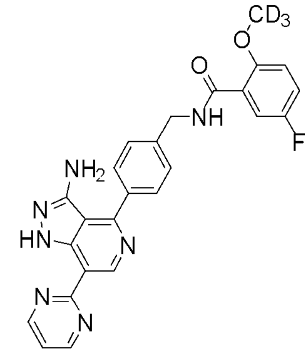,
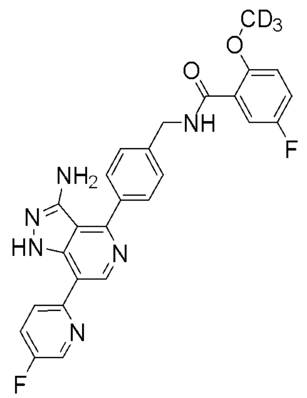,

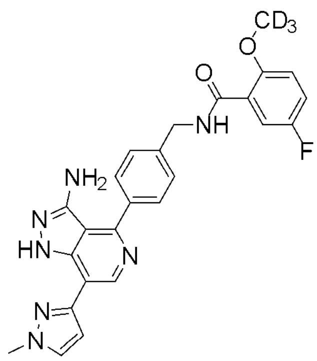,
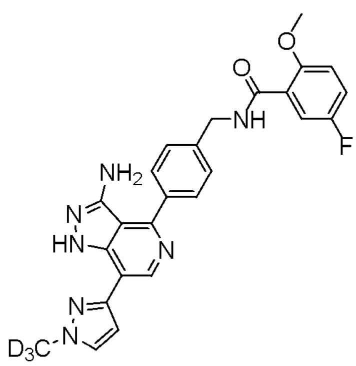,
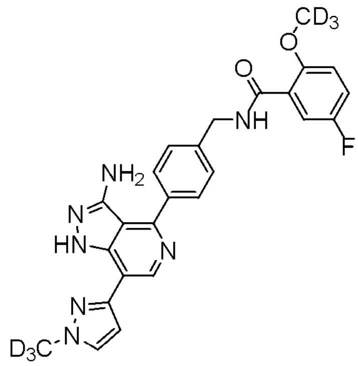,

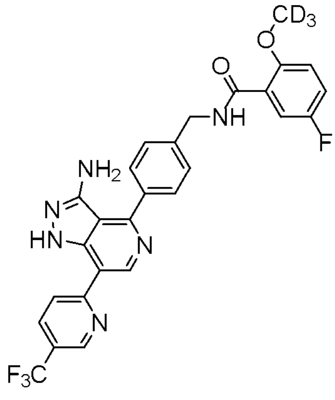,
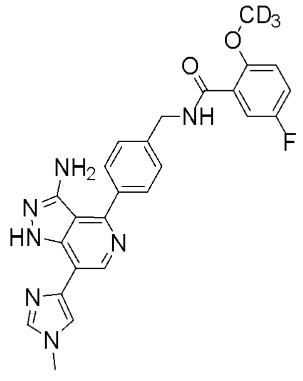,
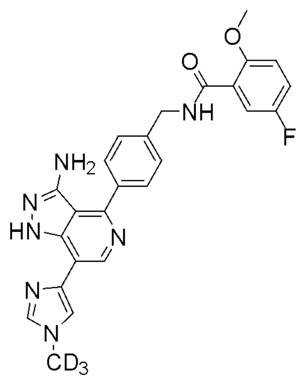,

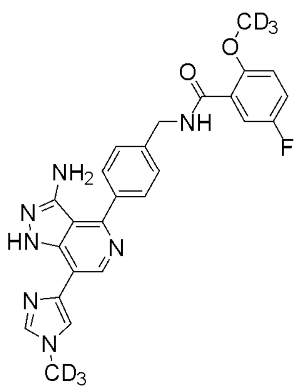,
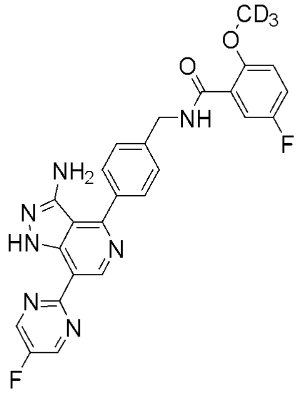,
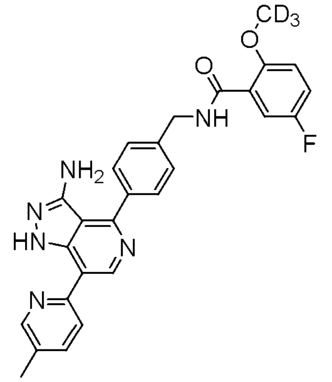,

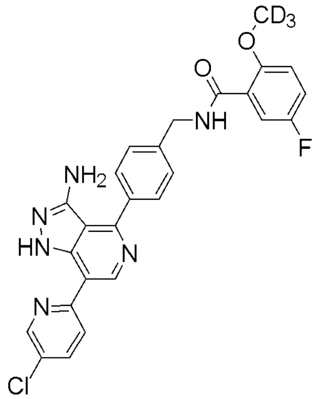,
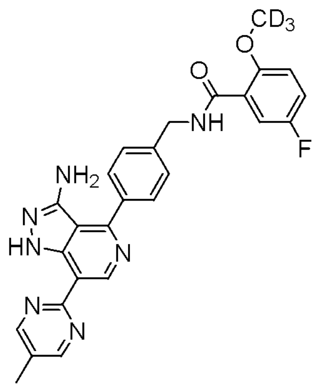,
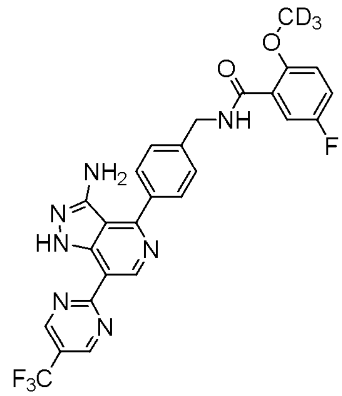,

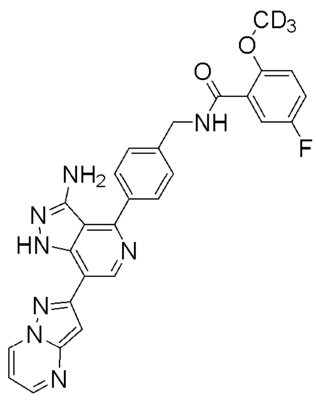,
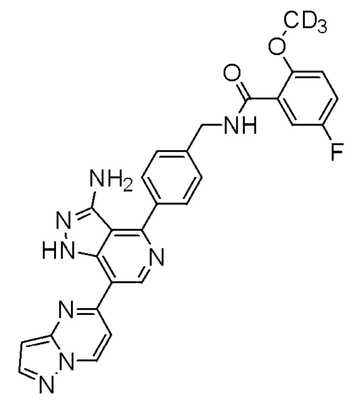,
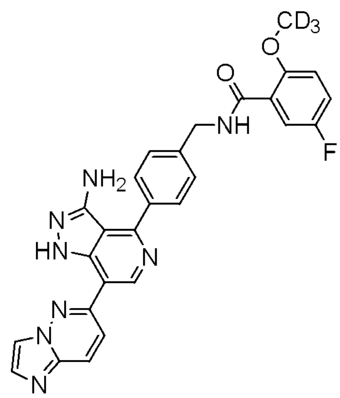,

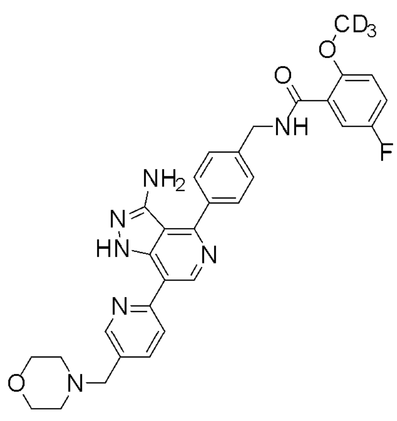

,

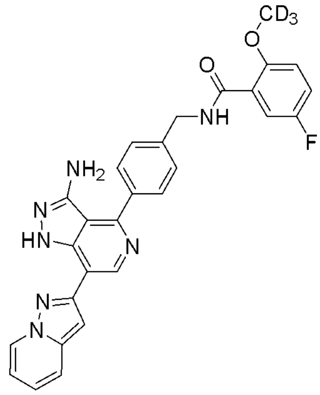

,

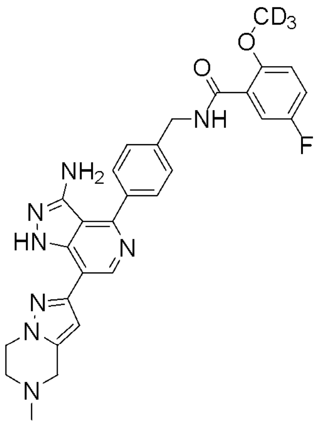

,

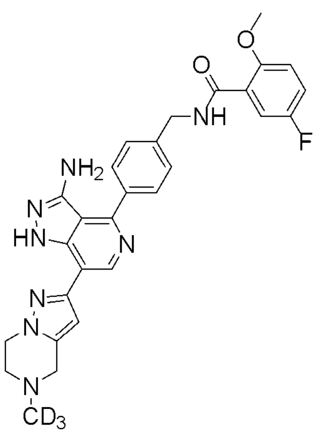

,

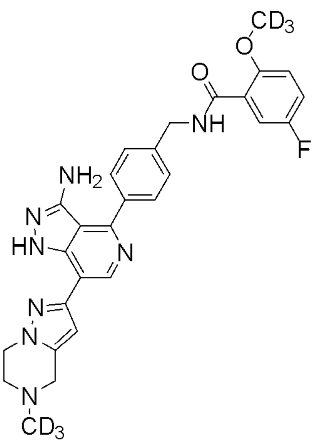

,

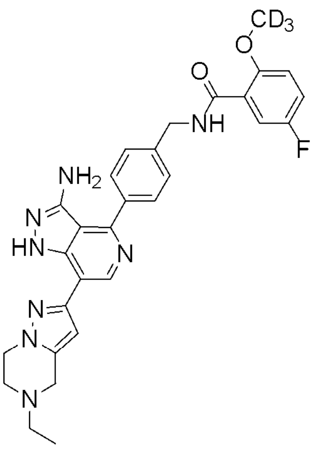

,

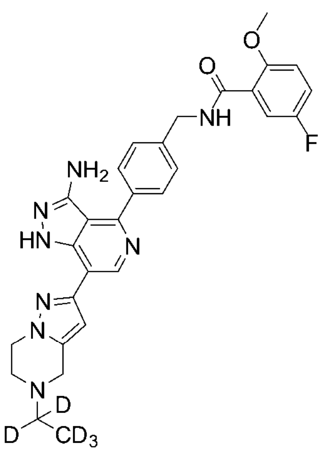

,

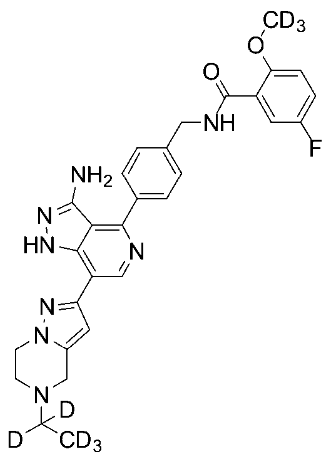

,

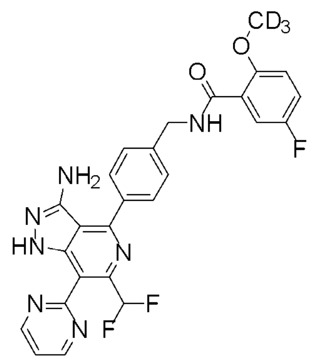

,

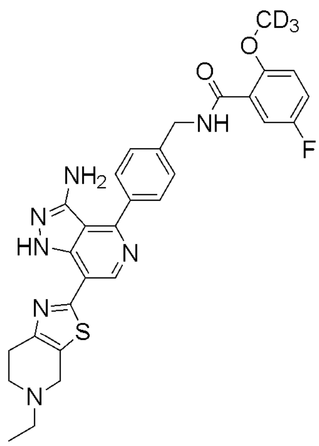
,
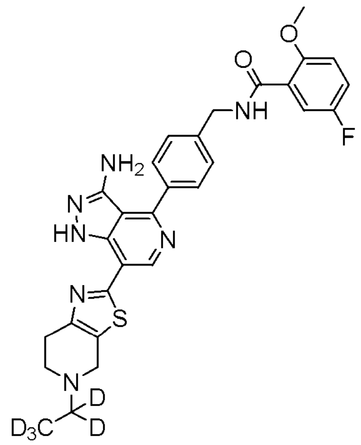
,
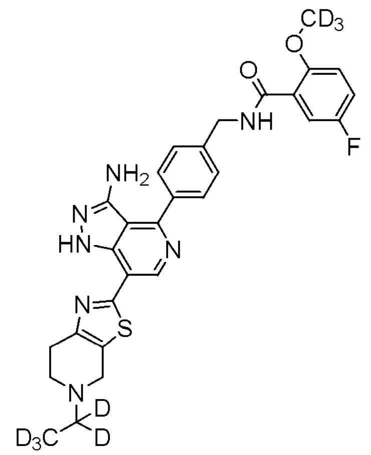
,
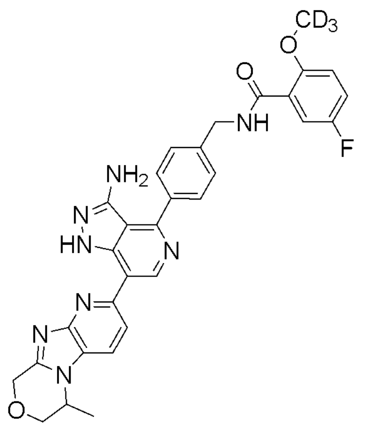
,
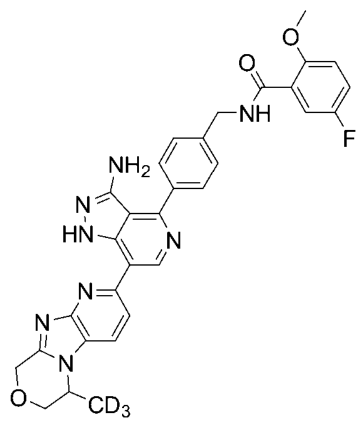
,
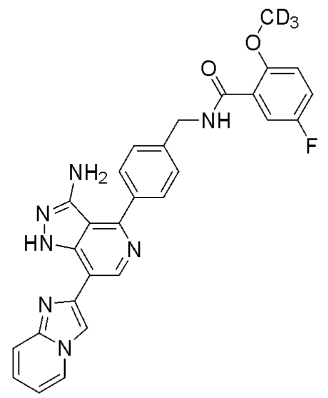
,
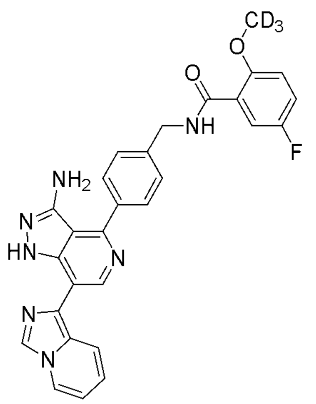
,
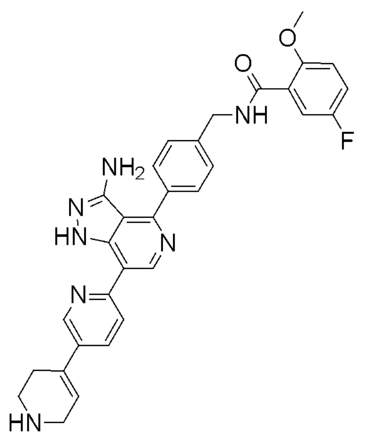
,
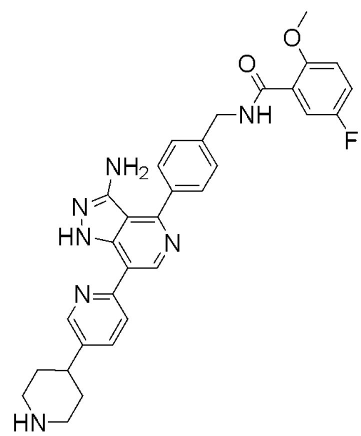
,

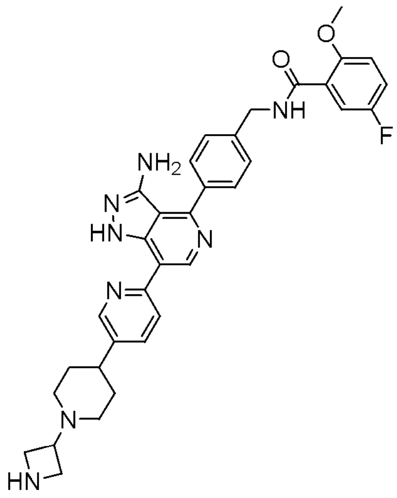
,
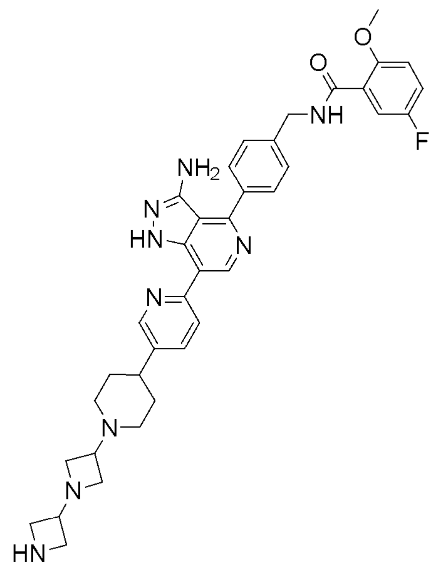
,
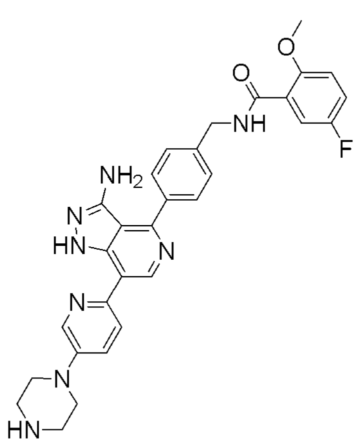
,
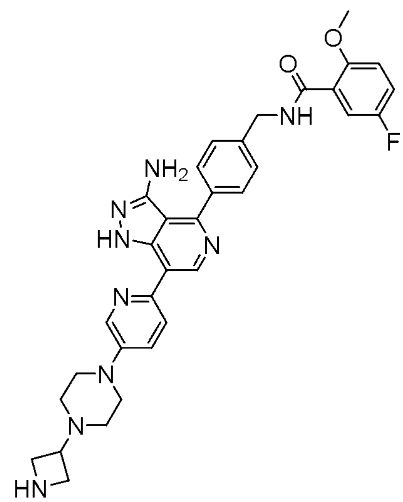
,
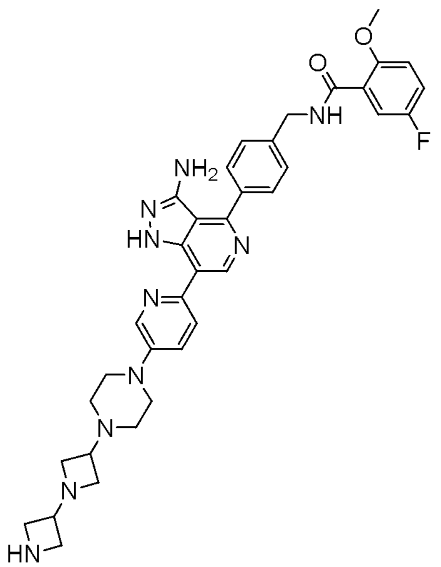
,
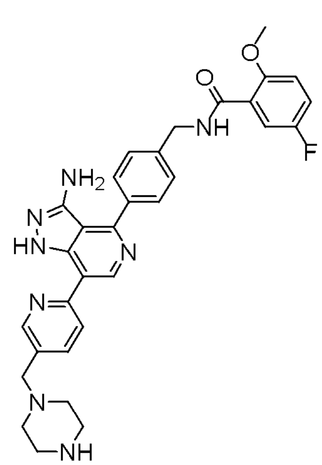
,
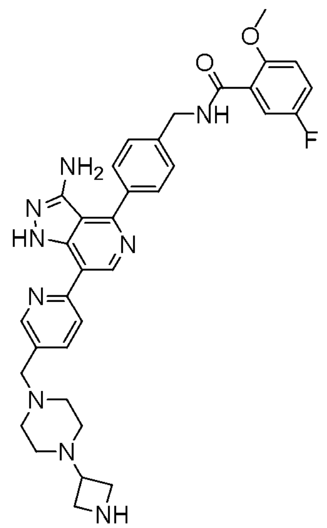
,

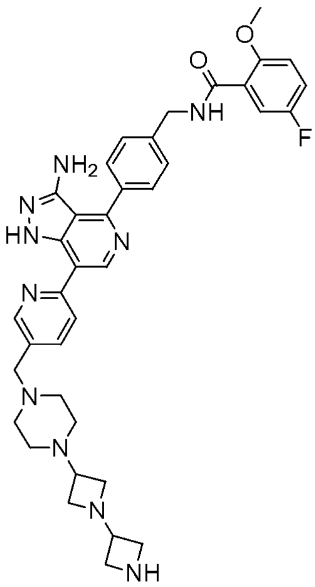
,
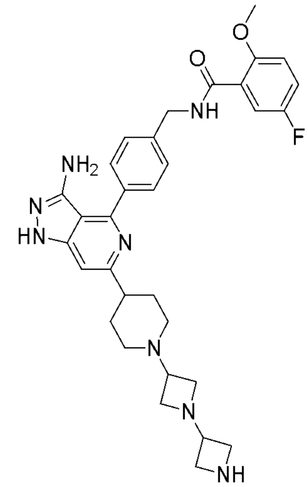
HCl
,
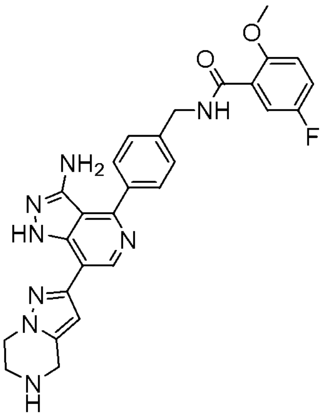
,

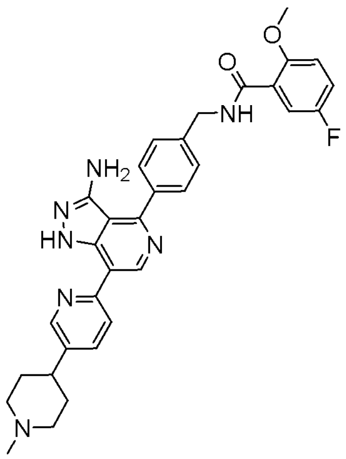
,
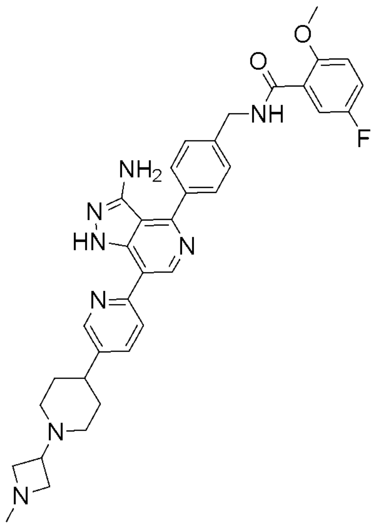
,
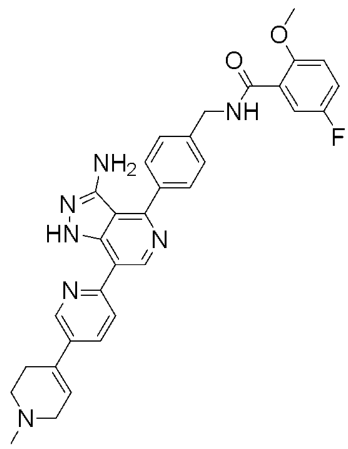
,

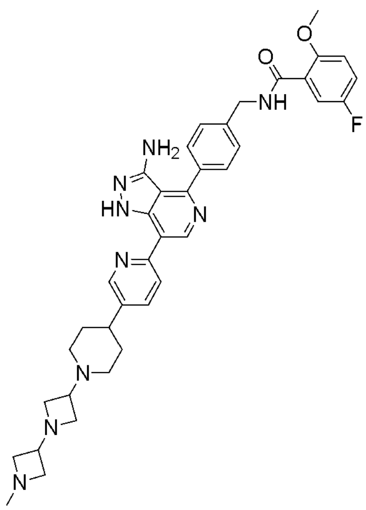
,
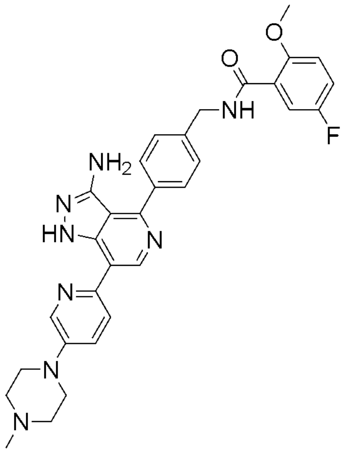
,
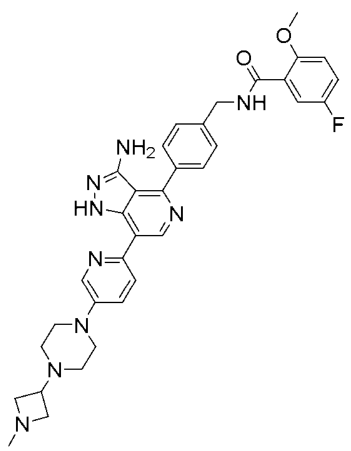
,

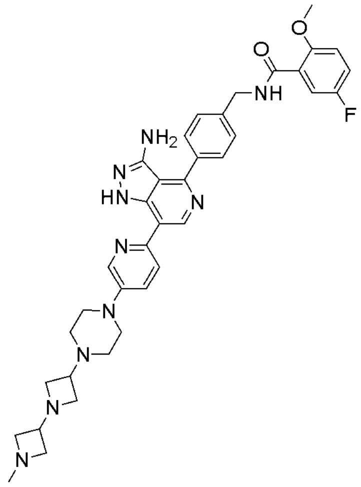
,
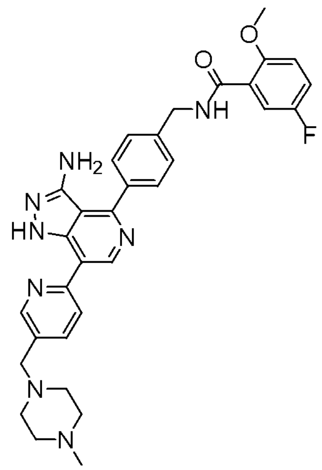
,
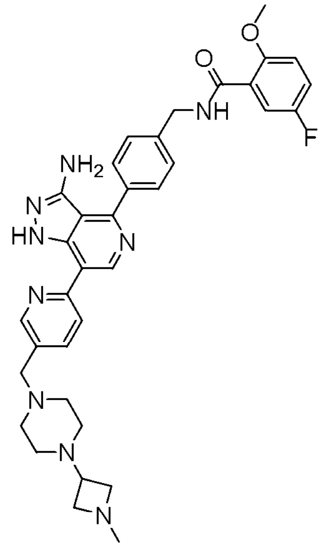
,

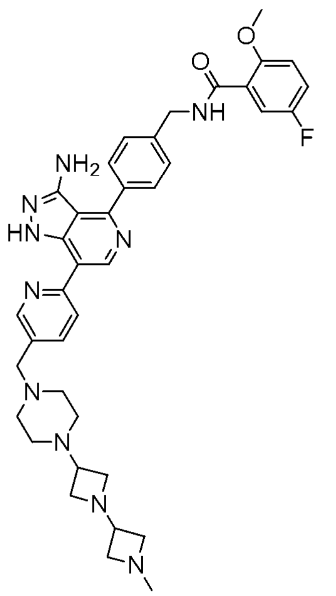
,
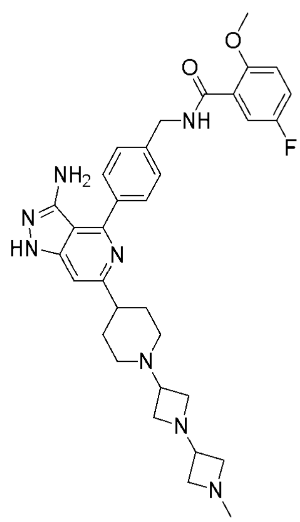
,
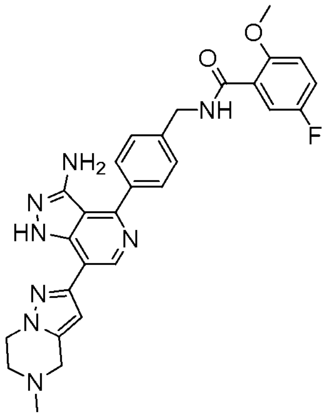
,

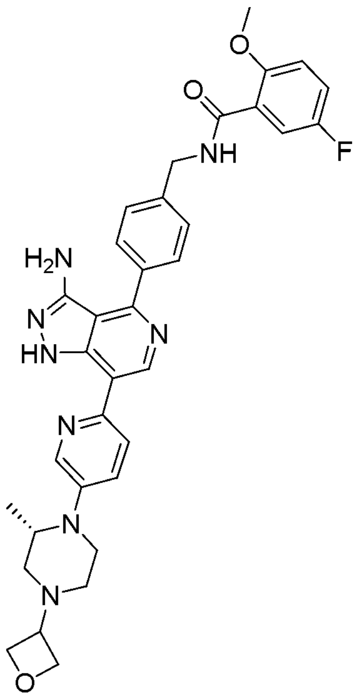

,

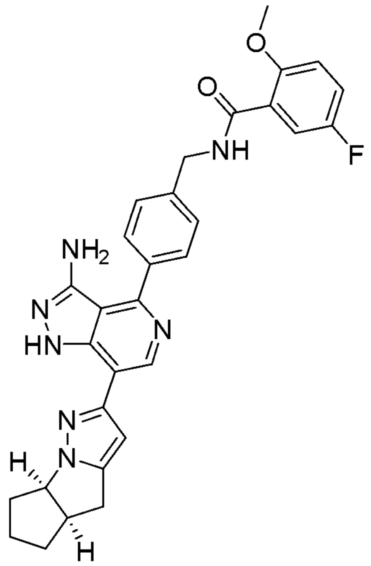

,

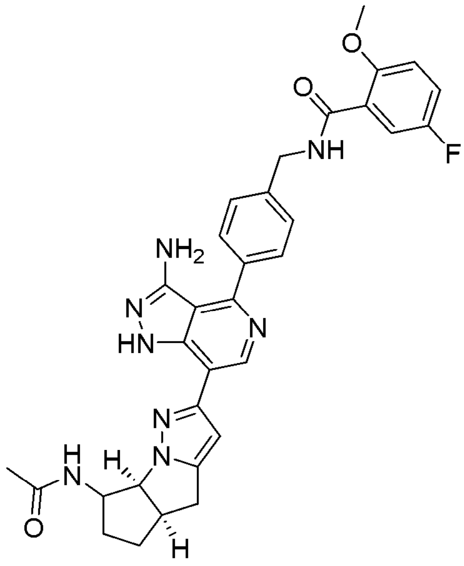

,

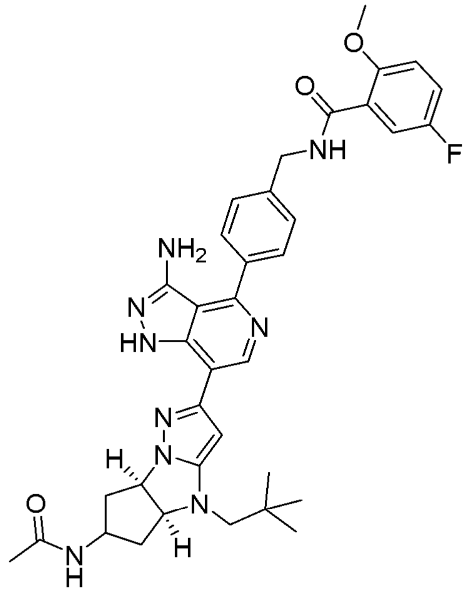

,

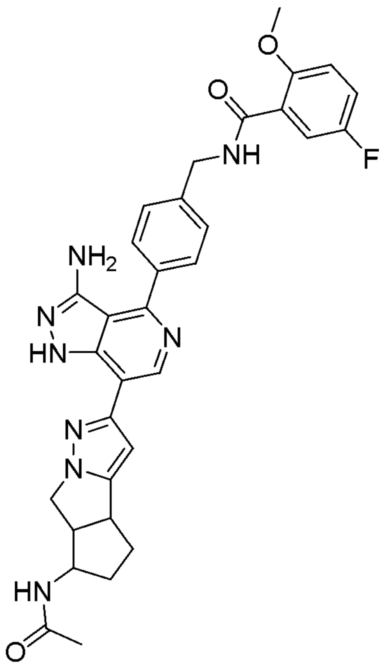

,

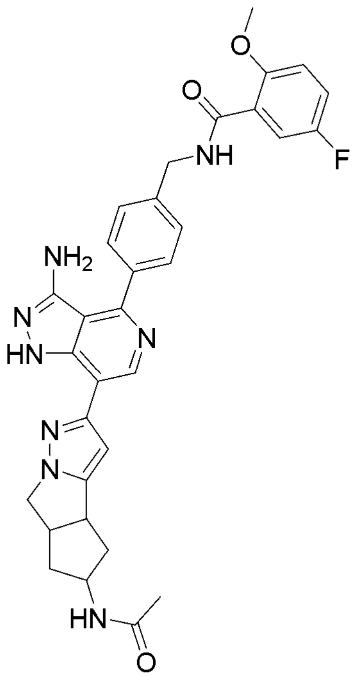

,

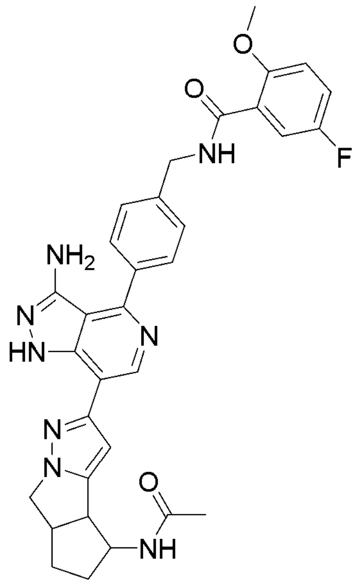

and

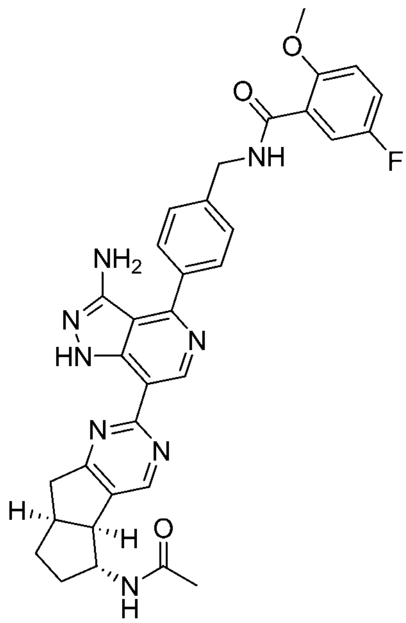

.

**56.** A pharmaceutical composition, comprising the compound according to any one of claims 1 to 55, or a tautomer, a stereoisomer, a prodrug, a crystal form, a pharmaceutically acceptable salt, a hydrate or a solvate thereof, and a pharmaceutically acceptable excipient, and optionally, other therapeutic agent(s).

**57.** Use of the compound according to any one of claims 1 to 55, or a tautomer, a stereoisomer, a prodrug, a crystal form, a pharmaceutically acceptable salt, a hydrate or a solvate thereof, or the pharmaceutical composition according to claim 56 in the manufacture of a medicament for the treatment and/or prevention of a wild and/or mutated BTK kinase-mediated disease; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S; optionally, the mutated BTK kinase is BTK V416L, BTK A428D, BTK M437R, BTK T474I or BTK L528W; optionally, the mutated BTK kinase is BTK T474I or BTK L528W.

**58.** A method of treating and/or preventing a wild and/or mutated BTK kinase-mediated disease in a subject, comprising administering to the subject the compound according to any one of claims 1 to 55, or a tautomer, a stereoisomer, a prodrug, a crystal form, a pharmaceutically acceptable salt, a hydrate or a solvate thereof, or the pharmaceutical composition according to claim 56; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S; optionally, the mutated BTK kinase is BTK V416L, BTK A428D, BTK M437R, BTK T474I or BTK L528W; optionally, the mutated BTK kinase is BTK T474I or BTK L528W.

**59.** The compound according to any one of claims 1 to 55, or a tautomer, a stereoisomer, a prodrug, a crystal form, a pharmaceutically acceptable salt, a hydrate or a solvate thereof, or the pharmaceutical composition according to claim 56, for use in the treatment and/or prevention of a wild and/or mutated BTK kinase-mediated disease; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S; optionally, the mutated BTK kinase is BTK V416L, BTK A428D, BTK M437R, BTK T474I or BTK L528W; optionally, the mutated BTK kinase is BTK T474I or BTK L528W.

**60.** The use according to claim 57, or the method according to claim 58, or the compound or composition for use according to claim 59, wherein the BTK-mediated disease is selected from an allergic disease, an autoimmune disease, an inflammatory disease, and a cancer.

**61.** The use according to claim 57, or the method according to claim 58, or the compound or composition for use according to claim 59, wherein the BTK-mediated disease is a B-cell proliferative disease selected from chronic lymphocytic lymphoma, non-Hodgkin's lymphoma, diffuse large B-cell lymphoma, mantle cell lymphoma, follicular lymphoma, chronic lymphocytic leukemia, acute lymphocytic leukemia, acute myeloid leukemia, acute promyelocytic leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplasia, mixed lineage leukemia, myelodysplastic syndrome, myeloproliferative disorder, marginal zone lymphoma, and Waldenstrom's macroglobulinemia.

**62.** The use according to claim 57 or the method according to claim 58 or the compound or composition for use according to claim 59, wherein the BTK-mediated disease is multiple myeloma.

## INTERNATIONAL SEARCH REPORT

International application No.
**PCT/CN2024/103173**

**A. CLASSIFICATION OF SUBJECT MATTER**

C07D 471/04(2006.01)i； A61P35/00(2006.01)i； A61P37/00(2006.01)i

According to International Patent Classification (IPC) or to both national classification and IPC

**B. FIELDS SEARCHED**

Minimum documentation searched (classification system followed by classification symbols)

IPC: C07D A61P

Documentation searched other than minimum documentation to the extent that such documents are included in the fields searched

Electronic data base consulted during the international search (name of data base and, where practicable, search terms used)

CNTXT; ENTXT; ENTXTC; VCN; VEN; STN; CJFD; CNKI; ISI-Web of Science: 超星读秀, CHAOXING DUXIU; 万方, WANFANG: 塔吉瑞生物医药, 布鲁顿, Bruton, 酪氨酸激酶, BTK, 吡唑, 吡啶, 嘧啶, 杂环, 氟, 酰胺, 抑制剂, 结构式(I), structural formula (I), Bruton Tyrosine Kinase, Fluoro+, Pyrazol+, Pyridin+, Pyrimidin+, Heterocycl+, Inhibitor+

**C. DOCUMENTS CONSIDERED TO BE RELEVANT**

| Category* | Citation of document, with indication, where appropriate, of the relevant passages | Relevant to claim No. |
|---|---|---|
| A | CN 114075190 A (BEIJING INNOCARE PHARMACEUTICAL TECHNOLOGY CO., LTD.) 22 February 2022 (2022-02-22)<br>claims 9-14 | 1-57, 60-62 |
| A | CN 103917545 A (MERCK SHARP & DOHME CORP. et al.) 09 July 2014 (2014-07-09)<br>entire document | 1-57, 60-62 |
| A | CN 108431007 A (LOXO ONCOLOGY, INC.) 21 August 2018 (2018-08-21)<br>entire document | 1-57, 60-62 |
| A | CN 115611902 A (GLUETACS THERAPEUTICS (SHANGHAI) CO., LTD.) 17 January 2023 (2023-01-17)<br>entire document | 1-57, 60-62 |
| A | US 2014073626 A1 (PRINCIPIA BIOPHARMA INC. et al.) 13 March 2014 (2014-03-13)<br>entire document | 1-57, 60-62 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 September 2024** | **30 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/103173**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **58-59**
because they relate to subject matter not required to be searched by this Authority, namely:

Claim 58 sets forth a method for treating and/or preventing wild and/or mutated BTK kinase-mediated diseases in a subject, and claim 59 sets forth the use of the compound of any one of claims 1-55, or a tautomer, stereoisomer, prodrug, crystal form, pharmaceutically acceptable salt, hydrate or solvate thereof, or the pharmaceutical composition of claim 56 for treating and/or preventing wild and/or mutated BTK kinase-mediated diseases. The above methods fall within the scope of methods for treatment of the human body, i.e., fall within the cases set out in PCT Rule 39.1(iv) for which an international search is not required.

Claim 57 relates to the use in the preparation of a drug for treating and/or preventing wild and/or mutated BTK kinase-mediated diseases. Therefore, no expectation of pharmaceutical use is provided with regard to claims 58 and 59, and the search and written opinion are not provided.

2. ☑ Claims Nos.: **60-62 (in part)**
because they relate to parts of the international application that do not comply with the prescribed requirements to such an extent that no meaningful international search can be carried out, specifically:

Claims 58 and 59 fall within the scope of methods for treatment of the human body, i.e. fall within the cases set out in PCT Rule 39.1(iv) for which an international search is not required. Therefore, claims 60-62, which directly refer to claims 58 and 59, also fall within the scope of methods for treatment of the human body, i.e., fall within the cases set out in PCT Rule 39.1(iv) for which an international search is not required, and the search and written opinion are not provided.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/103173**

| Patent document cited in search report | Publication date (day/month/year) | Patent family member(s) | Publication date (day/month/year) |
|---|---|---|---|
| CN 114075190 A | 22 February 2022 | WO 2022037649 A1 | 24 February 2022 |
| | | US 2023192691 A1 | 22 June 2023 |
| | | JP 2023538091 A | 06 September 2023 |
| CN 103917545 A | 09 July 2014 | EP 2734529 A1 | 28 May 2014 |
| | | EP 2734529 A4 | 03 June 2015 |
| | | EP 2734529 B1 | 05 September 2018 |
| | | AU 2012286426 A1 | 30 January 2014 |
| | | EP 2548877 A1 | 23 January 2013 |
| | | WO 2013010380 A1 | 24 January 2013 |
| | | CA 2841899 A1 | 24 January 2013 |
| | | MX 2014000747 A | 15 October 2014 |
| | | KR 20140068020 A | 05 June 2014 |
| | | JP 2014520866 A | 25 August 2014 |
| | | BR 112014001279 A2 | 24 July 2018 |
| | | US 2017008899 A1 | 12 January 2017 |
| | | US 9718828 B2 | 01 August 2017 |
| | | RU 2014106020 A | 27 August 2015 |
| | | US 2014221333 A1 | 07 August 2014 |
| CN 108431007 A | 21 August 2018 | HK 1258779 A1 | 22 November 2019 |
| | | CL 2018000706 A1 | 13 July 2018 |
| | | AU 2016322063 A1 | 12 April 2018 |
| | | EP 4116303 A1 | 11 January 2023 |
| | | US 2018362537 A1 | 20 December 2018 |
| | | US 10399990 B2 | 03 September 2019 |
| | | US 2018298008 A1 | 18 October 2018 |
| | | US 10611766 B2 | 07 April 2020 |
| | | EA 201890730 A1 | 31 October 2018 |
| | | IL 258154 A | 31 May 2018 |
| | | CA 2998796 A1 | 23 March 2017 |
| | | KR 20180109842 A | 08 October 2018 |
| | | MX 2018003351 A | 17 September 2018 |
| | | US 2018362533 A1 | 20 December 2018 |
| | | US 10399989 B2 | 03 September 2019 |
| | | PH 12018500587 A1 | 15 October 2018 |
| | | ES 2929528 T3 | 29 November 2022 |
| | | EP 3350184 A1 | 25 July 2018 |
| | | EP 3350184 B1 | 19 October 2022 |
| | | JP 2023134428 A | 27 September 2023 |
| | | WO 2017046604 A1 | 23 March 2017 |
| | | JP 2018527384 A | 20 September 2018 |
| | | JP 7337502 B2 | 04 September 2023 |
| | | CN 108431007 B | 07 June 2022 |
| CN 115611902 A | 17 January 2023 | WO 2023284703 A1 | 19 January 2023 |
| US 2014073626 A1 | 13 March 2014 | MX 2013013331 A | 17 October 2014 |
| | | US 9187487 B2 | 17 November 2015 |
| | | EP 2710006 A1 | 26 March 2014 |
| | | JP 2014513728 A | 05 June 2014 |
| | | AU 2012255792 A1 | 07 November 2013 |
| | | CA 2836417 A1 | 22 November 2012 |
| | | WO 2012158785 A1 | 22 November 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310806589
- US 5376645 A


### Non-patent literature cited in the description


- **BERGE et al.** *J. Pharmaceutical Sciences*, 1977, vol. 66, 1-19
- Prodrugs as Novel Delivery Systems, A.C.S. Symposium Series. **T. HIGUCHI** ; **V. STELLA**. Bioreversible Carriers in Drug Design. American Pharmaceutical Association and Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130
- Remington's Pharmaceutical Sciences. Mack Publishing Company, 1985